# FASCICULE DE BREVET EUROPEEN

(11) **EP 1 905 833 B1**
(45) Date de publication et mention de la délivrance du brevet: **12.08.2015**
(21) Numéro de dépôt: 07019306.5
(22) Date de dépôt: 08.10.2003
(51) Int. Cl.: C12N 15/52, C12N 9/00, C12N 15/63, C12N 1/21, C07K 14/36, C12P 19/62, C12R 1/465

(54) **Polypeptides impliqués dans la biosynthèse des spiramycines, séquences nucléotidiques codant ces polypeptides et leurs applications**
Polypeptide, die an der Biosynthese von Spiramycinen beteiligt sind, Nukleotidsequenzen, die diese Polypetide kodieren, und ihre Anwendungen
Polypeptides involved in the biosynthesis of spiramycins, nucleotide sequences coding these polypeptides and their uses

(30) Priorité: 08.10.2002 FR 0212489; 27.02.2003 FR 0302439; 07.08.2003 US 493490 P
(43) Date de publication de la demande: 02.04.2008
(62) Demande divisionnaire de: 03807883.8
(73) Titulaire: Aventis Pharma S.A., 92160 Antony (FR); Centre National de la Recherche Scientifique (CNRS), 75794 Paris Cedex 16 (FR)
(72) Inventeur: Blondelet-Rouault, Marie-Hélène, 91400 Orsay (FR); Dominguez, Hélène, 84000 Avignon (FR); Darbon-Rongere, Emmanuelle, 78960 Voisins Le Bretonneux (FR); Gerbaud, Claude, 93220 Gagny (FR); Gondran, Anne, 75014 Paris (FR); Karray, Fatma, 91440 Bures sur Yvette (FR); Lacroix, Patricia, 94170 Le Perreux (FR); Oestreicher-Mermet-Bouvier, Nathalie, 92140 Clamart (FR); Pernodet, Jean-Luc, 94230 Cachan (FR); Tuphile, Karine, 75013 Paris (FR)
(74) Mandataire: Blot, Philippe Robert Emile

(56) Documents cités:
- EP-A- 0 345 546
- EP-A- 0 346 000
- EP-A- 0 354 641
- EP-A- 0 459 525
- EPP J K ET AL: "PRODUCTION OF A HYBRID MACROLIDE ANTIBIOTIC IN STREPTOMYCES AMBOFACIENS AND STREPTOMYCES LIVIDANS BY INTRODUCTION OF A CLONED CARBOMYCIN BIOSYNTHETIC GENE FROM STREPTOMYCES THERMOTOLERANS" GENE, ELSEVIER BIOMEDICAL PRESS. AMSTERDAM, NL, vol. 85, no. 2, 28 décembre 1989 (1989-12-28), pages 293-301, XP000094837 ISSN: 0378-1119 -& DATABASE EMBL [Online] 27 juillet 1994 (1994-07-27), ARISAWA A: "Streptomyces thermotolerans carB, acyB1 or carE, and acyB2 genes for carbomycin resistance determinant, 4'-mycarosyl isovaleryl-CoA transferase and regulatory protein, partial and complete cds." XP002284347 Database accession no. D31821
- BATE NEIL ET AL: "The mycarose-biosynthetic genes of Streptomyces fradiae, producer of tylosin" MICROBIOLOGY (READING), vol. 146, no. 1, janvier 2000 (2000-01), pages 139-146, XP002284343 ISSN: 1350-0872 -& DATABASE EMBL [Online] 20 avril 1994 (1994-04-20), BATE N ET AL: "Streptomyces fradiae cytochrome P-450, dTDP-glucose synthase, dTDP-glucose dehydratase, thioesterase, TylCVI (tylCVI), and TylR (tylR) genes, complete cds; and unknown gene." XP002284348 Database accession no. U08223
- KARRAY FATMA ET AL: "Organization of the biosynthetic gene cluster for the macrolide antibiotic spiramycin in Streptomyces ambofaciens" MICROBIOLOGY (READING), vol. 153, no. Part 12, décembre 2007 (2007-12), pages 4111-4122, XP002481781 ISSN: 1350-0872 -& DATABASE EMBL [Online] 7 novembre 2007 (2007-11-07), "Streptomyces ambofaciens region of the spiramycin biosynthetic gene cluster upstream of the PKS genes, strain ATCC 23877" XP002481782 extrait de EBI accession no. EMBL:AM709784 Database accession no. AM709784

## Description

La présente invention concerne l'isolement et l'identification de nouveaux gènes de la voie de biosynthèse des spiramycines et de nouveaux polypeptides impliqués dans cette biosynthèse. Elle est également relative à l'utilisation de ces gènes dans le but d'augmenter les taux de production et la pureté de la spiramycine produite.

Les *Streptomyces* sont des bactéries filamenteuses Gram positif du sol. Elles jouent un rôle important dans la décomposition et la minéralisation des matières organiques grâce à la grande diversité des enzymes dégradatives qu'elles sécrètent. Elles présentent des phénomènes de différenciations morphologiques uniques chez les procaryotes s'accompagnant d'une différenciation métabolique caractérisée par la production de métabolites secondaires présentant une extraordinaire diversité de structures chimiques et d'activités biologiques. Parmi ces métabolites, on retrouve les spiramycines produites à l'état naturel par la bactérie *Streptomyces ambofaciens.*

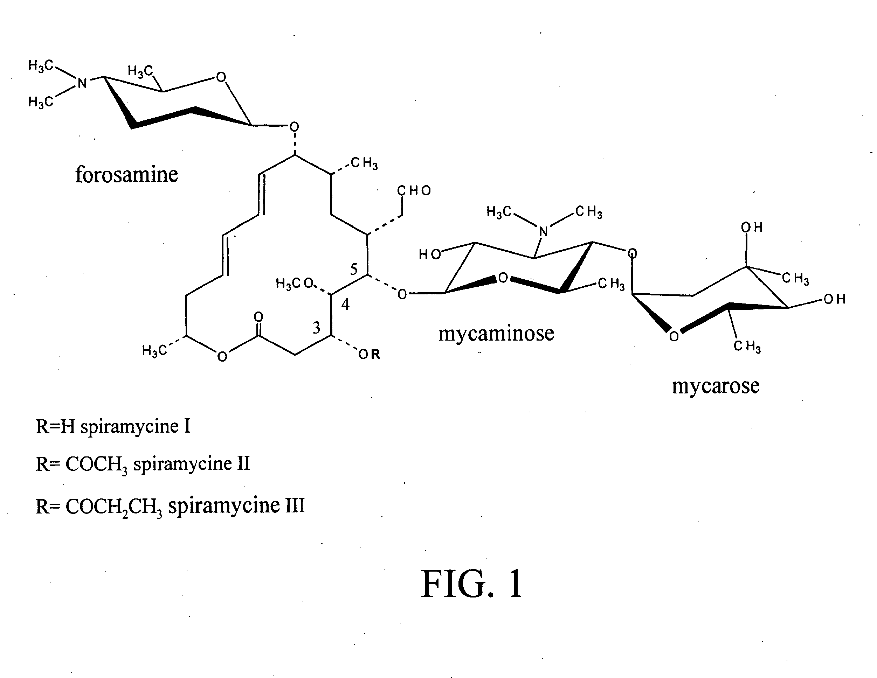

La spiramycine est un antibiotique de la famille des macrolides utile aussi bien en médecine vétérinaire qu'en médecine humaine. Les macrolides sont caractérisés par la présence d'un cycle lactone sur lequel sont greffés un ou plusieurs sucres. *Streptomyces ambofaciens* produit à l'état naturel la spiramycine I, II et III (cf. Figure 1), cependant l'activité antibiotique de la spiramycine I est nettement supérieure à celle des spiramycines II et III (Liu *et al.*, 1999). La molécule de spiramycine I est constituée d'un macro-cycle lactonique, appellé platénolide et de trois sucres : la forosamine, le mycaminose et le mycarose (cf. Figure 1). L'activité antibiotique des spiramycines est due à une inhibition de la synthèse protéique chez les procaryotes par un mécanisme impliquant la liaison de l'antibiotique au ribosome bactérien.

Certains composés membres de la famille des macrolides et possédant également un cycle lactone ont donné lieu à des utilisations hors du domaine des antibiotiques. Ainsi le produit FK506 a des effets immunosuppresseurs et offre des perspectives d'application thérapeutique dans le domaine de la transplantation d'organe, de l'arthrite rhumatoïde et plus généralement dans des pathologies liées à des réactions autoimmunes. D'autres macrolides comme l'avermectine ont des activités insecticides et anti-helminthiques.

De nombreuses voies de biosynthèse, concernant des antibiotiques appartenant à des classes variées ainsi que d'autres métabolites secondaires (pour une revue, Chater K. 1990), ont à ce jour déjà été étudiées chez les *Streptomycètes*. Cependant, la connaissance des voies de biosynthèse des spiramycines n'est que très partielle à ce jour.

La biosynthèse des spiramycines est un processus complexe comportant de nombreuses étapes et impliquant de nombreuses enzymes (Omura *et al.,* 1979a, Omura *et al.,* 1979b). Les spiramycines appartiennent à la large classe des polyketides qui regroupe des molécules complexes particulièrement abondantes dans les microorganismes du sol. Ces molécules sont regroupées non pas par analogie de structure mais par une certaine similarité au niveau d'étapes de leur voie de biosynthèse. En effet, les polyketides sont produits par une série complexe de réactions mais possèdent en commun d'avoir dans leur voie de biosynthèse une série de réactions catalysées par une ou des enzymes appelées « polyketides synthases » (PKS). Chez *Streptomyces ambofaciens*, la biosynthèse du macro-cycle lactonique des spiramycines (platénolide) est réalisée par une série de huit modules codés par cinq gènes PKS différents (Kuhstoss S., 1996, brevet US 5,945,320). Les spiramycines sont obtenues à partir de ce cycle lactone. Cependant, les diverses étapes et enzymes impliquées dans la synthèse des sucres, ainsi que leur liaison au cycle lactone et la modification de ce cycle pour l'obtention des spiramycines restent encore inconnues à ce jour.

Le brevet US 5,514,544 décrit le clonage d'une séquence appelée *srmR* chez *Streptomyces ambofaciens.* Il est fait l'hypothèse dans ce brevet que le gène *srmR* code une protéine régulatrice de la transcription des gènes impliqués dans la biosynthèse des macrolides.

Epp et al. (Gene 1989, 85(2), 293-301) ont décrit une protéine régulatrice impliquée dans la biosynthèse de carbomycine chez *S.thermotolerans* et codée par le gène acyB2.

En 1987, Richardson et collaborateurs (Richardson et al., 1987) ont montré que la résistance à la spiramycine de *S. ambofaciens* est conférée par au moins trois gènes, ces derniers ont été appelés *srmA, srmB* et *srmC*. Le brevet US 4,886,757 décrit plus particulièrement la caractérisation d'un fragment d'ADN de *S. ambofaciens* contenant le gène *srmC*. Cependant la séquence de ce gène n'a pas été divulguée. En 1990, Richardson et collaborateurs (Richardson *et al..,* 1990) ont posé l'hypothèse de l'existence de trois gènes de biosynthèse de la spiramycine proche de *srmB*. Le brevet US 5,098,837 rapporte le clonage de cinq gènes potentiellement impliqués dans la biosynthèse de la spiramycine. Ces gènes ont été baptisés *srmD, srmE*, *srmF*, *srmG* et *srmH.*

Une des difficultés majeures dans la production de composés tels que les spiramycines réside dans le fait que de très grands volumes de fermentation sont nécessaires à la production d'une quantité relativement faible de produit. Il est donc souhaitable de pouvoir augmenter l'efficacité de production de telles molécules afin d'en diminuer les coûts de production.

La voie de biosynthèse des spiramycines est un processus complexe et il serait souhaitable d'identifier et de supprimer les réactions parasites qui pourraient exister au cours de ce processus. Le but d'une telle manipulation est d'obtenir un antibiotique plus pur et/ou une amélioration de la productivité. A ce sujet, *Streptomyces ambofaciens* produit à l'état naturel la spiramycine I, II et III (cf. Figure 1), cependant l'activité antibiotique de la spiramycine I est nettement supérieure à celle des spiramycines II et III (Liu *et al.,* 1999). Il serait donc souhaitable de pouvoir disposer de souches ne produisant que de la spiramycine I.

### Description générale de l'invention

La présente invention résulte du clonage de gènes dont le produit intervient dans la biosynthèse des spiramycines. L'invention concerne tout d'abord de nouveaux gènes de la voie de biosynthèse des spiramycines et de nouveaux polypeptides impliqués dans cette biosynthèse.

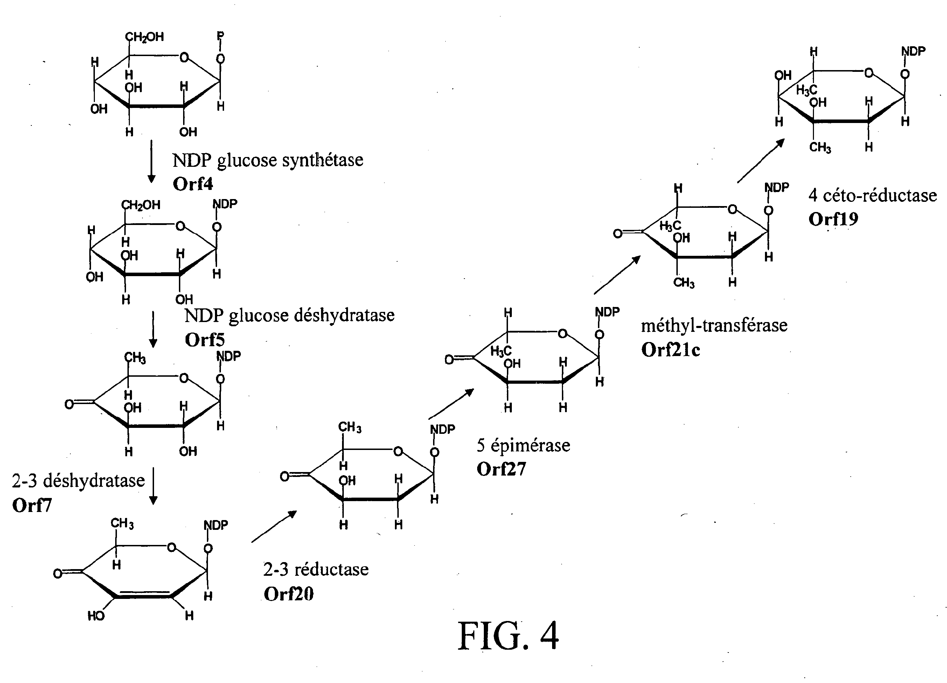

Les gènes de la voie de biosynthèse et les séquences codantes associées ont été clonés et la séquence d'ADN de chacun a été déterminée. Les séquences codantes clonées seront désignées par la suite *orf1*c, orf2*c, orf3*c, orf4*c, orf5*, orf6*, orf7*c, orf8* orf9*, orf10*, orf1, orf2, orf3, orf4, orf5, orf6, orf7, orf8, orf9c, orf10, orf11c, orf12, orf13c, orf14, orf15c, orf16, orf17, orf18, orf19, orf20, orf21c, orf22c, orf23c***,** *orf24c, orf25c, orf26***,** *orf27, orf28c, orf29, orf30c, orf31, orf32c, orf33 et orf34c.* La fonction des protéines codées par ces séquences dans la voie de biosynthèse des spiramycines est développée dans la discussion ci-après qui est illustrée par les figures 4, 5, 6 et 8.

L'invention a donc pour objet, tel qu'énoncé dans les revendications 1 à 19 :
(1) Un polynucléotide codant un polypeptide impliqué dans la biosynthèse des spiramycines caractérisé en ce que la surexpression dudit polynucléotide permet une augmentation de la production des spiramycines et en ce que la séquence dudit polynucléotide est :
   (a) l'une des séquences SEQ ID N° 111 ou 141,
   (b) un polynucléotide présentant au moins 80%, 85%, 90%, 95% ou 98% d'identité en nucléotides avec le polynucléotide selon (a).
(2) Un polynucléotide tel que mentionné au point (1) caractérisé en ce qu'il est isolé à partir d'une bactérie du genre *Streptomyces.*
(3) Un polypeptide impliqué dans la biosynthèse des spiramycines caractérisé en ce que sa surexpression permet une augmentation de la production des spiramycines et en ce que la séquence dudit polypeptide comprend :
   (a) l'une des séquences SEQ ID N° 112 ou 142,
   (b) un polypeptide présentant au moins 80%, 85%, 90%, 95% ou 98% d'identité en acides aminés avec le polypeptide selon (a).
(4) Un polypeptide tel que mentionné au point (3) caractérisé en ce que sa séquence comprend l'une des séquences SEQ ID N° 112 ou 142.
(5) Un polypeptide tel que mentionné au point (3) caractérisé en ce qu'il est isolé à partir d'une bactérie du genre *Streptomyces.*
(6) Un vecteur d'expression caractérisé en ce qu'il comprend au moins une séquence d'acide nucléique codant un polypeptide tel que mentionné au point (3), (4) ou (5).
(7) Une cellule hôte comprenant un vecteur d'expression approprié permettant l'expression d'un ou plusieurs polypeptides tels que mentionnés au point (3), (4) ou (5).
(8) Une cellule hôte telle que mentionnée au point (7) caractérisée en ce qu'elle est choisie parmi les cellules hôtes procaryotes ou les cellules hôtes eucaryotes.
(9) Une cellule hôte telle que mentionnée au point (8) caractérisée en ce qu'elle est choisie parmi la bactérie *E. coli*, les cellules d'insectes, les cellules de levures, les cellules de mammifères.
(10) Une cellule hôte telle que mentionnée au point (7) dans laquelle a été introduit au moins un polynucléotide tel que défini au point (1) ou (2), et/ou au moins un vecteur d'expression tel que défini au point (6).
(11) Un procédé de production d'un polypeptide tel que défini selon l'un des points (3) à (5), caractérisé en ce que ledit procédé comprend les étapes suivantes :
   a) insérer au moins un acide nucléique codant ledit polypeptide dans un vecteur approprié;
   b) cultiver, dans un milieu de culture approprié, une cellule hôte préalablement transformée ou transfectée avec le vecteur de l'étape a) ;
   c) récupérer le milieu de culture conditionné ou un extrait cellulaire;
   d) séparer et purifier à partir dudit milieu de culture ou encore à partir de l'extrait cellulaire obtenu à l'étape c), ledit polypeptide ;
   e) le cas échéant, caractériser le polypeptide recombinant produit.
(12) L'utilisation d'un polynucléotide tel que défini selon l'un des points (1) et (2) pour améliorer la production en spiramycine d'un microorganisme.
(13) Un microorganisme mutant producteur de spiramycine caractérisé en ce qu'il surexprime au moins une séquence codante comprenant une séquence telle que définie selon l'un des points (1) et (2), ou une séquence codante qui code un polypeptide tel que défini selon l'un des points (3), (4) et (5), la surexpression de la séquence codante considérée étant obtenue en transformant un microorganisme producteur de spiramycine par un vecteur d'expression tel que défini au point (6), permettant la surexpression de cette séquence codante.
(14) Un microorganisme mutant tel que défini au point (13) caractérisé en ce que le microorganisme surexprime une ou plusieurs séquences codantes comprenant une des séquences polynucléotidiques telles que définies au point (1).
(15) Un microorganisme mutant tel que défini au point (13) ou (14) caractérisé en ce que ledit microorganisme est une bactérie du genre *Streptomyces.*
(16) Un microorganisme mutant tel que défini au point (13), (14) ou (15), caractérisé en ce que ledit microorganisme est une souche de *S*. *ambofaciens..*
(17) Un procédé de production de spiramycine(s), caractérisé en ce qu'il comprend les étapes suivantes :
   (a) cultiver, dans un milieu de culture approprié, un microorganisme tel que défini selon l'un des points (13) à (16),
   (b) récupérer le milieu de culture conditionné ou un extrait cellulaire,
   (c) séparer et purifier à partir dudit milieu de culture ou encore à partir de l'extrait cellulaire obtenu à l'étape b), les spiramycines produites.
(18) Une souche de *Streptomyces ambofaciens* caractérisée en ce qu'il s'agit de la souche OSC2/pSPM75(2) déposée auprès de la Collection Nationale de Cultures de Microorganismes (CNCM) Institut Pasteur, 25, rue du Docteur Roux 75724 Paris Cedex 15, France, le 6 octobre 2003 sous le numéro d'enregistrement 1-3101.
(19) Un procédé de production de spiramycine(s), caractérisé en ce qu'il comprend les étapes suivantes :
   (a) cultiver, dans un milieu de culture approprié, un microorganisme tel que défini au point (18),
   (b) récupérer le milieu de culture conditionné ou un extrait cellulaire,
   (c) séparer et purifier à partir dudit milieu de culture ou encore à partir de l'extrait cellulaire obtenu à l'étape b), les spiramycines.

### Définitions générales

Le terme " isolé " au sens de la présente invention désigne un matériel biologique (acide nucléique ou protéine) qui a été soustrait à son environnement originel (l'environnement dans lequel il est localisé naturellement).

Par exemple un polynucléotide présent à l'état naturel dans une plante ou un animal n'est pas isolé. Le même polynucléotide séparé des acides nucléiques adjacents au sein desquels il est naturellement inséré dans le génome de la plante ou l'animal est considéré comme " isolé ".

Un tel polynucléotide peut être inclus dans un vecteur et/ou un tel polynucléotide peut être inclus dans une composition et demeurer néanmoins à l'état isolé du fait que le vecteur ou la composition ne constitue pas son environnement naturel.

Le terme " purifié " ne nécessite pas que le matériel soit présent sous une forme de pureté absolue, exclusive de la présence d'autres composés. Il s'agit plutôt d'une définition relative.

Un polynucléotide est à l'état " purifié " après purification du matériel de départ ou du matériel naturel d'au moins un ordre de grandeur, de préférence 2 ou 3 et préférentiellement 4 ou 5 ordres de grandeur.

Aux fins de la présente invention le terme ORF (« Open Reading Frame », c'est à dire cadre ouvert de lecture) a été employé pour désigner en particulier la séquence codante d'un gène.

Aux fins de la présente description, l'expression " séquence nucléotidique " peut être employée pour désigner indifféremment un polynucléotide ou un acide nucléique. L'expression " séquence nucléotidique " englobe le matériel génétique lui-même et n'est donc pas restreinte à l'information concernant sa séquence.

Les termes " acide nucléique ", " polynucléotide ", " oligonucléotide " ou encore " séquence nucléotidique " englobent des séquences d'ARN, d'ADN, d'ADNc ou encore des séquences hybrides ARN/ADN de plus d'un nucléotide, indifféremment sous la forme simple chaîne ou sous la forme de duplex.

Le terme " nucléotide " désigne à la fois les nucléotides naturels (A, T, G, C) ainsi que des nucléotides modifiés qui comprennent au moins une modification telle que (1) un analogue d'une purine, (2) un analogue d'une pyrimidine, ou (3) un sucre analogue, des exemples de tels nucléotides modifiés étant décrits par exemple dans la demande PCT N°WO 95/04 064.

Aux fins de la présente invention, un premier polynucléotide est considéré comme étant " complémentaire " d'un second polynucléotide lorsque chaque base du premier polynucléotide est appariée à la base complémentaire du second polynucléotide dont l'orientation est inversée. Les bases complémentaires sont A et T (ou A et U), ou C et G.

Le terme « gènes de la voie de biosynthèse des spiramycines » comprend également les gènes régulateurs et les gènes conférant la résistance aux microorganismes producteurs.

On entendra par " fragment " d'un acide nucléique de référence selon l'invention, une séquence nucléotidique de longueur réduite par rapport à l'acide nucléique de référence et comprenant, sur la partie commune, une séquence en nucléotides identique à l'acide nucléique de référence.

Un tel " fragment " d'acide nucléique selon l'invention peut être le cas échéant, compris dans un polynucléotide plus grand duquel il est constitutif.

De tels fragments comprennent ou alternativement consistent en, des polynucléotides de longueur allant de 8, 10, 12, 15, 18, 20 à 25, 30, 40, 50, 60, 70, 80, 90, 100, 150, 200, 250, 300, 350, 400, 450, 500, 550, 600, 650, 700, 750, 800, 850, 900, 950, 1000, 1050, 1100, 1150, 1200, 1250, 1300, 1350, 1400, 1450, 1500, 1550, 1600, 1650, 1700, 1750, 1800, 1850 ou 1900 nucléotides consécutifs d'un acide nucléique selon l'invention.

Par " fragment " d'un polypeptide selon l'invention, on entendra un polypeptide dont la séquence d'acides aminés est plus courte que celle du polypeptide de référence et qui comprend sur toute la partie commune avec ces polypeptides de référence, une séquence en acides aminés identique.

De tels fragments peuvent, le cas échéant, être compris au sein d'un polypeptide plus grand duquel ils font partie.

De tels fragments d'un polypeptide selon l'invention peuvent avoir une longueur de 10, 15, 20, 30 à 40, 50, 60, 70, 80, 90,100, 120, 140, 160, 180, 200, 220, 240, 260, 280, 300, 320, 340, 360, 380, 400, 420, 440, 460, 480, 500, 520, 540, 560, 580, 600, 620 ou 640 acides aminés.

Par " conditions d'hybridation de forte stringence " au sens de la présente invention, on entendra des conditions d'hybridation défavorisant l'hybridation de brins d'acides nucléiques non homologues. Des conditions d'hybridations de forte stringence peuvent être par exemple décrites comme des conditions d'hybridation dans le tampon décrit par Church & Gilbert (Church & Gilbert, 1984) à une température comprise entre 55°C et 65°C, de manière préférée la température d'hyridation est de 55°C, de manière encore plus préférée la températue d'hybridation est de 60°C et de manière tout à fait préférée la température d'hybridation est de 65°C, suivi d'un ou plusieurs lavages effectué en tampon 2X SSC (le tampon 1X SSC correspond à une solution aqueuse 0,15M NaCl, 15 mM de citrate de sodium) à une température comprise entre 55°C et 65°C, de manière préférée cette température est de 55°C, de manière encore plus préférée cette températue est de 60°C et de manière tout à fait préférée cette température est de 65°C, suivi d'un ou plusieurs lavages en tampon 0.5X SSC à une température comprise entre 55°C et 65°C, de manière préférée cette température est de 55°C, de manière encore plus préférée cette températue est de 60°C et de manière tout à fait préférée cette température est de 65°C.

Il va sans dire que les conditions d'hybridation ci-dessus décrites peuvent être adaptées en fonction de la longueur de l'acide nucléique dont l'hybridation est recherchée ou du type de marquage choisi, selon des techniques connues de l'homme du métier. Les conditions convenables d'hybridation peuvent par exemple être adaptées selon l'ouvrage de F. Ausubel *et al* (2002).

Par " variant " d'un acide nucléique selon l'invention, on entendra un acide nucléique qui diffère d'une ou plusieurs bases par rapport au polynucléotide de référence. Un acide nucléique variant peut être d'origine naturelle, tel qu'un variant allélique retrouvé naturellement, ou peut être aussi un variant non naturel obtenu par exemple par des techniques de mutagénèse.

En général, les différences entre l'acide nucléique de référence et l'acide nucléique variant sont réduites de telle sorte que les séquences nucléotidiques de l'acide nucléique de référence et de l'acide nucléique variant sont très proches et, dans de nombreuses régions, identiques. Les modifications de nucléotides présentes dans un acide nucléique variant peuvent être silencieuses, ce qui signifie qu'elles n'altèrent pas les séquences d'aminoacides codées par ledit acide nucléique variant.

Cependant, les changements de nucléotides dans un acide nucléique variant peuvent aussi résulter de substitutions, additions, délétions dans le polypeptide codé par l'acide nucléique variant par rapport aux peptides codés par l'acide nucléique de référence. En outre, des modifications de nucléotides dans les régions codantes peuvent produire des substitutions, conservatives ou non conservatives dans la séquence d'aminoacides.

De préférence, les acides nucléiques variants selon l'invention codent des polypeptides qui conservent sensiblement la même fonction ou activité biologique que le polypeptide de l'acide nucléique de référence ou encore la capacité à être reconnus par des anticorps dirigés contre les polypeptides codés par l'acide nucléique initial.

Certains acides nucléiques variants coderont ainsi des formes mutées des polypeptides dont l'étude systématique permettra de déduire des relations structure activité des protéines en question.

Par " variant " d'un polypeptide selon l'invention, on entendra principalement un polypeptide dont la séquence d'acides aminés contient une ou plusieurs substitutions, additions ou délétions d'au moins un résidu d'acide aminé, par rapport à la séquence d'acides aminés du polypeptide de référence, étant entendu que les substitutions d'aminoacides peuvent être indifféremment conservatives ou non conservatives.

De préférence, les polypeptides variants selon l'invention conservent sensiblement la même fonction ou activité biologique que le polypeptide de référence ou encore la capacité à être reconnus par des anticorps dirigés contre les polypeptides initiaux.

Par polypeptide ayant " une activité similaire " à un polypeptide de référence au sens de l'invention, on entend un polypeptide ayant une activité biologique proche, mais pas nécéssairement identique, de celle du polypeptide de référence tel que mesuré dans un essai biologique convenable à la mesure de l'activité biologique du polypeptide de référence.

### Description détaillée de l'invention

La présente invention a plus particulièrement pour objet, de nouveaux gènes de la voie de biosynthèse des spiramycines et de nouveaux polypeptides impliqués dans cette biosynthèse tels que présentés dans la description détaillée ci-dessous.

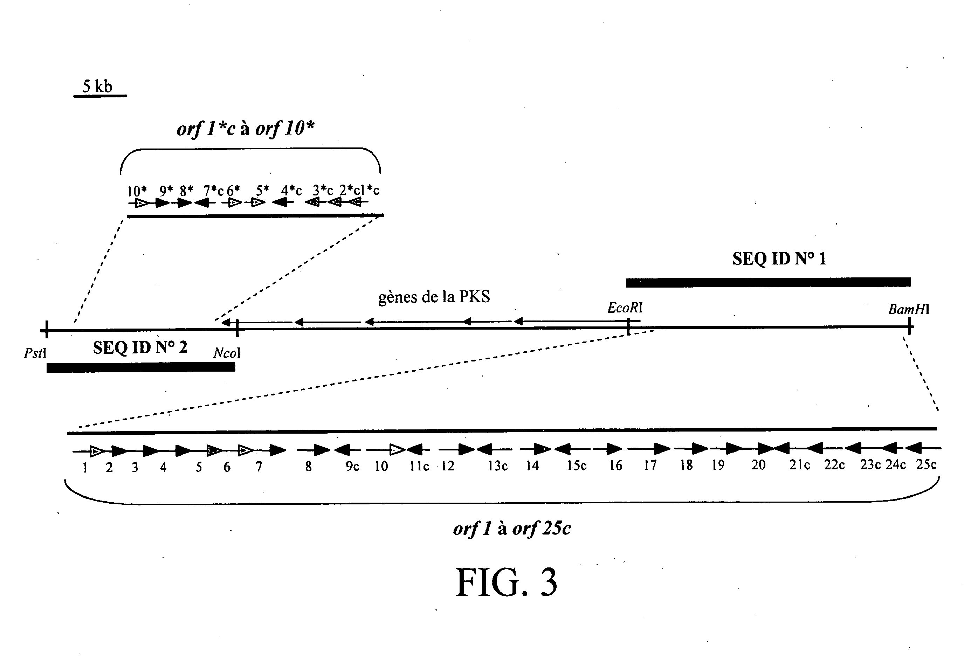

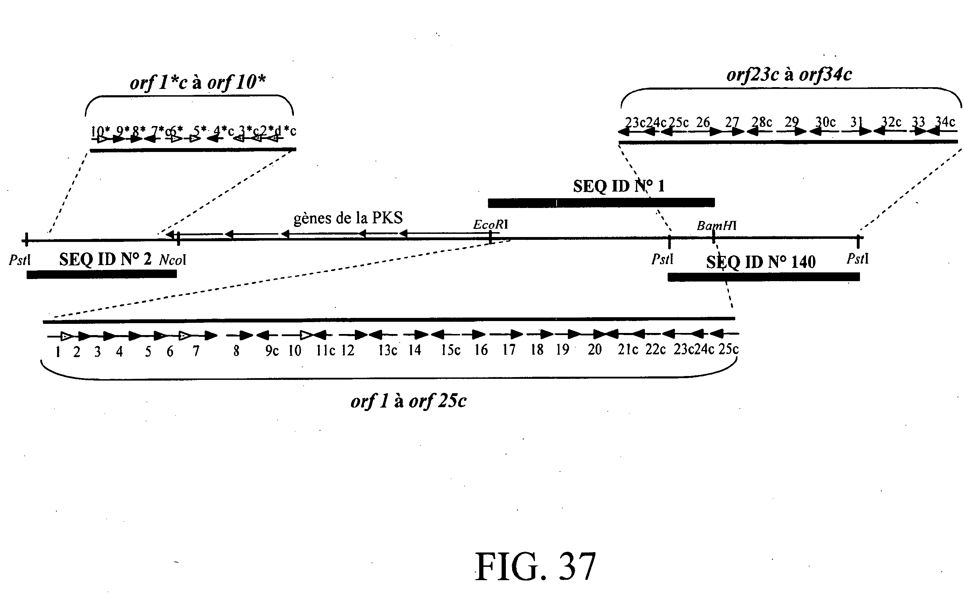

Les gènes de la voie de biosynthèse ont été clonés et la séquence d'ADN de ces gènes a été déterminée. Les séquences obtenues ont été analysées grâce au programme FramePlot (Ishikawa J & Hotta K. 1999). On a identifié, parmi les phases ouvertes de lecture, les phases ouvertes de lecture présentant un usage des codons typiques de *Streptomyces.* Cette analyse a montré que cette région comporte 44 ORFs, localisées de part et d'autre de cinq gènes codant l'enzyme « polyketide synthase » (PKS), et présentant un usage des codons typique de *Streptomyces.* De part et d'autre de ces cinq gènes codant la PKS, respectivement 10 et 34 ORFs ont été identifiées en aval et en amont (l'aval et l'amont étant définis par l'orientation des 5 gènes de PKS tous orientés dans le même sens) (cf. figure 3 et 37). Ainsi, les 34 phases ouvertes de lecture de ce type, occupant une région d'environ 41,7 kb (cf. SEQ ID N° 1 présentant une première région de 31 kb contenant 25 ORFs et SEQ ID N° 140 présentant une région d'environ 12,1 kb dont 1,4 kb chevauchent la sequence précédente (SEQ ID N° 1) et environ 10,7 kb correspondent à la suite de la séquence, cette dernière partie d'environ 10,7 kb contenant 9 ORFs supplémentaires (dont un ORF de séquence partielle), cf. également figure 3 et 37 et ci-dessous), ont été identifiées en amont des 5 gènes codant la PKS et 10 occupant une région d'environ 11,1 kb (SEQ ID N° 2 et figure 3), ont été identifiées en aval des gènes de la PKS. Les 10 gènes situés en aval des 5 gènes PKS ont ainsi été nommés *orf1*c, orf2*c, orf3*c, orf4*c, orf5*, orf6*, orf7*c, orf8*, orf9*, orf10** (SEQ ID N° 3, 5, 7, 9, 11, 13, 15, 17, 19 et 21). Le « c » ajouté dans le nom du gène signifiant pour l'ORF en question que la séquence codante est dans l'orientation inverse (le brin codant est donc le brin complémentaire de la séquence donnée en SEQ ID N° 2 pour ces gènes). En utilisant la même nomenclature, les 34 ORFs en amont des gènes de la PKS ont été nommés *orf1, orf2, orf3, orf4, orf5, orf6, orf7, orf8, orf9c, orf10, orf11c, orf12, orf13c, orf14, orf15c, orf16, orf17, orf18, orf19, orf20, orf21c, orf22c, orf23c, orf24c, orf25c, orf26, orf27, orf28c, orf29, orf30c, orf31, orf32c, orf33 et orf34c* (SEQ ID N° 23, 25, 28, 30, 34, 36, 40, 43, 45, 47, 49, 53, 60, 62, 64, 66, 68, 70, 72, 74, 76, 78, 80, 82, 84, 107, 109, 111, 113, 115, 118, 120, 141, 143, 145, 147 et 149) (cf. figure 3 et 37).

Les séquences protéiques déduites de ces phases ouvertes de lecture ont été comparées avec celles présentes dans différentes bases de données grâce à différents programmes : BLAST *(Altschul et al.,* 1990) *(Altschul et al.,* 1997), CD-search, COGs (Cluster of Orthologous Groups) (ces trois programmes sont accessibles notamment auprès du National Center for Biotechnology Information (NCBI) (Bethesda, Maryland, USA)), FASTA ((Pearson W. R & D. J. Lipman, 1988) et (Pearson W. R., 1990), BEAUTY (Worley K. C.. *et al.,* 1995)), (ces deux programmes sont accessibles notamment auprès du centre de ressources INFOBIOGEN, Evry, France). Ces comparaisons ont permis de formuler des hypothèses sur la fonction des produits de ces gènes et d'identifier ceux susceptibles d'être impliqués dans la biosynthèse des spiramycines.

### Gènes situés en aval des gènes codant les PKS

Une représentation schématique de l'organisation de la région est présentée en figure 3. Ainsi qu'il sera démontré ci-dessous, sur les 10 gènes identifiés en aval des gènes codant les PKS 9 semblent impliqués dans la biosynthèse ou la résistance aux spiramycines. II s'agit des 9 gènes suivants: *orf1*c, orf2*c, orf3*c, orf4*c, orf5*, orf6*, orf7*c, orf8** et *orf9*.*

Dans le tableau 1 suivant sont présentées les références à la séquence en ADN et en acides aminés des 10 gènes identifiés en aval des 5 gènes PKS.

**Tableau 1**

| Gène | Position dans la séquence SEQ ID N 2 | Séquence ADN | Séquences polypeptidiques |
|---|---|---|---|
| *orf1***c* | 10882 à 10172 | SEQ ID N 3 | SEQ ID N 4 |
| *orf2***c* | 10052 à 8781 | SEQ ID N 5 | SEQ ID N 6 |
| *orf3***c* | 8741 à 7476 | SEQ ID N 7 | SEQ ID N 8 |
| *orf4*c* | 7459 à 6100 | SEQ ID N 9 | SEQ ID N 10 |
| *orf5** | 5302 à 5976 | SEQ ID N 11 | SEQ ID N 12 |
| *orf6** | 4061 à 5305 | SEQ ID N 13 | SEQ ID N 14 |
| *orf7*c* | 3665 à 2817 | SEQ ID N 15 | SEQ ID N 16 |
| *orf8** | 1925 à 2755 | SEQ ID N 17 | SEQ ID N 18 |
| *orf9** | 1007 à 1888 | SEQ ID N 19 | SEQ ID N 20 |
| *orf10** | 710 à 937 | SEQ ID N 21 | SEQ ID N 22 |

Le « c » ajouté dans le nom du gène indique que la séquence codante est dans l'orientation inverse (le brin codant est donc le brin complémentaire de la séquence donnée en SEQ ID N° 2 pour ces gènes).

Dans le but de déterminer la fonction des polypeptides identifiés, trois types d'expériences ont été menés : la comparaison des séquences identifiées avec des séquences de fonctions connues, des expériences d'inactivation de gènes, conduisant à la construction de souches mutantes et des analyses de la production en spiramycines et en intermédiaires de biosynthèse des spiramycines par ces souches mutantes.

Les séquences protéiques déduites de ces phases ouvertes de lecture ont tout d'abord été comparées avec celles présentes dans différentes bases de données grâce à différents programmes : BLAST (Altschul *et al..,* 1990) *(Altschul et al.,* 1997), CD-search, COGs (Cluster of Orthologous Groups), FASTA ((Pearson W. R & D. J. Lipman, 1988) et (Pearson W. R., 1990), BEAUTY (Worley K. C.. *et ad.,* 1995)). Ces comparaisons ont permis de formuler des hypothèses sur la fonction des produits de ces gènes et d'identifier ceux susceptibles d'être impliqués dans la biosynthèse de spiramycine. Le tableau 2 montre les protéines présentant une forte similitude avec les produits 10 gènes situés en aval des 5 gènes PKS.

**Tableau 2**

| Produit de gène | Protéine présentant une similitude significative | Numéro d'accès GenBank | Score BLAST* | Fonction rapportée |
|---|---|---|---|---|
| *orf1*c* | TylMI(orf3*) (*S.fradiae*) | CAA57473 | 287 | N-méthyltransférase |
| *orf2*c* | dnrQ gene product (*Streptomyces peucetius*) | AAD 15266 | 153 | Inconnue |
| *orf3***c* | TylMII(orf2*) (*S*. *fradiae*) | CAA57472 | 448 | Glycosyltransféras e |
| *orf4*c* | Crotonyl-CoA réductase (*S. coelicolor*) | NP_630556 | 772 | Crotonyl-CoA réductase |
| *orf5** | MdmC (*S*. *mycarofaciens*) | B42719 | 355 | O-méthyltransférase |
| *orf6** | 3-O-acyltransférase (*S*. *mycarofaciens*) | Q00718 | 494 | Acyltransférase |
| *orf7***c* | MdmA (*S*. *mycarofaciens*) | A60725 | 380 | Protéine impliquée dans la résistance à la midécamycine |
| *orf8** | ABC-transporteur (*S*. *griseus*) | CAC22119 | 191 | ABC-transporteur |
| *orf9** | ABC-transporteur (*S*. *griseus*) | CAC22118 | 269 | ABC-transporteur |
| *orf10** | Putative small conserved hypothetical protein (*S*. *coelicolor*) | NP_627432 | 109 | Inconnue |

| | | | | |
|---|---|---|---|---|
| * une plus grande similitude de séquence est associée à un score BLAST plus élevé (Altschul *et al.,* 1990). | | | | |

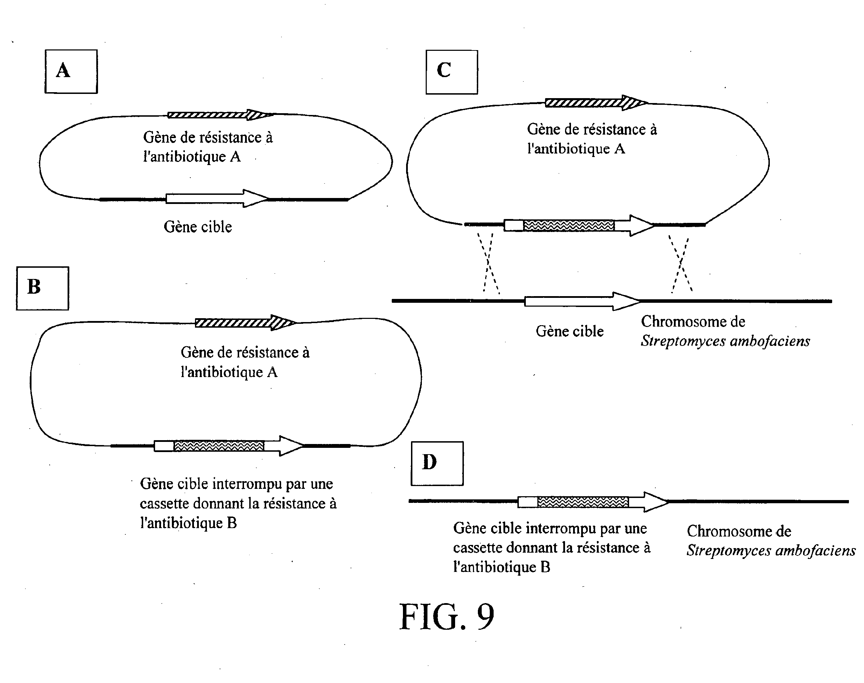

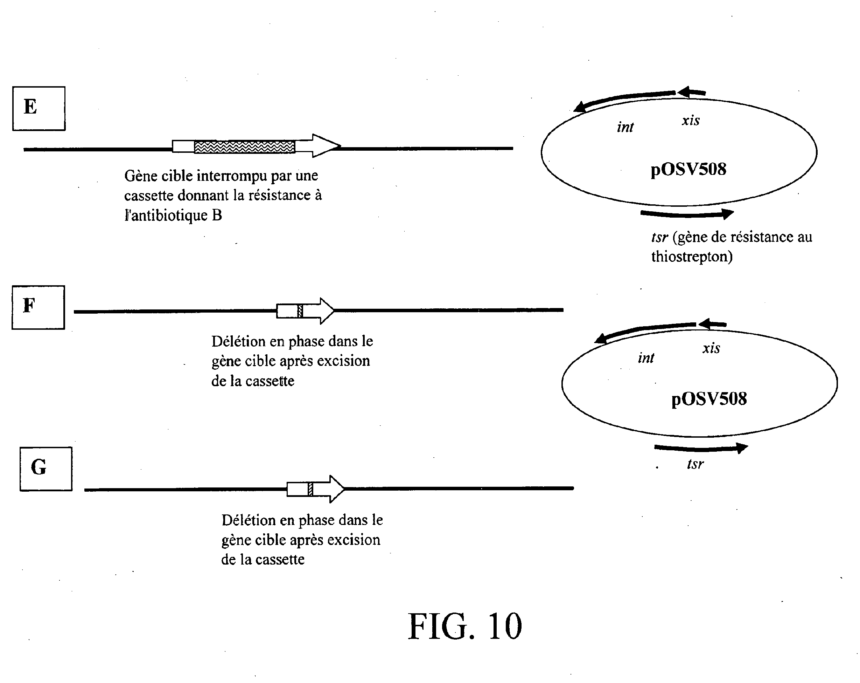

Des expériences d'inactivation de gènes ont été réalisées pour confirmer ces résultats. Les méthodes utilisées consistent à effectuer un remplacement de gène. Le gène cible à interrompre est remplacé par une copie de ce gène interrompue par une cassette conférant la résistance à un antibiotique (par exemple l'apramycine, la généticine ou l'hygromycine). Les cassettes utilisées sont bordées de part et d'autre par des codons de terminaison de la traduction dans toutes les phases de lecture et par des terminateurs de transcription actifs chez *Streptomyces.* L'insertion de la cassette dans le gène cible peut s'accompagner ou non d'une délétion dans ce gène cible. La taille des régions flanquant la cassette peut aller de quelques centaines à plusieurs milliers de paires de bases. Un deuxième type de cassettes peut être utilisé pour l'inactivation de gènes : des cassettes dites «cassettes excisables ». Ces cassettes présentent l'avantage de pouvoir être excisées chez *Streptomyces* par un événement de recombinaison de site spécifique après avoir été introduites dans le génome de *S. ambofaciens.* Le but est d'inactiver certains gènes dans des souches de *Streptomyces* sans laisser dans la souche finale de marqueurs de sélection ou de grandes séquences d'ADN n'appartenant pas à la souche. Après excision il subsiste uniquement une courte séquence d'une trentaine de paires de bases (appelé site « cicatriciel ») dans le génome de la souche (cf. figure 10). La mise en oeuvre de ce système consiste, dans un premier temps, au remplacement de la copie sauvage du gène cible (grâce à deux événements de recombinaison homologue, cf. figure 9) par une construction dans laquelle une cassette excisable a été insérée dans ce gène cible. L'insertion de cette cassette est accompagnée d'une délétion dans le gène cible (cf. figure 9). Dans un deuxième temps, l'excision de la cassette excisable du génome de la souche est provoquée. La cassette excisable fonctionne grâce à un système de recombinaison site spécifique et a pour avantage de permettre l'obtention de mutants de *Streptomyces* ne portant finalement pas de gène de résistance. On s'affranchit également d'éventuels effets polaires sur l'expression des gènes situés en aval du ou des gènes inactivés (cf. figure 10). Les souches ainsi construites ont été testées pour leur production en spiramycines.

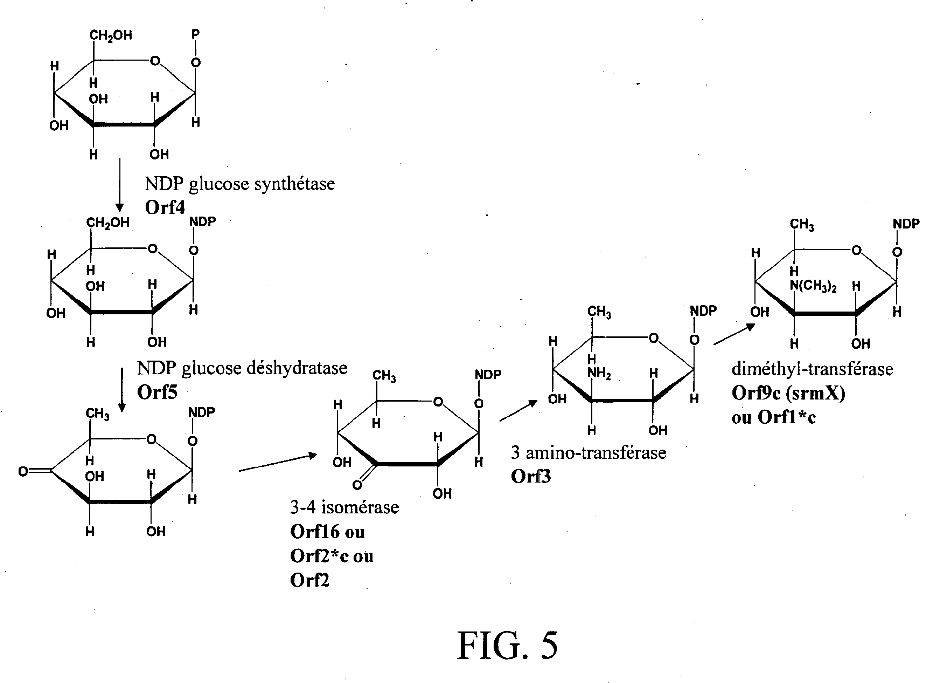

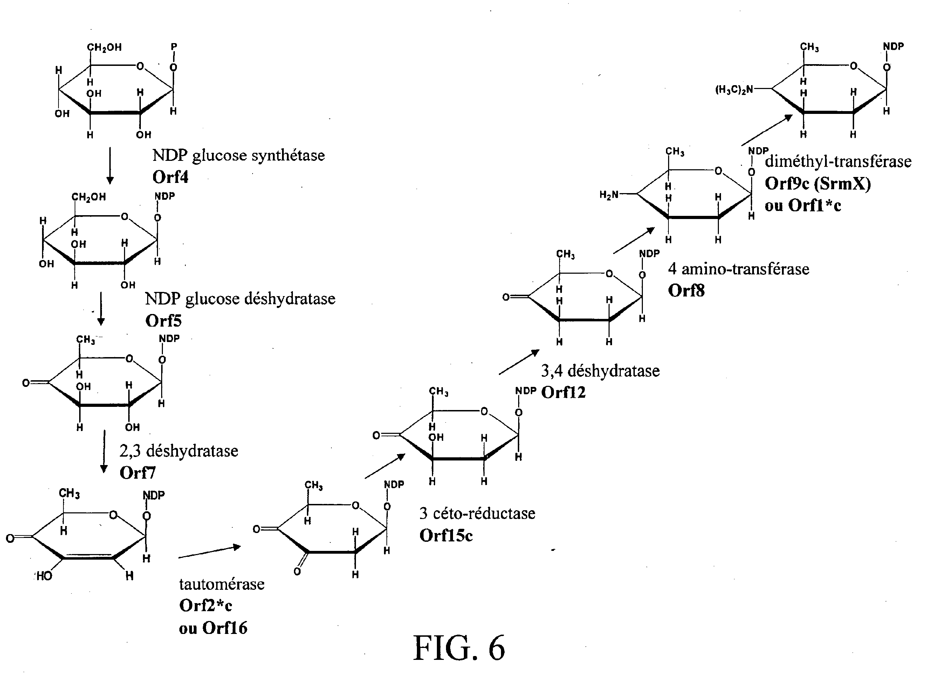

L'inactivation des gènes *orf1*c, orf2*c, orf3*c* et *orf4*c* n'a pas été effectuée car les expériences de comparaison de séquence ont permis de déterminer que ces gènes avaient une similitude relativement importante avec des gènes impliqués dans la biosynthèse d'un antibiotique relativement proche. Ainsi, le gène *orf1*c* code une protéine présentant une identité de 66% (déterminée grâce au programme BLAST) avec la protéine codée par gène *tylMI* qui code une N-méthyltransférase impliquée dans la biosynthèse de tylosine et catalysant la 3 N-méthylation durant la production du mycaminose chez *Streptomyces fradiae* (Gandecha,A.R. *et al.,* 1997 ; Numéro d'accès GenBank : CAA57473 ; Score BLAST : 287). Cette similitude avec une protéine impliquée dans la voie de biosynthèse d'un autre antibiotique relativement proche et plus particulièrement dans la biosynthèse du mycaminose suggère que le gène *orf1*c* code une N-methyltransférase responsable d'une N-méthylation lors de la biosynthèse de la forosamine ou du mycaminose (cf. figure 5 et 6). Cette hypothèse est étayée par le fait que la protéine codée par le gène *orf1***c* présente une forte similitude avec d'autres protéines de fonction similaire chez d'autres organismes (cf. tableau 3).

**Tableau 3**

| Protéine présentant une similitude significative | Numéro d'accès GenBank | Score BLAST* | Fonction rapportée |
|---|---|---|---|
| méthyltransferase (*S. antibioticus*) | CAA05643 | 277 | méthyltransférase |
| N,N-diméthyltransferase (*S. venezuelae*) | AAC68678 | 268 | N,N-diméthyltransférase |
| probable N-méthylase snogX (*S*. *nogalater*) | T46679 | 243 | N-méthyltransférase |

| | | | |
|---|---|---|---|
| * une plus grande similitude de séquence est associée à un score BLAST plus élevé (Altschul *et al..,* 1990). | | | |

Le gène *orf2*c* code une protéine présentant une relative forte similitude (35% d'identité) avec une protéine codée par le gène *tylMIII* codant une NDP hexose 3,4 isomérase impliquée dans la biosynthèse de la tylosine chez *Streptomyces fradiae* (Gandecha,A.R., *et al.,* 1997 ; Numéro d'accès GenBank : CAA57471 ; Score BMAST : 130). Cette similitude avec une protéine impliquée dans la voie de biosynthèse d'un autre antibiotique proche et plus particulièrement dans la biosynthèse du mycaminose suggère fortement que le gène *orf*2c* code une NDP hexose 3,4 isomérase responsable d'isomérisation lors de la biosynthèse d'un des sucres de la spiramycine, éventuellement le mycaminose (cf. figure 5 et 6).

Le gène *orf3*c* code une protéine présentant une relative forte similitude (59% d'identité) avec une protéine codée par le gène *tylMII* codant une glycosyltransférase impliquée dans la biosynthèse de la tylosine chez *Streptomyces fradiae* (Gandecha,A.R. *et al.,* 1997 ; Numéro d"accès GenBank : CAA57472 ; Score BLAST : 448). Cette similitude avec une protéine impliquée dans la voie de biosynthèse d'un autre antibiotique proche suggère fortement que le gène *orf*3c* code une glycosyltransférase. Cette hypothèse est étayée par le fait que la protéine codée par le gène *orf3*c* présente une forte similitude avec d'autres protéines de fonction similaire chez d'autres organismes (cf. tableau 4).

**Tableau 4**

| Protéine présentant une similitude significative | Numéro d'accès GenBank | Score BLAST* | Fonction rapportée |
|---|---|---|---|
| Glycosyl transférase (*S*. *venezuelae*) | AAC68677 | 426 | Glycosyltransférase |
| Glycosyltransférase (*S*. *antibioticus*) | CAA05642 | 425 | Glycosyltransférase |
| Glycosyltransférase (*Saccharopolyspora erythraea*) | CAA74710 | 395 | Glycosyltransférase |
| Glycosyltransférase (*S. antibioticus*) | CAA05641 | 394 | Glycosyltransférase |

| | | | |
|---|---|---|---|
| * une plus grande similitude de séquence est associée à un score BLAST plus élevé (Altschul *et al.,* 1990). | | | |

Le gène *orf4*c* code une protéine présentant une relative forte similitude avec plusieurs crotonyl-CoA réductases. Notamment, la protéine codée par *orf4*c* possède une similitude importante avec une crotonyl CoA réductase de chez *Streptomyces coelicolor* (Redenbach,M *et al.,* 1996 ; Numéro d'accès GenBank : NP_630556 ; Score BLAST : 772). Cette similitude avec une protéine impliquée dans la voie de biosynthèse d'un autre antibiotique proche suggère fortement que le gène *orf4*c* code également une crotonyl-CoA réductase. Cette hypothèse est étayée par le fait que la protéine codée par le gène *orf4*c* présente une forte similitude avec d'autres protéines de fonction similaire chez d'autres organismes (cf. tableau 5).

**Tableau 5**

| Protéine présentant une similitude significative | Numéro d'accès GenBank | Score BLAST* | Fonction rapportée |
|---|---|---|---|
| trans-2-énoyl-CoA réductase (EC1.3.1.38) (*S. collinus*) | S72400 | 764 | trans-2-énoyl-CoA réductase |
| Crotonyl CoA réductase (*S.fradiae*) | CAA57474 | 757 | Crotonyl CoA réductase |
| Crotonyl-CoA réductase (*S*. *cinnamonensis*) | AAD53915 | 747 | Crotonyl-CoA réductase |

| | | | |
|---|---|---|---|
| * une plus grande similitude de séquence est associée à un score BLAST plus élevé (Altschul *et al.,* 1990). | | | |

Le gène *orf6** présente une certaine similitude avec le gène *mdmB* présent chez *Streptomyces mycarofaciens* (Hara et Hutchisnson, 1992 ; numéro d'accès GenBank : A42719 ; Score BLAST : 489) producteur d'antibiotique macrolide. Chez ce producteur, le gène est impliqué dans l'acylation du cycle lactone. Le gène *orf6** coderait donc une acyltransférase. Cette hypothèse est étayée par le fait que la protéine codée par le gène *orf6** présente une forte similitude avec d'autres protéines de fonction similaire chez d'autres organismes (cf. tableau 6).

**Tableau 6**

| Protéine présentant une similitude significative | Numéro d'accès GenBank | Score BLAST* | Fonction rapportée |
|---|---|---|---|
| *AcyA* (*Streptomyces thermotolerans*) | J4001 | 450 | macrolide 3-O-acyltransférase |
| Midécamycine 4"-O-propionyl transférase (*S*. *mycarofaciens*) | BAA09815 | 234 | Midécamycine 4"-O-propionyl transférase |
| Mycarose O-acyltransférase (*Micromonospora megalomicea subsp. Nigra*) | AAG13909 | 189 | Mycarose O-acyltransférase |

| | | | |
|---|---|---|---|
| * une plus grande similitude de séquence est associée à un score BLAST plus élevé (Altschul *et al.,* 1990). | | | |

L'inactivation du gène *orf6** a été réalisée par une délétion/insertion en phase et il a été montré que la souche résultante ne produit plus de spiramycine II et III mais uniquement de la spiramycine I (cf figure 1). Ceci confirme que le gène *orf6** est bien impliqué dans la synthèse de spiramycine II et III. L'enzyme codée par ce gène est responsable de la formation de spiramycine II et III par fixation d'un groupement acétyl ou butyryl sur le carbone 3. Les souches n'exprimant plus la protéine codée par le gène *orf6** sont particulièrement intéressantes puisqu'elles ne produisent plus de spiramycine II et III mais seulement de la spiramycine I. Comme il a été précisé ci-dessus, l'activité antibiotique de la spiramycine I est nettement supérieure à celle des spiramycines II et III (Liu *et al.,* 1999).

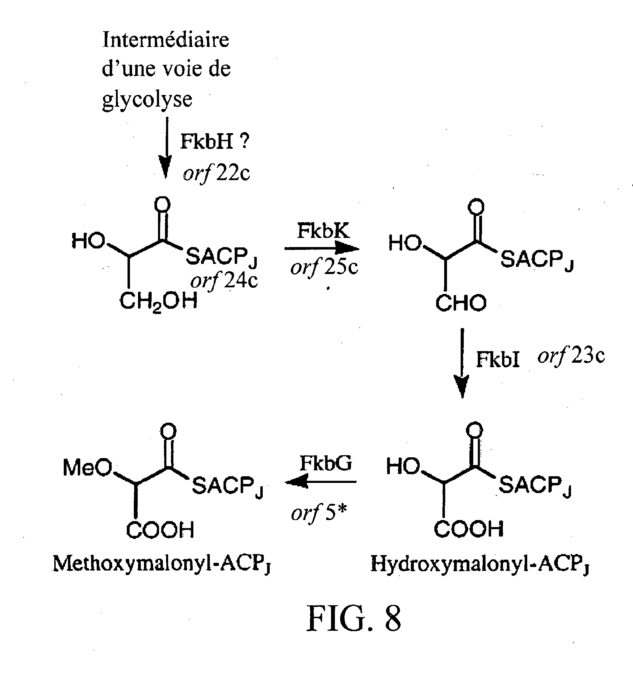

Le gène *orf5** code une protéine présentant une relative forte similitude avec plusieurs O-méthyltransférase. Notamment, la protéine codée par *orf5** possède une similitude importante avec une O-méthyltransférase (EC 2.1.1.-) MdmC de *Streptomyces mycarofaciens* (Hara & Hutchinson, 1992 ; Numéro d'accès GenBank :B42719; Score BLAST : 355). Cette similitude avec une protéine impliquée dans la voie de biosynthèse d'un autre antibiotique suggère fortement que le gène *orf5** code également une O-méthyltransférase. Le gène *orf5** serait impliqué dans la formation de précurseurs incorporés dans le cycle lactone. En effet, d'après les comparaisons de séquences, le produit du gène *orf5** est également relativement proche de FkbG qui est responsable de la méthylation de l'hydroxymalonyl-ACP d'après (Wu *et al.,* 2000 ; Hoffmeister *et al.,* 2000 ; Numéro d'accès GenBank : AAF86386 ; Score BLAST : 247) (cf. figure 8). Cette hypothèse est étayée par le fait que la protéine codée par le gène *orf5** présente une forte similitude avec d'autres protéines de fonction similaire chez d'autres organismes (cf. tableau 7).

**Tableau 7**

| Protéine présentant une similitude significative | Numéro d'accès GenBank | Score BLAST* | Fonction rapportée |
|---|---|---|---|
| Probable O-méthyltransferase (EC 2.1.1.-) safC (*Myxococcus xanthus*) | T18553 | 223 | O-méthyltransférase |
| 4-O-méthyltransferase (EC 2.1.1.-)-(*Streptomyces sp.*) | JC4004 | 222 | O-méthyltransférase |

| | | | |
|---|---|---|---|
| * une plus grande similitude de séquence est associée à un score BLAST plus élevé (Altschul *et al.,* 1990). | | | |

Grâce à l'effet polaire de l'insertion d'une cassette non excisée dans le gène *orf 6**, il a pu être déterminé que le gène *orf5** est essentiel à la voie de biosynthèse des spiramycines. En effet, l'insertion de la cassette excisable dans la partie codante du gène *orf6** entraîne un arrêt total de la production en spiramycines. Cependant, une fois que la cassette insérée a été excisée (et donc lorsque seul le gène *orf6** est inactivé, cf exemples 14 et 15), on retrouve une production en spiramycine I. Ceci montre que le gène *orf5** est essentiel à la voie de biosynthèse des spiramycines puisque son inactivation entraîne un arrêt total de la production en spiramycines.

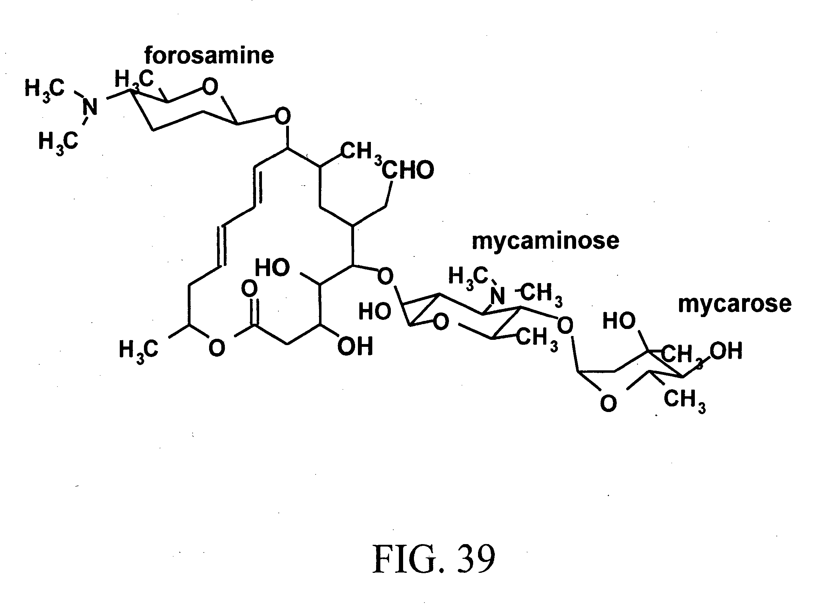

Le gène *orf5** code une protéine présentant une relative forte similitude avec plusieurs O-méthyltransférase. Le gène *orf5** serait une O-méthyl transférase impliquée soit directement dans la synthèse du platénolide, soit dans la synthèse d'un précurseur méthylé (méthoxymalonyl, voir figure 8) incorporé dans le platénolide par la PKS. Pour vérifier cette hypothèse, des expériences d'analyse CL/SM et de RMN ont été conduites sur une souche de *S*. *ambofaciens* de génotype : *orf6*::att1Ωhyg*+. Dans cette souche le gène *orf5** n'est pas exprimé à cause de l'effet polaire de l'insertion, dans le gène *orf6*,* de la cassette qui contient des terminateurs de transcription (cf. exemple 27). Il a été montré que cette souche produit une molécule dont le spectre UV a une allure similaire à celui de la spiramycine I mais le spectre de masse montre un ion moléculaire à 829. La différence de masse de 14 par rapport à la masse de la spiramycine peut s'expliquer par l'absence de méthyl sur l'oxygène portée par le carbone n°4 du cycle lactone (la strucutre de ce composé est présenté en figure 39). Les résultats obtenus par RMN sont compatibles avec cette hypothèse. La présence d'un composé à 829 permet de valider l'hypothèse du rôle de *orf5** dans la biosynthèse des spiramycines. En outre, le produit correspondant à la spiramycine sans le groupement méthyle présente une très faible activité microbologique (plus faible d'un facteur 10) par rapport à la spiramycine non modifiée, lorsque testée sur le microorganisme *Micrococcus luteus.*

Le gène *orf7*c* code une protéine présentant une relative forte similitude avec une protéine codée par le gène *mdmA* de *Streptomyces mycarofaciens,* ce dernier codant une protéine impliquée dans la résistance à la midécamycine chez cet organisme producteur (Hara *et al.,* 1990 ; Numéro d'accès GenBank : A60725 ; Score BLAST : 380). Cette similitude avec une protéine impliquée dans la voie de biosynthèse d'un autre antibiotique suggère fortement que le gène *orf7*c* code également une protéine impliquée dans la résistance à la spiramycine. Plus particulièrement, l'enzyme codée par le gène *orf7*c* possède une activité méthyltransférase et est impliquée dans la résistance à la spiramycine chez *Streptomyces ambofaciens.* Il a été démontré que ce gène confère une résistance de type MLS I, résistance dont on sait qu'elle est dûe à la mono-méthylation en position A2058 de l'ARN ribosomal 23S (Pernodet *et al.,* 1996). Cette hypothèse est étayée par le fait que la protéine codée par le gène *orf7*c* présente une forte similitude avec d'autres protéines de fonction similaire chez d'autres organismes (cf. tableau 8).

**Tableau 8**

| Protéine présentant une similitude significative | Numéro d'accès GenBank | Score BLAST* | Fonction rapportée |
|---|---|---|---|
| macrolide-lincosamide-streptogramin B résistance déterminant (*S*. *fradiae*) | JC5319 | 238 | 23S rRNA méthyltransférase |
| 23S ribosomal RNA methyltransferase ErmML (*Micrococcus luteus*) | AAL68827 | 119 | 23S rRNA méthyltransférase |

| | | | |
|---|---|---|---|
| * une plus grande similitude de séquence est associée à un score BLAST plus élevé (Altschul *et al..,* 1990). | | | |

Le gène *orf8** code une protéine présentant une relative forte similitude avec une protéine de type transporteur ABC chez *Streptomyces griseus* (Campelo, 2002, Numéro d'accés GenBank: CAC22119; Score BLAST: 191). Cette similitude avec une protéine de type transporteur ABC suggère fortement que le gène *orf8** code également une protéine de type transporteur ABC pouvant être impliquée dans la résistance à la spiramycine. Cette hypothèse est étayée par le fait que la protéine codée par le gène *orf8** présente une forte similitude avec d'autres protéines de fonction similaire chez d'autres organismes (cf. tableau 9).

**Tableau 9**

| Protéine présentant une similitude significative | Numéro d'accès GenBank | Score BLAST* | Fonction rapportée |
|---|---|---|---|
| AcrW (*Streptomyces galilaeus*) | BAB72060 | 94 | Transporteur ABC |
| Daunorubicin resistance transmembrane protéine (*Streptomyces peucetius*) | P32011 | 89 | Résistance à la daunorubicine |
| Probable ABC-transporter, transmembrane component. (*Streptomyces coelicolor*) | NP_626506 | 89 | Transporteur ABC |

| | | | |
|---|---|---|---|
| * une plus grande similitude de séquence est associée à un score BLAST plus élevé (Altschul *et al.,* 1990). | | | |

Le gène *orf9** code une protéine présentant une relative forte similitude avec une protéine de type transporteur ABC chez *Streptomyces griseus* (Campelo, 2002, Numéro d'accés GenBank: CAC22118; Score BLAST: 269). Cette similitude avec une protéine de type transporteur ABC suggère fortement que le gène *orf9** code également une protéine de type transporteur ABC pouvant être impliquée dans la résistance à la spiramycine. Cette hypothèse est étayée par le fait que la protéine codée par le gène *orf8** présente une forte similitude avec d'autres protéines de fonction similaire chez d'autres organismes (cf. tableau 10).

**Tableau 10**

| Protéine présentant une similitude significative | Numéro d'accès GenBank | Score BLAST* | Fonction rapportée |
|---|---|---|---|
| Probable ABC-type transport protein, ATP-binding component (*S. coelicolor*) | NP_626505 | 231 | Transporteur ABC |
| Putative ABC transporter ATP-binding | NP_627624 | 228 | Transporteur |
| component (*Streptomyces Coelicolor*) | | | ABC |

| | | | |
|---|---|---|---|
| * une plus grande similitude de séquence est associée à un score BLAST plus élevé (Altschul *et al.,* 1990). | | | |

Le gène *orf10** code une protéine présentant une relative forte similitude avec une protéine de fonction inconnue. Cependant des gènes similaires à *orf10** sont retrouvés au milieu de plusieurs groupes de gènes impliqués dans la biosynthèse d'antibiotiques. Ainsi un gène proche de *orf10** est retrouvé chez *S. coelicolor* (Redenbach *et al.,* 1996, numéro d'accès GenBank : NP_627432, score BLAST : 109). Un gène proche (*CouY*) est également retrouvé chez *S. rishiriensis* (Wang *et al..,* 2000, numéro d'accès GenBank : AAG29779, score BLAST 97).

### Gènes situés en amont des gènes codant les PKS

Dans la séquence d'ADN située en amont des gènes codant les PKS (l'aval et l'amont étant défini par l'orientation des 5 gènes de PKS tous orientés dans le même sens) (cf. figure 3), 34 ORFs ont été identifiées (cf. ci-dessus). Ainsi, les 34 phases ouvertes de lecture de ce type, occupant une région d'environ 41,7 kb (cf. SEQ ID N° 1 présentant une première région de 31 kb contenant 25 ORFs et SEQ ID N° 140 présentant une région d'environ 12,1 kb dont 1,4 kb chevauchent la sequence précédente (SEQ ID N° 1) et environ 10,7 kb correspondent à la suite de la séquence), cette dernière partie d'environ 10,7 kb contenant 9 ORFs supplémentaires (dont un ORF de séquence partielle), cf. également figure 3 et 37 et ci-dessous). Une représentation schématique de l'organisation de la région est présentée en figure 3 et 37. Les 34 gènes identifiés ont été nommés : *orf1, orf2, orf3, orf4, orf5, orf6, orf7, orf8, orf9c, orf10, orf11c, orf12, orf13c, orf14, orf15c, orf1l6, orf17, orf18, orf19, orf20, orf21c, orf22c, orf23c***,** *orf24c, orf25c, orf26, orf27, orf28c, orf29, orf30c, orf31, orf32c, orf33* et *orf34c*.

Dans le tableau 11 suivant sont présentées les références à la séquence en ADN et en acides aminés des 34 gènes identifiés en amont des 5 gènes PKS.

**Tableau 11**

| **Gène¹** | **Position dans la séquence SEQ ID N 1** | **Séquence ADN** | **Séquence(s) polypeptidique(s)²** |
|---|---|---|---|
| *orf1* | 658 à 1869 | SEQ ID N 23 | SEQ ID N 24 |
| *orf2* | 1866 à 2405 | SEQ ID N 25 | SEQ ID N 26 et 27 |
| *orf3* | 2402 à 3568 | SEQ ID N 28 | SEQ ID N 29 |
| *orf4* | 3565 à 4473 | SEQ ID N 30 | SEQ ID N 31, 32 et 33 |
| *orf5* | 4457 à 5494 | SEQ ID N 34 | SEQ ID N 35 |
| *orf6* | 5491 à 6294 | SEQ ID N 36 | SEQ ID N 37, 38 et 39 |
| *orf7* | 6296 à 7705 | SEQ ID N 40 | SEQ ID N 41 et 42 |
| *orf8* | 8011 à 9258 | SEQ ID N 43 | SEQ ID N 44 |
| *orf9c* | 10081 à 9362 | SEQ ID N 45 | SEQ ID N 46 |
| *orf10* | 10656 à 12623 | SEQ ID N 47 | SEQ ID N 48 |
| *orf11c* | 14482 à 12734 | SEQ ID N 49 | SEQ ID N 50, 51 et 52 |
| *orf12* | 14601 à 16031 | SEQ ID N 53 | SEQ ID N 54, 55, 56, 57, 58 et 59 |
| *orf13c* | 17489 à 16092 | SEQ ID N 60 | SEQ ID N 61 |
| *orf14* | 17809 à 18852 | SEQ ID N 62 | SEQ ID N 63 |
| *orf15c* | 20001 à 18961 | SEQ ID N 64 | SEQ ID N 65 |
| *orf16* | 20314 à 21552 | SEQ ID N 66 | SEQ ID N 67 |
| *orf17* | 21609 à 22879 | SEQ ID N 68 | SEQ ID N 69 |
| *orf18* | 22997 à 24175 | SEQ ID N 70 | SEQ ID N 71 |
| *orf19* | 24177 à 25169 | SEQ ID N 72 | SEQ ID N 73 |
| *orf20* | 25166 à 26173 | SEQ ID N 74 | SEQ ID N 75 |
| *orf21c* | 27448 à 26216 | SEQ ID N 76 | SEQ ID N 77 |
| *orf22c* | 28560 à 27445 | SEQ ID N 78 | SEQ ID N 79 |
| *orf23c* | 29770 à 28649 | SEQ ID N 80 | SEQ ID N 81 |
| *orf24c* | 30074 à 29763 | SEQ ID N 82 | SEQ ID N 83 |
| *orf25c* | 30937 à 30071 | SEQ ID N 84 | SEQ ID N 85 |

| **Gène¹** | **Position dans la séquence SEQ ID NO : 140** | **Séquence ADN** | **Séquence(s) polypeptidique(s)²** |
|---|---|---|---|
| *orf26* | 1647 à 2864 | SEQ ID N 107 | SEQ ID N 108 |
| *orf27* | 2914 à 3534 | SEQ ID N 109 | SEQ ID N 110 |
| *orf28c* | 4967 à 3804 | SEQ ID N° 141 | SEQ ID N° 142 |
| *orf29* | 5656 à 6663 | SEQ ID N 113 | SEQ ID N 114 |
| *orf30c* | 7723 à 6686 | SEQ ID N 115 | SEQ ID N 116 et 117 |
| | 7534 à 6686 | SEQ ID N° 143 | SEQ ID N° 144 |
| *orf31* | 7754 à 8728 | SEQ ID N 118 | SEQ ID N 119 |
| *orf32c* | 10488 à 8977 | SEQ ID N 145 | SEQ ID N 146 |
| *orf33* | 10562 à 10837 | SEQ ID N 147 | SEQ ID N 148 |
| *orf34c* | 12134 à 10899 | SEQ ID N 149 | SEQ ID N 150 |

| | | | |
|---|---|---|---|
| ¹ Le « c » ajouté dans le nom du gène indique que la séquence codante est dans l'orientation inverse (le brin codant est donc le brin complémentaire de la séquence donnée en SEQ ID N° 1 ou SEQ ID N° 140 pour ces gènes). ² Lorsque plusieurs séquences protéiques sont indiquées pour une seule orf, les protéines correspondantes sont issues de plusieurs sites possibles de démarrage de la traduction. | | | |

Dans le but de déterminer la fonction des polypeptides identifiés dans le tableau 11 ci-dessus, trois types d'expériences ont été menées : la comparaison de l'identité des séquences identifiées avec des séquences de fonctions connues, des expériences d'inactivation de gènes et des analyses de la production en spiramycines de ces souches mutantes.

Les séquences protéiques déduites de ces phases ouvertes de lecture ont tout d'abord été comparées avec celles présentes dans différentes bases de données grâce à différents programmes : BLAST (Altschul *et al.,* 1990) *(Altschul et al.,* 1997), CD-search, COGs (Cluster of Orthologous Groups), FASTA ((Pearson W. R & D. J. Lipman, 1988) et (Pearson W. R., 1990), BEAUTY (Worley K. C.. *et al.,* 1995)), (cf. ci-dessus). Ces comparaisons ont permis de formuler des hypothèses sur la fonction des produits de ces gènes et d'identifier ceux susceptibles d'être impliqués dans la biosynthèse de spiramycine. Le tableau 12 montre les protéines présentant une forte similitude avec les 34 gènes situés en amont des 5 gènes PKS.

**Tableau 12**

| Gène | Protéine présentant une similitude significative | Numéro d'accès GenBank | Score BLAST* | Fonction rapportée |
|---|---|---|---|---|
| *orf1* | Cytochrome P450 tylI (*Streptomyces fradiae*) | S49051 | 530 | Cytochrome P450 |
| *orf2* | ORF15x4 (*Listonella anguillarum*) | AAB81630 | 113 | Inconnue |
| *orf3* | aminotransferase-like protein (*Streptomyces antibioticus*) | AAF59939 | 431 | Amino-transférase |
| *orf4* | alpha-D-glucose-1-phosphate thymidylyltransferase (*Streptomyces venezuelae*) | AAC68682 | 404 | alpha-D-glucose-1-phosphate thymidylyltransfé rase |
| *orf5* | AprE (*Streptomyces tenebrarius*) | AAG18457 | 476 | dTDP-glucose 4,6-déshydratase |
| *orf6* | Thioesterase (*Streptomyces avermitilis*) | BAB69315 | 234 | Thioestérase |
| *orf7* | TylCVI (*Streptomyces fradiae*) | AAF29379 | 461 | dNTP hexose 2,3-déshydratase |
| *orf8* | probable aminotransferase (*Saccharopolyspora spinosa*) | AAG23279 | 465 | Aminotransférase |
| *orf9c* | SrmX (*Streptomyces ambofaciens*) | S25204 | 445 | Méthyltransférase |
| *orf10* | SrmR (*Streptomyces ambofaciens*) | S25203 | 1074 | Protéine de régulation |
| *orf11c* | SrmB (*Streptomyces ambofaciens*) | S25202 | 955 | Résistance à la spiramycine |
| *orf12* | UrdQ (*Streptomyces fradiae*) | AAF72550 | 634 | NDP-hexose 3,4-déshydratase |
| *orf13c* | SC4H2.17 (*Streptomyces coelicolor*) | T35116 | 619 | Inconnue |
| *orf14* | putative reductase (*Streptomyces coelicolor*) | CAB90862 | 147 | Réductase |
| *orf15c* | Probable 3-ketoreductase (*Streptomyces antibiotics*) | T51102 | 285 | 3-cétoréductase |
| *orf16* | Hypothetical NDP hexose 3,4 isomerase (*Streptomyces fradiae*) | CAA57471 | 209 | NDP hexose 3,4 isomérase |
| *orf17* | Glycosyl transférase (*Streptomyces venezuelae*) | AAC68677 | 400 | Glycosyl transférase |
| *orf18* | Glycosyl transférase (*Streptomyces rishiriensis*) | AAG29785 | 185 | Glycosyl transférase |
| *orf19* | NDP-hexose 4-cétoreductase TylCIV (*Streptomyces fradiae*) | AAD41822 | 266 | NDP-hexose 4-cétoréductase |
| *orf20* | EryBII (*Saccharopolyspora erythraea*) | AAB84068 | 491 | aldocéto réductase |
| *orf21c* | TylCIII (*Streptomyces fradiae*) | AAD41823 | 669 | NDP-hexose 3-C-méthyltransférase |
| *orf22c* | FkbH (*Streptomyces hygroscopicus*) | AAF86387 | 463 | Impliqué dans la biosynthèse du méthoxymalonyl |
| *orf23c* | FkbI (*Streptomyces hygroscopicus*) | AAF86388 | 387 | Acyl CoA déshydrogénase |
| *orf24c* | FkbJ (*Streptomyces hygroscopicus*) | AAF86389 | 87 | Impliqué dans la biosynthèse du méthoxymalonyl |
| *orf25c* | FkbK (*Streptomyces hygroscopicus*) | AAF86390 | 268 | Acyl CoA déshydrogénase |
| *orf26* | TylCV (*Streptomyces fradiae*) | AAD41824 | 471 | Mycarosyl transferase |
| *orf27* | TylCVII (*Streptomyces fradiae*) | AAD41825 | 243 | NDP-hexose 3,5-(ou 5-) épimerase |
| *orf28c* | AcyB2 (*Streptomyces thermotolerans*) | JC2032 | 451 | protéine régulatrice |
| *orf29* | Béta-manannase (*Sorangium cellulosum*) | AAK19890 | 139 | Glycosyl hydrolase |
| *orf30c* | Epimérase de sucre-nucléoside-diphosphate (*Corynebacterium glutamicum*) | NP 600590 | 89 | Epimérase de sucre-nucléoside-diphosphate |
| *orf31* | Oxidoréductase (*Streptomyces coelicolor*) | NP_631148 | 261 | Oxidoréductase |
| *orf32c* | Protéine régulatrice de la famille GntR (*Streptomyces avermitilis*) | NP_824604 | 282 | Protéine régulatrice |
| *orf33* | Protéine hypothetique (*Xanthomonas campestris*) | NP_635564 | 54 | Inconnue |
| *orf34c* | Arabinofuranosidase (*Streptomyces coelicolor*) | NP_630049 | 654 | Arabinofuranosid ase |

| | | | | |
|---|---|---|---|---|
| * une plus grande similitude de séquence est associée à un score BLAST plus élevé (Altschul *et al.,* 1990). | | | | |

Des expériences d'inactivation de gènes ont été réalisées pour confirmer ces résultats. Les méthodes utilisées consistent à effectuer un remplacement de gène. Le gène cible à interrompre est remplacé par une copie de ce gène interrompue par une cassette conférant la résistance à un antibiotique (par exemple l'apramycine ou l'hygromycine). Les cassettes utilisées sont bordées de part et d'autre par des codons de terminaison de la traduction dans toutes les phases de lecture et par des terminateurs de transcription actifs chez *Streptomyces.* L'insertion de la cassette dans le gène cible peut s'accompagner ou non d'une délétion dans ce gène cible. La taille des régions flanquant la cassette peut aller de quelques centaines à plusieurs milliers de paires de bases. Un deuxième type de cassettes peut être utilisé pour l'inactivation de gènes : des cassettes dites « cassettes excisables » (cf. ci dessus). Les souches ainsi construites ont été testées pour leur production en spiramycines.

Le gène *orf1* code une protéine présentant une relative forte similitude avec plusieurs cytochrome P450. Notamment, la protéine codée par *orf1* possède une similitude importante avec la protéine codée par le gène *ylI* impliquée dans la biosynthèse de tylosine chez *Streptomyces fradiae* (Merson-Davies,L.A. *et al.,* 1994 ; Numéro d'accès GenBank : S49051 ; Score BLAST : 530). Cette similitude avec une protéine impliquée dans la voie de biosynthèse d'un autre antibiotique proche suggère fortement que le gène *orf1* code également une cytochrome P450. Cette hypothèse est étayée par le fait que la protéine codée par le gène *orf1* présente une forte similitude avec d'autres protéines de fonction similaire chez d'autres organismes (cf. tableau 13).

**Tableau 13**

| Protéine présentant une similitude significative | Numéro d'accès GenBank | Score BLAST* | Fonction rapportée |
|---|---|---|---|
| Putative cytochrome P450 YJIB *(Bacillus subtilis)* | O34374 | 248 | Cytochrome P450 |
| Cytochrome P450 113A1 (*Saccharopolyspora erythraea*) | P48635 | 237 | Cytochrome P450 |
| Cytochrome P-450 hydroxylase homolog *(Streptomyces caelestis)* | AAC46023 | 208 | Cytochrome P-450 hydroxylase |
| Cytochrome P450 monooxygénase (*Streptomyces venezuelae*) | AAC64105 | 206 | Cytochrome P450 monooxygénase |

| | | | |
|---|---|---|---|
| * une plus grande similitude de séquence est associée à un score BLAST plus élevé (Altschul *et al.,* 1990). | | | |

Le gène *orf2* code une protéine présentant une relative forte similitude avec une dTDP-6-deoxy-3,4-keto-hexulose isomerase de *An.euf°in.ibacillus thermoaerophilus* (Pfoestl,A. *et al.,* 2003, Numéro d'accès GenBank : AAO06351 ; Score BLAST : 118). Cette similitude suggère fortement que le gène *orf2* code une isomérase responsable de la réaction d'isomérisation nécessaire à la biosynthèse d'un des sucres présents dans la molécule de spiramycine, ce sucre pouvant être le mycarose (cf. figure 5). L'inactivation du gène *orf2* a été réalisée. Il a pu être montré que la souche résultante ne produit plus de spiramycines. Ceci confirme que le gène *orf2* est bien impliqué dans la biosynthèse des spiramycines.

Le gène *orf3* code une protéine présentant une relative forte similitude avec plusieurs aminotransférases. Notamment, la protéine codée par *orf3* possède une similitude importante avec une aminotransférase de *Streptomyces antibioticus* impliquée dans la biosynthèse de l'oléandomycine (Draeger,G., *et al.,* 1999 ; Numéro d'accès GenBank : AAF59939; Score BLAST: 431). Cette similitude avec une protéine impliquée dans la voie de biosynthèse d'un autre antibiotique proche suggère fortement que le gène *orf3* code une 3 amino-transférase responsable de la réaction de transamination nécessaire à la biosynthèse d'un des sucres aminés des spiramycines (cf. figure 5). Cette hypothèse est étayée par le fait que la protéine codée par le gène *orf3* présente une forte similitude avec d'autres protéines de fonction similaire chez d'autres organismes (cf. tableau 15).

**Tableau 15**

| Protéine présentant une similitude significative | Numéro d'accès GenBank | Score BLAST***** | Fonction rapportée |
|---|---|---|---|
| Aminotransférase (*Streptomyces antibioticus*) | T51111 | 429 | Aminotransférase |
| Transaminase *(Streptomyces venezuelae)* | AAC68680 | 419 | Transaminase |

| | | | |
|---|---|---|---|
| * une plus grande similitude de séquence est associée à un score BLAST plus élevé (Altschul *et al.,* 1990). | | | |

L'inactivation du gène *orf3* a été réalisée. Il a ainsi pu être montré que la souche résultante ne produit plus de spiramycines. Ceci confirme que le gène *orf3* est bien impliqué dans la biosynthèse des spiramycines. L'enzyme codée par ce gène est donc bien responsable d'une étape de bioconversion essentielle à la biosynthèse des spiramycines. La production de spiramycines peut être complémentée par l'expression de la protéine TylB de *S. fradiae* (cf exemple 23). Ceci démontre que le gène *orf3* code une 3 amino-transférase responsable de la réaction de transamination nécessaire à la biosynthèse du mycaminose (cf. figure 5). Comme le mycaminose est le premier sucre à être fixé sur le platénolide, il est attendu que la souche interrompue dans *orf3* (OS49.67) accumule du platénolide.

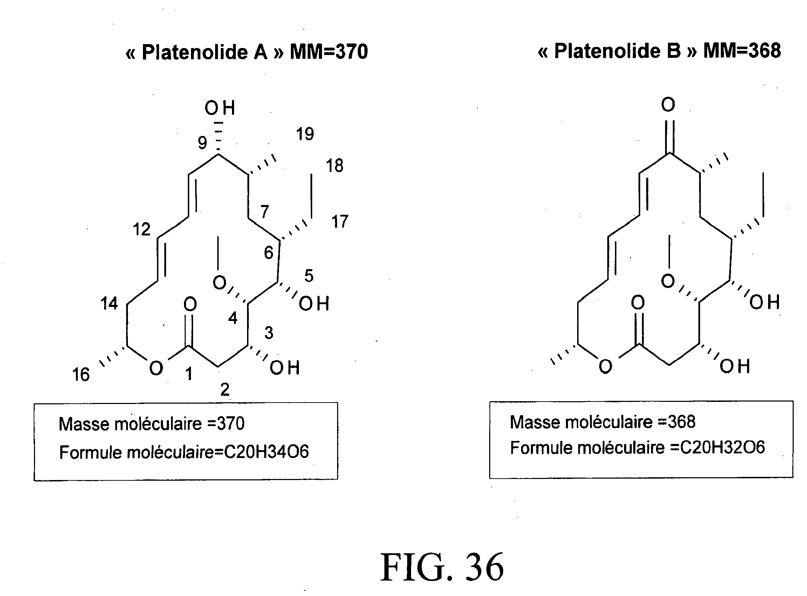

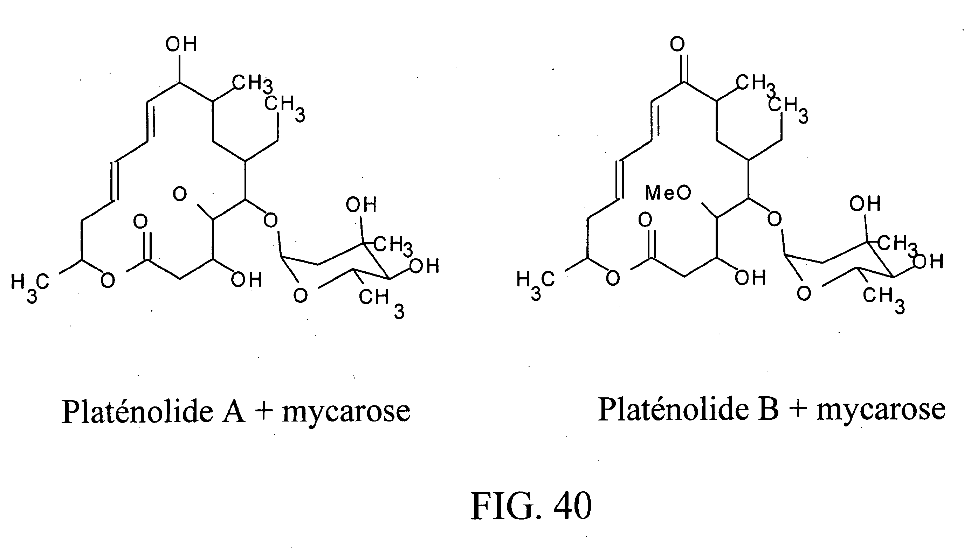

Les intermédaires de biosynthèse de la souche interrompue dans le gène *orf3* ont été étudiés (cf. exemple 20). Ces expériences ont permis de démontrer que cette souche produit deux formes de platénolide : le platénolide A et le platénolide B, la structure déduite de ces deux molécules est présentée en figure 36. Cette souche produit également du platénolide A + mycarose et du platénolide B + mycarose (cf. exemple 20 et figure 40). Ces composés comportent un sucre mais ne comportent pas de mycaminose. De plus si on les compare à de la spiramycine (cf. figure 1), ces composés comportent du mycarose à la place du mycaminose. Ces résultats sont en accord avec l'implication du produit du gène *orf3* dans la biosynthsèe du mycaminose et son rôle en tant que 3 amino-transférase responsable de la réaction de transamination nécessaire à la biosynthèse du mycaminose (cf. figure 5). On peut remarquer que la spécificité de glycosylation ne semble pas absolue puisque l'on trouve des molécules avec le mycarose fixé à la position noramalement occupée par le mycaminose (cf. figure 40).

Le gène *orf4* code une protéine présentant une relative forte similitude avec plusieurs NDP-glucose synthétases. Notamment, la protéine codée par *orf4* possède une similitude importante avec une alpha-D-glucose-1-phosphate thymidylyltransférase de *Streptomyces venezuelae* (Xue Y *et al.,* 1998 ; Numéro d'accès GenBank : AAC68682 ; Score BLAST 404). Cette similitude avec une protéine impliquée dans la voie de biosynthèse d'un autre antibiotique proche suggère fortement que le gène *orf4* code une NDP-glucose syntéthase responsable de la synthèse du NDP-glucose nécessaire à la biosynthèse des trois sucres atypiques incorporés dans la molécule de spiramycine (cf. figure 4, 5 et 6). Cette hypothèse est étayée par le fait que la protéine codée par le gène *orf4* présente une forte similitude avec d'autres protéines de fonction similaire chez d'autres organismes (cf. tableau 16).

**Tableau 16**

| Protéine présentant une similitude significative | Numéro d'accès GenBank | Score BLAST* | Fonction rapportée |
|---|---|---|---|
| Glucose-1-phosphate thymidyltransférase *(Streptomyces avermitilis)* | BAA84594 | 402 | Glucose-1-phosphate thymidyltransférase |
| AclY *(Streptomyces galilaeus)* | BAB72036 | 400 | dTDP-1-glucose synthétase |
| Putative glucose-1-phosphate thymidyltransférase (*Saccharopolyspora spinosa*) | AAK83289 | 399 | glucose-1-phosphate thymidyltransférase |

| | | | |
|---|---|---|---|
| * une plus grande similitude de séquence est associée à un score BLAST plus élevé (Altschul *et al.,* 1990). | | | |

Le gène *orf5* code une protéine présentant une relative forte similitude avec plusieurs glucose déshydratases. Notamment, la protéine codée par *orf5* possède une similitude importante avec une dTDP-glucose 4,6-déshydratase de *Streptomyces tenebrarius* (Li,T.B. *et al.*, 2001 ; Numéro d'accès GenBank: AAG18457, Score BLAST : 476). Cette similitude avec une protéine impliquée dans la voie de biosynthèse d'un autre antibiotique proche suggère fortement que le gène *orf5* code une NDP glucose déshydratase nécessaire à la biosynthèse des trois sucres atypiques incorporés dans la molécule de spiramycine (cf. figure 4, 5 et 6). Cette hypothèse, est étayée par le fait que la protéine codée par le gène *orf5* présente une forte similitude avec d'autres protéines de fonction similaire chez d'autres organismes (cf. tableau 17).

**Tableau 17**

| Protéine présentant une similitude significative | Numéro d'accès GenBank | Score BLAST* | Fonction rapportée |
|---|---|---|---|
| DTDP-glucose 4,6-déshydratase (*Saccharopolyspora spinosa*) | AAK83290 | 464 | DTDP-glucose 4,6-déshydratase |
| thymidine diphosphoglucose 4,6-déshydratase *(Saccharopolyspora erythraea)* | AAA68211 | 445 | thymidine diphosphoglucose 4,6-déshydratase |
| dTDPglucose 4,6-déshydratase (EC 4.2.1.46) - *(Streptomycesfradiae)* | S49054 | 443 | dTDPglucose 4,6-déshydratase |
| TDP-glucose-4,6-déshydratase *(Streptomyces venezuelae)* | AAC68681 | 421 | TDP-glucose-4,6-déshydratase |
| SgcA *(Streptomyces globisporus)* | AAF13998 | 418 | dNDP-glùcose-4,6-déshydratase |

| | | | |
|---|---|---|---|
| * une plus grande similitude de séquence est associée à un score BLAST plus élevé (Altschul *et al.,* 1990). | | | |

Le gène *orf6* code une protéine présentant une relative forte similitude avec plusieurs thioestérases. Notamment, la protéine codée par *orf6* possède une similitude importante avec une thioestérase de *Streptomyces avermitilis* (Omura,S. et al., 2001 ; Numéro d'accès GenBank : BAB69315 ; Score BLAST : 234). Cette similitude avec une protéine impliquée dans la voie de biosynthèse d'un autre antibiotique proche suggère fortement que le gène *orf6* code également une thioestérase. Cette hypothèse est étayée par le fait que la protéine codée par le gène *orf6* présente une forte similitude avec d'autres protéines de fonction similaire chez d'autres organismes (cf. tableau 18).

**Tableau 18**

| Protéine présentant une similitude significative | Numéro d'accès GenBank | Score BLAST* | Fonction rapportée |
|---|---|---|---|
| RifR (*Amycolatopsis mediterranei*) | AAG52991 | 216 | Thioestérase |
| Thioestérase - (*Streptomyces fradiae*) | S49055 | 215 | Thioestérase |
| Thioestérase (*Streptomyces avermitilis*) | BAB69188 | 213 | Thioestérase |
| Thioestérase II (EC 3.1.2.-) - *(Streptomyces venezuelae)* | T17413 | 203 | Thioestérase |
| PimI protein *(Streptomyces natalensis)* | CAC20922 | 201 | Thioestérase |
| Thioestérase *(Streptomyces griseus)* | CAC22116 | 200 | Thioestérase |

| | | | |
|---|---|---|---|
| * une plus grande similitude de séquence est associée à un score BLAST plus élevé (Altschul *et al.,* 1990). | | | |

Le gène *orf7* code une protéine présentant une relative forte similitude avec plusieurs hexose déshydratases. Notamment, la protéine codée par *orf7* possède une similitude importante avec une dNTP hexose 2,3-déshydratase (codée par lé gène TylCVI) de *Streptomyces fradiae* impliquée dans la biosynthèse de la tylosine (Merson-Davies,L.A. *et al.,* 1994 ; Numéro d'accès GenBank : AAF29379 ; Score BLAST : 461). Cette similitude avec une protéine impliquée dans la voie de biosynthèse d'un autre antibiotique proche suggère fortement que le gène *orf7* code également une hexose 2-3 déshydratase nécessaire à la biosynthèse de deux sucres atypiques incorporés dans la molécule de spiramycine (cf. figure 4 et 6). Cette hypothèse est étayée par le fait que la protéine codée par le gène *orf7* présente une forte similitude avec d'autres protéines de fonction similaire chez d'autres organismes (cf. tableau 19).

**Tableau 19**

| Protéine présentant une similitude significative | Numéro d'accès GenBank | Score BLAST* | Fonction rapportée |
|---|---|---|---|
| Rif18 *(Amycolatopsis mediterranei)* | AAG52988 | 459 | Hexose déshydratase |
| SimB3 *(Streptomyces antibioticus)* | AAK06810 | 444 | dNDP-4-keto-6-déoxy-glucose-2,3-déshydratase |

| | | | |
|---|---|---|---|
| * une plus grande similitude de séquence est associée à un score BLAST plus élevé (Altschul *et al.,* 1990). | | | |

Le gène *orf8* code une protéine présentant une relative forte similitude avec plusieurs aminotransférases. Notamment, la protéine codée par *orf8* possède une similitude importante avec une aminotransférase probablement impliquée dans la biosynthèse de la forosamine chez *Saccharopolyspora spinosa* (Waldron,C. *et al.,* 2001 ; Numéro d'accès GenBank : AAG23279 ; score BLAST : 465). Cette similitude avec une protéine impliquée dans la voie de biosynthèse d'un autre antibiotique proche suggère fortement que le gène *orf8* code une 4 amino-transférase responsable de la réaction de transamination nécessaire à la biosynthèse de la forosamine (cf. figure 6). Cette hypothèse est étayée par le fait que la protéine codée par le gène *orf8* présente une forte similitude avec d'autres protéines de fonction similaire chez d'autres organismes (cf. tableau 20).

**Tableau 20**

| Protéine présentant une similitude significative | Numéro d'accès GenBank | Score BLAST* | Fonction rapportée |
|---|---|---|---|
| Putative amino-sugar biosynthesis protein (*Bordetella bronchiseptica*) | CAA07666 | 213 | Proteine impliquée dans la biosynthèse d'amino-sucre |

| | | | |
|---|---|---|---|
| * une plus grande similitude de séquence est associée à un score BLAST plus élevé (Altschul *et al.,* 1990). | | | |

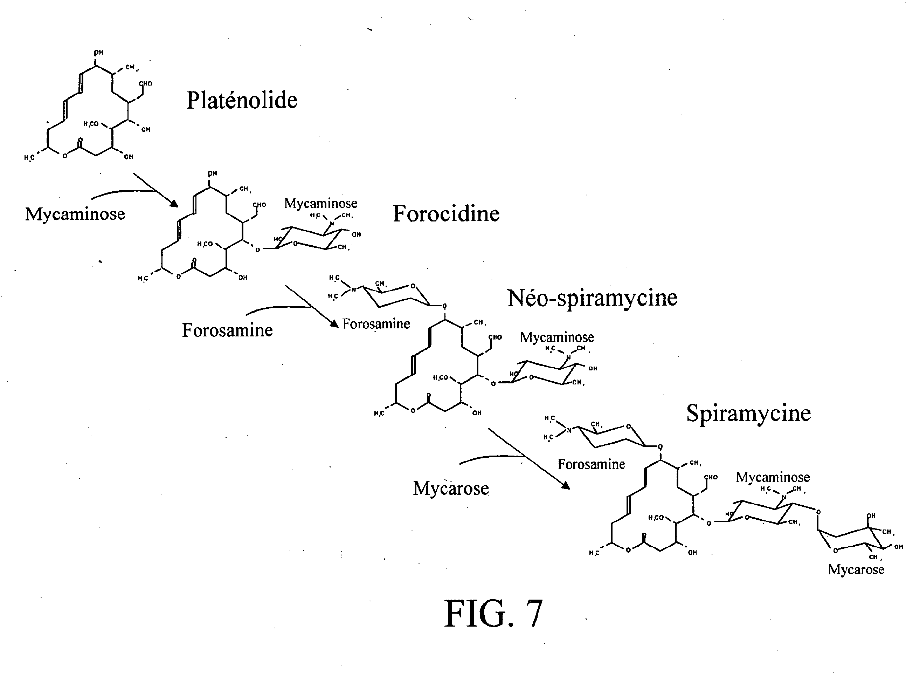

L'inactivation du gène *orf8* a été réalisée. Il a pu ainsi être montré que la souche résultante ne produit plus de spiramycines. Ceci confirme que le gène *orf8* est bien impliqué dans la biosynthèse des spiramycines. L'enzyme codée par ce gène est donc bien responsable d'une étape de bioconversion essentielle à la biosynthèse des spiramycines. La validation de l'hypothèse du rôle joué par le produit du gène *orf8* dans la biosynthèse de la forosamine est apportée par le fait qu'un mutant incativé pour le gène *orf8* produit de la forocidine, ce mutant est donc bloqué au stade de la forocidine et ne produit pas de néo-spiramycine (cf. figure 7 et exemple 25). Ces résultats sont en accord avec une implication du produit du gène *orf8* dans la biosynthèse de forosamine (cf. figure 6).

Le gène *orf9c* a déjà été identifié chez *Streptomyces ambofaciens* et a été désigné *srmX* par Geistlich *et al.* (Geistlich,M., *et al.,* 1992). La similitude de la protéine codée par ce gène avec plusieurs méthyltransférases impliquées dans la voie de biosynthèse d'autres antibiotiques proches suggère fortement que le gène *or9c* code une méthyltransférase responsable de la réaction de méthylation nécessaire à la biosynthèse du mycaminose ou de la forosamine (cf. figure 5 et 6). Cette hypothèse est étayée par le fait que la protéine codée par le gène *orf9c* présente une forte similitude avec d'autres protéines de fonction similaire chez d'autres organismes (cf. tableau 21).

**Tableau 21**

| Protéine présentant une similitude significative | Numéro d'accès GenBank | Score BLAST* | Fonction rapportée |
|---|---|---|---|
| N,N-diméthyltransférase *(Streptomyces venezuelae)* | AAC68678 | 240 | N,N-diméthyltransférase |
| Méthyltransférase *(Streptomyces antibioticus)* | CAA05643 | 232 | Méthyltransférase |
| Putative amino methylase *(Streptomyces nogalater*) | AAF01819 | 219 | Aminométhylase |

| | | | |
|---|---|---|---|
| * une plus grande similitude de séquence est associée à un score BLAST plus élevé (Altschul *et al.,* 1990). | | | |

Le gène *orf10* a déjà été identifié chez *Streptomyces ambofaciens* et a été désigné *srmR* par Geistlich *et al.* (Geistlich,M.*, et al.,* 1992). La protéine codée par ce gène est impliquée dans la régulation de la voie de biosynthèse des spiramycines chez *Streptomyces ambofaciens.* L'inactivation du gène *orf10* a été réalisée. Il a pu ainsi être montré que la souche résultante ne produit plus de spiramycines. Ceci confirme que le gène *orf10* est bien impliqué dans la biosynthèse des spiramycines. La protéine codée par ce gène est donc bien essentielle à la biosynthèse des spiramycines.

D'autre part ; le point de démarrage de la traduction de *orf 10* a été déterminé et il a pu être montré que la surexpression de ce gène entraine une amélioration de la production en spiramycines. Le site de démarrage de la traduction correspond à un ATG situé en amont de l'ATG proposé par Geistlich *et al.* (Geistlich,M.*, et al.,* 1992). Il a en outre été démontré que cette extrémité 5' est esentielle à la fonction de Orf10 puisqu'un messager tronqué en 5' est inactif (cf. exemple 17). Pour obtenir l'effet recherché sur la production en spiramycines, il est donc essentiel que la surexpression de *orf10* soit réalisée en prenant garde de ne pas exprimer un messager tronqué en 5' de *orf10.*

Le gène *orf11c* a déjà été identifié chez *Streptomyces ambofaciens* et a été désigné *srmB* par Geistlich *et al.* (Geistlich,M. *et al.,* 1992) et Schoner *et al.* (Schoner B *et al.,* 1992). La protéine codée par ce gène est impliquée dans la résistance à la spiramycine chez *Streptomyces ambofaciens* et est un transporteur de type ABC.

Le gène *orf12* code une protéine présentant une relative forte similitude avec plusieurs hexose déshydratases. Notamment, la protéine codée par *orf12* possède une similitude importante avec une NDP-hexose 3,4-déshydratase codée par le gène *UrdQ* de *Streptomyces fradiae* et impliquée dans la biosynthèse de l'urdamycine (Hoffmeister,D. *et al.,* 2000 ; Numéro d'accès GenBank : AAF72550 ; Score BLAST : 634). Cette similitude avec une protéine impliquée dans la voie de biosynthèse d'un autre antibiotique proche suggère fortement que le gène *orf12* code une 3,4 déshydratase responsable de la réaction de déshydratation nécessaire à la biosynthèse de la forosamine (cf. figure 6). Cette hypothèse est étayée par le fait que la protéine codée par le gène *orf12* présente une forte similitude avec d'autres protéines de fonction similaire chez d'autres organismes (cf. tableau 22).

**Tableau 22**

| Protéine présentant une similitude significative | Numéro d'accès GenBank | Score BLAST* | Fonction rapportée |
|---|---|---|---|
| AknP *(Streptomyces galilaeus)* | AAF73452 | 625 | 3-déshydratase |
| NDP-hexose 3,4-dehydratase homolog *(Streptomyces cyanogenus*) | AAD13547 | 624 | NDP-hexose 3,4-déshydratase |
| RdmI (*Streptomyces purpurascens*) | AAL24451 | 608 | hexose-C-3-déshydratase |
| Probable CDP-4-keto-6-deoxyglucose-3-dehydratase (E1) *(Streptomyces violaceoruber)* | T46528 | 602 | CDP-4-céto-6-déoxyglucose-3-déshydratase |
| Probable NDP-hexose-3,4-dehydratase (*Saccharopolyspora spinosa*) | AAG23278 | 582 | NDP-hexose-3,4-déshydratase |
| dNTP-hexose déshydratase (*Amycolatopsis mediterranei*) | AAC01730 | 576 | dNTP-hexose déshydratase |

| | | | |
|---|---|---|---|
| * une plus grande similitude de séquence est associée à un score BLAST plus élevé (Altschul *et al.*, 1990). | | | |

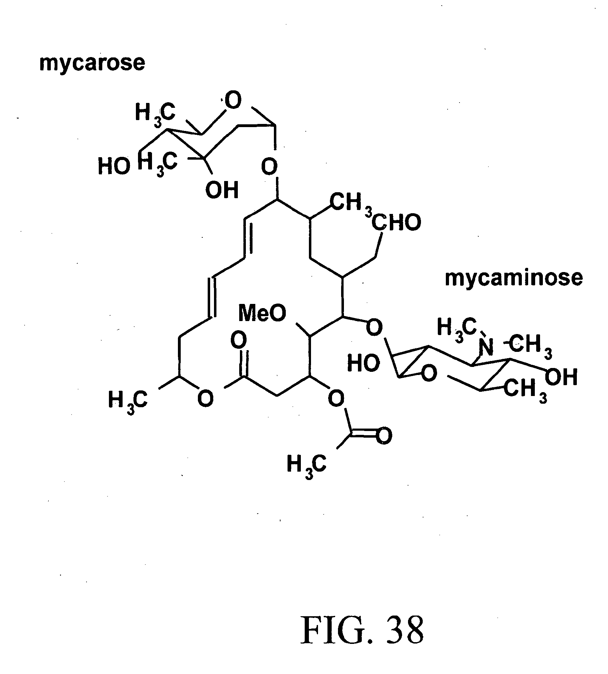

L'inactivation du gène *orf12* a été réalisée. Il a pu être montré que la souche résultante ne produit plus de spiramycines. Ceci confirme que le gène *orf12* est bien impliqué dans la biosynthèse de la spiramycine. L'enzyme codée par ce gène est donc bien responsable d'une étape de bioconversion essentielle à la biosynthèse de la spiramycine. La validation de l'hypothèse du rôle joué par Orf12 dans la biosynthèse de la forosamine est apportée par le fait qu'un mutant incativé pour le gène *orf12* ne produit plus de forosamine. Il produit cependant une faible quantité de forocidine. Ce mutant est donc bloqué au stade de la forocidine et ne produit pas de néo-spiramycine (cf. figure 7 et exemple 26). Ce mutant produit en outre un composé de structure présentée en figure 38. Ce dernier composé comporte deux sucres, le mycaminose et le mycarose mais ne comporte pas de forosamine. De plus si on le compare à la structure de la spiramycine (cf. figure 1), ce composé comporte le sucre mycarose à la place attendue de la forosamine. Ces résultats sont en accord avec l'implication du produit du gène *orf12* dans la biosynthsèe de la forosamine (cf. figure 6). On peut remarquer que la spécificité de glycosylation n'est pas absolue puisque l'on observe des molécules dans lesquelles le mycarose est fixé à la position normalement occupée par la forosamine (voir figure 38).

Le gène *orf13c* code une protéine présentant une relative forte similitude avec une protéine de fonction inconnue chez *Streptoinyces coelicolor.* Cette protéine a été nommée SC4H2.17 (numéro d'accès GeneBank: T35116; Score BLAST: 619). La protéine codée par le gène *orf13c* présente également une forte similitude avec d'autres protéines d'autres organismes (cf. tableau 23).

**Tableau 23**

| Protéine présentant une similitude significative | Numéro d'accès GenBank | Score BLAST* | Fonction rapportée |
|---|---|---|---|
| hfIX protein (*Mycobacterium leprae)* | S72938 | 473 | Inconnue |
| Possible ATP/GTP-binding protein (*Mycobacterium leprae)* | NP_301739 | 470 | Protéine fixant l'ATP/GTP |
| GTP-binding protein (*Mycobacterium tuberculosis CDC1551*) | AAK47114 | 468 | Protéine fixant le GTP |
| ATP/GTP-binding protein (*Streptomyces fradiae*) | T44592 | 388 | Protéine fixant l'ATP/GTP |

| | | | |
|---|---|---|---|
| * une plus grande similitude de séquence est associée à un score BLAST plus élevé (Altschul *et al.*, 1990). | | | |

Aucune fonction précise n'a été assignée aux protéines proches de celle codée par *orf13c.* L'inactivation du gène *orf13c* a été réalisée dans le but d'étudier la fonction de ce gène dans la voie de biosynthèse des spiramycines chez *Streptomyces ambofaciens.* Il a pu être montré que la souche résultante produit des spiramycines. Ceci indique que le gène *orf13c* n'est pas essentiel à la biosynthèse des spiramycines et qu'il n'est pas essentiel à la survie de la bactérie. L'enzyme codée par ce gène n'est donc pas responsable d'une étape de bioconversion essentielle à la biosynthèse des spiramycines.

Le gène *orf14* code une protéine présentant une relative forte similitude avec une réductase putative (Redenbach,M., *et al.*, 1996 ; Bentley *et al.*, 2002 ; numéro d'accès GenBank : CAB90862 ; Score BLAST : 147).

L'inactivation du gène *orf14* a été réalisée. Il a pu ainsi être montré que la souche résultante ne produit plus de spiramycines. Ceci confirme que le gène *orf14* est bien impliqué dans la biosynthèse des spiramycines. L'enzyme codée par ce gène est donc bien responsable d'une étape de bioconversion essentielle à la biosynthèse des spiramycines. Les intermédaires de biosynthèse de la souche interrompue dans le gène *orf14* ont été étudiés (cf. exemple 20). Ces expériences ont permis de démontrer que cette souche produit du platénolide A mais ne produit pas de platénolide B (cf. figure 36).

Le gène *orf15c* code une protéine présentant une relative forte similitude avec plusieurs cétoréductases. Notamment, la protéine codée par *orf15c* possède une similitude importante avec une 3-cétoréductase chez *Streptomyces antibioticus* (Numéro d'accès GenBak : T51102, Score BLAST : 285). Cette similitude suggère fortement que le gène *orf15c* code une 3 céto-réductase responsable de la réaction de réduction nécessaire à la biosynthèse de la forosamine (cf. figure 6). Cette hypothèse est étayée par le fait que la protéine codée par le gène *orf15c* présente une forte similitude avec d'autres protéines de fonction similaire chez d'autres organismes (cf. tableau 24).

**Tableau 24**

| Protéine présentant une similitude significative | Numéro d'accès GenBank | Score BLAST* | Fonction rapportée |
|---|---|---|---|
| oxidoreductase homolog *(Streptomyces cyanogenus*) | AAD13550 | 272 | Oxidoréductase |
| D-oliose 4-cétoreductase (*Streptomyces argillaceus*) | CAB96550 | 265 | D-oliose 4-cétoréductase |
| AknQ *(Streptomyces galilaeus)* | AAF73453 | 263 | putative 3-cétoréductase |
| Probable NDP-hexose-3-ketoreductase *(Saccharopolyspora spinosa)* | AAG23275 | 253 | NDP-hexose-3-cétoréductase |

| | | | |
|---|---|---|---|
| * une plus grande similitude de séquence est associée à un score BLAST plus élevé (Altschul *et al.,* 1990). | | | |

Le gène *orf16* code une protéine présentant une relative forte similitude avec plusieurs isomérases. Notamment, la protéine codée par *orf16* possède une similitude importante avec une NDP hexose 3,4 isomérase chez *Streptomyces fradiae* (Gandecha et al., 1997 ; Numéro d'accès GenBak : CAA57471, Score BLAST : 209). Cette similitude suggère fortement que le gène *orf16* code une protéine impliquée dans la biosynthèse d'un des sucres de la spiramycine (cf. figure 5 et 6). Cette hypothèse est étayée par le fait que la protéine codée par le gène *orf16* présente une forte similitude avec d'autres protéines de fonction similaire chez d'autres organismes (cf. tableau 25).

**Tableau 25**

| Protéine présentant une similitude significative | Numéro d'accès GenBank | Score BLAST* | Fonction rapportée |
|---|---|---|---|
| Putative tautomerase *(Streptomyces venezuelae)* | AAC68676 | 145 | Tautomérase |
| TDP-4-céto-6-déoxyhexose 3,4-isomérase *(Micromonospora megalomicea subsp. nigra*) | AAG13907 | 112 | TDP-4-céto-6-déoxyhexose 3,4-isomérase |

| | | | |
|---|---|---|---|
| * une plus grande similitude de séquence est associée à un score BLAST plus élevé (Altschul *et al.,* 1990). | | | |

Le gène *orf17* code une protéine présentant une relative forte similitude avec plusieurs glycosyl transférases. Notamment, la protéine codée par *orf17* possède une similitude importante avec une glycosyl transférase de *Streptomyces venezuelae* (Xue,Y. *et al.*, 1998 ; Numéro d'accès GenBank: AAC68677; score BLAST: 400). La similitude de la protéine codée par le gène *orf17* avec plusieurs glycosyl transférases impliquées dans la voie de biosynthèse d'autres antibiotiques proches suggère fortement que ce gène code également une glycosyl transférase. Cette hypothèse est étayée par le fait que la protéine codée par le gène *orf17* présente une forte similitude avec d'autres protéines de fonction similaire chez d'autres organismes (cf. tableau 26).

**Tableau 26**

| Protéine présentant une similitude significative | Numéro d'accès GenBank | Score BLAST* | Fonction rapportée |
|---|---|---|---|
| Glycosyltransférase (*Streptomyces fradiae*) | CAA57472 | 399 | Glycosyltransférase |
| Glycosyltransférase (*Streptomyces antibioticus*) | CAA05642 | 378 | Glycosyltransférase |
| Glycosyltransférase (*Streptomyces antibioticus*) | CAA05641 | 360 | Glycosyltransférase |
| Glycosyltransférase (*Saccharopolyspora erythraea*) | CAA74710 | 344 | Glycosyltransférase |

| | | | |
|---|---|---|---|
| * une plus grande similitude de séquence est associée à un score BLAST plus élevé (Altschul *et al.,* 1990). | | | |

Le gène *orf18* code une protéine présentant une relative forte similitude avec plusieurs glycosyl transférases. Notamment, la protéine codée par *orf18* possède une similitude importante avec une glycosyl transférase de *Streptomyces rishiriensis* (Wang *et al.,* 2000 ; Numéro d'accès GenBank : AAG29785 ; score BLAST : 185). La similitude de la protéine codée par le gène *orf18* avec plusieurs glycosyl transférases impliquées dans la voie de biosynthèse d'autres antibiotiques proches suggère fortement que ce gène code également une glycosyl transférase. Cette hypothèse est étayée par le fait que la protéine codée par le gène *orf18* présente une forte similitude avec d'autres protéines de fonction similaire chez d'autres organismes (cf. tableau 27).

**Tableau 27**

| Protéine présentant une similitude significative | Numéro d'accès GenBank | Score BLAST* | Fonction rapportée |
|---|---|---|---|
| NovM (*Streptomyces spheroides*) | AAF67506 | 184 | Glycosyltransférase |
| probable glycosyl transferase *(Streptomyces violaceoruber*) | T46519 | 169 | Glycosyltransférase |
| Glycosyl transferase homolog *(Streptomyces cyanogenus*) | AAD13553 | 167 | Glycosyltransférase |
| Glycosyl transferase homolog *(Streptomyces cyanogenus*) | AAD13555 | 163 | Glycosyltransférase |
| dNTP-hexose glycosyl transférase (*Amycolatopsis mediterranei*) | AAC01731 | 160 | Glycosyltransférase |

| | | | |
|---|---|---|---|
| * une plus grande similitude de séquence est associée à un score BLAST plus élevé (Altschul *et al.,* 1990). | | | |

Le gène *orf19* code une protéine présentant une relative forte similitude avec plusieurs cétoréductases. Notamment, la protéine codée par *orf19* possède une similitude importante avec une NDP-hexose 4-cétoréductase (TylCIV) de *Streptomyces fradiae* (Bate *et al.*, 2000 ; Numéro d'accès GenBank : AAD41822 ; score BLAST : 266). La similitude de la protéine codée par le gène *orf19* avec cette cétoréductase impliquée dans la voie de biosynthèse d'un antibiotique proche suggère fortement que ce gène code également une 4-cétoréductase responsable de la réaction de réduction nécessaire à la biosynthèse du mycarose (cf. figure 4). Cette hypothèse est étayée par le fait que la protéine codée par le gène *orf19* présente une forte similitude avec d'autres protéines de fonction similaire chez d'autres organismes (cf. tableau 28).

**Tableau 28**

| Protéine présentant une similitude significative | Numéro d'accès GenBank | Score BLAST* | Fonction rapportée |
|---|---|---|---|
| NDP-4-céto-6-déoxyhexose 4-cétoreductase *(Streptomyces venezuelae)* | AAL14256 | 251 | NDP-4-céto-6-déoxyhexose 4-cétoréductase |
| EryBIV *(Saccharopolyspora erythraea)* | AAB84071 | 249 | oxidoréductase |
| TDP-4-céto-6-déoxyhexose 4-cétoreductase (*Micromonospora megalomicea subsp. Nigra*) | AAG13916 | 218 | TDP-4-céto-6-déoxyhexose 4-cétoréductase |
| dTDP-4-céto-6-déoxy-L-hexose 4-réductase (*Streptomyces avermitilis*) | BAA84595 | 212 | dTDP-4-céto-6-déoxy-L-hexose 4-réductase |

| | | | |
|---|---|---|---|
| * une plus grande similitude de séquence est associée à un score BLAST plus élevé (Altschul *et al.,* 1990). | | | |

Le gène *orf20* code une protéine présentant une relative forte similitude avec plusieurs hexose réductases. Notamment, la protéine codée par *orf20* possède une similitude importante avec le gène EryBII de *Saccharopolyspora erythraea* qui code une dTDP-4-céto-L-6-déoxy-hexose 2,3-réductase (Summers,R.G., *et al.,* 1997), Numéro d'accès GenBank : AAB84068 ; Score BLAST : 491). La similitude de la protéine codée par le gène *orf20* avec plusieurs hexose réductases impliquées dans la voie de biosynthèse d'autres antibiotiques proches suggère fortement que ce gène code une 2-3 réductase responsable de la réaction de réduction nécessaire à la biosynthèse du mycarose (cf. figure 4). Cette hypothèse est étayée par le fait que la protéine codée par le gène *orf20* présente une forte similitude avec d'autres protéines de fonction similaire chez d'autres organismes (cf. tableau 29).

**Tableau 29**

| Protéine présentant une similitude significative | Numéro d'accès GenBank | Score BLAST* | Fonction rapportée |
|---|---|---|---|
| TylCII *(Streptomyces fradiae)* | AAD41821 | 464 | NDP-hexose 2,3-énoyl réductase |
| TDP-4-céto-6-deoxyhexose 2,3-réductase (*Micromonospora megalomicea subsp. Nigra)* | AAG13914 | 446 | TDP-4-céto-6-déoxyhexose 2,3-réductase |
| dTDP-4-céto-6-déoxy-L-hexose 2,3-réductase *(Streptomyces avermitilis*) | BAA84599 | 377 | dTDP-4-céto-6-déoxy-L-hexose 2,3-réductase |

| | | | |
|---|---|---|---|
| * une plus grande similitude de séquence est associée à un score BLAST plus élevé (Altschul *et al.*, 1990). | | | |

Le gène *orf21c* code une protéine présentant une relative forte similitude avec plusieurs hexose méthyltransférases. Notamment, la protéine codée par *orf21c* possède une similitude importante avec le gène *TylCIII* de *Streptomyces fradiae* qui code une NDP-hexose 3-C-méthyltransférase (Bate,N *et al.,* 2000 ; Numéro d'accès GenBank : AAD41823 ; Score BLAST : 669). La similitude de la protéine codée par le gène *orf21c* avec plusieurs hexose méthyltransférases impliquées dans la voie de biosynthèse d'autres antibiotiques proches suggère fortement que ce gène code une hexose méthyltransférase responsable de la réaction de méthylation nécessaire à la biosynthèse du mycarose (cf. figure 4). Cette hypothèse est étayée par le fait que la protéine codée par le gène *orf21c* présente une forte similitude avec d'autres protéines de fonction similaire chez d'autres organismes (cf. tableau 30).

**Tableau 30**

| Protéine présentant une similitude significative | Numéro d'accès GenBank | Score BLAST* | Fonction rapportée |
|---|---|---|---|
| EryH *(Saccharopolyspora erythraea)* | 228448 | 592 | Gène de biosynthèse de l'érythromycine |
| S-adenosyl-dependent methyl transferase *(Coxiella burnetii)* | AAK71270 | 358 | Méthyltransferase |
| NovU *(Streptomyces spheroides)* | AAF67514 | 184 | C-méthyltransférase |

| | | | |
|---|---|---|---|
| * une plus grande similitude de séquence est associée à un score BLAST plus élevé (Altschul *et al.,* 1990). | | | |

Le gène *orf22c* code une protéine présentant une relative forte similitude avec la protéine codée par le gène *fkbH* de *Streptomyces hygroscopicus var. ascomyceticus* qui code une enzyme impliquée dans la biosynthèse du méthoxymalonyl (Wu,K. *et al.*, 2000 ; Numéro d'accès GenBank : AAF86387 ; Score BLAST : 463). La similitude de la protéine codée par le gène *orf22c* avec cette protéine impliquée dans la voie de biosynthèse d'un autre macrolide proche suggère fortement que ce gène code également une enzyme impliquée dans la biosynthèse du méthoxymalonyl chez *Streptomyces ambofaciens* (cf. figure 8).

Le gène *orf23c* code une protéine présentant une relative forte similitude avec la protéine codée par le gène *fkbI* de *Streptomyces hygroscopicus var. ascomyceticus* qui code une acyl CoA déshydrogénase impliquée dans la biosynthèse du méthoxymalonyl (Wu,K. *et al.*, 2000 ; Numéro d'accès GenBank : AAF86388 ; Score BLAST 387). La similitude de la protéine codée par le gène *orf23c* avec plusieurs acyl CoA déshydrogénases impliquées dans la voie de biosynthèse d'autres antibiotiques proches suggère fortement que ce gène code une acyl CoA déshydrogénase impliquée dans la biosynthèse du méthoxymalonyl (cf. figure 8). Cette hypothèse est étayée par le fait que la protéine codée par le gène *orf23c* présente une forte similitude avec d'autres protéines de fonction similaire chez d'autres organismes (cf. tableau 31).

**Tableau 31**

| Protéine présentant une similitude significative | Numéro d'accès GenBank | Score BLAST* | Fonction rapportée |
|---|---|---|---|
| Acyl-CoA déshydrogénase *(Polyangium cellulosum)* | AAK19892 | 171 | Acyl-CoA déshydrogénase |
| Probable acyl-CoA dehydrogenase - (*Streptomyces coelicolor*) | T36802 | 160 | acyl-CoA déshydrogénase |

| | | | |
|---|---|---|---|
| * une plus grande similitude de séquence est associée à un score BLAST plus élevé (Altschul *et al.,* 1990). | | | |

Le gène *orf24c* code une protéine présentant une relative forte similitude avec la protéine codée par le gène *fkbJ* de *Streptomyces hygroscopicus var. ascomyceticus* qui coderait la protéine de liaison du groupement acyl (Acyl Carrier Protein (ACP)) impliquée dans la biosynthèse du méthoxymalonyl (Wu,K., *et al.,* 2000 ; Numéro d'accès GenBank : AAF86389 ; Score BLAST : 87). La similitude de la protéine codée par le gène *orf24c* avec cette protéine impliquées dans la voie de biosynthèse d'un autre macrolide proche suggère fortement que ce gène code une protéine impliquée dans la biosynthèse du méthoxymalonyl chez *Streptomyces ambofaciens* (cf. figure 8).

Le gène *orf25c* code une protéine présentant une relative forte similitude avec la protéine codée par le gène *fkbK* de *Streptomyces hygroscopicus var. ascomyceticus* qui code une acyl CoA déshydrogénase impliquée dans la biosynthèse du méthoxymalonyl (Wu,K., *et al.,* 2000 ; Numéro d'accès GenBank : AAF86390 ; score BLAST : 268). La similitude de la protéine codée par le gène *orf25c* avec plusieurs acyl CoA déshydrogénases impliquées dans la voie de biosynthèse d'autres antibiotiques proches suggère fortement que ce gène code une acyl CoA déshydrogénase impliquée dans la biosynthèse du méthoxymalonyl (cf. figure 8). Cette hypothèse est étayée par le fait que la protéine codée par le gène *orf25c* présente une forte similitude avec d'autres protéines de fonction similaire chez d'autres organismes (cf. tableau 32).

**Tableau 32**

| Protéine présentant une similitude significative | Numéro d'accès GenBank | Score BLAST* | Fonction rapportée |
|---|---|---|---|
| Probable 3-Hydroxybutyryl-CoA Dehydrogenase (*Bacillus subtilis*) | P45856 | 177 | 3-Hydroxybutyryl-CoA déshydrogénase |
| 3-hydroxybutyryl-CoA dehydrogenase protein *(Bacillus thuringiensis serovar kurstaki*) | AAL32270 | 174 | 3-hydroxybutyryl-CoA déshydrogénase |
| 3-hydroxybutyryl-CoA dehydrogenase *(Deinococcus radiodurans*) | NP_294792 | 167 | 3-hydroxybutyryl-CoA déshydrogénase |

| | | | |
|---|---|---|---|
| * une plus grande similitude de séquence est associée à un score BLAST plus élevé (Altschul *et al.*, 1990). | | | |

Le gène *orf26* code une protéine présentant une identité de 65% (déterminée grâce au programme BLAST) avec la protéine codée par le gène *tylCV* qui code une mycarosyl transferase impliquée dans la biosynthèse de tylosine chez *Streptomyces fradiae* (Bate,N. *et al.,* 2000 ; Numéro d'accès GenBank : AAD41824, Score BLAST : 471). Plus particulièrement, TylCV est une glycosyl-transférase liant la molécule de mycarose lors de la synthèse de la tylosine. Cette similitude avec une protéine impliquée dans la voie de biosynthèse d'un autre antibiotique relativement proche et plus particulièrement dans le transfert du mycarose, suggère que le gène *orf26* code une glycosyl transferase. Cette hypothèse est étayée par le fait que la protéine codée par le gène *orf26* présente une forte similitude avec d'autres protéines de fonction similaire chez d'autres organismes (cf. tableau 33).

**Tableau 33**

| Protéine présentant une similitude significative | Numéro d'accès GenBank | Score BLAST* | Fonction rapportée |
|---|---|---|---|
| Glycosyl transferase *(Streptomyces avermitilis)* | BAA84592 | 218 | Glycosyl transferase |
| CalG4 (*Micromonospora echinospora)* | AAM70365 | 217 | Glycosyl transferase |
| CalG2 (*Micromonospora echinospora*) | AAM70348 | 197 | Glycosyl transferase |

| | | | |
|---|---|---|---|
| * une plus grande similitude de séquence est associée à un score BLAST plus élevé (Altschul *et al.,* 1990). | | | |

Le gène *orf27* code une protéine présentant une identité de 70% (déterminée grâce au programme BLAST) avec la protéine codée par le gène *tylCVII* qui code une NDP-hexose 3,5- (ou 5-) épimerase impliquée dans la biosynthèse de tylosine chez *Streptomyces fradiae* (Bate,N. *et al.,* 2000; Numéro d'accès GenBank: AAD41825, Score BLAST: 243). Plus particulièrement, TylCVII est une hexose 3,5- (ou 5-) épimerase impliquée dans la biosynthèse du mycarose. Cette similitude avec une protéine impliquée dans la voie de biosynthèse d'un autre antibiotique relativement proche et plus particulièrement dans la biosynthèse du mycarose, suggère que le gène *orf27* code une épimérase. Cette hypothèse est étayée par le fait que la protéine codée par le gène *orf27* présente une forte similitude avec d'autres protéines de fonction similaire chez d'autres organismes (cf. tableau 34). Une analyse des séquences proches obtenues grâce au programme BLAST suggère fortement que le gène *orf27* code une 5 épimérase responsable de la réaction d'épimérisation nécessaire à la biosynthèse du mycarose (cf. figure 4).

**Tableau 34**

| Protéine présentant une similitude significative | Numéro d'accès GenBank | Score BLAST* | Fonction rapportée |
|---|---|---|---|
| LanZ1 *(Streptomyces cyanogenus*) | AAD13558 | 172 | NDP-hexose 3,5-epimerase |
| Epi (*Saccharopolyspora spinosa*) | AAK83288 | 169 | TDP-4-keto-6-deoxyglucose 3,5 epimerase |
| dNTP-hexose 3,5 epimerase (*Amycolatopsis mediterranei*) | AAC01732 | 166 | dNTP-hexose 3,5 epimerase |

| | | | |
|---|---|---|---|
| * une plus grande similitude de séquence est associée à un score BLAST plus élevé (Altschul *et al.*, 1990). | | | |

La séquence d'*orf28c* a dans un premier temps été déterminée de manière partielle, puisque la séquence d'une région d'environ 450 paires de bases n'a été déterminée qu'après reséquençage (cette région est symbolisée par des « N » dans la séquence incomplète SEQ ID N° 106). La séquence partielle de cette ORF (SEQ ID N° 111) a néanmoins été utilisée pour l'analyse avec les différents programmes informatiques comme expliqué ci-dessus. Il a ainsi pû être déterminé que le gène *orf28c* code une protéine présentant une identité de 64% sur la séquence déterminée (SEQ ID N° 112, qui est la séquence partielle de la protéine Orf28c) (déterminée grâce au programme BLAST) avec la protéine codée par le gène *acyB2* qui code une protéine régulatrice impliquée dans la biosynthèse de carbomycine chez *Streptomyces thermotolerans* (Arisawa,A., *et al.,* 1993 ; Numéro d'accès GenBank : JC2032, Score BLAST : 329). Cette similitude avec une protéine impliquée dans la voie de biosynthèse d'un antibiotique relativement proche suggère que le gène *orf18c* code une protéine régulatrice impliquée dans la biosynthèse des spiramycines. Cette hypothèse est étayée par le fait que la protéine codée par le gène *orf28c* présente également une forte similitude avec la protéine TylR qui est une protéine régulatrice impliquée dans la biosynthèse de tylosine chez *Streptomyces fradiae* (Bate,N. *et al.,* 1999; Numéro d'accès GenBank : AAF29380, Score BLAST : 167).

Le gène *orf28c* a pu être amplifié grâce à des oligonucléotides situés de part et d'autre de la séquence non déterminée et sous cloné dans un vecteur d'expression. Il a pu ainsi être démontré que la surexpression du gène *orf28c* augmente significativement la production en spiramycines de la souche OSC2 (cf. exemple 24). Ceci démontre que la surexpression de *orf28c* conduit à une augmentation de la production en spiramycines et confirme son rôle en tant que régulateur de la voie de biosynthèse des spiramycines.

La séquence partielle d'*orf28c* a été complétée par la suite et la région manquante d'environ 450 paires de bases a été déterminée (cf. SEQ ID N° 140 et SEQ ID N° 141). La séquence complète de cette ORF (SEQ ID N° 141) a été utilisée pour l'analyse avec les différents programmes informatiques comme expliqué ci-dessus. Il a ainsi pû être déterminé que le gène *orf28c* code une protéine présentant une identité de 69% sur la séquence déterminée (SEQ ID N° 142, qui est la séquence complète de la protéine Orf28c) (déterminée grâce au programme BLAST) avec la protéine codée par le gène *acyB2* qui code une protéine régulatrice impliquée dans la biosynthèse de carbomycine chez *Streptomyces thermotolerans* (Arisawa,A., *et al.,* 1993 ; Numéro d'accès GenBank : JC2032, Score BLAST : 451). Cette similitude avec une protéine impliquée dans la régulation de la biosynthèse d'un antibiotique relativement proche suggère que le gène *orf28c* code une protéine régulatrice impliquée dans la biosynthèse des spiramycines. Cette hypothèse est étayée par le fait que la protéine codée par le gène *orf28c* présente également une forte similitude avec la protéine TylR qui est une protéine régulatrice impliquée dans la régulation de la biosynthèse de tylosine chez *Streptomyces fradiae* (Bate,N. *et al.,* 1999; Numéro d'accès GenBank : AAF29380, Score BLAST : 224). Les résultats de la surexpression de ce gène (cf. exemple 24) confirment son rôle en tant que régulateur de la voie de biosynthèse des spiramycines.

L'inactivation du gène *orf28c* a été réalisée. Il a pu ainsi être montré que la souche résultante ne produit plus de spiramycines. Ceci confirme que le gène *orf28c* est bien impliqué dans la biosynthèse des spiramycines et est essentiel à la biosynthèse des spiramycines. Ces résultats associés aux résultats de la surexpression de ce gène (cf. exemple 24) sont en accord avec un rôle d'activateur essentiel à la biosynthèse des spiramycines d'Orf28c.

Le gène *orf29* code une protéine présentant une identité de 31 % (déterminée grâce au programme BLAST) avec une probable glycosyl hydrolase localisée dans le groupe de gènes impliqué dans la biosynthèse de soraphen A (un antifongique de la classe des polyketides) chez *Sorangium cellulosum* (Ligon,J., *et al.,* 2002 ; Numéro d'accès GenBank : AAK19890, Score BLAST : 139). Cette similitude avec une protéine impliquée dans la voie de biosynthèse d'une molécule relativement proche suggère que le gène *orf29* code une protéine ayant une activité glycosyl hydrolase. Cette hypothèse est étayée par le fait que la protéine codée par le gène *orf29* présente une forte similitude avec d'autres protéines de fonction similaire chez d'autres organismes (cf. tableau 35). Une analyse de la séquence de la protéine codée par *orf29* grâce au programme CD-search (cf. ci-dessus) suggère également que le gène *orf29* code une glycosyl hydrolase.

**Tableau 35**

| Protéine présentant une similitude significative | Numéro d'accès GenBank | Score BLAST* | Fonction rapportée |
|---|---|---|---|
| ManA (*Caldicellulosiruptor saccharolyticus*) | AAC44232 | 136 | beta-1,4-mannanase |
| ManA (*Dictyoglomus thermophilum*) | AAB82454 | 129 | beta-mannanase |

| | | | |
|---|---|---|---|
| * une plus grande similitude de séquence est associée à un score BLAST plus élevé (Altschul *et al.*, 1990). | | | |

L'analyse de la séquence protéique déduite de l'*orf29* par le programme SignalP (http://www.cbs.dtu.dk/services/SignalP/) (Nielsen,H., *et al.,* 1997) montre que cette protéine possède une séquence signal C-terminale avec un site de coupure prédit entre les positions 30 et 31 (QSA/QA). On peut prédire que cette protéine est extra-cellulaire. Elle pourrait, en temps que glycosyl-hydrolase, avoir un rôle dans la réactivation de la spiramycine inactivé par glycosylation par les glycosyl-transferases GimA et/ou GimB (Gourmelen *et al.*, 1998).

Le gène *orf30c* code une protéine présentant une identité de 31% (déterminée grâce au programme BLAST) avec une épimérase de sucre-nucléoside-diphosphate de *Corynebacterium glutamicum* (Numéro d'accès GenBank : NP_600590, Score BLAST : 89). Cette similitude suggère que le gène *orf30c* code une épimérase. Cette hypothèse est étayée par le fait qu'une analyse de la séquences grâce au programme CD-search (cf. ci-dessus) suggère également que le gène *orf30c* code une épimérase.

L'*orf30c* présente deux codons d'initiations possibles (cf. SEQ ID N° 115) qui donnent deux protéines possibles de 345 et 282 acides aminés respectivement (SEQ ID N° 116 et 117). Toutefois, l'usage des codons est typique de *Streptomyces* seulement à partir du deuxième ATG de plus, la séquence protéique déduite de la séquence entre le premier ATG et le deuxième ne s'aligne pas avec les séquences proches identifiées, alors que la séquence protéique la plus courte (à partir du 2^{ème} ATG : SEQ ID N° 144) s'aligne bien avec le début de ces protéines. On peut donc en déduire que le deuxième ATG est le bon codon d'initiation et que la séquence de cet orf est donc celui présenté en SEQ ID N° 143 qui une fois traduit corespond à la protéine de séquence SEQ ID N° 144.

Le gène *orf31* code une protéine présentant une identité de 52% (déterminée grâce au programme BLAST) avec une oxidoreductase chez *Streptomyces coelicolor* (Numéro d'accès GenBank : NP_631148, Score BLAST : 261). Cette similitude suggère que le gène *orf31* code une réductase. Cette hypothèse est étayée par le fait qu'une analyse de la séquences grâce au programme CD-search (cf. ci-dessus) suggère également que le gène *orf31* code une réductase. Cette hypothèse est également étayée par le fait que la protéine codée par le gène *orf31* présente une forte similitude avec d'autres protéines de fonction similaire chez d'autres organismes (cf. tableau 36).

**Tableau 36**

| Protéine présentant une similitude significative | Numéro d'accès GenBank | Score BLAST* | Fonction rapportée |
|---|---|---|---|
| Putative oxidoreductase (*Streptomyces griseus*) | BAB79295 | 173 | Oxidoreductase |
| MocA *(Xanthomonas axonopodis*) | NP_640644 | 171 | Oxidoreductase |

| | | | |
|---|---|---|---|
| * une plus grande similitude de séquence est associée à un score BLAST plus élevé (Altschul *et al.,* 1990). | | | |

L'inactivation du gène *orf31* a été réalisée. Il a pu ainsi être montré que la souche résultante ne produit plus de spiramycines. Ceci confirme que le gène *orf31* est bien impliqué dans la biosynthèse des spiramycines. L'enzyme codée par ce gène est donc bien responsable d'une étape de bioconversion essentielle à la biosynthèse des spiramycines.

La séquence d'*orf32c* a tout d'abord été déterminée de manière partielle (cf. exemple 19), puisque la séquence codante en 5' n'a été déterminée que dans un second temps. La séquence partielle de cette orf (SEQ ID N° 120) a néanmoins été utilisée pour l'analyse avec les différents programmes informatiques comme expliqué ci-dessus. Il a ainsi pû être déterminé que le gène *orf32c* code une protéine présentant une identité de 47% sur la séquence déterminée (SEQ ID N° 121, qui est la séquence partielle de la protéine Orf32c) (déterminée grâce au programme BLAST) avec une protéine régulatrice de la famille GntR chez *Streptomyces coelicolor* (Numéro d'accès GenBank : NP_625576, Score BLAST : 229). Cette similitude suggère que le gène *orf32c* code un régulateur transcriptionel de la famille GntR. Cette hypothèse est étayée par le fait que la protéine codée par le gène *orf32c* présente une forte similitude avec d'autres protéines de fonction similaire chez d'autres organismes.

La séquence partielle d'*orf32c* a été complétée par la suite et la région manquante a été déterminée (cf. SEQ ID N° 140 et SEQ ID N° 145). La séquence complète de cette orf code une protéine présentant une identité de 44% (déterminée grâce au programme BLAST) avec une protéine régulatrice de la famille GntR chez *Streptomyces avermitilis* (Numéro d'accès GenBank : NP_824604, Score BLAST : 282). Cette similitude suggère que le gène *orf32c* code un régulateur transcriptionel de la famille GntR. Cette hypothèse est étayée par le fait que la protéine codée par le gène *orf32c* présente une forte similitude avec d'autres protéines de fonction similaire chez d'autres organismes (cf. tableau 37).

**Tableau 37**

| Protéine présentant une similitude significative | Numéro d'accès GenBank | Score BLAST* | Fonction rapportée |
|---|---|---|---|
| Protéine régulatrice de la famille GntR (*Streptomyces avermitilis*) | NP_828241 | 270 | Protéine régulatrice de la famille GntR |
| Protéine régulatrice de la famille GntR *Streptomyces coelicolor*) | NP_625576 | 266 | Protéine régulatrice de la famille GntR |
| SC5G8.04 (*Streptomyces coelicolor*) | NP_628991 | 258 | Protéine régulatrice de la famille GntR |
| transcriptional regulator (*Streptomyces venezuelae*) | AAF01064 | 224 | Régulateur transcriptionel |
| Protéine régulatrice de la famille GntR (*Streptomyces avermitilis*) | NP_827432 | 239 | Protéine régulatrice de la famille GntR |

| | | | |
|---|---|---|---|
| * une plus grande similitude de séquence est associée à un score BLAST plus élevé (Altschul *et al*., 1990). | | | |

L'inactivation du gène *orf32c* a été réalisée dans le but d'étudier la fonction de ce gène dans la voie de biosynthèse des spiramycines chez *Streptomyces ambofaciens.* Il a pu être montré que la souche résultante produit des spiramycines. Ceci indique que le gène *orf32c* n'est pas essentiel à la biosynthèse des spiramycines et qu'il n'est pas essentiel à la survie de la bactérie.

Le gène *orf33* code une protéine présentant une identité de 49% (déterminée grâce au programme BLAST) avec une protéine hypothétique de *Xanthomonas campestris* (Numéro d'accès GenBank : NP_635564, Score BLAST : 54).

La séquence d'*orf34c* est partielle. En effet, les comparaisons effectuées entre le produit de cette orf et les banques de données suggèrent que la partie C-terminale de cette protéine n'est pas dans le produit déduit de la séquence nucléotidique et donc que cette orf serait plus longue et continuerait en dehors de la région séquencée. La séquence partielle de cette ORF a néanmoins été utilisée pour l'analyse avec les différents programmes informatiques comme expliqué ci-dessus. Il a ainsi pû être déterminé que le gène *orf34c* code une protéine présentant une identité de 91% sur la séquence déterminée (SEQ ID N° 150, qui est la séquence partielle de la protéine Orf34c) (déterminée grâce au programme BLAST) avec une arabinofuranosidase de chez *Streptomyces coelicolor* (Bentley *et al.,* 2002; Numéro d'accès GenBank : NP_630049, Score BLAST : 654). Chez *S. coelicolor* le gène codant cette arabinofuranosidase ne semble pas impliqué dans la biosynthèse de métabolite secondaire. Chez S. *ambofaciens,* ce gène n'est donc probablement pas impliqué dans la biosynthèse de spiramycine.

L'invention concerne un polynucléotide ayant au moins 80%, de façon plus préférée 85%, de façon encore plus préférée 90%, de façon encore plus préférée 95% et de manière tout à fait préférée 98% d'identité en nucléotides avec un polynucléotide de SEQ ID N° 111 ou 141. Préférentiellement, ces dits polynucléotides sont isolés à partir d'une bactérie du genre *Streptomyces.* De façon tout à fait préférée, un polynucléotide selon l'invention est choisi dans le groupe constitué des séquences nucléotidiques de SEQ ID N° 111 et 141.

L'alignement optimal des séquences pour la comparaison peut être réalisé de manière informatique à l'aide d'algorithmes connus, par exemple ceux du package FASTA (Pearson W. R & D. J. Lipman, 1988) et (Pearson W. R., 1990), accessible notamment auprès du centre de ressources INFOBIOGEN, Evry, France. À titre d'illustration, le pourcentage d'identité de séquence pourra être determiné à l'aide du logiciel LFASTA (Chao K.-M. *et al.,* 1992) ou LALIGN (Huang X. et Miller W., 1991). Les programmes LFASTA et LALIGN font partie du package FASTA. LALIGN retourne les alignements locaux optimaux : ce programme est plus rigoureux, mais aussi plus lent que LFASTA.

Un autre aspect de l'invention concerne un polypeptide résultant de l'expression d'une séquence d'acide nucléique telle que définie ci-dessus. Préferentiellement, les polypeptides selon l'invention présentent au moins 80%, de façon plus préférée 85%, de façon encore plus préférée 90%, de façon encore plus préférée 95% et de manière tout à fait préférée 98% d'identité en acides aminés avec un polypeptide de SEQ ID N° 112 ou 142. Préferentiellement les polypeptides selon l'invention sont exprimés à l'état naturel par une bactérie du genre *Streptomyces.* De façon préférée, un polypeptide selon l'invention est choisi dans le groupe constitué des séquences polypeptidiques de SEQ ID N° 112 et 142.

L'alignement optimal des séquences pour la comparaison peut être réalisé de manière informatique à l'aide d'algorithmes connus, par exemple ceux du package FASTA (Pearson W. R & D. J. Lipman, 1988) et (Pearson W. R., 1990), accessible notamment auprès du centre de ressources INFOBIOGEN, Evry, France. À titre d'illustration, le pourcentage d'identité de séquence pourra être déterminé à l'aide du logiciel LFASTA (Chao K.-M. *et al.,* 1992) ou LALIGN (Huang X. et Miller W., 1991), en utilisant les paramètres par défaut tel que défini par le centre de ressources INFOBIOGEN, Evry, France. Les programmes LFASTA et LALIGN font partie du package FASTA. LALIGN retourne les alignements locaux optimaux : ce programme est plus rigoureux, mais aussi plus lent que LFASTA.

Un autre aspect de l'invention concerne un vecteur. Plus préférentiellement, le vecteur est choisi parmi les bactériophages, les plasmides, les phagemides, les vecteurs intégratifs, les fosmides, les cosmides, les vecteurs navettes, les BAC (Bacterial Artificial Chromosome) ou les PAC (P1-derived Artificial Chromosome). A titre illustratif on peut citer comme bactériophages le phage lambda et le phage M13. Comme plasmides on peut citer les plasmides se répliquant chez *E. coli* par exemple pBR322 et ses dérivés, pUC18 et ses dérivés, pUC19 et ses dérivés, pGB2 et ses dérivés (Churchward G. *et al.,* 1984), pACYC177 (numéro d'accès GenBank : X06402) et ses dérivés, pACYC184 (numéro d'accès GenBank : X06403) et ses dérivés. On peut également citer les plasmides se répliquant chez *Streptomyces* comme par exemple pIJ101 et ses dérivés, pSG5 et ses dérivés, SLP1 et ses dérivés, SCP2* et ses dérivés *(Kieser et al.* 2000). Comme phagemides on peut citer à titre illustratif pBluescript II et ses dérivés (commercialisés notamment par la société Stratagene (LaJolla, Californie, USA)), pGEM-T et ses dérivés (commercialisé par la société Promega (Madison, Wisconcin, USA)), λZAPII et ses dérivés (commercialisés notamment par la société Stratagene (LaJolla, Californie, USA)). Comme vecteurs intégratifs on peut citer à titre illustratif, les vecteurs intégratifs chez *Streptomyces* comme par exemple ceux dérivés de SLP1 *(Kieser et al,* 2000), ceux dérivés de pSAM2 (Kieser *et al,* 2000), les vecteurs utilisant les systèmes d'intégration des phages PhiC31 *(Kieser et al,* 2000) (par exemple pSET152 (Bierman *et al.*, 1992)) ou de VWB (Van Mellaert L, *et al.* 1998), ainsi que les vecteurs utilisant le système d'intégration de IS117 (Kieser et al. 2000). Comme fosmides on peut citer à titre illustratif le fosmide pFOS 1 (commercialisé par la société New England Bioloabs Inc., Beverly, Massachussetts, USA) et ses dérivés. Comme cosmides on peut citer à titre illustratif le cosmide SuperCos et ses dérivés (commercialisés notamment par la société Stratagene (LaJolla, Californie, USA)), le cosmide pWED15 (Wahl *et al.,* 1987) et ses dérivés. Comme vecteurs navettes on peut citer à titre illustratif les plasmides navettes *E. coli* / *Streptomyces* comme par exemple pIJ903 et ses dérivés, la série des plasmides pUWL, pCAO106, pWHM3, pOJ446 et leurs dérivés (Kieser *et al.* 2000), les BAC navettes *E. coli* / *Streptomyces* comme par exemple ceux décrits dans la demande de brevet WO 01/40497. Comme BAC (Bacterial Artificial Chromosome) on peut citer à titre illustratif le BAC pBeloBAC11 (numéro d'accès GenBank :U51113). Comme PAC (P1-derived Artificial Chromosome) on peut citer à titre illustratif le vecteur pCYPAC6 (numéro d'accès GenBank : AF133437). De façon tout à fait préférée, un vecteur selon l'invention est pSPM75.

Est également décrit un système d'expression comprenant un vecteur d'expression approprié et une cellule hôte permettant l'expression d'un ou plusieurs polypeptides tels que décrits ci-dessus dans un système biologique. Les vecteurs d'expression selon l'invention comprennent une séquence d'acide nucléique codant un ou plusieurs polypeptides tels que décrits selon l'invention et peuvent être destinés à l'expression des différents polypeptides selon l'invention dans diverses cellules hôtes bien connues de l'homme du métier. A titre d'exemple, on peut citer les systèmes d'expression procaryote comme les systèmes d'expression chez la bactérie *E. coli,* les sytèmes d'expression eucaryotes, comme le système d'expression baculovirus permettant une expression dans des cellules d'insectes, les sytèmes d'expression permettant une expression dans des cellules de levures, les sytèmes d'expression permettant une expression dans des cellules de mammifères, notamment des cellules humaines. Les vecteurs d'expression utilisables dans de tels systèmes sont bien connus de l'homme du métier, en ce qui concerne les cellules procaryotes, on peut citer à titre illustratif les vecteurs d'expression chez *E. coli* par exemple, de la famille pET commercialisés par la société Stratagene (LaJolla, Californie, USA), les vecteurs de la famille GATEWAY commercialisés par la société Invitrogen (Carlsbad, Californie, USA), les vecteurs de la famille pBAD commercialisés par la société Invitrogen (Carlsbad, Californie, USA), les vecteurs de la famille pMAL commercialisés par la société New England Bioloabs Inc. (Beverly, Massachussetts, USA), les vecteurs d'expression inductibles par le rhamnose cités dans la publication Wilms B *et al.*, 2001 et leurs dérivés, on peut également citer les vecteurs d'expression chez *Streptomyces* comme par exemple les vecteurs pIJ4123, pIJ6021, pPM927, pANT849, pANT 850, pANT 851, pANT1200, pANT1201, pANT1202 et leurs dérivés (Kieser *et al.*, 2000). En ce qui concerne les cellules de levures, on peut citer à titre illustratif le vecteur pESC commercialisé par la société Stratagene (LaJolla, Californie, USA). En ce qui concerne le système d'expression baculovirus permettant une expression dans des cellules d'insectes on peut citer à titre illustratif le vecteur BacPAK6 commercialisé par la société BD Biosciences Clontech, (Palo Alto, Californie, USA). En ce qui concerne les cellules de mammifères, on peut citer à titre illustratif des vecteurs comprenant le promoteur des gènes précoces du virus CMV (Cytomegalovirus) (par exemple le vecteur pCMV et ses dérivés commercialisés par la société Stratagene (LaJolla, Californie, USA), ou le promoteur des gènes immédiats (SV40 early promoter) du virus simien vacuolisant SV40 (par exemple le vecteur pSG5 commercialisé par la société Stratagene (LaJolla, Californie, USA).

L'invention est également relative à un procédé de production d'un polypeptide tel que décrit selon l'invention ledit procédé comprenant les étapes suivantes:
a) insérer un acide nucléique codant ledit polypeptide dans un vecteur approprié ;
b) cultiver, dans un milieu de culture approprié, une cellule hôte préalablement transformée ou transfectée avec le vecteur de l'étape a) ;
c) récupérer le milieu de culture conditionné ou un extrait cellulaire, par exemple par sonication ou par choc osmotique ;
d) séparer et purifier à partir dudit milieu de culture ou encore à partir de l'extrait cellulaire obtenu à l'étape c), ledit polypeptide ;
e) le cas échéant, caractériser le polypeptide recombinant produit.

Un polypeptide recombinant selon l'invention peut être purifié par passage sur une série appropriée de colonnes de chromatographie, selon les méthodes connues de l'homme de l'art et décrites par exemple dans F.Ausubel et al (2002). On peut citer à titre illustratif la technique du « Tag-Histidine », qui consiste à ajouter une courte séquence poly-histidine au polypeptide à produire, ce dernier pouvant ensuite être purifié sur colonne de nickel. Un polypeptide selon l'invention peut être également préparé par les techniques de synthèse *in vitro*. A titre illustratif de telles techniques, un polypeptide selon l'invention pourra être préparé grâce au système « rapid translation system (RTS)», commercialisé notamment par la société Roche Diagnostics France S.A, Meylan, France.

Un autre aspect de l'invention concerne des cellules hôtes dans laquelle a été introduit au moins un polynucléotide et/ou au moins un vecteur d'expression selon l'invention.

Est également décrit un microorganisme produisant de la spiramycine I mais ne produisant pas de spiramycine II et III. Ce microorganisme comprend l'ensemble des gènes nécessaires à la biosynthèse de la spiramycine I mais ne produit pas de spiramycine II et III car le gène comprenant la séquence correspondant à SEQ ID N° 13 ou l'un de ses variants ou l'une des séquences dérivées de celles-ci en raison de la dégénérescence du code génétique et codant un polypeptide de séquence SEQ ID N° 14 ou l'un de ses variants n'est pas exprimé ou a été rendu inactif. L'inactivation de cette protéine peut être effectuée par toute méthode connue de l'homme du métier, par exemple par mutagénèse dans le gène codant ladite protéine ou par l'expression d'un ARN anti-sens complémentaire de l'ARN messager codant ladite protéine, étant entendu que si l'expression de *orf5** est affectée par cette manipulation, il sera nécessaire de procéder à une autre modification pour que le gène *orf5** soit correctement exprimé. La mutagénèse peut être effectuée dans la séquence codante ou dans une séquence non codante de manière à rendre inactive la protéine codée ou à en empêcher son expression ou sa traduction. La mutagénèse peut être effectuée par mutagénèse dirigée, par remplacement de gène ou toute autre méthode connue de l'homme du métier. Les conditions convenables d'une telle mutagénèse peuvent par exemple être adaptées selon l'enseignement contenu dans les ouvrages de Kieser, T *et al* (2000) et Ausubel *et al.,* 2002. La mutagénèse peut être effectuée *in vitro* ou *in situ*, par suppression, substitution, délétion et/ou addition d'une ou plusieurs bases dans le gène considéré ou par inactivation génique. Le microorganisme peut être également obtenu en exprimant les gènes de la voie de biosynthèse de la spiramycine sans que ceux-ci ne comprennent le gène comprenant la séquence SEQ ID N° 13 ou l'un de ses variants ou l'une des séquences dérivées de celles-ci en raison de la dégénérescence du code génétique et codant un polypeptide de séquence SEQ ID N° 14 ou l'un de ses variants. Préférentiellement, le microorganisme est une bactérie du genre *Streptomyces.* Plus préférentiellement, le microorganisme produisant de la spiramycine I mais ne produisant pas de spiramycine II et III est obtenu à partir d'un microorganisme de départ produisant les spiramycines I, II et III. Encore plus préférentiellement, le microorganisme est obtenu par mutagénèse dans un gène comprenant la séquence correspondant à SEQ ID N° 13 ou l'un de ses variants ou l'une des séquences dérivées de celles-ci en raison de la dégénérescence du code génétique et codant un polypeptide de séquence SEQ ID N° 14 ou l'un de ses variants ayant la même fonction. Encore plus préférentiellement, cette mutagénèse est effectuée par inactivation génique. De manière préférée le microorganisme est obtenu à partir d'une souche de *S*. *ambofaciens* produisant les spiramycines I, II et III dans laquelle est effectuée une inactivation génique du gène comprenant la séquence correspondant à SEQ ID N° 13 ou l'une des séquences dérivées de celle-ci en raison de la dégénérescence du code génétique. De manière tout à fait préférée, l'inactivation génique est effectuée par délétion en phase du gène ou d'une partie du gène comprenant la séquence correspondant à SEQ ID N° 13 ou l'une des séquences dérivées de celle-ci en raison de la dégénérescence du code génétique: A titre illustratif, la souche SPM502 (*orf6*::att1*, cf. exemple 14) peut être citée comme un microorganisme produisant de la spiramycine I mais ne produisant pas de spiramycine II et III.

Est également décrit un microorganisme produisant de la spiramycine I mais ne produisant pas de spiramycine II et III tel que décrit ci-dessus caractérisé en ce en ce qu'il surexprime en outre :
- un gène susceptible d'être obtenu par amplification en chaîne par polymérase en utilisant le couple d'amorces de séquence suivante : 5' AAGCTTGTGTGCCCGGTGTACCTGGGGAGC 3' (SEQ ID N° 138) et 5' GGATCCCGCGACGGACACGACCGCCGCGCA 3' (SEQ ID N° 139) et comme matrice le cosmide pSPM36 ou l'ADN total de *Streptomyces ambofaciens,* plus préférentiellement il s'agit du gène de séquence codante SEQ ID N° 141.
Un exemple de séquence d'un tel gène est donné en SEQ ID N° 111 (ADN), cependant cette séquence est partielle puisqu'elle ne comprend pas la partie 3' de la séquence codante correspondante. La traduction en protéine de cette partie de séquence codante est donnée en SEQ ID N° 112. L'homme du métier pourra aisément la compléter notamment en utilisant l'enseignement présenté dans l'exemple 24. La séquence indéterminée dans SEQ ID N° 111 a été déterminée dans un second temps et la séquence complète de cette orf (*orf28c*) est donnée en SEQ ID N° 141. La traduction en protéine de cette séquence codante est donnée en SEQ ID N° 142. Il est présenté dans l'exemple 24 une méthode pour cloner le gène *orf28c* et pour fabriquer un vecteur d'expression permettant l'expression de *orf28c.* Il est également montré dans cet exemple que la surexpression du gène *orf28c* dans la souche OSC2 conduit à l'amélioration de la production en spiramycines de cette souche. La surexpression du gène *orf28c* peut être obtenue en augmentant le nombre de copies de ce gène et/ou en plaçant un promoteur plus actif que le promoteur sauvage. De façon préférée, la surexpression dudit gène est obtenue en apportant dans le microorganisme une construction d'ADN recombinant, permettant la surexpression de ce gène. De façon préférée, cette construction d'ADN recombinant augmente le nombre de copie dudit gène et permet d'obtenir une surexpression dudit gène. Dans cette construction d'ADN recombinant, la séquence codante du gène peut être placée sous le contrôle d'un promoteur plus actif que le promoteur sauvage. On peut citer à titre d'illustration le promoteur ptrc actif chez *Streptomyces ambofaciens* (Amann, *E. et al.*, 1988) ainsi que le promoteur ermE*. Ainsi, de façon préférée, la ou les copies du gène *orf28c* apportée sont placées sous le contrôle du promoteur ermE* comme c'est le cas dans la construction pSPM75 (cf. exemple 24).

Un autre aspect de l'invention concerne l'utilisation d'une séquence nucléotidique selon l'invention pour améliorer la production en spiramycine d'un microorganisme. Ainsi, l'invention concerne un microorganisme mutant producteur de spiramycine caractérisé en ce qu'il surexprime au moins un gène comprenant une séquence telle que définie selon l'invention. Ainsi la surexpression du gène considéré peut être obtenue en apportant dans un microorganisme producteur de spiramycine une construction d'ADN recombinant selon l'invention, permettant la surexpression de ce gène. En effet, certaines étapes de la biosynthèse de la spiramycine sont limitantes et si l'on exprime une ou plusieurs protéines plus actives ou un niveau d'expression plus important de la ou des protéines sauvages impliquées dans ces étapes limitantes, on peut améliorer la production de la spiramycine. Une augmentation du taux de production de la tylosine a par exemple été obtenue chez *Streptomyces fradiae* par duplication du gène codant une methyl transférase limitante convertissant la macrocine en tylosine (Baltz R, 1997). Préférentiellement, ces microorganismes mutants améliorés pour leur production en macrolides sont des bactéries du genre *Streptomyces.* De façon préférée, le microorganisme surexprime le gène comprenant une séquence correspondant à SEQ ID N° 111 ou 141. La séquence donnée en SEQ ID N° 111 est partielle, cependant l'homme du métier pourra aisément la compléter notamment en utilisant l'enseignement présenté dans l'exemple 24. La séquence indéterminée dans SEQ ID N° 111 a été déterminée dans un second temps et la séquence complète de cette orf (*orf28c*) est donnée en SEQ ID N° 141. La traduction en protéine de cette séquence codante est donnée en SEQ ID N° 142. Il est ainsi présenté l'exemple 24 une méthode pour cloner le gène *orf28c* et pour fabriquer un vecteur d'expression permettant l'expression de *orf28c.* Il est également montré dans cet exemple que la surexpression du gène *orf28c* dans la souche OSC2 conduit à l'amélioration de la production en spiramycines de cette souche. La surexpression du gène *orf28c* peut être obtenue en augmentant le nombre de copies de ce gène et/ou en plaçant un promoteur plus actif que le promoteur sauvage. De façon préférée, la surexpression du gène *orf28c* est obtenue en apportant dans le microorganisme une construction d'ADN recombinant, permettant la surexpression de ce gène. De façon préférée, cette construction d'ADN recombinant augmente le nombre de copie du gène *orf28c* et permet d'obtenir une surexpression du gène *orf28c.* Dans cette construction d'ADN recombinant, la séquence codante de *orf28c* peut être placée sous le contrôle d'un promoteur plus actif que le promoteur sauvage. On peut citer à titre d'illustration le promoteur ptrc actif chez *Streptomyces ambofaciens* (Amann, E. *et al.,* 1988) ainsi que le promoteur ermE*. Ainsi, de façon préférée, la ou les copies du gène *orf28c* apportée sont placées sous le contrôle du promoteur ermE* comme c'est le cas dans la construction pSPM75 (cf. exemple 24).

Un autre aspect de l'invention concerne un procédé de production de spiramycine utilisant les microorganismes décrits au paragraphe précédent. Ce procédé consiste à cultiver dans un milieu de culture approprié un microorganisme défini dans le paragraphe précédent, récupérer le milieu de culture conditionné ou un extrait cellulaire, séparer et purifier à partir dudit milieu de culture ou encore à partir de l'extrait cellulaire obtenu à l'étape précédente la spiramycine produite. Les conditions de culture de tels microorganismes pourront être déterminées selon des techniques bien connues de l'homme du métier. Le milieu de culture pourra par exemple être le milieu MP5 ou le milieu SL11 pour les *Streptomyces* et notamment pour *Streptomyces ambofaciens* (Pernodet et al., 1993). L'homme du métier pourra notamment se référer à l'ouvrage de Kieser et al. 2000 en ce qui concerne la culture des *Streptomyces.* La spiramycine produite peut être récupérée par toutes techniques connues de l'homme du métier. L'homme du métier pourra se référer, par exemple, aux techniques enseignées dans le brevet américain US 3,000,785 et plus particulièrement aux méthodes d'extraction des spiramycines décrites dans ce brevet. Préférentiellement, les microorganismes utilisés dans un tel procédé sont des bactéries du genre *Streptomyces.* Plus préférentiellement, les microorganismes mutants améliorés pour leur production en spiramycines sont des souches de *S. ambofaciens.*

Est également décrit un ADN recombinant caractérisé en ce qu'il comprend :
- un polynucléotide susceptible d'être obtenu par amplification en chaîne par polymérase en utilisant le couple d'amorces de séquence suivante : 5' AAGCTTGTGTGCCCGGTGTACCTGGGGAGC 3' (SEQ ID N° 138) et 5' GGATCCCGCGACGGACACGACCGCCGCGCA 3' (SEQ ID N° 139) et comme matrice le cosmide pSPM36 ou l'ADN total de *Streptomyces ambofaciens,* plus préférentiellement il s'agit d'un polynucléotide de séquence SEQ ID N° 141.
Préférentiellement ; cet ADN recombinant est un vecteur. Encore plus préférentiellement, le vecteur est choisi parmi les bactériophages, les plasmides, les phagemides, les vecteurs intégratifs, les fosmides, les cosmides, les vecteurs navettes, les BAC (Bacterial Artificial Chromosome) ou les PAC (P1-derived Artificial Chromosome). A titre illustratif on peut citer comme bactériophages le phage lambda et le phage M13. Comme plasmides on peut citer les plasmides se répliquant chez *E. coli* par exemple pBR322 et ses dérivés, pUC18 et ses dérivés, pUC19 et ses dérivés, pGB2 et ses dérivés (Churchward G. *et al.,* 1984), pACYC177 (numéro d'accès GenBank : X06402) et ses dérivés, pACYC184 (numéro d'accès GenBank : X06403) et ses dérivés. On peut également citer les plasmides se répliquant chez *Streptomyces* comme par exemple pIJ101 et ses dérivés, pSG5 et ses dérivés, SLP1 et ses dérivés, SCP2* et ses dérivés *(Kieser et al.* 2000). Comme phagemides on peut citer à titre illustratif pBluescript II et ses dérivés (commercialisés notamment par la société Stratagene (LaJolla, Californie, USA)), pGEM-T et ses dérivés (commercialisé par la société Promega (Madison, Wisconcin, USA)), λZAPII et ses dérivés (commercialisés notamment par la société Stratagene (LaJolla, Californie, USA)). Comme vecteurs intégratifs on peut citer à titre illustratif, les vecteurs intégratifs chez *Streptomyces* comme par exemple ceux dérivés de SLP1 (Kieser *et al*, 2000), ceux dérivés de pSAM2 (Kieser *et al*, 2000), les vecteurs utilisant les systèmes d'intégration des phages PhiC31 *(Kieser et al,* 2000) (par exemple pSET152 (Bierman *et al.,* 1992)) ou de VWB (Van Mellaert L, *et al.* 1998), ainsi que les vecteurs utilisant le système d'intégration de IS117 (Kieser et al. 2000). Comme fosmides on peut citer à titre illustratif le fosmide pFOS1 (commercialisé par la société New England Bioloabs Inc., Beverly, Massachussetts, USA) et ses dérivés. Comme cosmides on peut citer à titre illustratif le cosmide SuperCos et ses dérivés (commercialisés notamment par la société Stratagene (LaJolla, Californie, USA)), le cosmide pWED15 (Wahl *et al.*, 1987) et ses dérivés. Comme vecteurs navettes on peut citer à titre illustratif les plasmides navettes *E*. *coli* / *Streptomyces* comme par exemple pIJ903 et ses dérivés, la série des plasmides pUWL, pCAO106, pWHM3, pOJ446 et leurs dérivés *(Kieser et al.* 2000), les BAC navettes *E. coli* / *Streptomyces* comme par exemple ceux décrits dans la demande de brevet WO 01/40497. Comme BAC (Bacterial Artificial Chromosome) on peut citer à titre illustratif le BAC pBeloBAC11 (numéro d'accès GenBank :U51113). Comme PAC (P1-derived Artificial Chromosome) on peut citer à titre illustratif le vecteur pCYPAC6 (numéro d'accès GenBank : AF133437). Plus préférentiellement, cet ADN recombinant est un vecteur d'expression. Les vecteurs d'expression utilisables dans différents systèmes d'expression sont bien connus de l'homme du métier, en ce qui concerne les cellules procaryotes, on peut citer à titre illustratif les vecteurs d'expression chez *E. coli* par exemple, de la famille pET commercialisés par la société Stratagene (LaJolla, Californie, USA), les vecteurs de la famille GATEWAY commercialisés par la société Invitrogen (Carlsbad, Californie, USA), les vecteurs de la famille pBAD commercialisés par la société Invitrogen (Carlsbad, Californie, USA), les vecteurs de la famille pMAL commercialisés par la société New England Bioloabs Inc. (Beverly, Massachussetts, USA), les vecteurs d'expression inductibles par le rhamnose cités dans la publication Wilms B *et al.,* 2001 et leurs dérivés, on peut également citer les vecteurs d'expression chez *Streptoinyces* comme par exemple les vecteurs pIJ4123, pIJ6021, pPM927, pANT849, pANT 850, pANT 851, pANT1200, pANT1201, pANT1202 et leurs dérivés (Kieser *et al.,* 2000). En ce qui concerne les cellules de levures, on peut citer à titre illustratif le vecteur pESC commercialisé par la société Stratagene (LaJolla, Californie, USA). En ce qui concerne le système d'expression baculovirus permettant une expression dans des cellules d'insectes on peut citer à titre illustratif le vecteur BacPAK6 commercialisé par la société BD Biosciences Clontech, (Palo Alto, Californie, USA). En ce qui concerne les cellules de mammifères, on peut citer à titre illustratif des vecteurs comprenant le promoteur des gènes précoces du virus CMV (Cytomegalovirus) (par exemple le vecteur pCMV et ses dérivés commercialisés par la société Stratagene (LaJolla, Californie, USA), ou le promoteur des gènes immédiats (SV40 early promoter) du virus simien vacuolisant SV40 (par exemple le vecteur pSG5 commercialisé par la société Stratagene (LaJolla, Californie, USA). Un autre aspect de l'invention concerne des cellules hôtes dans laquelle a été introduit un vecteur d'expression décrit dans ce paragraphe.

Un autre aspect de l'invention concerne un microorganisme producteur d'au moins une spiramycine caractérisé en ce qu'il surexprime :
- un gène susceptible d'être obtenu par amplification en chaîne par polymérase (PCR) en utilisant le couple d'amorces de séquence suivante : 5' AAGCTTGTGTGCCCGGTGTACCTGGGGAGC 3' (SEQ ID N° 138) et 5' GGATCCCGCGACGGACACGACCGCCGCGCA 3' (SEQ ID N° 139) et comme matrice le cosmide pSPM36 ou l'ADN total de *Streptomyces ambofaciens,* plus préférentiellement il s'agit du gène de séquence codante SEQ ID N° 141.
   Un exemple de séquence d'un tel gène est donné en SEQ ID N° 111 (ADN), cependant cette séquence est partielle puisqu'elle ne comprend pas la partie 3' de la séquence codante correspondante. La traduction en protéine de cette partie de séquence codante est donnée en SEQ ID N° 112. L'homme du métier pourra aisément la compléter notamment en utilisant l'enseignement présenté dans l'exemple 24. Il est ainsi présenté dans cet exemple une méthode pour cloner le gène *orf28c* et pour fabriquer un vecteur d'expression permettant l'expression de *orf28c.* Il est également montré dans cet exemple que la surexpression du gène *orf28c* dans la souche OSC2 conduit à l'amélioration de la production en spiramycines de cette souche. De façon préférée, le microorganisme surexprimant
- un gène susceptible d'être obtenu par amplification en chaîne par polymérase (PCR) en utilisant le couple d'amorces de séquence suivante : 5' AAGCTTGTGTGCCCGGTGTACCTGGGGAGC 3' (SEQ ID N° 138) et 5' GGATCCCGCGACGGACACGACCGCCGCGCA 3' (SEQ ID N° 139) et comme matrice le cosmide pSPM36 ou l'ADN total de *Streptomyces ambofaciens,* plus préférentiellement il s'agit du gène de séquence codante SEQ ID N° 141
   est une bactérie du genre *Streptomyces,* de manière encore plus préférée, il s'agit d'une bactérie de l'espèce *Streptomyces ambofaciens.* De façon préférée, la surexpression dudit gène est obtenue par transformation dudit microorganisme par un vecteur d'expression, de manière tout à fait préféré, la souche de microorganisme est la souche OSC2/pSPM75(2) déposée auprès de la Collection Nationale de Cultures de Microorganismes (CNCM) Institut Pasteur, 25, rue du Docteur Roux 75724 Paris Cedex 15, France, le 6 octobre 2003 sous le numéro d'enregistrement 1-3101.

Un autre aspect de l'invention concerne un procédé de production de spiramycine(s) utilisant les microorganismes décrits au paragraphe précédent. Ce procédé consiste à cultiver dans un milieu de culture approprié un microorganisme défini dans le paragraphe précédent, récupérer le milieu de culture conditionné ou un extrait cellulaire, séparer et purifier à partir dudit milieu de culture ou encore à partir de l'extrait cellulaire obtenu à l'étape précédente le ou les spiramycines produites. Les conditions de culture de tels microorganismes pourront être déterminées selon des techniques bien connues de l'homme du métier. Le milieu de culture pourra par exemple être le milieu MP5 ou le milieu SL11 pour les *Streptomyces* et notamment pour *Streptomyces ambofaciens* (Pernodet et al., 1993). L'homme du métier pourra notamment se référer à l'ouvrage de Kieser et al. 2000 en ce qui concerne la culture des *Streptomyces.* Le ou les spiramycines produites peuvent être récupérés par toutes techniques connues de l'homme du métier. L'homme du métier pourra se référer, par exemple, aux techniques enseignées dans le brevet américain US 3,000,785 et plus particulièrement aux méthodes d'extraction des spiramycines décrites dans ce brevet. Préférentiellement, les microorganismes utilisés dans un tel procédé sont des bactéries du genre *Streptonzyces.* Plus préférentiellement, les microorganismes sont des souches de *S*. *ambofaciens.*

Un autre aspect de l'invention concerne une souche de *Streptomyces ambofaciens* transformée par un vecteur défini dans le paragraphe précédent.

Un autre aspect de l'invention concerne un polypeptide caractérisé en ce que sa séquence comprend la séquence SEQ ID N° 112. L'invention est également relative à un polypeptide caractérisé en ce que sa séquence correspond à la séquence traduite de la séquence codante :
- d'un gène susceptible d'être obtenu par amplification en chaîne par polymérase (PCR) en utilisant le couple d'amorces de séquence suivante : 5' AAGCTTGTGTGCCCGGTGTACCTGGGGAGC 3' (SEQ ID N° 138) et 5' GGATCCCGCGACGGACACGACCGCCGCGCA 3' (SEQ ID N° 139) et comme matrice le cosmide pSPM36 ou l'ADN total de *Streptomyces ambofaciens,* plus préférentiellement il s'agit du gène de séquence codante SEQ ID N° 141.
Préferentiellement ces polypeptides sont exprimés à l'état naturel par une bactérie du genre *Streptomyces,* plus préférentiellement, ces polypeptides sont impliqués dans la biosynthèse des spiramycines.

Un autre aspect de l'invention concerne un vecteur d'expression permettant l'expression d'un polypeptide tel que défini dans le paragraphe précédent chez *Streptomyces ambofaciens.* Des exemples de vecteurs d'expression utilisables chez *Streptomyces* ont été donnés ci-dessus. Préférentiellemnt, le vecteur d'expression en question est le plasmide pSPM75.

### LISTE DES FIGURES

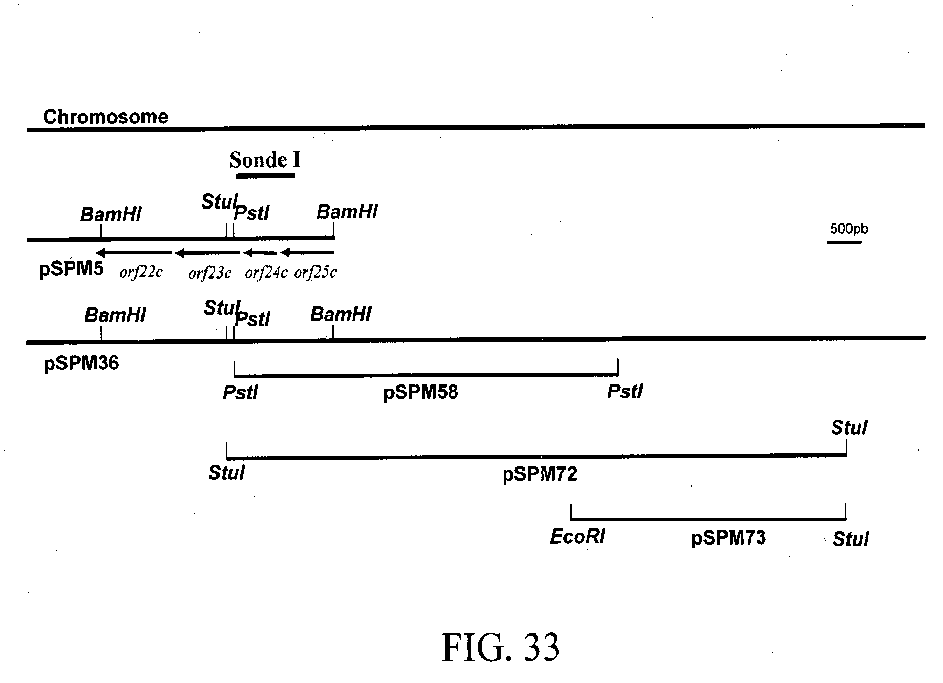

Figure 1 : Structure chimique des spiramycines I, II et III.
Figure 2 : Cosmides utilisés pour le séquençage de la région.
Figure 3 : Organisation d'un groupe de gènes impliqués dans la voie de biosynthèse des spiramycines.
Figure 4 : Voie de biosynthèse proposée du mycarose.
Figure 5 : Voie de biosynthèse proposée du mycaminose.
Figure 6 : Voie de biosynthèse proposée de la forosamine.
Figure 7 : Ordre préférentiel d'ajout des sucres dans la molécule de spiramycine et intermédiaires.
Figure 8 : Voie de biosynthèse proposée du méthoxymalonyl chez *S. ambofaciens.* Cette voie est proposée par analogie avec la biosynthèse du méthoxymalonyl chez *Streptomyces hygroscopicus var. ascomyceticus* (Wu,K. *et al*., 2000).
Figure 9 : Etapes conduisant à l'inactivation d'un gène:
   A) Clonage du gène cible dans un vecteur se répliquant chez *E. coli* mais pas chez *Streptomyces*;
   B) Insertion de la cassette de résistance dans le gène cible (par clonage ou recombinaison entre courtes séquences identiques);
   C) Introduction du plasmide chez *Streptomyces ambofaciens* (par transformation ou conjugaison avec *E. coli*) et sélection des clones ayant intégré la cassette puis criblage de clones ayant perdu la partie vecteur pour avoir un remplacement de gène;
   D) Région du chromosome de la souche mutante dans laquelle le gène cible est inactivé par remplacement de gène.
Figure 10 : Etapes optionnelles suivant l'inactivation d'un gène selon la méthode décrite en figure 9, pouvant être conduites si une cassette excisable a été employée pour interrompre le gène cible :
   E) Introduction dans la souche mutante du plasmide pOSV508 portant les gènes *xis* et *int* de pSAM2 dont les produits vont permettre l'excision efficace par recombinaison spécifique de sites entre les séquences attL et attR bordant la cassette;
   F) Obtention de clones ayant perdu la cassette excisable et sensibles à l'antibiotique à laquelle la cassette conférait la résistance;
   G) Après croissance et sporulation sur milieu solide sans antibiotique le plasmide pOSV508 est perdu à haute fréquence. On peut ainsi obtenir des clones sensibles au thiostrepton dans lesquels le gène cible contient une délétion en phase. La séquence du gène cible délété peut être contrôlée par amplification PCR et séquençage du produit PCR.

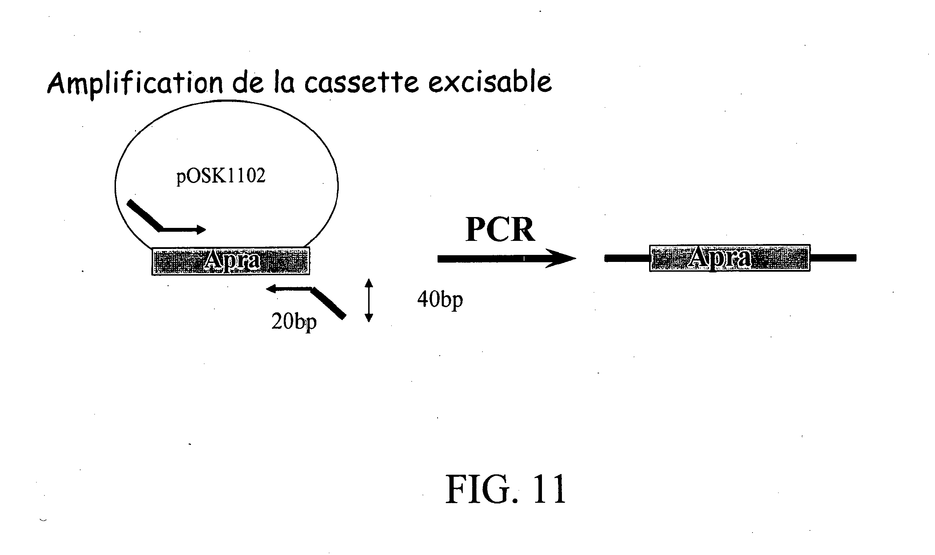

Figure 11 : Amplification de la cassette excisable dans le but de l'utiliser pour une expérience de recombinaison homologue. La technique de recombinaison homologue grâce à de courtes séquences homologues a été décrite par *(Chaveroche et al.,* 2000).
Les 39 ou 40 déoxy-nucléotides situés à l'extrémité 5' de ces oligonucléotides comportent une séquence correspondant à une séquence du gène à inactiver et les 20 déoxy-nucléotides situés le plus en 3' correspondent à la séquence d'une des extrémités de la cassette excisable. (ne fait pas partie de l'invention)

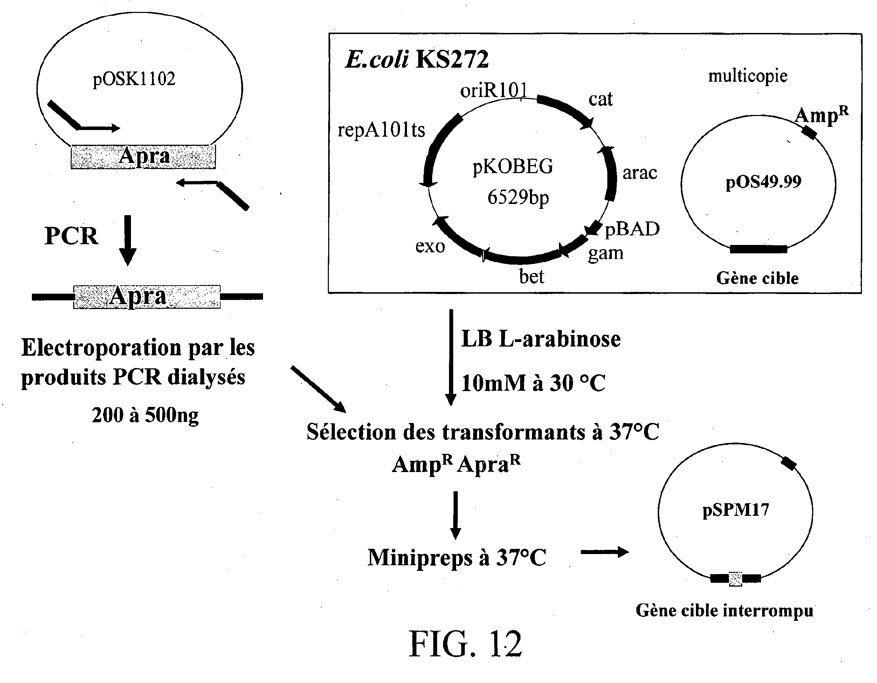

Figure 12 : Obtention d'une construction pour l'inactivation d'un gène cible grâce à la technique décrite par (Chaveroche *et al.,* 2000). (ne fait pas partie de l'invention)

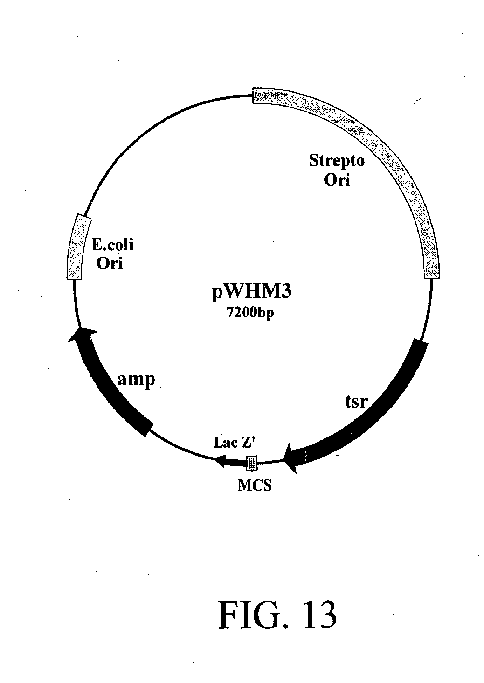

Figure 13 : Carte du plasmide pWHM3. Strepto ori : origine de réplication *Streptomyces*.

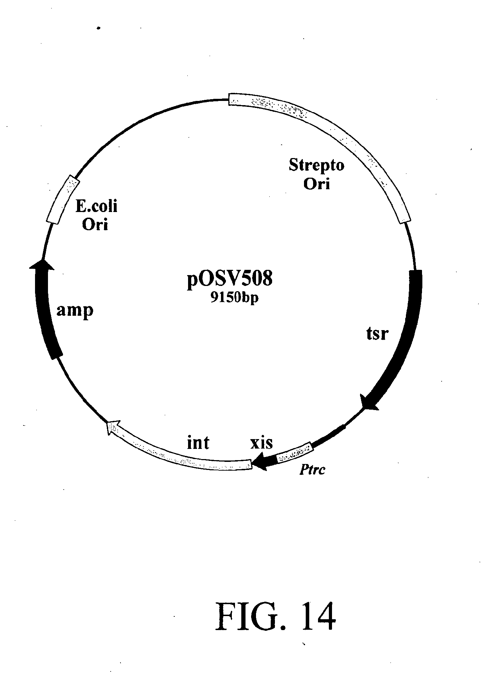

Figure 14 : Carte du plasmide pOSV508.

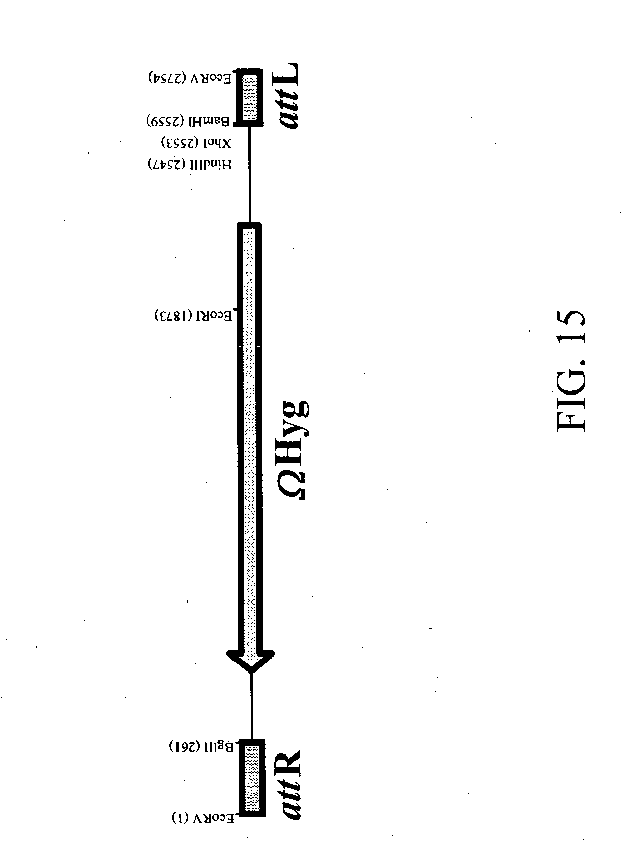

Figure 15 : Exemple de structure d'une cassette excisable. Celle-ci est constituée de la cassette Ω*hyg* (Blondelet-Rouault *et al..,* 1997) bordée des sites *attR* et *attL* (Raynal *et al..,* 1998), entre lesquels un événement de recombinaison permettra l'excision de la cassette, grâce à l'expression des gènes *xis* et *int.*

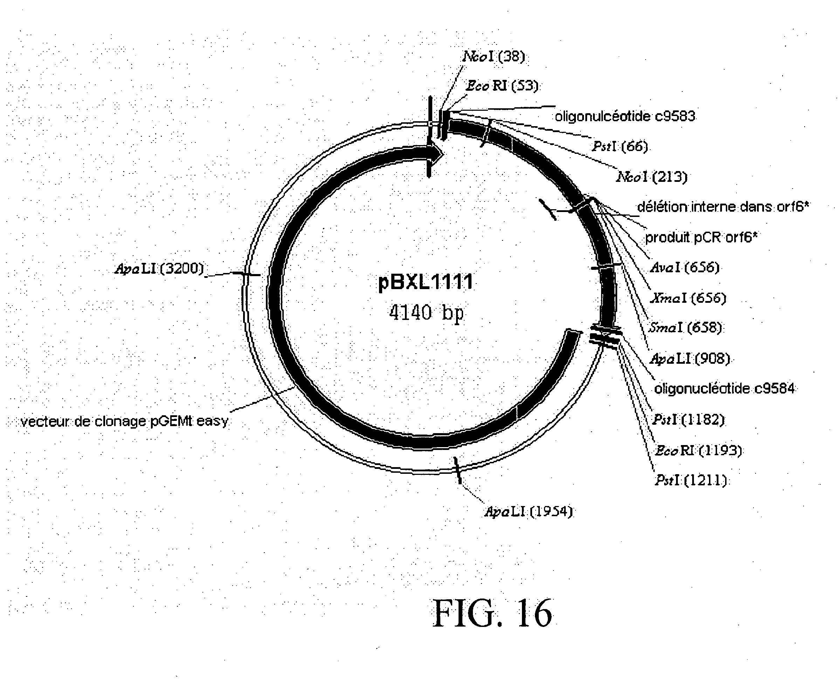

Figure 16 : Carte du plasmide pBXL1111. (ne fait pas partie de l'invention)

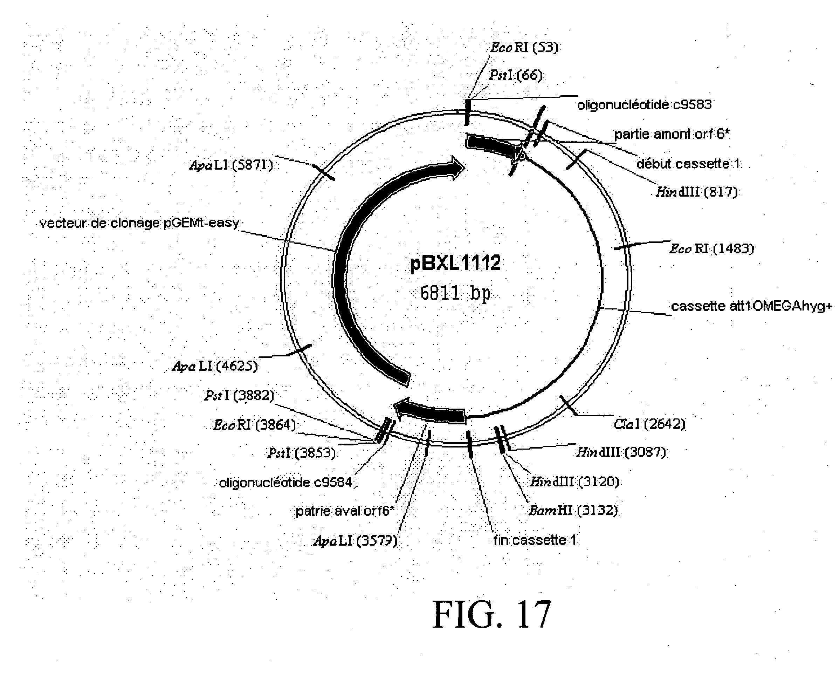

Figure 17 : Carte du plasmide pBXL1112. (ne fait pas partie de l'invention)

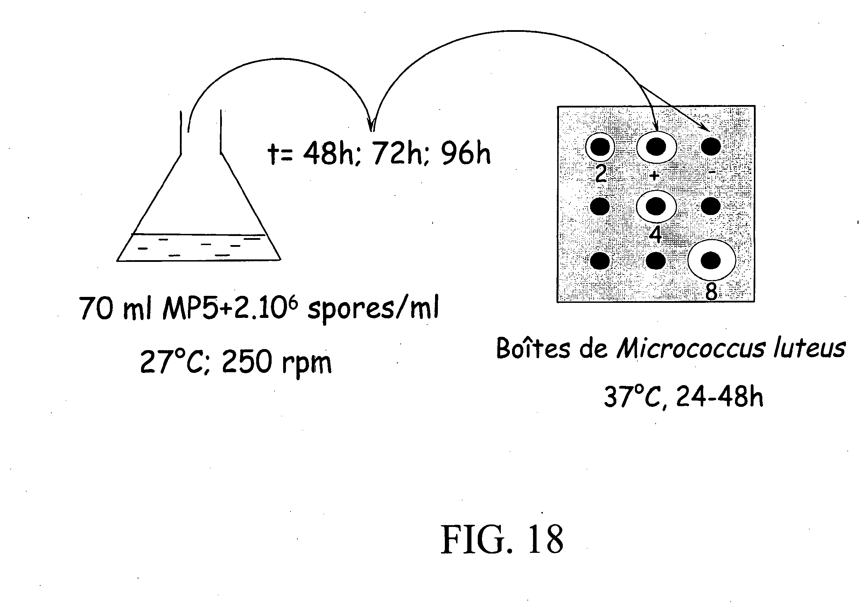

Figure 18 : Test microbiologique de production de spiramycine, basé sur la sensibilité d'une souche de *Micrococcus Luteus* à la spiramycine. La souche de *Micrococcus luteus* utilisée est une souche naturellement sensible à la spiramycine mais résistante à la congocidine. Les différentes souches de *Streptomyces* à tester ont été cultivées en erlenmeyers de 500 ml contenant 70 ml de milieu MP5, inoculés à une concentration initiale de 2.5 10⁶ spores/ml et mises à pousser à 27°C sous agitation orbitale de 250 rpm. Des prélèvements de moûts de fermentation ont été réalisés après 48, 72 et 96 heures de culture et centrifugés. Une dilution au dixième de ces surnageants dans du milieu de culture stérile est utilisée pour le test. La souche indicatrice de *Micrococcus luteus* résistante à la congocidine mais sensible à la spiramycine (Gourmelen *et al.,* 1998) a été cultivée dans une boîte carrée de 12x12 cm. Des disques en papier Whatman AA ont été imbibés de 70 µl de la dilution au dixième de chaque surnageant et déposés sur la surface de la boîte. Des disques imbibés d'une solution de spiramycine de différentes concentrations (2-4-8 µg/ml dans du milieu de culture MP5) sont utilisés comme gamme étalon. Les boîtes sont incubées à 37°C pendant 24 à 48h. Si le disque contient de la spiramycine, celle-ci diffuse dans l'agar et inhibe la croissance de la souche indicatrice de *Micrococcus luteus.* Cette inhibition crée un « halo » autour du disque, ce halo reflétant la zone où la souche de *Micrococcus luteus* n'a pas poussé. La présence de ce halo est donc une indication de la présence de spiramycine et permet de déterminer si la souche de *S. ambofaciens* en question est productrice ou non productrice de spiramycine. Une comparaison avec les diamètres d'inhibition obtenus pour la gamme étalon permet d'obtenir une indication de la quantité de spiramycine produite par cette souche.

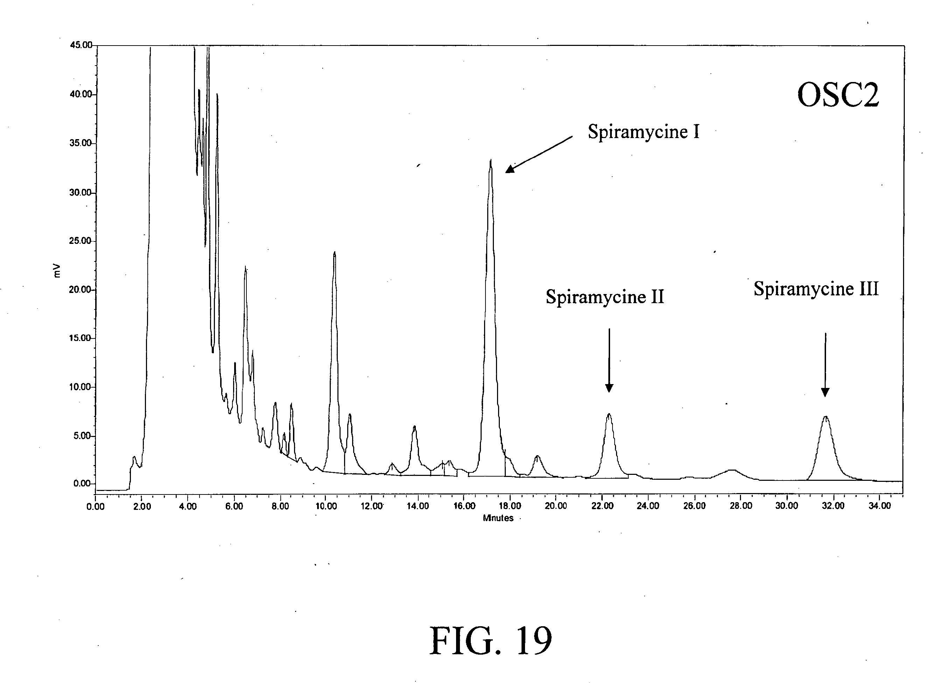

Figure 19 : Chromatogramme CLHP du surnageant filtré du milieu de culture de la souche OSC2.

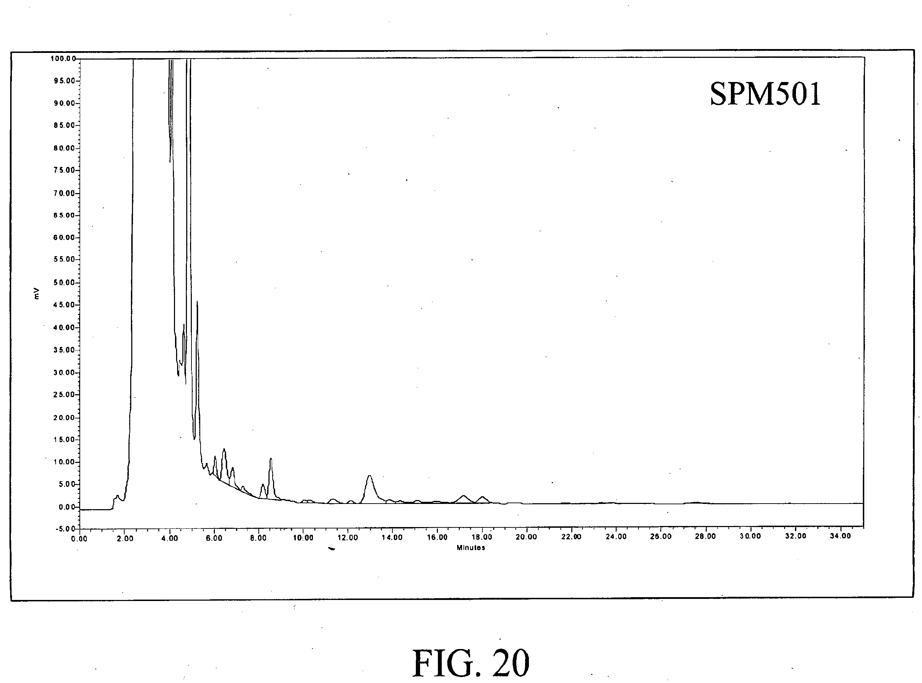

Figure 20 : Chromatogramme CLHP du surnageant filtré du milieu de culture de la souche SPM501. (ne fait pas partie de l'invention)

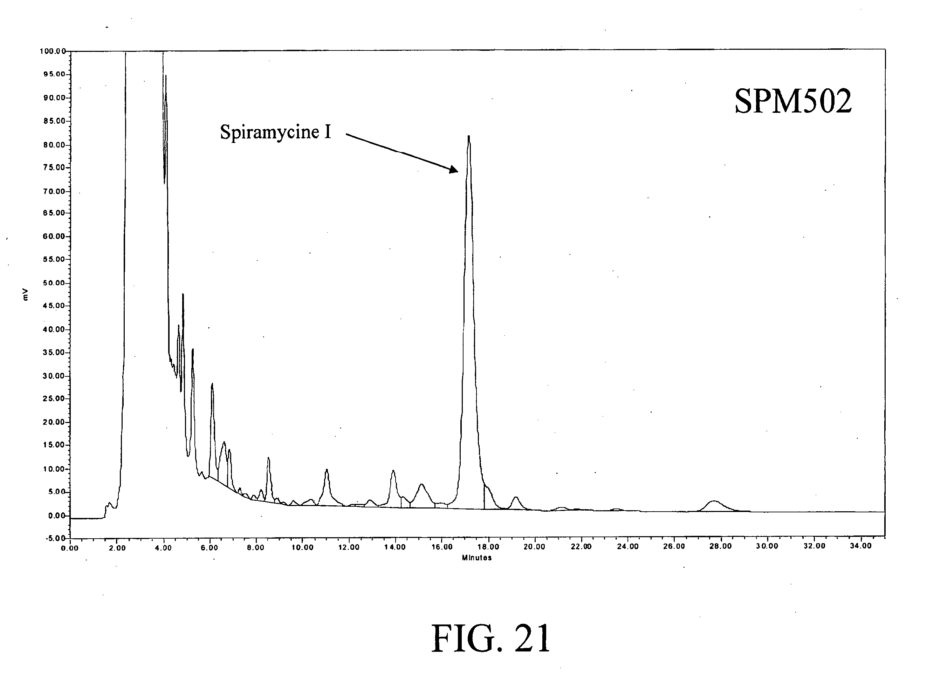

Figure 21 : Chromatogramme CLHP du surnageant filtré du milieu de culture de la souche SPM502. (ne fait pas partie de l'invention)

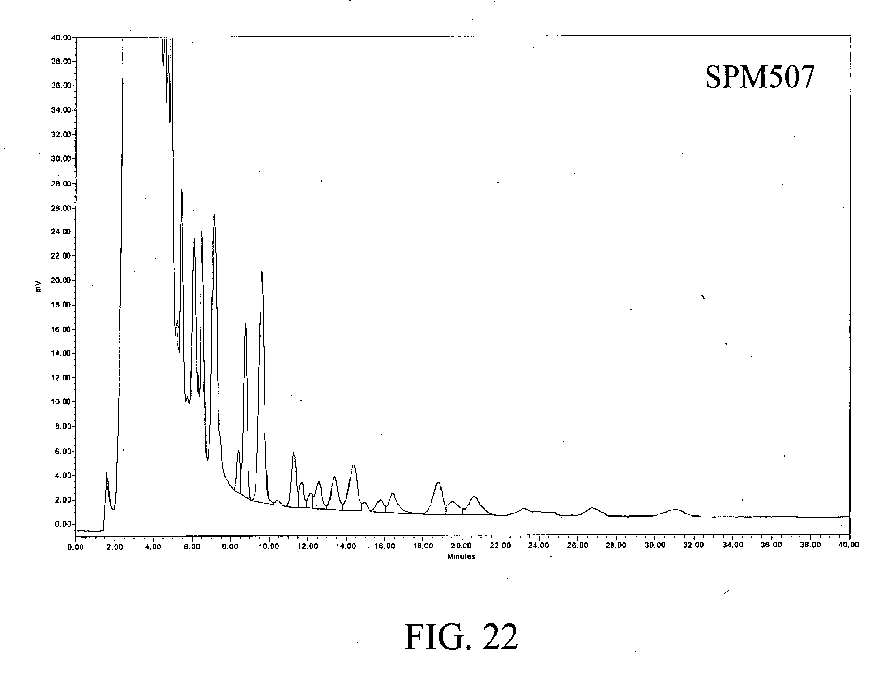

Figure 22 : Chromatogramme CLHP du surnageant filtré du milieu de culture de la souche SPM507. (ne fait pas partie de l'invention)

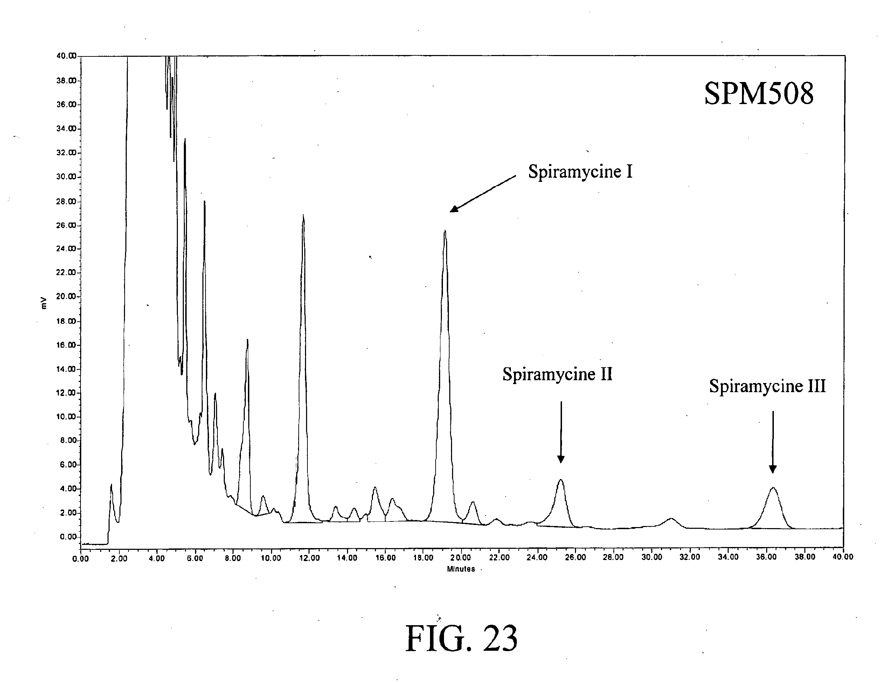

Figure 23 : Chromatogramme CLHP du surnageant filtré du milieu de culture de la souche SPM508. (ne fait pas partie de l'invention)

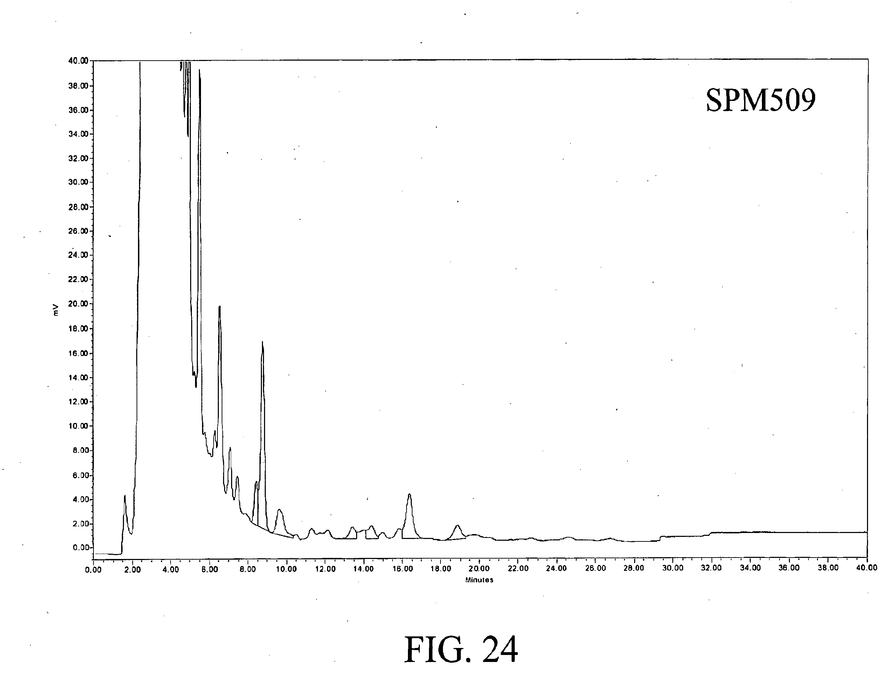

Figure 24 : Chromatogramme CLHP du surnageant filtré du milieu de culture de la souche SPM509. (ne fait pas partie de l'invention)

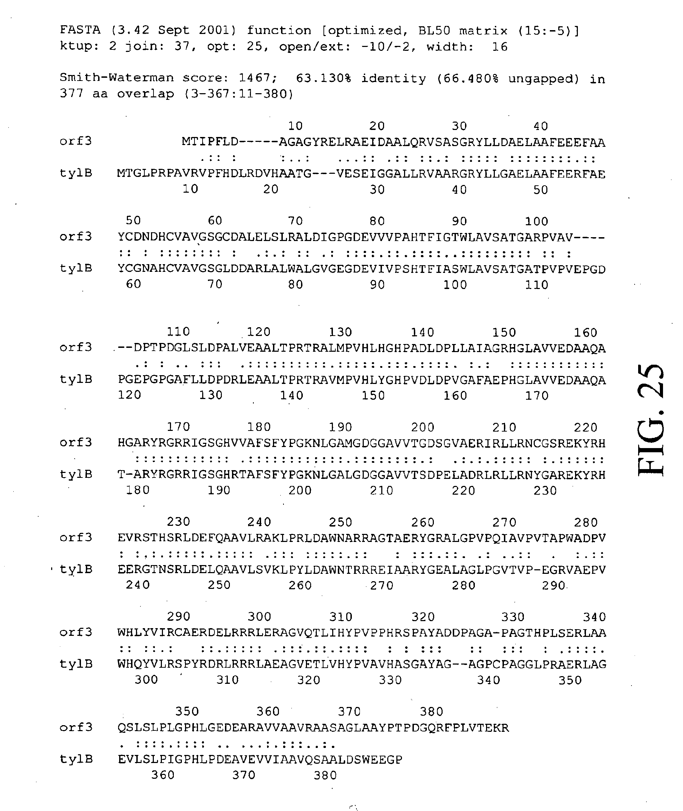

Figure 25 : Alignement de la protéine Orf3 (SEQ ID N° 29) avec la protéine TylB (SEQ ID N° 87) de *S. fradiae* réalisé grâce au programme FASTA.

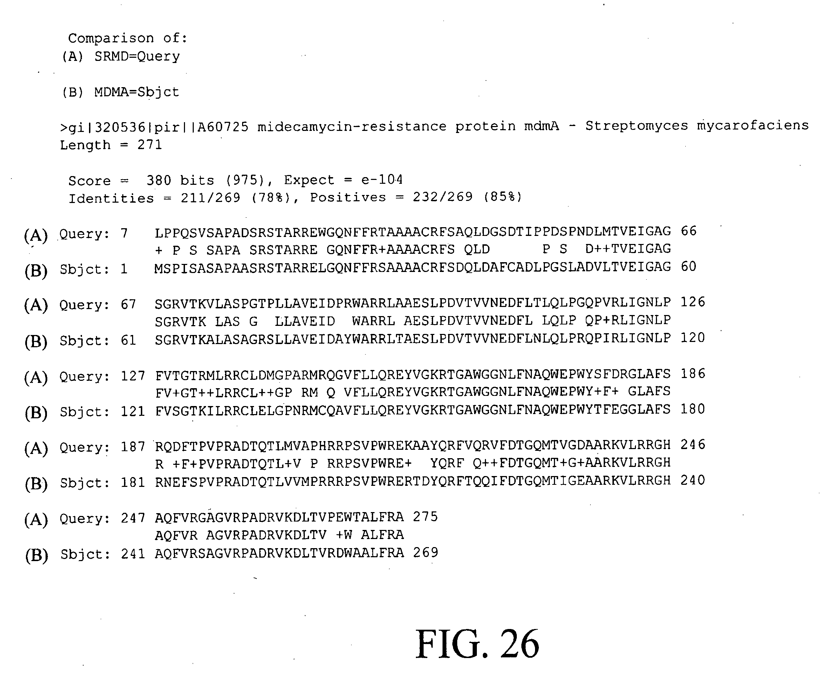

Figure 26 : Alignement de la protéine MdmA de *S. mycarofaciens* (SEQ ID N° 88) avec la protéine SrmD (SEQ ID N° 16) réalisé grâce au programme FASTA.

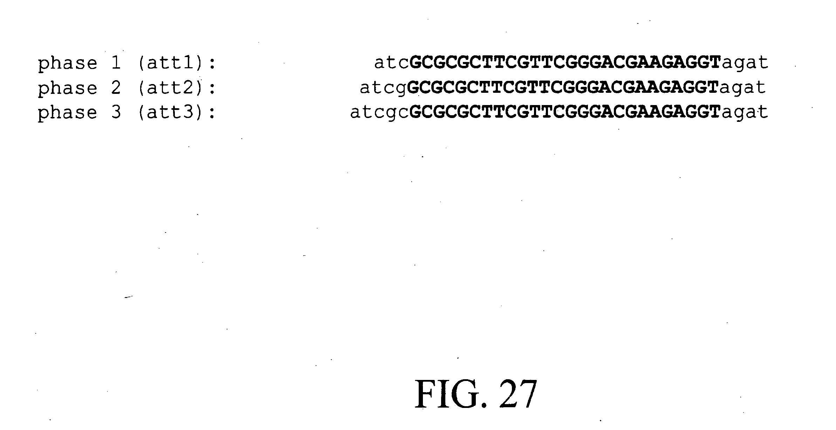

Figure 27 : Exemple de séquences des sites résiduels après excision de la cassette excisable. En gras est indiqué le site att26 minimum tel que défini dans *Raynal et al..,* 1998. La séquence de la phase 1 (att1) de 33 nucléotides est présentée en SEQ ID N° 104, la séquence de la phase 2 (att2) de 34 nucléotides est présentée en SEQ ID N° 105, la séquence de là phase 3 (att3) de 35 nucléotides est présentée en SEQ ID N° 95.

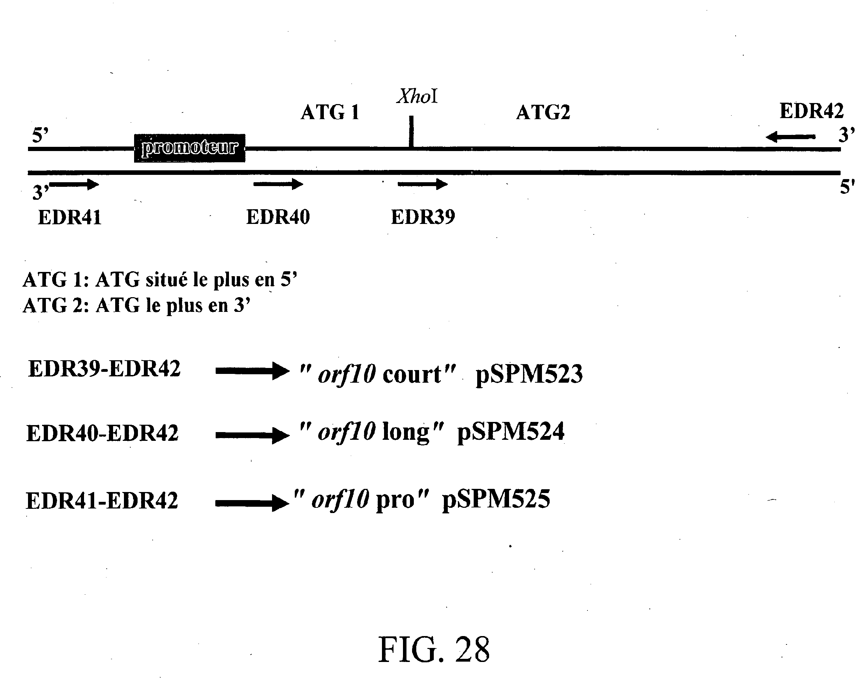

Figure 28 : Représentation schématique du gène *orf10,* localisation des amorces PCR utlisées et construction obtenue avec chaque couple d'amorces.

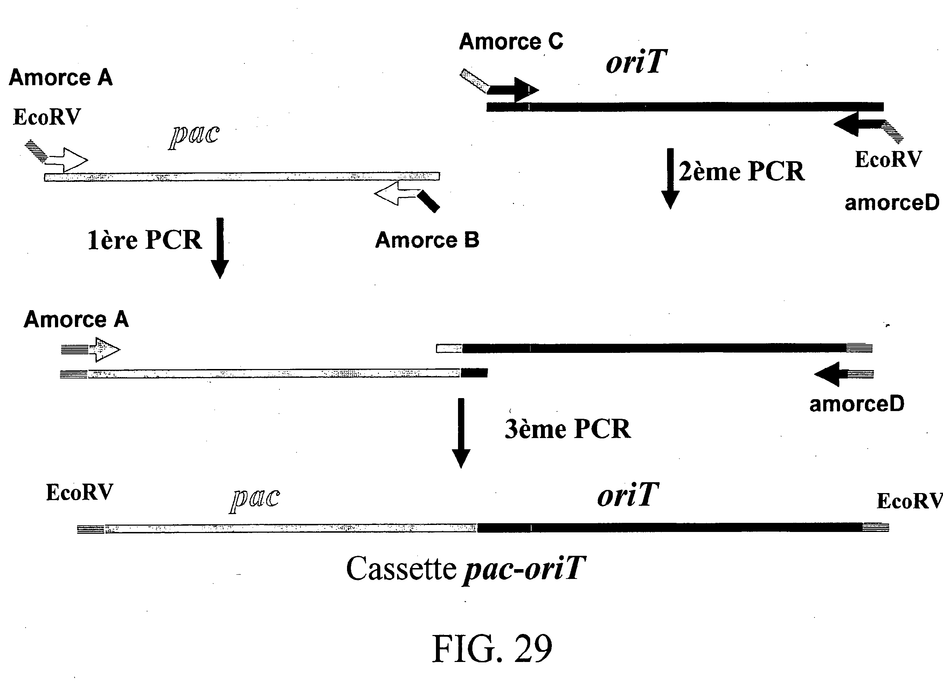

Figure 29 : Construction de la cassette *pac-oritT*.

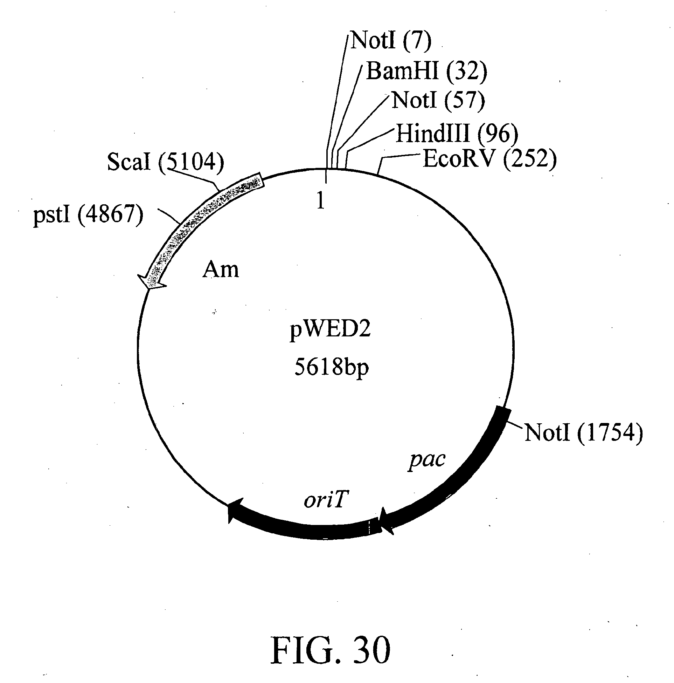

Figure 30 : Carte du cosmide pWED2.

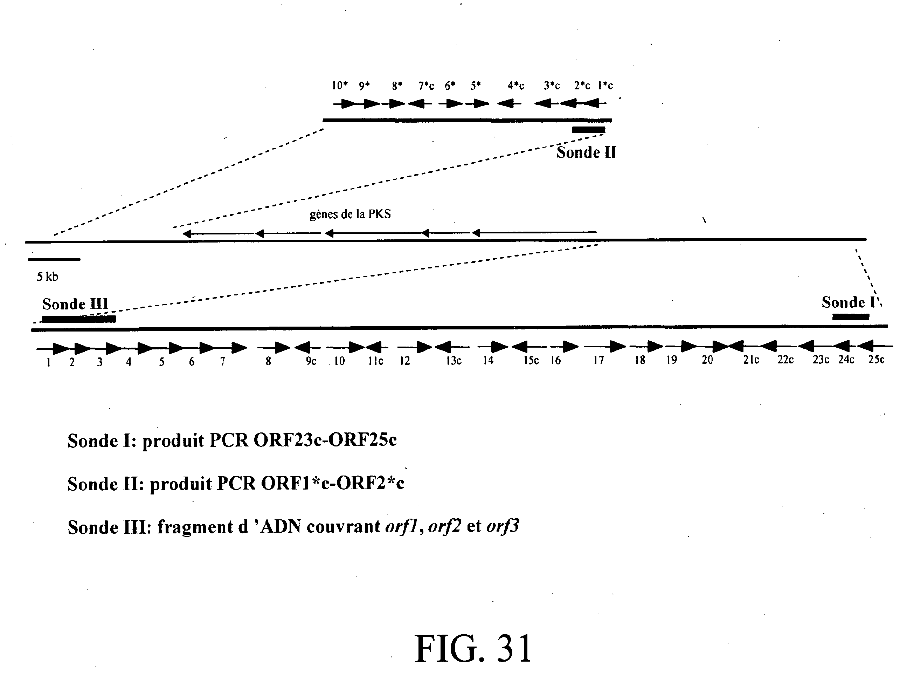

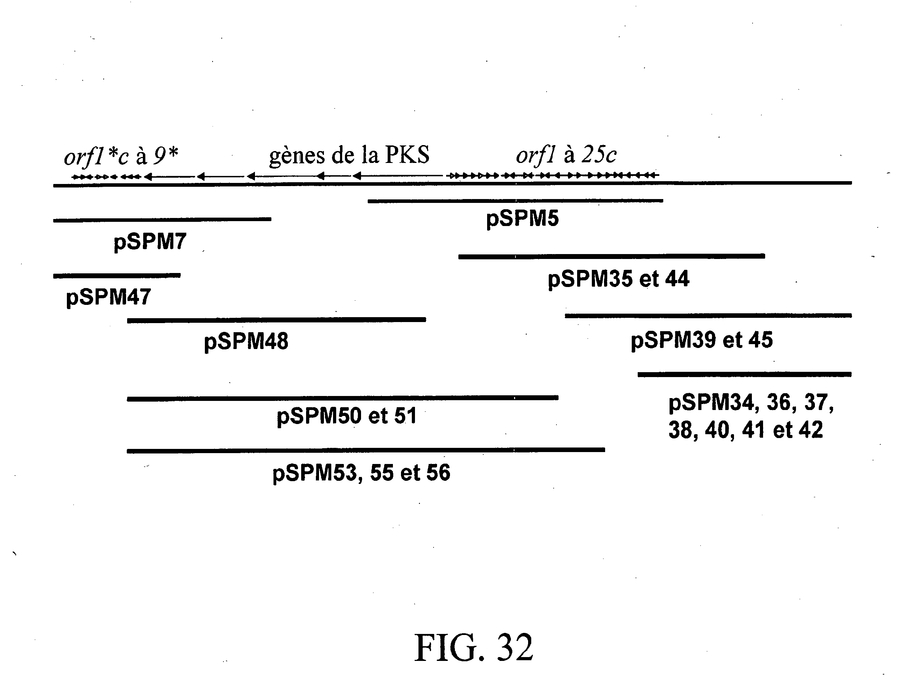

Figure 31 : Représentation schématique du groupe de gènes impliqués dans la voie de biosynthèse des spiramycines et de la localisation des trois sondes utilisées pour isoler les cosmides de la banque d'ADN génomique de la souche OSC2 de *Streptomyces ambofaciens* chez *E. coli* (cf. exemple 19).Figure 32 : Localisation des inserts des cosmides isolés de la banque d'ADN génomique de la souche OSC2 de *Streptomyces ambofaciens* chez *E. coli* (cf. exemple 19).
   Nouvelle banque d'ADN cosmidique.Figure 33 : Sous clonage du fragment *PstI-Pst*I du cosmide pSPM36 (insert du plasmide (pSPM58), sous clonage du fragment *Stu*I*-Stu*I du cosmide pSPM36 (insert du plasmide pSPM72) et sous clonage d'un fragment *EcoR*I-*Stu*I (insert du plasmide pSPM73).

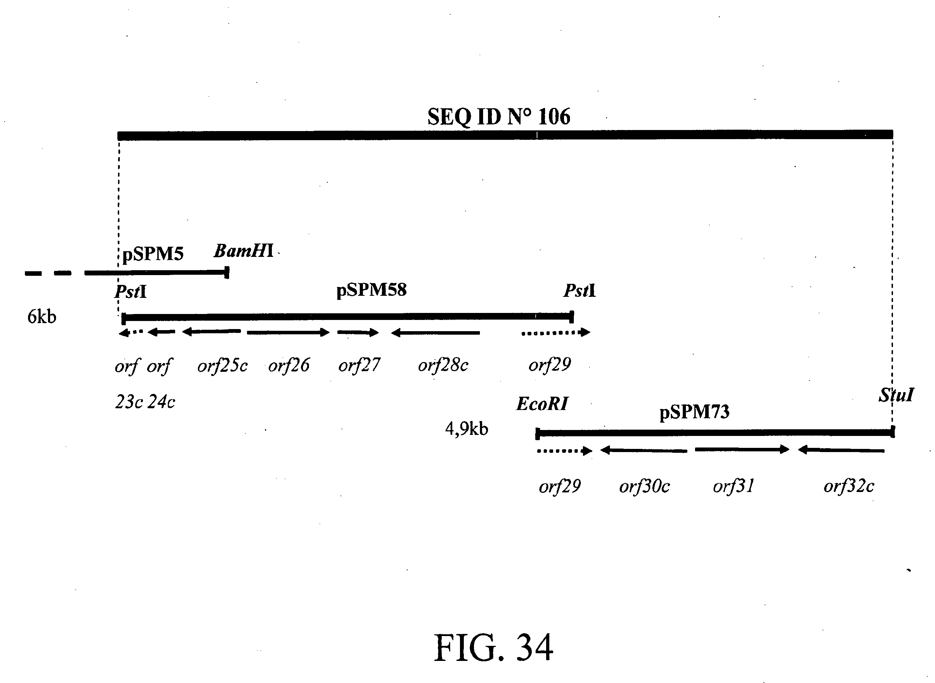

Figure 34 : Localisation des phases ouvertes de lecture indentifiées dans l'insert *Pst*I-*Pst*I du plasmide pSPM58 et dans l'insert *EcoR*I*-Stu*I du plasmide pSPM73.

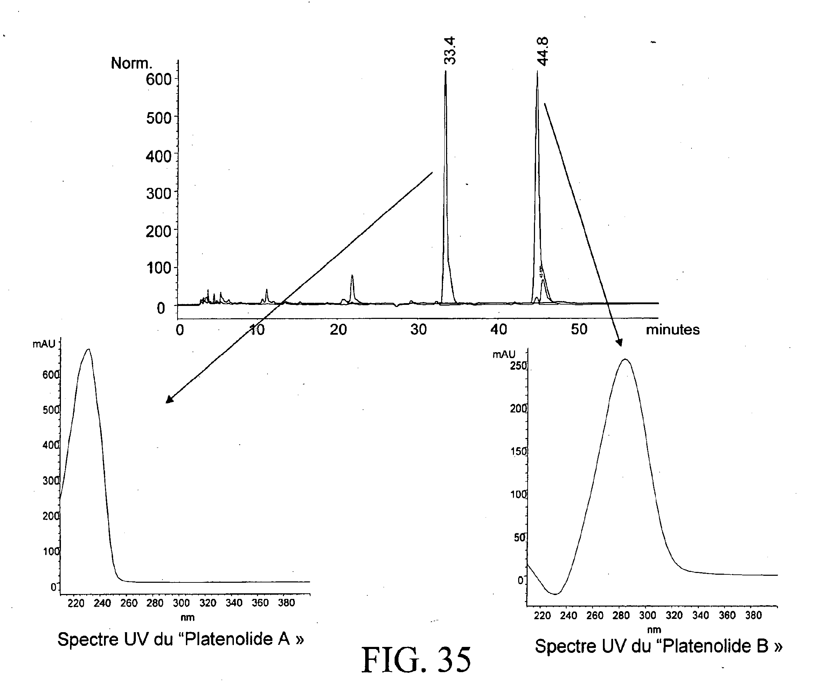

Figure 35 : Superposition des chromatogrammes CLHP du surnageant filtré du milieu de culture de la souche OS49.67 réalisés à 238 et 280nm (haut) et spectres UV des molécules élués à 33,4 minutes et 44,8 minutes (bas). (ne fait pas partie de l'invention) Figure 36 : Structure moléculaire des molécules platénolide A et platénolide B.
Figure 37 : Organisation du groupe de gènes impliqués dans la voie de biosynthèse des spiramycines.
Figure 38 : Structure moléculaire d'un intermédiaire de biosynthsèe produit par la souche SPM507.
Figure 39 : Structure d'un intermédiaire de biosynthsèse produit par une souche de *S*.
*ambofaciens* de génotype *orf6 *::att1Ωhyg+* obtenue à partir d'une souche surproduisant les spiramycines. L'insertion de la cassette *att1Ωhyg+* dans *orf6** exerce un effet polaire empéchant l'expression de *orf5*.*
Figure 40: Structure moléculaire des molécules platénolide A + mycarose et platénolide B + mycarose produites par la souche OS49.67

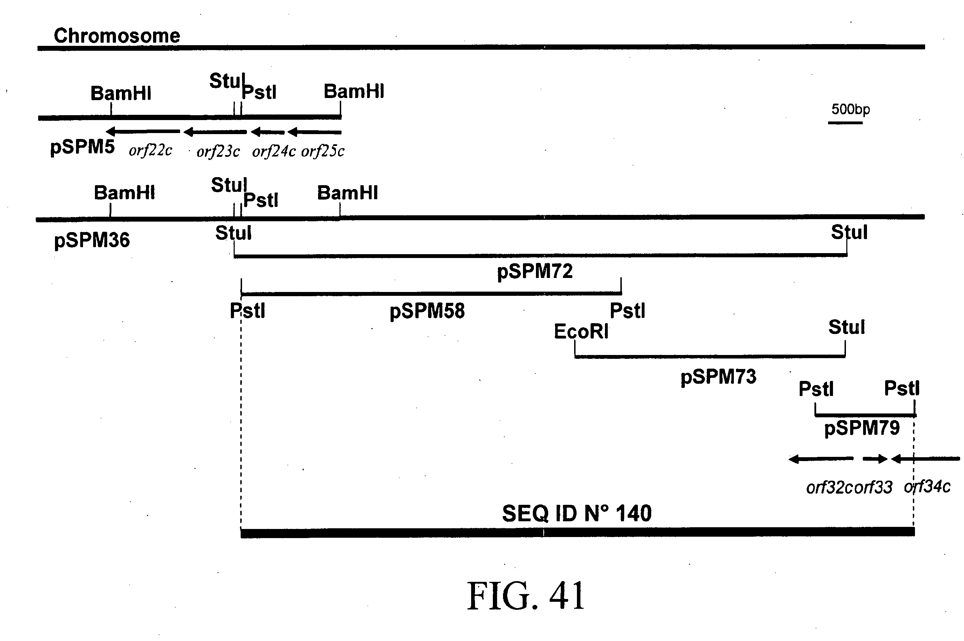

Figure 41 : Sous clonage d'un fragment *Pst*I*-Pst*I du cosmide pSPM36 (insert du plasmide (pSPM79)), localisation des phases ouvertes de lecture indentifiées dans l'insert *Pst*I*-Pst*I du plasmide pSPM79 et localisation de la séquence SEQ ID N° 140.

La présente invention est illustrée à l'aide des exemples qui suivent, qui doivent être considérés comme illustratifs et non limitatifs.

En résumé, les gènes de la voie de biosynthèse des spiramycines ont été isolés à partir d'une banque d'ADN génomique de *Streptomyces ambofaciens.* Cette banque a été obtenue par digestion partielle de l'ADN génomique de *Streptomyces ambofaciens.,* par l'enzyme de restriction *Bam*HI*.* De larges fragments d'ADN, de 35 à 45 kb en moyenne, ont été clonés dans le cosmide pWED1 (Gourmelen *et al.,* 1998) dérivé du cosmide pWED215 (Wahl *et al.,* 1987). Ces cosmides ont été introduits chez *E. coli* grâce à des particules phagiques. La banque ainsi obtenue a été hybridée avec une sonde (de séquence SEQ ID N° 86) correspondant à une partie du gène *tylB* de *S. fradiae* (Merson-Davies & Cundliffe, 1994, numéro d'accès GenBank : U08223). Après hybridation, un cosmide sur les 4 s'hybridant avec la sonde a été plus particulièrement sélectionné. Ce cosmide nommé pOS49.1 a ensuite été digéré par *Sac*I et un fragment de 3,3 kb contenant la région s'hybridant avec la sonde a été sous cloné dans le vecteur pUC19 et séquencé. Quatre phases ouvertes de lectures ont été identifiées et l'une d'entre elles code une protéine (SEQ ID N° 29) présentant de fortes similitudes de séquence avec la protéine TylB de *S. fradiae* (SEQ ID N° 87) (cf. figure 25). Ce gène a été nommé *orf3* (SEQ ID N° 28) et a été inactivé chez *S. ambofaciens.* Il a pu être démontré que les clones inactivés dans le gène *orf3* ne produisent plus de spiramycines. Ceci montre l'implication du gène *orf3* ou de gènes situés en aval dans la biosynthèse des spiramycines.

Une fois cette confirmation obtenue, une plus grande région du cosmide pOS49.1 a été séquencée, de part et d'autre du fragment *Sac*I précédemment étudié. Ainsi, à partir du cosmide pOS49.1, la séquence d'une région comportant sept phases ouvertes de lecture entières et deux autres incomplètes, situées de part et d'autre de ces sept phases ouvertes complètes, a pu être obtenue. Grâce à une recherche dans les bases de données, il a pu être montré que l'une des phases ouvertes de lecture incomplète correspondait au locus *srmG* (région codant une enzyme appelée « polyketide synthase » (PKS)). Les gènes correspondants ont été clonés par Burgett S. *et al.* en 1996 (Brevet américain US 5,945,320). Par ailleurs, les autres phases ouvertes de lecture, les sept ORFs entières nommées : *orf1, orf2, orf3, orf4, orf5, orf6, orf7* (SEQ ID N° 23, 25, 28, 30, 34, 36, 40) et le début de la huitième ORF nommé *orf18* (séquence SEQ ID N° 43) n'ont pas été retrouvées dans les bases de données.

Dans un deuxième temps et dans le but de cloner d'autres gènes impliqués dans la biosynthèse des spiramycines, d'autres cosmides comportant des fragments du génome de *S. ambofaciens* dans cette même région ont été isolés. Pour cela une nouvelle série d'hybridations sur colonies a été effectuée en utilisant trois sondes. La première sonde correspond à un fragment d'ADN de 3,7kb contenant *orf1*, *orf2* et le début de *orf3*, sous cloné à partir de pOS49.1. La deuxième sonde correspond à un fragment de 2 kb d'ADN contenant une partie de *orf7* et une partie de *orf8* et sous cloné à partir de pOS49.1. Une troisième sonde a également été utilisée. Cette dernière correspond à un fragment d'ADN de 1.8 kb contenant le gène *srmD.* Le gène *srmD* est un gène isolé de *S. ambofaciens* capable de conférer la résistance à la spiramycine. En effet, des travaux antérieurs avaient permis le clonage de plusieurs déterminants de résistance de *S*. *ambofaciens,* conférant la résistance à la spiramycine à une souche de *S. griseofuscus* (souche sensible à la spiramycine) (Pernodet et al., 1993) (Pernodet *et al.,* 1999). Pour l'isolement de gènes de résistances, une banque cosmidique de l'ADN génomique de la souche *S*. *ambofaciens* avait été réalisée dans le cosmide pKC505 (Richardson MA et al., 1987). Ce pool de cosmides avait été introduit chez *S. griseofuscus,* naturellement sensible à la spiramycine. Cinq cosmides capables de conférer la résistance à l'apramycine et la spiramycine chez *S. griseofuscus* avaient ainsi été obtenus. Parmi ces 5 cosmides, un cosmide nommé pOS44.1 possède dans son insert le gène *srmD* qui code une protéine présentant une certaine similitude avec la protéine codée par le gène *mdmA* de *Streptomyces mycarofaciens* et impliquée dans la résistance à la midécamycine chez cet organisme producteur (Hara et al., 1990, numéro d'accès Genbank : A60725) (figure 26). La troisième sonde utilisée pour localiser les gènes de biosynthèse de spiramycine a été un insert d'environ 1.8kb comprenant le gène *srmD.*

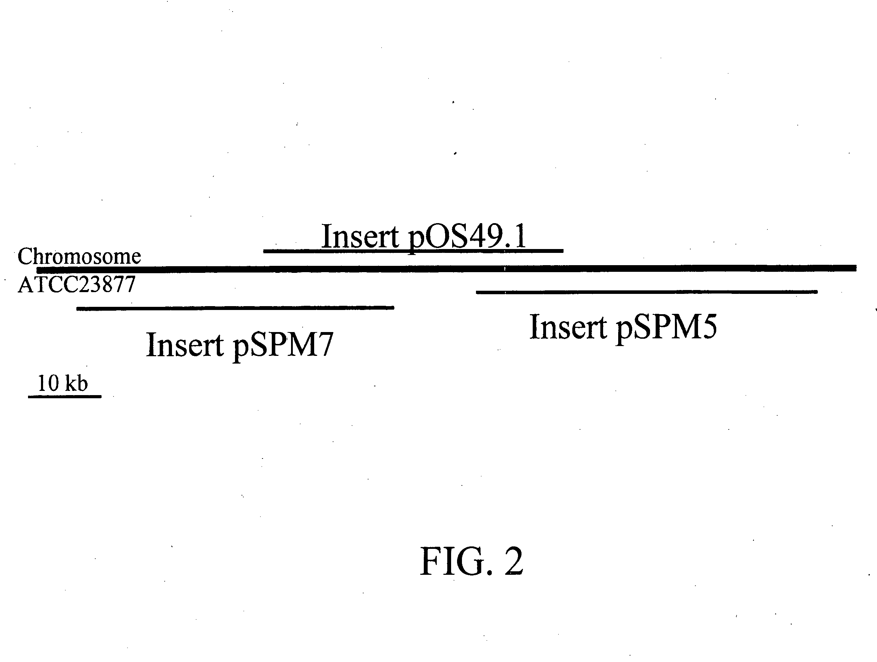

Ces trois sondes ont été utilisées pour hybrider la banque d'ADN génomique , décrite ci-dessus et ont permis de choisir deux cosmides (pSPM7 et pSPM5) susceptibles de contenir les inserts les plus longs et n'ayant pas de bandes communes. Le cosmide pSMP5 hybridait avec la première et la deuxième sonde, mais n'hybridait pas avec la troisième sonde, alors que le cosmide pSPM7 hybridait avec la troisième sonde seulement. Ces deux cosmides ont été séquencés en totalité par la technique du séquençage en aveugle (« shotgun sequencing »). Les séquences des inserts de ces deux cosmides : pSPM7 et pSPM5 ont pu être assemblées car bien que ne se chevauchant pas, chacun des inserts comprenait une séquence connue à l'une de ses extrémités. En effet, chacun de ces inserts comportait un fragment de la séquence d'un des gènes codant une enzyme appelée « polyketide synthase » (PKS). Ces 5 gènes ont été clonés par Burgett S. et al. en 1996 (Brevet américain US 5,945,320) (cf. figure 2). Ainsi, il a pu être déterminé une séquence unique d'ADN génomique de *S. ambofaciens.* Une séquence de 30943 nucléotides partant, en 5', d'un site *EcoR*I situé dans le premier gène PKS et allant jusqu'à un site *Bam*HI en 3' est présentée en SEQ ID N° 1. Cette séquence correspond à la région amont des gènes de la PKS (cf. figure 2 et 3). Une deuxième région de 11171 nucléotides, partant d'un site *Pst*I en 5' et allant jusqu'à un site *BstE*II en 3' situé dans le cinquième gène de la PKS est présentée en SEQ ID N° 2. Cette région est la région aval des gènes de la PKS (l'aval et l'amont étant définis par l'orientation des 5 gènes de PKS tous orientés dans le même sens) (cf. figures 2 et 3).

Dans un troisième temps et dans le but de cloner d'autres gènes impliqués dans la biosynthèse des spiramycines, d'autres cosmides comportant des fragments du génome de *S. ambofaciens* dans cette même région ont été isolés (cf. exemple 18 et 19).

### EXEMPLE 1 : Construction d'une banque d'ADN génomique de la souche ATCC23877 de Streptomyces ambofaciens chez E. coli

### 1.1. Extraction de l'ADN génomique de la souche ATCC23877 de Streptomyces ambofaciens.

La souche ATCC23877 de *Streptomyces ambofaciens.* (accessible notamment auprès de l'American Type Culture Collection (ATCC) (Manassas, Virginie, USA), sous le numéro 23877) a été cultivée en milieu YEME (Yeast Extract-Malt Extract) ((Kieser, T, *et al.,* 2000) et l'ADN génomique de cette souche a été extrait et purifié selon les techniques standards de lyse et précipitation (Kieser T. *et al.,* 2000).

### 1.2..Construction de la banque d'ADN génomique

L'ADN génomique de la souche ATCC23877 de *Streptomyces ambofaciens* tel qu'isolé ci-dessus a été digéré partiellement par l'enzyme de restriction *Bam*HI de manière à obtenir des fragments d'ADN de taille comprise entre environ 35 et 45 kb. Ces fragments ont été clonés dans le cosmide pWED1 (Gourmelen *et al.,* 1998) digéré au préalable par *Bam*HI. Le cosmide pWED1 est dérivé du cosmide pWE15 (Wahl, *et al.,* 1987) par délétion du fragment *Hpa*I*-Hpa*I de 4.1 kb contenant le module d'expression actif chez les mammifères (Gourmelen *et al.,* 1998). Le mélange de ligation a ensuite été encapsidé *in vitro* dans des particules de phages lambda grâce au système « Packagene® Lambda DNA packaging system » commercialisé par la société Promega en suivant les recommandations du fabriquant. Les particules phagiques obtenues ont été utilisées pour infecter la souche SURE® *d'E. coli* commercialisée par la société Stratagene (LaJolla, Californie, USA). La séléction des clones a été effectuée sur milieu LB + ampicilline (50 µg/ml) puisque le cosmide pWED1 confère la résistance à l'ampicilline.

### EXEMPLE 2 : Isolement et caractérisation de gènes impliqués dans la biosynthèse de spiramycines chez Streptomyces ambofaciens

### 2.1 Hybridation sur colonie des clones d'E. coli de la banque génomique de Streptomyces ambofaciens. ATCC23877

Environ 2000 clones *d'E. coli* de la banque obtenue ci-dessus, ont été transférés sur filtre pour hybridation sur colonies. La sonde utilisée pour l'hybridation est un fragment *Nae*I*-Nae*I d'ADN (SEQ ID N° 86) comportant une partie du gène *tylB* de *Streptomyces fradiae.* Ce fragment correspond aux nucléotides 2663 à 3702 du fragment d'ADN décrit par Merson-Davies, L.A. & Cundliffe E. (Merson-Davies, L.A. & Cundliffe E., 1994, numéro d'accès GenBank : U08223), dans lequel la séquence codante du gène *tylB* correspond aux nucléotides 2677 à 3843.

Le fragment *Nae*I*-Nae*I d'ADN portant une partie du gène *tylB* de *Streptomyces fradiae* (SEQ ID N° 86) a été marquée au ³²P par la technique du « random priming » (Kit commercialisé par la société Roche) et utilisé comme sonde pour l'hybridation des 2000 clones de la banque, transférés sur filtre. Les membranes utilisées sont des membranes nylon Hybond N commercialisées par la société Amersham (Amersham Biosciences, Orsay, France) et l'hybridation a été effectuée à 55°C dans le tampon décrit par Church & Gilbert (Church & Gilbert, 1984). Un lavage a été effectué en 2X SSC à 55°C pendant 15 minutes et deux lavages successifs en 0.5X SSC ont ensuite été effectués à 55°C, d'une durée de 15 minutes chacun. Dans ces conditions d'hybridation et de lavage, 4 clones parmi les 2000 hybridés présentaient un fort signal d'hybridation. Ces 4 clones ont été cultivés en milieu LB+ ampicilline (50 µg/ml) et les 4 cosmides correspondants ont été extraits par lyse alcaline standard (Sambrook *et al.,* 1989). Il a ensuite été vérifié que l'hybridation était bien due à un fragment d'ADN présent dans l'insert de ces quatre cosmides. Pour cela, les cosmides ont été digérés indépendamment par plusieurs enzymes (*Bam*HI*, PstiI* et *Sac*I*).* Les produits de digestions ont été séparés sur gel d'agarose, transférés sur membrane Nylon et hybridés par le fragment *Nae*I*-Nae*I d'ADN comportant une partie du gène *tylB* de *Streptomyces fradiae* (cf. ci-dessus) dans les mêmes conditions que ci-dessus. Les quatres cosmides ont pu être validés et un de ces cosmides a été plus particulièrement retenu et a été nommé pOS49.1.

### 2.2 Vérification de l'implication de la région identifiée et séquençage de l'insert du cosmide pOS49.1

Plusieurs fragments de l'insert du cosmide pOS49.1 ont été sous-clonés et leurs séquences ont été déterminées. Le cosmide pOS49.1 a été digéré par l'enzyme *Sac*I et il a été montré par Southern blot, dans les conditions décrites précédemment, qu'un fragment de 3,3 kb contient la région s'hybridant avec la sonde *TylB.* Ce fragment de 3,3 kb a été isolé par électroélution à partir d'un gel d'agarose 0.8% puis cloné dans le vecteur pUC19 (numéro d'accès GenBank M77789) et séquencé. Le plasmide ainsi obtenu a été nommé pOS49.11. Quatre phases ouvertes de lecture présentant un sage des codons typique de *Streptomyces* ont pu être identifiées dans ce fragment (deux complètes et deux tronquées), grâce au programme FramePlot (Ishikawa J & Hotta K. 1999). Des comparaisons de séquences grâce au programme FASTA (cf.. (Pearson W. R & D. J. Lipman, 1988) et (Pearson W. R., 1990), accessible notamment auprès du centre de ressources INFOBIOGEN, Evry, France) ont permis de montrer que la protéine déduite de l'une de ces 4 phases ouvertes de lecture présente de fortes similitudes de séquence avec la protéine TylB (SEQ ID N° 87; Numéro d'accès GenBank : U08223) de *S. fradiae* (cf. figure 25). Cette protéine a été nommée Orf3 (SEQ ID N° 29).

Dans le but de tester si le gène correspondant (le gène *orf3* (SEQ ID N° 28)) était impliqué dans la biosynthèse des spiramycines chez *S. ambofaciens,* ce gène a été interrompu par une cassette Ω*hyg* (Blondelet-Rouault M-H. *et al*.,1997, numéro d'accès GenBank : X99315). Pour cela, le plasmide pOS49.11 a été digéré par l'enzyme *Xho*I et le fragment contenant les quatre phases ouvertes de lecture (deux complètes et deux tronquées, dont *orf3* en entier) a été sous-cloné au niveau du site *Xho*I du vecteur pBC SK+ commercialisé par la société Stratagene (LaJolla, Califormie, USA). Le plasmide ainsi obtenu a été nommé pOS49.12. Dans le but d'inactiver *orf3,* un fragment *Pml*I-*BstE*II interne à *orf3* a été remplacé par la cassette Ω*hyg* par clonage bout franc dans ce dernier plasmide. Pour cela, le plasmide pOS49.12 a été digéré par les enzymes *Pml*I et *BstE*II dont le site unique se trouve dans la séquence codante du gène *orf3.* Les extrémités du fragment correspondant au vecteur ont été rendues bouts francs par un traitement par l'enzyme de Klenow (grand fragment de la DNA polymerase I). La cassette *Ωhyg* a été obtenue par digestion par l'enzyme *Bam*HI du plasmide pHP45 Ω*hyg* (Blondelet-Rouault *et al.,* 1997, numéro d'accès GenBank : X99315). Le fragment correspondant à la cassette Ω*hyg* a été récupéré sur gel d'agarose et ses extrémités ont été rendues bouts francs par un traitement par l'enzyme de Klenow. Les deux fragments bouts francs ainsi obtenus (la cassette Ω*hyg* et le plasmide pOS49.12) ont été ligués et le produit de ligation a été utilisé pour la transformation de bactéries *E. coli.* Le plasmide ainsi obtenu a été nommé pOS49.14 et contient le gène *orf3* interrompu par la cassette Ω*hyg.*

L'insert du plasmide pOS49.14, sous la forme d'un fragment *Xho*I*-Xho*I dont les extrémités ont été rendues non cohésives par un traitement par l'enzyme de Klenow, a été cloné au niveau du site *EcoRV* du plasmide pOJ260 (le plasmide pOJ260 est un plasmide conjugatif capable de se répliquer chez *E. coli* mais incapable de se répliquer chez *S. ambofaciens* (Bierman M *et al.,* 1992). Ce plasmide confère la résistance à l'apramycine chez *E. coli* et *Streptomyces*). Le plasmide obtenu (insert du plasmide pOS49.14 cloné dans le plasmide pOJ260) a été nommé pOS49.16. Ce dernier a été transféré dans la souche ATCC23877 de *S. ambofaciens* par conjugaison à l'aide de la souche conjugative *E. coli* S17-1, comme décrit par Mazodier *et al.* (Mazodier *et al.,* 1989). La souche *E. coli* S17-1 est dérivée de la souche *E. coli* 294 (Simon *et al.,* 1983) (Simon, *et al.,* 1986). Des clones transconjugants possédant le marqueur de résistance à l'hygromycine porté par la cassette Ω*hyg* et ayant perdu le marqueur de résistance à l'apramycine porté par le vecteur pOJ260 ont pu être obtenus. Pour cela, après conjugaison, les clones sont sélectionnés pour leur résistance à l'hygromycine. Les clones résistants à l'hygromycine sont ensuite repiqués respectivement sur milieu avec hygromycine (antibiotique B) et sur milieu avec apramycine (antibiotique A) (cf. figure 9). Les clones résistants à l'hygromycine (HygR) et sensibles à l'apramycine (ApraS) sont en principe ceux où un double événement de recombinaison s'est produit et qui possèdent donc le gène *orf3* interrompu par la cassette Ω*hyg.* Le remplacement de la copie sauvage de *orf3* par la copie interrompue a été vérifié par deux hybridations successives. Ainsi, l'ADN total des clones obtenus, a été digéré par différentes enzymes, séparé sur gel d'agarose, transféré sur membrane et hybridé avec une sonde correspondant à la cassette Ω*hyg* (cf. ci-dessus)) pour vérifier la présence de la cassette dans l'ADN génomique des clones obtenus. Une deuxième hybridation a été effectuée en utilisant comme sonde l'insert *Xho*I-*Xho*I du plasmide pOS49.11 contenant les quatre phases ouvertes de lecture (deux complètes et deux tronquées, dont *orf3* en entier). La vérification du génotype peut également être réalisée par toute méthode connue de l'homme du métier et notamment par PCR en utilisant les oligonucléotides appropriés et séquençage du produit de PCR. Un des clones *orf3*::Ω*hyg* ainsi obtenu et dont le génotype a été vérifié, a été choisi et a été appelé OS49.16.

La production en spiramycine du clone OS49.16 ainsi obtenu a été testée grâce au test de production décrit plus bas (cf. exemple 15). Il a ainsi pu être démontré que cette souche ne produit plus de spiramycine, confirmant l'implication de *orf3* et/ou des gènes situés en aval comme par exemple *orf4* dans la biosynthèse des spiramycines.

Une fois cette confirmation obtenue, une plus grande région du cosmide pOS49.1 a été séquencée, de part et d'autre du fragment *Sac*I précédemment étudié. Ainsi, à partir du cosmide pOS49.1, la séquence d'une région comportant sept phases ouvertes de lecture entières et deux autres incomplètes, situées de part et d'autre de ces sept phases ouvertes complètes, a pu être obtenue. Grâce à une recherche dans les bases de données, il a pu être montré que l'une des phases ouvertes de lecture incomplète correspondait au locus *srmG* (région codant une enzyme appelée « polyketide synthase » (PKS)). Les gènes correspondants ont été clonés par Burgett S. *et al.* en 1996 (Brevet américain US 5,945,320). Par ailleurs, les autres phases ouvertes de lecture: les sept ORFs entières nommées : *orf1, orf2, orf3, orf4, orf5, orf6, orf7* (SEQ ID N° 23, 25, 28, 30, 34, 36 et 40) et le début de la huitième ORF nommée *orf8* (la séquence entière de cette orf est donnée en SEQ ID N° 43) n'ont pas été retrouvées dans les bases de données.

### EXEMPLE 3 : Isolement et caractérisation d'autres gènes impliqués dans la biosynthèse de spiramycines chez Streptomyces ambofaciens (ne fait pas partie de l'invention)

Dans un deuxième temps et dans le but de cloner d'autres gènes impliqués dans la biosynthèse des spiramycines, d'autres cosmides comportant des fragments du génome de *S. ambofaciens* dans cette même région ont été isolés. Pour cela il a été procédé à une nouvelle série d'hybridations sur colonies en utilisant trois sondes :
- La première sonde utilisée correspond à un fragment d'ADN *Bam*HI*-Pst*I de 3,7kb contenant un fragment du gène de la PKS (Les gènes correspondant à la PKS ont été clonés par Burgett S. *et al.* en 1996 (Brevet américain US 5,945,320)), *orf1, orf2* et le début de *orf3,* sous cloné à partir de pOS49.1 et allant d'un site *Bam*HI situé 1300 paires de bases en amont du site *Eco*RI définissant la position 1 de SEQ ID N° 1, jusqu'au site *Pst*I situé en position 2472 (SEQ ID N° 1). Ce fragment *Bam*HI*-Pst*I a été sous cloné à partir de pOS49.1 dans le plasmide pBC SK+, ce qui a permis d'obtenir le plasmide pOS49.28.
- La deuxième sonde utilisée correspond à un fragment d'ADN *Pst*I*-BamH*I d'environ 2kb contenant un fragment d'*orf7* et d'*orf8*, sous cloné à partir de pOS49.1 et allant d'un site *Pst*I situé en position 6693 de SEQ ID N° 1 jusqu'au site *Bam*HI situé en position 8714 de SEQ ID N° 1. Ce fragment *Pst*I*-Bam*HI a été sous cloné à partir de pOS49.1 dans le plamside pBC SK+, ce qui a permis d'obtenir le plasmide pOS49.76.
- Une troisième sonde a également été utilisée. Cette dernière correspond à un fragment d'ADN de 1.8kb *Eco*RI*-Hind*III contenant le gène *srmD.* Le gène *srmD* est un gène isolé de *S. ambofaciens* capable de conférer la résistance à la spiramycine. En effet, des travaux antérieurs avaient permis le clonage de plusieurs déterminants de résistance de *S. ambofaciens.,* conférant la résistance à la spiramycine à une souche de *S. griseofuscus* (souche sensible à la spiramycine) (Pernodet *et al.,* 1993) (Pernodet *et al.,* 1999). Pour isoler des gènes de résistance, une banque cosmidique de l'ADN génomique de la souche *S. ambofaciens.* ATCC23877 avait été réalisée dans le cosmide pKC505 (Richardson MA *et al.,* 1987). Pour cela l'ADN génomique de la souche *S. ambofaciens.* ATCC23877 avait été digéré partiellement par *Sau3*AI de manière à obtenir des fragments de taille comprise entre environ 30 et 40 kb. L'ADN génomique ainsi digéré (3µg) avait été ligué avec 1µg du pKC505 préalablement digéré par l'enzyme *Bam*HI (Pernodet *et al.,* 1999). Le mélange de ligation avait ensuite été encapsidé *in vitro* dans des particules phagiques. Les particules phagiques obtenues avaient été utilisées pour infecter la souche *d'E. coli* HB101 (accessible notamment auprès de l'American Type Culture Collection (ATCC) (Manassas, Virginie, USA), sous le numéro 33694). Environ 20 000 clones *d'E. coli* résistant à l'apramycine avaient été poolés et les cosmides de ces clones avaient été extraits. Ce pool de cosmides avait été introduit par transformation de protoplastes dans la souche DSM 10191 de *S. griseofuscus* (Cox KL & Baltz RH. 1984), naturellement sensible à la spiramycine (Rao RN *et al.,* 1987, cette souche est disponible notamment auprès de la German Collection of Microorganisms and Cell Cultures (Deutsche Sammlung von Mikro-organismen und Zellkulturen GmbH, DSMZ), (Braunschweig, Allamagne), sous le numéro DSM 10191). Les transformants avaient été sélectionnés sur un milieu contenant de l'apramycine. 1300 des clones poussant sur milieu contenant de l'apramycine avaient été transférés sur milieu contenant 5µg/ml de spiramycine. Plusieurs clones résistants à l'apramycine avaient également poussé sur milieu contenant de la spiramycine et les cosmides de ces colonies avaient été extraits et utilisés pour transformer *E. coli* et *S. griseofuscus* (Pernodet *et al.*, 1999). Cinq cosmides capables de conférer la résistance à l'apramycine à *E. coli* et la co-résistance à l'apramycine et la spiramycine chez *S. griseofuscus* avaient ainsi été obtenus. Parmi ces 5 cosmides, il a été déterminé qu'un cosmide nommé pOS44.1 possède dans son insert un gène (SEQ ID N° 15) qui code une protéine (SEQ ID N° 16) présentant une certaine similitude avec une protéine codée par le gène *mdmA* de *Streptomyces mycarofaciens* (SEQ ID N° 88), ce gène a été nommé *srmD* (cf. alignement présenté en figure 26 réalisé grâce au programme FASTA (cf... (Pearson W. R & D. J. Lipman, 1988) et (Pearson W. R., 1990), accessibles notamment auprès du centre de ressources INFOBIOGEN, Evry, France).

Pour isoler le déterminant de résistance contenu dans le plasmide pOS44.1, celui-ci a été digéré partiellement par l'enzyme de restriction *Sau3*AI de manière à obtenir des fragments de taille d'environ 1,5 à 3 kb, ces fragments ont été ligués dans le vecteur pU486 linéarisé par l'enzyme *Bam*HI (Ward *et al.,* 1986). Un plasmide a été sélectionné pour sa capacité à conférer la résistance à la spiramycine chez la souche DSM 10191 de *S. griseofuscus* (Rao RN *et al.,* 1987), naturellement sensible à la spiramycine (cf. ci-dessus). Pour cela, le pool de plasmides correspondant aux fragment *Sau3*AI de pOS44.1 ligués dans le vecteur pIJ486 (cf. ci-dessus), a été introduit par transformation de protoplastes dans la souche DSM 10191 et les transformants ont été sélectionnés pour leur résistance au thiostrepton (due au gène *tsr* porté par pIJ486). Les clones poussant sur milieu contenant du thiostrepton ont été transférés sur milieu contenant de la spiramycine. Plusieurs clones résistants au thiostrepton ont également poussé sur milieu contenant de la spiramycine et les plasmides de ces colonies ont été extraits. Un plasmide conférant la résistance et contenant un insert d'environ 1.8kb a été sélectionné et nommé pOS44.2. Cet insert de 1.8kb peut être sorti facilement grâce à un site *Hind*III et un site *Eco*RI présents dans le vecteur de part et d'autre de l'insert. Cet insert de 1.8kb *Hind*III*-Eco*RI a été séquencé et le gène de résistance qu'il renferme a été nommé *srmD.* Ce fragment contenant le gène *srmD* a ainsi pu être facilement sous-cloné dans le vecteur pUC19 (numéro d'accès GenBank M77789) ouvert par *Eco*RI*-Hind*III*,* le plasmide obtenu a été nommé pOS44.4. L'insert de 1.8kb *Hind*III*-Eco*RI*,* contenant le gène *srmD,* de ce plasmide a été utilisé comme sonde pour localiser les gènes de biosynthèse de spiramycine (cf. ci-dessous).

Un échantillon d'une souche *Escherichia Coli* DH5α contenant le plasmide pOS44.4 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement 1-2918.

Environ 2000 clones de la banque, obtenus ci-dessus (cf. exemple 1), ont été transférés sur filtre pour hybridation sur colonies selon les techniques classiques (Sambrook *et al.,* 1989).

Les trois sondes décrites ci-dessus ont été marquées au ³²P par la technique du « random priming » (Kit commercialisé par Roche) et utilisées pour l'hybridation des 2000 clones de la banque, transférés sur filtre. L'hybridation a été effectuée à 65°C dans le tampon décrit par Church & Gilbert (Church & Gilbert, 1984). Un lavage a été effectué en 2X SSC à 65°C pendant 15 minutes et deux lavages successifs ont ensuite été effectués en 0.5X SSC à 65°C, d'une durée de 15 minutes chacun. Dans ces conditions d'hybridation et de lavage, 16 clones parmi les 2000 hybridés présentaient un fort signal d'hybridation avec au moins une des sondes. Cependant, aucun cosmide n'hybridait avec les trois sondes. Les 16 cosmides ont été extraits et digérés par l'enzyme de restriction *Bam*HI*.* La comparaison des profils de restriction de ces différents cosmides entre eux, a conduit à choisir deux cosmides susceptibles de contenir les inserts les plus longs de la région et n'ayant pas de bandes communes. Ainsi deux cosmides l'un nommé pSPM5 et l'autre pSPM7 ont été choisis. Le cosmide pSPM5 hybridait avec les sondes *orf1* à *orf4* et la sonde *orf8,* mais n'hybridait pas avec la sonde *srmD.* pSPM7 hybridait seulement avec la sonde *srmD* et pas avec les deux autres sondes.

Ces deux cosmides ont été séquencés en totalité par la technique du séquençage en aveugle (« shotgun sequencing »). Les séquences des inserts de ces deux cosmides : pSPM7 et pSPM5 ont pu être assemblées car bien que ne se chevauchant pas, chacun des inserts comprenait une séquence connue à l'une de ses extrémités. En effet, chacun de ces inserts comportait un fragment de la séquence d'un des gènes codant une enzyme appelée « polyketide synthase » (PKS). Ces 5 gènes ont été clonés par Burgett S. *et al..* en 1996 (Brevet américain US 5,945,320) (cf. figure 2). Ainsi, il a pu être déterminé une séquence unique d'ADN génomique de *S. ambofaciens.* Une séquence de 30943 nucléotides partant en 5' d'un site *Eco*RI situé dans le premier gène PKS et allant jusqu'à un site *Bam*HI en 3' est présentée en SEQ ID N° 1. Cette séquence correspond à la région amont des gènes de la PKS (cf. figure 2 et 3). Une deuxième région de 11171 nucléotides, partant d'un site *Pst*I en 5' et allant jusqu'à un site *Nco*I en 3' situé dans le cinquième gène de la PKS est présentée en SEQ ID N° 2. Cette deuxième région est la région aval des gènes de la PKS (l'aval et l'amont étant défini par l'orientation des 5 gènes de PKS tous orientés dans le même sens) (cf. figure 2 et 3).

### EXEMPLE 4 : Analyse des séquences nucléotidiques, détermination des phases ouvertes de lecture et caractérisation des gènes impliqués dans la biosynthèse des spiramycines. (ne fait pas partie de l'invention)

Les séquences obtenues ont été analysées grâce au programme FramePlot (Ishikawa J & Hotta K. 1999). Ceci a permis d'identifier, parmi les phases ouvertes de lecture, les phases ouvertes de lecture présentant un usage des codons typique de *Streptomyces.* Cette analyse a permis de déterminer que cette région comporte 35 ÔRFs localisées de part et d'autre de cinq gènes codant l'enzyme «polyketide synthase » (PKS). Respectivement 10 et 25 ORFs ont été identifiées en aval et en amont de ces gènes (l'aval et l'amont étant défini par l'orientation des 5 gènes de PKS tous orientés dans le même sens) (cf. figure 3). Ainsi, les 25 phases ouvertes de lecture de ce type, occupant une région d'environ 31 kb (SEQ ID N° 1 et figure 3) ont été identifiées en amont des 5 gènes codant la PKS et 10 occupant une région d'environ 11,1 kb (SEQ ID N° 2 et figure 3), ont été identifiées en aval des gènes de la PKS. Les gènes de la région amont ont été nommés *orf1, orf2, orf3, orf4, orf5, orf6, orf7, orf8, orf9c, orf10, orf11c, orf12, orf13c, orf14, orf15c, orf16, orf17, orf18, orf19, orf20, orf21c, orf22c, orf23c, orf24c et orf25c* (SEQ ID N° 23, 25, 28, 30, 34, 36, 40, 43, 45, 47, 49, 53, 60, 62, 64, 66, 68, 70, 72, 74, 76, 78, 80, 82 et 84). Les gènes de la région aval ont été nommés *orf1*c, orf2***c*, *orf3*c, orf4*c, orf5* orf6*, orf7*c, orf8*, orf9*, orf10** (SEQ ID N° 3, 5, 7, 9, 11, 13, 15, 17, 19 et 21). Le « c » ajouté dans le nom du gène signifiant pour l'ORF en question que la séquence codante est dans l'orientation inverse (le brin codant est donc le brin complémentaire de la séquence donnée en SEQ ID N° 1 ou SEQ ID N° 2 pour ces gènes) (cf. figure 3).

Les séquences protéiques déduites de ces phases ouvertes de lecture ont été comparées avec celles présentes dans différentes bases de données grâce à différents programmes : BLAST (Altschul *et al.,* 1990) (Altschul *et al.,* 1997), CD-search, COGs (Cluster of Orthologous Groups) (ces trois programmes sont accessibles notamment auprès du National Center for Biotechnology Information (NCBI) (Bethesda, Maryland, USA)), FASTA ((Pearson W. R & D. J. Lipman, 1988) et (Pearson W. R., 1990), BEAUTY (Worley K. C.. *et al.,* 1995)), (ces deux programmes sont accessibles notamment auprès du centre de ressources INFOBIOGEN, Evry, France). Ces comparaisons ont permis de formuler des hypothèses sur la fonction des produits de ces gènes et d'identifier ceux susceptibles d'être impliqués dans la biosynthèse de spiramycine.

### EXEMPLE 5 : Inactivation de gène : principe de la construction d'une souche de Streptomyces ambofaciens interrompue

Les méthodes utilisées consistent à effectuer un remplacement de gène. Le gène cible à interrompre est remplacé par une copie de ce gène interrompue par une cassette conférant la résistance à un antibiotique (par exemple l'apramycine ou l'hygromycine), comme l'illustre la figure 9. Les cassettes utilisées sont bordées de part et d'autre par des codons de terminaison de la traduction dans toutes les phases de lecture et par des terminateurs de transcription actifs chez *Streptomyces.*

L'insertion de la cassette dans le gène cible peut s'accompagner ou non d'une délétion dans ce gène cible. La taille des régions flanquant la cassette peut aller de quelques centaines à plusieurs milliers de paires de bases.

Les constructions nécessaires à l'inactivation du gène par la cassette ont été obtenues chez *E. coli,* organisme de référence pour l'obtention de constructions d'ADN recombinant. Le gène interrompu a été obtenu dans un plasmide se répliquant chez *E*. *coli* mais ne pouvant pas se répliquer chez *Streptomyces.*

Les constructions ont ensuite été sous-clonées dans des vecteurs pour permettre la transformation et l'inactivation du gène voulu chez *S. ambofaciens.* Pour cela, deux plasmides ont été utilisés :
- pOJ260 (Bierman M. *et al.,* 1992) (cf. exemple 2) qui confère la résistance à l'apramycine chez *E. coli* et *Streptomyces* et qui a été employé quand le gène cible a été interrompu par une cassette conférant la résistance à l'hygromycine.
- pOSK1205 (4726pb). Ce plasmide dérive du plasmide pBK-CMV (commercialisé par la société Stratagene (LaJolla, Californie, USA)) dans lequel un fragment Avr*II* contenant la séquence codant la résistance à la Néomycine/Kanamycine a été remplacé par une séquence codant la résistance à l'hygromycine, tout en conservant le promoteur P SV40. Pour cela, le plasmide pHP45-Ω*hyg* (Blondelet-Rouault *et al.,* 1997) a été digéré par les enzymes *Not*I et *Pflm*I et le fragment conférant la résistance à l'hygromycine a été sous-cloné au niveau du site *Avr*II du vecteur pBK-CMV après que toutes les extrémités aient été rendues bout franc par traitement à l'enzyme de Klenow. Dans pOSK1205, la cassette qui confère la résistance à l'hygromycine est précédée du promoteur pSV40. Ce plasmide confère la résistance à l'hygromycine chez *E. coli* et *Streptomyces* et a été employé quand le gène cible a été interrompu par une cassette conférant la résistance à l'apramycine.

Les cassettes ont été introduites dans le gène cible soit par clonage en utilisant des sites de restriction présents dans le gène cible, soit par recombinaison entre de courtes séquences identiques comme décrit par exemple par Chaveroche *et al* (Chaveroche MK *et al.,* 2000).

Le plasmide portant le gène interrompu par la cassette peut ensuite être introduit chez *Streptomyces ambofaciens,* par exemple par conjugaison entre *E. coli* et *Streptomyces* (Mazodier P, *et al.,* 1989). Cette technique a été utilisée dans le cas où le vecteur de base est le vecteur pOJ260. Une deuxième technique peut être utilisée : la technique de la transformation de protoplastes après dénaturation par traitement alcalin dé l'ADN (Kieser, T *et al.,* 2000), pour augmenter la fréquence de recombinaison comme décrit par exemple par Oh & Chater (Oh & Chater, 1997). Cette technique a été utilisée dans le cas où le vecteur de base est pOJ260 ou pOSK1205 (cf. ci-dessous). Les transformants sont ensuite sélectionnés avec l'antibiotique correspondant à la cassette présente dans le gène cible (cf. figure 9, antibiotique B). On sélectionne ainsi un mélange de clones parmi lesquels il y a eu intégration par un seul ou par deux événements de recombinaison. Dans un deuxième temps on recherche les clones sensibles à l'antibiotique pour lequel le gène de résistance est présent dans le vecteur (hors de la cassette de recombinaison) (cf. figure 9, antibiotique A). On peut ainsi sélectionner les clones pour lesquels il y a eu en principe deux événements de recombinaison aboutissant au remplacement du gène sauvage par la copie interrompue par la cassette. Ces étapes sont schématisées figure 9.

Plusieurs cassettes peuvent être utilisées pour l'interruption des gènes cibles. On peut par exemple utiliser la cassette Ω*hyg* qui confère la résistance à l'hygromycine (Blondelet-Rouault *et al.,* 1997, numéro d'accès GenBank : X99315).

### EXEMPLE 6: Construction d'une souche de Streptomyces ambofaciens interrompue en phase dans le gène orf3 (ne fait pas partie de l'invention)

Le gène *orf3* avait été interrompu par la cassette *Ωhyg* (cf. exemple 2.2) et il a pu être démontré qu'une souche *orf3*:: Ω*hyg* ne produit plus de spiramycine, confirmant l'implication d'un ou plusieurs gènes de la région clonée dans la biosynthèse des spiramycines (cf. exemple 2.2). Au vu de leur orientation, une cotranscription des ORFs 1 à 7 est probable (cf. figure 3) et le phénotype observé (non producteur de spiramycines) peut être dû à l'inactivation d'un ou plusieurs des gènes co-transcrits avec *orf3.* Pour confirmer l'implication de *orf3* dans la biosynthèse de spiramycines, une nouvelle inactivation du gène *orf3* a été effectuée, cette dernière étant réalisée en phase. Pour cela, un fragment *Dra*III interne à *orf3* de 504 paires de bases a été délété. Un fragment d'ADN obtenu à partir de pOS49.1, allant du site *Eco*RI situé en position 1 (SEQ ID N° 1) jusqu'au site *Sac*I situé en position 5274 (SEQ ID N° 1) et qui comporte une délétion entre les deux sites *Dra*III aux positions 2563 et 3067 (504 nucléotides retirés) a été cloné dans le plasmide pOJ260 (Bierman M. et al., 1992). Le plasmide ainsi obtenu a été nommé pOS49.67.

L'insert de pOS49.67 est donc constitué par un fragment d'ADN de *S. ambofaciens* contenant les gènes *orf1, orf2, orf3* avec la délétion en phase, *orf4* et une partie de *orf5.* Le vecteur dans lequel cet insert a été sous-cloné est pOJ260, le plasmide pOS49.67 confère donc la résistance à l'apramycine et a été introduit par transformation de protoplastes dans la souche OS49.16 (cf. exemple 2). La souche OS49.16 étant résistante à l'hygromycine, des transformants hygR et apraR ont été obtenus. Après deux passages de tels clones sur milieu non sélectif, les clones sensibles à l'apramycine et à l'hygromycine (apraS et hygS) ont été recherchés. Dans certains de ces clones, on s'attend en effet à ce qu'un événement de recombinaison entre séquences homologues conduise au remplacement de la copie de *orf3* interrompue par la cassette *Ωhyg* (contenu dans le génome de la souche OS49.16) par la copie de *orf3* avec la délétion en phase présente sur le vecteur. Les clones résultant de cette recombinaison sont attendus comme apraS et hygS après l'élimination des séquences du vecteur. Le génotype des souches ainsi obtenues peut être vérifié par hybridation ou par PCR et séquençage du produit de PCR (pour vérifier que seule une copie de *orf3* délétée en phase est présente dans le génome des clones obtenus). Des clones n'ayant que la copie de *orf3* avec une délétion en phase ont ainsi été obtenus et leur génotype a été vérifié. Un clone présentant les caractéristiques recherchées a été plus particulièrement sélectionné et a été nommé OS49.67.

Un échantillon de la souche OS49.67 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement 1-2916.

### EXEMPLE 7: Construction d'une souche de Streptomyces ambofaciens interrompue dans le gène orf8 (ne fait pas partie de l'invention)

Pour réaliser l'inactivation du gène *orf8,* une construction dans laquelle la cassette Ω*hyg* a été introduite dans la séquence codante de *orf8* a été obtenue. Pour cela, le plasmide pOS49.88 a tout d'abord été construit. Le plasmide pOS49.88 est dérivé du plasmide pUC19 (numéro d'accès GenBank M77789) par insertion d'un fragment de 3.7 kb (fragment *Pst*I*-EcoR*I obtenu à partir du cosmide pSPM5), contenant la fin de *orf7, orf8* et le début de *orf9*, cloné dans les sites *Pst*I*-EcoR*I de pUC19. La cassette Ω*hyg* (sous forme d'un fragment *Bam*HI*,* rendu bout franc par traitement à l'enzyme de Klenow) a été clonée au niveau du site unique *Sal*I de pOS49.88 situé dans *orf8* après que toutes les extrémités aient été rendues bout franc par traitement à l'enzyme de Klenow.

Le clonage étant bout franc, deux types de plasmides ont été obtenus selon le sens d'insertion de la cassette : pOS49.106 dans lequel les gènes *hyg* et *orf8* sont dans la même orientation et pOS49.120 dans lequel les gènes *hyg* et *orf8* sont dans des orientations opposées. L'insert du plasmide pOS49.106 a ensuite été sous-cloné dans le plasmide pOJ260 pour donner pOS49.107. Pour cela, le plasmide pOS49.106 a été digéré par l'enzyme *Asp*718I et les extrémités ont été rendues bout franc par traitement à l'enzyme de Klenow, ce produit de digestion a été redigéré par l'enzyme *Pst*I et le fragment contenant le gène *orf8* dans lequel la cassette Ω*hyg* a été insérée, a été cloné dans le vecteur pOJ260 (cf. ci dessus). Pour cela, le vecteur pOJ260 a été digéré par les enzymes *EcoR*V et *Pst*I et utilisé pour la ligation. Cette manipulation permet donc d'obtenir une ligation orientée puisque chacun des deux fragments est bout franc d'un côté et *Pst*I de l'autre. Le plasmide obtenu a été nommé pOS49.107.

Le plasmide pOS49.107 a été introduit dans la souche ATCC23877 de *S*. *ambofaciens* par transformation de protoplastes (Kieser, T *et al..,* 2000). Après transformation des protoplastes, les clones sont sélectionnés pour leur résistance à l'hygromycine. Les clones résistants à l'hygromycine sont ensuite repiqués respectivement sur milieu avec hygromycine (antibiotique B) et sur milieu avec apramycine (antibiotique A) (cf. figure 9). Les clones résistants à l'hygromycine (HygR) et sensibles à l'apramycine (ApraS) sont en principe ceux où un double événement de crossing over s'est produit et qui possèdent le gène *orf8* interrompu par la cassette Ω*hyg.* Le remplacement de la copie sauvage de *orf8* par la copie interrompue par la cassette Ω*hyg* a été vérifié par Southern Blot. Ainsi, l'ADN total des clones obtenus, a été digéré par plusieurs enzymes, séparé sur gel d'agarose, transféré sur membrane et hybridé avec une sonde correspondant à la cassette Ω*hyg* pour vérifier la présence de la cassette dans l'ADN génomique des clones obtenus. Une deuxième hybridation a été effectuée en utilisant comme sonde l'insert *Pst*I*-EcoR*I contenant la fin de *orf7, orf8* et le début de *orf9* d'une taille d'environ 3,7 kb du plasmide pOS49.88. La vérification du génotype peut être réalisée par toute méthode connue de l'homme du métier et notamment par PCR en utilisant les oligonucléotides appropriés et séquençage du produit de PCR.

Un clone *orf8*::Ω*hyg* a été choisi et nommé OS49.107. Un échantillon de la souche OS49.107 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement 1-2917.

### EXEMPLE 8: Construction d'une souche de Streptomyces ambofaciens interrompue dans le gène orf10 (ne fait pas partie de l'invention)

Un fragment d'ADN de 1,5 kb interne au gène *orf10* a été obtenu par PCR en utilisant comme matrice, l'ADN génomique de *S. ambofaciens* et les amorces suivantes :
SRMR1 : 5' CTGCCAGTCCTCTCCCAGCAGTACG 3' (SEQ ID N° 89)
SRMR2 : 5' TGAAGCTGGACGTCTCCTACGTCGG 3' (SEQ ID N° 90)
Ce fragment d'ADN issu de la PCR a été cloné dans le vecteur pCR2.1 commercialisé par la société Invitrogen (Carlsbad, Californie, USA). Le plasmide ainsi obtenu a été nommé pOS49.32. La cassette Ω*hyg* (sous forme d'un fragment *Bam*HI*,* cf. ci-dessus) a été clonée au niveau du site unique *BstE*II interne au fragment du gène *orf10,* après que toutes les extrémités aient été rendues bout franc par traitement à l'enzyme de Klenow. Le clonage étant bout franc, deux types de plasmides ont été obtenus selon le sens d'insertion de la cassette : pOS49.43 dans lequel les gènes *hyg* et *orf10* sont dans la même orientation et pOS49.44 dans lequel les gènes *hyg* et *orf10* sont dans des orientations opposées. L'insert du plasmide pOS49.43 a été transféré (sous forme d'un fragment *Asp718*I-*Xba*I dont les extrémités ont été rendues bout franc par traitement à l'enzyme de Klenow) dans le site *EcoR*V du plasmide pOJ260 ce qui a permis d'obtenir le plasmide pOS49.50. Le plasmide pOS49.50 contenant le fragment du gène *orf10* interrompu par la cassette Ω*hyg* a été introduit dans la souche ATCC23877 de *Streptomyces ambofaciens.* Après transformation, les clones sont sélectionnés pour leur résistance à l'hygromycine. Les clones résistants à l'hygromycine sont ensuite repiqués respectivement sur milieu avec hygromycine (antibiotique B) et sur milieu avec apramycine (antibiotique A) (cf. figure 9). Les clones résistants à l'hygromycine (HygR) et sensibles à l'apramycine (ApraS) sont en principe ceux où un double événement de crossing over s'est produit et qui possèdent le gène *orf10* interrompu par la cassette Ω*hyg.* Des clones qui possédaient le marqueur de résistance à l'hygromycine porté par la cassette et qui avaient perdu le marqueur de résistance à l'apramycine porté par le vecteur pOJ260 ont ainsi été obtenus. L'événement de remplacement de la copie sauvage de *orf10* par la copie interrompue *orf10*::Ω*hyg* a été vérifié par Southern Blot. Ainsi, l'ADN génomique total des clones obtenus, a été digéré par plusieurs enzymes, séparé sur gel d'agarose, transféré sur membrane et hybridé avec une sonde correspondant à la cassette Ω*hyg* pour vérifier la présence de la cassette dans l'ADN génomique des clones obtenus. Une deuxième hybridation a été effectuée en utilisant comme sonde le produit PCR de 1,5 kb interne au gène *orf10* (cf. ci-dessus).

Un clone présentant les caractéristiques attendues (*orf10*::Ω*hyg*) a été plus particulièrement sélectionné et nommé OS49.50. Il a pu en effet être vérifié grâce aux deux hybridations que la cassette Ω*hyg* était bien présente dans le génome de ce clone et que l'on obtient bien le profil de digestion attendu dans le cas d'un remplacement, à la suite d'un double événement de recombinaison, du gène sauvage par la copie interrompue par la cassette Ω*hyg* dans le génome de ce clone. La vérification du génotype peut également être réalisée par toute méthode connue de l'homme du métier et notamment par PCR en utilisant les oligonucléotides appropriés et séquençage du produit de PCR.

### EXEMPLE 9 : Inactivation de gènes : principe de la construction d'une souche de Streptomyces ambofaciens interrompue selon la technique des « cassettes excisables » (cf. Figure 9 et 10)

Un deuxième type de cassettes peut être utilisé pour l'inactivation de gènes : les cassettes dites « cassettes excisables ». Ces cassettes présentent l'avantage de pouvoir être excisées chez *Streptomyces* par un événement de recombinaison spécifique de site après avoir été introduites dans le génome de *S*. *ambofaciens.* Le but est d'inactiver certains gènes dans des souches de *Streptomyces* sans laisser dans la souche finale de marqueurs de sélection ou de grandes séquences d'ADN n'appartenant pas à la souche. Après excision il subsiste uniquement une courte séquence d'environ une trentaine de paires de bases (appelé site « cicatriciel ») dans le génome de la souche (cf. figure 10).

La mise en oeuvre de ce système consiste, dans un premier temps, au remplacement de la copie sauvage du gène cible (grâce à deux événements de recombinaison homologue, cf. figure 9) par une construction dans laquelle une cassette excisable a été insérée dans ce gène cible. L'insertion de cette cassette est accompagnée d'une délétion dans le gène cible (cf. figure 9). Dans un deuxième temps, l'excision de la cassette excisable du génome de la souche est provoquée. La cassette excisable fonctionne grâce à un système de recombinaison spécifique de site et a pour avantage de permettre l'obtention de mutants de *Streptomyces* ne portant finalement pas de gène de résistance. On s'affranchit également d'éventuels effets polaires sur l'expression des gènes situés en aval du ou des gènes inactivés (cf. figure 10).

L'emploi de cassettes excisables a été décrit et utilisé chez de nombreux organismes dont les cellules de mammifère, de levures et chez *E. coli* (Bayley *et al.*, 1992 ; Brunelli et Pall, 1993 ; Camilli *et al.*, 1994 ; Dale et Ow, 1991 ; Russell *et al.*, 1992; Lakso *et al.*, 1992). Ces cassettes excisables utilisent toutes la recombinase spécifique de site Cre agissant sur les sites lox. Ce système de recombinaison provient du bactériophage P1.

Pour la construction d'un système de type « cassette excisable » chez *Streptomyces,* il a été tiré partie du système de recombinaison spécifique de site décrit pour l'élément génétique mobile pSAM2 de *Streptomyces ambofaciens* (Boccard *et al.*, 1989a et b). Le système mis en place consiste dans un premier temps à construire un vecteur recombinant comportant le gène à interrompre dans lequel est insérée une cassette excisable. L'insertion dans le gène cible de la cassette excisable s'accompagne d'une délétion dans le gène cible. Elle peut être réalisée par clonage en utilisant des sites de restriction présents dans le gène cible ou par recombinaison entre de courtes séquences identiques comme décrit par exemple par Chaveroche *et al* (Chaveroche MK *et al.,* 2000). La cassette excisable peut être construite en utilisant par exemple la cassette Ω*hyg* (Blondelet Rouault *et al*., 1997). Cette cassette a été bordée par des séquences *attR* et *attL* qui flanquent normalement la copie intégrée de pSAM2 (cf. figure 15). Les séquences *attL* et *attR* contiennent tous les sites nécessaires à la recombinaison site-spécifique permettant l'excision de pSAM2 ou de tout fragment d'ADN situé entre ces deux régions (Sezonov *et al.,* 1997, Raynal *et al.*, 1998). La construction d'une telle cassette n'est évidemment pas limitée à l'utilisation d'une cassette Ω*hyg,* mais d'autres cassettes de résistances peuvent servir de base à la construction de cette cassettte (par exemple la cassette Ω*aac* ou Ω*vph* (Blondelet Rouault *et al.,* 1997)).

Après l'obtention de cette construction, la souche de *Streptomyces* est transformée avec le plasmide recombinant. Les transformants sont ensuite sélectionnés avec l'antibiotique correspondant à la cassette présente dans le gène cible (cf. figure 9, antibiotique B, il s'agit par exemple d'une sélection par l'hygromycine si la cassette excisable dérive de la cassette Ω*hyg*). On sélectionne ainsi un mélange de clones parmi lesquels il y a eu intégration par un seul ou par deux événements de recombinaison. Dans un deuxième temps on recherche les clones sensibles à l'antibiotique pour lesquels le gène de résistance est présent dans le vecteur (hors de la cassette de recombinaison) (cf. figure 9, antibiotique A). On peut ainsi sélectionner lés clones pour lesquel il y a eu en principe deux événements de recombinaison aboutissant au remplacement du gène sauvage par la copie interrompue par la cassette. Ces étapes sont schématisées figure 9, le génotype des clones ainsi obtenus est vérifié par Southern blot et une souche présentant les caractéristiques voulues (le remplacement du gène sauvage par la copie interrompue par la cassette excisable) est sélectionnée.

Dans un deuxième temps, la souche selectionnée ci-dessus, est transformée par un plasmide permettant l'expression des gènes *xis* et *int* qui sont tous deux nécessaires à la recombinaison spécifique de site entre les sites *attR* et *attL.* Cette recombinaison entraîne le départ de la cassette excisable du génome de la souche, grâce à un événement de recombinaison (cf. figure 10) (Raynal *et al.*, 1998). Il est intéressant de choisir le vecteur portant les gènes *xis* et *int* parmi les vecteurs relativement instables chez *Streptomyces* (par exemple dérivé du vecteur *Streptomyces* pWHM3, (Vara *et al.,* 1989)), ceci permet d'obtenir une souche ayant perdu ce dernier vecteur après quelques cycles de sporulation en absence de pression de sélection.

Pour exciser la cassette, on peut par exemple utiliser le plasmide pOSV508 (cf. figure 14) qui est introduit par transformation de protoplastes dans la souche de *S. ambofaciens* contenant un gène interrompu par la cassette excisable. Le plasmide pOSV508 est dérivé du plasmide pWHM3 (Vara J *et al*.,1989) (cf. figure 13) dans lequel ont été ajoutés les gènes *xis* et *int* de pSAM2 (Boccard F. *et al.,* 1989b) placés sous le contrôle du promoteur ptrc (Amann, *E. et al.,* 1988). Les gènes *xis* et *int* placés sous le contrôle du promoteur ptrc ont été sous clonés dans le plasmide pWHM3 à partir du plasmide pOSint3 (Raynal *et al.*, 1998) (cf. figure 14). L'introduction dans la souche mutante du plasmide pOSV508 portant les gènes *xis* et *int* de pSAM2 va permettre l'excision efficace par recombinaison spécifique de site de la cassette excisable entre les sites *attL* et *attR* flanquant cette cassette (Raynal A. *et al..,* 1998) (figure 10). Parmi les transformants, sélectionnés pour leur résistance au thiostrepton due au gène *tsr* porté par pOSV508, on choisit ceux devenus sensibles à l'antibiotique auquel la présence de la cassette confère la résistance (cf. figure 10). L'excision est efficace et il a été observé que plus de 90% des transformants sont de ce type. Après un ou plusieurs cycles de croissance et sporulation sur un milieu solide dépourvu de thiostrepton, on obtient des clones ayant perdu le plasmide pOSV508. Ces clones sont repérables par leur sensibilité au thiostrepton. La séquence du gène cible délété peut être vérifiée par PCR et séquençage du produit PCR.

Finalement, la souche résultante porte au niveau du gène inactivé (délétion interne par exemple) un site att « cicatriciel » correspondant au site *attB* minimal (Raynal *et al.,* 1998), issu de la recombinaison entre les sites *attR* et *attL.* Ce site *attB* minimal qui subsiste est semblable à celui présent naturellement dans les souches de *Streptomyces ambofaciens, Streptomyces pristinaespiralis* et *Streptomyces lividans* (Sezonov *et al.,* 1997).

Le gène que l'on veut inactiver peut être cotranscrit avec d'autres gènes situés en aval. Pour éviter que l'inactivation d'un des gènes ait un effet polaire sur l'expression des gènes en aval dans l'opéron, il est important d'obtenir une délétion en phase après excision de la cassette. Le système des cassettes excisables tel que décrit ci-dessus permet de répondre à une telle exigence. L'homme du métier pourra, en effet, aisément construire trois cassettes excisables distinctes, celles-ci laissant après excision une séquence de 33, 34 ou 35 nucléotides respectivement, sans codon stop quelle que soit la phase de lecture. Connaissant la séquence du gène cible et la taille de la délétion associée à l'insertion de la cassette, il est possible de choisir entre ces trois cassettes excisables de façon à ce que l'excision conduise à une délétion en phase. Sur les 33, 34 ou 35 nucléotides rajoutés, 26 correspondent à la séquence *attB* minimale (cf. figure 27).

Dans le cas de la présente demande, deux cassettes excisables ont été utilisées. Ces deux cassettes sont les suivantes : *att1Ωhyg+* (SEQ ID N° 91) et *att3Ωaac-* (SEQ ID N° 92), ces cassettes laissent respectivement 33 et 35 nucléotides après excision. Elles comportent respectivement la cassette *Ωhyg* ou la cassette *Ωaac,* les signes + et - correspondant à l'orientation de la cassette de résistance. Ces deux cassettes ont été construites et clonées au niveau du site *EcoR*V du vecteur pBC SK+ dont le site *Hind*III a été suprimé au préalable. Les plasmides obtenus ont été nommés patt1Ωhyg+ et patt3Ωaac- respectivement. Les cassettes excisables peuvent être facilement sorties par une digestion du plasmide par *EcoR*V*.*

### EXEMPLE 10: Construction d'une souche de Streptomyces ambofaciens interrompue dans le gène orf2 (ne fait pas partie de l'invention)

L'inactivation du gène *orf2* a été réalisée grâce à la technique des cassettes excisables (cf. ci-dessus). La souche de départ utilisée est la souche *Streptomyces ambofaciens* OSC2 qui dérive de la souche ATCC23877. Cependant, la souche OSC2 diffère de la souche ATCC23877 en ce qu'elle a perdu l'élément génétique mobile pSAM2 (Boccard *et al.*, 1989a et b). Cet élément mobile peut être perdu de manière spontanée au cours de la protoplastisation (action du lysozyme pour digérer la paroi bactérienne et fragmenter le mycélium (Kieser *et al.,* 2000)) et de la régénération des protoplastes de la souche ATCC23877. Pour sélectionner les clones ayant perdu l'élément pSAM2, il a été mis en place un crible, basé sur la répression du gène *pra* par le répresseur de transcription KorSA (Sezonov *et al.*, 1995) (Sezonov G. *et al.,* 2000). Pour cela, un fragment d'ADN contenant le promoteur du gène *pra* placé en amont du gène *aph* (conférant la résistance à la kanamycine et dépourvu de son propre promoteur) a été cloné dans le vecteur instable pWHM3Hyg, ce dernier dérivant du plasmide pWHM3 (Vara et al., 1989) dans lequel le gène *tsr* a été remplacé par le gène *hyg* (conférant la résistance à l'hygromycine). Le plasmide ainsi obtenu a été nommé pOSV510. La souche ATCC23877 a été transformée après protoplastisation par le plasmide pOSV510. Le promoteur Pra est un promoteur réprimé par le répresseur KorSA, le gène codant ce dernier étant situé au sein de l'élément mobile pSAM2 (Sezonov G. *et al.,* 2000). Après transformation par le plasmide pOSV510, les bactéries transformées sont sélectionnées pour leur résistance à la kanamycine (due au gène *aph* porté par pOSV510). Les clones ayant perdu l'élément intégratif pSAM2 ont perdu le répresseur KorSA et le promoteur Pra n'est donc plus réprimé et permet l'expression du gène de résistance à la kanamycine *aph.* Une sélection par la kanamycine après transformation par le plasmide pOSV510 permet donc de sélectionner les clones ayant perdu l'élément intégratif pSAM2 (et donc KorSA) et possédant le plasmide pOSV510. Le plasmide pOSV510 étant instable, après quelques cycles de sporulation sans antibiotique, des clones isolés sont repiqués sur milieu avec kanamycine, sur milieu avec hygromycine et sur milieu sans antibiotique. Les clones sensibles à la kanamycine et à l'hygromycine ont perdu pOSV510. La perte de l'élément pSAM2 a été vérifiée par hybridation et PCR. Un clone présentant les caractéristiques souhaitées a été sélectionné et a été nommé OSC2.

Un échantillon de la souche OSC2 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement I-2908.

L'inactivation du gène *orf2* a été réalisée grâce à la technique des cassettes excisables (cf. ci-dessus et figure 10). Pour cela, un insert de 4,5 kb dont la séquence part du site *EcoR*I situé en position 1 jusqu'au site *Bam*HI situé en position 4521 (SEQ ID N° 1) a été sous-cloné au niveau des sites *Eco*RI *et Bam*HI du plasmide pUC19 (numéro d'accès GenBank M77789) à partir du cosmide pSPM5. Le plasmide ainsi obtenu a été nommé pOS49.99.

Ce plasmide a été introduit dans la souche *E. coli KS272* qui contenait déjà le plasmide pKOBEG *(Chaveroche et al.,* 2000) (cf. figure 12).

En parallèle, la cassette excisable *att3Ωaac-* (SEQ ID N° 92, cf. ci dessus) a été amplifiée par PCR en utilisant comme matrice le plasmide pOSK1102 (Le plasmide pOSK1102 est un plasmide dérivé du vecteur pGP704Not *(Chaveroche et al.,* 2000) (Miller VL & Mekalanos JJ, 1988) dans lequel la cassette *att3Ωaac-* a été clonée comme un fragment *Eco*RV dans le site unique *Eco*RV de pGP704Not) et à l'aide des amorces suivantes :
ORF2A
   5' CCCGCGCGGCAGCCTCTCCGTGATCGAGTCCGGCGTGACC**ATCGCGCGCGCTTCGTTCGG**-3' (SEQ ID N° 93)
ORF2B
   5' GCTCCGTGCGTCATGCAGGAAGGTGTCGTAGTCGCGGTAG**ATCTGCCTCTTCGTCCCGAA**-3' (SEQ ID N° 94)

Les 40 déoxy-nucléotides situés à l'extrémité 5' de ces oligonucléotides comportent une séquence correspondant à une séquence dans le gène cible (*orf2* dans le cas présent) et les 20 déoxy-nucléotides situés le plus en 3' (figuré en gras ci-dessus) correspondent à la séquence d'une des extrémités de la cassette excisable *att3Ωaac-* (cf. figure 11).
Le produit de PCR ainsi obtenu a été utilisé pour transformer la souche *E. coli* contenant les plasmides pKOBEG et pOS49.99 comme décrit *(Chaveroche et al.,* 2000) (cf. figure 12). Ainsi, les bactéries ont été transformées par électroporation et sélectionnées pour leur résistance à l'apramycine. Les plasmides des clones obtenus ont été extraits et digérés par plusieurs enzymes de restriction, dans le but de vérifier que le profil de digestion obtenu correspond au profil attendu s'il y a eu insertion de la cassette (*att3Ωaac*-) dans le gène cible (*orf2*), c'est à dire si il y a bien eu recombinaison homologue entre les extrémités du produit PCR et le gène cible (Chaveroche *et al.,* 2000). La vérification de la construction peut également être réalisée par toute méthode connue de l'homme du métier et notamment par PCR en utilisant les oligonucléotides appropriés et séquençage du produit de PCR. Un clone dont le plasmide possède le profil attendu a été sélectionné et le plasmide correspondant a été nommé pSPM17. Ce plasmide dérive de pOS49.99 dans lequel *orf2* est interrompue par la cassette apramycine (cf. figure 12).
L'insertion de la cassette s'accompagne d'une délétion dans *orf2,* entre les nucléotides 211 et 492 de la partie codante de *orf2.*
Le plasmide pSPM17 a été digéré par l'enzyme *Eco*RI puis les extrémités ont été rendues bout franc par traitement à l'enzyme de Klenow, ce produit de digestion a été ensuite digéré par l'enzyme *Xba*I et l'insert contenant le gène *orf2* délété a été cloné dans le vecteur pOSK1205 (cf. ci dessus). Pour cela, le vecteur pOSK1205 a été digéré par l'enzyme *Bam*HI puis les extrémités ont été rendues bout franc par traitement à l'enzyme de Klenow, ce produit a été ensuite digéré par l'enzyme *Xba*I*,* et utilisé pour la ligation avec l'insert obtenu à partir de pSPM17 comme ci-dessus. Cette manipulation permet donc d'obtenir une ligation orientée puisque chacun des deux fragments est bout franc d'un côté et *Xba*I de l'autre. Le plasmide ainsi obtenu a été nommé pSPM21, il porte un gène de résistance à l'hygromycine (partie vecteur) et un insert dans lequel le gène *orf2* délété est remplacé par la cassette *att3Ωaac-.*

Le vecteur pSPM21 a été introduit dans la souche *Streptomyces ambofaciens* OSC2 (cf. ci dessus) par transformation de protoplastes (Kieser, T *et al.*, 2000). Après transformation, les clones sont sélectionnés pour leur résistance à l'apramycine Les clones résistants à l'apramycine sont ensuite repiqués respectivement sur milieu avec apramycine (antibiotique B) et sur milieu avec hygromycine (antibiotique A) (cf. figure 9). Les clones résistants à l'apramycine (ApraR) et sensibles à l'hygromycine (HygS) sont en principe ceux où un double événement de crossing over s'est produit et qui possèdent le gène *orf2* interrompu par la cassette *att3Ωaac-.* Ces clones ont été sélectionnés et le remplacement de la copie sauvage de *orf2* par la copie interrompue par la cassette a été vérifié. La présence de la cassette *att3Ωaac-* a été vérifiée par PCR sur colonie. Une hybridation a également été effectuée. Pour cela, l'ADN total des clones obtenus, a été digéré par plusieurs enzymes, séparé sur gel d'agarose, transféré sur membrane et hybridé avec une sonde de 3 kb correspondant à un fragment *Eco*RI-*Bam*HI de l'insert du plasmide pOS49.99 (cf. ci-dessus). La vérification du génotype peut également être réalisée par toute méthode connue de l'homme du métier et notamment par PCR en utilisant les oligonucléotides appropriés et séquençage du produit de PCR.

Un clone présentant les caractéristiques attendues a été sélectionné et nommé SPM21. Il a pu être vérifié grâce à la PCR et à l'hybridation que la cassette *att3Ωaac-*était bien présente dans le génome de ce clone et que l'on obtient bien le profil de digestion attendu dans le cas d'un remplacement, à la suite d'un double événement de recombinaison, du gène sauvage par la copie interrompue par la cassette *att3Ωaac-* dans le génome de ce clone. Ce clone possède donc le génotype : *orf2::att3Ωaac-.*

Un échantillon de la souche SPM21 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement I-2914.

La souche SPM21 a été transformée par le vecteur pOSV508 par transformation de protoplastes pour provoquer l'excision de la cassette (cf. figure 14). Le plasmide pOSV508 est dérivé du plasmide pWHM3 (Vara *J et al.*, 1989) (cf. figure 13) dans lequel il a été ajouté les gènes *xis* et *int* de pSAM2 (Boccard F. *et al.,* 1989b) placé sous le contrôle du promoteur ptrc (Amann, E. *et al.,* 1988) (cf. figure 14). L'introduction dans la souche SPM21 du plasmide pOSV508 portant les gènes *xis* et *int* de pSAM2 permet l'excision efficace par recombinaison spécifique de site de la cassette excisable entre les sites *attL* et *attR* flanquant cette cassette (Raynal *A. et al.*, 1998) (figure 10). Parmi les transformants sélectionnés pour leur résistance au thiostrepton due au gène *tsr* porté par pOSV508, on choisit ceux devenus sensibles à l'apramycine dont le gène de résistance est porté par la cassette *att3Ωaac-,* l'excision entraîne en effet la perte de ce gène de résistance (cf. figure 10). Le plasmide pOSV508 est instable et après deux passages successifs sur milieu sans antibiotique des clones isolés sont repiqués sur milieu avec thiostrepton et sur milieu sans thiostrepton. Les clones sensibles au thiostrepton ont perdu pOSV508. Il a été vérifié que l'excision de la cassette aboutit bien à une délétion en phase dans le gène *orf2* par PCR et séquençage du produit PCR, l'excision de la cassette laisse en effet une séquence « cicatricielle » att3 caractéristique (qui est similaire au site *attB* d'origine après recombinaison entre les sites *attL* et *attR) :*
5' ATCGCGCGCGCTTCGTTCGGGACGAAGAGGTAGAT 3' (SEQ ID N° 95).
La souche ainsi obtenue et possédant le génotype voulu (*orf2::att3*) a été nommée SPM22.

Un échantillon de la souche SPM22 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement I-2915.

### EXEMPLE 11 : Construction d'une souche de Streptomyces ambofaciens interrompue dans le gène orf12 (ne fait pas partie de l'invention)

Pour l'inactivation de *off12, orf13c* et *orf14* un même plasmide de départ (pSPM504) a été employé pour introduire à différentes positions une cassette de type «cassette excisable ». Ce plasmide possède un insert de 15,1 kb qui correspond à la région de *orf7* à *orf17.* Pour construire ce plasmide un fragment *Bgl*II de 15,1 kb provenant de la digestion du cosmide pSPM7 (cf. ci-dessus) a été cloné dans le plasmide pMAL18 (Nakano *et al.,* 1995) digéré par *Bam*HI*.* Les extrémités *Bam*HI et *Bgl*II étant compatibles, on obtient après ligation, le plasmide pSPM502. La totalité de l'insert de pSPM502 a ensuite été sous-cloné (sous forme d'un fragment *Hind*IIII/*Nhe*I) dans le plasmide pOSK1205 (digéré par *Hind*IIII/*Nhe*I) ce qui a permis d'obtenir le plasmide pSPM504.

Ce plasmide a été introduit dans la souche *E. coli* KS272 qui contenait déjà le plasmide pKOBEG (Chaveroche *et al.,* 2000) (cf. figure 12).

En parallèle, la cassette excisable *att3Ωaac-* a été amplifiée par PCR en utilisant comme matrice le plasmide pOSK1102 (le plasmide pOSK1102 est un plasmide dérivé du vecteur pGP704Not *(Chaveroche et al.,* 2000) (Miller VL & Mekalanos JJ, 1988) dans lequel la cassette *att3Ωaac-* a été clonée comme un fragment *EcoRV* dans le site unique *EcoRV* de pGP704Not), les amorces utilisées sont les suivantes :
EDR8:5' CGGGATGATCGCTTGTCCGGCGGCCGGATGCCTAGCCTC**ATCGCGCGCGCTTCGTTCGG** 3' (SEQ ID N° 96)
EDR9:5' CCCGATCCAGAACGTCTGGTCGGTGATCAGGTCGCTGTTC**ATCTGCCTCTTCGTCCCGAA** 3' (SEQ ID N° 97)

Les 40 (seulement 39 pour EDR8) déoxy-nucléotides situés à l'extrémité 5' de ces oligonucléotides comportent une séquence correspondant à une séquence dans le gène cible (*orf12* dans le cas présent) et les 20 déoxy-nucléotides situés le plus en 3' (figuré en gras ci-dessus) correspondent à la séquence d'une des extrémités de la cassette excisable *att.3Ωaac-* (cf. figure 11).

Le produit de PCR ainsi obtenu a été utilisé pour transformer la souche *E. coli KS272* contenant les plasmides pKOBEG et pSPM504 (cf. ci-dessus), comme décrit par Chaveroche *et al.* (Chaveroche *et al.,* 2000) (cf. figure 12 pour le principe, le plasmide pOS49.99 doit être remplacé par le plasmide pSPM504 et le plasmide obtenu n'est plus pSPM17 mais pSPM507). Ainsi, les bactéries ont été transformées par électroporation par ce produit de PCR et les clones ont été sélectionnés pour leur résistance à l'apramycine. Les plasmides des clones obtenus ont été extraits et digérés par plusieurs enzymes de restriction, dans le but de vérifier que le profil de digestion obtenu correspond au profil attendu s'il y a eu insertion de la cassette (*att3Ωaac-*) dans le gène cible (*orf12*), c'est à dire si il y a bien eu recombinaison homologue entre les extrémités du produit PCR et le gène cible (Chaveroche *et al.,* 2000). La vérification de la construction peut également être réalisée par toute méthode connue de l'homme du métier et notamment par PCR en utilisant les oligonucléotides appropriés et séquençage du produit de PCR. Un clone dont le plasmide possède le profil attendu a été sélectionné et le plasmide correspondant a été nommé pSPM507. Ce plasmide dérive de pSPM504 dans lequel *orf12* est interrompue par la cassette *att3Ωaac-* (cf. figure 12). L'insertion de la cassette s'accompagne d'une délétion dans le gène *orf12*, l'interruption commence au niveau du trentième codon de *orf12*. Il reste après la cassette les 46 derniers codons de *orf12*.

Le vecteur pSPM507 a été introduit dans la souche *Streptomyces ambofaciens* OSC2 (cf. ci-dessus) par transformation de protoplastes (Kieser, T *et al.,* 2000). Après transformation, les clones sont sélectionnés pour leur résistance à l'apramycine. Les clones résistants à l'apramycine sont ensuite repiqués respectivement sur milieu avec apramycine (antibiotique B) et sur milieu avec hygromycine (antibiotique A) (cf. figure 9). Les clones résistants à l'apramycine (ApraR) et sensibles à l'hygromycine (HygS) sont en principe ceux où un double événement de crossing over s'est produit et qui possèdent le gène *orf12* interrompu par la cassette *att3Ωaac-.* Ces clones ont été plus particulièrement sélectionnés et le remplacement de la copie sauvage de *orf12* par la copie interrompue par la cassette a été vérifié par hybridation. Ainsi, l'ADN total des clones obtenus, a été digéré par plusieurs enzymes, séparé sur gel d'agarose, transféré sur membrane et hybridé avec une sonde correspondant à la cassette *att3Ωaac-* pour vérifier la présence de la cassette dans l'ADN génomique des clones obtenus. Une deuxième hybridation a été effectuée en utilisant comme sonde un fragment d'ADN obtenu par PCR et correspondant à une très large partie de la séquence codante du gène *orf12.*

La vérification du génotype peut également être réalisée par toute méthode connue de l'homme du métier et notamment par PCR en utilisant les oligonucléotides appropriés et séquençage du produit de PCR.

Un clone présentant les caractéristiques attendues (*orf12*::*att3Ωaac*-) a été plus particulièrement sélectionné et nommé SPM507. Il a pu en effet être vérifié grâce aux deux hybridations que la cassette *att3Ωaac*- était bien présente dans le génome de ce clone et que l'on obtient bien le profil de digestion attendu dans le cas d'un remplacement, à la suite d'un double événement de recombinaison, du gène sauvage par la copie interrompue par la cassette *att3Ωaac-* dans le génome de ce clone. Ce clone possède donc le génotype : *orf12::att3Ωaac-* et a été nommé SPM507. Il est inutile de procéder à l'excision de la cassette pour l'étude de l'effet de l'inactivation de *orf12,* au vu de l'orientation des gènes (cf. figure 3). En effet, le fait que *orf13c* soit orienté en sens opposé à *orf12* montre que ces gènes ne sont pas co-transcrits. L'utilisation d'une cassette excisable permet en revanche d'avoir la possibilité de se débarrasser du marqueur de sélection à tout moment, notamment par transformation par le plasmide pOSV508. Un échantillon de la souche SPM507 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement 1-2911.

### EXEMPLE 12: Construction d'une souche de Streptomyces ambofaciens interrompue dans le gène orf13c (ne fait pas partie de l'invention)

La cassette excisable *att3Ωaac-* a été amplifiée par PCR en utilisant comme matrice le plasmide pOSK1102 (cf. ci-dessus) à l'aide des amorces suivantes :
EDR3:5' ACCGGGGCGGTCCTCCCCTCCGGGGCGTCACGGCCGCGGA**ATCTGCCTCTTCGTCCCGAA 3**' (SEQ ID N° 98)
EDR4:5' CACGCAGCGAGCCGACGCACTGATGGACGACACGATGGCCP**ATCGCGCGCGCTTCGTTCGG 3'** (SEQ ID N° 99)

Les 40 déoxy-nucléotides situés à l'extrémité 5' de ces oligonucléotides comportent une séquence correspondant à une séquence dans le gène cible (*orf13c* dans le cas présent) et les 20 déoxy-nucléotides situés le plus en 3' (figuré en gras ci-dessus) correspondent à la séquence d'une des extrémités de la cassette excisable *att3Ωaac-* (cf. figure 11).

Le produit de PCR ainsi obtenu a été utilisé pour transformer la souche *E. coli KS272* contenant les plasmides pKOBEG et pSPM504 (cf. ci-dessus), comme décrit par Chaveroche *et al.* (Chaveroche *et al..,* 2000) (cf. figure 12 pour le principe, le plasmide pOS49.99 doit être remplacé par le plasmide pSPM504 et le plasmide obtenu n'est plus pSPM17 mais pSPM508). Ainsi, les bactéries ont été transformées par électroporation par le produit de PCR et les clones ont été sélectionnés pour leur résistance à l'apramycine. Les plasmides des clones obtenus ont été extraits et digérés par plusieurs enzymes de restriction, dans le but de vérifier que le profil de digestion obtenu correspond au profil attendu s'il y a eu insertion de la cassette (*att3Ωaac*-) dans le gène cible (*orf13c*), c'est à dire si il y a bien eu recombinaison homologue entre les extrémités du produit PCR et le gène cible (Chaveroche *et al.,* 2000). La vérification de la construction peut également être réalisée par toute méthode connue de l'homme du métier et notamment par PCR en utilisant les oligonucléotides appropriés et séquençage du produit de PCR. Un clone dont le plasmide possède le profil attendu a été sélectionné et le plasmide correspondant a été nommé pSPM508. Ce plasmide dérive de pSPM504 dans lequel *orf13c* est interrompue par la cassette apramycine (cf. figure 12). L'insertion de la cassette s'accompagne d'une délétion dans le gène *orf13c,* l'interruption commence au niveau du sixième codon de *orf13c.* Il reste après la cassette les 3 derniers codons de *orf13c.*

Le vecteur pSPM508 a été introduit dans la souche *Streptomyces ambofaciens* OSC2 (cf. ci-dessus) par transformation de protoplastes (Kieser, *T et al.,* 2000). Après transformation, les clones sont sélectionnés pour leur résistance à l'apramycine. Les clones résistants à l'apramycine sont ensuite repiqués respectivement sur milieu avec apramycine (antibiotique B) et sur milieu avec hygromycine (antibiotique A) (cf. figure 9). Les clones résistants à l'apramycine (ApraR) et sensibles à l'hygromycine (HygS) sont en principe ceux où un double événement de crossing over s'est produit et qui possèdent le gène *orf13c* interrompu par la cassette *att3Ωaac-.* Ces clones ont été plus particulièrement sélectionnés et le remplacement de la copie sauvage de *orf13c* par la copie interrompue par la cassette a été vérifié par hybridation. Ainsi, l'ADN total des clones obtenus, a été digéré par plusieurs enzymes, séparé sur gel d'agarose, transféré sur membrane et hybridé avec une sonde correspondant à la cassette *att3Ωaac-* pour vérifier la présence de la cassette dans l'ADN génomique des clones obtenus. Une deuxième hybridation a été effectuée en utilisant comme sonde un produit de PCR correspondant à une séquence débordant d'une centaine de paires de base en amont et en aval de la séquence codante de *orf13c.* La vérification du génotype peut également être réalisée par toute méthode connue de l'homme du métier et notamment par PCR en utilisant les oligonucléotides appropriés et séquençage du produit de PCR.

Un clone présentant les caractéristiques attendues (*or13c::att3Ωaac*-) a été plus particulièrement sélectionné et nommé SPM508. Il a pu en effet être vérifié grâce aux deux hybridations que la cassette *att3Ωaac-* était bien présente dans le génome de ce clone et que l'on obtient bien le profil de digestion attendu dans le cas d'un remplacement, à la suite d'un double événement de recombinaison, du gène sauvage par la copie interrompue par la cassette *att3Ωaac-* dans le génome de ce clone. Ce clone possède donc le génotype : *orf13c::att3Ωaac-* et a été nommé SPM508. Il est inutile de procéder à l'excision de la cassette pour l'étude de l'effet de l'inactivation de *orf13c,* au vu de l'orientation des gènes (cf. figure 3), le fait que *orf14* soit orienté en sens opposé à *orf13c* montre que ces gènes ne sont pas co-transcrits. L'utilisation d'une cassette excisable permet en revanche d'avoir la possibilité de se débarrasser du marqueur de sélection à tout moment. Un échantillon de la souche SPM508 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement 1-2912.

### EXEMPLE 13 Construction d'une souche de Streptomyces ambofaciens interrompue dans le gène orf14 (ne fait pas partie de l'invention)

La cassette excisable *att3Ωaac-* a été amplifiée par PCR en utilisant comme matrice le plasmide pOSK1 102 (cf. ci-dessus) à l'aide des amorces suivantes :
EDR5: 5'GGGCGTGAAGCGGGCGAGTGTGGATGTCATGCGAGTACTCATCGCGCGCGCTTCGTTCGG 3' (SEQ ID N° 100)
EDR6: 5'CGGGAAACGGCGTCGCACTCCTCGGGGGCCGCGTCAGCCCATCTGCCTCTTCGTCCCGAA 3' (SEQ ID N° 101)

Les 40 déoxy-nucléotides situés à l'extrémité 5' de ces oligonucléotides comportent une séquence correspondant à une séquence dans le gène cible (*orf14* dans le cas présent) et les 20 déoxy-nucléotides situés le plus en 3' (figuré en gras ci-dessus) correspondent à la séquence d'une des extrémités de la cassette excisable *att3Ωaac-* (cf. figure 11).

Le produit de PCR ainsi obtenu a été utilisé pour transformer la souche *E. coli KS272* contenant les plasmides pKOBEG et pSPM504 (cf. ci-dessus), comme décrit par Chaveroche *et al.* (Chaveroche *et al.,* 2000) (cf. figure 12 pour le principe, le plasmide pOS49.99 doit être remplacé par le plasmide pSPM504 et le plasmide obtenu n'est plus pSPM17 mais pSPM509). Ainsi, les bactéries ont été transformées par électroporation par le produit de PCR et les clones ont été sélectionnés pour leur résistance à l'apramycine. Les plasmides des clones obtenus ont été extraits et digérés par plusieurs enzymes de restriction, dans le but de vérifier que le profil de digestion obtenu correspond au profil attendu s'il y a eu insertion de la cassette (*att3Ωaac*-) dans le gène cible (*orf14*), c'est à dire si il y a bien eu recombinaison homologue entre les extrémités du produit PCR et le gène cible (Chaveroche *et al.,* 2000). La vérification de la construction peut également être réalisée par toute méthode connue de l'homme du métier et notamment par PCR en utilisant les oligonucléotides appropriés et séquençage du produit de PCR. Un clone dont le plasmide possède le profil attendu a été sélectionné et le plasmide correspondant a été nommé pSPM509. Ce plasmide dérive de pSPM504 dans lequel *orf14* est interrompu par la cassette apramycine (cf. figure 12). L'insertion de la cassette s'accompagne d'une délétion dans le gène *orf14,* l'interruption commence au niveau du quatrième codon de *orf14.* Il reste après la cassette le dernier codon de *orf14.*

Le vecteur pSPM509 a été introduit dans la souche *Streptomyces ambofaciens.* OSC2 (cf. ci-dessus) par transformation de protoplastes (Kieser, *T et al.,* 2000). Après transformation, les clones sont sélectionnés pour leur résistance à l'apramycine. Les clones résistants à l'apramycine sont ensuite repiqués respectivement sur milieu avec apramycine (antibiotique B) et sur milieu avec hygromycine (antibiotique A) (cf. figure 9). Les clones résistants à l'apramycine (ApraR) et sensibles à l'hygromycine (HygS) sont en principe ceux où un double événement de crossing over s'est produit et qui possèdent le gène *orf14* interrompu par la cassette *att3Ωaac-.* Ces clones ont été plus particulièrement sélectionnés et le remplacement de la copie sauvage de *orf14* par la copie interrompue par la cassette a été vérifié par hybridation. Ainsi, l'ADN total des clones obtenus, a été digéré par plusieurs enzymes, séparé sur gel d'agarose, transféré sur membrane et hybridé avec une sonde correspondant à la cassette *att3Ωaac-* pour vérifier la présence de la cassette dans l'ADN génomique des clones obtenus. Une deuxième hybridation a été effectuée en utilisant comme sonde un produit de PCR correspondant à une séquence débordant d'une centaine de paires de base en amont et en aval de la séquence codante de *orf14.* La vérification du génotype peut également être réalisée par toute méthode connue de l'homme du métier et notamment par PCR en utilisant les oligonucléotides appropriés et séquençage du produit de PCR.

Un clone présentant les caractéristiques attendues (*orf14::att3Ωaac*-) a été plus particulièrement sélectionné et nommé SPM509. Il a pu en effet être vérifié grâce aux deux hybridations que la cassette *att3Ωaac-* était bien présente dans le génome de ce clone et que l'on obtient bien le profil de digestion attendu dans le cas d'un remplacement, à la suite d'un double événement de recombinaison, du gène sauvage par la copie interrompue par la cassette *att3Ωaac-* dans le génome de ce clone. Ce clone possède donc le génotype : *orf14::att3Ωaac-* et a été nommé SPM509. Il est inutile de procéder à l'excision de la cassette pour l'étude de l'effet de l'inactivation de *orf14,* au vu de l'orientation des gènes (cf. figure 3), le fait que *orf15c* soit orienté en sens opposé à *orf14* montre que ces gènes ne sont pas co-transcrits. L'utilisation d'une cassette excisable permet en revanche d'avoir la possibilité de se débarrasser du marqueur de sélection à tout moment. Un échantillon de la souche SPM509 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement 1-2913.

### EXEMPLE 14: Construction d'une souche de Streptomyces ambofaciens interrompue dans le gène orf6* (ne fait pas partie de l'invention)

L'inactivation du gène *orf6** a été réalisée grâce à la technique des cassettes excisables (cf. ci-dessus et figure 10). Pour cela, le cosmide pSPM7 a été utilisé comme matrice pour amplifier un fragment du gène *orf6** grâce aux oligonucléotides suivants :
C9583 : 5' **CTGCAG**GTGCTCCAGCGCGTCGATCT 3' (oligo sens) (SEQ ID N° 102)
C9584 : 5' **CTGCAG**ACGGAGGCGGACCTGCGGCT 3' (oligo antisens) (SEQ ID N° 103)

Les 20 déoxy-nucléotides situés à l'extrémité 3' de ces oligonucléotides correspondent à une séquence située dans la partie codante du gène *orf6** (SEQ ID N° 13) et les 6 déoxy-nucléotides situés les plus en 5' correspondent à la séquence d'un site *PstI* permettant de faciliter le clonage par la suite. Le fragment d'ADN amplifié fait une taille d'environ 1,11 kb. Ce produit de PCR est cloné dans le vecteur pGEM-T Easy (commercialisé par la société Promega (Madison, Wisconcin, USA)) ce qui a permis d'obtenir le plasmide nommé pBXL1111 (cf. figure 16).

La cassette excisable *att1Ωhyg*+ a ensuite été introduite dans la séquence codante du gène *orf6*.* Pour cela, le plasmide pBXL1111 a été digéré par les enzymes de restriction *Sma*I et *Asp*718I et le produit de digestion a été traité par l'enzyme de Klenow. Cette manipulation permet de réaliser une délétion interne de 120 pb dans la séquence codante du gène *orf6** (cf. figure 15). De plus, de part et d'autre des sites de restrictions, il reste respectivement 511 pb et 485 pb de la séquence de *orf6** qui permettront la recombinaison homologue pour l'inactivation du gène *orf6*.* La cassette excisable *att1Ωhyg*+ a été préparée à partir du plasmide patt1Ωhyg+ (cf. ci-dessus) par digestion de ce plasmide par *EcoRV.* Cette dernière a ensuite été sous-clonée dans le vecteur pBXL1111 préalablement préparé comme décrit ci-dessus (digestion *Sma*I et *Asp*718I puis traitement à l'enzyme de Klenow). Le plasmide obtenu a été nommé pBXL1112 (cf. figure 17). Dans cette construction, le gène *orf6** comporte une délétion de 120pb et est interrompu par la cassette *att1Ωhyg*+*.*

Le plasmide pBXL1112 a été ensuite digéré par l'enzyme *PstI* (site bordant la cassette puisque présent dans les oligonucléotides PCR) et l'insert *PstI* de 3.7 kb comprenant une partie de *orf6** interrompu par la cassette *att1Ωhyg*+ a ensuite été cloné au niveau du site *Pst*I du plasmide pOJ260 (cf. ci-dessus). Le plasmide ainsi obtenu a été nommé pBXL1 113.

Le vecteur pBXL1113 a été introduit dans la souche *Streptomyces ambofaciens* OSC2 (cf. ci-dessus) par transformation de protoplastes (Kieser, **T** *et al*., 2000). Après transformation, les clones sont sélectionnés pour leur résistance à l'hygromycine. Les clones résistants à l'hygromycine sont ensuite repiqués respectivement sur milieu avec hygromycine (antibiotique B) et sur milieu avec apramycine (antibiotique A) (cf. figure 9). Les clones résistants à l'hygromycine (HygR) et sensibles à l'apramycine (ApraS) sont en principe ceux où un double événement de crossing over s'est produit et qui possèdent le gène *orf6** interrompu par la cassette *att1Ωhyg*+*.* Ces clones ont été plus particulièrement sélectionnés et le remplacement de la copie sauvage de *orf6** par la copie interrompue par la cassette a été vérifié par la technique du Southern blot. Ainsi, l'ADN total des clones obtenus, a été digéré par plusieurs enzymes, séparé sur gel d'agarose, transféré sur membrane et hybridé avec une sonde correspondant au gène *hyg* (obtenu par PCR) pour vérifier la présence de la cassette dans l'ADN génomique des clones obtenus. Une deuxième hybridation a été effectuée en utilisant comme sonde l'insert *Pst*I*-Pst*I contenant le gène *orf6*,* d'une taille d'environ 1,1 kb et obtenu à partir du plasmide pBXL1111 (cf. ci-dessus et figure 16). La vérification du génotype peut également être réalisée par toute méthode connue de l'homme du métier et notamment par PCR en utilisant les oligonucléotides appropriés et séquençage du produit de PCR.

Un clone présentant les caractéristiques attendues (*orf6*::att1Ωhyg*+) a été plus particulièrement sélectionné et nommé SPM501. Il a pu en effet être vérifié grâce aux deux hybridations que la cassette *att1Ωhyg*+ était bien présente dans le génome de ce clone et que l'on obtient bien le profil de digestion attendu dans le cas d'un remplacement, à la suite d'un double événement de recombinaison, du gène sauvage par la copie interrompue par la cassette *att1Ωhyg*+ dans le génome de ce clone. Ce clone possède donc le génotype : *orf6*::att1Ωhyg*+ et a été nommé SPM501. Un échantillon de la souche SPM501 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement 1-2909.

La souche SPM501 a été transformée par le vecteur pOSV508 par transformation de protoplastes pour provoquer l'excision de la cassette (cf. figure 14). Le plasmide pOSV508 est dérivé du plasmide pWHM3 (Vara **J** *et al*.,1989) (cf. figure 13) dans lequel les gènes *xis* et *int* de pSAM2 (Boccard F. *et al.,* 1989b) placés sous le contrôle du promoteur ptrc (Amann, E*. et al.,* 1988) ont été ajoutés (cf. figure 14). L'introduction dans la souche SPM501 du plasmide pOSV508 portant les gènes *xis* et *int* de pSAM2 permet l'excision efficace par recombinaison site spécifique de la cassette excisable entre les sites *attL* et *attR* flanquant cette cassette (Raynal A. *et al.,* 1998) (figure 10). Parmi les transformants, sélectionnés pour leur résistance au thiostrepton due au gène *tsr* porté par pOSV508, on choisit ceux devenus sensibles à l'hygromycine dont le gène de résistance est porté par la cassette *att1Ωhyg*+*,* l'excision entraîne en effet la perte de ce gène de résistance (cf. figure 10). Le plasmide pOSV508 est instable et après deux passages successifs sur milieu sans antibiotique des clones isolés sont repiqués sur milieu avec thiostrepton et sur milieu sans thiostrepton. Les clones sensibles au thiostrepton ont perdu pOSV508. Il a été vérifié que l'excision de la cassette a bien eu lieu en phase dans le gène *orf6** par PCR et séquençage du produit PCR. L'interruption commence au 158^{ième} codon, 40 codons sont délétés (120pb) et l'excision de la cassette laisse une séquence « cicatricielle » att1 caractéristique de 33pb : 5' ATCGCGCGCTTCGTTCGGGACGAAGAGGTAGAT 3' (SEQ ID N° 104).

La souche ainsi obtenue et possédant le génotype voulu (*orf6*::att1*) a été nommée SPM502.

Un échantillon de la souche SPM502 a été déposé auprès de la Collection Nationale de Cultures de Microorganismes (CNCM) Institut Pasteur, 25, rue du Docteur Roux 75724 Paris Cedex 15, France, le 10 juillet 2002 sous le numéro d'enregistrement I-2910.

### EXEMPLE 15 : Analyse des souches de Streptomyces ambofaciens interrompue dans les gènes orf2, orf3, orf8, orf10, orf12, orf13c, orf14, orf6*

Pour tester la production en spiramycine des diverses souches obtenues, un test microbiologique basé sur la sensibilité d'une souche de *Micrococcus luteus* a été mis au point (cf. (Gourmelen A*. et al.,* 1998)). La souche de *Micrococcus luteus* utilisée est une souche dérivée de la souche DSM1790 naturellement sensible à la spiramycine (cette souche est disponible notamment auprès de la German Collection of Microorganisms and Cell Cultures (Deutsche Sammlung von Mikro-organismen und Zellkulturen GmbH, DSMZ), (Braunschweig, Allemagne), sous le numéro DSM1790), la souche utilisée dans le présent test diffère de la souche DSM1790 en ce qu'elle est résistante à la congocidine. Cette souche est un mutant spontané obtenu par sélection sur milieu contenant des doses croissantes de congocidine. Une telle souche a été sélectionnée du fait que *Streptomyces ambofaciens* produit à la fois de la spiramycine et de la congocidine. Le but étant de doser la production en spiramycine des diverses souches obtenues grâce à un test microbiologique basé sur la sensibilité d'une souche de *Micrococcus luteus,* il est nécessaire de disposer d'une souche résistante à la congocidine.

Les différentes souches de *Streptomyces* à tester ont été cultivées dans des erlenmeyers à baffles (erlenmeyers chicanés) de 500 ml contenant 70 ml de milieu MP5 (Pernodet *et al.,* 1993). Les erlenmeyers chicanés ont été inoculés à une concentration initiale de 2.5 10⁶ spores/ml des différentes souches de *S*. *ambofaciens* et mises à pousser à 27°C sous agitation orbitale de 250 rpm. Des prélèvements de 2 ml de suspensions ont été réalisés après 48, 72 et 96 heures de culture et centrifugés. Les différents surnageants ont ensuite été congelés à -20°C. Une dilution au dixième de ces surnageants dans du milieu de culture stérile est utilisée pour le test (cf. figure 18).

La souche indicatrice de *Micrococcus luteus* résistante à la congocidine mais sensible à la spiramycine a été cultivée dans du milieu 2TY (Sambrook *et al.,* 1989) contenant de la congocidine à 5 µg/ml pendant 48h à 37°C. La densité optique (DO) de la culture est mesurée et cette culture est diluée de façon à ajuster la densité optique à 4. 0,4 ml de cette pré-culture est dilué dans 40 ml de milieu DAM5 (Difco Antibiotic Medium 5, commercialisé par la société Difco), préalablement porté à une température d'environ 45°C. Ce milieu est ensuite coulé dans une boîte carrée de 12x12 cm et laissé à reposer à température ambiante.

Une fois le milieu refroidi et solidifié, des disques en papier Whatman AA (cf. Gourmelen A. *et al.,* 1998) de 12 mm de diamètre ont été imbibés de 70 µl de la dilution au dixième de chaque surnageant et déposés sur la surface de la boîte. Des disques imbibés d'une solution de spiramycine de différentes concentrations (2-4-8 µg/ml dans du milieu de culture MP5) sont utilisés comme gamme étalon. Les boîtes sont laissées à 4°C pendant 2 h de façon à permettre la diffusion des antibiotiques dans l'agar puis sont incubées à 37°C pendant 24 à 48h.

Si le disque contient de la spiramycine, celle-ci diffuse dans l'agar et inhibe la croissance de la souche indicatrice de *Micrococcus luteus.* Cette inhibition crée un « halo » autour du disque, ce halo reflétant la zone où la souche de *Micrococcus luteus* n'a pas poussé. La présence de ce halo est donc une indication de la présence de spiramycine et permet de déterminer si la souche de *S*. *ambofaciens* correspondant au disque en question est productrice ou non productrice de spiramycine. Une comparaison avec les diamètres d'inhibition obtenus pour la gamme étalon permet d'obtenir une indication de la quantité de spiramycine produite par cette souche.

Les différentes souches décrites dans les exemples précédents ont été utilisées dans ce test pour détecter leur production en spiramycine. Les résultats obtenus ont été regroupés dans le Tableau 38.

**Tableau 38**

| **Souche** | **Gène inactivé** | **Exemple dans lequel la souche est décrite** | **Phénotype : Producteur (+) ou non-producteur (-) de spiramycine** |
|---|---|---|---|
| ATCC23877 | Aucun | 1 | (+) |
| OS49.16 | *orf3::Ωhyg* | 2 | (-) |
| OS49.67 | *orf3délétion en phase* | 6 | (-) |
| OS49.107 | *orf8::Ωhyg* | 7 | (-) |
| OS49.50 | *orf10:: Ωhyg* | 8 | (-) |
| OSC2 | Aucun | 10 | (+) |
| SPM21 | *orf2::att3Ωaac-* | 10 | (-) |
| SPM22 | *orf2::att3* délétion en phase | 10 | (-) |
| SPM501 | *orf6*::att1Ωhyg*+ | 14 | (-) |
| SPM502 | *orf6*::att1* délétion en phase | 14 | (+) |
| SPM507 | *orf12::att3Ωaac-* | 11 | (-) |
| SPM508 | *orf13c::att3Ωaac-* | 12 | (+) |
| SPM509 | *orf14::att3Ωaac-* | 13 | (-) |

Ces résultats permettent de tirer un certain nombre de conclusions en ce qui concerne la fonction des différents gènes impliqués dans la biosynthèse de la spiramycine. Ainsi le gène *orf3* est essentiel à la biosynthèse de la spiramycine. En effet une inactivation en phase dans ce gène conduit à une souche (OS49.67, (cf. exemple 6)) ne produisant plus de spiramycine. L'inactivation en phase permet d'écarter l'hypothèse d'une éventuelle influence de la cassette introduite sur l'expression des gènes situés en aval de *orf3.*

De même, les gènes *orf8* et *orf10* codent des protéines essentielles à la biosynthèse de la spiramycine puisque les souches OS49.107 et OS49.50 ont un phénotype non producteur. De plus, dans ces deux dernières souches, c'est bien l'inactivation du gène correspondant qui est responsable de ce phénotype non producteur, puisqu'au vu de l'orientation des différentes orfs (cf. figure 3), la construction introduite ne peut avoir d'effet polaire.

L'étude des souches possédant une cassette excisable permet également de tirer un certain nombre de conclusion en ce qui concerne la fonction des gènes interrompus. La souche SPM507 possède le génotype : *orf12::att3Ωaac-.* Il est inutile de procéder à l'excision de la cassette pour l'étude de l'effet de l'inactivation de *orf12,* au vu de l'orientation des gènes (cf. figure 3). Le fait que *orf13c* soit orienté en sens opposé à *orf12* montre que ces gènes ne sont pas co-transcrits. L'utilisation d'une cassette excisable permet en revanche d'avoir la possibilité de se débarrasser du marqueur de selection à tout moment. Le phénotype de la souche SPM507 est non producteur, on peut donc en conclure que le gène *orf12* est un gène essentiel à la biosynthèse de la spiramycine chez *S. ambofaciens.*

La souche SPM508 possède le génotype : *orf13c::att3Ωaac-.* Il est inutile de procéder à l'excision de la cassette pour l'étude de l'effet de l'inactivation de *orf13c,* au vu de l'orientation des gènes (cf. figure 3). Le fait que *orf14* soit orienté en sens opposé à *orf13c* montre que ces gènes ne sont pas co-transcrits. L'utilisation d'une cassette excisable permet en revanche d'avoir la possibilité de se débarrasser du marqueur de selection à tout moment. Le phénotype de la souche SPM508 est producteur, on peut donc en conclure que le gène *orf13c* n'est pas un gène essentiel à la biosynthèse de la spiramycine chez *S. ambofaciens.*

La souche SPM509 possède le génotype : *orf14::att3Ωaac-.* Il est inutile de procéder à l'excision de la cassette pour l'étude de l'effet de l'inactivation de *orf14,* au vu de l'orientation des gènes (cf. figure 3), le fait que *orf15c* soit orienté en sens opposé à *orf14* montre que ces gènes ne sont pas co-transcrits. L'utilisation d'une cassette excisable permet en revanche d'avoir la possibilité de se débarrasser du marqueur de selection à tout moment. Le phénotype de la souche SPM509 est non producteur; on peut donc en conclure que le gène *orf14* est un gène essentiel à la biosynthèse de la spiramycine chez *S. ambofaciens.*

La souche SPM21 possède le génotype : *orf2::att3Ωaac-.* Le phénotype de cette souche est non producteur de spiramycines. Cependant, l'orientation des gènes *orf1* à *orf8* (cf. figure 3) laisse penser que ces gènes sont cotranscrits. Aussi, le phénotype observé peut être dû à un effet polaire de la cassette introduite dans *orf2* sur l'expression de gènes situés en aval dans l'opéron. La souche SPM22 possède le génotype *orf2::att3* et a été obtenue après excision en phase de la cassette introduite. L'excision de la cassette laisse seulement une séquence « cicatricielle » caractéristique (cf. exemple 10). La souche SPM22 étant également de phénotype non producteur, on peut en conclure que le gène *orf2* est un gène essentiel à la biosynthèse de la spiramycine chez *S. ambofaciens.* On observe ici uniquement l'effet dû à l'inactivation de *orf2.*

La souche SPM501 possède le génotype : *orf6*::att1Ωhyg*+*.* Le phénotype de cette souche est non producteur de spiramycines. Cependant, les gènes *orf5** et *orf6** (cf. figure 3) ayant la même orientation, le phénotype observé peut être dû à un effet polaire de la cassette introduite dans *orf6** sur l'expression de *orf5*.* La disposition de ces gènes laisse penser qu'ils peuvent être cotranscrits. Pour répondre à cette question, la souche SPM502 a été obtenue après excision en phase de la cassette introduite. Dans cette souche, on observe uniquement l'effet de l'inactivation de *orf6*.* Cette souche possède le génotype *orf6*::att1* (cf. exemple 14). L'excision de la cassette laisse seulement une séquence « cicatricielle » en phase (cf. exemple 14). La souche SPM502 possède un phénotype producteur (cette souche ne produit cependant que de la spiramycine I (cf*. exemple 16)). On peut donc conclure que le gène *orf5** est un gène essentiel à la biosynthèse de la spiramycine chez *S. ambofaciens,* puisque son inactivation indirecte dans la souche SPM501 conduit à un phénotype non producteur. Par contre ; le gène *orf6** n'est pas un gène essentiel à la biosynthèse de la spiramycine I chez *S. ambofaciens* (par contre il est essentiel à la production de spiramycine II et III (cf. exemple 16)).

### EXEMPLE 16. Dosage de la production des spiramycines I, II et III dans les souches mutantes obtenues (ne fait pas partie de l'invention)

Les différentes souches à tester ont été cultivées chacune dans 7 erlensmeyers chicanés de 500 mL contenant 70 ml de milieu MP5 (Pernodet *et al.,* 1993). Les erlens ont été inoculés par 2.5 10⁶ spores/ml des différentes souches de *S. ambofaciens* et mises à pousser à 27°C sous agitation orbitale de 250 rpm pendant 72 heures. Les cultures correspondant au même clone sont rassemblées, éventuellement filtrées sur filtre plissé, et centrifugées 15 min à 7000 tr/min. Les différents surnageants ont ensuite été stockés à -30°C.

Les dosages ont été effectués par Chromatographie Liquide à Haute Performance (CLHP) par appariement d'ions. L'analyse CLHP du milieu de culture permet de déterminer précisément la concentration des trois formes de spiramycine. La colonne utilisée (Macherey-Nagel) est remplie avec une phase Nucleosil de silice greffée octyle. La granulométrie est de 5µm et la taille des pores 100Å. Le diamètre interne de la colonne est de 4,6mm et sa longueur 25cm. La phase mobile est un mélange de tampon H₃PO₄ (pH2,2) et d'acétonitrile 70/30 (v/v) contenant 6,25g/L de perchlorate de NaClO₄. L'analyse est réalisée en régime isocratique avec un débit fixé à 1 ml/min. La colonne est thermorégulée à 23°C. La détection est assurée par spectrophotométrie UV à 238nm. L'échantillon est réfrigéré à +10°C et la quantification est déterminée à partir de l'aire des pics (par étalonnage externe). Dans ces conditions, les temps de rétention de la spiramycine I, II et III sont respectivement d'environ 17; 21 et 30 minutes, comme il a pu être vérifié grâce à un échantillon commercial comprenant les trois formes de spiramycine.

La souche OSC2 possède un phénotype producteur de spiramycine. C'est la souche parentale utilisée pour l'obtention des souches possédant une cassette excisable (cf. exemple 15). Cette souche a donc été utilisée comme témoin positif de production des trois formes de spiramycine. Cette souche produit bien les trois formes de spiramycines comme il a été vérifié par CLHP (cf. figure 19).

L'étude dés souches possédant une cassette excisable permet de tirer un certain nombre de conclusions en ce qui concerne la fonction des gènes interrompus. La souche SPM507 possède le génotype : *orf12::att3Ωaac-.* Le phénotype de la souche SPM507 est non producteur (cf. exemple 15), on peut donc en conclure que le gène *orf12* est un gène essentiel à la biosynthèse de la spiramycine chez *S. ambofaciens.* Cette souche ne produit plus de spiramycines comme il a été vérifié par CLHP (cf. figure 22). Ce résultat confirme le caractère essentiel du gène *orf12* dans la biosynthèse de la spiramycine.

La souche SPM508 possède le génotype : *orf13c::att3Ωaac-.* Le phénotype de la souche SPM508 est producteur de spiramycine (cf. exemple 15), on peut donc en conclure que le gène *orf13c* n'est pas un gène essentiel à la biosynthèse de la spiramycine chez *S. ambofaciens.* Cette souche produit de la spiramycine I, II et III comme il a été vérifié par CLHP (cf. figure 23). Ce résultat confirme que le gène *orf13c* n'est pas un gène essentiel à la biosynthèse des spiramycines I, II et III ches *S*. *ambofaciens.*

La souche SPM509 possède le génotype : *orf14::att3Ωaac-.* Le phénotype de la souche SPM509 est non producteur, on peut donc en conclure que le gène *orf14* est un gène essentiel à la biosynthèse de la spiramycine chez *S. ambofaciens.* Cette souche ne produit plus de spiramycines comme il a été vérifié par CLHP (cf. figure 24). Ce résultat confirme l'essentialité du gène *orf14* dans la biosynthèse de la spiramycine.

La souche SPM501 possède le génotype: *orf6*::att1Ωhyg*+*.* Le phénotype de cette souche est non producteur de spiramycines. Cette souche ne produit plus de spiramycines comme il a été vérifié par CLHP (cf. figure 20). Cependant, les gènes *orf5** et *orf6** (cf. figure 3) ayant la même orientation, le phénotype observé peut être dû à un effet polaire de la cassette introduite dans *orf6** sur l'expression de *orf5** dans l'opéron. Ceci laisse penser que ces gènes sont cotranscrits. Pour répondre à cette question, la souche SPM502 a été obtenue par excision de la cassette introduite, produisant une délétion en phase dans le gène *or6** et restaurant l'expression de *orf5*.* Cette souche possède le génotype *orf6*::att1* (cf. exemple 14 et 15). L'excision de la cassette laisse seulement une séquence att « cicatricielle » en phase (cf. exemple 14). La souche SPM502 possède un phénotype producteur de spiramycine. Cependant, comme il a été prouvé par CLHP, cette souche ne produit que de la spiramycine I et ne produit pas de spiramycine II et III (cf. figure 21). On peut donc conclure de ces résultats que le gène *orf5** est un gène essentiel à la biosynthèse de la spiramycine chez *S. ambofaciens,* puisque son inactivation indirecte dans la souche SPM501 conduit à un phénotype non producteur de spiramycine (cf. figure 20). Par contre ; le gène *orf6** n'est pas un gène essentiel à la biosynthèse de la spiramycine I chez *S. ambofaciens,* puisque l'inactivation de ce gène conduit à un phénotype producteur de spiramycine I (cf. figure 21). Cependant, *orf6** est essentiel à la production de spiramycine II et III (cf. exemple 16)). Le gène *orf6** code donc bien une acyl-transférase responsable de la modification du platénolide à la position 3 (cf. figure 1).

### EXEMPLE 17 : Détermination du point de démarrage de la traduction de orf10 et amélioration de la production en spiramycines

### 17.1 Construction des plasmides pSPM523, pSPM524 et pSPM525 :

Le gène *orf10* a été identifié chez *Streptomyces ambofaciens* et a été désigné *srmR* par Geistlich *et al.* (Geistlich, M., *et al.,* 1992). L'inactivation du gène *orf10* a été réalisée (cf. exemple 8). Il a pu ainsi être montré que la souche résultante ne produit plus de spiramycines (cf. exemple 15). Ceci confirme que le gène *orf10* est bien impliqué dans la biosynthèse des spiramycines. La protéine codée par ce gène est donc bien essentielle à la biosynthèse des spiramycines. Cependant, l'analyse de la séquence montre que deux codons ATG situés dans la même phase de lecture peuvent être utilisés pour la traduction de *orf10* (cf. Figure 28). Un des deux codons possible (le codons le plus en amont) démarre à la position 10656 de la séquence donnée en SEQ ID N° 1, alors que l'autre codon possible situé plus en aval démarre à la position 10809 (cf. SEQ ID N° 1). Avant de tester un éventuel effet de la surexpression de *srmR* sur la production de spiramycine, il est important de déterminer dans un premier temps le point de démarrage de la traduction.

Dans le but de determiner le site de démarrage de la traduction, trois constructions comportant trois formes d'*orf10* ont été réalisées. Ces dernières ont été obtenues par PCR en utilisant des oligonucléotides comportant soit un site de restriction *Hind*III soit un site de restriction *Bam*HI*.*
Le premier couple utilisé pour l'amplification correspond aux oligonucléotides suivants :
EDR39 :
   5'CCC**AAGCTT**GAGAAGGGAGCGGACATTCATGGCCCGCGCCGAACGC3' (SEQ ID N° 122) (le site *Hind*III figure en gras)
EDR42 :
   5'CG**GGATCCG**GCTGACCATGGGAGACGGGCGCATCGCCGAGTTCAGC3' (SEQ ID N° 123) (le site *BamH*I figure en gras)
Le couple d'amorces EDR39-EDR42 permet l'amplification d'un fragment d'*orf10* comportant l'ATG situé le plus en 3' (position 10809 dans la séquence donnée en SEQ ID N° 1) (cf. Figure 28). Le fragment obtenu fait une taille d'environ 2 kb et sera appelé par la suite "*orf10* court", il ne contient pas le promoteur de *orf10.* Ce fragment de 2kb a été cloné dans le vecteur pGEM-T easy pour donner naissance au plasmide pSPM520.
Le deuxième couple utilisé pour l'amplification correspond aux oligonucléotides suivants :
EDR40 :
   5'CCC**AAGCTT**GAGAAGGGAGCGGACATTCAATGCTTTGGTAAAGCAC3' (SEQ ID N° 124) (le site *Hind*III figure en gras)
EDR42 :
   5'CG**GGATCC**GGCTGACCATGGGAGACGGGCGCATCGCCGAGTTCAGC3' (SEQ ID N° 123) (le site *BamH*I figure en gras)
Le couple d'amorces EDR40-EDR42 permet l'amplification d'un fragment d'*orf10* comportant l'ATG situé le plus en 5' (position 10656 dans la séquence donnée en SEQ ID N° 1) (cf. Figure 28). Ce fragment de 2,2 kb appelé par la suite "*orf10* long" a été cloné dans le vecteur pGEM-T easy pour donner naissance au plasmide pSPM521, ce plasmide ne contient pas le promoteur de *orf10.*
Le troisième couple utilisé pour l'amplification correspond aux oligonucléotides suivants :
EDR41 :
   5'-CCC**AAGCTT**TCAAGGAACGACGGGGTGGTCAGTCAAGT-3' (SEQ ID N° 125) (le site *Hind*III figure en gras)
EDR42 :
   5'C**GGGATCC**GGCTGACCATGGGAGACGGGCGCATCGCCGAGTTCAGC3' (SEQ ID N° 123) (le site *BamH*I figure en gras)
Le couple d'amorces EDR41-EDR42 permet l'amplification du gène *orf10* avec les deux ATG, ainsi que son propre promoteur (cf. Figure 28). Ce fragment de 2,8 kb appelé par la suite *"orf10* pro" a été cloné dans le vecteur pGEM-T easy pour donner naissance au plasmide pSPM522.
Le fragment "*orf10* pro" a été obtenu en utilisant comme matrice l'ADN chromosomique de la souche OSC2. Les fragments "*orf10* court" et "*orf10* long" ont quant à eux été obtenus en utilisant comme matrice l'ADN du fragment "*orf10* pro" préalablement purifié.

Les inserts *Hind*III*-Bam*HI des plasmides pSPM520, pSPM521 et pSPM522 ont ensuite été sous-clonés dans le vecteur pUWL201 (plamside dérivé du plasmide pUWL199 (Wehmeier UF, 1995) dans lequel le fragment *Kpn*I*-Bam*HI de la région du promoteur de *ermE* (cf. Bibb et al., 1985, notamment figure 2) portant une mutation augmentant la force du promoteur (promoteur ermE*) (Bibb *et al.,* 1994) a été introduit (cf. Doumith *et al.,* 2000)) préalablement digéré par les enzymes *Hind*III*-Bam*HI*.* Ainsi, il a été obtenu trois plasmides : pSPM523 (dérivé du pUWL201 avec la forme "*off410* court" comme insert), pSPM524 (dérivé du pUWL201 avec la forme "*orf10* long" comme insert) et pSPM525 (dérivé du pUWL201 avec la forme "*orf10* pro") (Figure 28).

### 17.2 Transformation de la souche OS49.50 par les constructions pSPM523. pSPM524 et pSPM525 :

La souche OS49.50 (souche interrompue dans le gène *orf10,* cf. exemple 8) a été transformée indépendamment par transformation de protoplastes (Kieser, T *et al.,* 2000) par chacun des plasmides pSPM523, pSPM524 et pSPM525. Un témoin négatif a également été réalisé en transformant la souche OS49.50 par le plasmide pUWL201 sans insert. Après transformation des protoplastes, les clones sont sélectionnés pour leur résistance au au thiostrepton. La transformation des clones par chacun des plamides est vérifiée par extraction de ces plasmides. Ainsi quatre nouvelles souches ont été obtenues : la souche OSC49.50 pUWL201, issue de la transformation par le plasmide pUWL201 sans insert, la souche OSC49.50 pSPM523, issue de la transformation par le plasmide pSPM523, la souche OSC49.50 pSPM524, issue de la transformation par le plasmide pSPM524 et enfin la souche OS49.50 pSPM525, issue de la transformation par le plasmide pSPM525.

La production de spiramycines de chacune de ces quatre souches a été testée par CLHP. Pour cela, les différentes souches de *Streptomyces* à tester ont été cultivées dans des erlenmeyers à baffles (erlenmeyers chicanés) de 500 ml contenant 70 ml de milieu MP5 (Pernodet *et al.,* 1993). Lorsque la souche contient le plasmide pUWL201 ou l'un de ses dérivés, il est ajouté 5 µg/ml de thiostrepton. Les erlenmeyers chicanés ont été inoculés à une concentration initiale de 2.5 10⁶ spores/ml des différentes souches de *S*. *ambofaciens* et les cultures ont été incubées à 27°C sous agitation orbitale de 250 rpm pendant 96 heures. Les cellules ont ensuite été séparées du milieu par centrifugation et le surnageant a été analysé par CLHP (cf. exemple 16) pour déterminer la quantité de spiramycine produite. Grâce à un échantillon étalon et à la mesure de l'air des pics, il a été possible de déterminer la quantité de chacune des spiramycines produites par ces souches. Les résultats de cette analyse sont présentés dans le tableau 39, les données sont exprimées en mg par litre de surnageant. Les résultats correspondent à la production totale en spiramycines (obtenue en additionnant la production en spiramycine I, II et III).

**Tableau 39. Production en spiramycines des souches dérivées de OS49.50, (résultats exprimés en mg/l).**

| **Souche** | **Production en Spiramycines** |
|---|---|
| OS49.50 pUWL201 | 0 |
| OS49.50 pSPM523 | 0 |
| OS49.50 pSPM524 | 93 |
| OS49.50 pSPM525 | 149 |

Comme le montre les résultats donnés dans le tableau 39, le témoin négatif (souche OS49.50 transformée par le plasmide pUWL201) ne produit pas de spiramycine. Lorsque le plasmide pSPM523 (qui contient la forme "*orf10* court") est introduit dans la souche OS49.50, aucune production de spiramycine n'est observée. En revanche, la présence du plasmide pSPM524 (qui contient la forme "*orf10* long") et du plasmide pSPM525 (qui contient la forme "*orf10* pro") restaure la production de spiramycine dans la souche hôte OS49.50. Ainsi, seuls les fragments de *orf10* contenant l'ATG le plus en amont permettent de restaurer la synthèse de spiramycine.

Dans le but de confirmer ces résultats, le plasmide pSPM521 (plasmide pGEM-T easy contenant la forme "*orf10* long") a été digéré par l'enzyme de restriction *Xho*I (cette enzyme possède un site unique dans ce plasmide, localisé entre les deux ATG (cf. Figure 28)). Les extrémités *Xho*I ont ensuite été rendues bout franc par traitement à l'enzyme de Klenow. Le plasmide a alors été refermé sur lui-même par action de la T4 DNA ligase pour donner naissance au plasmide pSPM527. Si l'ATG le plus en amont (position 10656 dans la séquence donnée en SEQ ID N° 1) est bien utilisé comme site d'initiation de la traduction, ce traitement entrainera un décalage du cadre de lecture au niveau du site *Xho*I et aura pour effet la production d'une protéine ne présentant pas d'activité activatrice. Par contre si l'initiation de la traduction a lieu au niveau de l'ATG plus en aval (position 10809 dans la séquence donnée en SEQ ID N° 1), ce traitement devrait avoir peu ou pas d'effet sur l'expression de Orf10 (compte tenu de la localisation du point de démarrage de la transcription) et aucun effet sur la protéine produite.

L'insert *BamH*I*-Hind*III de pSPM527 a ensuite été sous-cloné dans le vecteur pUWL201 pour donner naissance au plasmide pSPM528. Ce plasmide a été introduit dans la souche OS49.50 et un clone possédant le plasmide voulu a plus particulièrement été sélectionné. La production de spiramycine de la souche résultante a ensuite été testée par CLHP (Cf. exemple 16 et ci-dessus). Contrairement à ce qui avait été observé avec le plasmide pSPM524 (cf. tableau 39), la présence du plasmide pSPM528 dans la souche OS49.50 ne rétablit pas la production de spiramycine. Ceci confirme que le démarrage de la traduction du gène *orf10* est l'ATG situé le plus en aval (ATG 1 cf. Figure 28).

### 17.3 Amélioration de la production en spiramycines de la souche OSC2 de S. ambofaciens :

Afin de tester l'effet de la surexpression du gène *orf10* sur la production de spiramycines, les plasmides pSPM523, pSPM524, pSPM525 et pSPM528 ont été introduits dans la souche OSC2. Pour cela, des protoplastes de la souche OSC2 ont été transformés (Kieser, *T et al.,* 2000) indépendamment par chacun des plasmides pSPM523, pSPM524, pSPM525 et pSPM528. Un témoin négatif a également été réalisé en transformant la souche OSC2 par le plasmide pUWL201 sans insert. Après transformation des protoplastes, les clones sont sélectionnés pour leur résistance au au thiostrepton. Ainsi cinq nouvelles souches ont été obtenues : la souche OSC2 pUWL201, issue de la transformation par le plasmide pUWL201 sans insert, la souche OSC2 pSPM523, issue de la transformation par le plasmide pSPM523, la souche OSC2 pSPM524, issue de la transformation par le plasmide pSPM524, la souche OSC2 pSPM525, issue de la transformation par le plasmide pSPM525 et enfin la souche OSC2 pSPM528, issue de la transformation par le plasmide pSPM528. La production en spiramycines de ces souches a alors été analysée par CLHP (de la même manière que dans l'exemple 17.2). L'analyse de la production en spiramycine de la souche OSC2 a également été effectée en parallèle pour comparaison. Les résultats de cette analyse sont présentés dans le tableau 40, les données sont exprimées en mg par litre de surnageant. Les résultats correspondent à la production totale en spiramycines (obtenue en additionnant la production en spiramycine I, II et III).

**Tableau 40. Production en spiramycines des souches dérivées de OSC2, (résultats exprimés en mg/l).**

| **Souche** | **Production en spiramycines** |
|---|---|
| OSC2 | 69 |
| OSC2 pUWL201 | 103 |
| OSC2 pSPM523 | 19 |
| OSC2 pSPM524 | 135 |
| OSC2 pSPM525 | 278 |
| OSC2 pSPM528 | 68 |

Ainsi, il est observé que la présence du plasmide pSPM524 ou du plasmide pSPM525 augmente significativement la production en spiramycines de la souche OSC2. Ceci démontre bien que la surexpression de Orf10 a un effet positif sur la production en spiramycines. Le plasmide pSPM528 n'a par contre pas d'effet sur la production en spiramycines.

Les plasmides pSPM525 et pUWL201 ont de la même manière été introduit dans la souche SPM502 (cf. exemple 14). Ainsi deux nouvelles souches ont été obtenues : la souche SPM502 pUWL201, issue de la transformation par le plasmide pUWL201 sans insert, et la souche SPM502 pSPM525, issue de la transformation par le plasmide pSPM525.

Un échantillon de la souche SPM502 pSPM525 (cette souche contient le plasmide pSPM525, cf. ci-dessus) a été déposé auprès de la Collection Nationale de Cultures de Microorganismes (CNCM) Institut Pasteur, 25, rue du Docteur Roux 75724 Paris Cedex 15, France, le 26 février 2003 sous le numéro d'enregistrement 1-2977.

La production en spiramycines des souches SPM502 pUWL201 et SPM502 pSPM525 a été analysée par CLHP (de la même manière que dans l'exemple 17.2). L'analyse de la production en spiramycines de la souche SPM502 a également été effectée en parallèle pour comparaison. Les résultats de cette analyse sont présentés dans le tableau 41, les données sont exprimées en mg par litre de surnageant. Les résultats correspondent à la production en spiramycine I. En effet, aucune de ces souches ne produit de spiramycine II et III.

**Tableau 41. Production en spiramycine I des souches dérivées de SPM502, (résultats exprimés en mg/l).**

| **Souche** | **Spiramycine I** |
|---|---|
| SPM502 | 47 |
| SPM502 pUWL201 | 72 |
| SPM502 pSPM525 | 130 |

Ainsi, il a pu être observé que la surexpression du gène *orf10* dans la souche SPM502 augmente de facon importante la production de spiramycine-I.

### EXEMPLE 18 : Construction d'une banque d'ADN génomique de la souche OSC2 de Streptomyces ambofaciens chez E. coli dans la cosmide pWED2.

### 18.1 Construction du cosmide pWED2 :

Dans le but de faciliter l'inactivation de gènes chez *Streptomyces,* un cosmide portant la séquence *oriT* du plasmide RK2 (ce qui permet son introduction par conjugaison dans *Streptomyces* à partir d'une souche appropriée de *E. coli*) et portant également un gène de résistance à un antibiotique conférant un phénotype repérable chez *Streptomyces,* a été construit. Un tel cosmide contenant de grands inserts d'ADN génomique de *Streptomyces ambofaciens* peut être utilisé dans des expériences d'inactivation de gènes.

Pour construire ce vecteur, une cassette *pac-oriT* (fragment *EcoRV)* a été introduite dans le cosmide pWED1 (*Gourmelen et al.,* 1998), dérivé du cosmide pWED15 (Wahl *et al.,* 1987), au niveau du site unique *Hpa*I*.* La cassette *pac-oriT* a été obtenue par PCR. Pour cela, le gène *pac* a été amplifié par PCR à partir du plasmide pVF 10.4 (Vara *et al.,* 1985; Lacalle *et al.,* 1989) en utilisant comme première amorce, l'amorce A (de séquence 5'-CCAGTAGATATC**CCGCCAACCCGGAGCTGCAC**-3' (SEQ ID N° 126), le site de restriction *EcoRV* a été souligné et la séquence en gras de 20 nucléotides correspond à une région en amont du promoteur du gène *pac)* et comme deuxième amorce, l'amorce B (de séquence 5'-GAAAAGATCCGT**CATGGGGTCGTGCGCTCCTT**-3' (SEQ ID N° 127), qui comporte à son extrémité 5' une séquence de 12 nucléotides correspondant au début de la séquence *oriT* (souligné double) et une séquence de 20 nucléotides (en gras) correspondant à la fin du gène *pac* (cf. Figure 29, 1^{ère} PCR).

Le gène *oriT* a quant à lui été amplifié par PCR à partir du plasmide pPM803 (Mazodier,**P***. et al.,* 1989) en utilisant comme première amorce, l'amorce C (de séquence 5'**-CACGACCCCATG**ACGGATCTTTTCCGCTGCAT-3' (SEQ ID N° 128)), qui comporte à son extrémité 5' une séquence de 12 nucléotides correspondant à une séquence en aval de la séquence codante du gène *pac* (en gras) et une séquence de 20 nucléotides correspondant au début de la séquence *oriT*) et comme deuxième amorce, l'amorce D (de séquence 5'-GAGCCGGATATCATCGGTCTTGCCTTGCTCGT-3' (SEQ ID N° 129)) qui comporte le site de restriction *Eco*RV (souligné simple) et une séquence de 20 nucléotides correspondant à la fin de la séquence *oriT* (souligné double) (cf. Figure 29, 2^{ième} PCR).

Le produit d'amplification obtenu avec les amorces A et B et celui obtenu avec les amorces C et D ont à une de leur extrémité, une séquence commune de 24 nucléotides. Une troisième PCR a été réalisée en mélangeant les deux produits d'amplification obtenus précédemment et en utilisant comme amorces, les amorces A et D (cf. figure 29, 3^{ième} PCR). Ceci a permis d'obtenir un produit d'amplification correspondant à l'ensemble *pac+oriT.* Ce fragment *pac*-*oriT* a alors été cloné dans le vecteur pGEM-T Easy (commercialisé par la société Promega (Madison, Wisconcin, USA)), ce qui a permis d'obtenir le plasmide pGEM-T*-pac-oriT.* L'insert de ce plasmide a ensuite été sous cloné dans le cosmide pWED1. Pour cela le plasmide pGEM-*pac-oriT* a été digéré par l'enzyme *EcoRV* et l'insert *EcoRV* contenant l'ensemble *pac*+*oriT* a été inséré dans le cosmide pWED1 ouvert au préalable par l'enzyme *Hpa*I*.* Le cosmide ainsi obtenu a été baptisé pWED2 (cf. Figure 30).

Ce cosmide permet de faciliter l'inactivation de gènes chez *Streptomyces.* En effet, il porte la séquence *oriT* (ce qui permet son introduction par conjugaison dans *Streptomyces* à partir d'une souche appropriée de *E. coli*) mais également un gène de résistance à un antibiotique conférant un phénotype repérable chez *Streptomyces.* Un tel cosmide contenant de grands inserts d'ADN génomique de *Streptomyces ambofaciens* peut être utilisé dans des expériences d'inactivation de gènes.

Ainsi un cosmide dérivé de pWED2 contenant le gène cible pourra par exmeple être introduit dans la souche de *E. coli* KS272 contenant le plasmide pKOBEG (cf. Chaveroche *et al.* 2000) et une cassette sera introduite dans le gène cible selon la technique décrite par Chaveroche *et al.* 2000. Le cosmide obtenu par cette technqiue (cosmide dans lequel le gène cible est inactivé), pourra ensuite être introduit dans une souche de *E. coli* telle que la souche S17.1 ou toute autre souche permettant de transférer par conjugaison des plasmides contenant la séquence *oriT* vers *Streptomyces.*

Après conjugaison entre *E. coli* et *Streptomyces,* des clones de *Streptomyces* dans lesquelles la copie sauvage du gène cible aura été remplacée par la copie interrompue pourront être obtenues comme décrit dans l'exemple 2.

Le gène de résistance exprimé chez *Streptomyces* présent sur ce nouveau cosmide est le gène *pac* de *Streptomyces alboniger* (Vara, J *et al.,* 1985 ; Lacalle *et al*., 1989) qui code la puromycine N-acétyl transférase et qui confère la résistance à la puromycine. Lors d'expériences d'inactivation de gène, on cherchera les clones dans lesquels un double évènement de recombinaison aura eu lieu. Ces clones auront éliminer le cosmide pWED2 et seront donc redevenus sensibles à la puromycine.

### 18.2 Construction d'une banque d'ADN génomique de la souche OSC2 de Streptomyces ambofaciens chez E. coli dans le cosmide pWED2

L'ADN génomique de la souche OSC2 de *Streptomyces ambofaciens* a été digéré partiellement par l'enzyme de restriction *Bam*HI de manière à obtenir des fragments d'ADN de taille comprise entre environ 35 et 45 kb. Ces fragments ont ensuite été clonés dans le cosmide pWED2, ce dernier ayant été au préalable digéré par *Bam*HI*,* puis traité par la phosphatase alcaline. Le mélange de ligation a ensuite été encapsidé *in vitro* dans des particules de phages lambda grâce au système « Packagene® Lambda DNA packaging system » commercialisé par la société Promega (Madison, Wisconcin, USA) en suivant les recommandations du fabriquant. Les particules phagiques obtenues ont été utilisées pour infecter la souche SURE® d'*E. coli* commercialisée par la société Stratagene (LaJolla, Californie, USA). La séléction des clones a été effectuée sur milieu LB + ampicilline (50 µg/ml), le cosmide pWED2 conférant la résistance à l'ampicilline.

### EXEMPLE 19 : Isolement de cosmides de la nouvelle banque couvrant la région de la voie de biosynthèse des spiramycines. Sous clonage et séquençage de fragments de ces cosmides.

### 19.1 Hybridation sur colonies de la banque génomique de Streptomyces ambofaciens OSC2 :

Des cosmides de la nouvelle banque de *Streptomyces ambofaciens.* OSC2 (cf. exemple 18) couvrant les *orf1* * à *orf10** ou une partie ou la totalité des *orf1* à *orf25c,* ou une région plus en amont de l'*orf25c* ont été isolés. Pour cela, des hybridations successives sur les colonies d'*E. coli* obtenues selon l'exemple 18 ont été réalisées en utilisant les 3 sondes suivantes (cf. figure 31):
- La première sonde utilisée correspond à un fragment d'ADN d'environ 0,8kb amplifié par PCR en utilisant comme matrice le cosmide pSPM5 et les amorces suivantes :
   ORF23c: 5'-ACGTGCGCGGTGAGTTCGCCGTTGC-3' (SEQ ID N° 130) et
   ORF25c : 5'-CTGAACGACGCCATCGCGGTGGTGC-3' (SEQ ID N° 131).
   Le produit de PCR ainsi obtenu contient un fragment du début de l'*orf23c, l'orf24c* en entier et la fin de l'*orf25c* (cf. figure 31, sonde I).
- La deuxième sonde utilisée correspond à un fragment d'ADN d'environ 0,7kb amplifié par PCR en utilisant comme matrice l'ADN total de la souche *S*. *ambofaciens* ATCC23877 et les amorces suivantes :
   ORF1*c : 5'-GACCACCTCGAACCGTCCGGCGTCA-3' (SEQ ID N° 132) et
   ORF2*c : 5'-GGCCCGGTCCAGCGTGCCGAAGC-3' (SEQ ID N° 133).
   Le produit de PCR ainsi obtenu contient un fragment de la fin de l'*orf1***c* et du début de l'*orf2*c* (cf. figure 31, sonde II).
- La troisième sonde utilisée correspond à un fragment *Eco*RI*-BamH*I d'environ 3kb contenant les *orf1, orf2* et *orf3* et obtenu par digestion du plasmide pOS49.99 (cf. figure 31, sonde III).

Environ 2000 clones de la banque obtenue dans l'exemple 18.2 ont été transférés sur filtre pour hybridation sur colonies selon les techniques classiques (Sambrook *et al.,* 1989).

La première sonde (cf. figure 31, sonde I) a été marquée au ³²P par la technique du « random priming » (Kit commercialisé par la société Roche) et utilisées pour l'hybridation sur 2000 clones de la banque, après transfert sur filtre. L'hybridation a été effectuée à 65°C dans le tampon décrit par Church & Gilbert (Church & Gilbert, 1984). Deux lavages ont été effectués en 2x SSC, 0,1% SDS à 65°C, le premier pendant 10 minutes et le deuxième pendant 20 minutes et un troisième lavage d'une durée de 30 minutes a ensuite été effectué en 0.2X SSC, 0,1% SDS à 65°C. Dans ces conditions d'hybridation et de lavage, 20 clones parmi les 2000 hybridés présentaient un fort signal d'hybridation avec la première sonde. Ces 20 clones ont été cultivés en milieu LB+ ampicilline (50 µg/ml) et les 20 cosmides correspondants ont été extraits par lyse alcaline (Sambrook *et al.,* 1989) puis ils ont été digérés par l'enzyme de restriction *Bam*HI*.* Les produits de digestions ont ensuite été séparés sur gel d'agarose, transférés sur membrane de Nylon et hybridés par la première sonde (cf. ci-dessus : le produit de PCR ORF23c-ORF25c, sonde I) dans les mêmes conditions que ci-dessus. Douze cosmides possédaient un fragment *Bam*HI hybridant fortement avec la sonde utilisée. Ces 12 cosmides ont été nommés pSPM34, pSPM35, pSPM36, pSPM37, pSPM38, pSPM39, pSPM40, pSPM41, pSPM42, pSPM43, pSPM44 et pSPM45. Les profils, après digestion par *BamHI,* de ces 12 cosmides ont été comparés entre eux et avec celui du cosmide pSPM5. De plus des expériences d'amplification par PCR en utilisant différentes amorces correspondant à différents gènes déjà identifiés dans la région *orf1-orf25c* ont permis de positionner l'insert de certains de ces cosmides les uns par rapport aux autres et de déterminer également la localisation de ces inserts dans la région *orf1-orf25c* déjà connue (cf. Figure 32). Le cosmide pSPM36 a plus particulièrement été choisi car il était susceptible de contenir une grande région en amont de l'*orf25c* (cf. Figure31 et 32).

Dans un deuxième temps, et en utilisant les mêmes conditions que celles décrites ci-dessus, les 2000 clones de la banque de *Streptomyces ambofaciens.* OSC2 ont été hybridés avec la deuxième sonde correspondant au produit de PCR: ORF1*c-ORF2*c (cf. figure 31, sonde II). Cette hybridation a permis d'isoler des cosmides dont l'insert est situé dans la région de *orf1*c* à *orf10*c.* Dans les conditions d'hybridation utilisés, 16 clones parmi les 2000 hybridés présentaient un fort signal d'hybridation avec cette deuxième sonde. Ces 16 clones ont été cultivés en milieu LB+ ampicilline (50 µg/ml) et les 16 cosmides correspondants ont été extraits par lyse alcaline (Sambrook *et al.,* 1989) puis ils ont été digérés par l'enzyme de restriction *Bam*HI*.* Les profils de digestion (après digestion par *BamH*I) de ces 16 cosmides ont été comparés entre eux et avec celui du cosmide pSPM7. Des expériences d'amplication par PCR avec les amorces ORF1*c et ORF2*c ont permis de choisir deux cosmides qui renfermaient bien les gènes *orf1*c* et *orf2*c* et dont les profils possédaient des bandes communes mais aussi des bandes différentes. De plus d'autres expériences d'amplification par PCR en utilisant différentes amorces correspondant à différents gènes déjà identifiés dans la région *orf1*c* à *orf10*c* ont permis de positionner l'insert de ces cosmides les uns par rapport aux autres et de déterminer également la localisation de ces inserts dans la région *orf1*c* à *orf10*c* déjà connue (cf. Figure 32). Les deux cosmides plus particulièrement sélectionnés ont été nommés pSPM47 et pSPM48 (cf. Figure 32).

En utilisant les mêmes conditions que celles décrites ci-dessus, les 2000 clones de la banque de *Streptomyces ambofaciens* OSC2 ont également été hybridés avec la troisième sonde correspondant au fragment d'ADN *Eco*RI-*Bam*HI du plasmide pOS49.99 (cf. figure 31 sonde III). Cette hybridation a permis d'isoler les cosmides contenant la région de l'*orf1* jusqu'à l'*orf3* et susceptibles de contenir soit une grande partie des gènes des PKS, soit une grande partie des gènes *orf1* à *orf25c* de la voie de biosynthèse des spiramycines. Dans ces conditions d'hybridation, 35 clones parmi les 2000 hybridés présentaient un fort signal d'hybridation avec la troisième sonde. Ces 35 clones ont été cultivés en milieu LB+ ampicilline (50 µg/ml) et les 35 cosmides correspondants ont été extraits par lyse alcaline (Sambrook *et al.,* 1989) puis digérés par l'enzyme de restriction *Bam*HI*.* Les profils, après digestion par *Bam*HI*,* de ces 35 cosmides ont été comparés entre eux et avec celui du cosmide pSPM5. De plus des expériences d'amplification par PCR en utilisant différentes amorces correspondant à différents gènes déjà identifiés dans la région *orf1* à *orf25c* ont permis de vérifier que ces cosmides renfermaient bien des inserts provenant de la région *orf1* à *orf25c* et de positionner les inserts de ces cosmides les uns par rapport aux autres et par rapport à la la région *orf1* à *orf25c* déjà connue (cf. Figure 32). Cinq cosmides ont été plus particulièrement sélectionnés, ils ont été nommés pSPM50, pSPM51, pSPM53, pSPM55 et pSPM56 (cf. Figure 32).

### 19.2 Sous clonage et séquençage d'une partie de l'insert du cosmide pSPM36.

La sonde d'environ 0,8 kb obtenue par PCR avec les amorces ORF23c et ORF25c (cf. ci-dessus et figure 31, sonde I) a également été utilisée dans des expériences de Southern Blot sur l'ADN total dé *S. ambofaciens.* OSC2 digéré par l'enzyme *PstI.* Dans les conditions d'hybridation décrite ci-dessus, cette sonde révèle un fragment unique *PstI* d'environ 6 kb lorsque hybridé sur l'ADN total de *S. ambofaciens.* OSC2 digéré par l'enzyme *PstI.* Il existe un site *PstI* au niveau de l'*orf23c* (cf. SEQ ID N° 80) mais aucun autre site *PstI* jusqu'à l'extrémité (site *Bam*HI) de la séquence connue (cf. SEQ ID N° 1). Ce fragment *Pst*I*-Bam*HI a une taille d'environ 1.4 kb. Le fragment *PstI* de 6 kb hybridé sur l'ADN total de *S. ambofaciens* digéré par l'enzyme *PstI* contient donc une région d'environ 4,6 kb située en amont de *orf25c.* Cette région est susceptible de contenir d'autres gènes dont les produits sont impliqués dans la voie de biosynthèse de la spiramycine. Il a été vérifié par digestion que le cosmide pSPM36 contenait bien ce fragment *PstI* de 6 kb. Ce fragment a été isolé à partir de pSPM36, dans le but de déterminer la séquence plus en amont de *orf25c.* Pour cela, le cosmide pSPM36 a été digéré par l'enzyme de restriction *PstI.* Le fragment *Pst*I*-Pst*I d'une taille d'environ 6kb a été isolé par électroélution à partir d'un gel d'agarose 0.8% puis cloné dans le vecteur pBK-CMV (4512bp) (commercialisé par la société Stratagene (La Jolla, Californie, USA)). Le plasmide ainsi obtenu a été nommé pSPM58 (cf. Figure 33) et la séquence de son insert a été déterminée. La séquence de cet insert est présentée en SEQ ID N° 134. Toutefois, toute la séquence n'a pas été déterminée et il subsite un trou d'environ 450 nucléotides, la partie de la séquence non determinée a été notée par une succession de « N » dans la séquence correspondante.

### 19.3 Analyse des nouvelles séquences nucléotidiques, détermination des phases ouvertes de lecture et caractérisation des gènes impliqués dans la biosynthèse des spiramycines.

La séquence de l'insert du cosmide pSPM58 obtenue a été analysée grâce au programme FramePlot (Ishikawa J & Hotta K. 1999). Ceci a permis d'identifier, parmi les phases ouvertes de lecture, les phases ouvertes de lecture présentant un usage des codons typique de *Streptomyces.* Cette analyse a permis de déterminer que cet insert comporte 4 nouvelles ORFs en amont de l'*orf25c* (cf. figure 34). Ces gènes ont été nommés *orf26* (SEQ ID N° 107), *orf27* (SEQ ID N° 109), *orf28c* (SEQ ID N° 111, la séquence de cette orf n'a pas été déterminée complètement puisqu'un trou d'environ 450 nucléotide subsite lors du séquençage de l'insert de pSPM58, ces 450 nucléotides figurent sous la forme d'une sére de « N » dans la sequence SEQ ID N° 111) et *orf29* (la séquence de cette dernière orf était incomplète dans cette insert). Le « c » ajouté dans le nom du gène signifiant pour l'ORF en question que la séquence codante est dans l'orientation inverse (cf. figure 34).

Les séquences protéiques déduites de ces phases ouvertes de lecture ont été comparées avec celles présentes dans différentes bases de données grâce à différents programmes : *BLAST* (Altschul *et al.,* 1990) (Altschul *et al.,* 1997), CD-search, (ces deux programmes sont accessibles notamment auprès du National Center for Biotechnology Information (NCBI) (Bethesda, Maryland, USA)), FASTA ((Pearson W. R & D. J. Lipman, 1988) et (Pearson W. R., 1990) (ce programme est accessible notamment auprès du centre de ressources INFOBIOGEN, Evry, France). Ces comparaisons ont permis de formuler des hypothèses sur la fonction des produits de ces gènes et d'identifier ceux susceptibles d'être impliqués dans la biosynthèse de spiramycines.

### 19.4 Sous clonage et séquençage d'une autre partie de l'insert du cosmide pSPM36.

La sonde d'environ 0,8 kb obtenue par PCR avec les amorces ORF23c et ORF25c (cf. ci-dessus et figure 31, sonde I) a également été utilisée dans des expériences de Southern Blot sur l'ADN total de la souche OSC2 digéré par l'enzyme *Stu*I*.* Dans les conditions, d'hybridation décrite ci-dessus pour cette sonde, cette sonde révèle un fragment unique *Stu*I d'environ 10 kb lorsque hybridé sur l'ADN total de la souche OSC2 digéré par l'enzyme *Stu*I*.* Compte tenu de la présence d'un site *Stu*I dans l'*orf23c* (cf. SEQ ID N° 80) et de la localisation de ce site par rapport au site *Pst*I*,* ce fragment de 10 kb inclut la totalité du fragment *Pst*I précédemment étudié (insert de pSPM58) et permet d'avoir accès à une région non encore étudiée d'environ 4 kb. (cf figure 33). Il a été vérifié par digestion que le cosmide pSPM36 contenait bien ce fragment *Stu*I de 10 kb. Ce fragment a été isolé à partir du cosmide pSPM36, dans le but de déterminer la séquence de la fin d'*orf29* et d'autres gènes plus en amont de *orf29.* Pour cela, le cosmide pSPM36 a été digéré par l'enzyme de restriction *StuI.* Le fragment *Stu*I*-Stu*I d'une taille d'environ 10 kb a été isolé par électroélution à partir d'un gel d'agarose 0.8% puis cloné dans le vecteur pBK-CMV (4512bp) (commercialisé par la société Stratagene (La Jolla, Californie, USA)). Le plasmide ainsi obtenu a été nommé pSPM72 (cf. Figure 33). Ce dernier a ensuite été digéré par *Eco*RI (site *Eco*RI dans l'insert de pSPM58) et *Hind*III (site *Hind*III dans le site de clonage multiple du vecteur, immédiatement après le site *StuI* de l'extrémité de l'insert) (cf. Figure 33). Le fragment d'ADN *Eco*RI*-Hind*III ainsi obtenu, correspond à un fragment de l'insert du plasmide pSPM72 (cf. figure 33) et a été sous-cloné dans le vecteur pBC-SK+ (commercialisé par la société Stratagene (La Jolla, Californie, USA)) digéré au préalable par *Eco*RI *et Hind*III. Le plasmide ainsi obtenu a été nommé pSPM73 et la séquence de son insert a été déterminée. La séquence de cet insert est présentée en SEQ ID N° 135.
Un assemblage des séquences des inserts de pSPM58 et pSPM73 est présenté en SEQ ID N° 106. Cette séquence part du site *Pst*I au niveau de l'*orf23c* (cf. SEQ ID N° 80) et va jusqu'au site *Stu*I au niveau de l'*orf32c* (cf. figure 34). La séquence de l'*orf28c* (SEQ ID N° 111), n'étant pas complète (cf. exemple 19.3), une région d'environ 450 nucléotides n'est pas séquencée, ces 450 nucléotides figurent sous la forme d'une série de « N » dans la sequence SEQ ID N° 106).

### 19.5 Analyse des nouvelles séquences nucléotidiques, détermination des phases ouvertes de lecture et caractérisation des gènes impliqués dans la biosynthèse des spiramycines.

La séquence partielle de l'insert du cosmide pSPM73 obtenue a été analysée grâce au programme FramePlot (Ishikawa J & Hotta K. 1999). Ceci a permis d'identifier, parmi les phases ouvertes de lecture, les phases ouvertes de lecture présentant un usage des codons typique de *Streptomyces.* Cette analyse a permis de déterminer que cet insert comporte 4 ORFs, une incomplètes et trois complètes (cf. figure 34). L'ORF incomplète correspond à la partie 3' de la séquence codante de *orf29* ce qui à permis de compléter la séquence de ce gène grâce à la séquence partielle de ce même orf obtenue lors du séquençage de l'insert du plasmide pSPM58 (cf. exemple 19.2 et 19.3), l'ensemble des deux séquences à ainsi permis d'obtenir la séquence complète de *orf29.* Les 4 gènes ont ainsi été nommés *orf29* (SEQ ID N° 113), *orf30c* (SEQ ID N° 115), *orf31* (SEQ ID N° 118) et *orf32c* (SEQ ID N° 120). Le « c » ajouté dans le nom du gène signifiant pour l'ORF en question que la séquence codante est dans l'orientation inverse (cf. figure 34).

Les séquences protéiques déduites de ces phases ouvertes de lecture (SEQ ID N° 114 pour *orf29,* SEQ ID N° 116 et 117 pour *orf30c,* SEQ ID N° 119 pour *orf31* et SEQ ID N° 121 pour *orf32c*) ont été comparées avec celles présentes dans différentes bases de données grâce à différents programmes : BLAST (Altschul *et al.,* 1990) (Altschul *et al.,* 1997), CD-search, (ces deux programmes sont accessibles notamment auprès du National Center for Biotechnology Information (NCBI) (Bethesda, Maryland, USA)), FASTA ((Pearson W. R & D. J. Lipman, 1988) et (Pearson W. R., 1990) (ce programme est accessible notamment auprès du centre de ressources INFOBIOGEN, Evry, France). Ces comparaisons ont permis de formuler des hypothèses sur la fonction des produits de ces gènes et d'identifier ceux susceptibles d'être impliqués dans la biosynthèse de spiramycines.

### 19.6 Sous clonage et séquençage d'une troisième partie de l'insert du cosmide pSPM36.

Une sonde (fragment d'ADN de 0,8 kb) correspondant à une séquence interne à *orf32c* a été obtenue par PCR en utilisant comme matrice l'ADN total de la souche de *Streptomyces ambofaciens* et les amorces suivantes
**KF36:** 5'- TTGCCGTAGCCGAGGACCAGCG-3' (SEQ ID N° 151) et
**KF37:** 5'- CACATGGCCCTGGAGGACCCTG-3' (SEQ ID N° 152).

Le produit PCR ainsi obtenu représente une séquence interne de *l'orf32c.* Cette sonde à été utilisée dans des expériences de Southern Blot sur l'ADN chromosomique de la souche OSC2 et sur l'ADN du cosmide pSPM36 digérés par l'enzyme *PstI.* En utilisant les mêmes conditions d'hybridation que celles décrites ci-dessus (cf. exemple 19.1), cette sonde révèle deux fragments *PstI* d'environ 3,4kb et 2,5kb lorsque hybridée sur l'ADN total de la souche OSC2 et sur l'ADN du cosmide pSPM36 digérés par l'enzyme *PstI.* Compte tenu de la présence d'un site *PstI* dans la sonde utilisée on peut expliquer ces résultats. Le premier fragment d'ADN qui a une taille d'environ 3,4kb est un fragment dont la séquence est déjà connue en totalité. La séquence du fragment de 2,5 kb n'est connue que partiellement, sur une région de 700 pb. Ce fragment. a été isolé à partir du cosmide pSPM36 dans le but de déterminer la séquence de la fin de l'*orf32c* et d'autres gènes en amont de ce dernier. Pour cela, le cosmide pSPM36 a été digéré par l'enzyme de restriction *PstI.* Le fragment *Pst*I*-Pst*I d'une taille d'environ 2,5kb a été isolé par purification à partir d'un gel d'agarose 0.8% puis cloné dans le vecteur pBK-CMV (4518bp) (commercialisé par la société Stratagene (La Jolla, Californie, USA)). Le plasmide ainsi obtenu a été nommé pSPM79 (cf. Figure 41) et la séquence de son insert a été déterminée.

La séquence de l'*orf28c* (SEQ ID N° 111) n'était pas complète (cf. exemple 19.3). En effet, une région d'environ 450 nucléotides n'avait pas pu être déterminée, ces 450 nucléotides figurent sous la forme d'une sére de « N » dans la sequence SEQ ID N° 106. La séquence de la totalité de la région manquante a été déterminée par reséquençage de cette région. La séquence des inserts de pSPM58 et pSPM73 a donc été déterminée en entier. La séquence complète de la partie codante de l'*orf28c* est présentée en SEQ ID N° 141 et la protéine déduite de cette séquence en SEQ ID N° 142. La séquence de l'insert du plasmide pSPM79 est présentée en SEQ ID N° 161.

Un assemblage des séquences des inserts de pSPM58, pSPM73 et pSPM79 est présentée en SEQ ID N° 140 (cf. figure 41). Cette séquence part du site *PstI* au niveau de l'*orf23c* (cf. SEQ ID N° 80) et va jusqu'au site *PstI* au niveau de l'*orf34c* (cf. figure 41).

### 19.7 Analyse des nouvelles séquences nucléotidiques, détermination des phases ouvertes de lecture et caractérisation des gènes pouvant être impliqués dans la biosynthèse des spiramycines.

La séquence de l'insert du plasmide pSPM79 obtenue a été analysée grâce au programme FramePlot (Ishikawa J & Hotta K. 1999). Ceci a permis d'identifier, parmi les phases ouvertes de lecture, les phases ouvertes de lecture présentant un usage des codons typique de *Streptomyces.* Cette analyse a permis de déterminer que cet insert comporte 3 ORFs, deux incomplètes (*orf32c* et *orf34c)* et une complète (*orf33*) (cf. figure 41).

La première ORF incomplète correspond à la partie 5' de la séquence codante de *orf32c.* Ceci a permis de compléter la séquence de ce gène grâce à la séquence partielle de cette même orf obtenue lors du séquençage de l'insert du plasmide pSPM73 (cf. exemple 19.4 et 19.5), l'ensemble des deux séquences à ainsi permis d'obtenir la séquence complète de *orf32c.* (SEQ ID N° 145). Le « c » ajouté dans le nom du gène signifie pour l'ORF en question que la séquence codante est dans l'orientation inverse (cf. figure 41). L'orf complète a été nommée *orf33* (SEQ ID N° 147). La troisième ORF a été nommée *orf34c* (SEQ ID N° 149). Le « c » ajouté dans le nom du gène signifie pour l'ORF en question que la séquence codante est dans l'orientation inverse (cf. figure 37). Les comparaisons effectuées entre le produit de cette orf et les banques de données suggèrent que la partie C-terminale de cette protéine n'est pas dans le produit déduit de la séquence nucléotidique et donc que cette orf serait plus longue et continuerait en dehors de la région séquencée.

Les séquences protéiques déduites de ces phases ouvertes de lecture ont été comparées avec celles présentes dans différentes bases de données grâce à différents programmes : BLAST (Altschul et al., 1990) (Altschul et al., 1997), CD-search, (ces deux programmes sont accessibles notamment auprès du National Center for Biotechnology Information (NCBI) (Bethesda, Maryland, USA)), FASTA ((Pearson W. R & D. J. Lipman, 1988) et (Pearson W. R., 1990) (ce programme est accessible notamment auprès du centre de ressources INFOBIOGEN, Evry, France). Ces comparaisons ont permis de formuler des hypothèses sur la fonction des produits de ces gènes et d'identifier ceux susceptibles d'être impliqués dans la biosynthèse de spiramycines.

### EXEMPLE 20 : Analyse de la production d'intermédiaires de biosynthèse de spiramycines : (ne fait pas partie de l'invention)

### 20.1 Préparation des échantillons :

Les différentes souches à tester ont été cultivées chacune dans 7 erlenmeyers chicanés de 500 ml contenant 70 ml de milieu MP5 (Pernodet et al., 1993). Les erlens ont été inoculés par 2.5 10⁶ spores/ml des différentes souches de *S. ambofaciens* et mises à pousser à 27°C sous agitation orbitale de 250 rpm pendant 96 heures. Les cultures correspondant au même clone sont rassemblées, éventuellement filtrées sur filtre plissé, et centrifugées 15 min à 7000 tr/min.

Le pH du moût est ensuite ajusté à 9 avec de la soude et le surnageant est extrait par de la méthyl iso-butyryl cétone (MIBK). La phase organique (MIBK) est alors récupérée et évaporée. L'extrait sec est ensuite repris dans 1ml d'acétonitrile, puis dilué au 1/10 (100µl qsp 1ml avec de l'eau) avant d'être utilisé pour les analyses en chromatographie liquide couplée à la spectrométrie de masse (CL/SM).

### 20.2 Analyse des échantillons en CL/SM :

Les échantillons ont été analyés en CL/SM dans le but de déterminer la masse des différents produits synthétisés par les souches à tester.

La colonne de chromatographie liquide haute performance utilisée est une colonne Kromasil C8 150*4,6mmm, 5mmm, 100Å.
La phase mobile est un gradient constitué d'un mélange d'acétonitrile et d'une solution aqueuse d'acide trifluoroacétique à 0.05%, le débit est fixé à 1 ml/min. La température du four colonne est maintenue à 30°C.
La détection UV en sortie de la colonne a été effectuée à deux longueurs d'onde différentes : 238 nm et 280 nm.
Le spectrométre de masse couplé à la colonne de chromatographie est un appareil Simple Quadripole commercialisé par la société Agilent, avec des tensions de cone à 30 et à 70V.

### 20.3 Analyse des intermédiaires de biosynthèse produits par la souche OS49.67 :

La souche OS49.67 dans laquelle le gène *orf3* est inactivé par une délétion en phase ne produit pas de spiramycines (cf. exemple 6 et 15).
Un échantillon a été préparé selon la méthode décrite ci-dessus (cf. paragraphe 20.1) et a été analysé par CL/SM comme décrit ci-dessus (cf. le paragraphe 20.2).
Plus particulièrement, l'analyse par chromatographie a été réalisée en gradient de solvant avec comme phase mobile : 20% d'acétonitrile du temps T=0 à 5min puis montée linéaire à 30% à T=35 minutes, suivie d'un palier jusqu'à T=50 minutes.

Dans ces conditions, deux produits ont plus particulièrement été observés : un absorbant à 238 nm (temps de rétention de 33.4 min) et un absorbant à 280 nm (temps de rétention de 44.8 min) (cf. figure 35). La figure 35 montre la superposition des chromatogrammes CLHP réalisés à 238 et 280nm (haut) ainsi que les spectres UV des molécules élués à 33,4 minutes et 44,8 minutes (bas).

Les conditions d'analyses en spéctrométrie de masse couplée étaient les suivantes : le balayage se fait en mode scan, couvrant une gamme de masse comprise entre 100 et 1000 Da. Le gain de l'électro-multiplicateur était de 1V. Concernant la source électrospray, la pression du gaz de nébulisation était de 35 psig, le débit du gaz séchant de 12.0 1.min⁻¹, la température du gaz séchant de 350°C, la tension du capilaire était portée à +/- 3000 V. Ces expériences ont permis de déterminer la masse des deux produits séparés. Ces masses sont respectivement de 370 g/mole pour le produit élué en premier ([M-H₂O]⁺=353 produit majoritaire) et 368 g/mole pour le second produit ([M-H₂O]⁺=351 produit majoritaire).

Afin d'obtenir la structure, les produits mentionnés ci-dessus ont été isolés et purifiés dans les conditions suivantes : la phase mobile est un mélange d'une solution aqueuse d'acide trifluoroacétique à 0.05% et d'acétonitrile 70/30 (v/v). La chromatographie est réalisée en régime isocratique avec un débit fixé de 1 ml/min. Dans ces conditions, les temps de rétention des 2 produits sont respectivement de 8 et 13.3 minutes. De plus dans ce cas, l'échantillon préparé (cf. paragraphe 20.1) n'est pas dilué dans l'eau avant injecton de 10µl.

Les 2 produits sont récupérés à la sortie de la colonne chromatographique et isolés dans les conditions suivantes : une cartouche Oasis HLB 1 cc 30mg (Waters) est conditionnée séquentiellement par 1 ml d'acétonitrile, puis 1 ml d'eau/acétonitrile (20v/80v) et 1ml d'eau/acétonitrile 80/20. L'échantillon est ensuite introduit et la cartouche lavée successivement par 1 ml d'eau/acétonitrile (95/5), 1ml d'eau/acétonitrile deutéré (95/5), puis éluée avec 600µl d'eau/acétonitrile deutéré 40/60. La solution récupérée est ensuite directement analysée en RMN.
Les spectres RMN obtenus pour ces deux composés sont les suivants:
- Premier produit élué: Platenolide A: (Spectre 9646V)
   Spectre 1H dans CD3CN (déplacements chimiques en ppm): 0,90 (3H, t, J=6Hz), 0,93 (3H, d, J=5Hz), 1,27 (3H, d, J=5Hz), entre 1,27 et 1,40 (3H, m), 1,51 (1H, m), 1,95 (1H, m), 2,12 (1H, m), 2,30 (1H, d, J=12Hz), 2,50 (1H, d, J=11Hz), 2,58 (1H, dd, J=9 et 12 Hz), 2,96 (1H, d, J=7Hz), 3,43 (3H, s), 3,70 (1H, d, J=9Hz), 3,86 (1H, d, J=7Hz), 4,10 (1H, m), 5,08 (1H, m), 5,58 (1H, dt, J=3 et 12Hz), 5,70 (1H, dd, J=8 et 12 Hz), 6,05 (1H, dd, J=9 et 12 Hz), 6,24 (1H, dd, J=9 et 12Hz).
- Deuxième produit élué: Platenolide B: (Spectre 9647V)
   Spectre 1H dans CD3CN (déplacements chimiques en ppm): 0,81 (3H, t, J=6Hz), 0,89 (1H, m), 1,17 (3H, d, J=5Hz), 1,30 (4H, m), 1,47 (2H, m), 1,61 (1H, t, J=10Hz), 2,20 (1H, m), 2,38 (1H, d, J=13Hz), 2,52 (1H, m), 2,58 (1H, m), 2,68 (1H, dd, J=8 et 13Hz), 3,10 (1H, d, J=7Hz), 3,50 (3H, s), 3,61 (1H, d, J=8Hz), 3,82 (1H, d, J=7Hz), 5,09 (1H, m), 6,20 (1H, m), 6,25 (1H, dd, J=9 et 12 Hz), 6,58 (1H, d, J=12 Hz), 7,19 (1H, dd, J=9 et 12Hz).

Ces expériences ont ainsi permis de déterminer la structure de ces deux composés. Le premier produit élué est le platénolide A et le deuxième est le platénolide B, la structure déduite de ces deux méolécules est présentée en figure 36.

Il a également pu être déterminé grâce à la technique de CL/SM associée à la RMN, dans des conditions légèremment différentes de celles décrites précédemment mais dont la mise au point est bien connue de l'homme du métier, que la souche OS49.67 produit en plus du platénolide A et B un dérivé de ces deux composés. Il s'agit du platénolide A+mycaorse et du platénolide B+mycarose (la strucutre de ces deux composés est présenté en figure 40). Les résultats de l'analyse du moût de la souche OS49.67 sont présentés tableau 42.

**Tableau 42. Les résultats de l'analyse CL/SM du moût de la souche OS49.67**

| Identité | [ ] (mg/L) | Masses des ions | Max d'absorption |
|---|---|---|---|
| Platénolide A | 16,1 | [M + Na]⁺ 393,0 | 231nm |
| Masse exacte=370 | | [M + K]⁺ 408,9 | |
| Formule | | [M - H₂O + H]⁺ 353,0 | |
| Moléculaire=C₂₀H₃₄O₆ | | | |
| | | [2M + Na]⁺ 763,2 | |
| Platénolide B | 1.4 | [M - H₂O + H]⁺ 351,0 | 283nm |
| Masse exacte=368 | | [M + Na]⁺ 391,0 | |
| Formule | | [M +K]⁺ 406,9 | |
| Moléculaire=C₂₀H₃₂O₆ | | | |
| | | [2M + Na]⁺ 759,1 | |
| Platénolide A + 'mycarose' | 4,27 | [M + Na]⁺ 537,0 | 230nm |
| | | | |
| Masse exacte=514 | | [M +K]⁺ 553,0 | |
| Formule | | | |
| Moléculaire=C₂₇H₄₆O₉ | | | |
| Platénolide B + 'mycarose' | ND | [M + Na]⁺ 535,0 | 284nm |
| Masse exacte=512 | | [M +K]⁺ 550,9 | |
| | | | |
| Formule Moléculaire=C₂₇H₄₄O₉ | | [PlatB - H₂O+ H]⁺350,9 | |

### 20.4 Analyse des intermédiaires de biosynthèse produits par la souche SPM509 :

La souche SPM509 dans laquelle le gène *orf14* est inactivé (*orf14::att3Ωaac*-) ne produit pas de spiramycines (cf. exemple 13, 15 et 16). Un échantillon a été préparé selon la méthode décrite ci-dessus (cf. paragraphe 20.1) et a été analysé par CL/SM comme décrit ci-dessus (cf. le paragraphe 20.2 et 20.3). L'analyse des intermédiaires de biosynthèse présents dans le surnageant de culture de la souche SPM509 cultivée en milieu MP5 a montré que cette souche ne produisait que la forme B du platénolide (« platénolide B », cf. figure 36) mais pas la forme A (« platénolide A », cf. figure 36).

### EXEMPLE 21: Interruption du gène orf14 dans une souche interrompue dans le gène orf3 (OS49.67) (ne fait pas partie de l'invention)

Le produit du gène *orf14* est indispensable à la biosynthèse des spiramycines (cf. exemple 13, 15 et 16 : la souche SPM509 dans laquelle ce gène est interrompu ne produit plus de spiramycines). L'analyse des intermédiaires de biosynthèse présents dans le surnageant de culture de la souche SPM509 cultivée en milieu MP5 a montré que cette souche ne produisait que la forme B du platénolide mais pas la forme A (cf. exemple 20). Une des hypothèses pouvant expliquer cette observation est que le produit de *orf14* soit impliqué dans la conversion de la forme B du platénolide en forme A par une étape enzymatique de réduction. Pour tester cette hypothèse, le gène *orf14* a été inactivé dans un mutant ne produisant plus de spiramycine, mais produisant les formes A et B du platénolide. C'est le cas de la souche OS49.67 (cf. exemple 6 et 20) dans laquelle le gène *orf3* est inactivé par une délétion en phase (Δ*orf3*). Pour inactiver le gène *orf14* dans cette souche le plasmide pSPM509 a été introduit par transformation de protoplastes de la souche OS49.67 (Kieser, T *et al*., 2000). L'inactivation du gène *orf14* a déjà été décrite dans le cas de la souche OSC2 (cf. exemple 13) et il a été procédé de la même manière pour l'inactivation du gène *orf14* dans la souche OS49.67. Après transformation par le plasmide pSPM509, les clones sont sélectionnés pour leur résistance à l'apramycine. Les clones résistants à l'apramycine sont ensuite repiqués respectivement sur milieu avec apramycine et sur milieu avec hygromycine. Les clones résistants à l'apramycine (ApraR) et sensibles à l'hygromycine (HygS) sont en principe ceux où un double événement de crossing over s'est produit et dans lesquels le gène *orf14* a été remplacé par une copie de *orf14* interrompue par la cassette *att3Ωaac*-. Ces clones ont été plus particulièrement sélectionnés et le remplacement de la copie sauvage de *orf14* par la copie interrompue par la cassette a été vérifié par hybridation. Ainsi, l'ADN total des clones obtenus, a été digéré par plusieurs enzymes, séparé sur gel d'agarose, transféré sur membrane et hybridé avec une sonde correspondant à la cassette *att3Ωaac*- pour vérifier que le remplacement de gène avait bien eu lieu. La vérification du génotype peut également être réalisée par toute méthode connue de l'homme du métier et notamment par PCR en utilisant les oligonucléotides appropriés et séquençage du produit PCR.

Un clone présentant les caractéristiques attendues (*Δorf3*, *orf14::att3Ωaac*-) a été plus particulièrement sélectionné et nommé SPM510. Il a pu en effet être vérifié grâce aux deux hybridations que la cassette *att3Ωaac*- était bien présente dans le génome de ce clone et que l'on obtient bien le profil de digestion attendu dans le cas d'un remplacement, à la suite d'un double événement de recombinaison, du gène sauvage *orf14* par la copie interrompue par la cassette *att3Ωaac*- dans le génome de ce clone.

### EXEMPLE 22 : Complémentation fonctionnelle de l'interruption du gène orf14 (ne fait pas partie de l'invention)

### 22.1 Construction du plasmide pSPM519 :

Lé gène *orf14* a été amplifié par PCR en utilisant le couple d'oligonucléotides suivant: EDR31 : 5' CCC**AAGCTT**CTGCGCCCGCGGGCGTGAA 3' (SEQ ID N° 136) et EDR37: 5' GC**TCTAGA**ACCGTGTAGCCGCGCCCCGG 3' (SEQ ID N° 137) et comme matrice l'ADN chromosomique de la souche OSC2. Les oligonucléotides EDR31 et EDR37 portent respectivement le site de restriction *Hind*III et *Xba*I (séquence en gras). Le fragment de 1,2 kb ainsi obtenu a été cloné dans le vecteur pGEM-T easy (commercialisé par la société Promega (Madison, Wisconcin, USA)) pour donner naissance au plasmide pSPM515. Ce plasmide a ensuite été digéré par les enzymes de restriction *Hind*III et *Xba*I. L'insert *Hind*III/*Xba*I de 1,2 kb obtenu a été cloné dans le vecteur pUWL201 (cf. exemple 17.1) préalablement digéré par les mêmes enzymes. Le plasmide ainsi obtenu a été nommé pSPM519.

### 22.1 Transformation des souches SPM509 et SPM510 par le plasmide pSPM519:

Le plasmide pSPM519 a été introduit dans les souches SPM509 (cf. exemple 13) et SPM510 (cf. exemple 17) par transformation de protoplastes (Kieser, T *et al*., 2000). Après transformation, les clones sont sélectionnés pour leur résistance au thiostrepton. Les clones sont ensuite repiqués sur un milieu contenant du thiostrepton.

La souche SPM509 est une souche non productrice de spiramycine (cf. exemple 13, 15 et 16 et figure 24). La production en spiramycines de la souche SPM509 transformée par le vecteur pSPM519 (souche nommée SPM509 pSPM519) a été analysée en cultivant cette souche dans du milieu MP5 en présence de thiostrepton. Les surnageants de culture ont ensuite été analysés par CLHP (cf. exemple 16 et 17). Les résultats de cette analyse sont présentés dans le tableau 43, les données sont exprimées en mg par litre de surnageant. Les résultats correspondent à la production totale en spiramycines (obtenue en additionnant la production en spiramycine I, II et III). Il a été observé que la présence du vecteur pSPM519 dans la souche SPM509 restaure la production de spiramycines (cf. tableau 43).

**Tableau 43. Production en spiramycines de la souche SPM509 transformée par le vecteur pSPM519, (résultats exprimés en mg/l de surnageant).**

| **Souche** | **Production en spyramicines** |
|---|---|
| SPM509 pSPM519 | 58 |

La souche SPM510 transformée par le plasmide pSPM519 a été nommée SPM510 pSPM509.

### EXEMPLE 23 : Complémentation fonctionnelle de l'interruption du gène orf3 par le gène tylB de S. fradiae (ne fait pas partie de l'invention)

### 23.1 Construction du plasmide pOS49.52:

Le plasmide pOS49.52 correspond à un plasmide permettant l'expression de la protéine TylB chez *S. ambofaciens.* Pour le construire, la séquence codante du gène *tylB* de *S. fradiae* (Merson-Davies & Cundliffe, 1994, numéro d'accès GenBank : U08223 (séquence de la région), SFU08223 (séquence ADN) et AA21342 (séquence protéique)) a été introduite dans le plasmide pKC1218 (Bierman *et al*., 1992, Kieser *et al.,* 2000, une souche de *E. coli* contenant ce plasmide est accessible notamment auprès de l'ARS (NRRL) Agricultural Research Service Culture Collection) (Peoria, Illinois, USA), sous le numéro B-14790). De plus cette séquence codante à été placée sous le contrôle du promoteur ermE* (cf. ci-dessus, notamment exemple 17.1, Bibb *et al*., 1985, Bibb et al., 1994).

### 23.1 Transformation de la souches OS49.67 par le plasmide pOS49.52:

La souche OS49.67 dans laquelle le gène *orf3* est inactivé par une délétion en phase ne produit pas de spiramycines (cf. exemple 6 et 15). Le plasmide pOS49.52 a été introduit dans la souche OS49.67 par transformation de protoplastes (Kieser, T *et al*., 2000). Après transformation, les clones sont sélectionnés pour leur résistance à l'apramycine. Les clones sont ensuite repiqués sur un milieu contenant de l'apramycine. Un clone a été plus particulièrement sélectionné et a été nommé OS49.67 pOS49.52.

Comme il a été démontré ci-dessus, la souche OS49.67 ne produit pas de spiramycines (cf. exemple 6 et 15). La production en spiramycines de la souche OS49.67 transformée par le vecteur pOS49.52 a été analysée par la technique décrite dans l'exemple 15. Il a ainsi pu être démontré que cette souche possède un phénotype producteur de spiramycines. Ainsi la protéine TylB permet la complémentation fonctionnelle de l'interruption du gène *orf3.*

### EXEMPLE 24 : Amélioration de la production en spiramycines par surexpression du gène orf28c

### 24.1 Construction du plasmide pSPM75 :

Le gène *orf28c* a été amplifié par PCR en utilisant un couple d'oligonucléotides comportant un site de restriction *Hind*III ou un site de restriction *Bam*HI. Ces amorces ont la séquence suivante :
KF30 : 5' **AAGCTT**GTGTGCCCGGTGTACCTGGGGAGC 3' (SEQ ID N° 138) avec un site de restriction *Hin*dIII (qui figure en gras)
KF31:5' **GGATCC**CGCGACGGACACGACCGCCGCGCA 3' (SEQ ID N° 139) avec le site de restriction *Bam*HI (qui figure en gras)
Les amorces KF30 et KF31 portent respectivement le site de restriction *Hind*III et *Bam*HI (séquence en gras). Le couple d'amorces KF30 et KF31 permet d'amplifier un fragment d'ADN d'une taille d'environ 1,5 kb contenant le gène *orf28c* en utilisant comme matrice le cosmide pSPM36 (cf. ci-dessus). Le fragment de 1,5 kb ainsi obtenu a été cloné dans le vecteur pGEM-T easy (commercialisé par la société Promega (Madison, Wisconcin, USA)) pour donner naissance au plasmide pSPM74. Le plasmide pSPM74 a ensuite été digéré par les enzymes de restriction *Hind*III et *Bam*HI et l'insert *Hind*III/*Bam*HI d'environ 1,5 kb obtenu a été sous cloné dans le vecteur pUWL201 (cf. exemple 17.1) préalablement digéré par les mêmes enzymes. Le plasmide ainsi obtenu a été nommé pSPM75, il contient l'ensemble de la séquence codante de *orf28c* placée sous le contrôle du promoteur ermE*.

### 24.2 Transformation de la souche OSC2 par le plasmide pSPM75 :

Le plasmide pSPM75 a été introduit dans les souches OSC2 par transformation de protoplastes (Kieser, *T et al.,* 2000). Après transformation des protoplastes, les clones sont sélectionnés pour leur résistance au thiostrepton. Les clones sont ensuite repiqués sur un milieu contenant du thiostrepton et la transformation par le plasmide est vérifiée par extraction de plasmides. Deux clones ont plus particulièrement été sélectionnés et nommés OSC2/pSPM75(1) et OSC2/pSPM75(2).

Un échantillon de la souche OSC2/pSPM75(2) a été déposé auprès de la Collection Nationale de Cultures de Microorganismes (CNCM) Institut Pasteur, 25, rue du Docteur Roux 75724 Paris Cedex 15, France, le 6 octobre 2003 sous le numéro d'enregistrement I-3101.

Afin de tester l'effet de la surexpression du gène *orf28c* sur la production de spiramycines, la production en spiramycines des clones OSC2/pSPM75(1) et OSC2/pSPM75(2) a été testée par la technique décrite dans l'exemple 15. L'analyse de la production en spiramycines de la souche OSC2 a également été effectée en parallèle pour comparaison. Il a ainsi pu être observé que la présence du plasmide pSPM75 augmente significativement la production en spiramycines de la souche OSC2. Ceci démontre que la surexpression de *orf28c* conduit à une augmentation de la production en spiramycines et confirme son rôle en tant que régulateur.

La production en spiramycines des clones OSC2/pSPM75(1) et OSC2/pSPM75(2) a également été analysée par CLHP (de la même manière que dans l'exemple 17.2). L'analyse de la production en spiramycines de la souche OSC2 a également été effectée en parallèle pour comparaison. Les résultats de cette analyse sont présentés dans le tableau 44, les données sont exprimées en mg par litre de surnageant. Les résultats correspondent à la production totale en spiramycines (obtenue en additionnant la production en spiramycine I, II et III).

**Tableau 44. Production en spiramycines des souches dérivées de OSC2 transformé par pSPM75, (résultats exprimés en mg/l).**

| **Souche** | **Spiramycines** |
|---|---|
| OSC2 | 50 |
| OSC2/pSPM75(1) | 120 |
| OSC2/pSPM75(2) | 155 |

Ainsi, il est observé que la présence du plasmide pSPM75 augmente significativement la production en spiramycines de la souche OSC2. Ceci démontre bien que la surexpression de *orf28c* a un effet positif sur la production en spiramycines.

### EXEMPLE 25 : Analyse de la production d'intermédiaires de biosynthèse de spiramycines d'une souche inactivée dans le gène orf8 : (ne fait pas partie de l'invention)

La souche OS49.107, dans laquelle le gène *orf8* est inactivé par insertion de la cassette *Ωhyg,* ne produit pas de spiramycines (cf. exemple 7 et 15). Le gène *orf8* code une protéine présentant une relative forte similitude avec plusieurs aminotransférases et suggère fortement que le gène *orf8* code une 4 amino-transférase responsable de la réaction de transamination nécessaire à la biosynthèse de la forosamine (cf. figure 6). On s'attend donc à ce que la biosynthèse des spiramycines soit bloquée au stade de la forocidine (cf. figure 7). La souche OS49.107 qui est non productrice de spiramycine devrait donc produire de la forocidine.

Un échantillon de surnageant de la souche OS49.107 a été préparé selon la méthode décrite ci-dessus (cf. exemple 16, sans extraction au MIBK) et a été analysé par CL/SM comme décrit ci-dessus (cf. le paragraphe 20.2 et 20.3). En mode SIM, la masse 558 relative à l'ion moléculaire de la forocidine a été sélectionné et plusieurs pics ont été détectés. La présence de composés de masse 558 est compatible avec l'hypothèse du rôle de orf8 dans synthèse de forosamine.

### EXEMPLE 26 : Analyse de la production d'intermédiaires de biosynthèse de spiramycines d'une souche inactivée dans le gène orf12 : (ne fait pas partie de l'invention)

La souche SPM507, dans laquelle le gène *orf12* est inactivé, ne produit pas de spiramycines (cf. exemple 11 et 15). Le gène *orf12* coderait une 3,4 déshydratase responsable de la réaction de déshydratation nécessaire à la biosynthèse de la forosamine (cf. figure 6). On s'attend donc à ce que la biosynthèse des spiramycines soit bloquée au stade de la forocidine (ci. figure 7). La souche SPM507 qui est non productrice de spiramycine devrait donc produire de la forocidine.

Un échantillon de surnageant de la souche SPM507 a été préparé selon la méthode décrite ci-dessus (cf. exemple 16, sans extraction au MIBK) et a été analysé par CL/SM comme décrit ci-dessus (cf. le paragraphe 20.2 et 20.3). Dans ces conditions, le temps de rétention de la forocidine est d'environ 12,9 minutes. En mode SIM, la masse 558 relative à l'ion moléculaire [M+H]⁺ de la forocidine a été sélectionné et un pic a été détecté. La présence d'un composé à 558 permet de valider l'hypothèse du rôle du produit de *orf12* dans la biosynthèse des spiramycines.

Toutefois, la forocidine est présente en quantité relativement faible et dans ces conditions, un produit absorbant à 238 nm a plus particulièrement été observé (temps de rétention de 17,1 min). L'analyse en LC/SM a permis de déterminer la masse de ce composé qui est de 744,3 g/mole ([M+H]⁺=744,3 produit majoritaire).

Afin d'obtenir la structure, le produits mentionné ci-dessus a été isolé et purifié dans les conditions décrites précédemment (cf. paragraphe 20.1) ). La phase organique (MIBK) est alors récupérée et évaporée. L'extrait sec est repris avec de l'eau et extrait avec de l'heptane. La solution aqueuse est ensuite extraite par fixation sur une cartouche Oasis HLB 1 g (Waters SAS, St-Quentin en-Yvelines, France). Le composé est récupéré par élution avec un mélange eau/acétonitrile 30/70. Cette solution est ensuite injectée (100µL) sur la colonne analytique et les fractions récupérées sur cartouche Oasis HLB 1 cc 30mg (Waters). Avant utilisation, les cartouches Oasis HLB 1 cc 30mg (Waters) sont conditionnées séquentiellement par de l'acétonitrile, puis un mélangeeau/acétonitrile (20v/80v) et un mélanged'eau/acétonitrile 80/20.

La cartouche Oasis HLB 1 cc 30mg (Waters) est ensuite lavée successivement par 1 ml d'eau/acétonitrile (95/5), 1ml d'eau/acétonitrile deutéré (95/5), puis éluée avec 600µl d'eau/acétonitrile deutéré 40/60. La solution récupérée est ensuite directement analysée en RMN.

Le spectre RMN obtenu pour ce composé est le suivant (Spectre RMN 19312V) :
Spectre 1H dans CD3CN/D2O (déplacements chimiques en ppm): 0,92 (3H, d, J=6Hz), 1,10 (1H, m), 1,14 (3H, s), 1,17 (3H, d, J=6Hz), 1,22 (3H, d, J=6Hz), 1,25 (3H, d, J=6Hz), 1,40 (1H, m), 1,75 (1H, dd, J=12 et 2Hz), 1,81 (1H, m), 1,90 (1H, d, J=12Hz), 2,05 (1H, m), 2,12 (3H, s), 2,15 (1H, m), 2,35 (2H, m), 2,45 (6H, s large), 2,53 (1H, m), 2,64 (1H, dd, J=12 et 9Hz), 2,80 (1H, dd, J=9 et 16Hz), 2,95 (1H, d, J=8Hz), 3,23 (2H, m), 3,34 (1H, d, J=7Hz), 3,45 (3H, s), 3,49 (1H, m), 3,93 (1H, dd, J=7 et 3Hz), 4,08 (1H, m), 4,37 (1H, d, J=6Hz), 4,88 (1H, m), 5,05 (2H, m), 5,65 (2H, m), 6,08 (1H, dd, J=8 et 12Hz), 6,40 (1H, dd, J=12 et 9Hz), 9,60(1H,s).

Ces expériences ont ainsi permis de déterminer la structure de ce composé, celle-ci est présentée en figure 38.

### EXEMPLE 27 : Analyse de la production d'intermédiaires de biosynthèse de spiramycines d'une souche inactivée dans le gène orf5*: (ne fait pas partie de l'invention)

La souche SPM501 possède le génotype *orf6^{*}::att1Ωhyg*+. Grâce à l'effet polaire de l'insertion de la cassette *att1Ωhyg*+ dans le gène *orf 6**, il a pu être déterminé que le gène *orf 5** est indispensable à la voie de biosynthèse des spiramycines. En effet, l'insertion de la cassette excisable dans la partie codante du gène *orf 6** entraîne un arrêt total de la production en spiramycines par effet polaire sur l'expression du gène *orf5*.* Cependant, une fois que la cassette insérée a été excisée (et donc lorsque seul le gène *orf6** est inactivé, cf exemples 14 et 15), on restaure une production de spiramycine I. Ceci démontre que le gène *orf 5** est indispensable à la biosynthèse des spiramycines puisque son inactivation entraîne un arrêt total de la production en spiramycines.

Le gène *orf5** code une protéine présentant une relative forte similitude avec plusieurs O-méthyltransférases. Le gène *orf5** serait une O-méthyl transférase impliquée dans la biosynthèse du platénolide. Pour vérifier cette hypothèse, des expériences d'analyse CL/SM et de RMN ont été conduites sur une souche de S. *ambofaciens* de génotype *orf6*:: att1Ωhyg+* obtenue à partir d'une souche surproduisant les spiramycines.

Un échantillon du surnageant de cette souche a été préparé selon la méthode décrite ci-dessus (cf. exemple 16, sans extraction au MIBK) et a été analysé par CL/SM comme décrit ci-dessus (cf. le paragraphe 20.2 et 20.3). Toutefois, la colonne utilisée est une colonne X-Terra (Waters SAS, St-Quentin en-Yvelines, France), et la tension de cone du spectromètre est réglée à 380V pour obtenir la fragmentation du composé analysé. Dans ces conditions, un produit dont le temps de rétention est d'environ 13,1 minutes est observé. Le spectre de masse de ce composé a une allure similaire à celui de la spiramycine I mais l'ion moléculaire est à 829. La différence de masse de 14 par rapport à la masse de la spiramycine peut s'expliquer par l'absence de groupement méthyl sur l'oxygène portée par le carbone n°4 du cycle lactone (la strucutre de ce composé est présenté en figure 39). La présence d'un composé à 829 permet de valider l'hypothèse du rôle de *orf5** dans la biosynthèse des spiramycines.

Par un test microbioogique effectué sur une souche sensible de *M. luteus* (cf. exemple 15 et figure 18) il a été démontré que la molécule intermédiaire (spiramycine moins groupement méhyle dont la structure est présentée en figure 39) produite par la souche *orf6*::Ωatthyg+* est beaucoup moins active (d'un facteur 10) que la spiramycine d'origine avec le groupement méthyl en position 4.

### EXEMPLE 28 : Construction de nouvelles « cassettes excisables » :

De nouvelles cassettes excisables ont été construites. Ces cassettes sont très semblables aux cassettes excisables déjà décrites dans l'exemple 9. La différence principale entre les anciennes et les nouvelles cassettes est l'absence dans ces dernières des séquences correspondant aux extrémités de l'interposon Ω, séquences qui contiennent un terminateur de transcription provenant du phage T4.

Dans les cassettes sans terminateur, le gène qui confère la résistance à un antibiotique est flanqué des séquences *attR* et *attL* permettant l'excision. Le gène de résistance est le gène *aac(3)IV* qui code une acétyltransferase qui confère la résistance à l'apramycine. Ce gène est présent dans la cassette *Ωaac* (numéro d'accès GenBank : X99313, Blondelet-Rouault, M.H. *et al*., 1997) et a été amplifié par PCR en utilisant comme matrice le plasmide pOSK1102 (cf. ci-dessus) et comme amorces les oligonucléotides KF42 et KF43 contenant chacun le site de restriction *Hind*III (en gras)(**AAGCTT**) en 5'.
KF42: 5'-**AAGCTT**GTACGGCCCACAGAATGATGTCAC 3' (SEQ ID N° 153) et
KF43: 5'-**AAGCTT**CGACTACCTTGGTGATCTCGCCTT-3' (SEQ ID N° 154).

Le produit PCR obtenu d'environ 1kb a été cloné dans le vecteur *E. coli* pGEMT Easy donnant naissance au plasmide pSPM83.

Le vecteur pSPM83 a été digéré par l'enzyme de restriction *Hind*III. Le fragment *Hind*III-*Hind*III de l'insert a été isolé par purification à partir d'un gel d'agarose 0,8% puis cloné dans le site *Hind*III situé entre les séquences *att*L et *att*R des différents plasmides portant les différentes cassettes excisables possibles (cf. exemple 9 et figure 27) de façon à remplacer le fragment *Hind*III correspondant à *Ωacc* par le fragment *Hind*III correspondant au gène *aac* seul. Ceci a permit d'obtenir les cassettes *att1aac, att2aac et att3aac* (selon la phase souhaitée, cf. exemple 9). Selon l'orientation du gène *aac* par rapport aux séquences *att*L et *att*R, on distingue *att1aac*+*, att1aac*-*, att2aac*+*, att2aac*-*, att3aac*+ et *att3aac*- (selon les mêmes conventions que celles adoptées dans l'exemple 9).

### EXEMPLE 29 : Construction d'une souche de S. ambofaciens interrompue dans le gène orf28c :

L'inactivation du gène *orf28c* a été réalisée grâce à la technique des cassettes excisables. La cassette excisable *att3aac*+ (cf. exemple 28) a été amplifiée par PCR en utilisant comme matrice le plasmide pSPM101 (Le plasmide pSPM101 est un plasmide dérivé du vecteur pGP704Not (Chaveroche *et al.,* 2000) (Miller VL & Mekalanos JJ, 1988) dans lequel la cassette *att3aac*+ a été clonée comme un fragment *EcoR*V dans le site unique *EcoRV* de pGP704Not) et à l'aide des amorces suivantes :
KF32:
et KF33:

Les 39 nucléotides situés à l'extrémité 5' de ces oligonucléotides comportent une séquence correspondant à une séquence dans le gène *orf28c* et les 26 nucléotides situés le plus en 3' (figuré en gras et soulignés ci-dessus) correspondent à la séquence d'une des extrémités de la cassette excisable *att3aac+*
Le produit de PCR ainsi obtenu a été utilisé pour transformer la souche *E. coli* hyper-recombinante DY330 (Yu *et al*., 2000) (cette souche contient les gènes *exo*, *bet* et *gam* du phage lambda intégrés dans son chromosome, ces gènes sont exprimés à 42°C, elle a été utilisée à la place de la souche *E.coli* KS272 (Chaveroche *et al*., 2000)) contenant le cosmide pSPM36. Ainsi, les bactéries ont été transformées par électroporation par ce produit de PCR et les clones ont été sélectionnés pour leur résistance à l'apramycine. Les cosmides des clones obtenus ont été extraits et digérés par l'enzyme de restriction *BamH*I, dans le but de vérifier que le profil de digestion obtenu correspondait au profil attendu s'il y a eu insertion de la cassette (*att3aac*+) dans le gène *orf28c,* c'est à dire s'il y avait bien eu recombinaison homologue entre les extrémités du produit PCR et le gène cible. La vérification de la construction peut également être réalisée par toute méthode connue de l'homme du métier et notamment par PCR en utilisant les oligonucléotides appropriés et séquençage du produit de PCR. Un clone dont le cosmide possède le profil attendu a été sélectionné et le cosmide correspondant a été nommé pSPM107. Ce cosmide est un dérivé de pSPM36 dans lequel *orf28c* est interrompue par la cassette *att3aac+.* L'insertion de la cassette s'accompagne d'une délétion dans le gène *orf28c,* l'interruption commence au niveau du 28ème codon de orf28c Il reste après la cassette les 137 derniers codons de orf28c.

Le cosmide pSPM107 a dans un premier temps été introduit dans la souche *E. coli* DH5α puis dans la souche *Streptomyces ambofaciens* OSC2 par transformation de protoplastes. Après transformation, les clones sont sélectionnés pour leur résistance à l'apramycine. Les clones résistants à l'apramycine sont ensuite repiqués respectivement sur milieu avec apramycine (antibiotique B) et sur milieu avec puromycine (antibiotique A) (cf. figure 9). Les clones résistants à l'apramycine (ApraR) et sensibles à la puromycine (PuroS) sont en principe ceux où un double événement de crossing over s'est produit et qui possèdent le gène *orf28c* interrompu par la cassette *att3aac*+. Ces clones ont été plus particulièrement sélectionnés et le remplacement de la copie sauvage de *orf28c* par la copie interrompue par la cassette a été vérifié par hybridation. Ainsi, l'ADN total des clones obtenus, a été digéré par plusieurs enzymes, séparé sur gel d'agarose, transféré sur membrane et hybridé avec une sonde correspondant à la cassette *att3aac*+ pour vérifier la présence de la cassette au locus attendu dans l'ADN génomique des clones obtenus. La vérification du génotype peut également être réalisée par toute méthode connue de l'homme du métier et notamment par PCR en utilisant les oligonucléotides appropriés et séquençage du produit de PCR.

Un clone présentant les caractéristiques attendues (*orf28c::att3aac*+) a été plus particulièrement sélectionné et nommé SPM107. Ce clone possède donc le génotype : *orf28c::att3aac*+ et a été nommé SPM107. Il est inutile de procéder à l'excision de la cassette pour l'étude de l'effet de l'inactivation de *orf28c,* au vu de l'orientation des gènes (cf. figure 3). En effet, le fait que *orf29* soit orienté en sens opposé à *orf28c* montre que ces gènes ne sont pas co-transcrits. L'utilisation d'une cassette excisable permet en revanche d'avoir la possibilité de se débarrasser du marqueur de sélection à tout moment, notamment par transformation par le plasmide pOSV508.

Afin de tester l'effet de l'extinction du gène *orf28c* sur la production de spiramycines, la production en spiramycines de la souche SPM107 a été testée par la technique décrite dans l'exemple 15. Il a ainsi pu être démontré que cette souche possède un phénotype non producteur de spiramycines. Ceci démontre que le gène *orf28c* est un gène essentiel à la biosynthèse de la spiramycine chez *S. ambofaciens.*

### EXEMPLE 30 : Construction d'une souche de S. ambofaciens interrompue dans le gène orf31: (ne fait pas partie de l'invention)

L'inactivation du gène *orf31* a été réalisée grâce à la technique des cassettes excisables. La cassette excisable *att3aac+* a été amplifiée par PCR en utilisant comme matrice le plasmide pSPM101 et les oligonucléotides EDR71 et EDR72.
EDR71:
EDR72:

Les 39 nucléotides situés à l'extrémité 5' de ces oligonucléotides comportent une séquence correspondant à une séquence dans le gène *orf31* et les 26 nucléotides situés le plus en 3' (figurés en gras et soulignés ci-dessus) correspondent à la séquence d'une des extrémités de la cassette excisable *att3aac*+.

Le produit de PCR ainsi obtenu a été utilisé pour transformer la souche *E. coli* KS272 contenant le plasmide pKOBEG et le cosmide pSPM36, comme décrit par Chaveroche et al. (Chaveroche et al., 2000) (cf. figure 12 pour le principe, le plasmide pOS49.99 doit être remplacé par le cosmide pSPM36 et le plasmide obtenu n'est plus pSPM17 mais pSPM543). Ainsi, les bactéries ont été transformées par électroporation par ce produit de PCR et les clones ont été sélectionnés pour leur résistance à l'apramycine. Les cosmides des clones obtenus ont été extraits et digérés par plusieurs enzymes de restriction, dans le but de vérifier que le profil de digestion obtenu correspond au profil attendu s'il y a eu insertion de la cassette (*att3aac*+) dans le gène *orf31*, c'est à dire s'il y a bien eu recombinaison homologue entre les extrémités du produit PCR et le gène cible. La vérification de la construction peut également être réalisée par toute méthode connue de l'homme du métier et notamment par PCR en utilisant les oligonucléotides appropriés et séquençage du produit de PCR. Un clone dont le cosmide possède le profil attendu a été sélectionné et le cosmide correspondant a été nommé pSPM543. Ce cosmide est un dérivé de pSPM36 dans lequel *orf31* est interrompue par la cassette *att3aac*+ (cf. figure 12). L'insertion de la cassette s'accompagne d'une délétion dans le gène *orf31*, l'interruption commence au niveau du trente sixième codon de *orf31.* Il reste après la cassette les 33 derniers codons de *orf31.*

Le cosmide pSPM543 a été introduit dans la souche *Streptomyces ambofaciens* OSC2 (cf. ci-dessus) par transformation de protoplastes (Kieser, T et al., 2000). Après transformation, les clones sont sélectionnés pour leur résistance à l'apramycine. Les clones résistants à l'apramycine sont ensuite repiqués respectivement sur milieu avec apramycine (antibiotique B) et sur milieu avec puromycine (antibiotique A) (cf. figure 9). Les clones résistants à l'apramycine (ApraR) et sensibles à la puromycine (PuroS) sont en principe ceux où un double événement de crossing over s'est produit et qui possèdent le gène *orf31* interrompu par la cassette *att3aac*+. Ces clones ont été plus particulièrement sélectionnés et le remplacement de la copie sauvage de *orf31* par la copie interrompue par la cassette a été vérifié par hybridation. Ainsi, l'ADN total des clones obtenus, a été digéré par plusieurs enzymes, séparé sur gel d'agarose, transféré sur membrane et hybridé avec une sonde correspondant à la cassette *att3aac*+ pour vérifier la présence de la cassette au locus attendu dans l'ADN génomique des clones obtenus. Une deuxième hybridation a été effectuée en utilisant comme sonde un fragment d'ADN obtenu par PCR et correspondant à une très large partie de la séquence codante du gène *orf31*.

La vérification du génotype peut également être réalisée par toute méthode connue de l'homme du métier et notamment par PCR en utilisant les oligonucléotides appropriés et séquençage du produit de PCR.

Un clone présentant les caractéristiques attendues (*orf31::att3aac*+) a été plus particulièrement sélectionné et nommé SPM543. Il a pu en effet être vérifié grâce aux deux hybridations que la cassette *att3aac*+ était bien présente dans le génome de ce clone et que l'on obtient bien le profil de digestion attendu dans le cas d'un remplacement, à la suite d'un double événement de recombinaison, du gène sauvage par la copie interrompue par la cassette *att3aac*+ dans le génome de ce clone. Ce clone possède donc le génotype : *orf31::att3aac*+ et a été nommé SPM543. Il est inutile de procéder à l'excision de la cassette pour l'étude de l'effet de l'inactivation de *orf31*, au vu de l'orientation des gènes (cf. figure 3). En effet, le fait que *orf32c* soit orienté en sens opposé à *orf31* montre que ces gènes ne sont pas co-transcrits. L'utilisation d'une cassette excisable permet en revanche d'avoir la possibilité de se débarrasser du marqueur de sélection à tout moment, notamment par transformation par le plasmide pOSV508.

Afin de tester l'effet de l'extinction du gène *orf31* sur la production de spiramycines, la production en spiramycines de la souche SPM543 a été testée par la technique décrite dans l'exemple 15. Il a ainsi pu être démontré que cette souche possède un phénotype non producteur de spiramycines. Ceci démontre que le gène *orf31* est un gène essentiel à la biosynthèse de la spiramycine chez *S. ambofaciens.*

### EXEMPLE 31 Construction d'une souche de S. ambofaciens interrompue dans le gène orf32c: (ne fait pas partie de l'invention)

L'inactivation du gène orf32c a été réalisée grâce à la technique des cassettes excisables. La cassette excisable *att3aac+* a été amplifiée par PCR en utilisant comme matrice le plasmide pSPM101 et à l'aide des amorces suivantes :
KF52:
et KF53:

Les 40 nucléotides situés à l'extrémité 5' de ces oligonucléotides comportent une séquence correspondant à une séquence dans le gène *orf32c* et les 26 nucléotides situés le plus en 3' (figuré en gras et soulignés ci-dessus) correspondent à la séquence d'une des extrémités de la cassette excisable *att3aac*+.

Le produit de PCR ainsi obtenu a été utilisé pour transformer la souche *E. coli* hyper-recombinante DY330 (Yu *et al.,* 2000) contenant le cosmide pSPM36. Ainsi, les bactéries ont été transformées par électroporation par ce produit de PCR et les clones ont été sélectionnés pour leur résistance à l'apramycine. Les cosmides des clones obtenus ont été extraits et digérés par l'enzymes de restriction *BamH*I, dans le but de vérifier que le profil de digestion obtenu correspond au profil attendu s'il y a eu insertion de la cassette (*att3aac*+) dans le gène *orf32c*, c'est à dire s'il y a bien eu recombinaison homologue entre les extrémités du produit PCR et le gène cible. La vérification de la construction peut également être réalisée par toute méthode connue de l'homme du métier et notamment par PCR en utilisant les oligonucléotides appropriés et séquençage du produit de PCR. Un clone dont le cosmide possède le profil attendu a été sélectionné et le cosmide correspondant a été nommé pSPM106. Ce cosmide est un dérivé de pSPM36 dans lequel *orf32c* est interrompue par la cassette *att3aac*+. L'insertion de la cassette s'accompagne d'une délétion dans le gène *orf32c*, l'interruption commence au niveau du 112ème codon de *orf32c*. Il reste après la cassette les 91 derniers codons de *orf32c.*

Le cosmide pSPM106 a dans un premier temps été introduit dans la souche E. coli DH5α puis, dans la souche *Streptomyces ambofaciens* OSC2 par transformation. Après transformation, les clones sont sélectionnés pour leur résistance à l'apramycine. Les clones résistants à l'apramycine sont ensuite repiqués respectivement sur milieu avec apramycine (antibiotique B) et sur milieu avec puromycine (antibiotique A) (cf. figure 9). Les clones résistants à l'apramycine (ApraR) et sensibles à la puromycine (PuroS) sont en principe ceux où un double événement de crossing over s'est produit et qui possèdent le gène *orf32c* interrompu par la cassette *att3aac*+. Ces clones ont été plus particulièrement sélectionnés et le remplacement de la copie sauvage de *orf32c* par la copie interrompue par la cassette a été vérifié par hybridation. Ainsi, l'ADN total des clones obtenus, a été digéré par plusieurs enzymes, séparé sur gel d'agarose, transféré sur membrane et hybridé avec une sonde correspondant à la cassette *att3aac+* pour vérifier la présence de la cassette dans l'ADN génomique des clones obtenus. La vérification du génotype peut également être réalisée par toute méthode connue de l'homme du métier et notamment par PCR en utilisant les oligonucléotides appropriés et séquençage du produit de PCR.

Un clone présentant les caractéristiques attendues (*orf32c::att3aac*+) a été plus particulièrement sélectionné. Ce clone possède donc le génotype : *orf32c::att3aac*+ et a été nommé SPM106. Il est inutile de procéder à l'excision de la cassette pour l'étude de l'effet de l'inactivation de *orf32c,* au vu de l'orientation des gènes (cf. figure 3). En effet, le fait que *orf33* soit orienté en sens opposé à *orf32c* montre que ces gènes ne sont pas co-transcrits. L'utilisation d'une cassette excisable permet en revanche d'avoir la possibilité de se débarrasser du marqueur de sélection à tout moment, notamment par transformation par le plasmide pOSV508.

Afin de tester l'effet de l'extinction du gène *orf32c* sur la production de spiramycines, la production en spiramycines de la souche SPM106 a été testée par la technique décrite dans l'exemple 15. Il a ainsi pu être démontré que cette souche possède un phénotype producteur de spiramycines. Ceci démontre que le gène *orf32c* n'est pas un gène essentiel à la biosynthèse de la spiramycine chez *S. ambofaciens.*

### Liste des constructions décrites dans la présente demande

Liste des abréviations : Am : Ampicilline ; Hyg: Hygromycine ; Sp : Spiramycine ; Ts : Thiostrepton ; Cm : Chloramphénicol. Kn : Kanamycine, Apra : apramycine.

| **Nom de la construction** | **Marqueur de sélection** | **Principales caractéristiques** | **Référence** |
|---|---|---|---|
| pWE15 | Am | | (Wahl, *et al.*, 1987) |
| pWED1 | Am | pWE15 dans lequel un fragment *Hpa*I*-Hpa*I de 4.1 kb a été délété | (Gourmelen *et al.*, 1998) |
| pOJ260 | Apra | Conjugatif, non réplicatif chez *Streptomyces* | (Bierman *et al.,* 1992) |
| pHP45 *Ωhyg* | Hyg | Cassette *Ωhyg* dans pHP45. | (Blondelet-Rouault *et al.,* 1997) |
| pKC505 | Apra | Cosmide | (Richardson MA *et al.,* 1987) |
| pIJ486 | Ts | Plasmide réplicatif multicopies *Streptomyces* | (Ward *et al.*, 1986) |
| pOSint3 | Am | *ptrc*-*xis*-*int* dans pTrc99A | (Raynal *et al.,* 1998) |
| pWHM3 | Am, Ts | Vecteur navette réplicatif *E. coli*/*Streptomyces*. | (Vara *et al.,* 1989) |
| pKOBEG | Cm | | (Chaveroche *et al.*, 2000) |
| pGP704Not | Am | | (Chaveroche *et al.*, 2000) |
| pMBL18 | Am | | (Nakano *et al.*, 1995) |
| pGEM-T Easy | Am | vecteur *E. coli* pour clonage de produits PCR | Mezei *et al.,* 1994 |
| pOS49.1 | Am | pWED1 avec insert au niveau du site *Bam*HI | Exemple 2 |
| pOS49.11 | Am | Fragment *Sac*I de pOS49.1 dans pUC 19. | Exemple 2 |
| pOSC49.12 | Ch | Fragment *Xho*I de pOS49.11 dans pBC SK+ | Exemple 2 |
| pOS49.14 | Cm, Hyg | pOSC49.12 avec le gène *orf3* interrompu par la cassette *Ωhyg* | Exemple 2 |
| pOS49.16 | Apra, Hyg | Insert de pOS49.14 dans pOJ260 | Exemple 2 |
| pOS49.28 | Cm | Fragment *Bam*HI*-Pst*I de 3,7kb de pOS49.1 dans pBC SK+ | Exemple 3 |
| pOS44.1 | Apra, Sp | pKC505 contenant un insert conférant la résistance la spiramycine chez S. *griseofuscus* | (Pernodet *et al.,* 1999) |
| pOS44.2 | Ts, Sp | Fragment *Sau3A*I de 1,8 kb pOS44.1 dans pIJ486 | Exemple 3 |
| pOS44.4. | Am | Insert de pOS44.2 dans pUC 19 | Exemple 3 |
| pSPM5 | Am | pWED1 avec insert d'ADN de *S. ambofaciens* au niveau du site *Bam*HI | Exemple 3 |
| pSPM7 | Am | pWED1 avec insert d'ADN de *S. ambofaciens* au niveau du site *Bam*HI | Exemple 3 |
| pOSK1205 | Hyg | pBK-CMV dans lequel *hyg* remplace *neo* | Exemple 5 |
| pOS49.67 | Apra | Fragment *Eco*RI*-Sac*I de pOS49.1, comportant une délétion interne de 504 nucléotides, dans pOJ260 | Exemple 6 |
| pOS49.88 | Am | Fragment de 3.7 kb *Pst*I-*EcoRI* de pOS49.1 dans pUC19 | Exemple 7 |
| pOS49.106 | Am | pO49.88 avec *hyg* dans *orf8* (*hyg* et *orf8* dans la même orientation) | Exemple 7 |
| pOS49.120 | Am | pOS49.88 avec *hyg* dans *orf8* (*hyg et orf8* dans des orientations opposées) | Exemple 7 |
| pOS49.107 | Apra, Hyg | Insert de pOS49.106 dans pOJ260 | Exemple 7 |
| pOS49.32 | Am, Kn | Fragment de 1,5 kb interne à *orf10* dans pCR2.1-TOPO | Exemple 8 |
| pOS49.43 | Am, Kn | pOS49.32 avec *hyg* dans *orf10* (*hyg* et *orf10* dans la même orientation) | Exemple 8 |
| pOS49.44 | Am, Kn | pOS49.32 avec *hyg* dans *orf10* (*hyg* et *orf10* dans des orientations opposées) | Exemple 8 |
| pOS49.50 | Apra, Hyg | Insert de pOS49.43 dans pOJ260 | Exemple 8 |
| pWHM3Hyg | Am, Hyg | pWHM3 dans lequel *tsr* est remplacé par *hyg* | Exemple 10 |
| pOSV508 | Am, Ts | p*trc-xis-int* dans pWHM3 | Exemple 9 |
| patt1Ωhyg+ | Cm, Hyg | Cassette att1Ωhyg+ dans pBC SK+ dont le site *Hind*III a été supprimé | Exemple 9 |
| patt3Ωaac- | Cm, Gn | Cassette att3Ωaac- dans pBC SK+ dont le site *Hind*III a été supprimé | Exemple 9 |
| pOSV510 | Am, Hyg | pro *pra*-*Amh* dans pWHM3Hyg | Exemple 10 |
| pOS49.99 | Am | Fragment *Eco*RI*-Bam*HI de 4,5 kb de pSPM5 dans pUC19 | Exemple 10 |
| pOSK1102 | Am, Apra | pGP704Not contenant la cassette *att3Ωaac-* | Exemple 10 |
| pSPM17 | Am, Apra | pOS49.99 dans lequel *orf2* est interrompue par la cassette *att3Ωaac-* | Exemple 10 |
| pSPM21 | Hyg, Apra | pOSK1205 contenant l'insert *Eco*RI*-Xba*I de pSPM17 (dans lequel *orf2* est interrompue par la cassette *att3*Ω*aac-*) | Exemple 10 |
| pSPM502 | Am | Fragment *Bgl*II de 15,1 kb de pSPM7 dans pMBL18 | Exemple 11 |
| pSPM504 | Hyg | Insert de pSPM502 dans pOSK1205 | Exemple 11 |
| pSPM507 | Hyg, Apra | pSPM504 dans lequel *orf12* est interrompue par la cassette *att3Ωaac-* | Exemple 11 |
| pSPM508 | Hyg, Apra | pSPM504 dans lequel *orf13c* est interrompue par la cassette *att3Ωaac-* | Exemple 12 |
| pSPM509 | Hyg, Apra | pSPM504 dans lequel *orf14* est interrompue par la cassette *att3Ωaac*- | Exemple 13 |
| pBXL1111 | Am | Fragment de 1,11 kb contenant *orf6** amplifié par PCR à partir de pSPM7, dans le vecteur pGEM-T Easy | Exemple 14 |
| pBXL1112 | Am, Hyg | pBXL1111 dans lequel la cassette *att1Ωhyg*+ a été introduite après délétion de 120 pb dans la séquence codante du gène *orf6** | Exemple 14 |
| pBXL1113 | Apra, Hyg | Insert *Pst*I de 3.7 kb de pBXL1112 dans pOJ260 | Exemple 14 |
| pSPM520 | Am | Fragment de PCR amplifié par les oligonucléotides EDR39-EDR42 dans pGEM-T Easy | Exemple 17 |
| pSPM521 | Am | Fragment de PCR amplifié par les oligonucléotides EDR40-EDR42 dans pGEM-T Easy | Exemple 17 |
| pSPM522 | Am | Fragment de PCR amplifié par les oligonucléotides EDR41-EDR42 dans pGEM-T Easy | Exemple 17 |
| pUWL201 | Am, Ts | | (Doumith *et al.,* 2000) |
| pSPM523 | Am, Ts | Fragment *Hind*III*-Bam*HI de l'insert du plasmide pSPM520 dans le vecteur pUWL201 | Exemple 17 |
| pSPM524 | Am, Ts | Fragment *Hind*III*-Bam*HI de l'insert du plasmide pSPM521 dans le vecteur pUWL201 | Exemple 17 |
| pSPM525 | Am, Ts | Fragment *Hind*III*-Bam*HI de l'insert du plasmide pSPM522 dans le vecteur pUWL201 | Exemple 17 |
| pSPM527 | Am | pSPM521 avec décalage du cadre de lecture au niveau du site *Xho*I | Exemple 17 |
| pSPM528 | Am, Ts | Fragment *Hind*III-*Bam*HI de l'insert du plasmide pSPM527 dans le vecteur pUWL201 | Exemple 17 |
| pVF 10.4 | | | *(Vara et al.,* 1985; Lacalle *et al.,* 1989) |
| pPM803 | Ts | | (Mazodier,P. *et al.,* 1989) |
| pGEM-T-*pac-oriT* | Am | Cassette *pac-oriT* (amplifiée par PCR à partir de pVF 10.4 et pPM803) dans pGEM-T Easy | Exemple 18 |
| pWED2 | Am | Cassette *pac-oriT* obtenue à partir de *p*GEM*-*T-*pac-oriT* insérée dans pWED1 | Exemple 18 |
| pSPM34 | Am | pWED2 avec insert au niveau du site *Bam*HI | Exemple 19 |
| pSPM35 | Am | pWED2 avec insert au niveau du site *Bam*HI | Exemple 19 |
| pSPM36 | Am | pWED2 avec insert au niveau du site *Bam*HI | Exemple 19 |
| pSPM37 | Am | pWED2 avec insert au niveau du site *Bam*HI | Exemple 19 |
| pSPM38 | Am | pWED2 avec insert au niveau du site *Bam*HI | Exemple 19 |
| pSPM39 | Am | pWED2 avec insert au niveau du site *Bam*HI | Exemple 19 |
| pSPM40 | Am | pWED2 avec insert au niveau du site *BamH*I | Exemple 19 |
| pSPM41 | Am | pWED2 avec insert au niveau du site *Bam*HI | Exemple 19 |
| pSPM42 | Am | pWED2 avec insert au niveau du site *Bam*HI | Exemple 19 |
| pSPM43 | Am | pWED2 avec insert au niveau du site *Bam*HI | Exemple 19 |
| pSPM44 | Am | pWED2 avec insert au niveau du site *Bam*HI | Exemple 19 |
| pSPM45 | Am | pWED2 avec insert au niveau du site *Bam*HI | Exemple 19 |
| pSPM47 | Am | pWED2 avec insert au niveau du site *Bam*HI | Exemple 19 |
| pSPM48 | Am | pWED2 avec insert au niveau du site *Bam*HI | Exemple 19 |
| pSPM50 | Am | pWED2 avec insert au niveau du site *Bam*HI | Exemple 19 |
| pSPM51 | Am | pWED2 avec insert au niveau du site *Bam*HI | Exemple 19 |
| pSPM52 | Am | pWED2 avec insert au niveau du site *Bam*HI | Exemple 19 |
| pSPM53 | Am | pWED2 avec insert au niveau du site *Bam*HI | Exemple 19 |
| pSPM55 | Am | pWED2 avec insert au niveau du site *Bam*HI | Exemple 19 |
| pSPM56 | Am | pWED2 avec insert au niveau du site *Bam*HI | Exemple 19 |
| pSPM58 | Kn | Fragment *Pst*I*-Pst*I d'environ 6kb de l'insert de pSPM36 dans pBK-CMV | Exemple 19 |
| pSPM72 | Kn | Fragment *Stu*I*-Stu*I d'environ 10 kb de l'insert de pSPM36 cloné dans pBK-CMV | Exemple 19 |
| pSPM73 | Cm | Fragment *Eco*RI*-Hin*dIII de l'insert de pSPM72 dans pBC-SK+ | Exemple 19 |
| pSPM515 | Am | Fragment de PCR amplifié par EDR31-EDR37 dans pGEM-T easy | Exemple 22 |
| pSPM519 | Am, Ts | Insert *Hind*II/*Xba*I de pSPM515 dans pUWL201 | Exemple 22 |
| pOS49.52 | Apra | Séquence codante de *tylB* sous contrôle du promoteur ermE* dans le plasmide pKC1218 | Exemple 23 |
| pSPM74 | Am | Fragment de PCR amplifié par KF30-KF31 dans pGEM-T easy | Exemple 24 |
| pSPM75 | Am, Ts | Insert *Hind*III/*Bam*HI de pSPM74 dans pUWL201 | Exemple 24 |
| pSPM79 | Kn | Fragment *Pst*I*-Pst*I d'environ 2,5kb de l'insert de pSPM36 dans pBK-CMV | Exemple 19 |
| pSPM83 | Am | Fragment de PCR amplifié par KF42-KF43 dans pGEM-T easy | Exemple 28 |
| pSPM107 | Am, Apra | pSPM36 dans lequel *orf28c* est interrompue par la cassette *att3aac*+ | Exemple 29 |
| pSPM543 | Am, Apra | pSPM36 dans lequel *orf31* est interrompue par la cassette *att3aac*+ | Exemple 30 |
| pSPM106 | Am, Apra | pSPM36 dans lequel *orf32c* est interrompue par la cassette *att3aac*+ | Exemple 31 |

### Dépôt de matériel biologique

Les organismes suivants ont été déposés le 10 juillet 2002 auprès de la Collection Nationale de Cultures de Microorganismes (CNCM), 25 rue du Docteur Roux, 75724 Paris Cedex 15, France, selon les dispositions du Traité de Budapest.
- Souche OSC2 sous le numéro d'enregistrement 1-2908.
- Souche SPM501 sous le numéro d'enregistrement 1-2909.
- Souche SPM502 sous le numéro d'enregistrement 1-2910.
- Souche SPM507 sous le numéro d'enregistrement 1-2911.
- Souche SPM508 sous le numéro d'enregistrement 1-2912.
- Souche SPM509 sous le numéro d'enregistrement 1-2913.
- Souche SPM21 sous le numéro d'enregistrement 1-2914.
- Souche SPM22 sous le numéro d'enregistrement 1-2915.
- Souche OS49.67 sous le numéro d'enregistrement 1-2916.
- Souche OS49.107 sous le numéro d'enregistrement 1-2917.
- Souche *Escherichia coli* DH5α contenant le plasmide pOS44.4 sous le numéro d'enregistrement 1-2918.

Les organismes suivants ont été déposés le 26 février 2003 auprès de la Collection Nationale de Cultures de Microorganismes (CNCM), 25 rue du Docteur Roux, 75724 Paris Cedex 15, France, selon les dispositions du Traité de Budapest.
- Souche SPM502 pSPM525 sous le numéro d'enregistrement I-2977.

Les organismes suivants ont été déposés auprès de la Collection Nationale de Cultures de Microorganismes (CNCM) Institut Pasteur, 25, rue du Docteur Roux 75724 Paris Cedex 15, France, le 6 octobre 2003 selon les dispositions du Traité de Budapest.
- Souche OSC2/pSPM75(2) sous le numéro d'enregistrement 1-3101.

Toutes les publications et brevets cités sont incorporés à la présente demande par référence.

### Bibliographie :

- Altschul SF, Gish W, Miller W, Myers EW, Lipman DJ. Basic local alignment search tool. J Mol Biol. (1990). 215 (3):403-410.
- Altschul SF, Madden TL, Schaffer AA, Zhang J, Zhang Z, Miller W, Lipman DJ. Gapped BLAST and PSI-BLAST: a new génération of protein database search programs. Nucleic Acids Res. (1997). 25 (17):3389-402.
- Amann,E., Ochs,B. and Abel,K.J. Tightly regulated tac promoter vectors useful for the expression of unfused and fused proteins in Escherichia coli Gene (1988) 69 (2), 301-315.
- Arisawa,A., Kawamura,N., Tsunekawa,H., Okamura,K., Tone,H. and Okamoto,R. Cloning and nucleotide sequences of two genes involved in the 4"-O-acylation of macrolide antibiotics from Streptomyces thermotolerans. Biosci. Biotechnol. Biochem. (1993). 57 (12) : 2020-2025.
- Arisawa A, Kawamura N, Takeda K, Tsunekawa H, Okamura K, Okamoto R. Cloning of the macrolide antibiotic biosynthesis gene acyA, which encodes 3-O-acyltransferase, from Streptomyces thermotolerans and its use for direct fermentative production of a hybrid macrolide antibiotic. Appl Environ. Microbiol. (1994). 60 (7):2657-2660.
- August,P.R., Tang,L., Yoon,Y.J., Ning,Streptomyces, Mueller,R., Yu,T.W., Taylor,M., Hoffmann,D., Kim,C.G., Zhang,X., Hutchinson,C.R. et Floss,H.G. Biosynthesis of the ansamycin antibiotic rifamycin: déductions from the molecular analysis of the rif biosynthetic gene cluster of Amycolatopsis mediterranei S699. Chem. Biol. (1998) 5 (2), 69-79.
- Ausubel Fred M., Brent Roger, Kingston Robert E., Moore David D., Seidman J.G., Smith John A., Struhl Kevin (Editeurs). Current Protocols in Molecular Biology, publié par John Wiley & Sons Inc. Current Protocols Customer Service, 605 Third Avenue, 9th Floor New York, NY 10158 USA (édition mise à jour de Mars 2002).
- Baltz RH, McHenney MA, Cantwell CA, Queener SW, Solenberg PJ. Applications of transposition mutagenesis in antibiotic producing streptomycetes. Antonie Van Leeuwenhoek. (1997). 71 (1-2): 179-187.
- Bate N, Butler AR, Gandecha AR, Cundliffe E. Multiple regulatory genes in the tylosin biosynthetic cluster of Streptomyces fradiae. Chem Biol. (1999). 6 (9): 617-624.
- Bate,N., Butler,A.R., Smith,I.P. et Cundliffe,E. The mycarose-biosynthetic genes of Streptomyces fradiae, producer of tylosin. Microbiology (2000). 146 (Pt 1), 139-146.
- Bayley C., Morgan, Dale E. C., Ow D. W. Exchange of gene activity in transgenic plants catalysed by the Cre-lox site specific system. Plant Mol. Biol (1992). 18 :353-361.
- Bentley,S.D., Chater,K.F., Cerdeno-Tarraga,A.M., Challis,G.L., Thomson,N.R., James,K.D., Harris,D.E., Quail,M.A., Kieser,H., Harper,D., Bateman,A., Brown,S., Chandra,G., Chen,C.W., Collins,M., Cronin,A., Fraser,A., Goble,A., Hidalgo,J., Hornsby,T., Howarth,S., Huang,C.H., Kieser,T., Larke,L., Murphy,L., Oliver,K., O'Neil,S., Rabbinowitsch,E., Rajandream,M.A., Rutherford,K., Rutter,S., Seeger,K., Saunders,D., Sharp,S., Squares,R., Squares,S., Taylor,K., Warren,T., Wietzorrek,A., Woodward,J., Barrell,B.G., Parkhill,J. et Hopwood,D.A. Complete genome sequence of the model actinomycete Streptomyces coelicolor A3(2). Nature (2002). 417 (68,85), 141-147.
- Bibb MJ, Findlay PR, Johnson MW. The relationship between base composition and codon usage in bacterial genes and its use for the simple and reliable identification of protein-coding sequences. Gene. (1984) 30 (1-3):157-166.
- Bibb MJ, Janssen GR, Ward JM. Cloning and analysis of the promoter région of the erythromycin résistance gene (ermE) of Streptomyces erythraeus. Gene. (1985). 38 (1-3) : 215-26.
- Bibb MJ, White J, Ward JM, Janssen GR. The mRNA for the 23S rRNA methylase encoded by the ermE gene of Saccharopolyspora erythraea is translated in the absence of a conventional ribosome-binding site. Mol. Microbiol. (1994). 14 (3): 533-45.
- Bierman M, Logan R, O'Brien K, Seno ET, Rao RN, Schoner BE. Plasmid cloning vectors for the conjugal transfer of DNA from Escherichia coli to Streptomyces spp. Gene. 1992;116(1):43-9.
- Blondelet-Rouault M-H., Weiser J., Lebrihi A., Branny P. and Pernodet J-L. Antibiotic résistance gene cassettes derived from the Ω interposon for use in E. coli and Streptomyces. Gene (1997) 190 : 315-317.
- Boccard F., Smokvina T., J.-L., Pernodet Fridmann A., and Guérineau M., Structural analysis of loci involved in pSAM2 site-specific intégration in Streptomyces. Plasmid, (1989a). 21 : 59-70.
- Boccard F., Smokvina T., J.-L., Pernodet Fridmann A., and Guérineau M.. The integrated conjugative plasmpid pSAM2 of Streptomyces ambofaciens is related to temperate bacteriophages. EMBOJ. (1989b) 8 : 973-980.
- Brunelli J.P., Pall M.L., A series of Yeast/ E. coli lambda expression vectors designed for directional cloning of cDNA and cre/lox mediated plasmid excision. Yeast. (1993) 9: 1309-1318.
- Camilli A., Beattie D. T. et Mekalanos J. J. Use of genetic recombination as a reporter of gene expression. Proc. Natl. Acad. Sci. USA (1994) 91(7), 2634-2638.
- Campelo,A.B. and Gil,J.A. The candicidin gene cluster from Streptomyces griseus IMRU 3570. Microbiology (2002) 148 (Pt 1), 51-59.
- Carreras C, Frykman S, Ou S, Cadapan L, Zavala S, Woo E, Leaf T, Carney J, Burlingame M, Patel S, Ashley G, Licari P. Saccharopolyspora erythraea-catalyzed bioconversion of 6-deoxyerythronolide B analogs for production of novel erythromycins. J Biotechnol. (2002). 92(3): 217-28.
- Chao K.-M., Pearson W.R., Miller W. Aligning two sequences within a specified diagonal band. Complut. Appl. Biosci. (1992) 8 : 481-487.
- Chater K. F. The improving prospects for yield increase by genetic engineering in antibiotic-producing Streptomycetes. Biotechnology. (1990). 8 (2) : 115-121.
- Chaveroche MK, Ghigo JM, d'Enfert C. A rapid method for efficient gene replacement in the filamentous fungus Aspergillus nidulans. Nucleic Acids Res. 2000; 28(22) :E97.
- Church GM, Gilbert W. Genomic sequencing. Proc Natl Acad Sci U S A. (1984) 81 (7):1991-5.
- Churchward G, Belin D, Nagamine Y. A pSC101-derived plasmid which shows no sequence homology to other commonly used cloning vectors. Gene. (1984) 31 (1-3):165-71.
- Comstock,L.E., Coyne,M.J., Tzianabos,A.O. and Kasper,D.L. Interstrain variation of the polysaccharide B biosynthesis locus of Bacteroides fragilis: characterization of the région from strain 638R J. Bacteriol. (1999). 181 (19) : 6192-6196.
- Cox KL, Baltz RH. Restriction of bacteriophage plaque formation in Streptomyces spp. J Bacteriol. (1984) 159(2): 499-504.
- Dale E.C., Ow D.W., Gene transfer with subséquent removal of the selection gene from the host genome. Proc. Natl. Acad. Sci. USA (1991). 88 : 10558-10562.
- Doumith M, Weingarten P, Wehmeier UF, Salah-Bey K, Benhamou B, Capdevila C, Michel JM, Piepersberg W, Raynal MC. Analysis of genes involved in 6-deoxyhexose biosynthesis and transfer in Saccharopolyspora erythraea. Mol Gen Genet. (2000) 264(4): 477-485.
- Draeger,G., Park,Streptomyces-H.H. et Floss,H.G. Mechanism of the 2-deoxygenation step in the biosynthesis of the deoxyhexose moieties of the antibiotics granaticin and oleandomycin J. Am. Chem. Soc. (1999) 121 : 2611-2612.
- Gandecha,A.R., Large,S.L. et Cundliffe,E. Analysis of four tylosin biosynthetic genes from the tylLM région of the Streptomyces fradiae genome. Gene. (1997). 184 (2) :197-203.
- Geistlich,M., Losick,R., Turner,J.R. et Rao,R.N. Characterization of a novel regulatory gene governing the expression of a polyketide synthase gene in Streptomyces ambofaciens. Mol. Microbiol. (1992). 6 (14) : 2019-2029.
- Gourmelen A, Blondelet-Rouault MH, Pernodet JL. Characterization of a glycosyl transferase inactivating macrolides, encoded by gimA from Streptomyces ambofaciens. Antimicrob Agents Chemother. (1998). 42(10): 2612-9.
- Huang X. et Miller W. A time-efficient, linear-space local similarity algorithin. Adv. Appl. Math. (1991). 12 : 337-357.
- Hara O et Hutchinson CR. Cloning of midecamycin(MLS)-resistance genes from Streptomyces mycarofaciens, Streptomyces lividans and Streptomyces coelicolor A3(2). JAntibiot (Tokyo). (1990). 43 (8):977-991.
- Hara O et Hutchinson CR. A macrolide 3-O-acyltransferase gene from the midecamycin-producing species Streptomyces mycarofaciens. J Bacteriol. (1992). 174(15) :5141-5144.
- Hoffmeister D, Ichinose K, Domann S, Faust B, Trefzer A, Drager G, Kirschning A, Fischer C, Kunzel E, Bearden D, Rohr J et Bechthold A. The NDP-sugar co-substrate concentration and the enzyme expression level influence the substrate specificity of glycosyltransferases: cloning and characterization of deoxysugar biosynthetic genes of the urdamycin biosynthetic gene cluster. Chem Biol. (2000). 7(11):821-831.
- Hopwood, D.A. Future possibilities for the discovery of new antibiotics by genetic engineering. In Beta-lactam antibiotics. Edited by M.R.J. Salton and G.D. Shockman. New York: Academic Press. (1981). 585-598.
- Hopwood D. A., Malpartida F., Kieser H. M., Ikeda H., Duncan J., Fujii I., Rudd A. M., Floss H. G. et Omura S. Production of 'hybrid' antibiotics by genetic engineering. Nature. (1985a). 314 (6012): 642-644.
- Hopwood D. A., Malpartida F., Kieser H. M., Ikeda H. et Omura S. (1985b) In Microbiology (ed S. Silver). American Society for Microbiology, Washington D. C., 409-413.
- Houben Weyl, 1974, in Meuthode der Organischen Chemie, E. Wunsch Ed., Volume 15-I et 15-II,
- Hutchinson, C.R. Prospects for the discovery of new (hybrid) antibiotics by genetic engineering of antibiotic-producing bacteria. Med Res Rev. (1988). 8 (4) : 557-567.
- Hutchinson C. R., Borell C. W., Otten S. L., Stutzman-Engwall K. J. et Wang Y. Drug discovery and development through the genetic engineering of antibiotic-producing microorganisms J. Med. Chem. (1989) 32 (5) : 929-937.
- Ishikawa J, Hotta K. FramePlot: a new implementation of the frame analysis for predicting protein-coding régions in bacterial DNA with a high G + C content. FEMS Microbiol Lett. (1999) 174(2):251-3.
- Kieser, T, Bibb, MJ, Buttner MJ, Chater KF, Hopwood DA. Practical Streptomyces Genetics. 2000. The John Innes Foundation, Norwich UK.
- Kuhstoss et al. Production of a novel polyketide through the construction of a hybrid polyketide synthase. Gene. (1996). 183(1-2): 231-236.
- Lacalle RA, Pulido D, Vara J, Zalacain M, Jimenez A. Molecular analysis of the pac gene encoding a puromycin N-acetyl transferase from Streptomyces alboniger. Gene. (1989). 79(2):375-380.
- Lakso M, Sauer B, Mosinger B Jr, Lee EJ, Manning RW, Yu SH, Mulder KL, Westphal H. Targeted oncogene activation by site-specific recombination in transgenic mice. Proc Natl Acad Sci U S A. (1992) 89 (14) :6232-6.
- Li,T.B., Shang,G.D., Xia,H.Z. and Wang,Y.G. Cloning of the sugar related biosynthesis gene cluster from Streptomyces tenebrarius H6. Sheng Wu Gong ChengXue Bao (2001). 17 (3) : 329-331.
- Ligon,J., Hill,S., Beck,J., Zirkle,R., Molnar,I., Zawodny,J., Money,S. and Schupp,T. Characterization of the biosynthetic gene cluster for the antifungal polyketide soraphen A from Sorangium cellulosum So ce26. Gene (2002). 285 (1-2), 257-267.
- Liu L, Saevels J, Louis P, Nelis H, Rico S, Dierick K, Guyomard S, Roets E, Hoogmartens J. Interlaboratory study comparing the microbiological potency of spiramycins I, II and III. J Pharm Biomed Anal. (1999). 20 (1-2):217-24.
- Mazodier P, Petter R, Thompson C. Intergeneric conjugation between Escherichia coli and Streptomyces species. J Bacteriol. (1989). 171 (6):3583-5.
- Merrifield RB, 1965a, Nature, 207(996): 522-523.
- Merrifield RB., 1965b, Science, 150(693): 178-185.
- Merson-Davies,L.A. et Cundliffe,E. Analysis of five tylosin biosynthetic genes from the tyllBA région of the Streptomyces fradiae genome, Molecular microbiology. (1994). 13 (2) : 349-355.
- Miller VL, Mekalanos JJ. A novel suicide vector and its use in construction of insertion mutations: osmoregulation of outer membrane proteins and virulence déterminants in Vibrio cholerae requires toxR. J Bacteriol. (1988) 170(6):2575-83.
- Nakano, Y., Yoshida, Y., Yamashita, Y. & Koga, T. Construction of a series of pACYC-derived plasmid vectors. Gene (1995). 162: 157-8.
- Nielsen H., Engelbrecht J., Brunak S. et von Heijne G. Identification of prokaryotic and eukaryotic signal peptides and prédiction of their cleavage sites. Protein Engineering. (1997). 10: 1-6.
- Oh SH, Chater KF. Denaturation of circular or linear DNA facilitates targeted integrative transformation of Streptomyces coelicolor A3(2): possible relevance to other organisms. J Bacteriol. 1997; 179 (1):122-7.
- Olano C, Lomovskaya N, Fonstein L, Roll JT, Hutchinson CR. A two-plasmid system for the. glycosylation of polyketide antibiotics: bioconversion of epsilon-rhodomycinone to rhodomycin D. Chem Biol. (1999). 6 (12):845-55.
- Omura S, Kitao C, Hamada H, Ikeda H. Bioconversion and biosynthesis of 16-membered macrolide antibiotics. X. Final steps in the biosynthesis of spiramycin, using enzyme inhibitor: cerulenin. Chem Pharm Bull (Tokyo). (1979a). 27(1): 176-82.
- Omura S, Ikeda H, Kitao C. Isolation and properties of spiramycin I 3-hydroxyl acylase from Streptomyces and ambofaciens. J Biochem (Tokyo). (1979b). 86(6): 1753-8.
- Omura,S., Ikeda,H., Ishikawa,J., Hanamoto,A., Takahashi,C., Shinose,M., Takahashi,Y., Horikawa,H., Nakazawa,H., Osonoe,T., Kikuchi,H., Shiba,T., Sakaki,Y. and Hattori,M. Genome sequence of an industrial microorganism Streptomyces avermitilis: Deducing the ability of producing secondary metabolites. Proc. Natl. Acad. Sci. U.S.A. (2001). 98 (21), 12215-12220.
- Pearson W.R. and D. J. Lipman. Improved Tools for Biological Sequence Analysis. Proc. Natl. Acad. Sci. USA 1988, 85: 2444-2448.
- Pearson W. R. Rapid and Sensitive Sequence Comparison with FASTP and FASTA. Methods in Enzymology. 1990, 183: 63-98.
- Pernodet JL, Simonet JM, Guerineau M. Plasmids in différent strains of Streptomyces ambofaciens: free and integrated form of plasmid pSAM2. Mol Gen Genet. (1984);198 (1):35-41.
- Pernodet JL, Alegre MT, Blondelet-Rouault MH, Guerineau M. Résistance to spiramycin in Streptomyces ambofaciens, the producer organism, involves at least two différent mechanisms. J Gen Microbiol. (1993) ; 139 (Pt 5):1003-11.
- Pernodet JL, Fish S, Blondelet-Rouault MH, Cundliffe E. The macrolide-lincosamide-streptogramin B résistance phenotypes characterized by using a specifically deleted, antibiotic-sensitive strain of Streptomyces lividans. Antimicrob Agents Chemother. (1996), 40 (3): 581-5.
- Pernodet JL, Gourmelen A, Blondelet-Rouault MH, Cundliffe E. Dispensable ribosomal résistance to spiramycin conferred by srmA in the spiramycin producer Streptomyces ambofaciens. Microbiology. (1999), 145 (Pt 9):2355-64.
- Pfoestl,A., Hofinger,A., Kosma,P. and Messner,P. Biosynthesis of dTDP-3-acetamido-3,6-dideoxy-alpha-D-galactose in Aneurinibacillus thermoaerophilus L420-91T. J. Biol. Chem. (2003), 278 (29): 26410-26417.
- Rao RN, Richardson MA, Kuhstoss S. Cosmid shuttle vectors for cloning and analysis of Streptomyces DNA. Methods Enzymol. (1987); 153: 166-98.
- Raynal A, Tuphile K, Gerbaud C, Luther T, Guerineau M, and Pernodet JL.. Structure of the chromosomal insertion site for pSAM2: functional analysis in Escherichia coli. Molecular Microbiology (1998) 28 (2) 333-342.
- Redenbach,M., Kieser,H.M., Denapaite,D., Eichner,A., Cullum,J., Kinashi,H. et Hopwood,D.A. A set of ordered cosmids and a detailed genetic and physical map for the 8 Mb Streptomyces coelicolor A3(2) chromosome Mol. Microbiol. (1996). 21 (1) : 77-96.
- Richardson MA, Kuhstoss S, Solenberg P, Schaus NA, Rao RN. A new shuttle cosmid vector, pKC505, for streptomycetes: its use in the cloning of three différent spiramycin-resistance genes from a Streptomyces ambofaciens library. Gene. (1987); 61 (3):231-41.
- Robinson, J.A. Enzymes of Secondary Metabolism in Microorganisms. Chem Soc Rev. (1988). 17:383-452.
- Russell SH, Hoopes JL, Odell JT. Directed excision of a transgene from the plant genome. Mol Gen Genet. (1992). 234 (1): 49-59.
- Sambrook J, Frisch EF, Maniatis T . Molecular Cloning : a laboratory manual 2nd ed. Cold Spring Harbor Laboratory Press, Cold Spring Harbor, New York USA (1989).
- Schoner B, Geistlich M, Rosteck P Jr, Rao RN, Seno E, Reynolds P, Cox K, Burgett S et Hershberger C. Sequence similarity between macrolide-resistance déterminants and ATP-binding transport proteins. Gene. (1992). 115 (1-2), 93-96.
- Sezonov G, Hagege J, Pernodet JL, Friedmann A and Guerineau M. Characterization of pra, a gene for replication control in pSAM2, the integrating element of Streptomyces ambofaciens. Mol Microbiol. (1995). 17(3): 533-44.
- Sezonov G., Blanc V., Bamas-Jacques N., Friedmann A., Pernodet J.L. and M. Guerineau, Complete conversion of antibiotic precursor to pristinamycin IIA by over expression of Streptomyces pristinaespiralis biosynthetic genes, Sature Biotechnology. (1997) 15 : 349-353.
- Sezonov G, Possoz C, Friedmann A, Pernodet JL, Guerineau M. KorSA from the Streptomyces integrative element pSAM2 is a central transcriptional repressor: target genes and binding sites. JBacteriol. (2000). 182(5):1243-50.
- Sinon R., Priefer U and Pühler. A broad host range mobilisation system for in vivo genetic engineering transposon mutagenesis in gram négative bacteria. Bio/Technology (1983).1: 784-791.
- Simon, et al., Plasmid vectors for the genetic analysis and manipulation of rhizobia and other gram-negative bacteria, p. 640-659. In A. Weissbach, and H. Weissbach (eds.), Methods in enzymology, vol 118, Academic Press, Inc., Orlando, 1986
- Summers,R.G., Donadio,Streptomyces, Staver,M.J., Wendt-Pienkowski,E., Hutchinson,C.R. et Katz,L. Sequencing and mutagenesis of genes from the erythromycin biosynthetic gene cluster of Saccharopolyspora erythraea that are involved in L-mycarose and D-desosamine production. Microbiology (1997). 143 (Pt 10) : 3251-3262.
- Van Mellaert L, Mei L, Lammertyn E, Schacht S, Anne J. Site-specific intégration of bacteriophage VWB genome into Streptomyces venezuelae and construction of a VWB-based integrative vector. Microbiology. (1998). 144 (Pt 12): 3351-8.
- Vara J, Lewandowska-Skarbek M, Wang YG, Donadio S, Hutchinson CR.Cloning of genes governing the deoxysugar portion of the erythromycin biosynthesis pathway in Saccharopolyspora erythraea (Streptomyces erythreus). J Bacteriol. (1989). 171(11): 5872-81.
- Vara J, Malpartida F, Hopwood DA, Jimenez A. Cloning and expression of a puromycin N-acetyl transferase gene from Streptomyces alboniger in Streptomyces lividans and Escherichia coli. Gene. (1985). 33(2): 197-206.
- Wahl, G. M., K. A. Lewis, J. C. Ruiz, B. Rothenberg, J. Zhao, and G. A. Evans. Cosmid vectors for rapid genomic walking, restriction mapping, and gene transfer. Ploc. Natl. Acad. Sci. USA (1987). 84: 2160-2164.
- Waldron,C., Matsushima,P., Rosteck,P.R., Broughton,M.C., Turner,J., Madduri,K., Crawford,K.P., Merlo,D.J. et Baltz,R.H. Cloning and analysis of the spinosad biosynthetic gene cluster of Saccharopolyspora spinosa(1) Chem. Biol. (2001) 8 (5) : 487-499.
- Walczak RJ, Hines JV, Strohl WR, Priestley ND.Bioconversion of the anthracycline analogue desacetyladriamycin by recombinant DoxA, a P450-monooxygenase from Streptomyces sp. strain C5. Org Lett. (2001). 3 (15):2277-9.
- Wang,Z.X., Li,S.M. et Heide,L. Identification of the coumermycin A(1) biosynthetic gene cluster of Streptomyces rishiriensis DSM 40489. Antimicrob. Agents Chemother. (2000) 44 (11), 3040-3048.
- Ward, J.M., Janssen, G.R., Kieser, T, Bibb, M.J., Buttner, M.J. & Bibb, M.J. Construction and characterization of a series of multi-copy promoter-probe plasmid vectors for Streptomyces using the aminoglycoside phosphotransferase from Tn5 as indicator. Mol Gen Genet (1986). 203 : 468-478.
- Wehmeier UF. New multifunctional Escherichia coli-Streptomyces shuttle vectors allowing blue-white screening on XGal plates. Gene. (1995). 165(1): 149-150.
- Wilms B, Hauck A, Reuss M, Syldatk C, Mattes R, Siemann M, Altenbuchner J. High-cell-density fermentation for production of L-N-carbamoylase using an expression system based on the Escherichia coli rhaBAD promoter. Biotechnol Bioeng. (2001). 73(2) :95-103.
- Worley K. C., Wiese B. A., and Smith R. F. BEAUTY: An enhanced BLAST-based search tool that integrates multiple biological information resources into sequence similarity search results. Genome Research (1995). 5: 173-184.
- Wu K, Chung L, Revill WP, Katz L et Reeves CD. The FK520 gene cluster of Streptomyces hygroscopicus var. ascomyceticus (ATCC 14891) contains genes for biosynthesis of unusual polyketide extender units. Gene. (2000). 251 (1): 81-90.
- Xue Y, Zhao L, Liu HW et Sherman DH. A gene cluster for macrolide antibiotic biosynthesis in Streptomyces venezuelae: architecture of metabolic diversity. Proc Natl Acad Sci U S A. (1998). 95 (21): 12111-12116.
- Yu D, Ellis HM, Lee EC, Jenkins NA, Copeland NG et Court DL. An efficient recombination system for chromosome engineering in Escherichia coli. Proc Natl Acad Sci U S A. (2000). 97(11): 5978-83

## Revendications

1. Polynucléotide codant un polypeptides impliqué dans la biosynthèse des spiramycines **caractérisé en ce que** la surexpression dudit polynucléotide permet une augmentation de la production des spiramycines et **en ce que** la séquence dudit polynucléotide est :
(a) l'une des séquences SEQ ID N° 111 ou 141, .
(b) un polynucléotide présentant au moins 80%, 85%, 90%, 95% ou 98% d'identité en nucléotides avec le polynucléotide selon (a).

2. Polynucléotide selon la revendication 1 **caractérisé en ce qu'**il est isolé à partir d'une bactérie du genre *Streptomyces.*

3. Polypeptide impliqué dans la biosynthèse des spiramycines **caractérisé en ce que** sa surexpression permet une augmentation de la production des spiramycines et **en ce que** la séquence dudit polypeptide comprend :
(a) l'une des séquences SEQ ID N° 112 ou 142,
(b) un polypeptide présentant au moins 80%, 85%, 90%, 95% ou 98% d'identité en acides aminés avec le polypeptide selon (a).

4. Polypeptide selon la revendication 3 **caractérisé en ce que** sa séquence comprend l'une des séquences SEQ ID N° 112 ou 142.

5. Polypeptide selon la revendication 3 **caractérisé en ce qu'**il est isolé à partir d'une bactérie du genre *Streptomyces.*

6. Vecteur d'expression **caractérisé en ce qu'**il comprend au moins une séquence d'acide nucléique codant un polypeptide selon la revendication 3, 4 ou 5.

7. Cellule hôte comprenant un vecteur d'expression approprié permettant l'expression d'un ou plusieurs polypeptides selon la revendication 3, 4 ou 5.

8. Cellule hôte selon la revendication 7 **caractérisée en ce qu'**elle est choisie parmi les cellules hôtes procaryotes ou les cellules hôtes eucaryotes.

9. Cellule hôte selon la revendication 8 **caractérisée en ce qu'**elle est choisie, parmi la bactérie *E. coli,* les cellules d'insectes, les cellules de levures, les cellules de mammifères.

10. Cellule hôte selon la revendication 7 dans laquelle a été introduit au moins un polynucléotide tel que défini à la revendication 1 ou 2, et/ou au moins un vecteur d'expression selon la revendication 6.

11. Procédé de production d'un polypeptide selon l'une des revendications 3 à 5, **caractérisé en ce que** ledit procédé comprend les étapes suivantes
a) insérer au moins un acide nucléique codant ledit polypeptide dans un vecteur approprié;
b) cultiver, dans un milieu de culture approprié, une cellule hôte préalablement transformée ou transfectée avec le vecteur de l'étape a) ;
c) récupérer le milieu de culture conditionné ou un extrait cellulaire;
d) séparer et purifier à partir dudit milieu de culture ou encore à partir de l'extrait cellulaire obtenu à l'étape c), ledit polypeptide ;
e) le cas échéant, caractériser le polypeptide recombinant produit.

12. Utilisation d'un polynucléotide selon l'une des revendications 1 et 2 pour améliorer la production en spiramycine d'un microorganisme.

13. Microorganismes mutant producteur de spiramycine **caractérisé en ce qu'**il surexprime au moins une séquence codante comprenant une séquence selon l'une des revendications 1 et 2, ou une séquence codante qui code un polypeptide selon l'une des revendications 3, 4 et 5, la surexpression de la séquence codante considérée étant obtenue en transformant un microorganisme producteur de spiramycine par un vecteur d'expression selon la revendication 6, permettant la surexpression de cette séquence codante.

14. Microorganisme mutant selon la revendication 13 **caractérisé en ce que** le microorganisme surexprime une ou plusieurs séquences codantes comprenant une des séquences polynucléotidiques telles que définies à la revendication 1.

15. Microorganisme mutant selon la revendication 13 ou 14 **caractérisé en ce que** ledit microorganisme est une bactérie du genre *Streptomyces.*

16. Microorganisme mutant selon la revendication 13, 14 ou 15, **caractérisé en ce que** ledit microorganisme est une souche de S. *ambofaciens.*

17. Procédé de production de spiramycine(s), **caractérisé en ce qu'**il comprend les étapes suivantes :
(a) cultiver, dans un milieu de culture approprié, un microorganisme selon l'une des revendications 13 à 16,
(b) récupérer le milieu de culture conditionné ou un extrait cellulaire,
(c) séparer et purifier à partir dudit milieu de culture ou encore à partir de l'extrait cellulaire obtenu à l'étape b), les spiramycines produites.

18. Souche de *Streptomyces ambofaciens* **caractérisée en ce qu'**il s'agit de la souche OSC2/pSPM75(2) déposée auprès de la Collection Nationale de Cultures de Microorganismes (CNCM) Institut Pasteur, 25, rue du Docteur Roux 75724 Paris Cedex 15, France, le 6 octobre 2003 sous le, numéro d'enregistrement 1-3101. " , "

19. Procédé de production de spiramycine(s), **caractérisé en ce qu'**il comprend les étapes suivantes :
(a) cultiver, dans un milieu de culture approprié, un microorganisme selon la revendication 18,
(b) récupérer le milieu de culture conditionné ou un extrait cellulaire,
(c) séparer et purifier à partir dudit milieu de culture ou encore à partir de l'extrait cellulaire obtenu à l'étape b), les spiramycines.

## Patentansprüche

1. Polynukleotid, codierend für ein Polypeptid, das an der Biosynthese von Spiramycinen beteiligt ist, **dadurch gekennzeichnet, dass** die Überexpression des Polynukleotids eine Erhöhung der Produktion von Spiramycinen gestattet, und dass die Sequenz des Polynukleotids wie folgt ist:
(a) eine der Sequenzen SEQ ID Nr. 111 oder 141,
(b) ein Polynukleotid, das mindestens 80%, 85%, 90%, 95% oder 98% Nukleotididentität mit dem Polynukleotid nach (a) aufweist.

2. Polynukleotid nach Anspruch 1, **dadurch gekennzeichnet, dass** es aus einem Bakterium der Gattung *Streptomyces* isoliert wird.

3. Polypeptid, das an der Biosynthese von Spiramycinen beteiligt ist, **dadurch gekennzeichnet, dass** seine Überexpression eine Erhöhung der Produktion von Spiramycinen gestattet, und dass die Sequenz des Polypeptids folgendes umfasst:
(a) eine der Sequenzen SEQ ID Nr. 112 oder 142,
(b) ein Polypeptid, das mindestens 80%, 85%, 90%, 95% oder 98% Aminosäureidentität mit dem Polypeptid nach (a) aufweist.

4. Polypeptid nach Anspruch 3, **dadurch gekennzeichnet, dass** seine Sequenz eine der Sequenzen SEQ ID Nr. 112 oder 142 umfasst.

5. Polypeptid nach Anspruch 3, **dadurch gekennzeichnet, dass** es aus einem Bakterium der Gattung *Streptomyces* isoliert wird.

6. Expressionsvektor, **dadurch gekennzeichnet, dass** er mindestens eine Nukleinsäuresequenz umfasst, die für ein Polypeptid nach Anspruch 3, 4, oder 5 codiert.

7. Wirtszelle, umfassend einen geeigneten Expressionsvektor, der die Expression von einem oder mehreren Polypeptiden nach Anspruch 3, 4, oder 5 gestattet.

8. Wirtszelle nach Anspruch 7, **dadurch gekennzeichnet, dass** sie aus prokaryotischen Wirtszellen oder eukaryotischen Wirtszellen ausgewählt wird.

9. Wirtszelle nach Anspruch 8, **dadurch gekennzeichnet, dass** sie aus dem Bakterium *E. coli*, Insektenzellen, Hefezellen, Säugerzellen ausgewählt wird.

10. Wirtszelle nach Anspruch 7, in die mindestens ein Polynukleotid nach Anspruch 1 oder 2 und/oder mindestens ein Expressionsvektor nach Anspruch 6 eingebracht wird.

11. Verfahren zur Herstellung eines Polypeptids nach den Ansprüchen 3 bis 5, **dadurch gekennzeichnet, dass** das Verfahren die folgenden Schritte umfasst:
a) Inserieren mindestens einer für das Polypeptid codierenden Nukleinsäure in einen geeigneten Vektor,
b) Züchten einer zuvor mit dem Vektor von Schritt a) transformierten oder transfizierten Wirtszelle in einem geeigneten Kulturmedium,
c) Gewinnen des konditionierten Kulturmediums oder eines Zellextrakts,
d) Abtrennen und Reinigen des Polypeptids aus dem Kulturmedium oder aus dem Zellextrakt, das/der in Schritt c) erhalten wurde,
e) gegebenenfalls Charakterisieren des hergestellten rekombinanten Polypeptids.

12. Verwendung eines Polynukleotids nach einem der Ansprüche 1 und 2 zur Verbesserung der Spiramycinproduktion eines Mikroorganismus.

13. Mutierter, Spiramycin produzierender Mikroorganismus, **dadurch gekennzeichnet, dass** er mindestens eine codierende Sequenz, die eine Sequenz nach einem der Ansprüche 1 und 2 umfasst, oder eine Sequenz, die für ein Polypeptid nach einem der Ansprüche 3, 4 und 5 codiert, überexprimiert, wobei die Überexpression der betreffenden codierenden Sequenz durch Transformation eines Spiramycin produzierenden Mikroorganismus mit einem Expressionsvektor nach Anspruch 6 erhalten wird, der die Überexpression dieser codierenden Sequenz gestattet.

14. Mutierter Mikroorganismus nach Anspruch 13, **dadurch gekennzeichnet, dass** der Mikroorganismus eine oder mehrere codierende Sequenzen überexprimiert, die eine der Polynukleotidsequenzen nach Anspruch 1 umfassen.

15. Mutierter Mikroorganismus nach Anspruch 13 oder 14, **dadurch gekennzeichnet, dass** es sich bei dem Mikroorganismus um ein Bakterium der Gattung *Streptomyces* handelt.

16. Mutierter Mikroorganismus nach Anspruch 13, 14 oder 15, **dadurch gekennzeichnet, dass** der Mikroorganismus ein Stamm von *S*. *ambofaciens* ist.

17. Verfahren zur Herstellung von Spiramycin(en), **dadurch gekennzeichnet, dass** es die folgenden Schritte umfasst:
(a) Züchten eines Mikroorganismus nach einem der Ansprüche 13 bis 16 in einem geeigneten Kulturmedium,
(b) Gewinnen des konditionierten Kulturmediums oder eines Zellextrakts,
(c) Abtrennen und Reinigen der produzierten Spiramycine aus dem Kulturmedium oder aus dem Zellextrakt, das/der in Schritt b) erhalten wurde.

18. Stamm von *Streptomyces ambofaciens*, **dadurch gekennzeichnet, dass** es sich um den Stamm OSC2/pSPM75(2) handelt, der bei der Collection Nationale de Cultures de Microorganismes (CNCM) Institut Pasteur, 25, rue du Docteur Roux 75724 Paris Cedex 15, Frankreich, am 6. Oktober 2003 unter der Registrierungsnummer 1-3101 hinterlegt wurde.

19. Verfahren zur Herstellung von Spiramycin(en), **dadurch gekennzeichnet, dass** es die folgenden Schritte umfasst:
(a) Züchten eines Mikroorganismus nach Anspruch 18 in einem geeigneten Kulturmedium,
(b) Gewinnen des konditionierten Kulturmediums oder eines Zellextrakts,
(c) Abtrennen und Reinigen der Spiramycine aus dem Kulturmedium oder aus dem Zellextrakt, das/der in Schritt b) erhalten wurde.

## Claims

1. Polynucleotide encoding a polypeptide involved in the biosynthesis of spiramycins, **characterized in that** the overexpression of said polynucleotide makes it possible to increase the production of spiramycins and **in that** the sequence of said polynucleotide is:
(a) one of the sequences SEQ ID NO 111 or 141,
(b) a polynucleotide exhibiting at least 80%, 85%, 90%, 95% or 98% nucleotide identity with the polynucleotide according to (a).

2. Polynucleotide according to Claim 1, **characterized in that** it is isolated from a bacterium of the *Streptomyces* genus.

3. Polypeptide involved in the biosynthesis of spiramycins, **characterized in that** its overexpression makes it possible to increase the production of spiramycins and **in that** the sequence of said polypeptide comprises:
(a) one of the sequences SEQ ID NO 112 or 142,
(b) a polypeptide exhibiting at least 80%, 85%, 90%, 95% or 98% amino acid identity with the polypeptide according to (a).

4. Polypeptide according to Claim 3, **characterized in that** its sequence comprises one of the sequences SEQ ID NO 112 or 142.

5. Polypeptide according to Claim 3, **characterized in that** it is isolated from a bacterium of the *Streptomyces* genus.

6. Expression vector **characterized in that** it comprises at least one nucleic acid sequence encoding a polypeptide according to Claim 3, 4 or 5.

7. Host cell comprising an appropriate expression vector which allows the expression of one or more polypeptides according to Claim 3, 4 or 5.

8. Host cell according to Claim 7, **characterized in that** it is chosen from prokaryotic host cells or eukaryotic host cells.

9. Host cell according to Claim 8, **characterized in that** it is chosen from the bacterium *E*. *coli*, insect cells, yeast cells and mammalian cells.

10. Host cell according to Claim 7, into which has been introduced at least one polynucleotide as defined in Claim 1 or 2, and/or at least one expression vector according to Claim 6.

11. Method for producing a polypeptide according to one of Claims 3 to 5, **characterized in that** said method comprises the following steps:
a) inserting at least one nucleic acid encoding said polypeptide into an appropriate vector;
b) culturing, in an appropriate culture medium, a host cell transformed or transfected beforehand with the vector of step a);
c) recovering the conditioned culture medium or a cell extract;
d) separating and purifying said polypeptide from said culture medium or else from the cell extract obtained in step c);
e) where appropriate, characterizing the recombinant polypeptide produced.

12. Use of a polynucleotide according to either of Claims 1 and 2, for improving the spiramycin production of a microorganism.

13. Spiramycin-producing mutant microorganism **characterized in that** it overexpresses at least one coding sequence comprising a sequence according to either of Claims 1 and 2, or a coding sequence which encodes a polypeptide according to one of Claims 3, 4 and 5, the overexpression of the coding sequence under consideration being obtained by transforming a spiramycin-producing microorganism with an expression vector according to Claim 6, allowing the overexpression of this coding sequence.

14. Mutant microorganism according to Claim 13, **characterized in that** the microorganism overexpresses one or more coding sequences comprising one of the polynucleotide sequences as defined in Claim 1.

15. Mutant microorganism according to Claim 13 or 14, **characterized in that** said microorganism is a bacterium of the *Streptomyces* genus.

16. Mutant microorganism according to Claim 13, 14 or 15, **characterized in that** said microorganism is an *S*. *ambofaciens* strain.

17. Method for producing spiramycin(s), **characterized in that** it comprises the following steps:
(a) culturing, in an appropriate culture medium, a microorganism according to one of Claims 13 to 16,
(b) recovering the conditioned culture medium or a cell extract,
(c) separating and purifying the produced spiramycins from said culture medium or else from the cell extract obtained in step b).

18. *Streptomyces ambofaciens* strain **characterized in that** it is the OSC2/pSPM75(2) strain deposited with the Collection Nationale de Cultures de Microorganismes (CNCM) [national collection of microorganism cultures], Institut Pasteur, 25, rue du Docteur Roux 75724 Paris Cedex 15, France, on 6 October 2003 under registration number 1-3101.

19. Method for producing spiramycin(s), **characterized in that** it comprises the following steps:
(a) culturing, in an appropriate culture medium, a microorganism according to Claim 18,
(b) recovering the conditioned culture medium or a cell extract,
(c) separating and purifying the spiramycins from said culture medium or else from the cell extract obtained in step b).


### SEQUENCE LISTING

<110> Aventis Pharma SA CNRS
<120> POLYPEPTIDES IMPLIQUES DANS LA BIOSYNTHESE DES SPIRAMYCINES, SEQUENCES NUCLEOTIDIQUES CODANT CES POLYPEPTIDES ET LEURS APPLICATIONS
<130> FRAV2002/0028
<150> FR 0212489
   <151> 2002-10-08
<150> FR 0302439
   <151> 2003-02-27
<150> US 60/493,490
   <151> 2003-08-07
<160> 161
<170> PatentIn version 3.2
<210> 1
   <211> 30943
   <212> DNA
   <213> Streptomyces ambofaciens
<400> 1
   gaattcggca attcccgggg cgccgggaaa ccggcacgcc attccgacca cggcaacggc 60
   gtcggaccgg tcgtcactgc gggaaatcgc gagttctcca gacacctgca tccctcatat 120
   gttcgccgta ccacgccgtg gcttttctgc cttttcttga tcttcccgag cgtacagcgg 180
   gcgaactgcc gggcgacagg aacggccggt gaacgacaat caattgtgtc aagttcggag 240
   attgtccacc gattctcgtc accggagacg gcgcgggatc atgcgggacc acacggcatc 300
   gcaccaggtc cgcagggtcg gcgctccgac gtcccggccg gtcgcgcact ccggtgacct 360
   gcacgcggag tcctgggcga gcggagaaga tttcagtacc tcgcggcgcg ggacaaccac 420
   ttcgcgacga atatgtcagg tctccccgag cggtccgtgc cgccccgccc tgacccgtcc 480
   gcgcccgtcc cgctccgccc cggtcgtcat ctcggccgga atttcagtcg gcagctcatt 540
   gtgtcaggtt cgccttgccg acattctccg gagattccta agctctgccg gtaaccggga 600
   ccggaaccac cgtgccgcgc gttcggtcca cacaccgctt ttcgaggagt ccgactgatg 660
   ggtgaggccg tgacgggacc gatggagctg agcaaggacg cggacgcccg ggggctgctt 720
   gagtggttcg cgtacaacag gacgcgtcat ccggtgttct gggacgagac ccgacaggcg 780
   tggcaggtct tcggctacga cgactacgtg acggtgtcga acaacccgca gttcttctcc 840
   tcggacttca acatggtgat gccgacgccg cccgaactgg agatgatcat cggtccgggc 900
   acgatcggcg cgctggaccc gcccgcgcac ggaccgatgc gcaagctggt gagccaggcg 960
   ttcacccccc gacggatcgc ccggctggag cccagggtgc gcgcgatcac cgaggagctc 1020
   ctggacaagg tggggcagca ggacgtcgtc gacgccgtgg gtgacctgtc ctacgcgctg 1080
   ccggtcatcg tgatcgccga actgctgggc atacccgccg gcgaccgtga cctgttccgg 1140
   gagtgggtcg acaccctgct gacgaacgag ggcctggagt acccgaacct cccggacaac 1200
   ttcaccgaga cgatcgcgcc cgcgctcaag gagatgaccg actacctcct gaagcagatc 1260
   cacgccaagc gggacgcgcc cgccgacgac ctggtcagcg ggctggtcca ggcggagcag 1320
   gacggccgcc ggctgaccga cgtcgagatc gtcaacatcg tcgcgctgct cctgacggcg 1380
   gggcacgtct cctccagcac cctgctcagc aacctgttcc tggtcctgga ggagaacccg 1440
   caggcgctgg aggacctgcg ggccgatcgc tccctggtgc ccggcgcgat cgaggagacg 1500
   ctgcgctacc gcagcccctt caacaacatc ttccggttcg tcaaggagga caccaccgtc 1560
   ctcggtccgc tcatggagaa gggccagatg gtgatcgcct ggagccagtc cgccaaccgg 1620
   gacccccggc acttcccgga cccggacacc ttcgacatcc gccgctcgga cggcacccgg 1680
   cacatggcct tcgggcacgg catccaccac tgcctgggtg ccgccctcgc ccgcctggag 1740
   ggcaaggtca tgctcgaact cctcctggac cgggtccaag gcttccgcat cgaccacgag 1800
   cacaccgtgt tctacgaggc cgaccagctc actccgaagt acctgcccgt ccgggtcgac 1860
   tggaactgaa cccgagggtc tcgtcccgga gtccagggcc gtcccgagcc ggccctggac 1920
   ctcacgaccg cccgataagg agcgccgcca tcgccgagaa cacagccgag ctccctgccc 1980
   ggcgggtcgg caggatcaag ccgtgccggc tgatcaggct cgagcagcac atcgacccgc 2040
   gcggcagcct ctccgtgatc gagtccggcg tgaccgtgga cttccccgtc cgacgcgtct 2100
   actacatgca tggccagacc cagtcctctc ccccgcgcgg cctgcacgcg caccgcaccc 2160
   tggaacaact cgtcatcgcc gtccacggcg ccttctccat caccctcgac gacggcttcc 2220
   agcacgccac ctaccgtctg gacgaacccg gagccggact ctgcatcggc cccatggtct 2280
   ggcgcgtcct gaaggacttc gaccccgaca ccgtggccct ggtcctcgcc tcgcagcact 2340
   acgaggagtc cgactactac cgcgactacg acaccttcct gcatgacgca cggagcctca 2400
   catgaccatc cccttcctcg acgcgggcgc cggctaccgg gagttgcgag ccgagatcga 2460
   cgcggccctg cagcgggtgt ccgcctccgg ccgctatctg ctcgacgcgg aactcgcggc 2520
   cttcgaggag gagttcgccg cgtactgcga caacgaccac tgtgtggcgg tgggcagtgg 2580
   ctgcgacgcg ctggagctgt ccctgcgggc gctggacatc ggtcccgggg acgaggtggt 2640
   ggtgcccgcg cacaccttca tcgggacctg gctggccgtg tccgctaccg gggcacggcc 2700
   ggtggccgtc gacccgacgc cggacgggct ctccctcgac ccggcgctgg tggaggcggc 2760
   gctcacccct cggaccagag ccctgatgcc ggtgcacctg cacgggcacc cggccgacct 2820
   cgacccgcta ctggcgatcg ccggacggca cggcctggcc gtggtcgagg acgccgcgca 2880
   ggcccacggc gcccgttacc ggggccgcag gatcggctcg ggccacgtgg tcgcgttcag 2940
   cttctacccc ggcaagaacc tcggcgccat gggggacggc ggcgcggtgg tcacgggtga 3000
   ctccggtgtg gccgagcgga tccggttgct gcgcaactgc ggctcgcggg agaagtaccg 3060
   gcacgaggtg cgctcgaccc actcccggct cgacgagttc caggcggccg tgctgcgggc 3120
   caaactgccg cggctcgacg cgtggaacgc ccgccgggcc ggcacggccg aacggtacgg 3180
   gcgggccctg ggtccggtac cgcagatcgc cgtcccggtc accgctccct gggccgaccc 3240
   ggtgtggcac ctgtacgtga tccgctgcgc ggagcgcgac gagctgcgcc gccggctgga 3300
   acgagccggg gtccagaccc tgatccacta ccccgtgccc ccgcaccggt ccccggccta 3360
   cgccgacgac ccggccggcg caccggcggg gacccacccg ctcagtgagc gcctggcggc 3420
   gcagagcctc agccttcccc tgggaccgca cctcggggag gacgaggccc gcgccgtcgt 3480
   ggcggcggtc cgggcggcgt ccgcagggct ggcggcgtac ccgacgccgg acggccagcg 3540
   ttttcctcta gtgacggaga aacgatgacc gaggtcatgt cagggcgtcc cggaatgaaa 3600
   gggatcatcc tcgcaggcgg cggagggacc cgcctacgcc ccttgaccgg cacgctgtcc 3660
   aagcaactgc tgcccgtcta cgacaagccg atgatctact acccgctgtc cgtcctgatg 3720
   ctgggcggca tccgcgagat cctcgtcgtc tcctccaccc agcacatcga gctgttccag 3780
   cggctgctgg gcgacggctc ccgcctcggc ctcgacatca cctacgccga acaggccgag 3840
   cccgagggca tagcgcaggc catcaccatc ggcaccgacc acatcggcga ctcaccggtc 3900
   gcgctcatcc tgggcgacaa catcttccac ggccccggct tctcggccgt gctccagggc 3960
   agcatccgcc acctcgacgg ctgtgtgctg ttcggctacc cggtcagcga cccgaagcgc 4020
   tacggcgtcg gcgagatcga cgaccagggc gtactgctgt ccctggagga gaaaccggcc 4080
   cggccccgct ccaacctcgc cgtcaccggc ctctacctct acgacaacga cgtggtcgac 4140
   atcgccaaga acatccggcc ctcggcgcgc ggcgaactcg agatcacgga cgtcaacagg 4200
   acctacctgg agcagaaacg cgcccggctc atcgaactgg gccacggctt cgcctggctc 4260
   gacatgggca cccacgactc cctcctccag ggcggccagt acgtccagct catcgagcag 4320
   cgccagggag tgcggatcgc ctgcatcgag gagatcgccc tgcgcatggg cttcatcgac 4380
   gccgacaccc tccaccggct cggccgcgaa ctgggcacct ccggatacgg cgcgtacctg 4440
   atggaggtgg ccacccgtgc aggcaccgaa tgagacgccg cgccggcccg cccgctccgc 4500
   cggccgacgg ccgccggccc ggatcctcgt caccgggggc gccggcttca tcggctcgcg 4560
   cttcgtgaac gcgctgctgg acggctccct gccggagttc ggcaaacccg aggtgagggt 4620
   gctcgacgcg ctcacctacg cgggcaacct ggccaatctg gccccggtgg gcgactgtcc 4680
   ccggctgcgg atcttcccgg gggacatccg cgaccgcggc gcggtcaccc aggcgatggc 4740
   gggggtcgac ctggtggtgc acttcgcggc cgagtcgcac gtggaccgct cgatcgacga 4800
   cgccgacgcc ttcgtgcgca ccaacgtgct gggcacccag gtcctcctcc aggaggcact 4860
   ggccgtacgc cccgggctgt tcgtgcacgt ctcgacggac gaggtgtacg gctccatcga 4920
   ggaggggtcc tggcccgagg agcacccgct gaaccccaac tcgccctacg ccgcctcgaa 4980
   ggcgtcctcc gacctgctgg cgctggccca ccaccgcacg cacggactgc cggtgtgcgt 5040
   cacccgctgc tccaacaact acgggcccta ccagtacccg gagaagatca tcccgctgtt 5100
   caccagcagc ctcctcgacg gcgggaccgt cccgctctac ggggacggcg gcaaccggcg 5160
   cgactggctg cacgtggacg accactgccg gggcatcgcc ctggtggccc ggggcggccg 5220
   gcccggcgag gtctacaaca tcggcggcgg caccgagctg agcaacgtcg agctcacgga 5280
   gcgtctgctg aaactgtgcg gagccgactg gtcggcggtg cggcgggtgc ccgaccgcaa 5340
   gggccacgac cggcgctact ccgtcgacta caccaagatc gcggacgagc tgggttacgc 5400
   gccgcggatc accatcgacg aagggctgga gcggaccgtg cactggtacc gggagaaccg 5460
   cgcgtggtgg gcgcccgcga agagggggcg atgacggtga cgaccgcatc cgtggacccg 5520
   ctcgacctgt ggctccgccg gtaccagccg tccgcgtcac ccgccgtccg gctggtgtgc 5580
   ttcccgcacg cgggcggctc ggcgagttcg ttcctgccgt tcacccggca gctgccggac 5640
   cggatcgagg tcgtggccgt ccagtacccc gggcgccagg accgcaggag cgaaccgctg 5700
   gtcgacacca tcgagggact ggccgagccc ctggccggcc tgctggaggc gcaggccggc 5760
   cccccggtgg tgctgttcgg gcacagcatg ggcgcgctgg tggcctacga ggtcgcccgc 5820
   gcgctccagc ggcggggagc ggctccggtg cgcctggtgg tctccgggcg ccgggccccc 5880
   gccgtcgacc ggccgatgac cgtgcacctc tacgacgacg accggctggt cgaggaactc 5940
   cgcaagctcg acggcaccga cagccaggtg ttcgccgatc cggagctgct ccggctggtg 6000
   ctgcccgtga tccgcaacga ctaccgggcc gtggcggcct acgcccaccg cccgggggcg 6060
   ccgctggact gccccctcac cgtgttcacc ggcgccgacg accccaccgt gaccgcggcc 6120
   gaggcggcgg cctggcacga ggcggcggcg tccgacgtcg agacgcgcac cttccccggt 6180
   ggccacttct tcccgtacca gcggaccgcg gaggtgtgcg gggccctggt cgacacgctc 6240
   gagccgctgc tgtcggccgg gacgcgcggt gtccggcggg tccgcccggg gtgacgtggg 6300
   cacggtcgag tacgccgtcc accggcgtac cgcggaacgg gtgagggtct ccgccgacac 6360
   cctggacagc ccggtcaccg cgctggcgga ggtgccccgc tggctggagg aataccaccg 6420
   ggcgcaccgc ttccacgtcg agccgatccc cttcgaccgg ctccggcggt ggtccttcga 6480
   gccgggcacc ggcgacctgc ggcacgagac gggccgcttc ttctccgtgg aggggctgcg 6540
   caccagctcg gacgccgatc cggtcgcccg tgtccagccg atcatcgtgc agcccgaggt 6600
   ggggctgctc ggcatcctgg cccgggagtt cgacggggtg ctgcacttcc tgatgcaggc 6660
   caaacccgag cccggcaacg tcaacgggct gcagatctcc cccacggtgc aggccacgcg 6720
   cagcaacttc gacgaggtgc accacggccg gtccaccccg ttcctcgacc acttcatcca 6780
   ccgccccggc cgccgggtcc tgatcgacag catccagtcc gaacagggcg actggttcct 6840
   gcacaagcgc aaccgcaaca tggtcgtcga gatcgacacc gacatcgagg ccgacgccgc 6900
   gttccgctgg ctgaccctcg ggcagatccg ccggctgatg ctccaggacg acctcgtcaa 6960
   catggacacc cgcagtgtgc tggcctgtct gcccaccgcg cacggcacgc ccgacgacgg 7020
   tgacgactcc ttcccggcgg cgctgcgccg ctccctctac ggggagaccg cgccgttgca 7080
   cgatctgcac gccatcacca gctgcctcac cgacgtccgg gcgctgcggg tgctgcgcca 7140
   gcagagcgtg ccgctcgacg acgcccggcg ggacggctgg gagcggaccg ggagcgcgat 7200
   ccggcatcgc agcggcaggc atttcgagat catggcggtg gaggtgaccg cggagcgccg 7260
   tgaagtggcc tcgtggaccc agccgttgct gcgcccgtgc tcgcagggac tggcggccct 7320
   gatcacccgg cggatcaacg gggtgctgca cgccctggtg gcggcgcggt cggaggtcgg 7380
   cacgctcaac gtcgccgagt tcggaccgac cgtccagtgc cggcccgacg aggcggacgg 7440
   ccagtcgccc ccgtacctgg accgggtgct gacggccgga gccgaccgcg tccgctacga 7500
   cgtggtgcag tcggaggagg gcgggcgctt ctaccacgcg cgcaaccgct atctggtggt 7560
   cgaggcgggg ccggagctcg acacgggctg cccgcccggc ttctgctggg ctaccttcgg 7620
   ccagctcacc gaactgctcg cgcacggcaa ctatctcaac gtcgaactcc gcaccctcat 7680
   ggcgtgcgca cacgcctcct actgaatggt cacgaaagct gcaccgcgcg ggagaatcgg 7740
   cagcgcgcca ccggccggcc ggcacccgga aggtaaagcg ccgttctccc gcatcggcgc 7800
   cctgcgggaa acggcggaac ggccggcccg gaccgcgcgc aattcccggc gggacacggt 7860
   gggagcccgc acgaggaacc gctttccccg ccttcggtgc gcccggccgc gggaccaccc 7920
   ccgcctcccg gccgggccgc ggaatacgac gggggcggcc gaggacattc ctttcccgcc 7980
   tccggaaaag cgcgccccga gggcccccga atgccgggcg ggacggacgg cgactgcgcg 8040
   cggacggcgg cccggcgtcg aacgcacctg cccgagtccg gacgagacag cgcgacgcga 8100
   gaggcgaaaa tgatcaatct cttccagccc cagatggggg ccgaggaact ggcggcggtg 8160
   tccgaggtct tcgacgacca atggctcggt cacggacccc ggaccgcggc gttcgagtcc 8220
   gcgttcgccg agcacctcgg ggtcggcccc gagcacgtcg tcttcctcaa ctcgggcacc 8280
   gccggcctct tcctggccct ggagtcgctc ggcctgcggc ccggcgacga ggtcgtgctc 8340
   ccctcgccca gcttcctcgc cgcggcgaac gccgtacagc tctcgggagc gcgcccggtg 8400
   ttctgcgaca ccgacccgcg gacgctgaac cccgccctgg agcacatcga ggcggccgtc 8460
   accccgcgca ccagggccgt catcgcgctc cactacggcg gccaccccgg cgacatcgtg 8520
   cgcatcgccg agcgctgccg ggagcggggc atcaccctga tcgaggacgc cgcgtgctcc 8580
   gtggcctccc gcgtcgacgg ccgaccggtc ggcaccttcg gcgacctcgc catgtggagc 8640
   ttcgacgcca tgaaggtcct ggtcaccggc gacggaggga tgatctacgt caaggacccc 8700
   ggggcggccg cccggatccg gcgcctcgcc taccacggcc tcacgcggtc cagcggcctg 8760
   ggatacgcca gggtctcggc gcgctggtgg gagatggacg tccccgaacc gggccgccgc 8820
   gtcatcggga acgacctcac cgcggccatc ggcgcggtcc agttgcgccg gcttcccggc 8880
   ttcgtggccc gccgcaggga gatcgtcgcc ctgtacgaca gcgaactgag ctcgctggag 8940
   ggcgtgctga caccgcccgc gccacccgcg gggcacgagt ccacgcacta cttctactgg 9000
   atccagctgg cccccggcgt ccgggaccgg gtggcacgcg acctgctcac cgacggcatc 9060
   tacaccacct tccgctacgc acctctgcac aaggtgcccg cctacggcca caccggaggc 9120
   gaactgcccg gcgtggagcg ggcgtccgaa cggaccctgt gcctgcccct gcaccccggc 9180
   ctgtcggacg ccgacgtccg caccgtcgtg tcctccctgc gcagagccct gagcgccgcg 9240
   gatccggccc ccgcctgacg cgcgcacgcg ccacggcccc tgtcccggcg gtgaccgccg 9300
   ggacaggggc cgtggcgcgt cctgcggacg gtccgggcca ccccgccgtc ccgtcggtgc 9360
   gtcagcgacg cgtgccgagg aagaagcccg gtgagccctc gcccgtcacg acgtactcga 9420
   cgtccaggcc ggccttgcgg tacgcggcct cgtactccgc acgcgagaac agcgtgaggt 9480
   agtccacctc gctgcggtgc cggatgccct ccgcggtgtg cgcgatcagg tagtggatct 9540
   ccatccgggt ccgcccgccc tcccgggtcg agtgggagac acgggcgacg ccctggtcgc 9600
   ccggtgccgt ccgcagggcg tgggtggaga cgtggccgtc caggaaggtg tcggggaagt 9660
   accacggttc gacggccagg acaccgtccg cggtcaggtg ccgcgccatg gcggcgacgg 9720
   cgtcctccag gtcggccgtg gtctccaggt acccgatcga gctgaacatg cagaccacgg 9780
   cgtcgaacgt ctcgccgagg tcgaacgccc gcatgtcacc gcggtggagg gtgacgccgg 9840
   ggagccgctc ctcggcgcgg gccgccatcc actcggacag ctccaggccg ctcacgcggt 9900
   cgtagagctt ggcgaaggcc tccaggtggg taccggtgcc gcacgcgatg tcgagcaggc 9960
   tggccgcgtc cggcgtacgt tcccggatca ggtcggtgac ccgggcggcc tcacccgcgt 10020
   agtccttgcg gtcctggtag agcaggtcgt agacctcggc ggcactgtcg ttctcgtaca 10080
   tgggaatcct ccgggccgtg aagggggaac cgtgggtctg tcgaagagga gtgcgccgtg 10140
   cgctcggggc gggggcggcc tgccgccggc ccctcgcgat gatctccgta cggacaccca 10200
   acagttcacg ccgagccggg gtcaaggaac gacggggtgg tcagtcaagt cgggcgctcc 10260
   gcccccgggg cggggcgcgc cggcgacgcg cacggattcg gccaaccggt tgctcgttcc 10320
   gccggaaatc acggtgtggc cccgggccca ccgggtagct tatgcctcgt tcaccgcagc 10380
   ggttgaagag gcagccttca accccggccc ggcctttatg gaattcattt ccaccgtgcc 10440
   gcaacaccca ctgaaggacg gccggatatc ggccatgaag ccccggcctt tcagccaggc 10500
   accctctctt gtcgaataga gtatgtcctc cgctgaagcc gccgaagacg gacgaagggg 10560
   acgaacggtc acctcggtcg atctagacgg aatccttgaa agcgtaatag cctgtcaatg 10620
   ctttggtaaa gcacagggat gggggtgcct gcgggatgag tgacctgggt tctggtgaag 10680
   aagggtccga agaagacgag tcggacgacg cactcgcctt cctcgagttc atcgcccggt 10740
   cggcaccacg gagcgaatac gaccggctca tggcccgcgc cgaacgctcg ggcgccgacg 10800
   aggaccggat gcgccgactg gagcgcttca accggctcgc cctcaccgcg cagtcgatga 10860
   tcgagtaccg ccgcgaccgg gaggcggagc tcgcggccct ggtcgacgcc gcgcacgagt 10920
   tcgtcgccgc ccggcggggc aaggacctgc tggagtccat cgcccgcaga gcacggctgc 10980
   tgctgaagct ggacgtctcc tacgtcggcc tgcacgagga ggaccggccc ggcacggtgg 11040
   tgctgagcgc cgacggcaac gcggtcaagg tcgccgagag ctaccggctg ccggccgacg 11100
   gcggactggg cgccatggtg cgcacctgcc gcgctccctt ctggaccccg gactacctcg 11160
   gggacaacag cttcacgcac gtcgaggccg tcgacgacat cgtccgcgcc gaaggcctgc 11220
   gcgcggtcct ggccgtcccg ctgtgcgccg gggcgaaccc gatgggggtc ctctacgtcg 11280
   ccgaccgtca ggtgcggcat ctgaccccca acgaggtcac cctgctgtgc tcgctcgccg 11340
   atctggccgc ggtggcgatc gagcgcaacc ggctggtcga ggagctccac gacaccatcg 11400
   ggcaactgcg ccaggacatc ggcgaggccc gcaccgccct cgcgcgcacc cgcaggtccg 11460
   ccgacctcca gtcgcacctg gtcacgcagg tgatggacag gcgcggcgcc gactcgttac 11520
   tcgcgacggc cgccgaggcg ctcggcggcg gagccggcct gtgcagcccg ctcgggcgcc 11580
   cgctcgccga gtacgggacc ctgcgccccg tcgcccccac ggaactgcgc gcggcgtgcc 11640
   gccgggccgc cgagaccggc cggcccacct ccgtggcccc gggggtctgg acggtgcccc 11700
   tgcttcccgg gggcaacgcc ggcttcctgc tgaccgacct cggtccggac gcggaccaca 11760
   ccgccgtccc cctgctcccg atggtcgccc gcaccctcgc gctgcacctg cgcgtccagc 11820
   acgacgactc ccccaaggcg cagagccacc aggagttctt cgacgacctg atcggggcgc 11880
   cccgctcacc cacgctcctc agggaacgcg ccctgatgtt ctccctcagc ttccgccgcc 11940
   cgcacgtggt gctggtggcg gacggacccc gcgggacctc gccgcggctg gaggcctccg 12000
   gcgccgacta cgcgaaggag ctcggcgggc tgtgcagcgt gcgggacggc gccgtcgtcc 12060
   tgctgctgcc cggcgacgac cccgtcgccg tggcgcagac cgccgccccg gagctgaccg 12120
   accgcgccgg gcaccccgtc accgtggggg tcgcgggccc cgcctcgacc gtcgacggca 12180
   tcgccgacgc gcaccgtgag gccgcgaagt gtctggagac cctccgcgcg ctcggcggcg 12240
   acggcggcac cgcgtgcgcc tccgacctgg gtttcctcgg catgctcctc gccgaggaga 12300
   acgacgtccc cggttacatc aggacgacga tcggccccgt ggtcgactac gacacccacc 12360
   gcttcacgga tctggttccc actctgaggg tgtacctgga gtcgggcagg agccccacgc 12420
   gtgccgcaga gacactgcgc gtgcacccga acaccgtctc acggcggctg gagcgcatcg 12480
   gcgtactgct gggagaggac tggcagtcac cggagcgggt gctggacata caactggccc 12540
   tgcggctcta tcaggtgcgc tcggcgctct cctcgcaacc ggcgtccgag acccgggccg 12600
   tgctcggatc gctgcgcgag tgacctctcc ggcacgggcg gcgccccgcc cgtcggcgcg 12660
   gatcgcgccg acaggcgggc gttgaccacc ggccaccggt cccccgtacg acgaggtccc 12720
   gggcccgcgt ggttcagccg gcgctgaact cggcgatgcg cccgtctccc atggtcagcc 12780
   gggcgccggt gaaccgggac cgcatccgac ggtcgtgggt gacgaccacg acggcgccgc 12840
   ggtagtccgc gagtgcctgc tccaactcct ccaccagcac cggggtgagg tggttggtgg 12900
   gctcgtccag cagcagcagg tccatcgggt cgctcaccag ccgggcgatc tcgatccggc 12960
   ggcgctgccc gtaggacaga tccttcacgc gtcgccgcag gtcggacggg ctgaacaggc 13020
   cgagcgacag cagtttctcc gcgtggtcct ccaggtagcc ctcccggccc tgggcgaagg 13080
   cccgcagcac ggtcagtccg ggcgcccagg gcgtctcgtc ctgccgcaga tgaccgaccc 13140
   ggcagccgac gcgcaccgag ccgccgtccg gctccagttc cccggacagc acccgcaaca 13200
   aggtggactt gcccgcaccg ttgggacctg tgacgagcag ccgttcgccg ggccggatcg 13260
   tcagggagtc cacggcgagc cgacccgcga cgcgcacgtc ggtgagttcg gccaccgcct 13320
   cctccgcctc cgggcccgcg gtgtcgatgc gggcggcgaa ggacaacggg tcggcgggag 13380
   cgtggaccgg gttctcggtc agctgcgcca cgcgttgctt cgcgttgcgg atccgcacca 13440
   tcgcgccgtg gtcgcgccct cgcctgcggt aggcgccgtg gccgaacacg gagagggaca 13500
   tcttgcgcgg gatgccgtcc atccgcgcca cgttggaggt gatcagcccg cggttgcggt 13560
   cgagttcggc acgccactcc tcgtactccc gcagccgccg ctcgcgttcc acggccttgg 13620
   ccgtcaggta gccctcgtag ccgttgccgt agcgggtgac gctgccggag tcgacctcca 13680
   ggatcgtggt ggtgagccgg tcgaggaaga cccggtcgtg ggtgaccgcg atcaccgtgc 13740
   cgcggtggcc ggccaggtgg tcctccagcc attccatcgc ccggtcgtcg aggtcgttgg 13800
   tcggttcgtc caggagcagc agctccggcg acgaggcgag ggtcgcggcg agcgcgaggc 13860
   gggagcgttc gccaccggag agggttccga gcttgcggtc gcggtccagg ctcggcagtc 13920
   cgaggccgtg cagcgcgacc tccacgcgca cgtcggcctc gtagccgcca cgcgcctggt 13980
   actgctcgac cagagcggcg tagcgctgga ggccggcgga cagctcgcgc tcggagccgt 14040
   tctcgtcgct ctcgcccagc tccgcctcgg cctcgcgcat cgccgcttcg agctcgcgca 14100
   ggtcggacag ggccaggtcg acggcgtcct ggacggtggc gtcgaggggc agttccagtg 14160
   tctgcgccag gtagccgacg ccgccgggag cgaccacggt gagcgcgccg ttgtcgggct 14220
   ccacgcggcc ggcgaggatc ttgagcagcg tggacttgcc ggaaccgttg tcgccgatca 14280
   cgccgacctt ctcgcccggc ttgatgctga aaccgacccg gtcgagcacg acacagtcgt 14340
   ggtagcgctt cgtgatgtcg tgtagggcgt attgcgcaat cgacacgcgt aagtctcctg 14400
   tttccacgat gaggatgagt ggatgcgtga gcgcgctcgc agaaagaacg gaaagcagaa 14460
   gggacgccac cactgcggac atggccggtc agggggtgtc acgagcacgc tgctggatgc 14520
   ggcaggcgga gtcagctcaa cgccgggcat cctcatctat cacagagatc ccatgcgaag 14580
   aactatagcc gtgctacccg gtgccgcgca acggtatgcg tgtcgcaccg gccgacgtga 14640
   tgcagtcgga ccgggatgat cgcttgtccg gcggccggat gcctagcctc gggagcaacc 14700
   acagcggtct ttcacgagag gggtcgacca tgggcgatct caggaaccgc atcaccgagc 14760
   tggtccgcgc gtaccaccgg gaacaggcgc ccgggggctt cgttcccggg acgacgcacg 14820
   taccggtctc cggcgcggtg ctgagcgagg aggaccggct ggcgctggtg gagacggcgc 14880
   tggagatgcg gatcgcggcc ggcccggcct cccggggctt cgagcggcag ttcgcccggt 14940
   acctcgggct ccggaaggcg cacctgacca actccggttc ctccgccaac ctcctcgccc 15000
   tcggcgcgct cacctcgccg cagctggagg agagacggct gcgtccgggg gacgaggtcg 15060
   tcacggtcgc cgccgggttc cccacgacgg tcaacccgat cttccacaac gggctggtgc 15120
   ccgtcttcgt ggacgtcgag ctcggcacgt acaacacgac gcccgagcgc atcgagcggg 15180
   ccatcggccc ccggaccagg gcgatcatga tcgcgcacgc cctgggcaac cccttcgagg 15240
   ccgaagaggt ggcccgcctc gcggacgagc gggagctgtt cctcatcgag gacaactgcg 15300
   acgcggtggg gtcccgctac cggggcaggc tcaccggctc cttcggcgac ctgtcgaccg 15360
   tcagcttcta tcccgcgcac cacatcgcga tgggtgaggg gggctgcgtg ctcaccgaca 15420
   acctggccct ggcgcggatc gtggaatcac tgcgcgactg ggggcgcgac tgctggtgcg 15480
   agccgggtga ggacaaccgc tgcctcaagc ggttcgacca gaagatgggt gacctgccgc 15540
   ccgggtacga ccacaagtac atcttctcgc acgtcggtta caacctgaag tcgaccgacc 15600
   tgcaggcggc cctcgggctg tcccagctga cccggatcga ggagttcacc gaggccaggc 15660
   gcgccaactg gcggcatctg cgcgccgcgt tggacgggct gcccggtctg ctgctgcctc 15720
   atgccacacc gggcagcgat ccgagctggt tcgggttcct catcaccgtg gacccggacg 15780
   ccgcgtacag cagggcggcc ctggtcgacc acctggaatc gcgccggatc agcacccgcc 15840
   gcctgttcgg gggcaacctc gtgcggcacc ccgcctacac cgaccgtcgg taccgggtgt 15900
   ccggctccct ggagaacagc gacctgatca ccgaccagac gttctggatc ggggtcttcc 15960
   ccggcatcac cccggagatg atcgcctacg tcggcgacac gatccgggag tgcgtgctca 16020
   agcactcctg aggggcggcg gtacgggggt gcgcgtggag gggaggaccg gggcggtcct 16080
   cctccggggc gtcacggccg cggaagcgtc cgaagctccg aggcgaggtc ggggtggacc 16140
   cgcgcggtga gcagggtgcc ctccggcgtg tgctcggtgc tgagcacctc gccctcgtcg 16200
   tgcacccgcg ccaccaggct cccctcgtcg taggggatca cgacctccac ctccacctcg 16260
   gggtgcggca gcaggcggtc gatcagttcc cgcagctcgt cgatgccccg acccgaacgg 16320
   gcggacacga cgatcgcgtc cggctcctgc tccagcagac gggcgaggac gtccgggtcc 16380
   gcgacatcgg ccttgttgac gaccacgacc tcggtggact cgccggcgcc cacgtcccgc 16440
   agcacctcgc gcaccgaggc cagctgcgcg ccggggtccg ggtgcgaacc gtcgaccacg 16500
   tgcagcacca gatgcgcgtc cgcgacctct tcgatcgtgg aacggaacgc ctccaccagg 16560
   tggtgcggga ggtggcgtac gaagcccacg gtgtcggcga tggtgtaggg gcgcccgctc 16620
   ggcgtcgtcg cccgccgcac ggtcgtgtcc agggtggcga acagggcgtt ctccaccagc 16680
   acgccggctc cggtgaggcg gttgagcagc gatgacttgc cggcgttggt gtagccggcg 16740
   agggcgaccg acagcacctt gttgcgccgt cgctcctccc ggttcacgtc ccggccggtc 16800
   ttcagctgct ccagctcccg gcggagcctg gccatcttgt cgttgatccg ccgccggtcc 16860
   gtctcgatct tcgtctcacc gggaccgcgc gtggccatgc caccgccgcc accgccgccc 16920
   atctgccggg acagcgactg gccccagccg cgcagccgcg gcagcatgta ctgcatctgc 16980
   gccagcgcca cctgcgcctt gccctcccgg gactgggcgt gctgcgcgaa gatgtccagg 17040
   atcagggccg tgcggtccac gaccttgacc ccgacgacct cctccaggtg catcagctga 17100
   ctggggctca gttccccgtc gcacaccacg gtgtcggcgc cggtctcctc gacgatgtcg 17160
   cgcagctgcg acgccttgcc cgagccgatg tacgtcgccg ggtccggctt ctgccggcgc 17220
   tgcacgacgc cgtccagcac gagggcgccc gcggtctcgg cgagcgccgc cagctcggcg 17280
   agcgaactgt cggcctccgc ggccgttccc gaggtccaga tgccgacgag caccacccgc 17340
   tcgagtcgca gctggcggta ctcgacctcg gagacgtcgg tcagttcggt ggagagcccc 17400
   gcgacgcggc ggagggaggc ccggtcctcg cggtctaact gatcgccgtc ccattcctcg 17460
   gcgtcggcgc cggccatcgt gtcgtccatc agtgcgtcgg ctcgctgcgt gccggcgaag 17520
   tcctcgggat gcgtcaaagt acttccaatc tggggggttc gagagcgttc cggggcgggg 17580
   cgcgtccggc ctgcggggcc gggcgcgggg ccggggggtc ggggccgacg tgtccgcacc 17640
   tcgccgtcgg gggccggtgc cgtggaggtc gctgccggaa ctcacatgcc gtccacccta 17700
   gccatgtcgc cggggtgtgc aaggacgtat cggcgatcac ctccccagct gacagagtgt 17760
   gcaccgggta ctgcgcccgc gggcgtgaag cgggcgagtg tggatgtcat gcgagtactc 17820
   attatcgggg gttcacagtt cgtgggccgg gccttcgccg ccgaggcact ggccgcgggg 17880
   caccgggtca ccacgttcaa ccggggtgtc agcggcaccg acctgcccgg cgtcgaggcg 17940
   gtcaggggcg accgcgaggt ggccggcgac ctggagcggc tggtgtccgg aaggcactgg 18000
   gacgcggtcg tggacacctg cggttacgtg ccccgcacgg tgggggcctc ggccgcggcg 18060
   ctgtccgggc acgcggacac ctacctctac gtctccagca tcgcctgcct gcccgactgg 18120
   gcgcaggcgg tccgtccggt ggacgacgac tcacctgccc acgactgccc gccggacgcc 18180
   ggaccggacc acgccgacgg tgactacggc gtcctgaagg ccggctgcga gcgcgccgtg 18240
   gaccggcact tcgcgggccg gaccctgcac ctgcgggccg gtgtcatcct cgggccgcac 18300
   gacaccatgc gcatgctcga cgcctggctg tggcgcatgc gcgtcgccga gggggagcac 18360
   cgccgggtgc tcgccccggg caaccccgag gtgggcatgc gcctgatcga cgtacgcgat 18420
   gtcgccgtct tcggcctcga ctgcctcgcg gacggccgta ccggcgcctt catcgtcaac 18480
   ccgccggaga agaacaccac cttcggggag ttgctcacgg agtgcgtcaa ggccaccggt 18540
   tcggccgcgg agccggtgtg ggtcgacgag gggttcctcg ccgagcacgg cgtgagtccg 18600
   tggacggacc tgccgatgtg ggtgcccgac accgcgcggg acaccctcgt gtgggcggcc 18660
   ggagcaccgc gcgcccgggc cgcgggtctg gcctgccggc ccttctccga caccgtgcgg 18720
   gacgcctggg aggtcgtccg ggaccggccc gtcccggaac tgccgctcgc ggccggctgc 18780
   ggcctgtccc tgagccggga gaaggagctg ctcgccgcct gggacgctcg cggcggtgcg 18840
   gcggcgggct gacgcggccc ccgaggagtg cgacgccgtt tcccgctcgc ggggagcggc 18900
   gctcgcgtgt ccgccccgcg gcccggcggc gcgtcctgtg tcccggcgcc cggggcgcgg 18960
   ctacacggtg aaggtctcgg cgcgggccac gatctcgtcg accagccccg cctggcgcag 19020
   cgcggcgtac gactcggcgg agacgtcctc ggcccggatc accgcacggc ggaagaccga 19080
   caggatgttc acgaagtgcc ggtccacggg caggacgcgc tcctcccggt ggtcctggcg 19140
   ggacagccgc agtacggggt ggtggctgtc cggggtcgtg aagacgtggc tcagggcgag 19200
   cgagccggtg ctgccgtgca gctcgtacgc cgagcggtag ccgtgttcca tgccgaaggc 19260
   gaggtgggcg gccacgccct ggggggcggc gagcaggacg ctgccggaga ccacgacgcc 19320
   ccggcggcgg tcacggcgca gcacggcgcc ggtcaggcgc agttcgggtc cgaggaagtg 19380
   cagcgcggcc cgcagcgggt agacgccgtt gtcgagcagt gcgccgccgc cgatgtcggg 19440
   tcggtagcgc atgtcgtcgt cggcgcgcgg cgggatggtg aaggcggcgg agaaggtgcg 19500
   cagctctccg atggcaccgg cctccaacag ggctttgacg gtggcgtgtt gagagtggtg 19560
   gaggaacatg aagttctcca tcagtaccag tccgcgctcc cgggccatgg cgaacaggcg 19620
   ggccgcgtcg gcgtggttgg cggcggcggg cttctccacg aggacgtgtt tgcccgcccg 19680
   cagcgccgcc gcggcccatt cggcgtgcag catgctgggc accgcgatgt agacggcgtc 19740
   cacgtcgggg cgttccagca gggcctcgta gggcgcgacc gcctcgcagt cgaagtgcct 19800
   gcccagggcc ttggcccggt ccgcgtcgcg gctgccgacg caggtcagca cggtgccggg 19860
   ggtggagagc agggcgggca gggtgcgacg gcccgcgatg tcgccgcagc cgatcgctcc 19920
   gaagcgcagc accggggacg cttccggccg cggcgggccg ggtgcggcga acggttcagc 19980
   agaagtaccc agggcggtca tggatggttc cgtcaatcgg tccggatggt cacggcacag 20040
   cgtggtgaac ggccggcgca cacccacgag tcgcttccgg caatgggcgg ccctgaagct 20100
   acccggcggc ccgacgcgac ggcaaggccg atctgacaca gtgccgcgcc gagggaccgt 20160
   cggtgccccg cgtacccgct gacctgacac tacgccgacc aatctgtgga cggggcggtg 20220
   gccgcccggg ttaggtgagc gcggcgcccg cggtgaccgc gacgccggtc cgaagcggcc 20280
   ctcggggcac gccgttggag agaggagacg cgggtgacgg acgcgatcac gaccgagctg 20340
   gccgaccgcg aactggggcg cagactgcac cggatacgcg gcgtccactg gtatttcggc 20400
   aaccacggtg acccgtacgc cctcatcctg cgcggtcaga ccgacgaccc gtcggtgtac 20460
   gaggagcggg tccgcgaggg cgggccgctg ttccgcagcc gtaccgggac ctgggtgacc 20520
   gcggacccgg aggtggccgc ggccgtgctg ggcgactcgc gcttcggtgc gctggaccgc 20580
   gccggacggc gcccggagga gtacctccag ccgtcgcccg ccacgtacct ggggctggac 20640
   cgcgccgcgt acgcgcgtct gcggcgggtg gccgagcccg tgctgggcgc ggacgccgcc 20700
   gccgcgtggc gccggctcgg cgaggacgtc gggcgccggc tgctcgccgg ccgcggttcc 20760
   ggcctcgacc tgacggcgga cttcgcccgc cggctgccgg cattggtcct ggccgcgtgg 20820
   ctcggggtgc cgggcgaacg gtgcgacgag tgggaggagt cgctgcgggc ggcggggccg 20880
   ctgctggacg gtctgctgtg tccgcagacg ctggcggcca cccgtgcggc ggactcggcc 20940
   gccgaggggc tgcgcgcgct gttggacgag gtggtcgccg cgcgtcccgg cgggtccggc 21000
   gagggtgcgg tggcccgcat ggtcggcgcc ggagccgccc ccgacgacgc ggtggccgcc 21060
   gccgtgtgcc tggcgctctc ggccgtcgaa ccgacgacga ccctggtgtg cgaagcggtc 21120
   cggctgctgc tcgaccgacc cgagtggtgg cggcggttgt gcgactcccc cgctctggcg 21180
   ccggccgcgg tccggcacac cctgcggcac gcgcccccgg tgcggctgga gagccgggtg 21240
   gcccacgagg acgtgacggt ggcggatcgt ccgctgcccg ccgggagcca cgtggtggtg 21300
   ctcgtgggcg cggcacggcg cgcgggcgcc ccggccgcgg agccggcgga cctggcgggc 21360
   gcaccggcgg cggagctgcc ggacgacctg tggttcgcgc tgtccgggga gttcgtcggc 21420
   cgtgccgccg agaccgcgct gggcgtgctg gccgaggccg ccccgggact gcggcgggac 21480
   ggcgacatcg tccggcggcg ccgttccccg gtcctcggca ggtacgcgcg gttccccgtc 21540
   gcgtactcct gacgggcccg cggccggcgt cccctcagtc ccccacgacg tttcatgaaa 21600
   ggagtgccgt gcgcgtcctg gtgacctcca tcccgcacca cacgcactac taccacctgg 21660
   taccgctgat ctgggctctg cgtgcctcgg ggcacgaggt ggtggcggcc ggccagccgt 21720
   cgctggtcga cgccatcacc gccagcggca tcccggcgtt cgccctggcc gaggaggagt 21780
   cgctggcgca gatcttcgag gaggtcgagg gcgatctcca gccgtatcag cacggcatcg 21840
   acgagttcga cttcttcggc accctgaagg acgagctgga ctgggagaag ctgctcgccc 21900
   agcaggtgat cctgtccggc ctgtggctgg aaccgctcaa cggcgccacg accctcgaca 21960
   gcatcgtcga cttcgcccgg gcctggaagc ccgacctggt gctgtgggag ccgttcacct 22020
   atgcggggcc ggtggcggcc cgggcgtgcg gggccgcgca cgcccgcgtc ctgtgggggc 22080
   cggacacgat cgggctgctg cggacgaagt tccttcaggc ccaggcgcgt cagcccgagg 22140
   agcaccggga cgacccggtc gcggagtgga tgacctgggc cctggcgcgc tacgggtgcg 22200
   acttccggga ggaggacgtg ctcggtcagt ggagcgtgga cccgatggcg gagggcgtca 22260
   gtctgggcct cgacctgccg accgtcccga tgcgctacac cccgtacaac gggtcggcgg 22320
   tgatccccga ctggctgacc gaggaaccga aacggcctcg ggtctgcctg accctggggg 22380
   tgtcctcgcg ggagcacagt gaggacgagg tcccggtgca gaggtttatc gaggcgctgg 22440
   ccgatctcga catcgagctg gtggcgaccc tggacgacgc ccagcgggac ctgctgccga 22500
   ggatcccgga caacacgcgc atcgtcgact tcgtgcccat ggacgcgttg ctgccgacgt 22560
   gctcggcgat catcaaccac agcggttcgg gcacgtgcaa caccgccgcg ctgcacgggg 22620
   tgccgcagat catcctcggc ggcatcctgg acgccgccgt acggcagcac atgttcgcgc 22680
   agaactccgc cgccctcacc ttcgctccgg aggaggtgac cggcgcgtcg ctgaggagcg 22740
   cgctggtgcg cctgctcgag gagccgcggt tccgcgacgg cgcgcggcgg ctgaaggagc 22800
   ggatgcgggc catgcccagc ccggccggga tcgtgccgac cctggagcgc ctcacggccc 22860
   agcaccgccg ggcgtgttga accggcgcgc gggcccgtgc cggcggtgac cgcccgaccc 22920
   gactctcgcg tgtgatcgat ctcgtcgact cagccgtgga ccggttcgcc tgtccgcgcc 22980
   cgacactgga gtgctgatgc gggccctctt cacgaccgcg ccgctcgcgg gccacctgct 23040
   tccgctggtg cccatcgcgt gggccctgcg ggcggccggc cacgaggtac tggtggcgac 23100
   ccgggaggac ttcgtgccgg tcgccctgcg gtcggggctg ccgtccgcct cgtgcgggcc 23160
   gcccgccgcg gacctggcgg gcgcggccga ggcgggggcg ctcgcgcggc cccgcggagc 23220
   ggcggaggct cggggggtcc tgagcggggc gctggcgcgc gtcgcccggg gcagtctggc 23280
   gggggtgcgg cggctggcgg acgcctggcg gccggatctg atcgtcagcg aacgggccga 23340
   gttcgccggg ccgctggtcg cggcggccct cggggtcccg tgggtccgct accactggtc 23400
   ggtctcgtcc ctggaggagt accggcgagc ggccgaggcc gagttcgcgc ccgagctggc 23460
   ggcgctcggc ctcgaccggt tcccggaggc ggcgcgcgtg ctcgatccgt ggccggtgtc 23520
   gctgcgccgg ccggacgcgg tcgcccacga cggggtccgg cacgtaccgg cccacgggga 23580
   cgcccccgtc cccgactggg cgttcacgcg cggtcgcggg ccgcggatct gcgtgacgct 23640
   cggcaccatg ctgccccggt acggcgccgc cgggatggcc gacttcctga cggagctggt 23700
   ggcggagacc cgcggagggg actgcgaact gctcgtggcg gtcgacgacg acgtcgtcgc 23760
   gcggtggccg tcgctgccct ccgcggtgcg gtacgccggc cggctgccgc tggcggaggt 23820
   gctgcccgcg tgcgacgcgg tggtgcacca cggcgggcag ggcacgtccc tgaccgcgct 23880
   ggccgcgggt cggccgcagg tcgtcatggc gcggctcgac gaccagttcg acaacgcgcg 23940
   ggcactggcg gcggcggggg cggccctgct cgtaccgccg tcccgggcca ctcccgcggc 24000
   cgtggccgcg gggtgcgccg aagtgctgga gaacgccctg tatgccaagg cggcagccgg 24060
   gctcgccgag gagatggcgc tgctgccgtc gccgtcggcg gcggtcggac tcctggaaca 24120
   cccggggccc gggccggaca tgccgcggag ttacccgaac gaggatgcgg tgtgacgtga 24180
   atctggaagt actcaaccgt tcgaacgatc cgcgcgggcc ggtgatcacg gtggtcggcg 24240
   cgtccggctt catcgggtcc gccctggtcg ccgagctggc gcgcatgccg gtgcggctgc 24300
   gggcggtggc ccggcgcgag acccccgttc ccgcgggggc acgggccgcc gtcgaggtcc 24360
   gccgggcgga cctcgcccgg ccggacgagg tcggggccgc cgtcgagggg gcggacgccg 24420
   tcgtgcacct cgccgcccac atcggcggcg cgcggtcgtg gcgcgcggcc gacgagcggt 24480
   cgctgcgggt gaacgtcggt ctgctgcgcg acgtggccga cgcgttccgg gaccgctcgg 24540
   ggcccgcccc ggccgtggtc ctggccagta ccctccaggc cggcgtcgag ctgtcccggc 24600
   agggcccgta cgcccggcag aagtcggcgg ccgaggaggt cctgctgcgg gccgcctccg 24660
   aggaggtggt ccgcggcgtc gtgctgcggc tgccgaccgt ctacgggcgc agcccgctga 24720
   ccgggtggac gggccgcggg gtggtcgcgt cggtggcacg gcaggccgtc tcgggcgagc 24780
   cggtcacgat gtggcacgac ggcacggtcg ggcgcgatct gctccacgtg gaggacgcgg 24840
   cccgcgcctt cgcggcggcg ctcggtcacg tggagcggct ggacggcggc acgtggtccg 24900
   tcggtacggg ccggctggag cccttgggag aggtgttctc ggccctcgcc gggctggtgg 24960
   ccgagcggac ggggaggccc cccgtaccgg tggtctccac ggagccgccc gaccatgccg 25020
   aggcgggcga cttcgagagc gcggtctgtg acccctccgc gttccgcgcg gtgaccgggt 25080
   ggtctcccct cgttccgttg cgggcggggc tcggcgccgt ggtggagacg atggtggccg 25140
   acggagcgag gggtgggatc cgaacgtgag cacggaccgg gagcaggccg cgcacacgcg 25200
   gctcggtcgc agcgcgaccc tggtgagccg gctctggctg ggcaccgtga acttcagcgg 25260
   ccgggtcgag gacggtgacg cgatgcagct gatggaggcg gcggtcgacc gcggcatcaa 25320
   ctgcatcgac accgcggaca tctacggctg gcggatccac aagggccaca ccgaggaact 25380
   ggtgggccgg tggctggcca agagcgccgc gcggcgggag gacgtcctgc tggccaccaa 25440
   ggtcggcggg gacatgagcg aacggctcaa cgacggcggc ctgtcggcgc ggcacatcgt 25500
   cacggcctgc gagcagtcgc tgcggcgcct gggcgtggac cacatcgacc tgtaccagat 25560
   gcaccgcgtc gaccacgccg cgccgtggga cgagatctgg caggcgatgg accgtctggt 25620
   ggcgagcggc aaggtgacct acgtggggtc gtcgaacttc gccggctgga acgtcgccgc 25680
   cgcgcaggac gcggcccggc ggcgccagtc cctcggtctg gtgtccgagc agtgcctgta 25740
   caacctggcg gtgcgccacg ccgagctgga actgctgccg gccgcccagg cgtacggact 25800
   gggcgtgttc gcctggtcgc cgctgcacgg cgggctgctc agcggggtgc tgcgcaagct 25860
   cgcggcgggc gtcgcggtga agtcggcaca ggggcgggcc cagctgctgc tgcccgagct 25920
   gcgcgcgacg atcgaggcgt acgaggggtt ctgcggccgg atcggcgcgg atccggccga 25980
   ggtcggtctg gcctgggtgc tgtcccggcc ggggatcagc ggcgcggtga tcggtccgcg 26040
   cacggtggac cagctggact cggcgctgcg gtccctggac ctggtcctcg gggaggccga 26100
   actggccgag ctggacgcca tcttcccgcc cctgggcaag ggcggccggg cgccggacgc 26160
   gtggatcagc tgaagggggt gcatcggccg acgtcacgcc ggccgatgcc gccggtcaca 26220
   cgacgtcgag cgcgggcagc gggaagacca gtcggccgcc gccgtcgagg aactcccgct 26280
   cccgttcgac gaacccgtcc cggtagatcc agggcaggac cagcaactgg tccggcttct 26340
   gcgccttcgc gtcctcctcg gacacgatgg ggatgcccgt cccgggggtg aaacgccccg 26400
   ccttctcctc gctcacctcg ccgatgcacg gcaggtcccg ttcggtgatc ccgcagtact 26460
   ggaggatgac gttgcccttg gtggaggcgc cgtacccgag ggtcagcagg ccctcttggc 26520
   gggagcggtc caggaagccg cgcagggcgt cccgctggtc ggcgacgcgg cgggcgaagg 26580
   cctcgaacgg tgccatgccg tccagtcccg cggcggcctc ggcggcccgg atgcgggcca 26640
   ggcccgcctc gtccctcggg tgccgggaac cggtcctggc gagcgtgacg cacaggctgc 26700
   cgccgtacac ctcggtgagc tcggcccgga tgacggtgag gccgacgcgt tccgccatcc 26760
   actcgatctg gcgcagcgcg tagtactcca ggtgctcgtg gcagacgatg tcgtacgcgt 26820
   cggcttcgag catggcgggc aggtagctct gctccatcat ccagacgccg tcctcggcga 26880
   ggacgtcgcg gacgtcgctc atgaagcgca gcgggtccgg caggtcgtag aacatcgcga 26940
   tggaggtgac ggccttcgcc cgccgcgccc cgaagcggtt ctcgaaggtc gcgcgggtga 27000
   agtagtccac gaccaactcg gcgttcggcg ggtacaggtc gcggaacttg ccgccggtcg 27060
   ggtcgatgcc gaccagtcgc gggccgtcgg cggggtagcc cttgaggagc gtggcgtcgt 27120
   tgctgccgat gtcgaggacc aggtcgtccg ggccgaggtc caccagccgg cggacggcgg 27180
   cgaccttgcc atgcaggtgg tcgaccatga agggccggat gcccgagcgg tagccgtagc 27240
   cctcgccgta catgaggtcc gggtcggggg tgtggcgcag ttgcacgagg ccgcatccgg 27300
   ccggggaaca cgcgacgagt tccagcggga ccgacggcac gacctcgtcg cggtcggccg 27360
   ggaacacccc ggtgagcgcc tgttcgccca ggtcgagtac ggagagcagc tccttgttgc 27420
   cgcagacgcg gcacgcggtg gcaatcatgg ggtcctttcg ggatcctggc cggggcgccg 27480
   ccggcccgtg gccaggtcgg gagcttccag acggagggtg tcgggggcgg gccgggggga 27540
   cggcaccagg tgcaggcggc cgatcgcgcc ggtggcggcg gaggcgtcct ggcacgggca 27600
   gggatcgtcg gtgaatccgg cgaagcggta ggccacttcc atcatccggt tgcgttcggt 27660
   ggcccggaag tcggctccga ggtgcacccc ggcccggtgc gcctggtccg tcagccagcg 27720
   caggatcacc gtgccggccc ccagcgacac cacccggcac gaggtggcca gcagcttgat 27780
   ccgccaggcc tcggggccgc gccgcagcag cacgacgccg accgcgccgt agggaccgaa 27840
   gcgatcggtg acggtggtga ccagcacctc gtggtcgggg tcgtcgatga gggcgcgcag 27900
   ctcgtcctcg gagtagtgca ctccggtggc gttcatctgg ctggtgcgca gggtcagttc 27960
   ctcgacccgc gacagctcca ggggcgtggc gcggctgatc cgcatccgga tgtccagcga 28020
   gcgcaggaag tccgcgtcgg gtccggtgaa gtcggaccgc tcggcgtccc ggcggaagga 28080
   cgcctggtac atggagcggc ggcgggtcga gtcgacggtg acggtgcccg ggctgaactc 28140
   cgggaggccg gtcagccggg tcgcctgctc ggcggcgtag gtgcggacct ccggcagttc 28200
   gtgggtgacc tcggcccgtt cgaagggctg gtcgtcgatg aaggcgaggg tgctcggcgc 28260
   gaagttcagc cggtcggcga tctcgcggac cgacttcgac ttcgggcccc agccgatcct 28320
   cgggagcacg aagtactcgg cgacgccgag ctgttcgagc ttcgcccagg cgtgatcgtg 28380
   gtcgttcttg ctggccaccg cctgcaggat gccgcgcgcg tccagctcgg tgatggtccg 28440
   caggacgtcc ggggcgagcc ggacctcgtc ctcctcgagg agggtgccct gccagagggt 28500
   gttgtccagg tcccagacca ggcatttgac caacggctcg gcggcgttgt cccggtgcac 28560
   gtcgttccct tctctcgata cggcggttgc gaccgcgact cctcgacagg gcgtacgcgg 28620
   cccgcggccg gcggggacac gggccggttc acccggccgc ggacatcacg tgccgggcca 28680
   ggaccagttc gcagatctcg ttgctgccct cgatgatctc catgagcttg gcgtcgcggt 28740
   gcgcccgtgc gacgacgtgt ccctcccgcg ccccggccga cgccagcacc tgcacggccc 28800
   gttcggcccc gcgtgcggcg ccggtggccg cgacgtgctt ggccaggacg gcggcgacca 28860
   ccatgtcggg gctgccctcg tcccactggg cgctggcgtg ctcgcacgcc cgggcggcgt 28920
   gctgttcggc gacgaacagt tcggcgaggt gccgggcgac gagctggtgg tccgagagcc 28980
   gcgtgccgaa ctgctcccgt ccgccggcgt gacgtgcggc ggcggccagg caggcgcgca 29040
   ggatgcccag ggagccccag gccaccgaca tccggccgta gctcagcgcg gtggtgacca 29100
   gcagggcggg ggtgcggtcg tggccctgga gcagggcgtc tgccggcagc cggaccccgt 29160
   ccaggtggat gtcggcgtgt ccggccgcgc ggcagccgtg cgcgtcggtg atgcgctcga 29220
   cgcgtacgcc gggggccgag gcgggcacca cgacggcgcc cgcgccctgc tccgtgcggc 29280
   cgaagaccac cagcaggtcc gcgtacgcgg cgttggtcgc ccacaccttg acgccgtcga 29340
   cgacgacttc gtcgccgtcg gaggtgatgc gggtgcgcag ggcggacagg tcgctgccgg 29400
   cgccggcctc ggtgaacgcc acggcggcca gttcgccgcc ggtcaaccgg ggcaccaggg 29460
   aggcctgctg gtccgctccg gcgagccggc gcagcgtcca ggcggccatg ccctgcgagg 29520
   tcatgacgct ccgcaacgag ctgcagaggg cgccgacgtg cgcggtgagt tcgccgttgc 29580
   gccggctggt ccagccgagg ccgccgtgga ccgcgggcgc ctgtgcgcac agcaggcccc 29640
   gggagccgag gtcgtgcagc aggccgaggg gcagctcgcc cgtccggtcc cactcggccg 29700
   cccggtcgcc gaccagcgcg gtgaacagct cctcggcctc ggtgccgtcg gcgtgggagg 29760
   tgtcagccac ctgcgtgccc ggtcgcctcg ccgggcgcgg ccagccgtcc gaccagccgg 29820
   accatcgcgt cgacggtgcg gaagttgtcg agcatcaggt cggcgccgct gatgacgatg 29880
   ccgtaggtct tctccaggtg cacgacgagc tgcatggcga acagcgagga catcccgccg 29940
   acggcgaaca ggtcctggtc gcgctcccag gtggtcttgg tgcggtcctc gaggaacccg 30000
   agcagttccc cggcgacctc gtcggcggtg ggggtcgtgc cggtggggtc gggccgaccg 30060
   gacgtcgtgg tcatcgcgtg gcctcctggt agtcgtagaa tccccggccg ctcttgcggc 30120
   cgagcaggcc ctggcggacc ttgtccagca gcagctcgct cgggcggagc gccggatcgc 30180
   cggtccgttc gtgcatcacc cgcagcgagt cggccaggtt gtccagtccg atcaggtcgg 30240
   ccgtggccag gggtccggtg cggtggccga tgcagtcgcg catcagcgcg tccacggtct 30300
   ccggggtggc ccggccctcg tgcaccaccg cgatggcgtc gttcagcatc cggtgcagca 30360
   ggcggctggt cacgaagccg gcgccgtcgc cgacgacgat gccccggcgg cccaggccgg 30420
   acagcaggtc ccgggtggcc cgggcggccg cctctccgct gcgcggtccg aggaccacct 30480
   cgaccgtggg gatcacgtac gcggggttca tgaagtgcac gccgacgagg tcctcggggc 30540
   gggggacggc gtcggccagc tcgtcgatgg ggacgcccga ggtgttgctg acgagcagcg 30600
   tccccgggcg cgccacggac gccaggtccg ccagcacctc ggccttccgc tcggggtcct 30660
   cggtgacggc ctcgatcacg gcggtcgcgg tggcgacggc ggccggcgcc tcctcgacgg 30720
   tcagctcccc gggcgggcgg tcgtggggca gcgcgcccat cagccgggcc gtccgcagat 30780
   gcagcgcgac cgcgtcgggg gcggccgcgc gcgccccggc ggaggtgtcg accagtgtga 30840
   ccgggtgccc gtgtccgacg gcgagtgccg cgatggccgt gcccatgacg cccgcgccga 30900
   gcacgacgag cggagaattt tccttggaat cgggcacgga tcc 30943
<210> 2
   <211> 11171
   <212> DNA
   <213> Streptomyces ambofaciens
<400> 2
   ctgcaggcgc gcgggctgct cgggcaggag gcgcccgccg agagcctcga cgcgatgatc     60
   gacgactact gcgcgcagat ccgcgaggtc cagcccgagg gcccgtaccg gctgctcggc    120
   tggtccatgg gcgggctgct cgcgcaccgg gtcgccaccc gcctccagcg ggcggggcag    180
   cgcgtcgacc tgctcgcgat cgtcgacgcc tatccgcccg cctcggtgcg ggccgactgg    240
   gacgcggcgg agatggtggc gcggatcggt gaggaactgg gcttcgacgt ggaccgggtc    300
   ggccccggcc aggaggaggc gctgctcgcg gatctgcggg cgaaggggca tcccctgggg    360
   catctgccgg gcggtgacat cggcgcggcg gtccgcgtgt acgtcaacag ctcgcgcctg    420
   acgaggaacc tgcggcccga ggcgttcgac ggggacgtcc tcttcttcgc ctccgggacc    480
   tcgttcgccg aggacgacca ggttcacgac gtcgccgcct ggcggccgta cgtcaccggg    540
   cagatcaccg agcacgtgat cgaccacctc cacgaggacc tcttgatcga accggccgcg    600
   gtcacggcca tctccgacgt gctcgtcggg catctgcccg cggacgacac accccgcgcg 660
   tccggcgcac cccgcgcacc tcgcgcatcc cgatgaggaa ggaaacatca tgagcaaccc 720
   gttcgaggac acggaagcca cctacgtcgt gctggtcaac gacgaggggc agcactcgct 780
   gtggccgtcg ttcgcggagg tcccggcggg ctggtccgtc gtggtgccgg agacggaccg 840
   gcagtcgtgc ctggactaca tcaacgagaa ctggaccgac atgcgcccca agagcctcgt 900
   cgaggcgatg gcgacggccg ggcaggacgc cccttgagcc aggacgccat gatccgggcg 960
   gtcggcgtcc gcaagagctt gcggcgacgt ccacgccctc gacggcgtgg acatcgaggt 1020
   cgaacggggc cgggtgctcg ggctgctggg tcacaacggg gccggcaaga cgaccttggt 1080
   gaacatcctt gccacggtct ccccggcgtc cgcgggcacg gtgaccgtcg ccggtttcga 1140
   cgtcgcgacg cagggcgccg agatccgcgc gcgcatcggg gtgaccggcc agttcgcgtc 1200
   ggtggacgag tacctgagcg gattccgcaa cctcgtcctg atcggccgcc tcctcggggc 1260
   gggacggcgt gaggcggcgg cccgggccac cgagctcctg gagctgttcg agctgaccgg 1320
   ggcggcccac cagccctccc gcacctactc gggcgggatg cgccgacggc tcgacctcgc 1380
   cgccagcctg gtcggccggc cggacgtgct gttcctcgac gagccgacga ccgggctgga 1440
   cccggcgacc cggatcgccc tgtgggagac ggtggagaag ctggtggcgg gcggcacgac 1500
   cgtcctgctg accacccagt acctggacga ggcggaccgg ctggccgact ggatcaccgt 1560
   cctgtcgaag ggccgggtgg tggcctcgga caccaccgac cggctcaagg ccgacctggg 1620
   ccaccggtcg gtgcgggtgg tccttccgcc cgccgccgac ctgacggccg ccgccgccgc 1680
   gctcaccgcc ggcgggttcc gtccgcggtc cgacgccggg gagcacgcgc tgaccacgcc 1740
   cgtggacacc tcggccggta tcgcgggcgt catccgcgcg ctggacaccg tcggaacgca 1800
   ggccgtcgag ctgaccgtca aggagccgtc cctggacgac gtctacctgg cgctcaccca 1860
   tccctcaccc gccgccgacg cggcctgatc cccaggagtt ccgttggcca tccaggagcg 1920
   cgccgtgaag gaggtggcgg tcgacaccgg taccccggcc ggccccgtgt ggcgtggtgc 1980
   cggcctcggc acccagctgt gggtactgac cgcgcggcag atccgttcca tgtacggcga 2040
   ccgccgcctg gcgctgttca gcctgatgca accggtgatc atgctgttgc tgctgagcga 2100
   gatcttcggc agcatggccg acccggacga cttcccgcag ggcgtgcgct acatcgacta 2160
   cgtggtgccc gcgctgctgg tcaccaccgg catcggctcg gcccagggcg cgggggtggg 2220
   cctggtcagg gacatggaca acgggatggt ggcgcgcttc cgcgtcctgc cggcccggct 2280
   gttcctggtg ctggtcgccc ggtcgctggc cgatctggtc cgtgtgttca ccgagttggt 2340
   cgtcctcgtg gccgtcggtg tgatcctgct gggcttccgt ccggccgggg gtttgtgggg 2400
   cacgtccgcc gccctgttgc tcaccctgtt cgtcatctgg tcgctgatct gggggttcat 2460
   cgccctcgcg gcgtggctgc gcagcgtgga ggtgatgtcc agcctcgcgg ttctggtgat 2520
   gttcccgctc atgttcgcct ccagtgcgtt cgtcccgctg gacgccctcc cggagtggct 2580
   gcgctcggtg gcgcacctca accccgtgac gtacgcggtc gacagcgccc gccgcctggc 2640
   gctggactgg gacccggggt ggagcgtgcc cggcgcgctg ctgaccagca ccgcgctcat 2700
   ggcggtgggg atgtacgtcg ccgggcgttc cttcaagagg cccccgaacg aatgattccg 2760
   gcgaacgggt ccgtcgcctg cccctccggg ctcgcccggg ggaggcgggc gcggtctcag 2820
   cggtcggccg tccgcccgta ggcgcggaag agtgcggtcc actccgggac cgtgaggtcc 2880
   ttgacccggt cggccggcct cacgcccgcc ccgcgcacga actgggcgtg tccgcggcgc 2940
   agcaccttcc gcgcggcgtc gcccaccgtc atctggccgg tgtcgaagac ccgttggacg 3000
   aaccgctggt aggcggcctt ctcacgccag ggcacggacg gcctgcggtg cggggcgacc 3060
   atcagggtct gggtgtccgc gcgcggtacg ggggtgaagt cctggcgtga gaaggccagg 3120
   ccccggtcga acgagtacca cggctcccac tgggcgttga agaggtttcc gccccaggct 3180
   ccggtccgct ttcccacgta ctcccgctgc aacaggaaca cgccctgccg catgcgcgcc 3240
   ggacccatgt cgaggcagcg cctcagcatt ctggtgccgg tgacgaaggg aagattcccg 3300
   atgagtctga ccggctgccc gggcagttgc agggtcagga aatcctcgtt caccaccgtg 3360
   acgtccggca gcgattcggc ggccagccgc cgagcccagc ggggatctat ttccaccgcg 3420
   agtaaaggcg tcccgggtga ggcgagcact ttggtcaccc gccctgatcc cgcgcctatt 3480
   tcgacggtca tcaggtcatt cggagaatcg ggcggaatgg tgtccgaacc gtccagctga 3540
   gcggagaaac gacaggccgc ggcggctgta cgaaagaagt tctgacccca ttctctccgc 3600
   gcggtgctgc gtgaatcagc gggtgcggag acggattgcg gtggcagtgg ggatttcaat 3660
   gtcacctcgg cgacattacc aagtcttgac ccaacggtcc atcaaccacc ggtatacccc 3720
   atgcaattca gccaccgtcc ggcccgatcc cgacgttcgg ccgcgggcct ttcgggcccg 3780
   accggtgacc agcgcgcgaa ccggagttcc cggcgatatt tccggctcct ggcgactcgc 3840
   cacagtctcg tcgggggcgg ccggcgccat gactccgacg ggggcgattc ccggccgact 3900
   tcgtcaggtc cggagggtcc cccgggcggc gcgggccgat ggccgctgac tgtgtcatct 3960
   gcgggttgtc ggccgaacac accgcgcccc aggatgactc agccgttcct gcggtcgtcc 4020
   cgaccccggt cccacccccg aaaagaggag cgagaatcca gtgctccagc gcgtcgatct 4080
   gtcgtcactc accggcctcc gctggtatgc ggcgctgacg gtattcgcct gtcacatcgc 4140
   ccagcagggc ttcttcgccg accagcaggt gggcagcgca ctgctgcaca tcaccccgct 4200
   cggttccatg gcggtctcga tcttcttcat actgagtgga ttcgtcctcg cctggtcggc 4260
   ccgcgacgag gactccgtgc cgactttctg gcggcgccgc atcgcgaaga tctatccgct 4320
   gcatctcgcg acgttcggca tcgcggctct catcattttc tccctgtcgg agccggtact 4380
   tcccggcggt tccgtatggg acgggctggt gcccaatgtt ctgctcgtgc agtcctggct 4440
   tcccgacgcg accctcacgg ccagtttcaa cacgcccagc tggtcgctct cctgtgagat 4500
   cgccttctat ctgtcgttcc cgctgtggta ccggctggtg cgcaggattc ccgcacggcg 4560
   gctgtggtgg tgcgccgcgg ggatcgccgt ggccgtgacg tgtgtgcccc tgctggcggg 4620
   cctgctcccg gcgagcgagg aggtggcccc cgggatgtcg ctcaacgagg tctggttcgc 4680
   gtactggctt ccgccggtgc gcatgctgga gttcgtcctc ggcatcgtga tggcgctgat 4740
   cctgcgcgcg gggatctgga agggccccgg tccggcggtc tgcacggcgc tcctcgccgc 4800
   gagttacggc ctcacccaga tggtgccccc gatcttcacc ctcgtcgcct gctccgtcgt 4860
   accggccgcg ctgctgatca cggcgctggc cgacgccgac gtgcacggcc ggcgcacggg 4920
   gctgcgttcg gcgacgctgg tgcggctggg ccagtggtcc ttcgccttct acctggtcca 4980
   cttcctgatc atccgctacg gacaccggct gatgggcggc gatctgggct acgagcggca 5040
   gtggagcacc ccggccgcga tcgcgctgtc cctggggatg ctgggggtgg cggtcctggc 5100
   cggcggtctg ctgcacaccg tcgtcgaaca gccctgcatg cgcctgttcg gcagccgcag 5160
   gtccgcctcc cgtccgaagc ccggcgccac cgcggctccc cggaactcac ccgcggccga 5220
   cgcggccggc gtgcccctgc tcccgggcgt acccgggccc gcgcacaccc ccgcagcgac 5280
   gaacgaaccc accccgagag gatgatgagc gtggcagacc agacggctct cagccccgcg 5340
   ctgctggagt acgcccggag cgtcgcgctg cgggacgacg gcctgctgcg cgaactgcac 5400
   gaggtgaccg ccgggctccc cggcggccgg gccatgcaga tcatgcccga ggaggcgcag 5460
   ttcctcgccc tgctgatccg gctcgtcggt gcccggcggg tgctggagat cggcaccttc 5520
   acggggtaca gcacgctgtg catggcgcgg gcactgcccg ccgacggcac cgtcgtcacc 5580
   tgcgacatca gcgacaggtg gcccggcgtc ggcgcaccgt actggcgccg ggccggggtg 5640
   gagtcccgga tcgacctgcg cgtcggcgac gccgtccgga ccctcgccga gctccgcgag 5700
   cacgaggggg acggctcgtt cgacctggtc ttcgtcgacg ccgacaagac cgggtacccg 5760
   cactactacg agcaggcgct ggccctggta cgccccggcg gactggtggc ggtcgacaac 5820
   accctgttct tcggccgggt ggccgacccg gccgtcgagg acgccgacac cgtcgccgtg 5880
   cgcgcgctca acgagctgct gcgcgacgac gaacgcgtgg acatcgccct gctgacggtc 5940
   gccgacggga tcactctggc ccgccggcgg gagtgagtcc gcacggggtg cggagcacct 6000
   ggtgccgtca ccggcccgca cacgtccgcc gccgacctgc ccgggcaggt cgccgagctg 6060
   ctgccctggc gtggccggcc ctcgtcgagg gccgcgcggt cagcggaacc ggttgatcgc 6120
   gtcgatgtgc cgcgcccgct tctcctcgtc gcgcacgccc agcccctccg tgggcgccag 6180
   gcagagcacg ccgaccttgc cctggtgctg gttgccgtgc acgtcgtgca cggcaaccgc 6240
   cgtgtcggcc agcgggtagg tgcgcgagag ggtggggtgg atcctcccct tggcgacgag 6300
   ccggttcgcc tcccacgcct cgcggtagtt ggcgaagtgg gtgccgacga tacgcttgag 6360
   gtgcatccag aggtagcggt tgtcgaactc gtggcggaag cccgaggtgg aggcgcaggt 6420
   gacgatcgtg ccgcccctgc gggtgacgta gacggacgcc ccgaaggtct cccggccggg 6480
   gtgctcgaag acgatgtcga cgtcctcgcc cccggtgacc tcgcggatgc gcttgccgaa 6540
   gcgcttccac tcccgcgggt cctgggtgtc ctcgtcgctc cagaagcggt agtcctcggc 6600
   ggagcggtcg atgatggcct ccgcgcccat ggcccggcac acctcggcct tgcgcgcgct 6660
   ggagaccacg cagacggggt gggccccgcc ggcgagggcc agctgggtgg cgtacgagcc 6720
   caggccgccg ctggcgcccc agatcagcac gttgtcgccc tgcttcatgc cggcgccgtt 6780
   gcgggagacc agctggcggt aggcggtgga gttgaccaga ccgggcgcgg cggcctcctc 6840
   ccaggtcaga tgggccgcct tgggcatcag ctggttggac ttgacgaggg ccacctcggc 6900
   caggcccccg aagttggtct cgaagcccca gatgcgctgg gccgggtcga gcatggtgtc 6960
   gtcgtgcccg tcggggctct ccagctccac cgacagacag tgcgcgacga cctcgtcacc 7020
   gggtttccag acgttcacgc cgggtcccgt gcgcagcacc acgccggaca ggtcggaacc 7080
   gaggatgtgg tacggcaggt cgtggcgcgc ggccagcggc gaggtgcgcc cgtagcgctc 7140
   caggaagccg aacgtcggga ggggctcgaa gatggaggac cagaccgtgt tgtagttgac 7200
   ggagctggcc atcaccgcga ccagcgcctc cccgggcccc acttcgggca gcggcacctc 7260
   gtcgacgtgc agggacttgc gcgggtcctt gtccgcgctc ggcatcccgc ggaacatgtc 7320
   ggtgtcctct ttgcggacgg tcacggcgcg gaaggactcg ggcaggggca gcgccgcgat 7380
   gtcctcgggg gtgcggtccg cggcggtgat cgcggcgagc agcgcgctct gcgcatggct 7440
   ttcgggcatg gaacggtctc cgatcgctcg tgtcgtcagg tggcccggtg cagggcggtg 7500
   aggcgctcca gggtggggat gatcccggcg ggcgtggggt cggcgagcat ctcctcgctc 7560
   agccgccgcg cgcccgccct gatcgcgggg tcgtgcacgg ccgtgtgcac cgcgtcggcc 7620
   agaccgcgcg ccgtgagcgt cgccgcgggc aggtcgaacc cggcggacag gcgctggagt 7680
   tgctgcgcct tgagcggcgc gtcccacagg gagggcagca ggatctgcgg cactccgtgg 7740
   cgcagggcgg tggaccaggt gcccgctccg ccgtggtgga tgatcgcggc gcagctcggc 7800
   agcagcgcgt cgagcggtac gaagtccacg ggcacgacgt tgtcggggag ggtgcccagg 7860
   cggtcgagct gggaggtgtc cagggtggcc accacctcga tgtccagccg gccgagcgcc 7920
   tccagcagtt cggagtagga gaccgcgtcg cggccgtagg tctcgcgggc ggacacgccg 7980
   agggtgaggc agacgcgggg gcggtcgggc ttcttgccga gccagggttc gatcacggag 8040
   cgcccgttgt agggcacgta gcacatgggc accgtggtct ggcccaggtc gaggcgggtg 8100
   ctgcgcgggc ccgggtcgat cacccagtgg ccgagcacgt cgcgttcgtc gaaggcgagc 8160
   ccgtaccggt ccagcgtcca gccgagccat tcggcgatgg ggtcctcgcg cagttcctcc 8220
   ggcatcccgt cgagggcggc gaggaagcgc aggcgggagc gtccgatggc gtcggggccc 8280
   cacaggatgc gcgcgtgggc ggcaccgcag gcgcgggccg cgaccggccc cgcgtaggtc 8340
   cagggttccc acacgaccag gtcgggccgc cagccacggg cgaagtcgac gatctcgtcc 8400
   atcgtggcgg cgccgttgaa cggggcgaag cacagggcgg ccatcatgct ctgctggccg 8460
   agcaggtact cccaggtggt ctcctcctcg ctctccacct caccgaactc gaagccccgc 8520
   tggtagggga cgaggtcgct gcccatctcg gcgagcagct ccgcggccgg tcggtcgtcg 8580
   cccaccggta cggcggtcag gccggtggag gtgatgacgt cggtgaggga gggcgggctc 8640
   gccacccgta cctcgtgccc ggcggcgcgc agcgcccagg cgaggggcac cagactgtag 8700
   tagtgggtgt tgtgggcgag ggatgtcagc aggacgcgca cagcggctcc ggtctgggag 8760
   ggggcgtacg gacggcgggg tcatcccggg tggacccgga ggcgggcgca ggcgcccagg 8820
   accggtgacc gcggccgggt caggggcggt ccctcggccc gcaggccggg caggcggccg 8880
   gccgtgacct ggaccgcggt ggtggcggcc aggcggatca gcgggagggc gagcgccagg 8940
   tgcggtccgt cggcgccccc gagcggctcc ggcccgggga tctcccgtcc ggcggcggcc 9000
   agcaccacga cgtgctcgtc ggcggtgatc cggcggccgc cgagttcgag gccggtgtgc 9060
   gcgacccggt tctccagccg ggccgggggg cgctcgcgca gcacctggtc gacggcgttc 9120
   gccgcggccg gcgtccggcg ggccctctcc cactcccccg gccggccgag cagccggtgg 9180
   accgtgtgcg cgacggcggt gacgacgggt tcgggtgcgc cgaccgcgag gagcagcgcg 9240
   atgcgctcga cgtcgccggg ggcgacgccg tcgtgcagga ggagggcgag cacgtcgtcg 9300
   ggccggggct cggcgttccc gaggccccgg gacttctccg cgacgagctc ggggaccagg 9360
   tcggccagtg tgcggacggc gtcggcggac tcccgggcca cggtcagcag ttgcggggcc 9420
   atccgggcgt cgagctgggg gccgcaggcg gcgagtgccc gcgcggcggt ggcgcggtcg 9480
   cggcccggca ctccgaggag cctgagcatc agctcgacgg cgtagggccg ggcgacctcg 9540
   ccgacaaggt cgaacccggc gtcaccggtg ggcagcaggc ggccgagcac tctgcgggcg 9600
   gtggtgcgcg ccgcgcaggg cgcctgggcc ggggcgtacc ggctgagcac cggggcggcc 9660
   agcgcccgca ggcgtacgag ctccgcgcgt tcgtggtggg ggaacgcctc ggcgaggggc 9720
   agcagttcct cgtccgggcg gcgtccggcc cggtccagcg tgccgaagcg cgggtcggcc 9780
   aggacggccg ccgccacctc ggggtccgcg gtcacccagg cgtccagctg ttcgctgcgg 9840
   aaccacggtc cgcgggcccg gatctcccgt tcgaacggct cggggtcggc gacggcgcgc 9900
   aggatcagcg cgtacgggtc gccctggttg ccggcgcacc agtgtgcggc ccgggtcagc 9960
   tggagccgac ggccgagcgc acggacgccc gtgctcgtgt ccgcgctcgt gctcgtgctc 10020
   gcgggggccg tttccgtggc aagggtgggc atggctgccc tgcctttctt ctcgggtcga 10080
   tgggggcgct cgcacgatcg ggggacccgg gtgtgccggg tgggtcgggg caacccgcct 10140
   ccgggccgcg gcggtccgcg tcggcaccgg gtcagcggcg ggtgccgatg aacagcccgc 10200
   gaccggacgg gccgccttcc tggtagacca cgtcgcagcc ggcgcgctcg aaggccgcct 10260
   cgtagtcggc gcgcgggaac agggtgatgg tgtggtcctc ggtcaggtga cggacgccgc 10320
   cgggtccggc gaggaggtag tgcacctcga tgcgggtggc gttcccctcc cgtacggagt 10380
   gcgagacccg gcagacggtg cgctcgcccg cctccgtgat gctggcgccg acgtaaccgg 10440
   gggtgaagga ctcggggaac caccagggtt cgacgacgat gacgccggac ggttcgaggt 10500
   ggtcggtgaa ggcccgcagc gtgctgtcga gttcgtcggt ggtccgcagg tggcctatgg 10560
   agctgaacat gcaggtcacc gcgtcgaacc ggcgtcccag ggagaacgag cgcatgtccc 10620
   cttggtggaa ggtgacaccg gggttccggc cggtcgcgag ggccagcatg tcggcggaga 10680
   gttccaggcc ctcgacgtgg tcgaagaggc cgtccaggtg gtgcaggtgc tggccggtgc 10740
   cgcaggccac gtcgagcagg gtccgggcgc ccggccggtg gacgcgcacg agtgcggcga 10800
   tctcctcggc ctcctgccgg tagtccttcc ccttcccctc gtggaccagg tcgtagacgg   10860
   ccgcgatgtc gtcggcgtac atcagtgttt ccctccggtg agcggggcgg gaccgggctg   10920
   gtgcgggagg ctgtccagcc attcgtgcac cagggacgcg gtgtgccggg cgtgttcggt   10980
   gagcatggtg aagtggttgc cgggcacgtc ggcgacggtc cgtgcgaacg ggacctggga   11040
   ccgccagtcg ccccgcgccc cgcccgcggg cggccacgcg cacaggggtt cggacgcccg   11100
   ggccagcagg acgggggctt cgagggcggt gagccgggtt ccgagcacca gccgctgata   11160
   gccggccatg g                                                        11171
<210> 3
   <211> 711
   <212> DNA
   <213> Streptomyces ambofaciens
<220>
   <221> CDS
   <222> (1)..(711)
<400> 3
   atg tac gcc gac gac atc gcg gcc gtc tac gac ctg gtc cac gag ggg     48
   Met Tyr Ala Asp Asp Ile Ala Ala Val Tyr Asp Leu Val His Glu Gly
   1               5                   10                  15
   aag ggg aag gac tac cgg cag gag gcc gag gag atc gcc gca ctc gtg     96
   Lys Gly Lys Asp Tyr Arg Gln Glu Ala Glu Glu Ile Ala Ala Leu Val
               20                  25                  30
   cgc gtc cac cgg ccg ggc gcc cgg acc ctg ctc gac gtg gcc tgc ggc    144
   Arg Val His Arg Pro Gly Ala Arg Thr Leu Leu Asp Val Ala Cys Gly
           35                  40                  45
   acc ggc cag cac ctg cac cac ctg gac ggc ctc ttc gac cac gtc gag    192
   Thr Gly Gln His Leu His His Leu Asp Gly Leu Phe Asp His Val Glu
       50                  55                  60
   ggc ctg gaa ctc tcc gcc gac atg ctg gcc ctc gcg acc ggc cgg aac    240
   Gly Leu Glu Leu Ser Ala Asp Met Leu Ala Leu Ala Thr Gly Arg Asn
   65                  70                  75                  80
   ccc ggt gtc acc ttc cac caa ggg gac atg cgc tcg ttc tcc ctg gga    288
   Pro Gly Val Thr Phe His Gln Gly Asp Met Arg Ser Phe Ser Leu Gly
                   85                  90                  95
   cgc cgg ttc gac gcg gtg acc tgc atg ttc agc tcc ata ggc cac ctg    336
   Arg Arg Phe Asp Ala Val Thr Cys Met Phe Ser Ser Ile Gly His Leu
               100                 105                 110
   cgg acc acc gac gaa ctc gac agc acg ctg cgg gcc ttc acc gac cac    384
   Arg Thr Thr Asp Glu Leu Asp Ser Thr Leu Arg Ala Phe Thr Asp His
           115                 120                 125
   ctc gaa ccg tcc ggc gtc atc gtc gtc gaa ccc tgg tgg ttc ccc gag    432
   Leu Glu Pro Ser Gly Val Ile Val Val Glu Pro Trp Trp Phe Pro Glu
       130                 135                 140
   tcc ttc acc ccc ggt tac gtc ggc gcc agc atc acg gag gcg ggc gag    480
   Ser Phe Thr Pro Gly Tyr Val Gly Ala Ser Ile Thr Glu Ala Gly Glu
   145                 150                 155                 160
   cgc acc gtc tgc cgg gtc tcg cac tcc gta cgg gag ggg aac gcc acc    528
   Arg Thr Val Cys Arg Val Ser His Ser Val Arg Glu Gly Asn Ala Thr
                   165                 170                 175
   cgc atc gag gtg cac tac ctc ctc gcc gga ccc ggc ggc gtc cgt cac    576
   Arg Ile Glu Val His Tyr Leu Leu Ala Gly Pro Gly Gly Val Arg His
               180                 185                 190
   ctg acc gag gac cac acc atc acc ctg ttc ccg cgc gcc gac tac gag    624
   Leu Thr Glu Asp His Thr Ile Thr Leu Phe Pro Arg Ala Asp Tyr Glu
           195                 200                 205
   gcg gcc ttc gag cgc gcc ggc tgc gac gtg gtc tac cag gaa ggc ggc    672
   Ala Ala Phe Glu Arg Ala Gly Cys Asp Val Val Tyr Gln Glu Gly Gly
       210                 215                 220
   ccg tcc ggt cgc ggg ctg ttc atc ggc acc cgc cgc tga                711
   Pro Ser Gly Arg Gly Leu Phe Ile Gly Thr Arg Arg
   225                 230                 235
<210> 4
   <211> 236
   <212> PRT
   <213> Streptomyces ambofaciens
<400> 4
   Met Tyr Ala Asp Asp Ile Ala Ala Val Tyr Asp Leu Val His Glu Gly
   1               5                   10                  15
   Lys Gly Lys Asp Tyr Arg Gln Glu Ala Glu Glu Ile Ala Ala Leu Val
               20                  25                  30
   Arg Val His Arg Pro Gly Ala Arg Thr Leu Leu Asp Val Ala Cys Gly
           35                  40                  45
   Thr Gly Gln His Leu His His Leu Asp Gly Leu Phe Asp His Val Glu
       50                  55                  60
   Gly Leu Glu Leu Ser Ala Asp Met Leu Ala Leu Ala Thr Gly Arg Asn
   65                  70                  75                  80
   Pro Gly Val Thr Phe His Gln Gly Asp Met Arg Ser Phe Ser Leu Gly
                   85                  90                  95
   Arg Arg Phe Asp Ala Val Thr Cys Met Phe Ser Ser Ile Gly His Leu
               100                 105                 110
   Arg Thr Thr Asp Glu Leu Asp Ser Thr Leu Arg Ala Phe Thr Asp His
           115                 120                 125
   Leu Glu Pro Ser Gly Val Ile Val Val Glu Pro Trp Trp Phe Pro Glu
       130                 135                 140
   Ser Phe Thr Pro Gly Tyr Val Gly Ala Ser Ile Thr Glu Ala Gly Glu
   145                 150                 155                 160
   Arg Thr Val Cys Arg Val Ser His Ser Val Arg Glu Gly Asn Ala Thr
                   165                 170                 175
   Arg Ile Glu Val His Tyr Leu Leu Ala Gly Pro Gly Gly Val Arg His
               180                 185                 190
   Leu Thr Glu Asp His Thr Ile Thr Leu Phe Pro Arg Ala Asp Tyr Glu
           195                 200                 205
   Ala Ala Phe Glu Arg Ala Gly Cys Asp Val Val Tyr Gln Glu Gly Gly
       210                 215                 220
   Pro Ser Gly Arg Gly Leu Phe Ile Gly Thr Arg Arg
   225                 230                 235
<210> 5
   <211> 1272
   <212> DNA
   <213> Streptomyces ambofaciens
<220>
   <221> CDS
   <222> (1)..(1272)
<400> 5
   atg ccc acc ctt gcc acg gaa acg gcc ccc gcg agc acg agc acg agc      48
   Met Pro Thr Leu Ala Thr Glu Thr Ala Pro Ala Ser Thr Ser Thr Ser
   1               5                   10                  15
   gcg gac acg agc acg ggc gtc cgt gcg ctc ggc cgt cgg ctc cag ctg      96
   Ala Asp Thr Ser Thr Gly Val Arg Ala Leu Gly Arg Arg Leu Gln Leu
               20                  25                  30
   acc cgg gcc gca cac tgg tgc gcc ggc aac cag ggc gac ccg tac gcg     144
   Thr Arg Ala Ala His Trp Cys Ala Gly Asn Gln Gly Asp Pro Tyr Ala
           35                  40                  45
   ctg atc ctg cgc gcc gtc gcc gac ccc gag ccg ttc gaa cgg gag atc     192
   Leu Ile Leu Arg Ala Val Ala Asp Pro Glu Pro Phe Glu Arg Glu Ile
       50                  55                  60
   cgg gcc cgc gga ccg tgg ttc cgc agc gaa cag ctg gac gcc tgg gtg     240
   Arg Ala Arg Gly Pro Trp Phe Arg Ser Glu Gln Leu Asp Ala Trp Val
   65                  70                  75                  80
   acc gcg gac ccc gag gtg gcg gcg gcc gtc ctg gcc gac ccg cgc ttc     288
   Thr Ala Asp Pro Glu Val Ala Ala Ala Val Leu Ala Asp Pro Arg Phe
                   85                  90                  95
   ggc acg ctg gac cgg gcc gga cgc cgc ccg gac gag gaa ctg ctg ccc     336
   Gly Thr Leu Asp Arg Ala Gly Arg Arg Pro Asp Glu Glu Leu Leu Pro
               100                 105                 110
   ctc gcc gag gcg ttc ccc cac cac gaa cgc gcg gag ctc gta cgc ctg     384
   Leu Ala Glu Ala Phe Pro His His Glu Arg Ala Glu Leu Val Arg Leu
           115                 120                 125
   cgg gcg ctg gcc gcc ccg gtg ctc agc cgg tac gcc ccg gcc cag gcg     432
   Arg Ala Leu Ala Ala Pro Val Leu Ser Arg Tyr Ala Pro Ala Gln Ala
       130                 135                 140
   ccc tgc gcg gcg cgc acc acc gcc cgc aga gtg ctc ggc cgc ctg ctg     480
   Pro Cys Ala Ala Arg Thr Thr Ala Arg Arg Val Leu Gly Arg Leu Leu
   145                 150                 155                 160
   ccc acc ggt gac gcc ggg ttc gac ctt gtc ggc gag gtc gcc cgg ccc      528
   Pro Thr Gly Asp Ala Gly Phe Asp Leu Val Gly Glu Val Ala Arg Pro
                   165                 170                 175
   tac gcc gtc gag ctg atg ctc agg ctc ctc gga gtg ccg ggc cgc gac      576
   Tyr Ala Val Glu Leu Met Leu Arg Leu Leu Gly Val Pro Gly Arg Asp
               180                 185                 190
   cgc gcc acc gcc gcg cgg gca ctc gcc gcc tgc ggc ccc cag ctc gac      624
   Arg Ala Thr Ala Ala Arg Ala Leu Ala Ala Cys Gly Pro Gln Leu Asp
           195                 200                 205
   gcc cgg atg gcc ccg caa ctg ctg acc gtg gcc cgg gag tcc gcc gac      672
   Ala Arg Met Ala Pro Gln Leu Leu Thr Val Ala Arg Glu Ser Ala Asp
       210                 215                 220
   gcc gtc cgc aca ctg gcc gac ctg gtc ccc gag ctc gtc gcg gag aag      720
   Ala Val Arg Thr Leu Ala Asp Leu Val Pro Glu Leu Val Ala Glu Lys
   225                 230                 235                 240
   tcc cgg ggc ctc ggg aac gcc gag ccc cgg ccc gac gac gtg ctc gcc      768
   Ser Arg Gly Leu Gly Asn Ala Glu Pro Arg Pro Asp Asp Val Leu Ala
                   245                 250                 255
   ctc ctc ctg cac gac ggc gtc gcc ccc ggc gac gtc gag cgc atc gcg      816
   Leu Leu Leu His Asp Gly Val Ala Pro Gly Asp Val Glu Arg Ile Ala
               260                 265                 270
   ctg ctc ctc gcg gtc ggc gca ccc gaa ccc gtc gtc acc gcc gtc gcg      864
   Leu Leu Leu Ala Val Gly Ala Pro Glu Pro Val Val Thr Ala Val Ala
           275                 280                 285
   cac acg gtc cac cgg ctg ctc ggc cgg ccg ggg gag tgg gag agg gcc      912
   His Thr Val His Arg Leu Leu Gly Arg Pro Gly Glu Trp Glu Arg Ala
       290                 295                 300
   cgc cgg acg ccg gcc gcg gcg aac gcc gtc gac cag gtg ctg cgc gag      960
   Arg Arg Thr Pro Ala Ala Ala Asn Ala Val Asp Gln Val Leu Arg Glu
   305                 310                 315                 320
   cgc ccc ccg gcc cgg ctg gag aac cgg gtc gcg cac acc ggc ctc gaa     1008
   Arg Pro Pro Ala Arg Leu Glu Asn Arg Val Ala His Thr Gly Leu Glu
                   325                 330                 335
   ctc ggc ggc cgc cgg atc acc gcc gac gag cac gtc gtg gtg ctg gcc     1056
   Leu Gly Gly Arg Arg Ile Thr Ala Asp Glu His Val Val Val Leu Ala
               340                 345                 350
   gcc gcc gga cgg gag atc ccc ggg ccg gag ccg ctc ggg ggc gcc gac     1104
   Ala Ala Gly Arg Glu Ile Pro Gly Pro Glu Pro Leu Gly Gly Ala Asp
           355                 360                 365
   gga ccg cac ctg gcg ctc gcc ctc ccg ctg atc cgc ctg gcc gcc acc     1152
   Gly Pro His Leu Ala Leu Ala Leu Pro Leu Ile Arg Leu Ala Ala Thr
       370                 375                 380
   acc gcg gtc cag gtc acg gcc ggc cgc ctg ccc ggc ctg cgg gcc gag     1200
   Thr Ala Val Gln Val Thr Ala Gly Arg Leu Pro Gly Leu Arg Ala Glu
   385                 390                 395                 400
   gga ccg ccc ctg acc cgg ccg cgg tca ccg gtc ctg ggc gcc tgc gcc     1248
   Gly Pro Pro Leu Thr Arg Pro Arg Ser Pro Val Leu Gly Ala Cys Ala
                   405                 410                 415
   cgc ctc cgg gtc cac ccg gga tga                                     1272
   Arg Leu Arg Val His Pro Gly
   420
<210> 6
   <211> 423
   <212> PRT
   <213> Streptomyces ambofaciens
<400> 6
   Met Pro Thr Leu Ala Thr Glu Thr Ala Pro Ala Ser Thr Ser Thr Ser
   1 5 10 15
   Ala Asp Thr Ser Thr Gly Val Arg Ala Leu Gly Arg Arg Leu Gln Leu
   20 25 30
   Thr Arg Ala Ala His Trp Cys Ala Gly Asn Gln Gly Asp Pro Tyr Ala
   35 40 45
   Leu Ile Leu Arg Ala Val Ala Asp Pro Glu Pro Phe Glu Arg Glu Ile
   50 55 60
   Arg Ala Arg Gly Pro Trp Phe Arg Ser Glu Gln Leu Asp Ala Trp Val
   65 70 75 80
   Thr Ala Asp Pro Glu Val Ala Ala Ala Val Leu Ala Asp Pro Arg Phe
   85 90 95
   Gly Thr Leu Asp Arg Ala Gly Arg Arg Pro Asp Glu Glu Leu Leu Pro
   100 105 110
   Leu Ala Glu Ala Phe Pro His His Glu Arg Ala Glu Leu Val Arg Leu
   115 120 125
   Arg Ala Leu Ala Ala Pro Val Leu Ser Arg Tyr Ala Pro Ala Gln Ala
   130 135 140
   Pro Cys Ala Ala Arg Thr Thr Ala Arg Arg Val Leu Gly Arg Leu Leu
   145 150 155 160
   Pro Thr Gly Asp Ala Gly Phe Asp Leu Val Gly Glu Val Ala Arg Pro
   165 170 175
   Tyr Ala Val Glu Leu Met Leu Arg Leu Leu Gly Val Pro Gly Arg Asp
   180 185 190
   Arg Ala Thr Ala Ala Arg Ala Leu Ala Ala Cys Gly Pro Gln Leu Asp
   195 200 205
   Ala Arg Met Ala Pro Gln Leu Leu Thr Val Ala Arg Glu Ser Ala Asp
   210 215 220
   Ala Val Arg Thr Leu Ala Asp Leu Val Pro Glu Leu Val Ala Glu Lys
   225 230 235 240
   Ser Arg Gly Leu Gly Asn Ala Glu Pro Arg Pro Asp Asp Val Leu Ala
   245 250 255
   Leu Leu Leu His Asp Gly Val Ala Pro Gly Asp Val Glu Arg Ile Ala
   260 265 270
   Leu Leu Leu Ala Val Gly Ala Pro Glu Pro Val Val Thr Ala Val Ala
   275 280 285
   His Thr Val His Arg Leu Leu Gly Arg Pro Gly Glu Trp Glu Arg Ala
   290 295 300
   Arg Arg Thr Pro Ala Ala Ala Asn Ala Val Asp Gln Val Leu Arg Glu
   305 310 315 320
   Arg Pro Pro Ala Arg Leu Glu Asn Arg Val Ala His Thr Gly Leu Glu
   325 330 335
   Leu Gly Gly Arg Arg Ile Thr Ala Asp Glu His Val Val Val Leu Ala
   340 345 350
   Ala Ala Gly Arg Glu Ile Pro Gly Pro Glu Pro Leu Gly Gly Ala Asp
   355 360 365
   Gly Pro His Leu Ala Leu Ala Leu Pro Leu Ile Arg Leu Ala Ala Thr
   370 375 380
   Thr Ala Val Gln Val Thr Ala Gly Arg Leu Pro Gly Leu Arg Ala Glu
   385 390 395 400
   Gly Pro Pro Leu Thr Arg Pro Arg Ser Pro Val Leu Gly Ala Cys Ala
   405 410 415
   Arg Leu Arg Val His Pro Gly
   420
<210> 7
   <211> 1266
   <212> DNA
   <213> Streptomyces ambofaciens
<220>
   <221> CDS
   <222> (1)..(1266)
<400> 7
   gtg cgc gtc ctg ctg aca tcc ctc gcc cac aac acc cac tac tac agt 48
   Val Arg Val Leu Leu Thr Ser Leu Ala His Asn Thr His Tyr Tyr Ser
   1 5 10 15
   ctg gtg ccc ctc gcc tgg gcg ctg cgc gcc gcc ggg cac gag gta cgg 96
   Leu Val Pro Leu Ala Trp Ala Leu Arg Ala Ala Gly His Glu Val Arg
   20 25 30
   gtg gcg agc ccg ccc tcc ctc acc gac gtc atc acc tcc acc ggc ctg      144
   Val Ala Ser Pro Pro Ser Leu Thr Asp Val Ile Thr Ser Thr Gly Leu
           35                  40                  45
   acc gcc gta ccg gtg ggc gac gac cga ccg gcc gcg gag ctg ctc gcc      192
   Thr Ala Val Pro Val Gly Asp Asp Arg Pro Ala Ala Glu Leu Leu Ala
       50                  55                  60
   gag atg ggc agc gac ctc gtc ccc tac cag cgg ggc ttc gag ttc ggt      240
   Glu Met Gly Ser Asp Leu Val Pro Tyr Gln Arg Gly Phe Glu Phe Gly
   65                  70                  75                  80
   gag gtg gag agc gag gag gag acc acc tgg gag tac ctg ctc ggc cag      288
   Glu Val Glu Ser Glu Glu Glu Thr Thr Trp Glu Tyr Leu Leu Gly Gln
                   85                  90                  95
   cag agc atg atg gcc gcc ctg tgc ttc gcc ccg ttc aac ggc gcc gcc      336
   Gln Ser Met Met Ala Ala Leu Cys Phe Ala Pro Phe Asn Gly Ala Ala
               100                 105                 110
   acg atg gac gag atc gtc gac ttc gcc cgt ggc tgg cgg ccc gac ctg      384
   Thr Met Asp Glu Ile Val Asp Phe Ala Arg Gly Trp Arg Pro Asp Leu
           115                 120                 125
   gtc gtg tgg gaa ccc tgg acc tac gcg ggg ccg gtc gcg gcc cgc gcc      432
   Val Val Trp Glu Pro Trp Thr Tyr Ala Gly Pro Val Ala Ala Arg Ala
       130                 135                 140
   tgc ggt gcc gcc cac gcg cgc atc ctg tgg ggc ccc gac gcc atc gga      480
   Cys Gly Ala Ala His Ala Arg Ile Leu Trp Gly Pro Asp Ala Ile Gly
   145                 150                 155                 160
   cgc tcc cgc ctg cgc ttc ctc gcc gcc ctc gac ggg atg ccg gag gaa      528
   Arg Ser Arg Leu Arg Phe Leu Ala Ala Leu Asp Gly Met Pro Glu Glu
                   165                 170                 175
   ctg cgc gag gac ccc atc gcc gaa tgg ctc ggc tgg acg ctg gac cgg      576
   Leu Arg Glu Asp Pro Ile Ala Glu Trp Leu Gly Trp Thr Leu Asp Arg
               180                 185                 190
   tac ggg ctc gcc ttc gac gaa cgc gac gtg ctc ggc cac tgg gtg atc      624
   Tyr Gly Leu Ala Phe Asp Glu Arg Asp Val Leu Gly His Trp Val Ile
           195                 200                 205
   gac ccg ggc ccg cgc agc acc cgc ctc gac ctg ggc cag acc acg gtg      672
   Asp Pro Gly Pro Arg Ser Thr Arg Leu Asp Leu Gly Gln Thr Thr Val
       210                 215                 220
   ccc atg tgc tac gtg ccc tac aac ggg cgc tcc gtg atc gaa ccc tgg      720
   Pro Met Cys Tyr Val Pro Tyr Asn Gly Arg Ser Val Ile Glu Pro Trp
   225                 230                 235                 240
   ctc ggc aag aag ccc gac cgc ccc cgc gtc tgc ctc acc ctc ggc gtg      768
   Leu Gly Lys Lys Pro Asp Arg Pro Arg Val Cys Leu Thr Leu Gly Val
                   245                 250                 255
   tcc gcc cgc gag acc tac ggc cgc gac gcg gtc tcc tac tcc gaa ctg      816
   Ser Ala Arg Glu Thr Tyr Gly Arg Asp Ala Val Ser Tyr Ser Glu Leu
               260                 265                 270
   ctg gag gcg ctc ggc cgg ctg gac atc gag gtg gtg gcc acc ctg gac      864
   Leu Glu Ala Leu Gly Arg Leu Asp Ile Glu Val Val Ala Thr Leu Asp
           275                 280                 285
   acc tcc cag ctc gac cgc ctg ggc acc ctc ccc gac aac gtc gtg ccc      912
   Thr Ser Gln Leu Asp Arg Leu Gly Thr Leu Pro Asp Asn Val Val Pro
       290                 295                 300
   gtg gac ttc gta ccg ctc gac gcg ctg ctg ccg agc tgc gcc gcg atc     960
   Val Asp Phe Val Pro Leu Asp Ala Leu Leu Pro Ser Cys Ala Ala Ile
   305                 310                 315                 320
   atc cac cac ggc gga gcg ggc acc tgg tcc acc gcc ctg cgc cac gga    1008
   Ile His His Gly Gly Ala Gly Thr Trp Ser Thr Ala Leu Arg His Gly
                   325                 330                 335
   gtg ccg cag atc ctg ctg ccc tcc ctg tgg gac gcg ccg ctc aag gcg    1056
   Val Pro Gln Ile Leu Leu Pro Ser Leu Trp Asp Ala Pro Leu Lys Ala
               340                 345                 350
   cag caa ctc cag cgc ctg tcc gcc ggg ttc gac ctg ccc gcg gcg acg    1104
   Gln Gln Leu Gln Arg Leu Ser Ala Gly Phe Asp Leu Pro Ala Ala Thr
           355                 360                 365
   ctc acg gcg cgc ggt ctg gcc gac gcg gtg cac acg gcc gtg cac gac    1152
   Leu Thr Ala Arg Gly Leu Ala Asp Ala Val His Thr Ala Val His Asp
       370                 375                 380
   ccc gcg atc agg gcg ggc gcg cgg cgg ctg agc gag gag atg ctc gcc    1200
   Pro Ala Ile Arg Ala Gly Ala Arg Arg Leu Ser Glu Glu Met Leu Ala
   385                 390                 395                 400
   gac ccc acg ccc gcc ggg atc atc ccc acc ctg gag cgc ctc acc gcc    1248
   Asp Pro Thr Pro Ala Gly Ile Ile Pro Thr Leu Glu Arg Leu Thr Ala
                   405                 410                 415
   ctg cac cgg gcc acc tga                                            1266
   Leu His Arg Ala Thr
               420
<210> 8
   <211> 421
   <212> PRT
   <213> Streptomyces ambofaciens
<400> 8
   Val Arg Val Leu Leu Thr Ser Leu Ala His Asn Thr His Tyr Tyr Ser
   1               5                   10                  15
   Leu Val Pro Leu Ala Trp Ala Leu Arg Ala Ala Gly His Glu Val Arg
               20                  25                  30
   Val Ala Ser Pro Pro Ser Leu Thr Asp Val Ile Thr Ser Thr Gly Leu
           35                  40                  45
   Thr Ala Val Pro Val Gly Asp Asp Arg Pro Ala Ala Glu Leu Leu Ala
       50                  55                  60
   Glu Met Gly Ser Asp Leu Val Pro Tyr Gln Arg Gly Phe Glu Phe Gly
   65                  70                  75                  80
   Glu Val Glu Ser Glu Glu Glu Thr Thr Trp Glu Tyr Leu Leu Gly Gln
                   85                  90                  95
   Gln Ser Met Met Ala Ala Leu Cys Phe Ala Pro Phe Asn Gly Ala Ala
               100                 105                 110
   Thr Met Asp Glu Ile Val Asp Phe Ala Arg Gly Trp Arg Pro Asp Leu
   115 120 125
   Val Val Trp Glu Pro Trp Thr Tyr Ala Gly Pro Val Ala Ala Arg Ala
   130 135 140
   Cys Gly Ala Ala His Ala Arg Ile Leu Trp Gly Pro Asp Ala Ile Gly
   145 150 155 160
   Arg Ser Arg Leu Arg Phe Leu Ala Ala Leu Asp Gly Met Pro Glu Glu
   165 170 175
   Leu Arg Glu Asp Pro Ile Ala Glu Trp Leu Gly Trp Thr Leu Asp Arg
   180 185 190
   Tyr Gly Leu Ala Phe Asp Glu Arg Asp Val Leu Gly His Trp Val Ile
   195 200 205
   Asp Pro Gly Pro Arg Ser Thr Arg Leu Asp Leu Gly Gln Thr Thr Val
   210 215 220
   Pro Met Cys Tyr Val Pro Tyr Asn Gly Arg Ser Val Ile Glu Pro Trp
   225 230 235 240
   Leu Gly Lys Lys Pro Asp Arg Pro Arg Val Cys Leu Thr Leu Gly Val
   245 250 255
   Ser Ala Arg Glu Thr Tyr Gly Arg Asp Ala Val Ser Tyr Ser Glu Leu
   260 265 270
   Leu Glu Ala Leu Gly Arg Leu Asp Ile Glu Val Val Ala Thr Leu Asp
   275 280 285
   Thr Ser Gln Leu Asp Arg Leu Gly Thr Leu Pro Asp Asn Val Val Pro
   290 295 300
   Val Asp Phe Val Pro Leu Asp Ala Leu Leu Pro Ser Cys Ala Ala Ile
   305 310 315 320
   Ile His His Gly Gly Ala Gly Thr Trp Ser Thr Ala Leu Arg His Gly
   325 330 335
   Val Pro Gln Ile Leu Leu Pro Ser Leu Trp Asp Ala Pro Leu Lys Ala
   340 345 350
   Gln Gln Leu Gln Arg Leu Ser Ala Gly Phe Asp Leu Pro Ala Ala Thr
   355 360 365
   Leu Thr Ala Arg Gly Leu Ala Asp Ala Val His Thr Ala Val His Asp
   370 375 380
   Pro Ala Ile Arg Ala Gly Ala Arg Arg Leu Ser Glu Glu Met Leu Ala
   385                 390                 395                 400
   Asp Pro Thr Pro Ala Gly Ile Ile Pro Thr Leu Glu Arg Leu Thr Ala
                   405                 410                 415
   Leu His Arg Ala Thr
               420
<210> 9
   <211> 1350
   <212> DNA
   <213> Streptomyces ambofaciens
<220>
   <221> CDS
   <222> (1)..(1350)
<400> 9
   atg ccc gaa agc cat gcg cag agc gcg ctg ctc gcc gcg atc acc gcc      48
   Met Pro Glu Ser His Ala Gln Ser Ala Leu Leu Ala Ala Ile Thr Ala
   1               5                   10                  15
   gcg gac cgc acc ccc gag gac atc gcg gcg ctg ccc ctg ccc gag tcc      96
   Ala Asp Arg Thr Pro Glu Asp Ile Ala Ala Leu Pro Leu Pro Glu Ser
               20                  25                  30
   ttc cgc gcc gtg acc gtc cgc aaa gag gac acc gac atg ttc cgc ggg     144
   Phe Arg Ala Val Thr Val Arg Lys Glu Asp Thr Asp Met Phe Arg Gly
           35                  40                  45
   atg ccg agc gcg gac aag gac ccg cgc aag tcc ctg cac gtc gac gag     192
   Met Pro Ser Ala Asp Lys Asp Pro Arg Lys Ser Leu His Val Asp Glu
       50                  55                  60
   gtg ccg ctg ccc gaa gtg ggg ccc ggg gag gcg ctg gtc gcg gtg atg     240
   Val Pro Leu Pro Glu Val Gly Pro Gly Glu Ala Leu Val Ala Val Met
   65                  70                  75                  80
   gcc agc tcc gtc aac tac aac acg gtc tgg tcc tcc atc ttc gag ccc     288
   Ala Ser Ser Val Asn Tyr Asn Thr Val Trp Ser Ser Ile Phe Glu Pro
                   85                  90                  95
   ctc ccg acg ttc ggc ttc ctg gag cgc tac ggg cgc acc tcg ccg ctg     336
   Leu Pro Thr Phe Gly Phe Leu Glu Arg Tyr Gly Arg Thr Ser Pro Leu
               100                 105                 110
   gcc gcg cgc cac gac ctg ccg tac cac atc ctc ggt tcc gac ctg tcc     384
   Ala Ala Arg His Asp Leu Pro Tyr His Ile Leu Gly Ser Asp Leu Ser
           115                 120                 125
   ggc gtg gtg ctg cgc acg gga ccc ggc gtg aac gtc tgg aaa ccc ggt     432
   Gly Val Val Leu Arg Thr Gly Pro Gly Val Asn Val Trp Lys Pro Gly
       130                 135                 140
   gac gag gtc gtc gcg cac tgt ctg tcg gtg gag ctg gag agc ccc gac     480
   Asp Glu Val Val Ala His Cys Leu Ser Val Glu Leu Glu Ser Pro Asp
   145                 150                 155                 160
   ggg cac gac gac acc atg ctc gac ccg gcc cag cgc atc tgg ggc ttc     528
   Gly His Asp Asp Thr Met Leu Asp Pro Ala Gln Arg Ile Trp Gly Phe
                   165                 170                 175
   gag acc aac ttc ggg ggc ctg gcc gag gtg gcc ctc gtc aag tcc aac      576
   Glu Thr Asn Phe Gly Gly Leu Ala Glu Val Ala Leu Val Lys Ser Asn
               180                 185                 190
   cag ctg atg ccc aag gcg gcc cat ctg acc tgg gag gag gcc gcc gcg      624
   Gln Leu Met Pro Lys Ala Ala His Leu Thr Trp Glu Glu Ala Ala Ala
           195                 200                 205
   ccc ggt ctg gtc aac tcc acc gcc tac cgc cag ctg gtc tcc cgc aac      672
   Pro Gly Leu Val Asn Ser Thr Ala Tyr Arg Gln Leu Val Ser Arg Asn
       210                 215                 220
   ggc gcc ggc atg aag cag ggc gac aac gtg ctg atc tgg ggc gcc agc      720
   Gly Ala Gly Met Lys Gln Gly Asp Asn Val Leu Ile Trp Gly Ala Ser
   225                 230                 235                 240
   ggc ggc ctg ggc tcg tac gcc acc cag ctg gcc ctc gcc ggc ggg gcc      768
   Gly Gly Leu Gly Ser Tyr Ala Thr Gln Leu Ala Leu Ala Gly Gly Ala
                   245                 250                 255
   cac ccc gtc tgc gtg gtc tcc agc gcg cgc aag gcc gag gtg tgc cgg      816
   His Pro Val Cys Val Val Ser Ser Ala Arg Lys Ala Glu Val Cys Arg
               260                 265                 270
   gcc atg ggc gcg gag gcc atc atc gac cgc tcc gcc gag gac tac cgc      864
   Ala Met Gly Ala Glu Ala Ile Ile Asp Arg Ser Ala Glu Asp Tyr Arg
           275                 280                 285
   ttc tgg agc gac gag gac acc cag gac ccg cgg gag tgg aag cgc ttc      912
   Phe Trp Ser Asp Glu Asp Thr Gln Asp Pro Arg Glu Trp Lys Arg Phe
       290                 295                 300
   ggc aag cgc atc cgc gag gtc acc ggg ggc gag gac gtc gac atc gtc      960
   Gly Lys Arg Ile Arg Glu Val Thr Gly Gly Glu Asp Val Asp Ile Val
   305                 310                 315                 320
   ttc gag cac ccc ggc cgg gag acc ttc ggg gcg tcc gtc tac gtc acc     1008
   Phe Glu His Pro Gly Arg Glu Thr Phe Gly Ala Ser Val Tyr Val Thr
                   325                 330                 335
   cgc agg ggc ggc acg atc gtc acc tgc gcc tcc acc tcg ggc ttc cgc     1056
   Arg Arg Gly Gly Thr Ile Val Thr Cys Ala Ser Thr Ser Gly Phe Arg
               340                 345                 350
   cac gag ttc gac aac cgc tac ctc tgg atg cac ctc aag cgt atc gtc     1104
   His Glu Phe Asp Asn Arg Tyr Leu Trp Met His Leu Lys Arg Ile Val
           355                 360                 365
   ggc acc cac ttc gcc aac tac cgc gag gcg tgg gag gcg aac cgg ctc     1152
   Gly Thr His Phe Ala Asn Tyr Arg Glu Ala Trp Glu Ala Asn Arg Leu
       370                 375                 380
   gtc gcc aag ggg agg atc cac ccc acc ctc tcg cgc acc tac ccg ctg     1200
   Val Ala Lys Gly Arg Ile His Pro Thr Leu Ser Arg Thr Tyr Pro Leu
   385                 390                 395                 400
   gcc gac acg gcg gtt gcc gtg cac gac gtg cac ggc aac cag cac cag     1248
   Ala Asp Thr Ala Val Ala Val His Asp Val His Gly Asn Gln His Gln
                   405                 410                 415
   ggc aag gtc ggc gtg ctc tgc ctg gcg ccc acg gag ggg ctg ggc gtg     1296
   Gly Lys Val Gly Val Leu Cys Leu Ala Pro Thr Glu Gly Leu Gly Val
               420                 425                 430
   cgc gac gag gag aag cgg gcg cgg cac atc gac gcg atc aac cgg ttc     1344
   Arg Asp Glu Glu Lys Arg Ala Arg His Ile Asp Ala Ile Asn Arg Phe
           435                 440                 445
   cgc tga 1350
   Arg
<210> 10
   <211> 449
   <212> PRT
   <213> Streptomyces ambofaciens
<400> 10
   Met Pro Glu Ser His Ala Gln Ser Ala Leu Leu Ala Ala Ile Thr Ala
   1 5 10 15
   Ala Asp Arg Thr Pro Glu Asp Ile Ala Ala Leu Pro Leu Pro Glu Ser
   20 25 30
   Phe Arg Ala Val Thr Val Arg Lys Glu Asp Thr Asp Met Phe Arg Gly
   35 40 45
   Met Pro Ser Ala Asp Lys Asp Pro Arg Lys Ser Leu His Val Asp Glu
   50 55 60
   Val Pro Leu Pro Glu Val Gly Pro Gly Glu Ala Leu Val Ala Val Met
   65 70 75 80
   Ala Ser Ser Val Asn Tyr Asn Thr Val Trp Ser Ser Ile Phe Glu Pro
   85 90 95
   Leu Pro Thr Phe Gly Phe Leu Glu Arg Tyr Gly Arg Thr Ser Pro Leu
   100 105 110
   Ala Ala Arg His Asp Leu Pro Tyr His Ile Leu Gly Ser Asp Leu Ser
   115 120 125
   Gly Val Val Leu Arg Thr Gly Pro Gly Val Asn Val Trp Lys Pro Gly
   130 135 140
   Asp Glu Val Val Ala His Cys Leu Ser Val Glu Leu Glu Ser Pro Asp
   145 150 155 160
   Gly His Asp Asp Thr Met Leu Asp Pro Ala Gln Arg Ile Trp Gly Phe
   165 170 175
   Glu Thr Asn Phe Gly Gly Leu Ala Glu Val Ala Leu Val Lys Ser Asn
   180 185 190
   Gln Leu Met Pro Lys Ala Ala His Leu Thr Trp Glu Glu Ala Ala Ala
   195 200 205
   Pro Gly Leu Val Asn Ser Thr Ala Tyr Arg Gln Leu Val Ser Arg Asn
   210 215 220
   Gly Ala Gly Met Lys Gln Gly Asp Asn Val Leu Ile Trp Gly Ala Ser
   225                 230                 235                 240
   Gly Gly Leu Gly Ser Tyr Ala Thr Gln Leu Ala Leu Ala Gly Gly Ala
                   245                 250                 255
   His Pro Val Cys Val Val Ser Ser Ala Arg Lys Ala Glu Val Cys Arg
               260                 265                 270
   Ala Met Gly Ala Glu Ala Ile Ile Asp Arg Ser Ala Glu Asp Tyr Arg
           275                 280                 285
   Phe Trp Ser Asp Glu Asp Thr Gln Asp Pro Arg Glu Trp Lys Arg Phe
       290                 295                 300
   Gly Lys Arg Ile Arg Glu Val Thr Gly Gly Glu Asp Val Asp Ile Val
   305                 310                 315                 320
   Phe Glu His Pro Gly Arg Glu Thr Phe Gly Ala Ser Val Tyr Val Thr
                   325                 330                 335
   Arg Arg Gly Gly Thr Ile Val Thr Cys Ala Ser Thr Ser Gly Phe Arg
               340                 345                 350
   His Glu Phe Asp Asn Arg Tyr Leu Trp Met His Leu Lys Arg Ile Val
           355                 360                 365
   Gly Thr His Phe Ala Asn Tyr Arg Glu Ala Trp Glu Ala Asn Arg Leu
       370                 375                 380
   Val Ala Lys Gly Arg Ile His Pro Thr Leu Ser Arg Thr Tyr Pro Leu
   385                 390                 395                 400
   Ala Asp Thr Ala Val Ala Val His Asp Val His Gly Asn Gln His Gln
                   405                 410                 415
   Gly Lys Val Gly Val Leu Cys Leu Ala Pro Thr Glu Gly Leu Gly Val
               420                 425                 430
   Arg Asp Glu Glu Lys Arg Ala Arg His Ile Asp Ala Ile Asn Arg Phe
           435                 440                 445
   Arg
<210> 11
   <211> 675
   <212> DNA
   <213> Streptomyces ambofaciens
<220>
   <221> CDS
   <222> (1)..(675)
<400> 11
   atg atg agc gtg gca gac cag acg gct ctc agc ccc gcg ctg ctg gag 48
   Met Met Ser Val Ala Asp Gln Thr Ala Leu Ser Pro Ala Leu Leu Glu
   1 5 10 15
   tac gcc cgg agc gtc gcg ctg cgg gac gac ggc ctg ctg cgc gaa ctg 96
   Tyr Ala Arg Ser Val Ala Leu Arg Asp Asp Gly Leu Leu Arg Glu Leu
   20 25 30
   cac gag gtg acc gcc ggg ctc ccc ggc ggc cgg gcc atg cag atc atg 144
   His Glu Val Thr Ala Gly Leu Pro Gly Gly Arg Ala Met Gln Ile Met
   35 40 45
   ccc gag gag gcg cag ttc ctc gcc ctg ctg atc cgg ctc gtc ggt gcc 192
   Pro Glu Glu Ala Gln Phe Leu Ala Leu Leu Ile Arg Leu Val Gly Ala
   50 55 60
   cgg cgg gtg ctg gag atc ggc acc ttc acg ggg tac agc acg ctg tgc 240
   Arg Arg Val Leu Glu Ile Gly Thr Phe Thr Gly Tyr Ser Thr Leu Cys
   65 70 75 80
   atg gcg cgg gca ctg ccc gcc gac ggc acc gtc gtc acc tgc gac atc 288
   Met Ala Arg Ala Leu Pro Ala Asp Gly Thr Val Val Thr Cys Asp Ile
   85 90 95
   agc gac agg tgg ccc ggc gtc ggc gca ccg tac tgg cgc cgg gcc ggg 336
   Ser Asp Arg Trp Pro Gly Val Gly Ala Pro Tyr Trp Arg Arg Ala Gly
   100 105 110
   gtg gag tcc cgg atc gac ctg cgc gtc ggc gac gcc gtc cgg acc ctc 384
   Val Glu Ser Arg Ile Asp Leu Arg Val Gly Asp Ala Val Arg Thr Leu
   115 120 125
   gcc gag ctc cgc gag cac gag ggg gac ggc tcg ttc gac ctg gtc ttc 432
   Ala Glu Leu Arg Glu His Glu Gly Asp Gly Ser Phe Asp Leu Val Phe
   130 135 140
   gtc gac gcc gac aag acc ggg tac ccg cac tac tac gag cag gcg ctg 480
   Val Asp Ala Asp Lys Thr Gly Tyr Pro His Tyr Tyr Glu Gln Ala Leu
   145 150 155 160
   gcc ctg gta cgc ccc ggc gga ctg gtg gcg gtc gac aac acc ctg ttc 528
   Ala Leu Val Arg Pro Gly Gly Leu Val Ala Val Asp Asn Thr Leu Phe
   165 170 175
   ttc ggc cgg gtg gcc gac ccg gcc gtc gag gac gcc gac acc gtc gcc 576
   Phe Gly Arg Val Ala Asp Pro Ala Val Glu Asp Ala Asp Thr Val Ala
   180 185 190
   gtg cgc gcg ctc aac gag ctg ctg cgc gac gac gaa cgc gtg gac atc 624
   Val Arg Ala Leu Asn Glu Leu Leu Arg Asp Asp Glu Arg Val Asp Ile
   195 200 205
   gcc ctg ctg acg gtc gcc gac ggg atc act ctg gcc cgc cgg cgg gag 672
   Ala Leu Leu Thr Val Ala Asp Gly Ile Thr Leu Ala Arg Arg Arg Glu
   210 215 220
   tga 675
<210> 12
   <211> 224
   <212> PRT
   <213> Streptomyces ambofaciens
<400> 12
   Met Met Ser Val Ala Asp Gln Thr Ala Leu Ser Pro Ala Leu Leu Glu
   1 5 10 15
   Tyr Ala Arg Ser Val Ala Leu Arg Asp Asp Gly Leu Leu Arg Glu Leu
   20 25 30
   His Glu Val Thr Ala Gly Leu Pro Gly Gly Arg Ala Met Gln Ile Met
   35 40 45
   Pro Glu Glu Ala Gln Phe Leu Ala Leu Leu Ile Arg Leu Val Gly Ala
   50 55 60
   Arg Arg Val Leu Glu Ile Gly Thr Phe Thr Gly Tyr Ser Thr Leu Cys
   65 70 75 80
   Met Ala Arg Ala Leu Pro Ala Asp Gly Thr Val Val Thr Cys Asp Ile
   85 90 95
   Ser Asp Arg Trp Pro Gly Val Gly Ala Pro Tyr Trp Arg Arg Ala Gly
   100 105 110
   Val Glu Ser Arg Ile Asp Leu Arg Val Gly Asp Ala Val Arg Thr Leu
   115 120 125
   Ala Glu Leu Arg Glu His Glu Gly Asp Gly Ser Phe Asp Leu Val Phe
   130 135 140
   Val Asp Ala Asp Lys Thr Gly Tyr Pro His Tyr Tyr Glu Gln Ala Leu
   145 150 155 160
   Ala Leu Val Arg Pro Gly Gly Leu Val Ala Val Asp Asn Thr Leu Phe
   165 170 175
   Phe Gly Arg Val Ala Asp Pro Ala Val Glu Asp Ala Asp Thr Val Ala
   180 185 190
   Val Arg Ala Leu Asn Glu Leu Leu Arg Asp Asp Glu Arg Val Asp Ile
   195 200 205
   Ala Leu Leu Thr Val Ala Asp Gly Ile Thr Leu Ala Arg Arg Arg Glu
   210 215 220
<210> 13
   <211> 1245
   <212> DNA
   <213> Streptomyces ambofaciens
<220>
   <221> CDS
   <222> (1)..(1245)
<400> 13
   gtg ctc cag cgc gtc gat ctg tcg tca ctc acc ggc ctc cgc tgg tat      48
   Val Leu Gln Arg Val Asp Leu Ser Ser Leu Thr Gly Leu Arg Trp Tyr
   1               5                   10                  15
   gcg gcg ctg acg gta ttc gcc tgt cac atc gcc cag cag ggc ttc ttc      96
   Ala Ala Leu Thr Val Phe Ala Cys His Ile Ala Gln Gln Gly Phe Phe
               20                  25                  30
   gcc gac cag cag gtg ggc agc gca ctg ctg cac atc acc ccg ctc ggt     144
   Ala Asp Gln Gln Val Gly Ser Ala Leu Leu His Ile Thr Pro Leu Gly
           35                  40                  45
   tcc atg gcg gtc tcg atc ttc ttc ata ctg agt gga ttc gtc ctc gcc     192
   Ser Met Ala Val Ser Ile Phe Phe Ile Leu Ser Gly Phe Val Leu Ala
       50                  55                  60
   tgg tcg gcc cgc gac gag gac tcc gtg ccg act ttc tgg cgg cgc cgc     240
   Trp Ser Ala Arg Asp Glu Asp Ser Val Pro Thr Phe Trp Arg Arg Arg
   65                  70                  75                  80
   atc gcg aag atc tat ccg ctg cat ctc gcg acg ttc ggc atc gcg gct     288
   Ile Ala Lys Ile Tyr Pro Leu His Leu Ala Thr Phe Gly Ile Ala Ala
                   85                  90                  95
   ctc atc att ttc tcc ctg tcg gag ccg gta ctt ccc ggc ggt tcc gta     336
   Leu Ile Ile Phe Ser Leu Ser Glu Pro Val Leu Pro Gly Gly Ser Val
               100                 105                 110
   tgg gac ggg ctg gtg ccc aat gtt ctg ctc gtg cag tcc tgg ctt ccc     384
   Trp Asp Gly Leu Val Pro Asn Val Leu Leu Val Gln Ser Trp Leu Pro
           115                 120                 125
   gac gcg acc ctc acg gcc agt ttc aac acg ccc agc tgg tcg ctc tcc     432
   Asp Ala Thr Leu Thr Ala Ser Phe Asn Thr Pro Ser Trp Ser Leu Ser
       130                 135                 140
   tgt gag atc gcc ttc tat ctg tcg ttc ccg ctg tgg tac cgg ctg gtg     480
   Cys Glu Ile Ala Phe Tyr Leu Ser Phe Pro Leu Trp Tyr Arg Leu Val
   145                 150                 155                 160
   cgc agg att ccc gca cgg cgg ctg tgg tgg tgc gcc gcg ggg atc gcc     528
   Arg Arg Ile Pro Ala Arg Arg Leu Trp Trp Cys Ala Ala Gly Ile Ala
                   165                 170                 175
   gtg gcc gtg acg tgt gtg ccc ctg ctg gcg ggc ctg ctc ccg gcg agc     576
   Val Ala Val Thr Cys Val Pro Leu Leu Ala Gly Leu Leu Pro Ala Ser
               180                 185                 190
   gag gag gtg gcc ccc ggg atg tcg ctc aac gag gtc tgg ttc gcg tac     624
   Glu Glu Val Ala Pro Gly Met Ser Leu Asn Glu Val Trp Phe Ala Tyr
           195                 200                 205
   tgg ctt ccg ccg gtg cgc atg ctg gag ttc gtc ctc ggc atc gtg atg     672
   Trp Leu Pro Pro Val Arg Met Leu Glu Phe Val Leu Gly Ile Val Met
       210                 215                 220
   gcg ctg atc ctg cgc gcg ggg atc tgg aag ggc ccc ggt ccg gcg gtc     720
   Ala Leu Ile Leu Arg Ala Gly Ile Trp Lys Gly Pro Gly Pro Ala Val
   225                 230                 235                 240
   tgc acg gcg ctc ctc gcc gcg agt tac ggc ctc acc cag atg gtg ccc     768
   Cys Thr Ala Leu Leu Ala Ala Ser Tyr Gly Leu Thr Gln Met Val Pro
                   245                 250                 255
   ccg atc ttc acc ctc gtc gcc tgc tcc gtc gta ccg gcc gcg ctg ctg     816
   Pro Ile Phe Thr Leu Val Ala Cys Ser Val Val Pro Ala Ala Leu Leu
               260                 265                 270
   atc acg gcg ctg gcc gac gcc gac gtg cac ggc cgg cgc acg ggg ctg      864
   Ile Thr Ala Leu Ala Asp Ala Asp Val His Gly Arg Arg Thr Gly Leu
           275                 280                 285
   cgt tcg gcg acg ctg gtg cgg ctg ggc cag tgg tcc ttc gcc ttc tac      912
   Arg Ser Ala Thr Leu Val Arg Leu Gly Gln Trp Ser Phe Ala Phe Tyr
       290                 295                 300
   ctg gtc cac ttc ctg atc atc cgc tac gga cac cgg ctg atg ggc ggc      960
   Leu Val His Phe Leu Ile Ile Arg Tyr Gly His Arg Leu Met Gly Gly
   305                 310                 315                 320
   gat ctg ggc tac gag cgg cag tgg agc acc ccg gcc gcg atc gcg ctg     1008
   Asp Leu Gly Tyr Glu Arg Gln Trp Ser Thr Pro Ala Ala Ile Ala Leu
                   325                 330                 335
   tcc ctg ggg atg ctg ggg gtg gcg gtc ctg gcc ggc ggt ctg ctg cac     1056
   Ser Leu Gly Met Leu Gly Val Ala Val Leu Ala Gly Gly Leu Leu His
               340                 345                 350
   acc gtc gtc gaa cag ccc tgc atg cgc ctg ttc ggc agc cgc agg tcc     1104
   Thr Val Val Glu Gln Pro Cys Met Arg Leu Phe Gly Ser Arg Arg Ser
           355                 360                 365
   gcc tcc cgt ccg aag ccc ggc gcc acc gcg gct ccc cgg aac tca ccc     1152
   Ala Ser Arg Pro Lys Pro Gly Ala Thr Ala Ala Pro Arg Asn Ser Pro
       370                 375                 380
   gcg gcc gac gcg gcc ggc gtg ccc ctg ctc ccg ggc gta ccc ggg ccc     1200
   Ala Ala Asp Ala Ala Gly Val Pro Leu Leu Pro Gly Val Pro Gly Pro
   385                 390                 395                 400
   gcg cac acc ccc gca gcg acg aac gaa ccc acc ccg aga gga tga         1245
   Ala His Thr Pro Ala Ala Thr Asn Glu Pro Thr Pro Arg Gly
                   405                 410
<210> 14
   <211> 414
   <212> PRT
   <213> Streptomyces ambofaciens
<400> 14
   Val Leu Gln Arg Val Asp Leu Ser Ser Leu Thr Gly Leu Arg Trp Tyr
   1               5                   10                  15
   Ala Ala Leu Thr Val Phe Ala Cys His Ile Ala Gln Gln Gly Phe Phe
               20                  25                  30
   Ala Asp Gln Gln Val Gly Ser Ala Leu Leu His Ile Thr Pro Leu Gly
           35                  40                  45
   Ser Met Ala Val Ser Ile Phe Phe Ile Leu Ser Gly Phe Val Leu Ala
       50                  55                  60
   Trp Ser Ala Arg Asp Glu Asp Ser Val Pro Thr Phe Trp Arg Arg Arg
   65                  70                  75                  80
   Ile Ala Lys Ile Tyr Pro Leu His Leu Ala Thr Phe Gly Ile Ala Ala
                   85                  90                  95
   Leu Ile Ile Phe Ser Leu Ser Glu Pro Val Leu Pro Gly Gly Ser Val
               100                 105                 110
   Trp Asp Gly Leu Val Pro Asn Val Leu Leu Val Gln Ser Trp Leu Pro
           115                 120                 125
   Asp Ala Thr Leu Thr Ala Ser Phe Asn Thr Pro Ser Trp Ser Leu Ser
       130                 135                 140
   Cys Glu Ile Ala Phe Tyr Leu Ser Phe Pro Leu Trp Tyr Arg Leu Val
   145                 150                 155                 160
   Arg Arg Ile Pro Ala Arg Arg Leu Trp Trp Cys Ala Ala Gly Ile Ala
                   165                 170                 175
   Val Ala Val Thr Cys Val Pro Leu Leu Ala Gly Leu Leu Pro Ala Ser
               180                 185                 190
   Glu Glu Val Ala Pro Gly Met Ser Leu Asn Glu Val Trp Phe Ala Tyr
           195                 200                 205
   Trp Leu Pro Pro Val Arg Met Leu Glu Phe Val Leu Gly Ile Val Met
       210                 215                 220
   Ala Leu Ile Leu Arg Ala Gly Ile Trp Lys Gly Pro Gly Pro Ala Val
   225                 230                 235                 240
   Cys Thr Ala Leu Leu Ala Ala Ser Tyr Gly Leu Thr Gln Met Val Pro
                   245                 250                 255
   Pro Ile Phe Thr Leu Val Ala Cys Ser Val Val Pro Ala Ala Leu Leu
               260                 265                 270
   Ile Thr Ala Leu Ala Asp Ala Asp Val His Gly Arg Arg Thr Gly Leu
           275                 280                 285
   Arg Ser Ala Thr Leu Val Arg Leu Gly Gln Trp Ser Phe Ala Phe Tyr
       290                 295                 300
   Leu Val His Phe Leu Ile Ile Arg Tyr Gly His Arg Leu Met Gly Gly
   305                 310                 315                 320
   Asp Leu Gly Tyr Glu Arg Gln Trp Ser Thr Pro Ala Ala Ile Ala Leu
                   325                 330                 335
   Ser Leu Gly Met Leu Gly Val Ala Val Leu Ala Gly Gly Leu Leu His
               340                 345                 350
   Thr Val Val Glu Gln Pro Cys Met Arg Leu Phe Gly Ser Arg Arg Ser
           355                 360                 365
   Ala Ser Arg Pro Lys Pro Gly Ala Thr Ala Ala Pro Arg Asn Ser Pro
       370                 375                 380
   Ala Ala Asp Ala Ala Gly Val Pro Leu Leu Pro Gly Val Pro Gly Pro
   385                 390                 395                 400
   Ala His Thr Pro Ala Ala Thr Asn Glu Pro Thr Pro Arg Gly
                   405                 410
<210> 15
   <211> 849
   <212> DNA
   <213> Streptomyces ambofaciens
<220>
   <221> CDS
   <222> (1)..(849)
<400> 15
   gtg aca ttg aaa tcc cca ctg cca ccg caa tcc gtc tcc gca ccc gct      48
   Val Thr Leu Lys Ser Pro Leu Pro Pro Gln Ser Val Ser Ala Pro Ala
   1               5                   10                  15
   gat tca cgc agc acc gcg cgg aga gaa tgg ggt cag aac ttc ttt cgt      96
   Asp Ser Arg Ser Thr Ala Arg Arg Glu Trp Gly Gln Asn Phe Phe Arg
               20                  25                  30
   aca gcc gcc gcg gcc tgt cgt ttc tcc gct cag ctg gac ggt tcg gac     144
   Thr Ala Ala Ala Ala Cys Arg Phe Ser Ala Gln Leu Asp Gly Ser Asp
           35                  40                  45
   acc att ccg ccc gat tct ccg aat gac ctg atg acc gtc gaa ata ggc     192
   Thr Ile Pro Pro Asp Ser Pro Asn Asp Leu Met Thr Val Glu Ile Gly
       50                  55                  60
   gcg gga tca ggg cgg gtg acc aaa gtg ctc gcc tca ccc ggg acg cct     240
   Ala Gly Ser Gly Arg Val Thr Lys Val Leu Ala Ser Pro Gly Thr Pro
   65                  70                  75                  80
   tta ctc gcg gtg gaa ata gat ccc cgc tgg gct cgg cgg ctg gcc gcc     288
   Leu Leu Ala Val Glu Ile Asp Pro Arg Trp Ala Arg Arg Leu Ala Ala
                   85                  90                  95
   gaa tcg ctg ccg gac gtc acg gtg gtg aac gag gat ttc ctg acc ctg     336
   Glu Ser Leu Pro Asp Val Thr Val Val Asn Glu Asp Phe Leu Thr Leu
               100                 105                 110
   caa ctg ccc ggg cag ccg gtc aga ctc atc ggg aat ctt ccc ttc gtc     384
   Gln Leu Pro Gly Gln Pro Val Arg Leu Ile Gly Asn Leu Pro Phe Val
           115                 120                 125
   acc ggc acc aga atg ctg agg cgc tgc ctc gac atg ggt ccg gcg cgc     432
   Thr Gly Thr Arg Met Leu Arg Arg Cys Leu Asp Met Gly Pro Ala Arg
       130                 135                 140
   atg cgg cag ggc gtg ttc ctg ttg cag cgg gag tac gtg gga aag cgg     480
   Met Arg Gln Gly Val Phe Leu Leu Gln Arg Glu Tyr Val Gly Lys Arg
   145                 150                 155                 160
   acc gga gcc tgg ggc gga aac ctc ttc aac gcc cag tgg gag ccg tgg     528
   Thr Gly Ala Trp Gly Gly Asn Leu Phe Asn Ala Gln Trp Glu Pro Trp
                   165                 170                 175
   tac tcg ttc gac cgg ggc ctg gcc ttc tca cgc cag gac ttc acc ccc     576
   Tyr Ser Phe Asp Arg Gly Leu Ala Phe Ser Arg Gln Asp Phe Thr Pro
               180                 185                 190
   gta ccg cgc gcg gac acc cag acc ctg atg gtc gcc ccg cac cgc agg      624
   Val Pro Arg Ala Asp Thr Gln Thr Leu Met Val Ala Pro His Arg Arg
           195                 200                 205
   ccg tcc gtg ccc tgg cgt gag aag gcc gcc tac cag cgg ttc gtc caa      672
   Pro Ser Val Pro Trp Arg Glu Lys Ala Ala Tyr Gln Arg Phe Val Gln
       210                 215                 220
   cgg gtc ttc gac acc ggc cag atg acg gtg ggc gac gcc gcg cgg aag      720
   Arg Val Phe Asp Thr Gly Gln Met Thr Val Gly Asp Ala Ala Arg Lys
   225                 230                 235                 240
   gtg ctg cgc cgc gga cac gcc cag ttc gtg cgc ggg gcg ggc gtg agg      768
   Val Leu Arg Arg Gly His Ala Gln Phe Val Arg Gly Ala Gly Val Arg
                   245                 250                 255
   ccg gcc gac cgg gtc aag gac ctc acg gtc ccg gag tgg acc gca ctc      816
   Pro Ala Asp Arg Val Lys Asp Leu Thr Val Pro Glu Trp Thr Ala Leu
               260                 265                 270
   ttc cgc gcc tac ggg cgg acg gcc gac cgc tga                          849
   Phe Arg Ala Tyr Gly Arg Thr Ala Asp Arg
           275                 280
<210> 16
   <211> 282
   <212> PRT
   <213> Streptomyces ambofaciens
<400> 16
   Val Thr Leu Lys Ser Pro Leu Pro Pro Gln Ser Val Ser Ala Pro Ala
   1               5                   10                  15
   Asp Ser Arg Ser Thr Ala Arg Arg Glu Trp Gly Gln Asn Phe Phe Arg
               20                  25                  30
   Thr Ala Ala Ala Ala Cys Arg Phe Ser Ala Gln Leu Asp Gly Ser Asp
           35                  40                  45
   Thr Ile Pro Pro Asp Ser Pro Asn Asp Leu Met Thr Val Glu Ile Gly
       50                  55                  60
   Ala Gly Ser Gly Arg Val Thr Lys Val Leu Ala Ser Pro Gly Thr Pro
   65                  70                  75                  80
   Leu Leu Ala Val Glu Ile Asp Pro Arg Trp Ala Arg Arg Leu Ala Ala
                   85                  90                  95
   Glu Ser Leu Pro Asp Val Thr Val Val Asn Glu Asp Phe Leu Thr Leu
               100                 105                 110
   Gln Leu Pro Gly Gln Pro Val Arg Leu Ile Gly Asn Leu Pro Phe Val
           115                 120                 125
   Thr Gly Thr Arg Met Leu Arg Arg Cys Leu Asp Met Gly Pro Ala Arg
   130 135 140
   Met Arg Gln Gly Val Phe Leu Leu Gln Arg Glu Tyr Val Gly Lys Arg
   145 150 155 160
   Thr Gly Ala Trp Gly Gly Asn Leu Phe Asn Ala Gln Trp Glu Pro Trp
   165 170 175
   Tyr Ser Phe Asp Arg Gly Leu Ala Phe Ser Arg Gln Asp Phe Thr Pro
   180 185 190
   Val Pro Arg Ala Asp Thr Gln Thr Leu Met Val Ala Pro His Arg Arg
   195 200 205
   Pro Ser Val Pro Trp Arg Glu Lys Ala Ala Tyr Gln Arg Phe Val Gln
   210 215 220
   Arg Val Phe Asp Thr Gly Gln Met Thr Val Gly Asp Ala Ala Arg Lys
   225 230 235 240
   Val Leu Arg Arg Gly His Ala Gln Phe Val Arg Gly Ala Gly Val Arg
   245 250 255
   Pro Ala Asp Arg Val Lys Asp Leu Thr Val Pro Glu Trp Thr Ala Leu
   260 265 270
   Phe Arg Ala Tyr Gly Arg Thr Ala Asp Arg
   275 280
<210> 17
   <211> 831
   <212> DNA
   <213> Streptomyces ambofaciens
<220>
   <221> CDS
   <222> (1)..(831)
<400> 17
   gtg aag gag gtg gcg gtc gac acc ggt acc ccg gcc ggc ccc gtg tgg      48
   Val Lys Glu Val Ala Val Asp Thr Gly Thr Pro Ala Gly Pro Val Trp
   1               5                   10                  15
   cgt ggt gcc ggc ctc ggc acc cag ctg tgg gta ctg acc gcg cgg cag      96
   Arg Gly Ala Gly Leu Gly Thr Gln Leu Trp Val Leu Thr Ala Arg Gln
               20                  25                  30
   atc cgt tcc atg tac ggc gac cgc cgc ctg gcg ctg ttc agc ctg atg      144
   Ile Arg Ser Met Tyr Gly Asp Arg Arg Leu Ala Leu Phe Ser Leu Met
           35                  40                  45
   caa ccg gtg atc atg ctg ttg ctg ctg agc gag atc ttc ggc agc atg      192
   Gln Pro Val Ile Met Leu Leu Leu Leu Ser Glu Ile Phe Gly Ser Met
       50                  55                  60
   gcc gac ccg gac gac ttc ccg cag ggc gtg cgc tac atc gac tac gtg      240
   Ala Asp Pro Asp Asp Phe Pro Gln Gly Val Arg Tyr Ile Asp Tyr Val
   65                  70                  75                  80
   gtg ccc gcg ctg ctg gtc acc acc ggc atc ggc tcg gcc cag ggc gcg      288
   Val Pro Ala Leu Leu Val Thr Thr Gly Ile Gly Ser Ala Gln Gly Ala
                   85                  90                  95
   ggg gtg ggc ctg gtc agg gac atg gac aac ggg atg gtg gcg cgc ttc      336
   Gly Val Gly Leu Val Arg Asp Met Asp Asn Gly Met Val Ala Arg Phe
               100                 105                 110
   cgc gtc ctg ccg gcc cgg ctg ttc ctg gtg ctg gtc gcc cgg tcg ctg      384
   Arg Val Leu Pro Ala Arg Leu Phe Leu Val Leu Val Ala Arg Ser Leu
           115                 120                 125
   gcc gat ctg gtc cgt gtg ttc acc gag ttg gtc gtc ctc gtg gcc gtc      432
   Ala Asp Leu Val Arg Val Phe Thr Glu Leu Val Val Leu Val Ala Val
       130                 135                 140
   ggt gtg atc ctg ctg ggc ttc cgt ccg gcc ggg ggt ttg tgg ggc acg      480
   Gly Val Ile Leu Leu Gly Phe Arg Pro Ala Gly Gly Leu Trp Gly Thr
   145                 150                 155                 160
   tcc gcc gcc ctg ttg ctc acc ctg ttc gtc atc tgg tcg ctg atc tgg      528
   Ser Ala Ala Leu Leu Leu Thr Leu Phe Val Ile Trp Ser Leu Ile Trp
                   165                 170                 175
   ggg ttc atc gcc ctc gcg gcg tgg ctg cgc agc gtg gag gtg atg tcc      576
   Gly Phe Ile Ala Leu Ala Ala Trp Leu Arg Ser Val Glu Val Met Ser
               180                 185                 190
   agc ctc gcg gtt ctg gtg atg ttc ccg ctc atg ttc gcc tcc agt gcg      624
   Ser Leu Ala Val Leu Val Met Phe Pro Leu Met Phe Ala Ser Ser Ala
           195                 200                 205
   ttc gtc ccg ctg gac gcc ctc ccg gag tgg ctg cgc tcg gtg gcg cac      672
   Phe Val Pro Leu Asp Ala Leu Pro Glu Trp Leu Arg Ser Val Ala His
       210                 215                 220
   ctc aac ccc gtg acg tac gcg gtc gac agc gcc cgc cgc ctg gcg ctg      720
   Leu Asn Pro Val Thr Tyr Ala Val Asp Ser Ala Arg Arg Leu Ala Leu
   225                 230                 235                 240
   gac tgg gac ccg ggg tgg agc gtg ccc ggc gcg ctg ctg acc agc acc      768
   Asp Trp Asp Pro Gly Trp Ser Val Pro Gly Ala Leu Leu Thr Ser Thr
                   245                 250                 255
   gcg ctc atg gcg gtg ggg atg tac gtc gcc ggg cgt tcc ttc aag agg      816
   Ala Leu Met Ala Val Gly Met Tyr Val Ala Gly Arg Ser Phe Lys Arg
               260                 265                 270
   ccc ccg aac gaa tga                                                  831
   Pro Pro Asn Glu
           275
<210> 18
   <211> 276
   <212> PRT
   <213> Streptomyces ambofaciens
<400> 18
   Val Lys Glu Val Ala Val Asp Thr Gly Thr Pro Ala Gly Pro Val Trp
   1               5                   10                  15
   Arg Gly Ala Gly Leu Gly Thr Gln Leu Trp Val Leu Thr Ala Arg Gln
               20                  25                  30
   Ile Arg Ser Met Tyr Gly Asp Arg Arg Leu Ala Leu Phe Ser Leu Met
   35 40 45
   Gln Pro Val Ile Met Leu Leu Leu Leu Ser Glu Ile Phe Gly Ser Met
   50 55 60
   Ala Asp Pro Asp Asp Phe Pro Gln Gly Val Arg Tyr Ile Asp Tyr Val
   65 70 75 80
   Val Pro Ala Leu Leu Val Thr Thr Gly Ile Gly Ser Ala Gln Gly Ala
   85 90 95
   Gly Val Gly Leu Val Arg Asp Met Asp Asn Gly Met Val Ala Arg Phe
   100 105 110
   Arg Val Leu Pro Ala Arg Leu Phe Leu Val Leu Val Ala Arg Ser Leu
   115 120 125
   Ala Asp Leu Val Arg Val Phe Thr Glu Leu Val Val Leu Val Ala Val
   130 135 140
   Gly Val Ile Leu Leu Gly Phe Arg Pro Ala Gly Gly Leu Trp Gly Thr
   145 150 155 160
   Ser Ala Ala Leu Leu Leu Thr Leu Phe Val Ile Trp Ser Leu Ile Trp
   165 170 175
   Gly Phe Ile Ala Leu Ala Ala Trp Leu Arg Ser Val Glu Val Met Ser
   180 185 190
   Ser Leu Ala Val Leu Val Met Phe Pro Leu Met Phe Ala Ser Ser Ala
   195 200 205
   Phe Val Pro Leu Asp Ala Leu Pro Glu Trp Leu Arg Ser Val Ala His
   210 215 220
   Leu Asn Pro Val Thr Tyr Ala Val Asp Ser Ala Arg Arg Leu Ala Leu
   225 230 235 240
   Asp Trp Asp Pro Gly Trp Ser Val Pro Gly Ala Leu Leu Thr Ser Thr
   245 250 255
   Ala Leu Met Ala Val Gly Met Tyr Val Ala Gly Arg Ser Phe Lys Arg
   260 265 270
   Pro Pro Asn Glu
   275
<210> 19
   <211> 882
   <212> DNA
   <213> Streptomyces ambofaciens
<220>
   <221> CDS
   <222> (1)..(882)
<400> 19
   gtg gac atc gag gtc gaa cgg ggc cgg gtg ctc ggg ctg ctg ggt cac      48
   Val Asp Ile Glu Val Glu Arg Gly Arg Val Leu Gly Leu Leu Gly His
   1               5                   10                  15
   aac ggg gcc ggc aag acg acc ttg gtg aac atc ctt gcc acg gtc tcc      96
   Asn Gly Ala Gly Lys Thr Thr Leu Val Asn Ile Leu Ala Thr Val Ser
               20                  25                  30
   ccg gcg tcc gcg ggc acg gtg acc gtc gcc ggt ttc gac gtc gcg acg      144
   Pro Ala Ser Ala Gly Thr Val Thr Val Ala Gly Phe Asp Val Ala Thr
           35                  40                  45
   cag ggc gcc gag atc cgc gcg cgc atc ggg gtg acc ggc cag ttc gcg      192
   Gln Gly Ala Glu Ile Arg Ala Arg Ile Gly Val Thr Gly Gln Phe Ala
       50                  55                  60
   tcg gtg gac gag tac ctg agc gga ttc cgc aac ctc gtc ctg atc ggc      240
   Ser Val Asp Glu Tyr Leu Ser Gly Phe Arg Asn Leu Val Leu Ile Gly
   65                  70                  75                  80
   cgc ctc ctc ggg gcg gga cgg cgt gag gcg gcg gcc cgg gcc acc gag      288
   Arg Leu Leu Gly Ala Gly Arg Arg Glu Ala Ala Ala Arg Ala Thr Glu
                   85                  90                  95
   ctc ctg gag ctg ttc gag ctg acc ggg gcg gcc cac cag ccc tcc cgc      336
   Leu Leu Glu Leu Phe Glu Leu Thr Gly Ala Ala His Gln Pro Ser Arg
               100                 105                 110
   acc tac tcg ggc ggg atg cgc cga cgg ctc gac ctc gcc gcc agc ctg      384
   Thr Tyr Ser Gly Gly Met Arg Arg Arg Leu Asp Leu Ala Ala Ser Leu
           115                 120                 125
   gtc ggc cgg ccg gac gtg ctg ttc ctc gac gag ccg acg acc ggg ctg      432
   Val Gly Arg Pro Asp Val Leu Phe Leu Asp Glu Pro Thr Thr Gly Leu
       130                 135                 140
   gac ccg gcg acc cgg atc gcc ctg tgg gag acg gtg gag aag ctg gtg      480
   Asp Pro Ala Thr Arg Ile Ala Leu Trp Glu Thr Val Glu Lys Leu Val
   145                 150                 155                 160
   gcg ggc ggc acg acc gtc ctg ctg acc acc cag tac ctg gac gag gcg      528
   Ala Gly Gly Thr Thr Val Leu Leu Thr Thr Gln Tyr Leu Asp Glu Ala
                   165                 170                 175
   gac cgg ctg gcc gac tgg atc acc gtc ctg tcg aag ggc cgg gtg gtg      576
   Asp Arg Leu Ala Asp Trp Ile Thr Val Leu Ser Lys Gly Arg Val Val
               180                 185                 190
   gcc tcg gac acc acc gac cgg ctc aag gcc gac ctg ggc cac cgg tcg      624
   Ala Ser Asp Thr Thr Asp Arg Leu Lys Ala Asp Leu Gly His Arg Ser
           195                 200                 205
   gtg cgg gtg gtc ctt ccg ccc gcc gcc gac ctg acg gcc gcc gcc gcc      672
   Val Arg Val Val Leu Pro Pro Ala Ala Asp Leu Thr Ala Ala Ala Ala
       210                 215                 220
   gcg ctc acc gcc ggc ggg ttc cgt ccg cgg tcc gac gcc ggg gag cac      720
   Ala Leu Thr Ala Gly Gly Phe Arg Pro Arg Ser Asp Ala Gly Glu His
   225                 230                 235                 240
   gcg ctg acc acg ccc gtg gac acc tcg gcc ggt atc gcg ggc gtc atc      768
   Ala Leu Thr Thr Pro Val Asp Thr Ser Ala Gly Ile Ala Gly Val Ile
                   245                     250                 255
   cgc gcg ctg gac acc gtc gga acg cag gcc gtc gag ctg acc gtc aag      816
   Arg Ala Leu Asp Thr Val Gly Thr Gln Ala Val Glu Leu Thr Val Lys
               260                 265                 270
   gag ccg tcc ctg gac gac gtc tac ctg gcg ctc acc cat ccc tca ccc      864
   Glu Pro Ser Leu Asp Asp Val Tyr Leu Ala Leu Thr His Pro Ser Pro
           275                 280                 285
   gcc gcc gac gcg gcc tga                                              882
   Ala Ala Asp Ala Ala
       290
<210> 20
   <211> 293
   <212> PRT
   <213> Streptomyces ambofaciens
<400> 20
   Val Asp Ile Glu Val Glu Arg Gly Arg Val Leu Gly Leu Leu Gly His
   1               5                   10                  15
   Asn Gly Ala Gly Lys Thr Thr Leu Val Asn Ile Leu Ala Thr Val Ser
               20                  25                  30
   Pro Ala Ser Ala Gly Thr Val Thr Val Ala Gly Phe Asp Val Ala Thr
           35                  40                  45
   Gln Gly Ala Glu Ile Arg Ala Arg Ile Gly Val Thr Gly Gln Phe Ala
       50                  55                  60
   Ser Val Asp Glu Tyr Leu Ser Gly Phe Arg Asn Leu Val Leu Ile Gly
   65                  70                  75                  80
   Arg Leu Leu Gly Ala Gly Arg Arg Glu Ala Ala Ala Arg Ala Thr Glu
                   85                  90                  95
   Leu Leu Glu Leu Phe Glu Leu Thr Gly Ala Ala His Gln Pro Ser Arg
               100                 105                 110
   Thr Tyr Ser Gly Gly Met Arg Arg Arg Leu Asp Leu Ala Ala Ser Leu
           115                 120                 125
   Val Gly Arg Pro Asp Val Leu Phe Leu Asp Glu Pro Thr Thr Gly Leu
       130                 135                 140
   Asp Pro Ala Thr Arg Ile Ala Leu Trp Glu Thr Val Glu Lys Leu Val
   145                 150                 155                 160
   Ala Gly Gly Thr Thr Val Leu Leu Thr Thr Gln Tyr Leu Asp Glu Ala
                   165                 170                 175
   Asp Arg Leu Ala Asp Trp Ile Thr Val Leu Ser Lys Gly Arg Val Val
   180 185 190
   Ala Ser Asp Thr Thr Asp Arg Leu Lys Ala Asp Leu Gly His Arg Ser
   195 200 205
   Val Arg Val Val Leu Pro Pro Ala Ala Asp Leu Thr Ala Ala Ala Ala
   210 215 220
   Ala Leu Thr Ala Gly Gly Phe Arg Pro Arg Ser Asp Ala Gly Glu His
   225 230 235 240
   Ala Leu Thr Thr Pro Val Asp Thr Ser Ala Gly Ile Ala Gly Val Ile
   245 250 255
   Arg Ala Leu Asp Thr Val Gly Thr Gln Ala Val Glu Leu Thr Val Lys
   260 265 270
   Glu Pro Ser Leu Asp Asp Val Tyr Leu Ala Leu Thr His Pro Ser Pro
   275 280 285
   Ala Ala Asp Ala Ala
   290
<210> 21
   <211> 228
   <212> DNA
   <213> Streptomyces ambofaciens
<220>
   <221> CDS
   <222> (1)..(228)
<400> 21
   atg agc aac ccg ttc gag gac acg gaa gcc acc tac gtc gtg ctg gtc 48
   Met Ser Asn Pro Phe Glu Asp Thr Glu Ala Thr Tyr Val Val Leu Val
   1 5 10 15
   aac gac gag ggg cag cac tcg ctg tgg ccg tcg ttc gcg gag gtc ccg 96
   Asn Asp Glu Gly Gln His Ser Leu Trp Pro Ser Phe Ala Glu Val Pro
   20 25 30
   gcg ggc tgg tcc gtc gtg gtg ccg gag acg gac cgg cag tcg tgc ctg 144
   Ala Gly Trp Ser Val Val Val Pro Glu Thr Asp Arg Gln Ser Cys Leu
   35 40 45
   gac tac atc aac gag aac tgg acc gac atg cgc ccc aag agc ctc gtc 192
   Asp Tyr Ile Asn Glu Asn Trp Thr Asp Met Arg Pro Lys Ser Leu Val
   50 55 60
   gag gcg atg gcg acg gcc ggg cag gac gcc cct tga 228
   Glu Ala Met Ala Thr Ala Gly Gln Asp Ala Pro
   65 70 75
<210> 22
   <211> 75
   <212> PRT
   <213> Streptomyces ambofaciens
<400> 22
   Met Ser Asn Pro Phe Glu Asp Thr Glu Ala Thr Tyr Val Val Leu Val
   1               5                   10                  15
   Asn Asp Glu Gly Gln His Ser Leu Trp Pro Ser Phe Ala Glu Val Pro
               20                  25                  30
   Ala Gly Trp Ser Val Val Val Pro Glu Thr Asp Arg Gln Ser Cys Leu
           35                  40                  45
   Asp Tyr Ile Asn Glu Asn Trp Thr Asp Met Arg Pro Lys Ser Leu Val
       50                  55                  60
   Glu Ala Met Ala Thr Ala Gly Gln Asp Ala Pro
   65                  70                  75
<210> 23
   <211> 1212
   <212> DNA
   <213> Streptomyces ambofaciens
<220>
   <221> CDS
   <222> (1)..(1212)
<400> 23
   atg ggt gag gcc gtg acg gga ccg atg gag ctg agc aag gac gcg gac      48
   Met Gly Glu Ala Val Thr Gly Pro Met Glu Leu Ser Lys Asp Ala Asp
   1               5                   10                  15
   gcc cgg ggg ctg ctt gag tgg ttc gcg tac aac agg acg cgt cat ccg      96
   Ala Arg Gly Leu Leu Glu Trp Phe Ala Tyr Asn Arg Thr Arg His Pro
               20                  25                  30
   gtg ttc tgg gac gag acc cga cag gcg tgg cag gtc ttc ggc tac gac     144
   Val Phe Trp Asp Glu Thr Arg Gln Ala Trp Gln Val Phe Gly Tyr Asp
           35                  40                  45
   gac tac gtg acg gtg tcg aac aac ccg cag ttc ttc tcc tcg gac ttc     192
   Asp Tyr Val Thr Val Ser Asn Asn Pro Gln Phe Phe Ser Ser Asp Phe
       50                  55                  60
   aac atg gtg atg ccg acg ccg ccc gaa ctg gag atg atc atc ggt ccg     240
   Asn Met Val Met Pro Thr Pro Pro Glu Leu Glu Met Ile Ile Gly Pro
   65                  70                  75                  80
   ggc acg atc ggc gcg ctg gac ccg ccc gcg cac gga ccg atg cgc aag     288
   Gly Thr Ile Gly Ala Leu Asp Pro Pro Ala His Gly Pro Met Arg Lys
                   85                  90                  95
   ctg gtg agc cag gcg ttc acc ccc cga cgg atc gcc cgg ctg gag ccc     336
   Leu Val Ser Gln Ala Phe Thr Pro Arg Arg Ile Ala Arg Leu Glu Pro
               100                 105                 110
   agg gtg cgc gcg atc acc gag gag ctc ctg gac aag gtg ggg cag cag     384
   Arg Val Arg Ala Ile Thr Glu Glu Leu Leu Asp Lys Val Gly Gln Gln
           115                 120                 125
   gac gtc gtc gac gcc gtg ggt gac ctg tcc tac gcg ctg ccg gtc atc     432
   Asp Val Val Asp Ala Val Gly Asp Leu Ser Tyr Ala Leu Pro Val Ile
       130                 135                 140
   gtg atc gcc gaa ctg ctg ggc ata ccc gcc ggc gac cgt gac ctg ttc     480
   Val Ile Ala Glu Leu Leu Gly Ile Pro Ala Gly Asp Arg Asp Leu Phe
   145                 150                 155                 160
   cgg gag tgg gtc gac acc ctg ctg acg aac gag ggc ctg gag tac ccg     528
   Arg Glu Trp Val Asp Thr Leu Leu Thr Asn Glu Gly Leu Glu Tyr Pro
                   165                 170                 175
   aac ctc ccg gac aac ttc acc gag acg atc gcg ccc gcg ctc aag gag     576
   Asn Leu Pro Asp Asn Phe Thr Glu Thr Ile Ala Pro Ala Leu Lys Glu
               180                 185                 190
   atg acc gac tac ctc ctg aag cag atc cac gcc aag cgg gac gcg ccc     624
   Met Thr Asp Tyr Leu Leu Lys Gln Ile His Ala Lys Arg Asp Ala Pro
           195                 200                 205
   gcc gac gac ctg gtc agc ggg ctg gtc cag gcg gag cag gac ggc cgc     672
   Ala Asp Asp Leu Val Ser Gly Leu Val Gln Ala Glu Gln Asp Gly Arg
       210                 215                 220
   cgg ctg acc gac gtc gag atc gtc aac atc gtc gcg ctg ctc ctg acg     720
   Arg Leu Thr Asp Val Glu Ile Val Asn Ile Val Ala Leu Leu Leu Thr
   225                 230                 235                 240
   gcg ggg cac gtc tcc tcc agc acc ctg ctc agc aac ctg ttc ctg gtc     768
   Ala Gly His Val Ser Ser Ser Thr Leu Leu Ser Asn Leu Phe Leu Val
                   245                 250                 255
   ctg gag gag aac ccg cag gcg ctg gag gac ctg cgg gcc gat cgc tcc     816
   Leu Glu Glu Asn Pro Gln Ala Leu Glu Asp Leu Arg Ala Asp Arg Ser
               260                 265                 270
   ctg gtg ccc ggc gcg atc gag gag acg ctg cgc tac cgc agc ccc ttc     864
   Leu Val Pro Gly Ala Ile Glu Glu Thr Leu Arg Tyr Arg Ser Pro Phe
           275                 280                 285
   aac aac atc ttc cgg ttc gtc aag gag gac acc acc gtc ctc ggt ccg     912
   Asn Asn Ile Phe Arg Phe Val Lys Glu Asp Thr Thr Val Leu Gly Pro
       290                 295                 300
   ctc atg gag aag ggc cag atg gtg atc gcc tgg agc cag tcc gcc aac     960
   Leu Met Glu Lys Gly Gln Met Val Ile Ala Trp Ser Gln Ser Ala Asn
   305                 310                 315                 320
   cgg gac ccc cgg cac ttc ccg gac ccg gac acc ttc gac atc cgc cgc    1008
   Arg Asp Pro Arg His Phe Pro Asp Pro Asp Thr Phe Asp Ile Arg Arg
                   325                 330                 335
   tcg gac ggc acc cgg cac atg gcc ttc ggg cac ggc atc cac cac tgc    1056
   Ser Asp Gly Thr Arg His Met Ala Phe Gly His Gly Ile His His Cys
               340                 345                 350
   ctg ggt gcc gcc ctc gcc cgc ctg gag ggc aag gtc atg ctc gaa ctc    1104
   Leu Gly Ala Ala Leu Ala Arg Leu Glu Gly Lys Val Met Leu Glu Leu
           355                 360                 365
   ctc ctg gac cgg gtc caa ggc ttc cgc atc gac cac gag cac acc gtg    1152
   Leu Leu Asp Arg Val Gln Gly Phe Arg Ile Asp His Glu His Thr Val
       370                 375                 380
   ttc tac gag gcc gac cag ctc act ccg aag tac ctg ccc gtc cgg gtc    1200
   Phe Tyr Glu Ala Asp Gln Leu Thr Pro Lys Tyr Leu Pro Val Arg Val
   385                 390                 395                 400
   gac tgg aac tga                                                    1212
   Asp Trp Asn
<210> 24
   <211> 403
   <212> PRT
   <213> Streptomyces ambofaciens
<400> 24
   Met Gly Glu Ala Val Thr Gly Pro Met Glu Leu Ser Lys Asp Ala Asp
   1               5                   10                  15
   Ala Arg Gly Leu Leu Glu Trp Phe Ala Tyr Asn Arg Thr Arg His Pro
               20                  25                  30
   Val Phe Trp Asp Glu Thr Arg Gln Ala Trp Gln Val Phe Gly Tyr Asp
           35                  40                  45
   Asp Tyr Val Thr Val Ser Asn Asn Pro Gln Phe Phe Ser Ser Asp Phe
       50                  55                  60
   Asn Met Val Met Pro Thr Pro Pro Glu Leu Glu Met Ile Ile Gly Pro
   65                  70                  75                  80
   Gly Thr Ile Gly Ala Leu Asp Pro Pro Ala His Gly Pro Met Arg Lys
                   85                  90                  95
   Leu Val Ser Gln Ala Phe Thr Pro Arg Arg Ile Ala Arg Leu Glu Pro
               100                 105                 110
   Arg Val Arg Ala Ile Thr Glu Glu Leu Leu Asp Lys Val Gly Gln Gln
           115                 120                 125
   Asp Val Val Asp Ala Val Gly Asp Leu Ser Tyr Ala Leu Pro Val Ile
       130                 135                 140
   Val Ile Ala Glu Leu Leu Gly Ile Pro Ala Gly Asp Arg Asp Leu Phe
   145                 150                 155                 160
   Arg Glu Trp Val Asp Thr Leu Leu Thr Asn Glu Gly Leu Glu Tyr Pro
                   165                 170                 175
   Asn Leu Pro Asp Asn Phe Thr Glu Thr Ile Ala Pro Ala Leu Lys Glu
               180                 185                 190
   Met Thr Asp Tyr Leu Leu Lys Gln Ile His Ala Lys Arg Asp Ala Pro
           195                 200                 205
   Ala Asp Asp Leu Val Ser Gly Leu Val Gln Ala Glu Gln Asp Gly Arg
       210                 215                 220
   Arg Leu Thr Asp Val Glu Ile Val Asn Ile Val Ala Leu Leu Leu Thr
   225 230 235 240
   Ala Gly His Val Ser Ser Ser Thr Leu Leu Ser Asn Leu Phe Leu Val
   245 250 255
   Leu Glu Glu Asn Pro Gln Ala Leu Glu Asp Leu Arg Ala Asp Arg Ser
   260 265 270
   Leu Val Pro Gly Ala Ile Glu Glu Thr Leu Arg Tyr Arg Ser Pro Phe
   275 280 285
   Asn Asn Ile Phe Arg Phe Val Lys Glu Asp Thr Thr Val Leu Gly Pro
   290 295 300
   Leu Met Glu Lys Gly Gln Met Val Ile Ala Trp Ser Gln Ser Ala Asn
   305 310 315 320
   Arg Asp Pro Arg His Phe Pro Asp Pro Asp Thr Phe Asp Ile Arg Arg
   325 330 335
   Ser Asp Gly Thr Arg His Met Ala Phe Gly His Gly Ile His His Cys
   340 345 350
   Leu Gly Ala Ala Leu Ala Arg Leu Glu Gly Lys Val Met Leu Glu Leu
   355 360 365
   Leu Leu Asp Arg Val Gln Gly Phe Arg Ile Asp His Glu His Thr Val
   370 375 380
   Phe Tyr Glu Ala Asp Gln Leu Thr Pro Lys Tyr Leu Pro Val Arg Val
   385 390 395 400
   Asp Trp Asn
<210> 25
   <211> 540
   <212> DNA
   <213> Streptomyces ambofaciens
<220>
   <221> CDS
   <222> (1)..(540)
<220>
   <221> CDS2
   <222> (190)..(540)
<400> 25
   ctg aac ccg agg gtc tcg tcc cgg agt cca ggg ccg tcc cga gcc ggc 48
   Leu Asn Pro Arg Val Ser Ser Arg Ser Pro Gly Pro Ser Arg Ala Gly
   1 5 10 15
   cct gga cct cac gac cgc ccg ata agg agc gcc gcc atc gcc gag aac 96
   Pro Gly Pro His Asp Arg Pro Ile Arg Ser Ala Ala Ile Ala Glu Asn
   20 25 30
   aca gcc gag ctc cct gcc cgg cgg gtc ggc agg atc aag ccg tgc cgg 144
   Thr Ala Glu Leu Pro Ala Arg Arg Val Gly Arg Ile Lys Pro Cys Arg
   35 40 45
   ctg atc agg ctc gag cag cac atc gac ccg cgc ggc agc ctc tcc gtg 192
   Leu Ile Arg Leu Glu Gln His Ile Asp Pro Arg Gly Ser Leu Ser Val
   50 55 60
   atc gag tcc ggc gtg acc gtg gac ttc ccc gtc cga cgc gtc tac tac 240
   Ile Glu Ser Gly Val Thr Val Asp Phe Pro Val Arg Arg Val Tyr Tyr
   65 70 75 80
   atg cat ggc cag acc cag tcc tct ccc ccg cgc ggc ctg cac gcg cac 288
   Met His Gly Gln Thr Gln Ser Ser Pro Pro Arg Gly Leu His Ala His
   85 90 95
   cgc acc ctg gaa caa ctc gtc atc gcc gtc cac ggc gcc ttc tcc atc 336
   Arg Thr Leu Glu Gln Leu Val Ile Ala Val His Gly Ala Phe Ser Ile
   100 105 110
   acc ctc gac gac ggc ttc cag cac gcc acc tac cgt ctg gac gaa ccc 384
   Thr Leu Asp Asp Gly Phe Gln His Ala Thr Tyr Arg Leu Asp Glu Pro
   115 120 125
   gga gcc gga ctc tgc atc ggc ccc atg gtc tgg cgc gtc ctg aag gac 432
   Gly Ala Gly Leu Cys Ile Gly Pro Met Val Trp Arg Val Leu Lys Asp
   130 135 140
   ttc gac ccc gac acc gtg gcc ctg gtc ctc gcc tcg cag cac tac gag 480
   Phe Asp Pro Asp Thr Val Ala Leu Val Leu Ala Ser Gln His Tyr Glu
   145 150 155 160
   gag tcc gac tac tac cgc gac tac gac acc ttc ctg cat gac gca cgg 528
   Glu Ser Asp Tyr Tyr Arg Asp Tyr Asp Thr Phe Leu His Asp Ala Arg
   165 170 175
   agc ctc aca tga 540
   Ser Leu Thr
<210> 26
   <211> 179
   <212> PRT
   <213> Streptomyces ambofaciens
<400> 26
   Leu Asn Pro Arg Val Ser Ser Arg Ser Pro Gly Pro Ser Arg Ala Gly
   1               5                   10                  15
   Pro Gly Pro His Asp Arg Pro Ile Arg Ser Ala Ala Ile Ala Glu Asn
               20                  25                  30
   Thr Ala Glu Leu Pro Ala Arg Arg Val Gly Arg Ile Lys Pro Cys Arg
           35                  40                  45
   Leu Ile Arg Leu Glu Gln His Ile Asp Pro Arg Gly Ser Leu Ser Val
       50                  55                  60
   Ile Glu Ser Gly Val Thr Val Asp Phe Pro Val Arg Arg Val Tyr Tyr
   65                  70                  75                  80
   Met His Gly Gln Thr Gln Ser Ser Pro Pro Arg Gly Leu His Ala His
                   85                  90                  95
   Arg Thr Leu Glu Gln Leu Val Ile Ala Val His Gly Ala Phe Ser Ile
               100                 105                 110
   Thr Leu Asp Asp Gly Phe Gln His Ala Thr Tyr Arg Leu Asp Glu Pro
           115                 120                 125
   Gly Ala Gly Leu Cys Ile Gly Pro Met Val Trp Arg Val Leu Lys Asp
       130                 135                 140
   Phe Asp Pro Asp Thr Val Ala Leu Val Leu Ala Ser Gln His Tyr Glu
   145                 150                 155                 160
   Glu Ser Asp Tyr Tyr Arg Asp Tyr Asp Thr Phe Leu His Asp Ala Arg
                   165                 170                 175
   Ser Leu Thr
<210> 27
   <211> 116
   <212> PRT
   <213> Streptomyces ambofaciens
<400> 27
   Val Ile Glu Ser Gly Val Thr Val Asp Phe Pro Val Arg Arg Val Tyr
   1               5                   10                  15
   Tyr Met His Gly Gln Thr Gln Ser Ser Pro Pro Arg Gly Leu His Ala
               20                  25                  30
   His Arg Thr Leu Glu Gln Leu Val Ile Ala Val His Gly Ala Phe Ser
           35                  40                  45
   Ile Thr Leu Asp Asp Gly Phe Gln His Ala Thr Tyr Arg Leu Asp Glu
       50                  55                  60
   Pro Gly Ala Gly Leu Cys Ile Gly Pro Met Val Trp Arg Val Leu Lys
   65                  70                  75                  80
   Asp Phe Asp Pro Asp Thr Val Ala Leu Val Leu Ala Ser Gln His Tyr
                   85                  90                  95
   Glu Glu Ser Asp Tyr Tyr Arg Asp Tyr Asp Thr Phe Leu His Asp Ala
               100                 105                 110
   Arg Ser Leu Thr
           115
<210> 28
   <211> 1167
   <212> DNA
   <213> Streptomyces ambofaciens
<220>
   <221> CDS
   <222> (1)..(1167)
<400> 28
   atg acc atc ccc ttc ctc gac gcg ggc gcc ggc tac cgg gag ttg cga      48
   Met Thr Ile Pro Phe Leu Asp Ala Gly Ala Gly Tyr Arg Glu Leu Arg
   1               5                   10                  15
   gcc gag atc gac gcg gcc ctg cag cgg gtg tcc gcc tcc ggc cgc tat      96
   Ala Glu Ile Asp Ala Ala Leu Gln Arg Val Ser Ala Ser Gly Arg Tyr
               20                  25                  30
   ctg ctc gac gcg gaa ctc gcg gcc ttc gag gag gag ttc gcc gcg tac     144
   Leu Leu Asp Ala Glu Leu Ala Ala Phe Glu Glu Glu Phe Ala Ala Tyr
           35                  40                  45
   tgc gac aac gac cac tgt gtg gcg gtg ggc agt ggc tgc gac gcg ctg     192
   Cys Asp Asn Asp His Cys Val Ala Val Gly Ser Gly Cys Asp Ala Leu
       50                  55                  60
   gag ctg tcc ctg cgg gcg ctg gac atc ggt ccc ggg gac gag gtg gtg     240
   Glu Leu Ser Leu Arg Ala Leu Asp Ile Gly Pro Gly Asp Glu Val Val
   65                  70                  75                  80
   gtg ccc gcg cac acc ttc atc ggg acc tgg ctg gcc gtg tcc gct acc     288
   Val Pro Ala His Thr Phe Ile Gly Thr Trp Leu Ala Val Ser Ala Thr
                   85                  90                  95
   ggg gca cgg ccg gtg gcc gtc gac ccg acg ccg gac ggg ctc tcc ctc     336
   Gly Ala Arg Pro Val Ala Val Asp Pro Thr Pro Asp Gly Leu Ser Leu
               100                 105                 110
   gac ccg gcg ctg gtg gag gcg gcg ctc acc cct cgg acc aga gcc ctg     384
   Asp Pro Ala Leu Val Glu Ala Ala Leu Thr Pro Arg Thr Arg Ala Leu
           115                 120                 125
   atg ccg gtg cac ctg cac ggg cac ccg gcc gac ctc gac ccg cta ctg     432
   Met Pro Val His Leu His Gly His Pro Ala Asp Leu Asp Pro Leu Leu
       130                 135                 140
   gcg atc gcc gga cgg cac ggc ctg gcc gtg gtc gag gac gcc gcg cag     480
   Ala Ile Ala Gly Arg His Gly Leu Ala Val Val Glu Asp Ala Ala Gln
   145                 150                 155                 160
   gcc cac ggc gcc cgt tac cgg ggc cgc agg atc ggc tcg ggc cac gtg     528
   Ala His Gly Ala Arg Tyr Arg Gly Arg Arg Ile Gly Ser Gly His Val
                   165                 170                 175
   gtc gcg ttc agc ttc tac ccc ggc aag aac ctc ggc gcc atg ggg gac     576
   Val Ala Phe Ser Phe Tyr Pro Gly Lys Asn Leu Gly Ala Met Gly Asp
               180                 185                 190
   ggc ggc gcg gtg gtc acg ggt gac tcc ggt gtg gcc gag cgg atc cgg     624
   Gly Gly Ala Val Val Thr Gly Asp Ser Gly Val Ala Glu Arg Ile Arg
           195                 200                 205
   ttg ctg cgc aac tgc ggc tcg cgg gag aag tac cgg cac gag gtg cgc     672
   Leu Leu Arg Asn Cys Gly Ser Arg Glu Lys Tyr Arg His Glu Val Arg
       210                 215                 220
   tcg acc cac tcc cgg ctc gac gag ttc cag gcg gcc gtg ctg cgg gcc      720
   Ser Thr His Ser Arg Leu Asp Glu Phe Gln Ala Ala Val Leu Arg Ala
   225                 230                 235                 240
   aaa ctg ccg cgg ctc gac gcg tgg aac gcc cgc cgg gcc ggc acg gcc      768
   Lys Leu Pro Arg Leu Asp Ala Trp Asn Ala Arg Arg Ala Gly Thr Ala
                   245                 250                 255
   gaa cgg tac ggg cgg gcc ctg ggt ccg gta ccg cag atc gcc gtc ccg      816
   Glu Arg Tyr Gly Arg Ala Leu Gly Pro Val Pro Gln Ile Ala Val Pro
               260                 265                 270
   gtc acc gct ccc tgg gcc gac ccg gtg tgg cac ctg tac gtg atc cgc      864
   Val Thr Ala Pro Trp Ala Asp Pro Val Trp His Leu Tyr Val Ile Arg
           275                 280                 285
   tgc gcg gag cgc gac gag ctg cgc cgc cgg ctg gaa cga gcc ggg gtc      912
   Cys Ala Glu Arg Asp Glu Leu Arg Arg Arg Leu Glu Arg Ala Gly Val
       290                 295                 300
   cag acc ctg atc cac tac ccc gtg ccc ccg cac cgg tcc ccg gcc tac      960
   Gln Thr Leu Ile His Tyr Pro Val Pro Pro His Arg Ser Pro Ala Tyr
   305                 310                 315                 320
   gcc gac gac ccg gcc ggc gca ccg gcg ggg acc cac ccg ctc agt gag     1008
   Ala Asp Asp Pro Ala Gly Ala Pro Ala Gly Thr His Pro Leu Ser Glu
                   325                 330                 335
   cgc ctg gcg gcg cag agc ctc agc ctt ccc ctg gga ccg cac ctc ggg     1056
   Arg Leu Ala Ala Gln Ser Leu Ser Leu Pro Leu Gly Pro His Leu Gly
               340                 345                 350
   gag gac gag gcc cgc gcc gtc gtg gcg gcg gtc cgg gcg gcg tcc gca     1104
   Glu Asp Glu Ala Arg Ala Val Val Ala Ala Val Arg Ala Ala Ser Ala
           355                 360                 365
   ggg ctg gcg gcg tac ccg acg ccg gac ggc cag cgt ttt cct cta gtg     1152
   Gly Leu Ala Ala Tyr Pro Thr Pro Asp Gly Gln Arg Phe Pro Leu Val
       370                 375                 380
   acg gag aaa cga tga                                                 1167
   Thr Glu Lys Arg
   385
<210> 29
   <211> 388
   <212> PRT
   <213> Streptomyces ambofaciens
<400> 29
   Met Thr Ile Pro Phe Leu Asp Ala Gly Ala Gly Tyr Arg Glu Leu Arg
   1               5                   10                  15
   Ala Glu Ile Asp Ala Ala Leu Gln Arg Val Ser Ala Ser Gly Arg Tyr
               20                  25                  30
   Leu Leu Asp Ala Glu Leu Ala Ala Phe Glu Glu Glu Phe Ala Ala Tyr
           35                  40                  45
   Cys Asp Asn Asp His Cys Val Ala Val Gly Ser Gly Cys Asp Ala Leu
       50                  55                  60
   Glu Leu Ser Leu Arg Ala Leu Asp Ile Gly Pro Gly Asp Glu Val Val
   65 70 75 80
   Val Pro Ala His Thr Phe Ile Gly Thr Trp Leu Ala Val Ser Ala Thr
   85 90 95
   Gly Ala Arg Pro Val Ala Val Asp Pro Thr Pro Asp Gly Leu Ser Leu
   100 105 110
   Asp Pro Ala Leu Val Glu Ala Ala Leu Thr Pro Arg Thr Arg Ala Leu
   115 120 125
   Met Pro Val His Leu His Gly His Pro Ala Asp Leu Asp Pro Leu Leu
   130 135 140
   Ala Ile Ala Gly Arg His Gly Leu Ala Val Val Glu Asp Ala Ala Gln
   145 150 155 160
   Ala His Gly Ala Arg Tyr Arg Gly Arg Arg Ile Gly Ser Gly His Val
   165 170 175
   Val Ala Phe Ser Phe Tyr Pro Gly Lys Asn Leu Gly Ala Met Gly Asp
   180 185 190
   Gly Gly Ala Val Val Thr Gly Asp Ser Gly Val Ala Glu Arg Ile Arg
   195 200 205
   Leu Leu Arg Asn Cys Gly Ser Arg Glu Lys Tyr Arg His Glu Val Arg
   210 215 220
   Ser Thr His Ser Arg Leu Asp Glu Phe Gln Ala Ala Val Leu Arg Ala
   225 230 235 240
   Lys Leu Pro Arg Leu Asp Ala Trp Asn Ala Arg Arg Ala Gly Thr Ala
   245 250 255
   Glu Arg Tyr Gly Arg Ala Leu Gly Pro Val Pro Gln Ile Ala Val Pro
   260 265 270
   Val Thr Ala Pro Trp Ala Asp Pro Val Trp His Leu Tyr Val Ile Arg
   275 280 285
   Cys Ala Glu Arg Asp Glu Leu Arg Arg Arg Leu Glu Arg Ala Gly Val
   290 295 300
   Gln Thr Leu Ile His Tyr Pro Val Pro Pro His Arg Ser Pro Ala Tyr
   305 310 315 320
   Ala Asp Asp Pro Ala Gly Ala Pro Ala Gly Thr His Pro Leu Ser Glu
   325 330 335
   Arg Leu Ala Ala Gln Ser Leu Ser Leu Pro Leu Gly Pro His Leu Gly
   340 345 350
   Glu Asp Glu Ala Arg Ala Val Val Ala Ala Val Arg Ala Ala Ser Ala
   355 360 365
   Gly Leu Ala Ala Tyr Pro Thr Pro Asp Gly Gln Arg Phe Pro Leu Val
   370 375 380
   Thr Glu Lys Arg
   385
<210> 30
   <211> 909
   <212> DNA
   <213> Streptomyces ambofaciens
<220>
   <221> CDS
   <222> (1)..(909)
<220>
   <221> CDS2
   <222> (10)..(909)
<220>
   <221> CDS3
   <222> (28)..(909)
<400> 30
   atg acc gag gtc atg tca ggg cgt ccc gga atg aaa ggg atc atc ctc      48
   Met Thr Glu Val Met Ser Gly Arg Pro Gly Met Lys Gly Ile Ile Leu
   1               5                   10                  15
   gca ggc ggc gga ggg acc cgc cta cgc ccc ttg acc ggc acg ctg tcc      96
   Ala Gly Gly Gly Gly Thr Arg Leu Arg Pro Leu Thr Gly Thr Leu Ser
               20                  25                  30
   aag caa ctg ctg ccc gtc tac gac aag ccg atg atc tac tac ccg ctg     144
   Lys Gln Leu Leu Pro Val Tyr Asp Lys Pro Met Ile Tyr Tyr Pro Leu
           35                  40                  45
   tcc gtc ctg atg ctg ggc ggc atc cgc gag atc ctc gtc gtc tcc tcc     192
   Ser Val Leu Met Leu Gly Gly Ile Arg Glu Ile Leu Val Val Ser Ser
       50                  55                  60
   acc cag cac atc gag ctg ttc cag cgg ctg ctg ggc gac ggc tcc cgc     240
   Thr Gln His Ile Glu Leu Phe Gln Arg Leu Leu Gly Asp Gly Ser Arg
   65                  70                  75                  80
   ctc ggc ctc gac atc acc tac gcc gaa cag gcc gag ccc gag ggc ata     288
   Leu Gly Leu Asp Ile Thr Tyr Ala Glu Gln Ala Glu Pro Glu Gly Ile
                   85                  90                  95
   gcg cag gcc atc acc atc ggc acc gac cac atc ggc gac tca ccg gtc     336
   Ala Gln Ala Ile Thr Ile Gly Thr Asp His Ile Gly Asp Ser Pro Val
               100                 105                 110
   gcg ctc atc ctg ggc gac aac atc ttc cac ggc ccc ggc ttc tcg gcc     384
   Ala Leu Ile Leu Gly Asp Asn Ile Phe His Gly Pro Gly Phe Ser Ala
           115                 120                 125
   gtg ctc cag ggc agc atc cgc cac ctc gac ggc tgt gtg ctg ttc ggc     432
   Val Leu Gln Gly Ser Ile Arg His Leu Asp Gly Cys Val Leu Phe Gly
       130                 135                 140
   tac ccg gtc agc gac ccg aag cgc tac ggc gtc ggc gag atc gac gac      480
   Tyr Pro Val Ser Asp Pro Lys Arg Tyr Gly Val Gly Glu Ile Asp Asp
   145                 150                 155                 160
   cag ggc gta ctg ctg tcc ctg gag gag aaa ccg gcc cgg ccc cgc tcc      528
   Gln Gly Val Leu Leu Ser Leu Glu Glu Lys Pro Ala Arg Pro Arg Ser
                   165                 170                 175
   aac ctc gcc gtc acc ggc ctc tac ctc tac gac aac gac gtg gtc gac      576
   Asn Leu Ala Val Thr Gly Leu Tyr Leu Tyr Asp Asn Asp Val Val Asp
               180                 185                 190
   atc gcc aag aac atc cgg ccc tcg gcg cgc ggc gaa ctc gag atc acg      624
   Ile Ala Lys Asn Ile Arg Pro Ser Ala Arg Gly Glu Leu Glu Ile Thr
           195                 200                 205
   gac gtc aac agg acc tac ctg gag cag aaa cgc gcc cgg ctc atc gaa      672
   Asp Val Asn Arg Thr Tyr Leu Glu Gln Lys Arg Ala Arg Leu Ile Glu
       210                 215                 220
   ctg ggc cac ggc ttc gcc tgg ctc gac atg ggc acc cac gac tcc ctc      720
   Leu Gly His Gly Phe Ala Trp Leu Asp Met Gly Thr His Asp Ser Leu
   225                 230                 235                 240
   ctc cag ggc ggc cag tac gtc cag ctc atc gag cag cgc cag gga gtg      768
   Leu Gln Gly Gly Gln Tyr Val Gln Leu Ile Glu Gln Arg Gln Gly Val
                   245                 250                 255
   cgg atc gcc tgc atc gag gag atc gcc ctg cgc atg ggc ttc atc gac      816
   Arg Ile Ala Cys Ile Glu Glu Ile Ala Leu Arg Met Gly Phe Ile Asp
               260                 265                 270
   gcc gac acc ctc cac cgg ctc ggc cgc gaa ctg ggc acc tcc gga tac      864
   Ala Asp Thr Leu His Arg Leu Gly Arg Glu Leu Gly Thr Ser Gly Tyr
           275                 280                 285
   ggc gcg tac ctg atg gag gtg gcc acc cgt gca ggc acc gaa tga          909
   Gly Ala Tyr Leu Met Glu Val Ala Thr Arg Ala Gly Thr Glu
       290                 295                 300
<210> 31
   <211> 302
   <212> PRT
   <213> Streptomyces ambofaciens
<400> 31
   Met Thr Glu Val Met Ser Gly Arg Pro Gly Met Lys Gly Ile Ile Leu
   1 5 10 15
   Ala Gly Gly Gly Gly Thr Arg Leu Arg Pro Leu Thr Gly Thr Leu Ser
   20 25 30
   Lys Gln Leu Leu Pro Val Tyr Asp Lys Pro Met Ile Tyr Tyr Pro Leu
   35 40 45
   Ser Val Leu Met Leu Gly Gly Ile Arg Glu Ile Leu Val Val Ser Ser
   50 55 60
   Thr Gln His Ile Glu Leu Phe Gln Arg Leu Leu Gly Asp Gly Ser Arg
   65 70 75 80
   Leu Gly Leu Asp Ile Thr Tyr Ala Glu Gln Ala Glu Pro Glu Gly Ile
   85 90 95
   Ala Gln Ala Ile Thr Ile Gly Thr Asp His Ile Gly Asp Ser Pro Val
   100 105 110
   Ala Leu Ile Leu Gly Asp Asn Ile Phe His Gly Pro Gly Phe Ser Ala
   115 120 125
   Val Leu Gln Gly Ser Ile Arg His Leu Asp Gly Cys Val Leu Phe Gly
   130 135 140
   Tyr Pro Val Ser Asp Pro Lys Arg Tyr Gly Val Gly Glu Ile Asp Asp
   145 150 155 160
   Gln Gly Val Leu Leu Ser Leu Glu Glu Lys Pro Ala Arg Pro Arg Ser
   165 170 175
   Asn Leu Ala Val Thr Gly Leu Tyr Leu Tyr Asp Asn Asp Val Val Asp
   180 185 190
   Ile Ala Lys Asn Ile Arg Pro Ser Ala Arg Gly Glu Leu Glu Ile Thr
   195 200 205
   Asp Val Asn Arg Thr Tyr Leu Glu Gln Lys Arg Ala Arg Leu Ile Glu
   210 215 220
   Leu Gly His Gly Phe Ala Trp Leu Asp Met Gly Thr His Asp Ser Leu
   225 230 235 240
   Leu Gln Gly Gly Gln Tyr Val Gln Leu Ile Glu Gln Arg Gln Gly Val
   245 250 255
   Arg Ile Ala Cys Ile Glu Glu Ile Ala Leu Arg Met Gly Phe Ile Asp
   260 265 270
   Ala Asp Thr Leu His Arg Leu Gly Arg Glu Leu Gly Thr Ser Gly Tyr
   275 280 285
   Gly Ala Tyr Leu Met Glu Val Ala Thr Arg Ala Gly Thr Glu
   290 295 300
<210> 32
   <211> 299
   <212> PRT
   <213> Streptomyces ambofaciens
<400> 32
   Val Met Ser Gly Arg Pro Gly Met Lys Gly Ile Ile Leu Ala Gly Gly
   1               5                   10                  15
   Gly Gly Thr Arg Leu Arg Pro Leu Thr Gly Thr Leu Ser Lys Gln Leu
   20 25 30
   Leu Pro Val Tyr Asp Lys Pro Met Ile Tyr Tyr Pro Leu Ser Val Leu
   35 40 45
   Met Leu Gly Gly Ile Arg Glu Ile Leu Val Val Ser Ser Thr Gln His
   50 55 60
   Ile Glu Leu Phe Gln Arg Leu Leu Gly Asp Gly Ser Arg Leu Gly Leu
   65 70 75 80
   Asp Ile Thr Tyr Ala Glu Gln Ala Glu Pro Glu Gly Ile Ala Gln Ala
   85 90 95
   Ile Thr Ile Gly Thr Asp His Ile Gly Asp Ser Pro Val Ala Leu Ile
   100 105 110
   Leu Gly Asp Asn Ile Phe His Gly Pro Gly Phe Ser Ala Val Leu Gln
   115 120 125
   Gly Ser Ile Arg His Leu Asp Gly Cys Val Leu Phe Gly Tyr Pro Val
   130 135 140
   Ser Asp Pro Lys Arg Tyr Gly Val Gly Glu Ile Asp Asp Gln Gly Val
   145 150 155 160
   Leu Leu Ser Leu Glu Glu Lys Pro Ala Arg Pro Arg Ser Asn Leu Ala
   165 170 175
   Val Thr Gly Leu Tyr Leu Tyr Asp Asn Asp Val Val Asp Ile Ala Lys
   180 185 190
   Asn Ile Arg Pro Ser Ala Arg Gly Glu Leu Glu Ile Thr Asp Val Asn
   195 200 205
   Arg Thr Tyr Leu Glu Gln Lys Arg Ala Arg Leu Ile Glu Leu Gly His
   210 215 220
   Gly Phe Ala Trp Leu Asp Met Gly Thr His Asp Ser Leu Leu Gln Gly
   225 230 235 240
   Gly Gln Tyr Val Gln Leu Ile Glu Gln Arg Gln Gly Val Arg Ile Ala
   245 250 255
   Cys Ile Glu Glu Ile Ala Leu Arg Met Gly Phe Ile Asp Ala Asp Thr
   260 265 270
   Leu His Arg Leu Gly Arg Glu Leu Gly Thr Ser Gly Tyr Gly Ala Tyr
   275 280 285
   Leu Met Glu Val Ala Thr Arg Ala Gly Thr Glu
   290 295
<210> 33
   <211> 293
   <212> PRT
   <213> Streptomyces ambofaciens
<400> 33
   Gly Met Lys Gly Ile Ile Leu Ala Gly Gly Gly Gly Thr Arg Leu Arg
   1 5 10 15
   Pro Leu Thr Gly Thr Leu Ser Lys Gln Leu Leu Pro Val Tyr Asp Lys
   20 25 30
   Pro Met Ile Tyr Tyr Pro Leu Ser Val Leu Met Leu Gly Gly Ile Arg
   35 40 45
   Glu Ile Leu Val Val Ser Ser Thr Gln His Ile Glu Leu Phe Gln Arg
   50 55 60
   Leu Leu Gly Asp Gly Ser Arg Leu Gly Leu Asp Ile Thr Tyr Ala Glu
   65 70 75 80
   Gln Ala Glu Pro Glu Gly Ile Ala Gln Ala Ile Thr Ile Gly Thr Asp
   85 90 95
   His Ile Gly Asp Ser Pro Val Ala Leu Ile Leu Gly Asp Asn Ile Phe
   100 105 110
   His Gly Pro Gly Phe Ser Ala Val Leu Gln Gly Ser Ile Arg His Leu
   115 120 125
   Asp Gly Cys Val Leu Phe Gly Tyr Pro Val Ser Asp Pro Lys Arg Tyr
   130 135 140
   Gly Val Gly Glu Ile Asp Asp Gln Gly Val Leu Leu Ser Leu Glu Glu
   145 150 155 160
   Lys Pro Ala Arg Pro Arg Ser Asn Leu Ala Val Thr Gly Leu Tyr Leu
   165 170 175
   Tyr Asp Asn Asp Val Val Asp Ile Ala Lys Asn Ile Arg Pro Ser Ala
   180 185 190
   Arg Gly Glu Leu Glu Ile Thr Asp Val Asn Arg Thr Tyr Leu Glu Gln
   195 200 205
   Lys Arg Ala Arg Leu Ile Glu Leu Gly His Gly Phe Ala Trp Leu Asp
   210 215 220
   Met Gly Thr His Asp Ser Leu Leu Gln Gly Gly Gln Tyr Val Gln Leu
   225 230 235 240
   Ile Glu Gln Arg Gln Gly Val Arg Ile Ala Cys Ile Glu Glu Ile Ala
   245 250 255
   Leu Arg Met Gly Phe Ile Asp Ala Asp Thr Leu His Arg Leu Gly Arg
   260 265 270
   Glu Leu Gly Thr Ser Gly Tyr Gly Ala Tyr Leu Met Glu Val Ala Thr
   275 280 285
   Arg Ala Gly Thr Glu
   290
<210> 34
   <211> 1038
   <212> DNA
   <213> Streptomyces ambofaciens
<220>
   <221> CDS
   <222> (1)..(1038)
<400> 34
   gtg cag gca ccg aat gag acg ccg cgc cgg ccc gcc cgc tcc gcc ggc 48
   Val Gln Ala Pro Asn Glu Thr Pro Arg Arg Pro Ala Arg Ser Ala Gly
   1 5 10 15
   cga cgg ccg ccg gcc cgg atc ctc gtc acc ggg ggc gcc ggc ttc atc 96
   Arg Arg Pro Pro Ala Arg Ile Leu Val Thr Gly Gly Ala Gly Phe Ile
   20 25 30
   ggc tcg cgc ttc gtg aac gcg ctg ctg gac ggc tcc ctg ccg gag ttc 144
   Gly Ser Arg Phe Val Asn Ala Leu Leu Asp Gly Ser Leu Pro Glu Phe
   35 40 45
   ggc aaa ccc gag gtg agg gtg ctc gac gcg ctc acc tac gcg ggc aac 192
   Gly Lys Pro Glu Val Arg Val Leu Asp Ala Leu Thr Tyr Ala Gly Asn
   50 55 60
   ctg gcc aat ctg gcc ccg gtg ggc gac tgt ccc cgg ctg cgg atc ttc 240
   Leu Ala Asn Leu Ala Pro Val Gly Asp Cys Pro Arg Leu Arg Ile Phe
   65 70 75 80
   ccg ggg gac atc cgc gac cgc ggc gcg gtc acc cag gcg atg gcg ggg 288
   Pro Gly Asp Ile Arg Asp Arg Gly Ala Val Thr Gln Ala Met Ala Gly
   85 90 95
   gtc gac ctg gtg gtg cac ttc gcg gcc gag tcg cac gtg gac cgc tcg 336
   Val Asp Leu Val Val His Phe Ala Ala Glu Ser His Val Asp Arg Ser
   100 105 110
   atc gac gac gcc gac gcc ttc gtg cgc acc aac gtg ctg ggc acc cag 384
   Ile Asp Asp Ala Asp Ala Phe Val Arg Thr Asn Val Leu Gly Thr Gln
   115 120 125
   gtc ctc ctc cag gag gca ctg gcc gta cgc ccc ggg ctg ttc gtg cac 432
   Val Leu Leu Gln Glu Ala Leu Ala Val Arg Pro Gly Leu Phe Val His
   130 135 140
   gtc tcg acg gac gag gtg tac ggc tcc atc gag gag ggg tcc tgg ccc      480
   Val Ser Thr Asp Glu Val Tyr Gly Ser Ile Glu Glu Gly Ser Trp Pro
   145                 150                 155                 160
   gag gag cac ccg ctg aac ccc aac tcg ccc tac gcc gcc tcg aag gcg      528
   Glu Glu His Pro Leu Asn Pro Asn Ser Pro Tyr Ala Ala Ser Lys Ala
                   165                 170                 175
   tcc tcc gac ctg ctg gcg ctg gcc cac cac cgc acg cac gga ctg ccg      576
   Ser Ser Asp Leu Leu Ala Leu Ala His His Arg Thr His Gly Leu Pro
               180                 185                 190
   gtg tgc gtc acc cgc tgc tcc aac aac tac ggg ccc tac cag tac ccg      624
   Val Cys Val Thr Arg Cys Ser Asn Asn Tyr Gly Pro Tyr Gln Tyr Pro
           195                 200                 205
   gag aag atc atc ccg ctg ttc acc agc agc ctc ctc gac ggc ggg acc      672
   Glu Lys Ile Ile Pro Leu Phe Thr Ser Ser Leu Leu Asp Gly Gly Thr
       210                 215                 220
   gtc ccg ctc tac ggg gac ggc ggc aac cgg cgc gac tgg ctg cac gtg      720
   Val Pro Leu Tyr Gly Asp Gly Gly Asn Arg Arg Asp Trp Leu His Val
   225                 230                 235                 240
   gac gac cac tgc cgg ggc atc gcc ctg gtg gcc cgg ggc ggc cgg ccc      768
   Asp Asp His Cys Arg Gly Ile Ala Leu Val Ala Arg Gly Gly Arg Pro
                   245                 250                 255
   ggc gag gtc tac aac atc ggc ggc ggc acc gag ctg agc aac gtc gag      816
   Gly Glu Val Tyr Asn Ile Gly Gly Gly Thr Glu Leu Ser Asn Val Glu
               260                 265                 270
   ctc acg gag cgt ctg ctg aaa ctg tgc gga gcc gac tgg tcg gcg gtg      864
   Leu Thr Glu Arg Leu Leu Lys Leu Cys Gly Ala Asp Trp Ser Ala Val
           275                 280                 285
   cgg cgg gtg ccc gac cgc aag ggc cac gac cgg cgc tac tcc gtc gac      912
   Arg Arg Val Pro Asp Arg Lys Gly His Asp Arg Arg Tyr Ser Val Asp
       290                 295                 300
   tac acc aag atc gcg gac gag ctg ggt tac gcg ccg cgg atc acc atc      960
   Tyr Thr Lys Ile Ala Asp Glu Leu Gly Tyr Ala Pro Arg Ile Thr Ile
   305                 310                 315                 320
   gac gaa ggg ctg gag cgg acc gtg cac tgg tac cgg gag aac cgc gcg     1008
   Asp Glu Gly Leu Glu Arg Thr Val His Trp Tyr Arg Glu Asn Arg Ala
                   325                 330                 335
   tgg tgg gcg ccc gcg aag agg ggg cga tga                             1038
   Trp Trp Ala Pro Ala Lys Arg Gly Arg
               340                 345
<210> 35
   <211> 345
   <212> PRT
   <213> Streptomyces ambofaciens
<400> 35
   Val Gln Ala Pro Asn Glu Thr Pro Arg Arg Pro Ala Arg Ser Ala Gly
   1               5                   10                  15
   Arg Arg Pro Pro Ala Arg Ile Leu Val Thr Gly Gly Ala Gly Phe Ile
               20                  25                  30
   Gly Ser Arg Phe Val Asn Ala Leu Leu Asp Gly Ser Leu Pro Glu Phe
   35 40 45
   Gly Lys Pro Glu Val Arg Val Leu Asp Ala Leu Thr Tyr Ala Gly Asn
   50 55 60
   Leu Ala Asn Leu Ala Pro Val Gly Asp Cys Pro Arg Leu Arg Ile Phe
   65 70 75 80
   Pro Gly Asp Ile Arg Asp Arg Gly Ala Val Thr Gln Ala Met Ala Gly
   85 90 95
   Val Asp Leu Val Val His Phe Ala Ala Glu Ser His Val Asp Arg Ser
   100 105 110
   Ile Asp Asp Ala Asp Ala Phe Val Arg Thr Asn Val Leu Gly Thr Gln
   115 120 125
   Val Leu Leu Gln Glu Ala Leu Ala Val Arg Pro Gly Leu Phe Val His
   130 135 140
   Val Ser Thr Asp Glu Val Tyr Gly Ser Ile Glu Glu Gly Ser Trp Pro
   145 150 155 160
   Glu Glu His Pro Leu Asn Pro Asn Ser Pro Tyr Ala Ala Ser Lys Ala
   165 170 175
   Ser Ser Asp Leu Leu Ala Leu Ala His His Arg Thr His Gly Leu Pro
   180 185 190
   Val Cys Val Thr Arg Cys Ser Asn Asn Tyr Gly Pro Tyr Gln Tyr Pro
   195 200 205
   Glu Lys Ile Ile Pro Leu Phe Thr Ser Ser Leu Leu Asp Gly Gly Thr
   210 215 220
   Val Pro Leu Tyr Gly Asp Gly Gly Asn Arg Arg Asp Trp Leu His Val
   225 230 235 240
   Asp Asp His Cys Arg Gly Ile Ala Leu Val Ala Arg Gly Gly Arg Pro
   245 250 255
   Gly Glu Val Tyr Asn Ile Gly Gly Gly Thr Glu Leu Ser Asn Val Glu
   260 265 270
   Leu Thr Glu Arg Leu Leu Lys Leu Cys Gly Ala Asp Trp Ser Ala Val
   275 280 285
   Arg Arg Val Pro Asp Arg Lys Gly His Asp Arg Arg Tyr Ser Val Asp
   290 295 300
   Tyr Thr Lys Ile Ala Asp Glu Leu Gly Tyr Ala Pro Arg Ile Thr Ile
   305 310 315 320
   Asp Glu Gly Leu Glu Arg Thr Val His Trp Tyr Arg Glu Asn Arg Ala
   325 330 335
   Trp Trp Ala Pro Ala Lys Arg Gly Arg
   340 345
<210> 36
   <211> 804
   <212> DNA
   <213> Streptomyces ambofaciens
<220>
   <221> CDS
   <222> (1)..(804)
<220>
   <221> CDS2
   <222> (7)..(804)
<220>
   <221> CDS3
   <222> (22)..(804)
<400> 36
   atg acg gtg acg acc gca tcc gtg gac ccg ctc gac ctg tgg ctc cgc      48
   Met Thr Val Thr Thr Ala Ser Val Asp Pro Leu Asp Leu Trp Leu Arg
   1               5                   10                  15
   cgg tac cag ccg tcc gcg tca ccc gcc gtc cgg ctg gtg tgc ttc ccg      96
   Arg Tyr Gln Pro Ser Ala Ser Pro Ala Val Arg Leu Val Cys Phe Pro
               20                  25                  30
   cac gcg ggc ggc tcg gcg agt tcg ttc ctg ccg ttc acc cgg cag ctg     144
   His Ala Gly Gly Ser Ala Ser Ser Phe Leu Pro Phe Thr Arg Gln Leu
           35                  40                  45
   ccg gac cgg atc gag gtc gtg gcc gtc cag tac ccc ggg cgc cag gac     192
   Pro Asp Arg Ile Glu Val Val Ala Val Gln Tyr Pro Gly Arg Gln Asp
       50                  55                  60
   cgc agg agc gaa ccg ctg gtc gac acc atc gag gga ctg gcc gag ccc     240
   Arg Arg Ser Glu Pro Leu Val Asp Thr Ile Glu Gly Leu Ala Glu Pro
   65                  70                  75                  80
   ctg gcc ggc ctg ctg gag gcg cag gcc ggc ccc ccg gtg gtg ctg ttc     288
   Leu Ala Gly Leu Leu Glu Ala Gln Ala Gly Pro Pro Val Val Leu Phe
                   85                  90                  95
   ggg cac agc atg ggc gcg ctg gtg gcc tac gag gtc gcc cgc gcg ctc     336
   Gly His Ser Met Gly Ala Leu Val Ala Tyr Glu Val Ala Arg Ala Leu
               100                 105                 110
   cag cgg cgg gga gcg gct ccg gtg cgc ctg gtg gtc tcc ggg cgc cgg     384
   Gln Arg Arg Gly Ala Ala Pro Val Arg Leu Val Val Ser Gly Arg Arg
           115                 120                 125
   gcc ccc gcc gtc gac cgg ccg atg acc gtg cac ctc tac gac gac gac     432
   Ala Pro Ala Val Asp Arg Pro Met Thr Val His Leu Tyr Asp Asp Asp
       130                 135                 140
   cgg ctg gtc gag gaa ctc cgc aag ctc gac ggc acc gac agc cag gtg     480
   Arg Leu Val Glu Glu Leu Arg Lys Leu Asp Gly Thr Asp Ser Gln Val
   145                 150                 155                 160
   ttc gcc gat ccg gag ctg ctc cgg ctg gtg ctg ccc gtg atc cgc aac      528
   Phe Ala Asp Pro Glu Leu Leu Arg Leu Val Leu Pro Val Ile Arg Asn
                   165                 170                 175
   gac tac cgg gcc gtg gcg gcc tac gcc cac cgc ccg ggg gcg ccg ctg      576
   Asp Tyr Arg Ala Val Ala Ala Tyr Ala His Arg Pro Gly Ala Pro Leu
               180                 185                 190
   gac tgc ccc ctc acc gtg ttc acc ggc gcc gac gac ccc acc gtg acc      624
   Asp Cys Pro Leu Thr Val Phe Thr Gly Ala Asp Asp Pro Thr Val Thr
           195                 200                 205
   gcg gcc gag gcg gcg gcc tgg cac gag gcg gcg gcg tcc gac gtc gag      672
   Ala Ala Glu Ala Ala Ala Trp His Glu Ala Ala Ala Ser Asp Val Glu
       210                 215                 220
   acg cgc acc ttc ccc ggt ggc cac ttc ttc ccg tac cag cgg acc gcg      720
   Thr Arg Thr Phe Pro Gly Gly His Phe Phe Pro Tyr Gln Arg Thr Ala
   225                 230                 235                 240
   gag gtg tgc ggg gcc ctg gtc gac acg ctc gag ccg ctg ctg tcg gcc      768
   Glu Val Cys Gly Ala Leu Val Asp Thr Leu Glu Pro Leu Leu Ser Ala
                   245                 250                 255
   ggg acg cgc ggt gtc cgg cgg gtc cgc ccg ggg tga                      804
   Gly Thr Arg Gly Val Arg Arg Val Arg Pro Gly
               260                 265
<210> 37
   <211> 267
   <212> PRT
   <213> Streptomyces ambofaciens
<400> 37
   Met Thr Val Thr Thr Ala Ser Val Asp Pro Leu Asp Leu Trp Leu Arg
   1               5                   10                  15
   Arg Tyr Gln Pro Ser Ala Ser Pro Ala Val Arg Leu Val Cys Phe Pro
               20                  25                  30
   His Ala Gly Gly Ser Ala Ser Ser Phe Leu Pro Phe Thr Arg Gln Leu
           35                  40                  45
   Pro Asp Arg Ile Glu Val Val Ala Val Gln Tyr Pro Gly Arg Gln Asp
       50                  55                  60
   Arg Arg Ser Glu Pro Leu Val Asp Thr Ile Glu Gly Leu Ala Glu Pro
   65                  70                  75                  80
   Leu Ala Gly Leu Leu Glu Ala Gln Ala Gly Pro Pro Val Val Leu Phe
                   85                  90                  95
   Gly His Ser Met Gly Ala Leu Val Ala Tyr Glu Val Ala Arg Ala Leu
               100                 105                 110
   Gln Arg Arg Gly Ala Ala Pro Val Arg Leu Val Val Ser Gly Arg Arg
           115                 120                 125
   Ala Pro Ala Val Asp Arg Pro Met Thr Val His Leu Tyr Asp Asp Asp
       130                 135                 140
   Arg Leu Val Glu Glu Leu Arg Lys Leu Asp Gly Thr Asp Ser Gln Val
   145                 150                 155                 160
   Phe Ala Asp Pro Glu Leu Leu Arg Leu Val Leu Pro Val Ile Arg Asn
                   165                 170                 175
   Asp Tyr Arg Ala Val Ala Ala Tyr Ala His Arg Pro Gly Ala Pro Leu
               180                 185                 190
   Asp Cys Pro Leu Thr Val Phe Thr Gly Ala Asp Asp Pro Thr Val Thr
           195                 200                 205
   Ala Ala Glu Ala Ala Ala Trp His Glu Ala Ala Ala Ser Asp Val Glu
       210                 215                 220
   Thr Arg Thr Phe Pro Gly Gly His Phe Phe Pro Tyr Gln Arg Thr Ala
   225                 230                 235                 240
   Glu Val Cys Gly Ala Leu Val Asp Thr Leu Glu Pro Leu Leu Ser Ala
                   245                 250                 255
   Gly Thr Arg Gly Val Arg Arg Val Arg Pro Gly
               260                 265
<210> 38
   <211> 265
   <212> PRT
   <213> Streptomyces ambofaciens
<400> 38
   Val Thr Thr Ala Ser Val Asp Pro Leu Asp Leu Trp Leu Arg Arg Tyr
   1 5 10 15
   Gln Pro Ser Ala Ser Pro Ala Val Arg Leu Val Cys Phe Pro His Ala
   20 25 30
   Gly Gly Ser Ala Ser Ser Phe Leu Pro Phe Thr Arg Gln Leu Pro Asp
   35 40 45
   Arg Ile Glu Val Val Ala Val Gln Tyr Pro Gly Arg Gln Asp Arg Arg
   50 55 60
   Ser Glu Pro Leu Val Asp Thr Ile Glu Gly Leu Ala Glu Pro Leu Ala
   65 70 75 80
   Gly Leu Leu Glu Ala Gln Ala Gly Pro Pro Val Val Leu Phe Gly His
   85 90 95
   Ser Met Gly Ala Leu Val Ala Tyr Glu Val Ala Arg Ala Leu Gln Arg
   100 105 110
   Arg Gly Ala Ala Pro Val Arg Leu Val Val Ser Gly Arg Arg Ala Pro
   115 120 125
   Ala Val Asp Arg Pro Met Thr Val His Leu Tyr Asp Asp Asp Arg Leu
   130 135 140
   Val Glu Glu Leu Arg Lys Leu Asp Gly Thr Asp Ser Gln Val Phe Ala
   145 150 155 160
   Asp Pro Glu Leu Leu Arg Leu Val Leu Pro Val Ile Arg Asn Asp Tyr
   165 170 175
   Arg Ala Val Ala Ala Tyr Ala His Arg Pro Gly Ala Pro Leu Asp Cys
   180 185 190
   Pro Leu Thr Val Phe Thr Gly Ala Asp Asp Pro Thr Val Thr Ala Ala
   195 200 205
   Glu Ala Ala Ala Trp His Glu Ala Ala Ala Ser Asp Val Glu Thr Arg
   210 215 220
   Thr Phe Pro Gly Gly His Phe Phe Pro Tyr Gln Arg Thr Ala Glu Val
   225 230 235 240
   Cys Gly Ala Leu Val Asp Thr Leu Glu Pro Leu Leu Ser Ala Gly Thr
   245 250 255
   Arg Gly Val Arg Arg Val Arg Pro Gly
   260 265
<210> 39
   <211> 260
   <212> PRT
   <213> Streptomyces ambofaciens
<400> 39
   Val Asp Pro Leu Asp Leu Trp Leu Arg Arg Tyr Gln Pro Ser Ala Ser
   1 5 10 15
   Pro Ala Val Arg Leu Val Cys Phe Pro His Ala Gly Gly Ser Ala Ser
   20 25 30
   Ser Phe Leu Pro Phe Thr Arg Gln Leu Pro Asp Arg Ile Glu Val Val
   35 40 45
   Ala Val Gln Tyr Pro Gly Arg Gln Asp Arg Arg Ser Glu Pro Leu Val
   50 55 60
   Asp Thr Ile Glu Gly Leu Ala Glu Pro Leu Ala Gly Leu Leu Glu Ala
   65 70 75 80
   Gln Ala Gly Pro Pro Val Val Leu Phe Gly His Ser Met Gly Ala Leu
   85 90 95
   Val Ala Tyr Glu Val Ala Arg Ala Leu Gln Arg Arg Gly Ala Ala Pro
   100 105 110
   Val Arg Leu Val Val Ser Gly Arg Arg Ala Pro Ala Val Asp Arg Pro
   115 120 125
   Met Thr Val His Leu Tyr Asp Asp Asp Arg Leu Val Glu Glu Leu Arg
   130 135 140
   Lys Leu Asp Gly Thr Asp Ser Gln Val Phe Ala Asp Pro Glu Leu Leu
   145 150 155 160
   Arg Leu Val Leu Pro Val Ile Arg Asn Asp Tyr Arg Ala Val Ala Ala
   165 170 175
   Tyr Ala His Arg Pro Gly Ala Pro Leu Asp Cys Pro Leu Thr Val Phe
   180 185 190
   Thr Gly Ala Asp Asp Pro Thr Val Thr Ala Ala Glu Ala Ala Ala Trp
   195 200 205
   His Glu Ala Ala Ala Ser Asp Val Glu Thr Arg Thr Phe Pro Gly Gly
   210 215 220
   His Phe Phe Pro Tyr Gln Arg Thr Ala Glu Val Cys Gly Ala Leu Val
   225 230 235 240
   Asp Thr Leu Glu Pro Leu Leu Ser Ala Gly Thr Arg Gly Val Arg Arg
   245 250 255
   Val Arg Pro Gly
   260
<210> 40
   <211> 1410
   <212> DNA
   <213> Streptomyces ambofaciens
<220>
   <221> CDS
   <222> (1)..(1410)
<220>
   <221> CDS2
   <222> (46)..(1410)
<400> 40
   gtg ggc acg gtc gag tac gcc gtc cac cgg cgt acc gcg gaa cgg gtg      48
   Val Gly Thr Val Glu Tyr Ala Val His Arg Arg Thr Ala Glu Arg Val
   1               5                   10                  15
   agg gtc tcc gcc gac acc ctg gac agc ccg gtc acc gcg ctg gcg gag      96
   Arg Val Ser Ala Asp Thr Leu Asp Ser Pro Val Thr Ala Leu Ala Glu
               20                  25                  30
   gtg ccc cgc tgg ctg gag gaa tac cac cgg gcg cac cgc ttc cac gtc      144
   Val Pro Arg Trp Leu Glu Glu Tyr His Arg Ala His Arg Phe His Val
           35                  40                  45
   gag ccg atc ccc ttc gac cgg ctc cgg cgg tgg tcc ttc gag ccg ggc      192
   Glu Pro Ile Pro Phe Asp Arg Leu Arg Arg Trp Ser Phe Glu Pro Gly
       50                  55                  60
   acc ggc gac ctg cgg cac gag acg ggc cgc ttc ttc tcc gtg gag ggg      240
   Thr Gly Asp Leu Arg His Glu Thr Gly Arg Phe Phe Ser Val Glu Gly
   65                  70                  75                  80
   ctg cgc acc agc tcg gac gcc gat ccg gtc gcc cgt gtc cag ccg atc      288
   Leu Arg Thr Ser Ser Asp Ala Asp Pro Val Ala Arg Val Gln Pro Ile
                   85                  90                  95
   atc gtg cag ccc gag gtg ggg ctg ctc ggc atc ctg gcc cgg gag ttc      336
   Ile Val Gln Pro Glu Val Gly Leu Leu Gly Ile Leu Ala Arg Glu Phe
               100                 105                 110
   gac ggg gtg ctg cac ttc ctg atg cag gcc aaa ccc gag ccc ggc aac      384
   Asp Gly Val Leu His Phe Leu Met Gln Ala Lys Pro Glu Pro Gly Asn
           115                 120                 125
   gtc aac ggg ctg cag atc tcc ccc acg gtg cag gcc acg cgc agc aac      432
   Val Asn Gly Leu Gln Ile Ser Pro Thr Val Gln Ala Thr Arg Ser Asn
       130                 135                 140
   ttc gac gag gtg cac cac ggc cgg tcc acc ccg ttc ctc gac cac ttc      480
   Phe Asp Glu Val His His Gly Arg Ser Thr Pro Phe Leu Asp His Phe
   145                 150                 155                 160
   atc cac cgc ccc ggc cgc cgg gtc ctg atc gac agc atc cag tcc gaa      528
   Ile His Arg Pro Gly Arg Arg Val Leu Ile Asp Ser Ile Gln Ser Glu
                   165                 170                 175
   cag ggc gac tgg ttc ctg cac aag cgc aac cgc aac atg gtc gtc gag      576
   Gln Gly Asp Trp Phe Leu His Lys Arg Asn Arg Asn Met Val Val Glu
               180                 185                 190
   atc gac acc gac atc gag gcc gac gcc gcg ttc cgc tgg ctg acc ctc      624
   Ile Asp Thr Asp Ile Glu Ala Asp Ala Ala Phe Arg Trp Leu Thr Leu
           195                 200                 205
   ggg cag atc cgc cgg ctg atg ctc cag gac gac ctc gtc aac atg gac      672
   Gly Gln Ile Arg Arg Leu Met Leu Gln Asp Asp Leu Val Asn Met Asp
       210                 215                 220
   acc cgc agt gtg ctg gcc tgt ctg ccc acc gcg cac ggc acg ccc gac      720
   Thr Arg Ser Val Leu Ala Cys Leu Pro Thr Ala His Gly Thr Pro Asp
   225                 230                 235                 240
   gac ggt gac gac tcc ttc ccg gcg gcg ctg cgc cgc tcc ctc tac ggg      768
   Asp Gly Asp Asp Ser Phe Pro Ala Ala Leu Arg Arg Ser Leu Tyr Gly
                   245                 250                 255
   gag acc gcg ccg ttg cac gat ctg cac gcc atc acc agc tgc ctc acc      816
   Glu Thr Ala Pro Leu His Asp Leu His Ala Ile Thr Ser Cys Leu Thr
               260                 265                 270
   gac gtc cgg gcg ctg cgg gtg ctg cgc cag cag agc gtg ccg ctc gac      864
   Asp Val Arg Ala Leu Arg Val Leu Arg Gln Gln Ser Val Pro Leu Asp
           275                 280                 285
   gac gcc cgg cgg gac ggc tgg gag cgg acc ggg agc gcg atc cgg cat      912
   Asp Ala Arg Arg Asp Gly Trp Glu Arg Thr Gly Ser Ala Ile Arg His
       290                 295                 300
   cgc agc ggc agg cat ttc gag atc atg gcg gtg gag gtg acc gcg gag      960
   Arg Ser Gly Arg His Phe Glu Ile Met Ala Val Glu Val Thr Ala Glu
   305                 310                 315                     320
   cgc cgt gaa gtg gcc tcg tgg acc cag ccg ttg ctg cgc ccg tgc tcg     1008
   Arg Arg Glu Val Ala Ser Trp Thr Gln Pro Leu Leu Arg Pro Cys Ser
                   325                 330                 335
   cag gga ctg gcg gcc ctg atc acc cgg cgg atc aac ggg gtg ctg cac     1056
   Gln Gly Leu Ala Ala Leu Ile Thr Arg Arg Ile Asn Gly Val Leu His
               340                 345                 350
   gcc ctg gtg gcg gcg cgg tcg gag gtc ggc acg ctc aac gtc gcc gag     1104
   Ala Leu Val Ala Ala Arg Ser Glu Val Gly Thr Leu Asn Val Ala Glu
           355                 360                 365
   ttc gga ccg acc gtc cag tgc cgg ccc gac gag gcg gac ggc cag tcg     1152
   Phe Gly Pro Thr Val Gln Cys Arg Pro Asp Glu Ala Asp Gly Gln Ser
       370                 375                 380
   ccc ccg tac ctg gac cgg gtg ctg acg gcc gga gcc gac cgc gtc cgc     1200
   Pro Pro Tyr Leu Asp Arg Val Leu Thr Ala Gly Ala Asp Arg Val Arg
   385                 390                 395                     400
   tac gac gtg gtg cag tcg gag gag ggc ggg cgc ttc tac cac gcg cgc     1248
   Tyr Asp Val Val Gln Ser Glu Glu Gly Gly Arg Phe Tyr His Ala Arg
                   405                 410                 415
   aac cgc tat ctg gtg gtc gag gcg ggg ccg gag ctc gac acg ggc tgc     1296
   Asn Arg Tyr Leu Val Val Glu Ala Gly Pro Glu Leu Asp Thr Gly Cys
               420                 425                 430
   ccg ccc ggc ttc tgc tgg gct acc ttc ggc cag ctc acc gaa ctg ctc     1344
   Pro Pro Gly Phe Cys Trp Ala Thr Phe Gly Gln Leu Thr Glu Leu Leu
           435                 440                 445
   gcg cac ggc aac tat ctc aac gtc gaa ctc cgc acc ctc atg gcg tgc     1392
   Ala His Gly Asn Tyr Leu Asn Val Glu Leu Arg Thr Leu Met Ala Cys
       450                 455                 460
   gca cac gcc tcc tac tga                                             1410
   Ala His Ala Ser Tyr
   465
<210> 41
   <211> 469
   <212> PRT
   <213> Streptomyces ambofaciens
<400> 41
   Val Gly Thr Val Glu Tyr Ala Val His Arg Arg Thr Ala Glu Arg Val
   1               5                   10                  15
   Arg Val Ser Ala Asp Thr Leu Asp Ser Pro Val Thr Ala Leu Ala Glu
               20                  25                  30
   Val Pro Arg Trp Leu Glu Glu Tyr His Arg Ala His Arg Phe His Val
           35                  40                  45
   Glu Pro Ile Pro Phe Asp Arg Leu Arg Arg Trp Ser Phe Glu Pro Gly
       50                  55                  60
   Thr Gly Asp Leu Arg His Glu Thr Gly Arg Phe Phe Ser Val Glu Gly
   65                  70                  75                  80
   Leu Arg Thr Ser Ser Asp Ala Asp Pro Val Ala Arg Val Gln Pro Ile
                   85                  90                  95
   Ile Val Gln Pro Glu Val Gly Leu Leu Gly Ile Leu Ala Arg Glu Phe
               100                 105                 110
   Asp Gly Val Leu His Phe Leu Met Gln Ala Lys Pro Glu Pro Gly Asn
           115                 120                 125
   Val Asn Gly Leu Gln Ile Ser Pro Thr Val Gln Ala Thr Arg Ser Asn
       130                 135                 140
   Phe Asp Glu Val His His Gly Arg Ser Thr Pro Phe Leu Asp His Phe
   145                 150                 155                 160
   Ile His Arg Pro Gly Arg Arg Val Leu Ile Asp Ser Ile Gln Ser Glu
                   165                 170                 175
   Gln Gly Asp Trp Phe Leu His Lys Arg Asn Arg Asn Met Val Val Glu
               180                 185                 190
   Ile Asp Thr Asp Ile Glu Ala Asp Ala Ala Phe Arg Trp Leu Thr Leu
           195                 200                 205
   Gly Gln Ile Arg Arg Leu Met Leu Gln Asp Asp Leu Val Asn Met Asp
       210                 215                 220
   Thr Arg Ser Val Leu Ala Cys Leu Pro Thr Ala His Gly Thr Pro Asp
   225                 230                 235                 240
   Asp Gly Asp Asp Ser Phe Pro Ala Ala Leu Arg Arg Ser Leu Tyr Gly
                   245                 250                 255
   Glu Thr Ala Pro Leu His Asp Leu His Ala Ile Thr Ser Cys Leu Thr
               260                 265                 270
   Asp Val Arg Ala Leu Arg Val Leu Arg Gln Gln Ser Val Pro Leu Asp
           275                 280                 285
   Asp Ala Arg Arg Asp Gly Trp Glu Arg Thr Gly Ser Ala Ile Arg His
       290                 295                 300
   Arg Ser Gly Arg His Phe Glu Ile Met Ala Val Glu Val Thr Ala Glu
   305 310 315 320
   Arg Arg Glu Val Ala Ser Trp Thr Gln Pro Leu Leu Arg Pro Cys Ser
   325 330 335
   Gln Gly Leu Ala Ala Leu Ile Thr Arg Arg Ile Asn Gly Val Leu His
   340 345 350
   Ala Leu Val Ala Ala Arg Ser Glu Val Gly Thr Leu Asn Val Ala Glu
   355 360 365
   Phe Gly Pro Thr Val Gln Cys Arg Pro Asp Glu Ala Asp Gly Gln Ser
   370 375 380
   Pro Pro Tyr Leu Asp Arg Val Leu Thr Ala Gly Ala Asp Arg Val Arg
   385 390 395 400
   Tyr Asp Val Val Gln Ser Glu Glu Gly Gly Arg Phe Tyr His Ala Arg
   405 410 415
   Asn Arg Tyr Leu Val Val Glu Ala Gly Pro Glu Leu Asp Thr Gly Cys
   420 425 430
   Pro Pro Gly Phe Cys Trp Ala Thr Phe Gly Gln Leu Thr Glu Leu Leu
   435 440 445
   Ala His Gly Asn Tyr Leu Asn Val Glu Leu Arg Thr Leu Met Ala Cys
   450 455 460
   Ala His Ala Ser Tyr
   465
<210> 42
   <211> 454
   <212> PRT
   <213> Streptomyces ambofaciens
<400> 42
   Val Arg Val Ser Ala Asp Thr Leu Asp Ser Pro Val Thr Ala Leu Ala
   1               5                   10                  15
   Glu Val Pro Arg Trp Leu Glu Glu Tyr His Arg Ala His Arg Phe His
               20                  25                  30
   Val Glu Pro Ile Pro Phe Asp Arg Leu Arg Arg Trp Ser Phe Glu Pro
           35                  40                  45
   Gly Thr Gly Asp Leu Arg His Glu Thr Gly Arg Phe Phe Ser Val Glu
       50                  55                  60
   Gly Leu Arg Thr Ser Ser Asp Ala Asp Pro Val Ala Arg Val Gln Pro
   65                  70                  75                  80
   Ile Ile Val Gln Pro Glu Val Gly Leu Leu Gly Ile Leu Ala Arg Glu
                   85                  90                  95
   Phe Asp Gly Val Leu His Phe Leu Met Gln Ala Lys Pro Glu Pro Gly
               100                 105                 110
   Asn Val Asn Gly Leu Gln Ile Ser Pro Thr Val Gln Ala Thr Arg Ser
           115                 120                 125
   Asn Phe Asp Glu Val His His Gly Arg Ser Thr Pro Phe Leu Asp His
       130                 135                 140
   Phe Ile His Arg Pro Gly Arg Arg Val Leu Ile Asp Ser Ile Gln Ser
   145                 150                 155                 160
   Glu Gln Gly Asp Trp Phe Leu His Lys Arg Asn Arg Asn Met Val Val
                   165                 170                 175
   Glu Ile Asp Thr Asp Ile Glu Ala Asp Ala Ala Phe Arg Trp Leu Thr
               180                 185                 190
   Leu Gly Gln Ile Arg Arg Leu Met Leu Gln Asp Asp Leu Val Asn Met
           195                 200                 205
   Asp Thr Arg Ser Val Leu Ala Cys Leu Pro Thr Ala His Gly Thr Pro
       210                 215                 220
   Asp Asp Gly Asp Asp Ser Phe Pro Ala Ala Leu Arg Arg Ser Leu Tyr
   225                 230                 235                 240
   Gly Glu Thr Ala Pro Leu His Asp Leu His Ala Ile Thr Ser Cys Leu
                   245                 250                 255
   Thr Asp Val Arg Ala Leu Arg Val Leu Arg Gln Gln Ser Val Pro Leu
               260                 265                 270
   Asp Asp Ala Arg Arg Asp Gly Trp Glu Arg Thr Gly Ser Ala Ile Arg
           275                 280                 285
   His Arg Ser Gly Arg His Phe Glu Ile Met Ala Val Glu Val Thr Ala
       290                 295                 300
   Glu Arg Arg Glu Val Ala Ser Trp Thr Gln Pro Leu Leu Arg Pro Cys
   305                 310                 315                 320
   Ser Gln Gly Leu Ala Ala Leu Ile Thr Arg Arg Ile Asn Gly Val Leu
                   325                 330                 335
   His Ala Leu Val Ala Ala Arg Ser Glu Val Gly Thr Leu Asn Val Ala
   340 345 350
   Glu Phe Gly Pro Thr Val Gln Cys Arg Pro Asp Glu Ala Asp Gly Gln
   355 360 365
   Ser Pro Pro Tyr Leu Asp Arg Val Leu Thr Ala Gly Ala Asp Arg Val
   370 375 380
   Arg Tyr Asp Val Val Gln Ser Glu Glu Gly Gly Arg Phe Tyr His Ala
   385 390 395 400
   Arg Asn Arg Tyr Leu Val Val Glu Ala Gly Pro Glu Leu Asp Thr Gly
   405 410 415
   Cys Pro Pro Gly Phe Cys Trp Ala Thr Phe Gly Gln Leu Thr Glu Leu
   420 425 430
   Leu Ala His Gly Asn Tyr Leu Asn Val Glu Leu Arg Thr Leu Met Ala
   435 440 445
   Cys Ala His Ala Ser Tyr
   450
<210> 43
   <211> 1248
   <212> DNA
   <213> Streptomyces ambofaciens
<220>
   <221> CDS
   <222> (1)..(1248)
<400> 43
   atg ccg ggc ggg acg gac ggc gac tgc gcg cgg acg gcg gcc cgg cgt      48
   Met Pro Gly Gly Thr Asp Gly Asp Cys Ala Arg Thr Ala Ala Arg Arg
   1               5                   10                  15
   cga acg cac ctg ccc gag tcc gga cga gac agc gcg acg cga gag gcg      96
   Arg Thr His Leu Pro Glu Ser Gly Arg Asp Ser Ala Thr Arg Glu Ala
               20                  25                  30
   aaa atg atc aat ctc ttc cag ccc cag atg ggg gcc gag gaa ctg gcg      144
   Lys Met Ile Asn Leu Phe Gln Pro Gln Met Gly Ala Glu Glu Leu Ala
           35                  40                  45
   gcg gtg tcc gag gtc ttc gac gac caa tgg ctc ggt cac gga ccc cgg      192
   Ala Val Ser Glu Val Phe Asp Asp Gln Trp Leu Gly His Gly Pro Arg
       50                  55                  60
   acc gcg gcg ttc gag tcc gcg ttc gcc gag cac ctc ggg gtc ggc ccc      240
   Thr Ala Ala Phe Glu Ser Ala Phe Ala Glu His Leu Gly Val Gly Pro
   65                  70                  75                  80
   gag cac gtc gtc ttc ctc aac tcg ggc acc gcc ggc ctc ttc ctg gcc      288
   Glu His Val Val Phe Leu Asn Ser Gly Thr Ala Gly Leu Phe Leu Ala
                   85                  90                  95
   ctg gag tcg ctc ggc ctg cgg ccc ggc gac gag gtc gtg ctc ccc tcg      336
   Leu Glu Ser Leu Gly Leu Arg Pro Gly Asp Glu Val Val Leu Pro Ser
           100                 105                 110
   ccc agc ttc ctc gcc gcg gcg aac gcc gta cag ctc tcg gga gcg cgc     384
   Pro Ser Phe Leu Ala Ala Ala Asn Ala Val Gln Leu Ser Gly Ala Arg
       115                 120                 125
   ccg gtg ttc tgc gac acc gac ccg cgg acg ctg aac ccc gcc ctg gag     432
   Pro Val Phe Cys Asp Thr Asp Pro Arg Thr Leu Asn Pro Ala Leu Glu
       130                 135                 140
   cac atc gag gcg gcc gtc acc ccg cgc acc agg gcc gtc atc gcg ctc     480
   His Ile Glu Ala Ala Val Thr Pro Arg Thr Arg Ala Val Ile Ala Leu
   145                 150                 155                 160
   cac tac ggc ggc cac ccc ggc gac atc gtg cgc atc gcc gag cgc tgc     528
   His Tyr Gly Gly His Pro Gly Asp Ile Val Arg Ile Ala Glu Arg Cys
                   165                 170                 175
   cgg gag cgg ggc atc acc ctg atc gag gac gcc gcg tgc tcc gtg gcc     576
   Arg Glu Arg Gly Ile Thr Leu Ile Glu Asp Ala Ala Cys Ser Val Ala
               180                 185                 190
   tcc cgc gtc gac ggc cga ccg gtc ggc acc ttc ggc gac ctc gcc atg     624
   Ser Arg Val Asp Gly Arg Pro Val Gly Thr Phe Gly Asp Leu Ala Met
           195                 200                 205
   tgg agc ttc gac gcc atg aag gtc ctg gtc acc ggc gac gga ggg atg     672
   Trp Ser Phe Asp Ala Met Lys Val Leu Val Thr Gly Asp Gly Gly Met
       210                 215                 220
   atc tac gtc aag gac ccc ggg gcg gcc gcc cgg atc cgg cgc ctc gcc     720
   Ile Tyr Val Lys Asp Pro Gly Ala Ala Ala Arg Ile Arg Arg Leu Ala
   225                 230                 235                 240
   tac cac ggc ctc acg cgg tcc agc ggc ctg gga tac gcc agg gtc tcg     768
   Tyr His Gly Leu Thr Arg Ser Ser Gly Leu Gly Tyr Ala Arg Val Ser
                   245                 250                 255
   gcg cgc tgg tgg gag atg gac gtc ccc gaa ccg ggc cgc cgc gtc atc     816
   Ala Arg Trp Trp Glu Met Asp Val Pro Glu Pro Gly Arg Arg Val Ile
               260                 265                 270
   ggg aac gac ctc acc gcg gcc atc ggc gcg gtc cag ttg cgc cgg ctt     864
   Gly Asn Asp Leu Thr Ala Ala Ile Gly Ala Val Gln Leu Arg Arg Leu
           275                 280                 285
   ccc ggc ttc gtg gcc cgc cgc agg gag atc gtc gcc ctg tac gac agc     912
   Pro Gly Phe Val Ala Arg Arg Arg Glu Ile Val Ala Leu Tyr Asp Ser
       290                 295                 300
   gaa ctg agc tcg ctg gag ggc gtg ctg aca ccg ccc gcg cca ccc gcg     960
   Glu Leu Ser Ser Leu Glu Gly Val Leu Thr Pro Pro Ala Pro Pro Ala
   305                 310                 315                 320
   ggg cac gag tcc acg cac tac ttc tac tgg atc cag ctg gcc ccc ggc    1008
   Gly His Glu Ser Thr His Tyr Phe Tyr Trp Ile Gln Leu Ala Pro Gly
                   325                 330                 335
   gtc cgg gac cgg gtg gca cgc gac ctg ctc acc gac ggc atc tac acc    1056
   Val Arg Asp Arg Val Ala Arg Asp Leu Leu Thr Asp Gly Ile Tyr Thr
               340                 345                 350
   acc ttc cgc tac gca cct ctg cac aag gtg ccc gcc tac ggc cac acc    1104
   Thr Phe Arg Tyr Ala Pro Leu His Lys Val Pro Ala Tyr Gly His Thr
           355                 360                 365
   gga ggc gaa ctg ccc ggc gtg gag cgg gcg tcc gaa cgg acc ctg tgc    1152
   Gly Gly Glu Leu Pro Gly Val Glu Arg Ala Ser Glu Arg Thr Leu Cys
   370 375 380
   ctg ccc ctg cac ccc ggc ctg tcg gac gcc gac gtc cgc acc gtc gtg 1200
   Leu Pro Leu His Pro Gly Leu Ser Asp Ala Asp Val Arg Thr Val Val
   385 390 395 400
   tcc tcc ctg cgc aga gcc ctg agc gcc gcg gat ccg gcc ccc gcc tga 1248
   Ser Ser Leu Arg Arg Ala Leu Ser Ala Ala Asp Pro Ala Pro Ala
   405 410 415
<210> 44
   <211> 415
   <212> PRT
   <213> Streptomyces ambofaciens
<400> 44
   Met Pro Gly Gly Thr Asp Gly Asp Cys Ala Arg Thr Ala Ala Arg Arg
   1 5 10 15
   Arg Thr His Leu Pro Glu Ser Gly Arg Asp Ser Ala Thr Arg Glu Ala
   20 25 30
   Lys Met Ile Asn Leu Phe Gln Pro Gln Met Gly Ala Glu Glu Leu Ala
   35 40 45
   Ala Val Ser Glu Val Phe Asp Asp Gln Trp Leu Gly His Gly Pro Arg
   50 55 60
   Thr Ala Ala Phe Glu Ser Ala Phe Ala Glu His Leu Gly Val Gly Pro
   65 70 75 80
   Glu His Val Val Phe Leu Asn Ser Gly Thr Ala Gly Leu Phe Leu Ala
   85 90 95
   Leu Glu Ser Leu Gly Leu Arg Pro Gly Asp Glu Val Val Leu Pro Ser
   100 105 110
   Pro Ser Phe Leu Ala Ala Ala Asn Ala Val Gln Leu Ser Gly Ala Arg
   115 120 125
   Pro Val Phe Cys Asp Thr Asp Pro Arg Thr Leu Asn Pro Ala Leu Glu
   130 135 140
   His Ile Glu Ala Ala Val Thr Pro Arg Thr Arg Ala Val Ile Ala Leu
   145 150 155 160
   His Tyr Gly Gly His Pro Gly Asp Ile Val Arg Ile Ala Glu Arg Cys
   165 170 175
   Arg Glu Arg Gly Ile Thr Leu Ile Glu Asp Ala Ala Cys Ser Val Ala
   180 185 190
   Ser Arg Val Asp Gly Arg Pro Val Gly Thr Phe Gly Asp Leu Ala Met
   195 200 205
   Trp Ser Phe Asp Ala Met Lys Val Leu Val Thr Gly Asp Gly Gly Met
   210 215 220
   Ile Tyr Val Lys Asp Pro Gly Ala Ala Ala Arg Ile Arg Arg Leu Ala
   225 230 235 240
   Tyr His Gly Leu Thr Arg Ser Ser Gly Leu Gly Tyr Ala Arg Val Ser
   245 250 255
   Ala Arg Trp Trp Glu Met Asp Val Pro Glu Pro Gly Arg Arg Val Ile
   260 265 270
   Gly Asn Asp Leu Thr Ala Ala Ile Gly Ala Val Gln Leu Arg Arg Leu
   275 280 285
   Pro Gly Phe Val Ala Arg Arg Arg Glu Ile Val Ala Leu Tyr Asp Ser
   290 295 300
   Glu Leu Ser Ser Leu Glu Gly Val Leu Thr Pro Pro Ala Pro Pro Ala
   305 310 315 320
   Gly His Glu Ser Thr His Tyr Phe Tyr Trp Ile Gln Leu Ala Pro Gly
   325 330 335
   Val Arg Asp Arg Val Ala Arg Asp Leu Leu Thr Asp Gly Ile Tyr Thr
   340 345 350
   Thr Phe Arg Tyr Ala Pro Leu His Lys Val Pro Ala Tyr Gly His Thr
   355 360 365
   Gly Gly Glu Leu Pro Gly Val Glu Arg Ala Ser Glu Arg Thr Leu Cys
   370 375 380
   Leu Pro Leu His Pro Gly Leu Ser Asp Ala Asp Val Arg Thr Val Val
   385 390 395 400
   Ser Ser Leu Arg Arg Ala Leu Ser Ala Ala Asp Pro Ala Pro Ala
   405 410 415
<210> 45
   <211> 720
   <212> DNA
   <213> Streptomyces ambofaciens
<220>
   <221> CDS
   <222> (1)..(720)
<400> 45
   atg tac gag aac gac agt gcc gcc gag gtc tac gac ctg ctc tac cag 48
   Met Tyr Glu Asn Asp Ser Ala Ala Glu Val Tyr Asp Leu Leu Tyr Gln
   1 5 10 15
   gac cgc aag gac tac gcg ggt gag gcc gcc cgg gtc acc gac ctg atc      96
   Asp Arg Lys Asp Tyr Ala Gly Glu Ala Ala Arg Val Thr Asp Leu Ile
               20                  25                  30
   cgg gaa cgt acg ccg gac gcg gcc agc ctg ctc gac atc gcg tgc ggc     144
   Arg Glu Arg Thr Pro Asp Ala Ala Ser Leu Leu Asp Ile Ala Cys Gly
           35                  40                  45
   acc ggt acc cac ctg gag gcc ttc gcc aag ctc tac gac cgc gtg agc     192
   Thr Gly Thr His Leu Glu Ala Phe Ala Lys Leu Tyr Asp Arg Val Ser
       50                  55                  60
   ggc ctg gag ctg tcc gag tgg atg gcg gcc cgc gcc gag gag cgg ctc     240
   Gly Leu Glu Leu Ser Glu Trp Met Ala Ala Arg Ala Glu Glu Arg Leu
   65                  70                  75                  80
   ccc ggc gtc acc ctc cac cgc ggt gac atg cgg gcg ttc gac ctc ggc     288
   Pro Gly Val Thr Leu His Arg Gly Asp Met Arg Ala Phe Asp Leu Gly
                   85                  90                  95
   gag acg ttc gac gcc gtg gtc tgc atg ttc agc tcg atc ggg tac ctg     336
   Glu Thr Phe Asp Ala Val Val Cys Met Phe Ser Ser Ile Gly Tyr Leu
               100                 105                 110
   gag acc acg gcc gac ctg gag gac gcc gtc gcc gcc atg gcg cgg cac     384
   Glu Thr Thr Ala Asp Leu Glu Asp Ala Val Ala Ala Met Ala Arg His
           115                 120                 125
   ctg acc gcg gac ggt gtc ctg gcc gtc gaa ccg tgg tac ttc ccc gac     432
   Leu Thr Ala Asp Gly Val Leu Ala Val Glu Pro Trp Tyr Phe Pro Asp
       130                 135                 140
   acc ttc ctg gac ggc cac gtc tcc acc cac gcc ctg cgg acg gca ccg     480
   Thr Phe Leu Asp Gly His Val Ser Thr His Ala Leu Arg Thr Ala Pro
   145                 150                 155                 160
   ggc gac cag ggc gtc gcc cgt gtc tcc cac tcg acc cgg gag ggc ggg     528
   Gly Asp Gln Gly Val Ala Arg Val Ser His Ser Thr Arg Glu Gly Gly
                   165                 170                 175
   cgg acc cgg atg gag atc cac tac ctg atc gcg cac acc gcg gag ggc     576
   Arg Thr Arg Met Glu Ile His Tyr Leu Ile Ala His Thr Ala Glu Gly
               180                 185                 190
   atc cgg cac cgc agc gag gtg gac tac ctc acg ctg ttc tcg cgt gcg     624
   Ile Arg His Arg Ser Glu Val Asp Tyr Leu Thr Leu Phe Ser Arg Ala
           195                 200                 205
   gag tac gag gcc gcg tac cgc aag gcc ggc ctg gac gtc gag tac gtc     672
   Glu Tyr Glu Ala Ala Tyr Arg Lys Ala Gly Leu Asp Val Glu Tyr Val
       210                 215                 220
   gtg acg ggc gag ggc tca ccg ggc ttc ttc ctc ggc acg cgt cgc tga     720
   Val Thr Gly Glu Gly Ser Pro Gly Phe Phe Leu Gly Thr Arg Arg
   225                 230                 235
<210> 46
   <211> 239
   <212> PRT
   <213> Streptomyces ambofaciens
<400> 46
   Met Tyr Glu Asn Asp Ser Ala Ala Glu Val Tyr Asp Leu Leu Tyr Gln
   1               5                   10                  15
   Asp Arg Lys Asp Tyr Ala Gly Glu Ala Ala Arg Val Thr Asp Leu Ile
               20                  25                  30
   Arg Glu Arg Thr Pro Asp Ala Ala Ser Leu Leu Asp Ile Ala Cys Gly
           35                  40                  45
   Thr Gly Thr His Leu Glu Ala Phe Ala Lys Leu Tyr Asp Arg Val Ser
       50                  55                  60
   Gly Leu Glu Leu Ser Glu Trp Met Ala Ala Arg Ala Glu Glu Arg Leu
   65                  70                  75                  80
   Pro Gly Val Thr Leu His Arg Gly Asp Met Arg Ala Phe Asp Leu Gly
                   85                  90                  95
   Glu Thr Phe Asp Ala Val Val Cys Met Phe Ser Ser Ile Gly Tyr Leu
               100                 105                 110
   Glu Thr Thr Ala Asp Leu Glu Asp Ala Val Ala Ala Met Ala Arg His
           115                 120                 125
   Leu Thr Ala Asp Gly Val Leu Ala Val Glu Pro Trp Tyr Phe Pro Asp
       130                 135                 140
   Thr Phe Leu Asp Gly His Val Ser Thr His Ala Leu Arg Thr Ala Pro
   145                 150                 155                 160
   Gly Asp Gln Gly Val Ala Arg Val Ser His Ser Thr Arg Glu Gly Gly
                   165                 170                 175
   Arg Thr Arg Met Glu Ile His Tyr Leu Ile Ala His Thr Ala Glu Gly
               180                 185                 190
   Ile Arg His Arg Ser Glu Val Asp Tyr Leu Thr Leu Phe Ser Arg Ala
           195                 200                 205
   Glu Tyr Glu Ala Ala Tyr Arg Lys Ala Gly Leu Asp Val Glu Tyr Val
       210                 215                 220
   Val Thr Gly Glu Gly Ser Pro Gly Phe Phe Leu Gly Thr Arg Arg
   225                 230                 235
<210> 47
   <211> 1968
   <212> DNA
   <213> Streptomyces ambofaciens
<220>
   <221> CDS
   <222> (1)..(1968)
<400> 47
   atg agt gac ctg ggt tct ggt gaa gaa ggg tcc gaa gaa gac gag tcg 48
   Met Ser Asp Leu Gly Ser Gly Glu Glu Gly Ser Glu Glu Asp Glu Ser
   1 5 10 15
   gac gac gca ctc gcc ttc ctc gag ttc atc gcc cgg tcg gca cca cgg 96
   Asp Asp Ala Leu Ala Phe Leu Glu Phe Ile Ala Arg Ser Ala Pro Arg
   20 25 30
   agc gaa tac gac cgg ctc atg gcc cgc gcc gaa cgc tcg ggc gcc gac 144
   Ser Glu Tyr Asp Arg Leu Met Ala Arg Ala Glu Arg Ser Gly Ala Asp
   35 40 45
   gag gac cgg atg cgc cga ctg gag cgc ttc aac cgg ctc gcc ctc acc 192
   Glu Asp Arg Met Arg Arg Leu Glu Arg Phe Asn Arg Leu Ala Leu Thr
   50 55 60
   gcg cag tcg atg atc gag tac cgc cgc gac cgg gag gcg gag ctc gcg 240
   Ala Gln Ser Met Ile Glu Tyr Arg Arg Asp Arg Glu Ala Glu Leu Ala
   65 70 75 80
   gcc ctg gtc gac gcc gcg cac gag ttc gtc gcc gcc cgg cgg ggc aag 288
   Ala Leu Val Asp Ala Ala His Glu Phe Val Ala Ala Arg Arg Gly Lys
   85 90 95
   gac ctg ctg gag tcc atc gcc cgc aga gca cgg ctg ctg ctg aag ctg 336
   Asp Leu Leu Glu Ser Ile Ala Arg Arg Ala Arg Leu Leu Leu Lys Leu
   100 105 110
   gac gtc tcc tac gtc ggc ctg cac gag gag gac cgg ccc ggc acg gtg 384
   Asp Val Ser Tyr Val Gly Leu His Glu Glu Asp Arg Pro Gly Thr Val
   115 120 125
   gtg ctg agc gcc gac ggc aac gcg gtc aag gtc gcc gag agc tac cgg 432
   Val Leu Ser Ala Asp Gly Asn Ala Val Lys Val Ala Glu Ser Tyr Arg
   130 135 140
   ctg ccg gcc gac ggc gga ctg ggc gcc atg gtg cgc acc tgc cgc gct 480
   Leu Pro Ala Asp Gly Gly Leu Gly Ala Met Val Arg Thr Cys Arg Ala
   145 150 155 160
   ccc ttc tgg acc ccg gac tac ctc ggg gac aac agc ttc acg cac gtc 528
   Pro Phe Trp Thr Pro Asp Tyr Leu Gly Asp Asn Ser Phe Thr His Val
   165 170 175
   gag gcc gtc gac gac atc gtc cgc gcc gaa ggc ctg cgc gcg gtc ctg 576
   Glu Ala Val Asp Asp Ile Val Arg Ala Glu Gly Leu Arg Ala Val Leu
   180 185 190
   gcc gtc ccg ctg tgc gcc ggg ggc gaa ccg atg ggg gtc ctc tac gtc 624
   Ala Val Pro Leu Cys Ala Gly Gly Glu Pro Met Gly Val Leu Tyr Val
   195 200 205
   gcc gac cgt cag gtg cgg cat ctg acc ccc aac gag gtc acc ctg ctg 672
   Ala Asp Arg Gln Val Arg His Leu Thr Pro Asn Glu Val Thr Leu Leu
   210 215 220
   tgc tcg ctc gcc gat ctg gcc gcg gtg gcg atc gag cgc aac cgg ctg 720
   Cys Ser Leu Ala Asp Leu Ala Ala Val Ala Ile Glu Arg Asn Arg Leu
   225 230 235 240
   gtc gag gag ctc cac gac acc atc ggg caa ctg cgc cag gac atc ggc 768
   Val Glu Glu Leu His Asp Thr Ile Gly Gln Leu Arg Gln Asp Ile Gly
   245 250 255
   gag gcc cgc acc gcc ctc gcg cgc acc cgc agg tcc gcc gac ctc cag 816
   Glu Ala Arg Thr Ala Leu Ala Arg Thr Arg Arg Ser Ala Asp Leu Gln
   260 265 270
   tcg cac ctg gtc acg cag gtg atg gac agg cgc ggc gcc gac tcg tta      864
   Ser His Leu Val Thr Gln Val Met Asp Arg Arg Gly Ala Asp Ser Leu
           275                 280                 285
   ctc gcg acg gcc gcc gag gcg ctc ggc ggc gga gcc ggc ctg tgc agc      912
   Leu Ala Thr Ala Ala Glu Ala Leu Gly Gly Gly Ala Gly Leu Cys Ser
       290                 295                 300
   ccg ctc ggg cgc ccg ctc gcc gag tac ggg acc ctg cgc ccc gtc gcc      960
   Pro Leu Gly Arg Pro Leu Ala Glu Tyr Gly Thr Leu Arg Pro Val Ala
   305                 310                 315                 320
   ccc acg gaa ctg cgc gcg gcg tgc cgc cgg gcc gcc gag acc ggc cgg     1008
   Pro Thr Glu Leu Arg Ala Ala Cys Arg Arg Ala Ala Glu Thr Gly Arg
                   325                 330                 335
   ccc acc tcc gtg gcc ccg ggg gtc tgg acg gtg ccc ctg ctt ccc ggg     1056
   Pro Thr Ser Val Ala Pro Gly Val Trp Thr Val Pro Leu Leu Pro Gly
               340                 345                 350
   ggc aac gcc ggc ttc ctg ctg acc gac ctc ggt ccg gac gcg gac cac     1104
   Gly Asn Ala Gly Phe Leu Leu Thr Asp Leu Gly Pro Asp Ala Asp His
           355                 360                 365
   acc gcc gtc ccc ctg ctc ccg atg gtc gcc cgc acc ctc gcg ctg cac     1152
   Thr Ala Val Pro Leu Leu Pro Met Val Ala Arg Thr Leu Ala Leu His
       370                 375                 380
   ctg cgc gtc cag cac gac gac tcc ccc aag gcg cag agc cac cag gag     1200
   Leu Arg Val Gln His Asp Asp Ser Pro Lys Ala Gln Ser His Gln Glu
   385                 390                 395                 400
   ttc ttc gac gac ctg atc ggg gcg ccc cgc tca ccc acg ctc ctc agg     1248
   Phe Phe Asp Asp Leu Ile Gly Ala Pro Arg Ser Pro Thr Leu Leu Arg
                   405                 410                 415
   gaa cgc gcc ctg atg ttc tcc ctc agc ttc cgc cgc ccg cac gtg gtg     1296
   Glu Arg Ala Leu Met Phe Ser Leu Ser Phe Arg Arg Pro His Val Val
               420                 425                 430
   ctg gtg gcg gac gga ccc cgc ggg acc tcg ccg cgg ctg gag gcc tcc     1344
   Leu Val Ala Asp Gly Pro Arg Gly Thr Ser Pro Arg Leu Glu Ala Ser
           435                 440                 445
   ggc gcc gac tac gcg aag gag ctc ggc ggg ctg tgc agc gtg cgg gac     1392
   Gly Ala Asp Tyr Ala Lys Glu Leu Gly Gly Leu Cys Ser Val Arg Asp
       450                 455                 460
   ggc gcc gtc gtc ctg ctg ctg ccc ggc gac gac ccc gtc gcc gtg gcg     1440
   Gly Ala Val Val Leu Leu Leu Pro Gly Asp Asp Pro Val Ala Val Ala
   465                 470                 475                 480
   cag acc gcc gcc ccg gag ctg acc gac cgc gcc ggg cac ccc gtc acc     1488
   Gln Thr Ala Ala Pro Glu Leu Thr Asp Arg Ala Gly His Pro Val Thr
                   485                 490                 495
   gtg ggg gtc gcg ggc ccc gcc tcg acc gtc gac ggc atc gcc gac gcg     1536
   Val Gly Val Ala Gly Pro Ala Ser Thr Val Asp Gly Ile Ala Asp Ala
               500                 505                 510
   cac cgt gag gcc gcg aag tgt ctg gag acc ctc cgc gcg ctc ggc ggc     1584
   His Arg Glu Ala Ala Lys Cys Leu Glu Thr Leu Arg Ala Leu Gly Gly
           515                 520                 525
   gac ggc ggc acc gcg tgc gcc tcc gac ctg ggt ttc ctc ggc atg ctc     1632
   Asp Gly Gly Thr Ala Cys Ala Ser Asp Leu Gly Phe Leu Gly Met Leu
       530                 535                 540
   ctc gcc gag gag aac gac gtc ccc ggt tac atc agg acg acg atc ggc      1680
   Leu Ala Glu Glu Asn Asp Val Pro Gly Tyr Ile Arg Thr Thr Ile Gly
   545                 550                 555                 560
   ccc gtg gtc gac tac gac acc cac cgc ttc acg gat ctg gtt ccc act      1728
   Pro Val Val Asp Tyr Asp Thr His Arg Phe Thr Asp Leu Val Pro Thr
                   565                 570                 575
   ctg agg gtg tac ctg gag tcg ggc agg agc ccc acg cgt gcc gca gag      1776
   Leu Arg Val Tyr Leu Glu Ser Gly Arg Ser Pro Thr Arg Ala Ala Glu
               580                 585                 590
   aca ctg cgc gtg cac ccg aac acc gtc tca cgg cgg ctg gag cgc atc      1824
   Thr Leu Arg Val His Pro Asn Thr Val Ser Arg Arg Leu Glu Arg Ile
           595                 600                 605
   ggc gta ctg ctg gga gag gac tgg cag tca ccg gag cgg gtg ctg gac      1872
   Gly Val Leu Leu Gly Glu Asp Trp Gln Ser Pro Glu Arg Val Leu Asp
       610                 615                 620
   ata caa ctg gcc ctg cgg ctc tat cag gtg cgc tcg gcg ctc tcc tcg      1920
   Ile Gln Leu Ala Leu Arg Leu Tyr Gln Val Arg Ser Ala Leu Ser Ser
   625                 630                 635                 640
   caa ccg gcg tcc gag acc cgg gcc gtg ctc gga tcg ctg cgc gag tga      1968
   Gln Pro Ala Ser Glu Thr Arg Ala Val Leu Gly Ser Leu Arg Glu
                   645                 650                 655
<210> 48
   <211> 655
   <212> PRT
   <213> Streptomyces ambofaciens
<400> 48
   Met Ser Asp Leu Gly Ser Gly Glu Glu Gly Ser Glu Glu Asp Glu Ser
   1               5                   10                  15
   Asp Asp Ala Leu Ala Phe Leu Glu Phe Ile Ala Arg Ser Ala Pro Arg
               20                  25                  30
   Ser Glu Tyr Asp Arg Leu Met Ala Arg Ala Glu Arg Ser Gly Ala Asp
           35                  40                  45
   Glu Asp Arg Met Arg Arg Leu Glu Arg Phe Asn Arg Leu Ala Leu Thr
       50                  55                  60
   Ala Gln Ser Met Ile Glu Tyr Arg Arg Asp Arg Glu Ala Glu Leu Ala
   65                  70                  75                  80
   Ala Leu Val Asp Ala Ala His Glu Phe Val Ala Ala Arg Arg Gly Lys
                   85                  90                  95
   Asp Leu Leu Glu Ser Ile Ala Arg Arg Ala Arg Leu Leu Leu Lys Leu
               100                 105                 110
   Asp Val Ser Tyr Val Gly Leu His Glu Glu Asp Arg Pro Gly Thr Val
           115                 120                 125
   Val Leu Ser Ala Asp Gly Asn Ala Val Lys Val Ala Glu Ser Tyr Arg
   130 135 140
   Leu Pro Ala Asp Gly Gly Leu Gly Ala Met Val Arg Thr Cys Arg Ala
   145 150 155 160
   Pro Phe Trp Thr Pro Asp Tyr Leu Gly Asp Asn Ser Phe Thr His Val
   165 170 175
   Glu Ala Val Asp Asp Ile Val Arg Ala Glu Gly Leu Arg Ala Val Leu
   180 185 190
   Ala Val Pro Leu Cys Ala Gly Gly Glu Pro Met Gly Val Leu Tyr Val
   195 200 205
   Ala Asp Arg Gln Val Arg His Leu Thr Pro Asn Glu Val Thr Leu Leu
   210 215 220
   Cys Ser Leu Ala Asp Leu Ala Ala Val Ala Ile Glu Arg Asn Arg Leu
   225 230 235 240
   Val Glu Glu Leu His Asp Thr Ile Gly Gln Leu Arg Gln Asp Ile Gly
   245 250 255
   Glu Ala Arg Thr Ala Leu Ala Arg Thr Arg Arg Ser Ala Asp Leu Gln
   260 265 270
   Ser His Leu Val Thr Gln Val Met Asp Arg Arg Gly Ala Asp Ser Leu
   275 280 285
   Leu Ala Thr Ala Ala Glu Ala Leu Gly Gly Gly Ala Gly Leu Cys Ser
   290 295 300
   Pro Leu Gly Arg Pro Leu Ala Glu Tyr Gly Thr Leu Arg Pro Val Ala
   305 310 315 320
   Pro Thr Glu Leu Arg Ala Ala Cys Arg Arg Ala Ala Glu Thr Gly Arg
   325 330 335
   Pro Thr Ser Val Ala Pro Gly Val Trp Thr Val Pro Leu Leu Pro Gly
   340 345 350
   Gly Asn Ala Gly Phe Leu Leu Thr Asp Leu Gly Pro Asp Ala Asp His
   355 360 365
   Thr Ala Val Pro Leu Leu Pro Met Val Ala Arg Thr Leu Ala Leu His
   370 375 380
   Leu Arg Val Gln His Asp Asp Ser Pro Lys Ala Gln Ser His Gln Glu
   385 390 395 400
   Phe Phe Asp Asp Leu Ile Gly Ala Pro Arg Ser Pro Thr Leu Leu Arg
   405 410 415
   Glu Arg Ala Leu Met Phe Ser Leu Ser Phe Arg Arg Pro His Val Val
   420 425 430
   Leu Val Ala Asp Gly Pro Arg Gly Thr Ser Pro Arg Leu Glu Ala Ser
   435 440 445
   Gly Ala Asp Tyr Ala Lys Glu Leu Gly Gly Leu Cys Ser Val Arg Asp
   450 455 460
   Gly Ala Val Val Leu Leu Leu Pro Gly Asp Asp Pro Val Ala Val Ala
   465 470 475 480
   Gln Thr Ala Ala Pro Glu Leu Thr Asp Arg Ala Gly His Pro Val Thr
   485 490 495
   Val Gly Val Ala Gly Pro Ala Ser Thr Val Asp Gly Ile Ala Asp Ala
   500 505 510
   His Arg Glu Ala Ala Lys Cys Leu Glu Thr Leu Arg Ala Leu Gly Gly
   515 520 525
   Asp Gly Gly Thr Ala Cys Ala Ser Asp Leu Gly Phe Leu Gly Met Leu
   530 535 540
   Leu Ala Glu Glu Asn Asp Val Pro Gly Tyr Ile Arg Thr Thr Ile Gly
   545 550 555 560
   Pro Val Val Asp Tyr Asp Thr His Arg Phe Thr Asp Leu Val Pro Thr
   565 570 575
   Leu Arg Val Tyr Leu Glu Ser Gly Arg Ser Pro Thr Arg Ala Ala Glu
   580 585 590
   Thr Leu Arg Val His Pro Asn Thr Val Ser Arg Arg Leu Glu Arg Ile
   595 600 605
   Gly Val Leu Leu Gly Glu Asp Trp Gln Ser Pro Glu Arg Val Leu Asp
   610 615 620
   Ile Gln Leu Ala Leu Arg Leu Tyr Gln Val Arg Ser Ala Leu Ser Ser
   625 630 635 640
   Gln Pro Ala Ser Glu Thr Arg Ala Val Leu Gly Ser Leu Arg Glu
   645 650 655
<210> 49
   <211> 1749
   <212> DNA
   <213> Streptomyces ambofaciens
<220>
   <221> CDS
   <222> (1)..(1749)
<220>
   <221> CDS2
   <222> (76)..(1749)
<220>
   <221> CDS3
   <222> (97)..(1749)
<400> 49
   atg tcc gca gtg gtg gcg tcc ctt ctg ctt tcc gtt ctt tct gcg agc      48
   Met Ser Ala Val Val Ala Ser Leu Leu Leu Ser Val Leu Ser Ala Ser
   1               5                   10                  15
   gcg ctc acg cat cca ctc atc ctc atc gtg gaa aca gga gac tta cgc      96
   Ala Leu Thr His Pro Leu Ile Leu Ile Val Glu Thr Gly Asp Leu Arg
               20                  25                  30
   gtg tcg att gcg caa tac gcc cta cac gac atc acg aag cgc tac cac     144
   Val Ser Ile Ala Gln Tyr Ala Leu His Asp Ile Thr Lys Arg Tyr His
           35                  40                  45
   gac tgt gtc gtg ctc gac cgg gtc ggt ttc agc atc aag ccg ggc gag     192
   Asp Cys Val Val Leu Asp Arg Val Gly Phe Ser Ile Lys Pro Gly Glu
       50                  55                  60
   aag gtc ggc gtg atc ggc gac aac ggt tcc ggc aag tcc acg ctg ctc     240
   Lys Val Gly Val Ile Gly Asp Asn Gly Ser Gly Lys Ser Thr Leu Leu
   65                  70                  75                  80
   aag atc ctc gcc ggc cgc gtg gag ccc gac aac ggc gcg ctc acc gtg     288
   Lys Ile Leu Ala Gly Arg Val Glu Pro Asp Asn Gly Ala Leu Thr Val
                   85                  90                  95
   gtc gct ccc ggc ggc gtc ggc tac ctg gcg cag aca ctg gaa ctg ccc     336
   Val Ala Pro Gly Gly Val Gly Tyr Leu Ala Gln Thr Leu Glu Leu Pro
               100                 105                 110
   ctc gac gcc acc gtc cag gac gcc gtc gac ctg gcc ctg tcc gac ctg     384
   Leu Asp Ala Thr Val Gln Asp Ala Val Asp Leu Ala Leu Ser Asp Leu
           115                 120                 125
   cgc gag ctc gaa gcg gcg atg cgc gag gcc gag gcg gag ctg ggc gag     432
   Arg Glu Leu Glu Ala Ala Met Arg Glu Ala Glu Ala Glu Leu Gly Glu
       130                 135                 140
   agc gac gag aac ggc tcc gag cgc gag ctg tcc gcc ggc ctc cag cgc     480
   Ser Asp Glu Asn Gly Ser Glu Arg Glu Leu Ser Ala Gly Leu Gln Arg
   145                 150                 155                 160
   tac gcc gct ctg gtc gag cag tac cag gcg cgt ggc ggc tac gag gcc     528
   Tyr Ala Ala Leu Val Glu Gln Tyr Gln Ala Arg Gly Gly Tyr Glu Ala
                   165                 170                 175
   gac gtg cgc gtg gag gtc gcg ctg cac ggc ctc gga ctg ccg agc ctg     576
   Asp Val Arg Val Glu Val Ala Leu His Gly Leu Gly Leu Pro Ser Leu
               180                 185                 190
   gac cgc gac cgc aag ctc gga acc ctc tcc ggt ggc gaa cgc tcc cgc     624
   Asp Arg Asp Arg Lys Leu Gly Thr Leu Ser Gly Gly Glu Arg Ser Arg
           195                 200                 205
   ctc gcg ctc gcc gcg acc ctc gcc tcg tcg ccg gag ctg ctg ctc ctg     672
   Leu Ala Leu Ala Ala Thr Leu Ala Ser Ser Pro Glu Leu Leu Leu Leu
       210                 215                 220
   gac gaa ccg acc aac gac ctc gac gac cgg gcg atg gaa tgg ctg gag      720
   Asp Glu Pro Thr Asn Asp Leu Asp Asp Arg Ala Met Glu Trp Leu Glu
   225                 230                 235                 240
   gac cac ctg gcc ggc cac cgc ggc acg gtg atc gcg gtc acc cac gac      768
   Asp His Leu Ala Gly His Arg Gly Thr Val Ile Ala Val Thr His Asp
                   245                 250                 255
   cgg gtc ttc ctc gac cgg ctc acc acc acg atc ctg gag gtc gac tcc      816
   Arg Val Phe Leu Asp Arg Leu Thr Thr Thr Ile Leu Glu Val Asp Ser
               260                 265                 270
   ggc agc gtc acc cgc tac ggc aac ggc tac gag ggc tac ctg acg gcc      864
   Gly Ser Val Thr Arg Tyr Gly Asn Gly Tyr Glu Gly Tyr Leu Thr Ala
           275                 280                 285
   aag gcc gtg gaa cgc gag cgg cgg ctg cgg gag tac gag gag tgg cgt      912
   Lys Ala Val Glu Arg Glu Arg Arg Leu Arg Glu Tyr Glu Glu Trp Arg
       290                 295                 300
   gcc gaa ctc gac cgc aac cgc ggg ctg atc acc tcc aac gtg gcg cgg      960
   Ala Glu Leu Asp Arg Asn Arg Gly Leu Ile Thr Ser Asn Val Ala Arg
   305                 310                 315                 320
   atg gac ggc atc ccg cgc aag atg tcc ctc tcc gtg ttc ggc cac ggc     1008
   Met Asp Gly Ile Pro Arg Lys Met Ser Leu Ser Val Phe Gly His Gly
                   325                 330                 335
   gcc tac cgc agg cga ggg cgc gac cac ggc gcg atg gtg cgg atc cgc     1056
   Ala Tyr Arg Arg Arg Gly Arg Asp His Gly Ala Met Val Arg Ile Arg
               340                 345                 350
   aac gcg aag caa cgc gtg gcg cag ctg acc gag aac ccg gtc cac gct     1104
   Asn Ala Lys Gln Arg Val Ala Gln Leu Thr Glu Asn Pro Val His Ala
           355                 360                 365
   ccc gcc gac ccg ttg tcc ttc gcc gcc cgc atc gac acc gcg ggc ccg     1152
   Pro Ala Asp Pro Leu Ser Phe Ala Ala Arg Ile Asp Thr Ala Gly Pro
       370                 375                 380
   gag gcg gag gag gcg gtg gcc gaa ctc acc gac gtg cgc gtc gcg ggt     1200
   Glu Ala Glu Glu Ala Val Ala Glu Leu Thr Asp Val Arg Val Ala Gly
   385                 390                 395                 400
   cgg ctc gcc gtg gac tcc ctg acg atc cgg ccc ggc gaa cgg ctg ctc     1248
   Arg Leu Ala Val Asp Ser Leu Thr Ile Arg Pro Gly Glu Arg Leu Leu
                   405                 410                 415
   gtc aca ggt ccc aac ggt gcg ggc aag tcc acc ttg ttg cgg gtg ctg     1296
   Val Thr Gly Pro Asn Gly Ala Gly Lys Ser Thr Leu Leu Arg Val Leu
               420                 425                 430
   tcc ggg gaa ctg gag ccg gac ggc ggc tcg gtg cgc gtc ggc tgc cgg     1344
   Ser Gly Glu Leu Glu Pro Asp Gly Gly Ser Val Arg Val Gly Cys Arg
           435                 440                 445
   gtc ggt cat ctg cgg cag gac gag acg ccc tgg gcg ccc gga ctg acc     1392
   Val Gly His Leu Arg Gln Asp Glu Thr Pro Trp Ala Pro Gly Leu Thr
       450                 455                 460
   gtg ctg cgg gcc ttc gcc cag ggc cgg gag ggc tac ctg gag gac cac     1440
   Val Leu Arg Ala Phe Ala Gln Gly Arg Glu Gly Tyr Leu Glu Asp His
   465                 470                 475                 480
   gcg gag aaa ctg ctg tcg ctc ggc ctg ttc agc ccg tcc gac ctg cgg     1488
   Ala Glu Lys Leu Leu Ser Leu Gly Leu Phe Ser Pro Ser Asp Leu Arg
                   485                 490                 495
   cga cgc gtg aag gat ctg tcc tac ggg cag cgc cgc cgg atc gag atc      1536
   Arg Arg Val Lys Asp Leu Ser Tyr Gly Gln Arg Arg Arg Ile Glu Ile
               500                 505                 510
   gcc cgg ctg gtg agc gac ccg atg gac ctg ctg ctg ctg gac gag ccc      1584
   Ala Arg Leu Val Ser Asp Pro Met Asp Leu Leu Leu Leu Asp Glu Pro
           515                 520                 525
   acc aac cac ctc acc ccg gtg ctg gtg gag gag ttg gag cag gca ctc      1632
   Thr Asn His Leu Thr Pro Val Leu Val Glu Glu Leu Glu Gln Ala Leu
       530                 535                 540
   gcg gac tac cgc ggc gcc gtc gtg gtc gtc acc cac gac cgt cgg atg      1680
   Ala Asp Tyr Arg Gly Ala Val Val Val Val Thr His Asp Arg Arg Met
   545                 550                 555                 560
   cgg tcc cgg ttc acc ggc gcc cgg ctg acc atg gga gac ggg cgc atc      1728
   Arg Ser Arg Phe Thr Gly Ala Arg Leu Thr Met Gly Asp Gly Arg Ile
                   565                 570                 575
   gcc gag ttc agc gcc ggc tga                                          1749
   Ala Glu Phe Ser Ala Gly
               580
<210> 50
   <211> 582
   <212> PRT
   <213> Streptomyces ambofaciens
<400> 50
   Met Ser Ala Val Val Ala Ser Leu Leu Leu Ser Val Leu Ser Ala Ser
   1               5                   10                  15
   Ala Leu Thr His Pro Leu Ile Leu Ile Val Glu Thr Gly Asp Leu Arg
               20                  25                  30
   Val Ser Ile Ala Gln Tyr Ala Leu His Asp Ile Thr Lys Arg Tyr His
           35                  40                  45
   Asp Cys Val Val Leu Asp Arg Val Gly Phe Ser Ile Lys Pro Gly Glu
       50                  55                  60
   Lys Val Gly Val Ile Gly Asp Asn Gly Ser Gly Lys Ser Thr Leu Leu
   65                  70                  75                  80
   Lys Ile Leu Ala Gly Arg Val Glu Pro Asp Asn Gly Ala Leu Thr Val
                   85                  90                  95
   Val Ala Pro Gly Gly Val Gly Tyr Leu Ala Gln Thr Leu Glu Leu Pro
               100                 105                 110
   Leu Asp Ala Thr Val Gln Asp Ala Val Asp Leu Ala Leu Ser Asp Leu
           115                 120                 125
   Arg Glu Leu Glu Ala Ala Met Arg Glu Ala Glu Ala Glu Leu Gly Glu
   130 135 140
   Ser Asp Glu Asn Gly Ser Glu Arg Glu Leu Ser Ala Gly Leu Gln Arg
   145 150 155 160
   Tyr Ala Ala Leu Val Glu Gln Tyr Gln Ala Arg Gly Gly Tyr Glu Ala
   165 170 175
   Asp Val Arg Val Glu Val Ala Leu His Gly Leu Gly Leu Pro Ser Leu
   180 185 190
   Asp Arg Asp Arg Lys Leu Gly Thr Leu Ser Gly Gly Glu Arg Ser Arg
   195 200 205
   Leu Ala Leu Ala Ala Thr Leu Ala Ser Ser Pro Glu Leu Leu Leu Leu
   210 215 220
   Asp Glu Pro Thr Asn Asp Leu Asp Asp Arg Ala Met Glu Trp Leu Glu
   225 230 235 240
   Asp His Leu Ala Gly His Arg Gly Thr Val Ile Ala Val Thr His Asp
   245 250 255
   Arg Val Phe Leu Asp Arg Leu Thr Thr Thr Ile Leu Glu Val Asp Ser
   260 265 270
   Gly Ser Val Thr Arg Tyr Gly Asn Gly Tyr Glu Gly Tyr Leu Thr Ala
   275 280 285
   Lys Ala Val Glu Arg Glu Arg Arg Leu Arg Glu Tyr Glu Glu Trp Arg
   290 295 300
   Ala Glu Leu Asp Arg Asn Arg Gly Leu Ile Thr Ser Asn Val Ala Arg
   305 310 315 320
   Met Asp Gly Ile Pro Arg Lys Met Ser Leu Ser Val Phe Gly His Gly
   325 330 335
   Ala Tyr Arg Arg Arg Gly Arg Asp His Gly Ala Met Val Arg Ile Arg
   340 345 350
   Asn Ala Lys Gln Arg Val Ala Gln Leu Thr Glu Asn Pro Val His Ala
   355 360 365
   Pro Ala Asp Pro Leu Ser Phe Ala Ala Arg Ile Asp Thr Ala Gly Pro
   370 375 380
   Glu Ala Glu Glu Ala Val Ala Glu Leu Thr Asp Val Arg Val Ala Gly
   385 390 395 400
   Arg Leu Ala Val Asp Ser Leu Thr Ile Arg Pro Gly Glu Arg Leu Leu
   405 410 415
   Val Thr Gly Pro Asn Gly Ala Gly Lys Ser Thr Leu Leu Arg Val Leu
   420 425 430
   Ser Gly Glu Leu Glu Pro Asp Gly Gly Ser Val Arg Val Gly Cys Arg
   435 440 445
   Val Gly His Leu Arg Gln Asp Glu Thr Pro Trp Ala Pro Gly Leu Thr
   450 455 460
   Val Leu Arg Ala Phe Ala Gln Gly Arg Glu Gly Tyr Leu Glu Asp His
   465 470 475 480
   Ala Glu Lys Leu Leu Ser Leu Gly Leu Phe Ser Pro Ser Asp Leu Arg
   485 490 495
   Arg Arg Val Lys Asp Leu Ser Tyr Gly Gln Arg Arg Arg Ile Glu Ile
   500 505 510
   Ala Arg Leu Val Ser Asp Pro Met Asp Leu Leu Leu Leu Asp Glu Pro
   515 520 525
   Thr Asn His Leu Thr Pro Val Leu Val Glu Glu Leu Glu Gln Ala Leu
   530 535 540
   Ala Asp Tyr Arg Gly Ala Val Val Val Val Thr His Asp Arg Arg Met
   545 550 555 560
   Arg Ser Arg Phe Thr Gly Ala Arg Leu Thr Met Gly Asp Gly Arg Ile
   565 570 575
   Ala Glu Phe Ser Ala Gly
   580
<210> 51
   <211> 557
   <212> PRT
   <213> Streptomyces ambofaciens
<400> 51
   Val Glu Thr Gly Asp Leu Arg Val Ser Ile Ala Gln Tyr Ala Leu His
   1 5 10 15
   Asp Ile Thr Lys Arg Tyr His Asp Cys Val Val Leu Asp Arg Val Gly
   20 25 30
   Phe Ser Ile Lys Pro Gly Glu Lys Val Gly Val Ile Gly Asp Asn Gly
   35 40 45
   Ser Gly Lys Ser Thr Leu Leu Lys Ile Leu Ala Gly Arg Val Glu Pro
   50 55 60
   Asp Asn Gly Ala Leu Thr Val Val Ala Pro Gly Gly Val Gly Tyr Leu
   65 70 75 80
   Ala Gln Thr Leu Glu Leu Pro Leu Asp Ala Thr Val Gln Asp Ala Val
   85 90 95
   Asp Leu Ala Leu Ser Asp Leu Arg Glu Leu Glu Ala Ala Met Arg Glu
   100 105 110
   Ala Glu Ala Glu Leu Gly Glu Ser Asp Glu Asn Gly Ser Glu Arg Glu
   115 120 125
   Leu Ser Ala Gly Leu Gln Arg Tyr Ala Ala Leu Val Glu Gln Tyr Gln
   130 135 140
   Ala Arg Gly Gly Tyr Glu Ala Asp Val Arg Val Glu Val Ala Leu His
   145 150 155 160
   Gly Leu Gly Leu Pro Ser Leu Asp Arg Asp Arg Lys Leu Gly Thr Leu
   165 170 175
   Ser Gly Gly Glu Arg Ser Arg Leu Ala Leu Ala Ala Thr Leu Ala Ser
   180 185 190
   Ser Pro Glu Leu Leu Leu Leu Asp Glu Pro Thr Asn Asp Leu Asp Asp
   195 200 205
   Arg Ala Met Glu Trp Leu Glu Asp His Leu Ala Gly His Arg Gly Thr
   210 215 220
   Val Ile Ala Val Thr His Asp Arg Val Phe Leu Asp Arg Leu Thr Thr
   225 230 235 240
   Thr Ile Leu Glu Val Asp Ser Gly Ser Val Thr Arg Tyr Gly Asn Gly
   245 250 255
   Tyr Glu Gly Tyr Leu Thr Ala Lys Ala Val Glu Arg Glu Arg Arg Leu
   260 265 270
   Arg Glu Tyr Glu Glu Trp Arg Ala Glu Leu Asp Arg Asn Arg Gly Leu
   275 280 285
   Ile Thr Ser Asn Val Ala Arg Met Asp Gly Ile Pro Arg Lys Met Ser
   290 295 300
   Leu Ser Val Phe Gly His Gly Ala Tyr Arg Arg Arg Gly Arg Asp His
   305 310 315 320
   Gly Ala Met Val Arg Ile Arg Asn Ala Lys Gln Arg Val Ala Gln Leu
   325 330 335
   Thr Glu Asn Pro Val His Ala Pro Ala Asp Pro Leu Ser Phe Ala Ala
   340 345 350
   Arg Ile Asp Thr Ala Gly Pro Glu Ala Glu Glu Ala Val Ala Glu Leu
   355 360 365
   Thr Asp Val Arg Val Ala Gly Arg Leu Ala Val Asp Ser Leu Thr Ile
   370 375 380
   Arg Pro Gly Glu Arg Leu Leu Val Thr Gly Pro Asn Gly Ala Gly Lys
   385 390 395 400
   Ser Thr Leu Leu Arg Val Leu Ser Gly Glu Leu Glu Pro Asp Gly Gly
   405 410 415
   Ser Val Arg Val Gly Cys Arg Val Gly His Leu Arg Gln Asp Glu Thr
   420 425 430
   Pro Trp Ala Pro Gly Leu Thr Val Leu Arg Ala Phe Ala Gln Gly Arg
   435 440 445
   Glu Gly Tyr Leu Glu Asp His Ala Glu Lys Leu Leu Ser Leu Gly Leu
   450 455 460
   Phe Ser Pro Ser Asp Leu Arg Arg Arg Val Lys Asp Leu Ser Tyr Gly
   465 470 475 480
   Gln Arg Arg Arg Ile Glu Ile Ala Arg Leu Val Ser Asp Pro Met Asp
   485 490 495
   Leu Leu Leu Leu Asp Glu Pro Thr Asn His Leu Thr Pro Val Leu Val
   500 505 510
   Glu Glu Leu Glu Gln Ala Leu Ala Asp Tyr Arg Gly Ala Val Val Val
   515 520 525
   Val Thr His Asp Arg Arg Met Arg Ser Arg Phe Thr Gly Ala Arg Leu
   530 535 540
   Thr Met Gly Asp Gly Arg Ile Ala Glu Phe Ser Ala Gly
   545 550 555
<210> 52
   <211> 550
   <212> PRT
   <213> Streptomyces ambofaciens
<400> 52
   Val Ser Ile Ala Gln Tyr Ala Leu His Asp Ile Thr Lys Arg Tyr His
   1 5 10 15
   Asp Cys Val Val Leu Asp Arg Val Gly Phe Ser Ile Lys Pro Gly Glu
   20 25 30
   Lys Val Gly Val Ile Gly Asp Asn Gly Ser Gly Lys Ser Thr Leu Leu
   35 40 45
   Lys Ile Leu Ala Gly Arg Val Glu Pro Asp Asn Gly Ala Leu Thr Val
   50 55 60
   Val Ala Pro Gly Gly Val Gly Tyr Leu Ala Gln Thr Leu Glu Leu Pro
   65 70 75 80
   Leu Asp Ala Thr Val Gln Asp Ala Val Asp Leu Ala Leu Ser Asp Leu
   85 90 95
   Arg Glu Leu Glu Ala Ala Met Arg Glu Ala Glu Ala Glu Leu Gly Glu
   100 105 110
   Ser Asp Glu Asn Gly Ser Glu Arg Glu Leu Ser Ala Gly Leu Gln Arg
   115 120 125
   Tyr Ala Ala Leu Val Glu Gln Tyr Gln Ala Arg Gly Gly Tyr Glu Ala
   130 135 140
   Asp Val Arg Val Glu Val Ala Leu His Gly Leu Gly Leu Pro Ser Leu
   145 150 155 160
   Asp Arg Asp Arg Lys Leu Gly Thr Leu Ser Gly Gly Glu Arg Ser Arg
   165 170 175
   Leu Ala Leu Ala Ala Thr Leu Ala Ser Ser Pro Glu Leu Leu Leu Leu
   180 185 190
   Asp Glu Pro Thr Asn Asp Leu Asp Asp Arg Ala Met Glu Trp Leu Glu
   195 200 205
   Asp His Leu Ala Gly His Arg Gly Thr Val Ile Ala Val Thr His Asp
   210 215 220
   Arg Val Phe Leu Asp Arg Leu Thr Thr Thr Ile Leu Glu Val Asp Ser
   225 230 235 240
   Gly Ser Val Thr Arg Tyr Gly Asn Gly Tyr Glu Gly Tyr Leu Thr Ala
   245 250 255
   Lys Ala Val Glu Arg Glu Arg Arg Leu Arg Glu Tyr Glu Glu Trp Arg
   260 265 270
   Ala Glu Leu Asp Arg Asn Arg Gly Leu Ile Thr Ser Asn Val Ala Arg
   275 280 285
   Met Asp Gly Ile Pro Arg Lys Met Ser Leu Ser Val Phe Gly His Gly
       290                 295                 300
   Ala Tyr Arg Arg Arg Gly Arg Asp His Gly Ala Met Val Arg Ile Arg
   305                 310                 315                 320
   Asn Ala Lys Gln Arg Val Ala Gln Leu Thr Glu Asn Pro Val His Ala
                   325                 330                 335
   Pro Ala Asp Pro Leu Ser Phe Ala Ala Arg Ile Asp Thr Ala Gly Pro
               340                 345                 350
   Glu Ala Glu Glu Ala Val Ala Glu Leu Thr Asp Val Arg Val Ala Gly
           355                 360                 365
   Arg Leu Ala Val Asp Ser Leu Thr Ile Arg Pro Gly Glu Arg Leu Leu
       370                 375                 380
   Val Thr Gly Pro Asn Gly Ala Gly Lys Ser Thr Leu Leu Arg Val Leu
   385                 390                 395                 400
   Ser Gly Glu Leu Glu Pro Asp Gly Gly Ser Val Arg Val Gly Cys Arg
                   405                 410                 415
   Val Gly His Leu Arg Gln Asp Glu Thr Pro Trp Ala Pro Gly Leu Thr
               420                 425                 430
   Val Leu Arg Ala Phe Ala Gln Gly Arg Glu Gly Tyr Leu Glu Asp His
           435                 440                 445
   Ala Glu Lys Leu Leu Ser Leu Gly Leu Phe Ser Pro Ser Asp Leu Arg
       450                 455                 460
   Arg Arg Val Lys Asp Leu Ser Tyr Gly Gln Arg Arg Arg Ile Glu Ile
   465                 470                 475                 480
   Ala Arg Leu Val Ser Asp Pro Met Asp Leu Leu Leu Leu Asp Glu Pro
                   485                 490                 495
   Thr Asn His Leu Thr Pro Val Leu Val Glu Glu Leu Glu Gln Ala Leu
               500                 505                 510
   Ala Asp Tyr Arg Gly Ala Val Val Val Val Thr His Asp Arg Arg Met
           515                 520                 525
   Arg Ser Arg Phe Thr Gly Ala Arg Leu Thr Met Gly Asp Gly Arg Ile
       530                 535                 540
   Ala Glu Phe Ser Ala Gly
   545                 550
<210> 53
   <211> 1431
   <212> DNA
   <213> Streptomyces ambofaciens
<220>
   <221> CDS
   <222> (1)..(1431)
<220>
   <221> CDS2
   <222> (16)..(1431)
<220>
   <221> CDS3
   <222> (37)..(1431)
<220>
   <221> CDS4
   <222> (40)..(1431)
<220>
   <221> CDS5
   <222> (79)..(1431)
<220>
   <221> CDS6
   <222> (130)..(1431)
<400> 53
   gtg ccg cgc aac ggt atg cgt gtc gca ccg gcc gac gtg atg cag tcg       48
   Val Pro Arg Asn Gly Met Arg Val Ala Pro Ala Asp Val Met Gln Ser
   1               5                   10                  15
   gac cgg gat gat cgc ttg tcc ggc ggc cgg atg cct agc ctc ggg agc       96
   Asp Arg Asp Asp Arg Leu Ser Gly Gly Arg Met Pro Ser Leu Gly Ser
               20                  25                  30
   aac cac agc ggt ctt tca cga gag ggg tcg acc atg ggc gat ctc agg      144
   Asn His Ser Gly Leu Ser Arg Glu Gly Ser Thr Met Gly Asp Leu Arg
           35                  40                  45
   aac cgc atc acc gag ctg gtc cgc gcg tac cac cgg gaa cag gcg ccc      192
   Asn Arg Ile Thr Glu Leu Val Arg Ala Tyr His Arg Glu Gln Ala Pro
       50                  55                  60
   ggg ggc ttc gtt ccc ggg acg acg cac gta ccg gtc tcc ggc gcg gtg      240
   Gly Gly Phe Val Pro Gly Thr Thr His Val Pro Val Ser Gly Ala Val
   65                  70                  75                  80
   ctg agc gag gag gac cgg ctg gcg ctg gtg gag acg gcg ctg gag atg      288
   Leu Ser Glu Glu Asp Arg Leu Ala Leu Val Glu Thr Ala Leu Glu Met
                   85                  90                  95
   cgg atc gcg gcc ggc ccg gcc tcc cgg ggc ttc gag cgg cag ttc gcc      336
   Arg Ile Ala Ala Gly Pro Ala Ser Arg Gly Phe Glu Arg Gln Phe Ala
               100                 105                 110
   cgg tac ctc ggg ctc cgg aag gcg cac ctg acc aac tcc ggt tcc tcc      384
   Arg Tyr Leu Gly Leu Arg Lys Ala His Leu Thr Asn Ser Gly Ser Ser
           115                 120                 125
   gcc aac ctc ctc gcc ctc ggc gcg ctc acc tcg ccg cag ctg gag gag      432
   Ala Asn Leu Leu Ala Leu Gly Ala Leu Thr Ser Pro Gln Leu Glu Glu
       130                 135                 140
   aga cgg ctg cgt ccg ggg gac gag gtc gtc acg gtc gcc gcc ggg ttc      480
   Arg Arg Leu Arg Pro Gly Asp Glu Val Val Thr Val Ala Ala Gly Phe
   145                 150                 155                 160
   ccc acg acg gtc aac ccg atc ttc cac aac ggg ctg gtg ccc gtc ttc      528
   Pro Thr Thr Val Asn Pro Ile Phe His Asn Gly Leu Val Pro Val Phe
                   165                 170                 175
   gtg gac gtc gag ctc ggc acg tac aac acg acg ccc gag cgc atc gag      576
   Val Asp Val Glu Leu Gly Thr Tyr Asn Thr Thr Pro Glu Arg Ile Glu
               180                 185                 190
   cgg gcc atc ggc ccc cgg acc agg gcg atc atg atc gcg cac gcc ctg      624
   Arg Ala Ile Gly Pro Arg Thr Arg Ala Ile Met Ile Ala His Ala Leu
           195                 200                 205
   ggc aac ccc ttc gag gcc gaa gag gtg gcc cgc ctc gcg gac gag cgg      672
   Gly Asn Pro Phe Glu Ala Glu Glu Val Ala Arg Leu Ala Asp Glu Arg
       210                 215                 220
   gag ctg ttc ctc atc gag gac aac tgc gac gcg gtg ggg tcc cgc tac      720
   Glu Leu Phe Leu Ile Glu Asp Asn Cys Asp Ala Val Gly Ser Arg Tyr
   225                 230                 235                 240
   cgg ggc agg ctc acc ggc tcc ttc ggc gac ctg tcg acc gtc agc ttc      768
   Arg Gly Arg Leu Thr Gly Ser Phe Gly Asp Leu Ser Thr Val Ser Phe
                   245                 250                 255
   tat ccc gcg cac cac atc gcg atg ggt gag ggg ggc tgc gtg ctc acc      816
   Tyr Pro Ala His His Ile Ala Met Gly Glu Gly Gly Cys Val Leu Thr
               260                 265                 270
   gac aac ctg gcc ctg gcg cgg atc gtg gaa tca ctg cgc gac tgg ggg      864
   Asp Asn Leu Ala Leu Ala Arg Ile Val Glu Ser Leu Arg Asp Trp Gly
           275                 280                 285
   cgc gac tgc tgg tgc gag ccg ggt gag gac aac cgc tgc ctc aag cgg      912
   Arg Asp Cys Trp Cys Glu Pro Gly Glu Asp Asn Arg Cys Leu Lys Arg
       290                 295                 300
   ttc gac cag aag atg ggt gac ctg ccg ccc ggg tac gac cac aag tac      960
   Phe Asp Gln Lys Met Gly Asp Leu Pro Pro Gly Tyr Asp His Lys Tyr
   305                 310                 315                 320
   atc ttc tcg cac gtc ggt tac aac ctg aag tcg acc gac ctg cag gcg     1008
   Ile Phe Ser His Val Gly Tyr Asn Leu Lys Ser Thr Asp Leu Gln Ala
                   325                 330                 335
   gcc ctc ggg ctg tcc cag ctg acc cgg atc gag gag ttc acc gag gcc     1056
   Ala Leu Gly Leu Ser Gln Leu Thr Arg Ile Glu Glu Phe Thr Glu Ala
               340                 345                 350
   agg cgc gcc aac tgg cgg cat ctg cgc gcc gcg ttg gac ggg ctg ccc     1104
   Arg Arg Ala Asn Trp Arg His Leu Arg Ala Ala Leu Asp Gly Leu Pro
           355                 360                 365
   ggt ctg ctg ctg cct cat gcc aca ccg ggc agc gat ccg agc tgg ttc     1152
   Gly Leu Leu Leu Pro His Ala Thr Pro Gly Ser Asp Pro Ser Trp Phe
       370                 375                 380
   ggg ttc ctc atc acc gtg gac ccg gac gcc gcg tac agc agg gcg gcc     1200
   Gly Phe Leu Ile Thr Val Asp Pro Asp Ala Ala Tyr Ser Arg Ala Ala
   385                 390                 395                 400
   ctg gtc gac cac ctg gaa tcg cgc cgg atc agc acc cgc cgc ctg ttc     1248
   Leu Val Asp His Leu Glu Ser Arg Arg Ile Ser Thr Arg Arg Leu Phe
                   405                 410                 415
   ggg ggc aac ctc gtg cgg cac ccc gcc tac acc gac cgt cgg tac cgg      1296
   Gly Gly Asn Leu Val Arg His Pro Ala Tyr Thr Asp Arg Arg Tyr Arg
               420                 425                 430
   gtg tcc ggc tcc ctg gag aac agc gac ctg atc acc gac cag acg ttc      1344
   Val Ser Gly Ser Leu Glu Asn Ser Asp Leu Ile Thr Asp Gln Thr Phe
           435                 440                 445
   tgg atc ggg gtc ttc ccc ggc atc acc ccg gag atg atc gcc tac gtc      1392
   Trp Ile Gly Val Phe Pro Gly Ile Thr Pro Glu Met Ile Ala Tyr Val
       450                 455                 460
   ggc gac acg atc cgg gag ttc gtg ctc aag cac tcc tga                  1431
   Gly Asp Thr Ile Arg Glu Phe Val Leu Lys His Ser
   465                 470                 475
<210> 54
   <211> 476
   <212> PRT
   <213> Streptomyces ambofaciens
<400> 54
   Val Pro Arg Asn Gly Met Arg Val Ala Pro Ala Asp Val Met Gln Ser
   1               5                   10                  15
   Asp Arg Asp Asp Arg Leu Ser Gly Gly Arg Met Pro Ser Leu Gly Ser
               20                  25                  30
   Asn His Ser Gly Leu Ser Arg Glu Gly Ser Thr Met Gly Asp Leu Arg
           35                  40                  45
   Asn Arg Ile Thr Glu Leu Val Arg Ala Tyr His Arg Glu Gln Ala Pro
       50                  55                  60
   Gly Gly Phe Val Pro Gly Thr Thr His Val Pro Val Ser Gly Ala Val
   65                  70                  75                  80
   Leu Ser Glu Glu Asp Arg Leu Ala Leu Val Glu Thr Ala Leu Glu Met
                   85                  90                  95
   Arg Ile Ala Ala Gly Pro Ala Ser Arg Gly Phe Glu Arg Gln Phe Ala
               100                 105                 110
   Arg Tyr Leu Gly Leu Arg Lys Ala His Leu Thr Asn Ser Gly Ser Ser
           115                 120                 125
   Ala Asn Leu Leu Ala Leu Gly Ala Leu Thr Ser Pro Gln Leu Glu Glu
       130                 135                 140
   Arg Arg Leu Arg Pro Gly Asp Glu Val Val Thr Val Ala Ala Gly Phe
   145                 150                 155                 160
   Pro Thr Thr Val Asn Pro Ile Phe His Asn Gly Leu Val Pro Val Phe
                   165                 170                 175
   Val Asp Val Glu Leu Gly Thr Tyr Asn Thr Thr Pro Glu Arg Ile Glu
   180 185 190
   Arg Ala Ile Gly Pro Arg Thr Arg Ala Ile Met Ile Ala His Ala Leu
   195 200 205
   Gly Asn Pro Phe Glu Ala Glu Glu Val Ala Arg Leu Ala Asp Glu Arg
   210 215 220
   Glu Leu Phe Leu Ile Glu Asp Asn Cys Asp Ala Val Gly Ser Arg Tyr
   225 230 235 240
   Arg Gly Arg Leu Thr Gly Ser Phe Gly Asp Leu Ser Thr Val Ser Phe
   245 250 255
   Tyr Pro Ala His His Ile Ala Met Gly Glu Gly Gly Cys Val Leu Thr
   260 265 270
   Asp Asn Leu Ala Leu Ala Arg Ile Val Glu Ser Leu Arg Asp Trp Gly
   275 280 285
   Arg Asp Cys Trp Cys Glu Pro Gly Glu Asp Asn Arg Cys Leu Lys Arg
   290 295 300
   Phe Asp Gln Lys Met Gly Asp Leu Pro Pro Gly Tyr Asp His Lys Tyr
   305 310 315 320
   Ile Phe Ser His Val Gly Tyr Asn Leu Lys Ser Thr Asp Leu Gln Ala
   325 330 335
   Ala Leu Gly Leu Ser Gln Leu Thr Arg Ile Glu Glu Phe Thr Glu Ala
   340 345 350
   Arg Arg Ala Asn Trp Arg His Leu Arg Ala Ala Leu Asp Gly Leu Pro
   355 360 365
   Gly Leu Leu Leu Pro His Ala Thr Pro Gly Ser Asp Pro Ser Trp Phe
   370 375 380
   Gly Phe Leu Ile Thr Val Asp Pro Asp Ala Ala Tyr Ser Arg Ala Ala
   385 390 395 400
   Leu Val Asp His Leu Glu Ser Arg Arg Ile Ser Thr Arg Arg Leu Phe
   405 410 415
   Gly Gly Asn Leu Val Arg His Pro Ala Tyr Thr Asp Arg Arg Tyr Arg
   420 425 430
   Val Ser Gly Ser Leu Glu Asn Ser Asp Leu Ile Thr Asp Gln Thr Phe
   435 440 445
   Trp Ile Gly Val Phe Pro Gly Ile Thr Pro Glu Met Ile Ala Tyr Val
   450 455 460
   Gly Asp Thr Ile Arg Glu Phe Val Leu Lys His Ser
   465 470 475
<210> 56
   <211> 464
   <212> PRT
   <213> Streptomyces ambofaciens
<400> 56
   Val Met Gln Ser Asp Arg Asp Asp Arg Leu Ser Gly Gly Arg Met Pro
   1               5                   10                  15
   Ser Leu Gly Ser Asn His Ser Gly Leu Ser Arg Glu Gly Ser Thr Met
               20                  25                  30
   Gly Asp Leu Arg Asn Arg Ile Thr Glu Leu Val Arg Ala Tyr His Arg
           35                  40                  45
   Glu Gln Ala Pro Gly Gly Phe Val Pro Gly Thr Thr His Val Pro Val
       50                  55                  60
   Ser Gly Ala Val Leu Ser Glu Glu Asp Arg Leu Ala Leu Val Glu Thr
   65                  70                  75                  80
   Ala Leu Glu Met Arg Ile Ala Ala Gly Pro Ala Ser Arg Gly Phe Glu
                   85                  90                  95
   Arg Gln Phe Ala Arg Tyr Leu Gly Leu Arg Lys Ala His Leu Thr Asn
               100                 105                 110
   Ser Gly Ser Ser Ala Asn Leu Leu Ala Leu Gly Ala Leu Thr Ser Pro
           115                 120                 125
   Gln Leu Glu Glu Arg Arg Leu Arg Pro Gly Asp Glu Val Val Thr Val
       130                 135                 140
   Ala Ala Gly Phe Pro Thr Thr Val Asn Pro Ile Phe His Asn Gly Leu
   145                 150                 155                 160
   Val Pro Val Phe Val Asp Val Glu Leu Gly Thr Tyr Asn Thr Thr Pro
                   165                 170                 175
   Glu Arg Ile Glu Arg Ala Ile Gly Pro Arg Thr Arg Ala Ile Met Ile
               180                 185                 190
   Ala His Ala Leu Gly Asn Pro Phe Glu Ala Glu Glu Val Ala Arg Leu
           195                 200                 205
   Ala Asp Glu Arg Glu Leu Phe Leu Ile Glu Asp Asn Cys Asp Ala Val
       210                 215                 220
   Gly Ser Arg Tyr Arg Gly Arg Leu Thr Gly Ser Phe Gly Asp Leu Ser
   225                 230                 235                 240
   Thr Val Ser Phe Tyr Pro Ala His His Ile Ala Met Gly Glu Gly Gly
                   245                 250                 255
   Cys Val Leu Thr Asp Asn Leu Ala Leu Ala Arg Ile Val Glu Ser Leu
   260 265 270
   Arg Asp Trp Gly Arg Asp Cys Trp Cys Glu Pro Gly Glu Asp Asn Arg
   275 280 285
   Cys Leu Lys Arg Phe Asp Gln Lys Met Gly Asp Leu Pro Pro Gly Tyr
   290 295 300
   Asp His Lys Tyr Ile Phe Ser His Val Gly Tyr Asn Leu Lys Ser Thr
   305 310 315 320
   Asp Leu Gln Ala Ala Leu Gly Leu Ser Gln Leu Thr Arg Ile Glu Glu
   325 330 335
   Phe Thr Glu Ala Arg Arg Ala Asn Trp Arg His Leu Arg Ala Ala Leu
   340 345 350
   Asp Gly Leu Pro Gly Leu Leu Leu Pro His Ala Thr Pro Gly Ser Asp
   355 360 365
   Pro Ser Trp Phe Gly Phe Leu Ile Thr Val Asp Pro Asp Ala Ala Tyr
   370 375 380
   Ser Arg Ala Ala Leu Val Asp His Leu Glu Ser Arg Arg Ile Ser Thr
   385 390 395 400
   Arg Arg Leu Phe Gly Gly Asn Leu Val Arg His Pro Ala Tyr Thr Asp
   405 410 415
   Arg Arg Tyr Arg Val Ser Gly Ser Leu Glu Asn Ser Asp Leu Ile Thr
   420 425 430
   Asp Gln Thr Phe Trp Ile Gly Val Phe Pro Gly Ile Thr Pro Glu Met
   435 440 445
   Ile Ala Tyr Val Gly Asp Thr Ile Arg Glu Phe Val Leu Lys His Ser
   450 455 460
<210> 57
   <211> 463
   <212> PRT
   <213> Streptomyces ambofaciens
<400> 57
   Met Gln Ser Asp Arg Asp Asp Arg Leu Ser Gly Gly Arg Met Pro Ser
   1 5 10 15
   Leu Gly Ser Asn His Ser Gly Leu Ser Arg Glu Gly Ser Thr Met Gly
   20 25 30
   Asp Leu Arg Asn Arg Ile Thr Glu Leu Val Arg Ala Tyr His Arg Glu
   35 40 45
   Gln Ala Pro Gly Gly Phe Val Pro Gly Thr Thr His Val Pro Val Ser
   50 55 60
   Gly Ala Val Leu Ser Glu Glu Asp Arg Leu Ala Leu Val Glu Thr Ala
   65 70 75 80
   Leu Glu Met Arg Ile Ala Ala Gly Pro Ala Ser Arg Gly Phe Glu Arg
   85 90 95
   Gln Phe Ala Arg Tyr Leu Gly Leu Arg Lys Ala His Leu Thr Asn Ser
   100 105 110
   Gly Ser Ser Ala Asn Leu Leu Ala Leu Gly Ala Leu Thr Ser Pro Gln
   115 120 125
   Leu Glu Glu Arg Arg Leu Arg Pro Gly Asp Glu Val Val Thr Val Ala
   130 135 140
   Ala Gly Phe Pro Thr Thr Val Asn Pro Ile Phe His Asn Gly Leu Val
   145 150 155 160
   Pro Val Phe Val Asp Val Glu Leu Gly Thr Tyr Asn Thr Thr Pro Glu
   165 170 175
   Arg Ile Glu Arg Ala Ile Gly Pro Arg Thr Arg Ala Ile Met Ile Ala
   180 185 190
   His Ala Leu Gly Asn Pro Phe Glu Ala Glu Glu Val Ala Arg Leu Ala
   195 200 205
   Asp Glu Arg Glu Leu Phe Leu Ile Glu Asp Asn Cys Asp Ala Val Gly
   210 215 220
   Ser Arg Tyr Arg Gly Arg Leu Thr Gly Ser Phe Gly Asp Leu Ser Thr
   225 230 235 240
   Val Ser Phe Tyr Pro Ala His His Ile Ala Met Gly Glu Gly Gly Cys
   245 250 255
   Val Leu Thr Asp Asn Leu Ala Leu Ala Arg Ile Val Glu Ser Leu Arg
   260 265 270
   Asp Trp Gly Arg Asp Cys Trp Cys Glu Pro Gly Glu Asp Asn Arg Cys
   275 280 285
   Leu Lys Arg Phe Asp Gln Lys Met Gly Asp Leu Pro Pro Gly Tyr Asp
   290 295 300
   His Lys Tyr Ile Phe Ser His Val Gly Tyr Asn Leu Lys Ser Thr Asp
   305 310 315 320
   Leu Gln Ala Ala Leu Gly Leu Ser Gln Leu Thr Arg Ile Glu Glu Phe
   325 330 335
   Thr Glu Ala Arg Arg Ala Asn Trp Arg His Leu Arg Ala Ala Leu Asp
   340 345 350
   Gly Leu Pro Gly Leu Leu Leu Pro His Ala Thr Pro Gly Ser Asp Pro
   355 360 365
   Ser Trp Phe Gly Phe Leu Ile Thr Val Asp Pro Asp Ala Ala Tyr Ser
   370 375 380
   Arg Ala Ala Leu Val Asp His Leu Glu Ser Arg Arg Ile Ser Thr Arg
   385 390 395 400
   Arg Leu Phe Gly Gly Asn Leu Val Arg His Pro Ala Tyr Thr Asp Arg
   405 410 415
   Arg Tyr Arg Val Ser Gly Ser Leu Glu Asn Ser Asp Leu Ile Thr Asp
   420 425 430
   Gln Thr Phe Trp Ile Gly Val Phe Pro Gly Ile Thr Pro Glu Met Ile
   435 440 445
   Ala Tyr Val Gly Asp Thr Ile Arg Glu Phe Val Leu Lys His Ser
   450 455 460
<210> 58
   <211> 450
   <212> PRT
   <213> Streptomyces ambofaciens
<400> 58
   Met Pro Ser Leu Gly Ser Asn His Ser Gly Leu Ser Arg Glu Gly Ser
   1 5 10 15
   Thr Met Gly Asp Leu Arg Asn Arg Ile Thr Glu Leu Val Arg Ala Tyr
   20 25 30
   His Arg Glu Gln Ala Pro Gly Gly Phe Val Pro Gly Thr Thr His Val
   35 40 45
   Pro Val Ser Gly Ala Val Leu Ser Glu Glu Asp Arg Leu Ala Leu Val
   50 55 60
   Glu Thr Ala Leu Glu Met Arg Ile Ala Ala Gly Pro Ala Ser Arg Gly
   65 70 75 80
   Phe Glu Arg Gln Phe Ala Arg Tyr Leu Gly Leu Arg Lys Ala His Leu
   85 90 95
   Thr Asn Ser Gly Ser Ser Ala Asn Leu Leu Ala Leu Gly Ala Leu Thr
   100 105 110
   Ser Pro Gln Leu Glu Glu Arg Arg Leu Arg Pro Gly Asp Glu Val Val
   115 120 125
   Thr Val Ala Ala Gly Phe Pro Thr Thr Val Asn Pro Ile Phe His Asn
   130 135 140
   Gly Leu Val Pro Val Phe Val Asp Val Glu Leu Gly Thr Tyr Asn Thr
   145 150 155 160
   Thr Pro Glu Arg Ile Glu Arg Ala Ile Gly Pro Arg Thr Arg Ala Ile
   165 170 175
   Met Ile Ala His Ala Leu Gly Asn Pro Phe Glu Ala Glu Glu Val Ala
   180 185 190
   Arg Leu Ala Asp Glu Arg Glu Leu Phe Leu Ile Glu Asp Asn Cys Asp
   195 200 205
   Ala Val Gly Ser Arg Tyr Arg Gly Arg Leu Thr Gly Ser Phe Gly Asp
   210 215 220
   Leu Ser Thr Val Ser Phe Tyr Pro Ala His His Ile Ala Met Gly Glu
   225 230 235 240
   Gly Gly Cys Val Leu Thr Asp Asn Leu Ala Leu Ala Arg Ile Val Glu
   245 250 255
   Ser Leu Arg Asp Trp Gly Arg Asp Cys Trp Cys Glu Pro Gly Glu Asp
   260 265 270
   Asn Arg Cys Leu Lys Arg Phe Asp Gln Lys Met Gly Asp Leu Pro Pro
   275 280 285
   Gly Tyr Asp His Lys Tyr Ile Phe Ser His Val Gly Tyr Asn Leu Lys
   290 295 300
   Ser Thr Asp Leu Gln Ala Ala Leu Gly Leu Ser Gln Leu Thr Arg Ile
   305 310 315 320
   Glu Glu Phe Thr Glu Ala Arg Arg Ala Asn Trp Arg His Leu Arg Ala
   325 330 335
   Ala Leu Asp Gly Leu Pro Gly Leu Leu Leu Pro His Ala Thr Pro Gly
   340 345 350
   Ser Asp Pro Ser Trp Phe Gly Phe Leu Ile Thr Val Asp Pro Asp Ala
   355 360 365
   Ala Tyr Ser Arg Ala Ala Leu Val Asp His Leu Glu Ser Arg Arg Ile
   370 375 380
   Ser Thr Arg Arg Leu Phe Gly Gly Asn Leu Val Arg His Pro Ala Tyr
   385 390 395 400
   Thr Asp Arg Arg Tyr Arg Val Ser Gly Ser Leu Glu Asn Ser Asp Leu
   405 410 415
   Ile Thr Asp Gln Thr Phe Trp Ile Gly Val Phe Pro Gly Ile Thr Pro
   420 425 430
   Glu Met Ile Ala Tyr Val Gly Asp Thr Ile Arg Glu Phe Val Leu Lys
   435 440 445
   His Ser
   450
<210> 59
   <211> 433
   <212> PRT
   <213> Streptomyces ambofaciens
<400> 59
   Met Gly Asp Leu Arg Asn Arg Ile Thr Glu Leu Val Arg Ala Tyr His
   1 5 10 15
   Arg Glu Gln Ala Pro Gly Gly Phe Val Pro Gly Thr Thr His Val Pro
   20 25 30
   Val Ser Gly Ala Val Leu Ser Glu Glu Asp Arg Leu Ala Leu Val Glu
   35 40 45
   Thr Ala Leu Glu Met Arg Ile Ala Ala Gly Pro Ala Ser Arg Gly Phe
   50 55 60
   Glu Arg Gln Phe Ala Arg Tyr Leu Gly Leu Arg Lys Ala His Leu Thr
   65 70 75 80
   Asn Ser Gly Ser Ser Ala Asn Leu Leu Ala Leu Gly Ala Leu Thr Ser
   85 90 95
   Pro Gln Leu Glu Glu Arg Arg Leu Arg Pro Gly Asp Glu Val Val Thr
   100 105 110
   Val Ala Ala Gly Phe Pro Thr Thr Val Asn Pro Ile Phe His Asn Gly
   115 120 125
   Leu Val Pro Val Phe Val Asp Val Glu Leu Gly Thr Tyr Asn Thr Thr
   130 135 140
   Pro Glu Arg Ile Glu Arg Ala Ile Gly Pro Arg Thr Arg Ala Ile Met
   145 150 155 160
   Ile Ala His Ala Leu Gly Asn Pro Phe Glu Ala Glu Glu Val Ala Arg
   165 170 175
   Leu Ala Asp Glu Arg Glu Leu Phe Leu Ile Glu Asp Asn Cys Asp Ala
   180 185 190
   Val Gly Ser Arg Tyr Arg Gly Arg Leu Thr Gly Ser Phe Gly Asp Leu
   195 200 205
   Ser Thr Val Ser Phe Tyr Pro Ala His His Ile Ala Met Gly Glu Gly
   210 215 220
   Gly Cys Val Leu Thr Asp Asn Leu Ala Leu Ala Arg Ile Val Glu Ser
   225 230 235 240
   Leu Arg Asp Trp Gly Arg Asp Cys Trp Cys Glu Pro Gly Glu Asp Asn
   245 250 255
   Arg Cys Leu Lys Arg Phe Asp Gln Lys Met Gly Asp Leu Pro Pro Gly
   260 265 270
   Tyr Asp His Lys Tyr Ile Phe Ser His Val Gly Tyr Asn Leu Lys Ser
   275 280 285
   Thr Asp Leu Gln Ala Ala Leu Gly Leu Ser Gln Leu Thr Arg Ile Glu
   290 295 300
   Glu Phe Thr Glu Ala Arg Arg Ala Asn Trp Arg His Leu Arg Ala Ala
   305 310 315 320
   Leu Asp Gly Leu Pro Gly Leu Leu Leu Pro His Ala Thr Pro Gly Ser
   325 330 335
   Asp Pro Ser Trp Phe Gly Phe Leu Ile Thr Val Asp Pro Asp Ala Ala
   340 345 350
   Tyr Ser Arg Ala Ala Leu Val Asp His Leu Glu Ser Arg Arg Ile Ser
   355 360 365
   Thr Arg Arg Leu Phe Gly Gly Asn Leu Val Arg His Pro Ala Tyr Thr
   370 375 380
   Asp Arg Arg Tyr Arg Val Ser Gly Ser Leu Glu Asn Ser Asp Leu Ile
   385 390 395 400
   Thr Asp Gln Thr Phe Trp Ile Gly Val Phe Pro Gly Ile Thr Pro Glu
   405 410 415
   Met Ile Ala Tyr Val Gly Asp Thr Ile Arg Glu Phe Val Leu Lys His
   420 425 430
   Ser
<210> 60
   <211> 1398
   <212> DNA
   <213> Streptomyces ambofaciens
<220>
   <221> CDS
   <222> (1)..(1398)
<400> 60
   atg gac gac acg atg gcc ggc gcc gac gcc gag gaa tgg gac ggc gat 48
   Met Asp Asp Thr Met Ala Gly Ala Asp Ala Glu Glu Trp Asp Gly Asp
   1 5 10 15
   cag tta gac cgc gag gac cgg gcc tcc ctc cgc cgc gtc gcg ggg ctc 96
   Gln Leu Asp Arg Glu Asp Arg Ala Ser Leu Arg Arg Val Ala Gly Leu
   20 25 30
   tcc acc gaa ctg acc gac gtc tcc gag gtc gag tac cgc cag ctg cga 144
   Ser Thr Glu Leu Thr Asp Val Ser Glu Val Glu Tyr Arg Gln Leu Arg
   35 40 45
   ctc gag cgg gtg gtg ctc gtc ggc atc tgg acc tcg gga acg gcc gcg 192
   Leu Glu Arg Val Val Leu Val Gly Ile Trp Thr Ser Gly Thr Ala Ala
   50 55 60
   gag gcc gac agt tcg ctc gcc gag ctg gcg gcg ctc gcc gag acc gcg 240
   Glu Ala Asp Ser Ser Leu Ala Glu Leu Ala Ala Leu Ala Glu Thr Ala
   65 70 75 80
   ggc gcc ctc gtg ctg gac ggc gtc gtg cag cgc cgg cag aag ccg gac 288
   Gly Ala Leu Val Leu Asp Gly Val Val Gln Arg Arg Gln Lys Pro Asp
   85 90 95
   ccg gcg acg tac atc ggc tcg ggc aag gcg tcg cag ctg cgc gac atc 336
   Pro Ala Thr Tyr Ile Gly Ser Gly Lys Ala Ser Gln Leu Arg Asp Ile
   100 105 110
   gtc gag gag acc ggc gcc gac acc gtg gtg tgc gac ggg gaa ctg agc 384
   Val Glu Glu Thr Gly Ala Asp Thr Val Val Cys Asp Gly Glu Leu Ser
   115 120 125
   ccc agt cag ctg atg cac ctg gag gag gtc gtc ggg gtc aag gtc gtg 432
   Pro Ser Gln Leu Met His Leu Glu Glu Val Val Gly Val Lys Val Val
   130 135 140
   gac cgc acg gcc ctg atc ctg gac atc ttc gcg cag cac gcc cag tcc 480
   Asp Arg Thr Ala Leu Ile Leu Asp Ile Phe Ala Gln His Ala Gln Ser
   145 150 155 160
   cgg gag ggc aag gcg cag gtg gcg ctg gcg cag atg cag tac atg ctg 528
   Arg Glu Gly Lys Ala Gln Val Ala Leu Ala Gln Met Gln Tyr Met Leu
   165 170 175
   ccg cgg ctg cgc ggc tgg ggc cag tcg ctg tcc cgg cag atg ggc ggc 576
   Pro Arg Leu Arg Gly Trp Gly Gln Ser Leu Ser Arg Gln Met Gly Gly
                   180                 185                 190
   ggt ggc ggc ggt ggc atg gcc acg cgc ggt ccc ggt gag acg aag atc        624
   Gly Gly Gly Gly Gly Met Ala Thr Arg Gly Pro Gly Glu Thr Lys Ile
           195                 200                 205
   gag acg gac cgg cgg cgg atc aac gac aag atg gcc agg ctc cgc cgg        672
   Glu Thr Asp Arg Arg Arg Ile Asn Asp Lys Met Ala Arg Leu Arg Arg
       210                 215                 220
   gag ctg gag cag ctg aag acc ggc cgg gac gtg aac cgg gag gag cga        720
   Glu Leu Glu Gln Leu Lys Thr Gly Arg Asp Val Asn Arg Glu Glu Arg
   225                 230                 235                 240
   cgg cgc aac aag gtg ctg tcg gtc gcc ctc gcc ggc tac acc aac gcc        768
   Arg Arg Asn Lys Val Leu Ser Val Ala Leu Ala Gly Tyr Thr Asn Ala
                   245                 250                 255
   ggc aag tca tcg ctg ctc aac cgc ctc acc gga gcc ggc gtg ctg gtg        816
   Gly Lys Ser Ser Leu Leu Asn Arg Leu Thr Gly Ala Gly Val Leu Val
               260                 265                 270
   gag aac gcc ctg ttc gcc acc ctg gac acg acc gtg cgg cgg gcg acg        864
   Glu Asn Ala Leu Phe Ala Thr Leu Asp Thr Thr Val Arg Arg Ala Thr
           275                 280                 285
   acg ccg agc ggg cgc ccc tac acc atc gcc gac acc gtg ggc ttc gta        912
   Thr Pro Ser Gly Arg Pro Tyr Thr Ile Ala Asp Thr Val Gly Phe Val
       290                 295                 300
   cgc cac ctc ccg cac cac ctg gtg gag gcg ttc cgt tcc acg atc gaa        960
   Arg His Leu Pro His His Leu Val Glu Ala Phe Arg Ser Thr Ile Glu
   305                 310                 315                 320
   gag gtc gcg gac gcg cat ctg gtg ctg cac gtg gtc gac ggt tcg cac       1008
   Glu Val Ala Asp Ala His Leu Val Leu His Val Val Asp Gly Ser His
                   325                 330                 335
   ccg gac ccc ggc gcg cag ctg gcc tcg gtg cgc gag gtg ctg cgg gac       1056
   Pro Asp Pro Gly Ala Gln Leu Ala Ser Val Arg Glu Val Leu Arg Asp
               340                 345                 350
   gtg ggc gcc ggc gag tcc acc gag gtc gtg gtc gtc aac aag gcc gat       1104
   Val Gly Ala Gly Glu Ser Thr Glu Val Val Val Val Asn Lys Ala Asp
           355                 360                 365
   gtc gcg gac ccg gac gtc ctc gcc cgt ctg ctg gag cag gag ccg gac       1152
   Val Ala Asp Pro Asp Val Leu Ala Arg Leu Leu Glu Gln Glu Pro Asp
       370                 375                 380
   gcg atc gtc gtg tcc gcc cgt tcg ggt cgg ggc atc gac gag ctg cgg       1200
   Ala Ile Val Val Ser Ala Arg Ser Gly Arg Gly Ile Asp Glu Leu Arg
   385                 390                 395                 400
   gaa ctg atc gac cgc ctg ctg ccg cac ccc gag gtg gag gtg gag gtc       1248
   Glu Leu Ile Asp Arg Leu Leu Pro His Pro Glu Val Glu Val Glu Val
                   405                 410                 415
   gtg atc ccc tac gac gag ggg agc ctg gtg gcg cgg gtg cac gac gag       1296
   Val Ile Pro Tyr Asp Glu Gly Ser Leu Val Ala Arg Val His Asp Glu
               420                 425                 430
   ggc gag gtg ctc agc acc gag cac acg ccg gag ggc acc ctg ctc acc       1344
   Gly Glu Val Leu Ser Thr Glu His Thr Pro Glu Gly Thr Leu Leu Thr
           435                 440                 445
   gcg cgg gtc cac ccc gac ctc gcc tcg gag ctt cgg acg ctt ccg cgg       1392
   Ala Arg Val His Pro Asp Leu Ala Ser Glu Leu Arg Thr Leu Pro Arg
   450 455 460
   ccg tga 1398
   Pro
   465
<210> 61
   <211> 465
   <212> PRT
   <213> Streptomyces ambofaciens
<400> 61
   Met Asp Asp Thr Met Ala Gly Ala Asp Ala Glu Glu Trp Asp Gly Asp
   1 5 10 15
   Gln Leu Asp Arg Glu Asp Arg Ala Ser Leu Arg Arg Val Ala Gly Leu
   20 25 30
   Ser Thr Glu Leu Thr Asp Val Ser Glu Val Glu Tyr Arg Gln Leu Arg
   35 40 45
   Leu Glu Arg Val Val Leu Val Gly Ile Trp Thr Ser Gly Thr Ala Ala
   50 55 60
   Glu Ala Asp Ser Ser Leu Ala Glu Leu Ala Ala Leu Ala Glu Thr Ala
   65 70 75 80
   Gly Ala Leu Val Leu Asp Gly Val Val Gln Arg Arg Gln Lys Pro Asp
   85 90 95
   Pro Ala Thr Tyr Ile Gly Ser Gly Lys Ala Ser Gln Leu Arg Asp Ile
   100 105 110
   Val Glu Glu Thr Gly Ala Asp Thr Val Val Cys Asp Gly Glu Leu Ser
   115 120 125
   Pro Ser Gln Leu Met His Leu Glu Glu Val Val Gly Val Lys Val Val
   130 135 140
   Asp Arg Thr Ala Leu Ile Leu Asp Ile Phe Ala Gln His Ala Gln Ser
   145 150 155 160
   Arg Glu Gly Lys Ala Gln Val Ala Leu Ala Gln Met Gln Tyr Met Leu
   165 170 175
   Pro Arg Leu Arg Gly Trp Gly Gln Ser Leu Ser Arg Gln Met Gly Gly
   180 185 190
   Gly Gly Gly Gly Gly Met Ala Thr Arg Gly Pro Gly Glu Thr Lys Ile
   195 200 205
   Glu Thr Asp Arg Arg Arg Ile Asn Asp Lys Met Ala Arg Leu Arg Arg
   210 215 220
   Glu Leu Glu Gln Leu Lys Thr Gly Arg Asp Val Asn Arg Glu Glu Arg
   225 230 235 240
   Arg Arg Asn Lys Val Leu Ser Val Ala Leu Ala Gly Tyr Thr Asn Ala
   245 250 255
   Gly Lys Ser Ser Leu Leu Asn Arg Leu Thr Gly Ala Gly Val Leu Val
   260 265 270
   Glu Asn Ala Leu Phe Ala Thr Leu Asp Thr Thr Val Arg Arg Ala Thr
   275 280 285
   Thr Pro Ser Gly Arg Pro Tyr Thr Ile Ala Asp Thr Val Gly Phe Val
   290 295 300
   Arg His Leu Pro His His Leu Val Glu Ala Phe Arg Ser Thr Ile Glu
   305 310 315 320
   Glu Val Ala Asp Ala His Leu Val Leu His Val Val Asp Gly Ser His
   325 330 335
   Pro Asp Pro Gly Ala Gln Leu Ala Ser Val Arg Glu Val Leu Arg Asp
   340 345 350
   Val Gly Ala Gly Glu Ser Thr Glu Val Val Val Val Asn Lys Ala Asp
   355 360 365
   Val Ala Asp Pro Asp Val Leu Ala Arg Leu Leu Glu Gln Glu Pro Asp
   370 375 380
   Ala Ile Val Val Ser Ala Arg Ser Gly Arg Gly Ile Asp Glu Leu Arg
   385 390 395 400
   Glu Leu Ile Asp Arg Leu Leu Pro His Pro Glu Val Glu Val Glu Val
   405 410 415
   Val Ile Pro Tyr Asp Glu Gly Ser Leu Val Ala Arg Val His Asp Glu
   420 425 430
   Gly Glu Val Leu Ser Thr Glu His Thr Pro Glu Gly Thr Leu Leu Thr
   435 440 445
   Ala Arg Val His Pro Asp Leu Ala Ser Glu Leu Arg Thr Leu Pro Arg
   450 455 460
   Pro
   465
<210> 62
   <211> 1044
   <212> DNA
   <213> Streptomyces ambofaciens
<220>
   <221> CDS
   <222> (1)..(1044)
<400> 62
   atg cga gta ctc att atc ggg ggt tca cag ttc gtg ggc cgg gcc ttc      48
   Met Arg Val Leu Ile Ile Gly Gly Ser Gln Phe Val Gly Arg Ala Phe
   1               5                   10                  15
   gcc gcc gag gca ctg gcc gcg ggg cac cgg gtc acc acg ttc aac cgg      96
   Ala Ala Glu Ala Leu Ala Ala Gly His Arg Val Thr Thr Phe Asn Arg
               20                  25                  30
   ggt gtc agc ggc acc gac ctg ccc ggc gtc gag gcg gtc agg ggc gac     144
   Gly Val Ser Gly Thr Asp Leu Pro Gly Val Glu Ala Val Arg Gly Asp
           35                  40                  45
   cgc gag gtg gcc ggc gac ctg gag cgg ctg gtg tcc gga agg cac tgg     192
   Arg Glu Val Ala Gly Asp Leu Glu Arg Leu Val Ser Gly Arg His Trp
       50                  55                  60
   gac gcg gtc gtg gac acc tgc ggt tac gtg ccc cgc acg gtg ggg gcc     240
   Asp Ala Val Val Asp Thr Cys Gly Tyr Val Pro Arg Thr Val Gly Ala
   65                  70                  75                  80
   tcg gcc gcg gcg ctg tcc ggg cac gcg gac acc tac ctc tac gtc tcc     288
   Ser Ala Ala Ala Leu Ser Gly His Ala Asp Thr Tyr Leu Tyr Val Ser
                   85                  90                  95
   agc atc gcc tgc ctg ccc gac tgg gcg cag gcg gtc cgt ccg gtg gac     336
   Ser Ile Ala Cys Leu Pro Asp Trp Ala Gln Ala Val Arg Pro Val Asp
               100                 105                 110
   gac gac tca cct gcc cac gac tgc ccg ccg gac gcc gga ccg gac cac     384
   Asp Asp Ser Pro Ala His Asp Cys Pro Pro Asp Ala Gly Pro Asp His
           115                 120                 125
   gcc gac ggt gac tac ggc gtc ctg aag gcc ggc tgc gag cgc gcc gtg     432
   Ala Asp Gly Asp Tyr Gly Val Leu Lys Ala Gly Cys Glu Arg Ala Val
       130                 135                 140
   gac cgg cac ttc gcg ggc cgg acc ctg cac ctg cgg gcc ggt gtc atc     480
   Asp Arg His Phe Ala Gly Arg Thr Leu His Leu Arg Ala Gly Val Ile
   145                 150                 155                 160
   ctc ggg ccg cac gac acc atg cgc atg ctc gac gcc tgg ctg tgg cgc     528
   Leu Gly Pro His Asp Thr Met Arg Met Leu Asp Ala Trp Leu Trp Arg
                   165                 170                 175
   atg cgc gtc gcc gag ggg gag cac cgc cgg gtg ctc gcc ccg ggc aac     576
   Met Arg Val Ala Glu Gly Glu His Arg Arg Val Leu Ala Pro Gly Asn
               180                 185                 190
   ccc gag gtg ggc atg cgc ctg atc gac gta cgc gat gtc gcc gtc ttc     624
   Pro Glu Val Gly Met Arg Leu Ile Asp Val Arg Asp Val Ala Val Phe
           195                 200                 205
   ggc ctc gac tgc ctc gcg gac ggc cgt acc ggc gcc ttc atc gtc aac     672
   Gly Leu Asp Cys Leu Ala Asp Gly Arg Thr Gly Ala Phe Ile Val Asn
       210                 215                 220
   ccg ccg gag aag aac acc acc ttc ggg gag ttg ctc acg gag tgc gtc     720
   Pro Pro Glu Lys Asn Thr Thr Phe Gly Glu Leu Leu Thr Glu Cys Val
   225                 230                 235                 240
   aag gcc acc ggt tcg gcc gcg gag ccg gtg tgg gtc gac gag ggg ttc      768
   Lys Ala Thr Gly Ser Ala Ala Glu Pro Val Trp Val Asp Glu Gly Phe
                   245                 250                 255
   ctc gcc gag cac ggc gtg agt ccg tgg acg gac ctg ccg atg tgg gtg      816
   Leu Ala Glu His Gly Val Ser Pro Trp Thr Asp Leu Pro Met Trp Val
               260                 265                 270
   ccc gac acc gcg cgg gac acc ctc gtg tgg gcg gcc gga gca ccg cgc      864
   Pro Asp Thr Ala Arg Asp Thr Leu Val Trp Ala Ala Gly Ala Pro Arg
           275                 280                 285
   gcc cgg gcc gcg ggt ctg gcc tgc cgg ccc ttc tcc gac acc gtg cgg      912
   Ala Arg Ala Ala Gly Leu Ala Cys Arg Pro Phe Ser Asp Thr Val Arg
       290                 295                 300
   gac gcc tgg gag gtc gtc cgg gac cgg ccc gtc ccg gaa ctg ccg ctc      960
   Asp Ala Trp Glu Val Val Arg Asp Arg Pro Val Pro Glu Leu Pro Leu
   305                 310                 315                 320
   gcg gcc ggc tgc ggc ctg tcc ctg agc cgg gag aag gag ctg ctc gcc     1008
   Ala Ala Gly Cys Gly Leu Ser Leu Ser Arg Glu Lys Glu Leu Leu Ala
                   325                 330                 335
   gcc tgg gac gct cgc ggc ggt gcg gcg gcg ggc tga                    1044
   Ala Trp Asp Ala Arg Gly Gly Ala Ala Ala Gly
               340                 345
<210> 63
   <211> 347
   <212> PRT
   <213> Streptomyces ambofaciens
<400> 63
   Met Arg Val Leu Ile Ile Gly Gly Ser Gln Phe Val Gly Arg Ala Phe
   1               5                   10                  15
   Ala Ala Glu Ala Leu Ala Ala Gly His Arg Val Thr Thr Phe Asn Arg
               20                  25                  30
   Gly Val Ser Gly Thr Asp Leu Pro Gly Val Glu Ala Val Arg Gly Asp
           35                  40                  45
   Arg Glu Val Ala Gly Asp Leu Glu Arg Leu Val Ser Gly Arg His Trp
       50                  55                  60
   Asp Ala Val Val Asp Thr Cys Gly Tyr Val Pro Arg Thr Val Gly Ala
   65                  70                  75                  80
   Ser Ala Ala Ala Leu Ser Gly His Ala Asp Thr Tyr Leu Tyr Val Ser
                   85                  90                  95
   Ser Ile Ala Cys Leu Pro Asp Trp Ala Gln Ala Val Arg Pro Val Asp
               100                 105                 110
   Asp Asp Ser Pro Ala His Asp Cys Pro Pro Asp Ala Gly Pro Asp His
           115                 120                 125
   Ala Asp Gly Asp Tyr Gly Val Leu Lys Ala Gly Cys Glu Arg Ala Val
       130                 135                 140
   Asp Arg His Phe Ala Gly Arg Thr Leu His Leu Arg Ala Gly Val Ile
   145                 150                 155                 160
   Leu Gly Pro His Asp Thr Met Arg Met Leu Asp Ala Trp Leu Trp Arg
                   165                 170                 175
   Met Arg Val Ala Glu Gly Glu His Arg Arg Val Leu Ala Pro Gly Asn
               180                 185                 190
   Pro Glu Val Gly Met Arg Leu Ile Asp Val Arg Asp Val Ala Val Phe
           195                 200                 205
   Gly Leu Asp Cys Leu Ala Asp Gly Arg Thr Gly Ala Phe Ile Val Asn
       210                 215                 220
   Pro Pro Glu Lys Asn Thr Thr Phe Gly Glu Leu Leu Thr Glu Cys Val
   225                 230                 235                 240
   Lys Ala Thr Gly Ser Ala Ala Glu Pro Val Trp Val Asp Glu Gly Phe
                   245                 250                 255
   Leu Ala Glu His Gly Val Ser Pro Trp Thr Asp Leu Pro Met Trp Val
               260                 265                 270
   Pro Asp Thr Ala Arg Asp Thr Leu Val Trp Ala Ala Gly Ala Pro Arg
           275                 280                 285
   Ala Arg Ala Ala Gly Leu Ala Cys Arg Pro Phe Ser Asp Thr Val Arg
       290                 295                 300
   Asp Ala Trp Glu Val Val Arg Asp Arg Pro Val Pro Glu Leu Pro Leu
   305                 310                 315                 320
   Ala Ala Gly Cys Gly Leu Ser Leu Ser Arg Glu Lys Glu Leu Leu Ala
                   325                 330                 335
   Ala Trp Asp Ala Arg Gly Gly Ala Ala Ala Gly
               340                 345
<210> 64
   <211> 1041
   <212> DNA
   <213> Streptomyces ambofaciens
<220>
   <221> CDS
   <222> (1)..(1041)
<400> 64
   atg acc gcc ctg ggt act tct gct gaa ccg ttc gcc gca ccc ggc ccg     48
   Met Thr Ala Leu Gly Thr Ser Ala Glu Pro Phe Ala Ala Pro Gly Pro
   1               5                   10                  15
   ccg cgg ccg gaa gcg tcc ccg gtg ctg cgc ttc gga gcg atc ggc tgc     96
   Pro Arg Pro Glu Ala Ser Pro Val Leu Arg Phe Gly Ala Ile Gly Cys
               20                  25                  30
   ggc gac atc gcg ggc cgt cgc acc ctg ccc gcc ctg ctc tcc acc ccc     144
   Gly Asp Ile Ala Gly Arg Arg Thr Leu Pro Ala Leu Leu Ser Thr Pro
           35                  40                  45
   ggc acc gtg ctg acc tgc gtc ggc agc cgc gac gcg gac cgg gcc aag     192
   Gly Thr Val Leu Thr Cys Val Gly Ser Arg Asp Ala Asp Arg Ala Lys
       50                  55                  60
   gcc ctg ggc agg cac ttc gac tgc gag gcg gtc gcg ccc tac gag gcc     240
   Ala Leu Gly Arg His Phe Asp Cys Glu Ala Val Ala Pro Tyr Glu Ala
   65                  70                  75                  80
   ctg ctg gaa cgc ccc gac gtg gac gcc gtc tac atc gcg gtg ccc agc     288
   Leu Leu Glu Arg Pro Asp Val Asp Ala Val Tyr Ile Ala Val Pro Ser
                   85                  90                  95
   atg ctg cac gcc gaa tgg gcc gcg gcg gcg ctg cgg gcg ggc aaa cac     336
   Met Leu His Ala Glu Trp Ala Ala Ala Ala Leu Arg Ala Gly Lys His
               100                 105                 110
   gtc ctc gtg gag aag ccc gcc gcc gcc aac cac gcc gac gcg gcc cgc     384
   Val Leu Val Glu Lys Pro Ala Ala Ala Asn His Ala Asp Ala Ala Arg
           115                 120                 125
   ctg ttc gcc atg gcc cgg gag cgc gga ctg gta ctg atg gag aac ttc     432
   Leu Phe Ala Met Ala Arg Glu Arg Gly Leu Val Leu Met Glu Asn Phe
       130                 135                 140
   atg ttc ctc cac cac tct caa cac gcc acc gtc aaa gcc ctg ttg gag     480
   Met Phe Leu His His Ser Gln His Ala Thr Val Lys Ala Leu Leu Glu
   145                 150                 155                 160
   gcc ggt gcc atc gga gag ctg cgc acc ttc tcc gcc gcc ttc acc atc     528
   Ala Gly Ala Ile Gly Glu Leu Arg Thr Phe Ser Ala Ala Phe Thr Ile
                   165                 170                 175
   ccg ccg cgc gcc gac gac gac atg cgc tac cga ccc gac atc ggc ggc     576
   Pro Pro Arg Ala Asp Asp Asp Met Arg Tyr Arg Pro Asp Ile Gly Gly
               180                 185                 190
   ggc gca ctg ctc gac aac ggc gtc tac ccg ctg cgg gcc gcg ctg cac     624
   Gly Ala Leu Leu Asp Asn Gly Val Tyr Pro Leu Arg Ala Ala Leu His
           195                 200                 205
   ttc ctc gga ccc gaa ctg cgc ctg acc ggc gcc gtg ctg cgc cgt gac     672
   Phe Leu Gly Pro Glu Leu Arg Leu Thr Gly Ala Val Leu Arg Arg Asp
       210                 215                 220
   cgc cgc cgg ggc gtc gtg gtc tcc ggc agc gtc ctg ctc gcc gcc ccc     720
   Arg Arg Arg Gly Val Val Val Ser Gly Ser Val Leu Leu Ala Ala Pro
   225                 230                 235                 240
   cag ggc gtg gcc gcc cac ctc gcc ttc ggc atg gaa cac ggc tac cgc     768
   Gln Gly Val Ala Ala His Leu Ala Phe Gly Met Glu His Gly Tyr Arg
                   245                 250                 255
   tcg gcg tac gag ctg cac ggc agc acc ggc tcg ctc gcc ctg agc cac     816
   Ser Ala Tyr Glu Leu His Gly Ser Thr Gly Ser Leu Ala Leu Ser His
               260                 265                 270
   gtc ttc acg acc ccg gac agc cac cac ccc gta ctg cgg ctg tcc cgc       864
   Val Phe Thr Thr Pro Asp Ser His His Pro Val Leu Arg Leu Ser Arg
           275                     280                     285
   cag gac cac cgg gag gag cgc gtc ctg ccc gtg gac cgg cac ttc gtg       912
   Gln Asp His Arg Glu Glu Arg Val Leu Pro Val Asp Arg His Phe Val
       290                     295                     300
   aac atc ctg tcg gtc ttc cgc cgt gcg gtg atc cgg gcc gag gac gtc       960
   Asn Ile Leu Ser Val Phe Arg Arg Ala Val Ile Arg Ala Glu Asp Val
   305                 310                 315                 320
   tcc gcc gag tcg tac gcc gcg ctg cgc cag gcg ggg ctg gtc gac gag      1008
   Ser Ala Glu Ser Tyr Ala Ala Leu Arg Gln Ala Gly Leu Val Asp Glu
                   325                 330                 335
   atc gtg gcc cgc gcc gag acc ttc acc gtg tag                           1041
   Ile Val Ala Arg Ala Glu Thr Phe Thr Val
               340                 345
<210> 65
   <211> 346
   <212> PRT
   <213> Streptomyces ambofaciens
<400> 65
   Met Thr Ala Leu Gly Thr Ser Ala Glu Pro Phe Ala Ala Pro Gly Pro
   1 5 10 15
   Pro Arg Pro Glu Ala Ser Pro Val Leu Arg Phe Gly Ala Ile Gly Cys
   20 25 30
   Gly Asp Ile Ala Gly Arg Arg Thr Leu Pro Ala Leu Leu Ser Thr Pro
   35 40 45
   Gly Thr Val Leu Thr Cys Val Gly Ser Arg Asp Ala Asp Arg Ala Lys
   50 55 60
   Ala Leu Gly Arg His Phe Asp Cys Glu Ala Val Ala Pro Tyr Glu Ala
   65 70 75 80
   Leu Leu Glu Arg Pro Asp Val Asp Ala Val Tyr Ile Ala Val Pro Ser
   85 90 95
   Met Leu His Ala Glu Trp Ala Ala Ala Ala Leu Arg Ala Gly Lys His
   100 105 110
   Val Leu Val Glu Lys Pro Ala Ala Ala Asn His Ala Asp Ala Ala Arg
   115 120 125
   Leu Phe Ala Met Ala Arg Glu Arg Gly Leu Val Leu Met Glu Asn Phe
   130 135 140
   Met Phe Leu His His Ser Gln His Ala Thr Val Lys Ala Leu Leu Glu
   145 150 155 160
   Ala Gly Ala Ile Gly Glu Leu Arg Thr Phe Ser Ala Ala Phe Thr Ile
   165 170 175
   Pro Pro Arg Ala Asp Asp Asp Met Arg Tyr Arg Pro Asp Ile Gly Gly
   180 185 190
   Gly Ala Leu Leu Asp Asn Gly Val Tyr Pro Leu Arg Ala Ala Leu His
   195 200 205
   Phe Leu Gly Pro Glu Leu Arg Leu Thr Gly Ala Val Leu Arg Arg Asp
   210 215 220
   Arg Arg Arg Gly Val Val Val Ser Gly Ser Val Leu Leu Ala Ala Pro
   225 230 235 240
   Gln Gly Val Ala Ala His Leu Ala Phe Gly Met Glu His Gly Tyr Arg
   245 250 255
   Ser Ala Tyr Glu Leu His Gly Ser Thr Gly Ser Leu Ala Leu Ser His
   260 265 270
   Val Phe Thr Thr Pro Asp Ser His His Pro Val Leu Arg Leu Ser Arg
   275 280 285
   Gln Asp His Arg Glu Glu Arg Val Leu Pro Val Asp Arg His Phe Val
   290 295 300
   Asn Ile Leu Ser Val Phe Arg Arg Ala Val Ile Arg Ala Glu Asp Val
   305 310 315 320
   Ser Ala Glu Ser Tyr Ala Ala Leu Arg Gln Ala Gly Leu Val Asp Glu
   325 330 335
   Ile Val Ala Arg Ala Glu Thr Phe Thr Val
   340 345
<210> 66
   <211> 1239
   <212> DNA
   <213> Streptomyces ambofaciens
<220>
   <221> CDS
   <222> (1)..(1239)
<400> 66
   gtg acg gac gcg atc acg acc gag ctg gcc gac cgc gaa ctg ggg cgc      48
   Val Thr Asp Ala Ile Thr Thr Glu Leu Ala Asp Arg Glu Leu Gly Arg
   1               5                   10                  15
   aga ctg cac cgg ata cgc ggc gtc cac tgg tat ttc ggc aac cac ggt      96
   Arg Leu His Arg Ile Arg Gly Val His Trp Tyr Phe Gly Asn His Gly
               20                  25                  30
   gac ccg tac gcc ctc atc ctg cgc ggt cag acc gac gac ccg tcg gtg     144
   Asp Pro Tyr Ala Leu Ile Leu Arg Gly Gln Thr Asp Asp Pro Ser Val
           35                  40                  45
   tac gag gag cgg gtc cgc gag ggc ggg ccg ctg ttc cgc agc cgt acc      192
   Tyr Glu Glu Arg Val Arg Glu Gly Gly Pro Leu Phe Arg Ser Arg Thr
       50                  55                  60
   ggg acc tgg gtg acc gcg gac ccg gag gtg gcc gcg gcc gtg ctg ggc      240
   Gly Thr Trp Val Thr Ala Asp Pro Glu Val Ala Ala Ala Val Leu Gly
   65                  70                  75                  80
   gac tcg cgc ttc ggt gcg ctg gac cgc gcc gga cgg cgc ccg gag gag      288
   Asp Ser Arg Phe Gly Ala Leu Asp Arg Ala Gly Arg Arg Pro Glu Glu
                   85                  90                  95
   tac ctc cag ccg tcg ccc gcc acg tac ctg ggg ctg gac cgc gcc gcg      336
   Tyr Leu Gln Pro Ser Pro Ala Thr Tyr Leu Gly Leu Asp Arg Ala Ala
               100                 105                 110
   tac gcg cgt ctg cgg cgg gtg gcc gag ccc gtg ctg ggc gcg gac gcc      384
   Tyr Ala Arg Leu Arg Arg Val Ala Glu Pro Val Leu Gly Ala Asp Ala
           115                 120                 125
   gcc gcc gcg tgg cgc cgg ctc ggc gag gac gtc ggg cgc cgg ctg ctc      432
   Ala Ala Ala Trp Arg Arg Leu Gly Glu Asp Val Gly Arg Arg Leu Leu
       130                 135                 140
   gcc ggc cgc ggt tcc ggc ctc gac ctg acg gcg gac ttc gcc cgc cgg      480
   Ala Gly Arg Gly Ser Gly Leu Asp Leu Thr Ala Asp Phe Ala Arg Arg
   145                 150                 155                 160
   ctg ccg gca ttg gtc ctg gcc gcg tgg ctc ggg gtg ccg ggc gaa cgg      528
   Leu Pro Ala Leu Val Leu Ala Ala Trp Leu Gly Val Pro Gly Glu Arg
                   165                 170                 175
   tgc gac gag tgg gag gag tcg ctg cgg gcg gcg ggg ccg ctg ctg gac      576
   Cys Asp Glu Trp Glu Glu Ser Leu Arg Ala Ala Gly Pro Leu Leu Asp
               180                 185                 190
   ggt ctg ctg tgt ccg cag acg ctg gcg gcc acc cgt gcg gcg gac tcg      624
   Gly Leu Leu Cys Pro Gln Thr Leu Ala Ala Thr Arg Ala Ala Asp Ser
           195                 200                 205
   gcc gcc gag ggg ctg cgc gcg ctg ttg gac gag gtg gtc gcc gcg cgt      672
   Ala Ala Glu Gly Leu Arg Ala Leu Leu Asp Glu Val Val Ala Ala Arg
       210                 215                 220
   ccc ggc ggg tcc ggc gag ggt gcg gtg gcc cgc atg gtc ggc gcc gga      720
   Pro Gly Gly Ser Gly Glu Gly Ala Val Ala Arg Met Val Gly Ala Gly
   225                 230                 235                 240
   gcc gcc ccc gac gac gcg gtg gcc gcc gcc gtg tgc ctg gcg ctc tcg      768
   Ala Ala Pro Asp Asp Ala Val Ala Ala Ala Val Cys Leu Ala Leu Ser
                   245                 250                 255
   gcc gtc gaa ccg acg acg acc ctg gtg tgc gaa gcg gtc cgg ctg ctg      816
   Ala Val Glu Pro Thr Thr Thr Leu Val Cys Glu Ala Val Arg Leu Leu
               260                 265                 270
   ctc gac cga ccc gag tgg tgg cgg cgg ttg tgc gac tcc ccc gct ctg      864
   Leu Asp Arg Pro Glu Trp Trp Arg Arg Leu Cys Asp Ser Pro Ala Leu
           275                 280                 285
   gcg ccg gcc gcg gtc cgg cac acc ctg cgg cac gcg ccc ccg gtg cgg      912
   Ala Pro Ala Ala Val Arg His Thr Leu Arg His Ala Pro Pro Val Arg
       290                 295                 300
   ctg gag agc cgg gtg gcc cac gag gac gtg acg gtg gcg gat cgt ccg      960
   Leu Glu Ser Arg Val Ala His Glu Asp Val Thr Val Ala Asp Arg Pro
   305                 310                 315                 320
   ctg ccc gcc ggg agc cac gtg gtg gtg ctc gtg ggc gcg gca cgg cgc      1008
   Leu Pro Ala Gly Ser His Val Val Val Leu Val Gly Ala Ala Arg Arg
                   325                 330                 335
   gcg ggc gcc ccg gcc gcg gag ccg gcg gac ctg gcg ggc gca ccg gcg      1056
   Ala Gly Ala Pro Ala Ala Glu Pro Ala Asp Leu Ala Gly Ala Pro Ala
               340                 345                 350
   gcg gag ctg ccg gac gac ctg tgg ttc gcg ctg tcc ggg gag ttc gtc      1104
   Ala Glu Leu Pro Asp Asp Leu Trp Phe Ala Leu Ser Gly Glu Phe Val
           355                 360                 365
   ggc cgt gcc gcc gag acc gcg ctg ggc gtg ctg gcc gag gcc gcc ccg      1152
   Gly Arg Ala Ala Glu Thr Ala Leu Gly Val Leu Ala Glu Ala Ala Pro
       370                 375                 380
   gga ctg cgg cgg gac ggc gac atc gtc cgg cgg cgc cgt tcc ccg gtc      1200
   Gly Leu Arg Arg Asp Gly Asp Ile Val Arg Arg Arg Arg Ser Pro Val
   385                 390                 395                 400
   ctc ggc agg tac gcg cgg ttc ccc gtc gcg tac tcc tga                  1239
   Leu Gly Arg Tyr Ala Arg Phe Pro Val Ala Tyr Ser
                   405                 410
<210> 67
   <211> 412
   <212> PRT
   <213> Streptomyces ambofaciens
<400> 67
   Val Thr Asp Ala Ile Thr Thr Glu Leu Ala Asp Arg Glu Leu Gly Arg
   1               5                   10                  15
   Arg Leu His Arg Ile Arg Gly Val His Trp Tyr Phe Gly Asn His Gly
               20                  25                  30
   Asp Pro Tyr Ala Leu Ile Leu Arg Gly Gln Thr Asp Asp Pro Ser Val
           35                  40                  45
   Tyr Glu Glu Arg Val Arg Glu Gly Gly Pro Leu Phe Arg Ser Arg Thr
       50                  55                  60
   Gly Thr Trp Val Thr Ala Asp Pro Glu Val Ala Ala Ala Val Leu Gly
   65                  70                  75                  80
   Asp Ser Arg Phe Gly Ala Leu Asp Arg Ala Gly Arg Arg Pro Glu Glu
                   85                  90                  95
   Tyr Leu Gln Pro Ser Pro Ala Thr Tyr Leu Gly Leu Asp Arg Ala Ala
               100                 105                 110
   Tyr Ala Arg Leu Arg Arg Val Ala Glu Pro Val Leu Gly Ala Asp Ala
           115                 120                 125
   Ala Ala Ala Trp Arg Arg Leu Gly Glu Asp Val Gly Arg Arg Leu Leu
   130 135 140
   Ala Gly Arg Gly Ser Gly Leu Asp Leu Thr Ala Asp Phe Ala Arg Arg
   145 150 155 160
   Leu Pro Ala Leu Val Leu Ala Ala Trp Leu Gly Val Pro Gly Glu Arg
   165 170 175
   Cys Asp Glu Trp Glu Glu Ser Leu Arg Ala Ala Gly Pro Leu Leu Asp
   180 185 190
   Gly Leu Leu Cys Pro Gln Thr Leu Ala Ala Thr Arg Ala Ala Asp Ser
   195 200 205
   Ala Ala Glu Gly Leu Arg Ala Leu Leu Asp Glu Val Val Ala Ala Arg
   210 215 220
   Pro Gly Gly Ser Gly Glu Gly Ala Val Ala Arg Met Val Gly Ala Gly
   225 230 235 240
   Ala Ala Pro Asp Asp Ala Val Ala Ala Ala Val Cys Leu Ala Leu Ser
   245 250 255
   Ala Val Glu Pro Thr Thr Thr Leu Val Cys Glu Ala Val Arg Leu Leu
   260 265 270
   Leu Asp Arg Pro Glu Trp Trp Arg Arg Leu Cys Asp Ser Pro Ala Leu
   275 280 285
   Ala Pro Ala Ala Val Arg His Thr Leu Arg His Ala Pro Pro Val Arg
   290 295 300
   Leu Glu Ser Arg Val Ala His Glu Asp Val Thr Val Ala Asp Arg Pro
   305 310 315 320
   Leu Pro Ala Gly Ser His Val Val Val Leu Val Gly Ala Ala Arg Arg
   325 330 335
   Ala Gly Ala Pro Ala Ala Glu Pro Ala Asp Leu Ala Gly Ala Pro Ala
   340 345 350
   Ala Glu Leu Pro Asp Asp Leu Trp Phe Ala Leu Ser Gly Glu Phe Val
   355 360 365
   Gly Arg Ala Ala Glu Thr Ala Leu Gly Val Leu Ala Glu Ala Ala Pro
   370 375 380
   Gly Leu Arg Arg Asp Gly Asp Ile Val Arg Arg Arg Arg Ser Pro Val
   385 390 395 400
   Leu Gly Arg Tyr Ala Arg Phe Pro Val Ala Tyr Ser
   405 410
<210> 68
   <211> 1272
   <212> DNA
   <213> Streptomyces ambofaciens
<220>
   <221> CDS
   <222> (1)..(1272)
<400> 68
   gtg cgc gtc ctg gtg acc tcc atc ccg cac cac acg cac tac tac cac      48
   Val Arg Val Leu Val Thr Ser Ile Pro His His Thr His Tyr Tyr His
   1               5                   10                  15
   ctg gta ccg ctg atc tgg gct ctg cgt gcc tcg ggg cac gag gtg gtg      96
   Leu Val Pro Leu Ile Trp Ala Leu Arg Ala Ser Gly His Glu Val Val
               20                  25                  30
   gcg gcc ggc cag ccg tcg ctg gtc gac gcc atc acc gcc agc ggc atc     144
   Ala Ala Gly Gln Pro Ser Leu Val Asp Ala Ile Thr Ala Ser Gly Ile
           35                  40                  45
   ccg gcg ttc gcc ctg gcc gag gag gag tcg ctg gcg cag atc ttc gag     192
   Pro Ala Phe Ala Leu Ala Glu Glu Glu Ser Leu Ala Gln Ile Phe Glu
       50                  55                  60
   gag gtc gag ggc gat ctc cag ccg tat cag cac ggc atc gac gag ttc     240
   Glu Val Glu Gly Asp Leu Gln Pro Tyr Gln His Gly Ile Asp Glu Phe
   65                  70                  75                  80
   gac ttc ttc ggc acc ctg aag gac gag ctg gac tgg gag aag ctg ctc     288
   Asp Phe Phe Gly Thr Leu Lys Asp Glu Leu Asp Trp Glu Lys Leu Leu
                   85                  90                  95
   gcc cag cag gtg atc ctg tcc ggc ctg tgg ctg gaa ccg ctc aac ggc     336
   Ala Gln Gln Val Ile Leu Ser Gly Leu Trp Leu Glu Pro Leu Asn Gly
               100                 105                 110
   gcc acg acc ctc gac agc atc gtc gac ttc gcc cgg gcc tgg aag ccc     384
   Ala Thr Thr Leu Asp Ser Ile Val Asp Phe Ala Arg Ala Trp Lys Pro
           115                 120                 125
   gac ctg gtg ctg tgg gag ccg ttc acc tat gcg ggg ccg gtg gcg gcc     432
   Asp Leu Val Leu Trp Glu Pro Phe Thr Tyr Ala Gly Pro Val Ala Ala
       130                 135                 140
   cgg gcg tgc ggg gcc gcg cac gcc cgc gtc ctg tgg ggg ccg gac acg     480
   Arg Ala Cys Gly Ala Ala His Ala Arg Val Leu Trp Gly Pro Asp Thr
   145                 150                 155                 160
   atc ggg ctg ctg cgg acg aag ttc ctt cag gcc cag gcg cgt cag ccc     528
   Ile Gly Leu Leu Arg Thr Lys Phe Leu Gln Ala Gln Ala Arg Gln Pro
                   165                 170                 175
   gag gag cac cgg gac gac ccg gtc gcg gag tgg atg acc tgg gcc ctg     576
   Glu Glu His Arg Asp Asp Pro Val Ala Glu Trp Met Thr Trp Ala Leu
               180                 185                 190
   gcg cgc tac ggg tgc gac ttc cgg gag gag gac gtg ctc ggt cag tgg     624
   Ala Arg Tyr Gly Cys Asp Phe Arg Glu Glu Asp Val Leu Gly Gln Trp
           195                 200                 205
   agc gtg gac ccg atg gcg gag ggc gtc agt ctg ggc ctc gac ctg ccg     672
   Ser Val Asp Pro Met Ala Glu Gly Val Ser Leu Gly Leu Asp Leu Pro
               210                     215                     220
   acc gtc ccg atg cgc tac acc ccg tac aac ggg tcg gcg gtg atc ccc      720
   Thr Val Pro Met Arg Tyr Thr Pro Tyr Asn Gly Ser Ala Val Ile Pro
   225                 230                     235                 240
   gac tgg ctg acc gag gaa ccg aaa cgg cct cgg gtc tgc ctg acc ctg      768
   Asp Trp Leu Thr Glu Glu Pro Lys Arg Pro Arg Val Cys Leu Thr Leu
                   245                     250                 255
   ggg gtg tcc tcg cgg gag cac agt gag gac gag gtc ccg gtg cag agg      816
   Gly Val Ser Ser Arg Glu His Ser Glu Asp Glu Val Pro Val Gln Arg
               260                     265                 270
   ttt atc gag gcg ctg gcc gat ctc gac atc gag ctg gtg gcg acc ctg      864
   Phe Ile Glu Ala Leu Ala Asp Leu Asp Ile Glu Leu Val Ala Thr Leu
           275                     280                 285
   gac gac gcc cag cgg gac ctg ctg ccg agg atc ccg gac aac acg cgc      912
   Asp Asp Ala Gln Arg Asp Leu Leu Pro Arg Ile Pro Asp Asn Thr Arg
       290                     295                 300
   atc gtc gac ttc gtg ccc atg gac gcg ttg ctg ccg acg tgc tcg gcg      960
   Ile Val Asp Phe Val Pro Met Asp Ala Leu Leu Pro Thr Cys Ser Ala
   305                 310                     315                 320
   atc atc aac cac agc ggt tcg ggc acg tgc aac acc gcc gcg ctg cac     1008
   Ile Ile Asn His Ser Gly Ser Gly Thr Cys Asn Thr Ala Ala Leu His
                   325                     330                 335
   ggg gtg ccg cag atc atc ctc ggc ggc atc ctg gac gcc gcc gta cgg     1056
   Gly Val Pro Gln Ile Ile Leu Gly Gly Ile Leu Asp Ala Ala Val Arg
               340                     345                 350
   cag cac atg ttc gcg cag aac tcc gcc gcc ctc acc ttc gct ccg gag     1104
   Gln His Met Phe Ala Gln Asn Ser Ala Ala Leu Thr Phe Ala Pro Glu
           355                     360                 365
   gag gtg acc ggc gcg tcg ctg agg agc gcg ctg gtg cgc ctg ctc gag     1152
   Glu Val Thr Gly Ala Ser Leu Arg Ser Ala Leu Val Arg Leu Leu Glu
       370                     375                 380
   gag ccg cgg ttc cgc gac ggc gcg cgg cgg ctg aag gag cgg atg cgg     1200
   Glu Pro Arg Phe Arg Asp Gly Ala Arg Arg Leu Lys Glu Arg Met Arg
   385                 390                     395                 400
   gcc atg ccc agc ccg gcc ggg atc gtg ccg acc ctg gag cgc ctc acg     1248
   Ala Met Pro Ser Pro Ala Gly Ile Val Pro Thr Leu Glu Arg Leu Thr
                   405                     410                 415
   gcc cag cac cgc cgg gcg tgt tga                                     1272
   Ala Gln His Arg Arg Ala Cys
               420
<210> 69
   <211> 423
   <212> PRT
   <213> Streptomyces ambofaciens
<400> 69
   Val Arg Val Leu Val Thr Ser Ile Pro His His Thr His Tyr Tyr His
   1 5 10 15
   Leu Val Pro Leu Ile Trp Ala Leu Arg Ala Ser Gly His Glu Val Val
   20 25 30
   Ala Ala Gly Gln Pro Ser Leu Val Asp Ala Ile Thr Ala Ser Gly Ile
   35 40 45
   Pro Ala Phe Ala Leu Ala Glu Glu Glu Ser Leu Ala Gln Ile Phe Glu
   50 55 60
   Glu Val Glu Gly Asp Leu Gln Pro Tyr Gln His Gly Ile Asp Glu Phe
   65 70 75 80
   Asp Phe Phe Gly Thr Leu Lys Asp Glu Leu Asp Trp Glu Lys Leu Leu
   85 90 95
   Ala Gln Gln Val Ile Leu Ser Gly Leu Trp Leu Glu Pro Leu Asn Gly
   100 105 110
   Ala Thr Thr Leu Asp Ser Ile Val Asp Phe Ala Arg Ala Trp Lys Pro
   115 120 125
   Asp Leu Val Leu Trp Glu Pro Phe Thr Tyr Ala Gly Pro Val Ala Ala
   130 135 140
   Arg Ala Cys Gly Ala Ala His Ala Arg Val Leu Trp Gly Pro Asp Thr
   145 150 155 160
   Ile Gly Leu Leu Arg Thr Lys Phe Leu Gln Ala Gln Ala Arg Gln Pro
   165 170 175
   Glu Glu His Arg Asp Asp Pro Val Ala Glu Trp Met Thr Trp Ala Leu
   180 185 190
   Ala Arg Tyr Gly Cys Asp Phe Arg Glu Glu Asp Val Leu Gly Gln Trp
   195 200 205
   Ser Val Asp Pro Met Ala Glu Gly Val Ser Leu Gly Leu Asp Leu Pro
   210 215 220
   Thr Val Pro Met Arg Tyr Thr Pro Tyr Asn Gly Ser Ala Val Ile Pro
   225 230 235 240
   Asp Trp Leu Thr Glu Glu Pro Lys Arg Pro Arg Val Cys Leu Thr Leu
   245 250 255
   Gly Val Ser Ser Arg Glu His Ser Glu Asp Glu Val Pro Val Gln Arg
   260 265 270
   Phe Ile Glu Ala Leu Ala Asp Leu Asp Ile Glu Leu Val Ala Thr Leu
   275 280 285
   Asp Asp Ala Gln Arg Asp Leu Leu Pro Arg Ile Pro Asp Asn Thr Arg
   290 295 300
   Ile Val Asp Phe Val Pro Met Asp Ala Leu Leu Pro Thr Cys Ser Ala
   305 310 315 320
   Ile Ile Asn His Ser Gly Ser Gly Thr Cys Asn Thr Ala Ala Leu His
   325 330 335
   Gly Val Pro Gln Ile Ile Leu Gly Gly Ile Leu Asp Ala Ala Val Arg
   340 345 350
   Gln His Met Phe Ala Gln Asn Ser Ala Ala Leu Thr Phe Ala Pro Glu
   355 360 365
   Glu Val Thr Gly Ala Ser Leu Arg Ser Ala Leu Val Arg Leu Leu Glu
   370 375 380
   Glu Pro Arg Phe Arg Asp Gly Ala Arg Arg Leu Lys Glu Arg Met Arg
   385 390 395 400
   Ala Met Pro Ser Pro Ala Gly Ile Val Pro Thr Leu Glu Arg Leu Thr
   405 410 415
   Ala Gln His Arg Arg Ala Cys
   420
<210> 70
   <211> 1179
   <212> DNA
   <213> Streptomyces ambofaciens
<220>
   <221> CDS
   <222> (1)..(1179)
<400> 70
   atg cgg gcc ctc ttc acg acc gcg ccg ctc gcg ggc cac ctg ctt ccg 48
   Met Arg Ala Leu Phe Thr Thr Ala Pro Leu Ala Gly His Leu Leu Pro
   1 5 10 15
   ctg gtg ccc atc gcg tgg gcc ctg cgg gcg gcc ggc cac gag gta ctg 96
   Leu Val Pro Ile Ala Trp Ala Leu Arg Ala Ala Gly His Glu Val Leu
   20 25 30
   gtg gcg acc cgg gag gac ttc gtg ccg gtc gcc ctg cgg tcg ggg ctg 144
   Val Ala Thr Arg Glu Asp Phe Val Pro Val Ala Leu Arg Ser Gly Leu
   35 40 45
   ccg tcc gcc tcg tgc ggg ccg ccc gcc gcg gac ctg gcg ggc gcg gcc 192
   Pro Ser Ala Ser Cys Gly Pro Pro Ala Ala Asp Leu Ala Gly Ala Ala
   50 55 60
   gag gcg ggg gcg ctc gcg cgg ccc cgc gga gcg gcg gag gct cgg ggg 240
   Glu Ala Gly Ala Leu Ala Arg Pro Arg Gly Ala Ala Glu Ala Arg Gly
   65 70 75 80
   gtc ctg agc ggg gcg ctg gcg cgc gtc gcc cgg ggc agt ctg gcg ggg 288
   Val Leu Ser Gly Ala Leu Ala Arg Val Ala Arg Gly Ser Leu Ala Gly
   85 90 95
   gtg cgg cgg ctg gcg gac gcc tgg cgg ccg gat ctg atc gtc agc gaa      336
   Val Arg Arg Leu Ala Asp Ala Trp Arg Pro Asp Leu Ile Val Ser Glu
               100                 105                 110
   cgg gcc gag ttc gcc ggg ccg ctg gtc gcg gcg gcc ctc ggg gtc ccg      384
   Arg Ala Glu Phe Ala Gly Pro Leu Val Ala Ala Ala Leu Gly Val Pro
           115                 120                 125
   tgg gtc cgc tac cac tgg tcg gtc tcg tcc ctg gag gag tac cgg cga      432
   Trp Val Arg Tyr His Trp Ser Val Ser Ser Leu Glu Glu Tyr Arg Arg
       130                 135                 140
   gcg gcc gag gcc gag ttc gcg ccc gag ctg gcg gcg ctc ggc ctc gac      480
   Ala Ala Glu Ala Glu Phe Ala Pro Glu Leu Ala Ala Leu Gly Leu Asp
   145                 150                 155                 160
   cgg ttc ccg gag gcg gcg cgc gtg ctc gat ccg tgg ccg gtg tcg ctg      528
   Arg Phe Pro Glu Ala Ala Arg Val Leu Asp Pro Trp Pro Val Ser Leu
                   165                 170                 175
   cgc cgg ccg gac gcg gtc gcc cac gac ggg gtc cgg cac gta ccg gcc      576
   Arg Arg Pro Asp Ala Val Ala His Asp Gly Val Arg His Val Pro Ala
               180                 185                 190
   cac ggg gac gcc ccc gtc ccc gac tgg gcg ttc acg cgc ggt cgc ggg      624
   His Gly Asp Ala Pro Val Pro Asp Trp Ala Phe Thr Arg Gly Arg Gly
           195                 200                 205
   ccg cgg atc tgc gtg acg ctc ggc acc atg ctg ccc cgg tac ggc gcc      672
   Pro Arg Ile Cys Val Thr Leu Gly Thr Met Leu Pro Arg Tyr Gly Ala
       210                 215                 220
   gcc ggg atg gcc gac ttc ctg acg gag ctg gtg gcg gag acc cgc gga      720
   Ala Gly Met Ala Asp Phe Leu Thr Glu Leu Val Ala Glu Thr Arg Gly
   225                 230                 235                 240
   ggg gac tgc gaa ctg ctc gtg gcg gtc gac gac gac gtc gtc gcg cgg      768
   Gly Asp Cys Glu Leu Leu Val Ala Val Asp Asp Asp Val Val Ala Arg
                   245                 250                 255
   tgg ccg tcg ctg ccc tcc gcg gtg cgg tac gcc ggc cgg ctg ccg ctg      816
   Trp Pro Ser Leu Pro Ser Ala Val Arg Tyr Ala Gly Arg Leu Pro Leu
               260                 265                 270
   gcg gag gtg ctg ccc gcg tgc gac gcg gtg gtg cac cac ggc ggg cag      864
   Ala Glu Val Leu Pro Ala Cys Asp Ala Val Val His His Gly Gly Gln
           275                 280                 285
   ggc acg tcc ctg acc gcg ctg gcc gcg ggt cgg ccg cag gtc gtc atg      912
   Gly Thr Ser Leu Thr Ala Leu Ala Ala Gly Arg Pro Gln Val Val Met
       290                 295                 300
   gcg cgg ctc gac gac cag ttc gac aac gcg cgg gca ctg gcg gcg gcg      960
   Ala Arg Leu Asp Asp Gln Phe Asp Asn Ala Arg Ala Leu Ala Ala Ala
   305                 310                 315                 320
   ggg gcg gcc ctg ctc gta ccg ccg tcc cgg gcc act ccc gcg gcc gtg     1008
   Gly Ala Ala Leu Leu Val Pro Pro Ser Arg Ala Thr Pro Ala Ala Val
                   325                 330                 335
   gcc gcg ggg tgc gcc gaa gtg ctg gag aac gcc ctg tat gcc aag gcg     1056
   Ala Ala Gly Cys Ala Glu Val Leu Glu Asn Ala Leu Tyr Ala Lys Ala
               340                 345                 350
   gca gcc ggg ctc gcc gag gag atg gcg ctg ctg ccg tcg ccg tcg gcg     1104
   Ala Ala Gly Leu Ala Glu Glu Met Ala Leu Leu Pro Ser Pro Ser Ala
           355                 360                 365
   gcg gtc gga ctc ctg gaa cac ccg ggg ccc ggg ccg gac atg ccg cgg      1152
   Ala Val Gly Leu Leu Glu His Pro Gly Pro Gly Pro Asp Met Pro Arg
       370                 375                 380
   agt tac ccg aac gag gat gcg gtg tga                                  1179
   Ser Tyr Pro Asn Glu Asp Ala Val
   385                 390
<210> 71
   <211> 392
   <212> PRT
   <213> Streptomyces ambofaciens
<400> 71
   Met Arg Ala Leu Phe Thr Thr Ala Pro Leu Ala Gly His Leu Leu Pro
   1               5                   10                  15
   Leu Val Pro Ile Ala Trp Ala Leu Arg Ala Ala Gly His Glu Val Leu
               20                  25                  30
   Val Ala Thr Arg Glu Asp Phe Val Pro Val Ala Leu Arg Ser Gly Leu
           35                  40                  45
   Pro Ser Ala Ser Cys Gly Pro Pro Ala Ala Asp Leu Ala Gly Ala Ala
       50                  55                  60
   Glu Ala Gly Ala Leu Ala Arg Pro Arg Gly Ala Ala Glu Ala Arg Gly
   65                  70                  75                  80
   Val Leu Ser Gly Ala Leu Ala Arg Val Ala Arg Gly Ser Leu Ala Gly
                   85                  90                  95
   Val Arg Arg Leu Ala Asp Ala Trp Arg Pro Asp Leu Ile Val Ser Glu
               100                 105                 110
   Arg Ala Glu Phe Ala Gly Pro Leu Val Ala Ala Ala Leu Gly Val Pro
           115                 120                 125
   Trp Val Arg Tyr His Trp Ser Val Ser Ser Leu Glu Glu Tyr Arg Arg
       130                 135                 140
   Ala Ala Glu Ala Glu Phe Ala Pro Glu Leu Ala Ala Leu Gly Leu Asp
   145                 150                 155                 160
   Arg Phe Pro Glu Ala Ala Arg Val Leu Asp Pro Trp Pro Val Ser Leu
                   165                 170                 175
   Arg Arg Pro Asp Ala Val Ala His Asp Gly Val Arg His Val Pro Ala
               180                 185                 190
   His Gly Asp Ala Pro Val Pro Asp Trp Ala Phe Thr Arg Gly Arg Gly
           195                 200                 205
   Pro Arg Ile Cys Val Thr Leu Gly Thr Met Leu Pro Arg Tyr Gly Ala
       210                 215                 220
   Ala Gly Met Ala Asp Phe Leu Thr Glu Leu Val Ala Glu Thr Arg Gly
   225                 230                 235                 240
   Gly Asp Cys Glu Leu Leu Val Ala Val Asp Asp Asp Val Val Ala Arg
                   245                 250                 255
   Trp Pro Ser Leu Pro Ser Ala Val Arg Tyr Ala Gly Arg Leu Pro Leu
               260                 265                 270
   Ala Glu Val Leu Pro Ala Cys Asp Ala Val Val His His Gly Gly Gln
           275                 280                 285
   Gly Thr Ser Leu Thr Ala Leu Ala Ala Gly Arg Pro Gln Val Val Met
       290                 295                 300
   Ala Arg Leu Asp Asp Gln Phe Asp Asn Ala Arg Ala Leu Ala Ala Ala
   305                 310                 315                 320
   Gly Ala Ala Leu Leu Val Pro Pro Ser Arg Ala Thr Pro Ala Ala Val
                   325                 330                 335
   Ala Ala Gly Cys Ala Glu Val Leu Glu Asn Ala Leu Tyr Ala Lys Ala
               340                 345                 350
   Ala Ala Gly Leu Ala Glu Glu Met Ala Leu Leu Pro Ser Pro Ser Ala
           355                 360                 365
   Ala Val Gly Leu Leu Glu His Pro Gly Pro Gly Pro Asp Met Pro Arg
       370                 375                 380
   Ser Tyr Pro Asn Glu Asp Ala Val
   385                 390
<210> 72
   <211> 993
   <212> DNA
   <213> Streptomyces ambofaciens
<220>
   <221> CDS
   <222> (1)..(993)
<400> 72
   gtg aat ctg gaa gta ctc aac cgt tcg aac gat ccg cgc ggg ccg gtg      48
   Val Asn Leu Glu Val Leu Asn Arg Ser Asn Asp Pro Arg Gly Pro Val
   1               5                   10                  15
   atc acg gtg gtc ggc gcg tcc ggc ttc atc ggg tcc gcc ctg gtc gcc      96
   Ile Thr Val Val Gly Ala Ser Gly Phe Ile Gly Ser Ala Leu Val Ala
               20                  25                  30
   gag ctg gcg cgc atg ccg gtg cgg ctg cgg gcg gtg gcc cgg cgc gag 144
   Glu Leu Ala Arg Met Pro Val Arg Leu Arg Ala Val Ala Arg Arg Glu
   35 40 45
   acc ccc gtt ccc gcg ggg gca cgg gcc gcc gtc gag gtc cgc cgg gcg 192
   Thr Pro Val Pro Ala Gly Ala Arg Ala Ala Val Glu Val Arg Arg Ala
   50 55 60
   gac ctc gcc cgg ccg gac gag gtc ggg gcc gcc gtc gag ggg gcg gac 240
   Asp Leu Ala Arg Pro Asp Glu Val Gly Ala Ala Val Glu Gly Ala Asp
   65 70 75 80
   gcc gtc gtg cac ctc gcc gcc cac atc ggc ggc gcg cgg tcg tgg cgc 288
   Ala Val Val His Leu Ala Ala His Ile Gly Gly Ala Arg Ser Trp Arg
   85 90 95
   gcg gcc gac gag cgg tcg ctg cgg gtg aac gtc ggt ctg ctg cgc gac 336
   Ala Ala Asp Glu Arg Ser Leu Arg Val Asn Val Gly Leu Leu Arg Asp
   100 105 110
   gtg gcc gac gcg ttc cgg gac cgc tcg ggg ccc gcc ccg gcc gtg gtc 384
   Val Ala Asp Ala Phe Arg Asp Arg Ser Gly Pro Ala Pro Ala Val Val
   115 120 125
   ctg gcc agt acc ctc cag gcc ggc gtc gag ctg tcc cgg cag ggc ccg 432
   Leu Ala Ser Thr Leu Gln Ala Gly Val Glu Leu Ser Arg Gln Gly Pro
   130 135 140
   tac gcc cgg cag aag tcg gcg gcc gag gag gtc ctg ctg cgg gcc gcc 480
   Tyr Ala Arg Gln Lys Ser Ala Ala Glu Glu Val Leu Leu Arg Ala Ala
   145 150 155 160
   tcc gag gag gtg gtc cgc ggc gtc gtg ctg cgg ctg ccg acc gtc tac 528
   Ser Glu Glu Val Val Arg Gly Val Val Leu Arg Leu Pro Thr Val Tyr
   165 170 175
   ggg cgc agc ccg ctg acc ggg tgg acg ggc cgc ggg gtg gtc gcg tcg 576
   Gly Arg Ser Pro Leu Thr Gly Trp Thr Gly Arg Gly Val Val Ala Ser
   180 185 190
   gtg gca cgg cag gcc gtc tcg ggc gag ccg gtc acg atg tgg cac gac 624
   Val Ala Arg Gln Ala Val Ser Gly Glu Pro Val Thr Met Trp His Asp
   195 200 205
   ggc acg gtc ggg cgc gat ctg ctc cac gtg gag gac gcg gcc cgc gcc 672
   Gly Thr Val Gly Arg Asp Leu Leu His Val Glu Asp Ala Ala Arg Ala
   210 215 220
   ttc gcg gcg gcg ctc ggt cac gtg gag cgg ctg gac ggc ggc acg tgg 720
   Phe Ala Ala Ala Leu Gly His Val Glu Arg Leu Asp Gly Gly Thr Trp
   225 230 235 240
   tcc gtc ggt acg ggc cgg ctg gag ccc ttg gga gag gtg ttc tcg gcc 768
   Ser Val Gly Thr Gly Arg Leu Glu Pro Leu Gly Glu Val Phe Ser Ala
   245 250 255
   ctc gcc ggg ctg gtg gcc gag cgg acg ggg agg ccc ccc gta ccg gtg 816
   Leu Ala Gly Leu Val Ala Glu Arg Thr Gly Arg Pro Pro Val Pro Val
   260 265 270
   gtc tcc acg gag ccg ccc gac cat gcc gag gcg ggc gac ttc gag agc 864
   Val Ser Thr Glu Pro Pro Asp His Ala Glu Ala Gly Asp Phe Glu Ser
   275 280 285
   gcg gtc tgt gac ccc tcc gcg ttc cgc gcg gtg acc ggg tgg tct ccc 912
   Ala Val Cys Asp Pro Ser Ala Phe Arg Ala Val Thr Gly Trp Ser Pro
   290 295 300
   ctc gtt ccg ttg cgg gcg ggg ctc ggc gcc gtg gtg gag acg atg gtg      960
   Leu Val Pro Leu Arg Ala Gly Leu Gly Ala Val Val Glu Thr Met Val
   305                 310                 315                 320
   gcc gac gga gcg agg ggt ggg atc cga acg tga                          993
   Ala Asp Gly Ala Arg Gly Gly Ile Arg Thr
                   325                 330
<210> 73
   <211> 330
   <212> PRT
   <213> Streptomyces ambofaciens
<400> 73
   Val Asn Leu Glu Val Leu Asn Arg Ser Asn Asp Pro Arg Gly Pro Val
   1               5                   10                  15
   Ile Thr Val Val Gly Ala Ser Gly Phe Ile Gly Ser Ala Leu Val Ala
               20                  25                  30
   Glu Leu Ala Arg Met Pro Val Arg Leu Arg Ala Val Ala Arg Arg Glu
           35                  40                  45
   Thr Pro Val Pro Ala Gly Ala Arg Ala Ala Val Glu Val Arg Arg Ala
       50                  55                  60
   Asp Leu Ala Arg Pro Asp Glu Val Gly Ala Ala Val Glu Gly Ala Asp
   65                  70                  75                  80
   Ala Val Val His Leu Ala Ala His Ile Gly Gly Ala Arg Ser Trp Arg
                   85                  90                  95
   Ala Ala Asp Glu Arg Ser Leu Arg Val Asn Val Gly Leu Leu Arg Asp
               100                 105                 110
   Val Ala Asp Ala Phe Arg Asp Arg Ser Gly Pro Ala Pro Ala Val Val
           115                 120                 125
   Leu Ala Ser Thr Leu Gln Ala Gly Val Glu Leu Ser Arg Gln Gly Pro
       130                 135                 140
   Tyr Ala Arg Gln Lys Ser Ala Ala Glu Glu Val Leu Leu Arg Ala Ala
   145                 150                 155                 160
   Ser Glu Glu Val Val Arg Gly Val Val Leu Arg Leu Pro Thr Val Tyr
                   165                 170                 175
   Gly Arg Ser Pro Leu Thr Gly Trp Thr Gly Arg Gly Val Val Ala Ser
               180                 185                 190
   Val Ala Arg Gln Ala Val Ser Gly Glu Pro Val Thr Met Trp His Asp
           195                 200                 205
   Gly Thr Val Gly Arg Asp Leu Leu His Val Glu Asp Ala Ala Arg Ala
       210                 215                 220
   Phe Ala Ala Ala Leu Gly His Val Glu Arg Leu Asp Gly Gly Thr Trp
   225                 230                 235                 240
   Ser Val Gly Thr Gly Arg Leu Glu Pro Leu Gly Glu Val Phe Ser Ala
                   245                 250                 255
   Leu Ala Gly Leu Val Ala Glu Arg Thr Gly Arg Pro Pro Val Pro Val
               260                 265                 270
   Val Ser Thr Glu Pro Pro Asp His Ala Glu Ala Gly Asp Phe Glu Ser
           275                 280                 285
   Ala Val Cys Asp Pro Ser Ala Phe Arg Ala Val Thr Gly Trp Ser Pro
       290                 295                 300
   Leu Val Pro Leu Arg Ala Gly Leu Gly Ala Val Val Glu Thr Met Val
   305                 310                 315                 320
   Ala Asp Gly Ala Arg Gly Gly Ile Arg Thr
                   325                 330
<210> 74
   <211> 1008
   <212> DNA
   <213> Streptomyces ambofaciens
<220>
   <221> CDS
   <222> (1)..(1008)
<400> 74
   gtg agc acg gac cgg gag cag gcc gcg cac acg cgg ctc ggt cgc agc      48
   Val Ser Thr Asp Arg Glu Gln Ala Ala His Thr Arg Leu Gly Arg Ser
   1               5                   10                  15
   gcg acc ctg gtg agc cgg ctc tgg ctg ggc acc gtg aac ttc agc ggc      96
   Ala Thr Leu Val Ser Arg Leu Trp Leu Gly Thr Val Asn Phe Ser Gly
               20                  25                  30
   cgg gtc gag gac ggt gac gcg atg cag ctg atg gag gcg gcg gtc gac      144
   Arg Val Glu Asp Gly Asp Ala Met Gln Leu Met Glu Ala Ala Val Asp
           35                  40                  45
   cgc ggc atc aac tgc atc gac acc gcg gac atc tac ggc tgg cgg atc      192
   Arg Gly Ile Asn Cys Ile Asp Thr Ala Asp Ile Tyr Gly Trp Arg Ile
       50                  55                  60
   cac aag ggc cac acc gag gaa ctg gtg ggc cgg tgg ctg gcc aag agc      240
   His Lys Gly His Thr Glu Glu Leu Val Gly Arg Trp Leu Ala Lys Ser
   65                  70                  75                  80
   gcc gcg cgg cgg gag gac gtc ctg ctg gcc acc aag gtc ggc ggg gac      288
   Ala Ala Arg Arg Glu Asp Val Leu Leu Ala Thr Lys Val Gly Gly Asp
                   85                  90                  95
   atg agc gaa cgg ctc aac gac ggc ggc ctg tcg gcg cgg cac atc gtc      336
   Met Ser Glu Arg Leu Asn Asp Gly Gly Leu Ser Ala Arg His Ile Val
               100                 105                 110
   acg gcc tgc gag cag tcg ctg cgg cgc ctg ggc gtg gac cac atc gac      384
   Thr Ala Cys Glu Gln Ser Leu Arg Arg Leu Gly Val Asp His Ile Asp
           115                 120                 125
   ctg tac cag atg cac cgc gtc gac cac gcc gcg ccg tgg gac gag atc      432
   Leu Tyr Gln Met His Arg Val Asp His Ala Ala Pro Trp Asp Glu Ile
       130                 135                 140
   tgg cag gcg atg gac cgt ctg gtg gcg agc ggc aag gtg acc tac gtg      480
   Trp Gln Ala Met Asp Arg Leu Val Ala Ser Gly Lys Val Thr Tyr Val
   145                 150                 155                 160
   ggg tcg tcg aac ttc gcc ggc tgg aac gtc gcc gcc gcg cag gac gcg      528
   Gly Ser Ser Asn Phe Ala Gly Trp Asn Val Ala Ala Ala Gln Asp Ala
                   165                 170                 175
   gcc cgg cgg cgc cag tcc ctc ggt ctg gtg tcc gag cag tgc ctg tac      576
   Ala Arg Arg Arg Gln Ser Leu Gly Leu Val Ser Glu Gln Cys Leu Tyr
               180                 185                 190
   aac ctg gcg gtg cgc cac gcc gag ctg gaa ctg ctg ccg gcc gcc cag      624
   Asn Leu Ala Val Arg His Ala Glu Leu Glu Leu Leu Pro Ala Ala Gln
           195                 200                 205
   gcg tac gga ctg ggc gtg ttc gcc tgg tcg ccg ctg cac ggc ggg ctg      672
   Ala Tyr Gly Leu Gly Val Phe Ala Trp Ser Pro Leu His Gly Gly Leu
       210                 215                 220
   ctc agc ggg gtg ctg cgc aag ctc gcg gcg ggc gtc gcg gtg aag tcg      720
   Leu Ser Gly Val Leu Arg Lys Leu Ala Ala Gly Val Ala Val Lys Ser
   225                 230                 235                 240
   gca cag ggg cgg gcc cag ctg ctg ctg ccc gag ctg cgc gcg acg atc      768
   Ala Gln Gly Arg Ala Gln Leu Leu Leu Pro Glu Leu Arg Ala Thr Ile
                   245                 250                 255
   gag gcg tac gag ggg ttc tgc ggc cgg atc ggc gcg gat ccg gcc gag      816
   Glu Ala Tyr Glu Gly Phe Cys Gly Arg Ile Gly Ala Asp Pro Ala Glu
               260                 265                 270
   gtc ggt ctg gcc tgg gtg ctg tcc cgg ccg ggg atc agc ggc gcg gtg      864
   Val Gly Leu Ala Trp Val Leu Ser Arg Pro Gly Ile Ser Gly Ala Val
           275                 280                 285
   atc ggt ccg cgc acg gtg gac cag ctg gac tcg gcg ctg cgg tcc ctg      912
   Ile Gly Pro Arg Thr Val Asp Gln Leu Asp Ser Ala Leu Arg Ser Leu
       290                 295                 300
   gac ctg gtc ctc ggg gag gcc gaa ctg gcc gag ctg gac gcc atc ttc      960
   Asp Leu Val Leu Gly Glu Ala Glu Leu Ala Glu Leu Asp Ala Ile Phe
   305                 310                 315                 320
   ccg ccc ctg ggc aag ggc ggc cgg gcg ccg gac gcg tgg atc agc tga     1008
   Pro Pro Leu Gly Lys Gly Gly Arg Ala Pro Asp Ala Trp Ile Ser
                   325                 330                 335
<210> 75
   <211> 335
   <212> PRT
   <213> Streptomyces ambofaciens
<400> 75
   Val Ser Thr Asp Arg Glu Gln Ala Ala His Thr Arg Leu Gly Arg Ser
   1 5 10 15
   Ala Thr Leu Val Ser Arg Leu Trp Leu Gly Thr Val Asn Phe Ser Gly
   20 25 30
   Arg Val Glu Asp Gly Asp Ala Met Gln Leu Met Glu Ala Ala Val Asp
   35 40 45
   Arg Gly Ile Asn Cys Ile Asp Thr Ala Asp Ile Tyr Gly Trp Arg Ile
   50 55 60
   His Lys Gly His Thr Glu Glu Leu Val Gly Arg Trp Leu Ala Lys Ser
   65 70 75 80
   Ala Ala Arg Arg Glu Asp Val Leu Leu Ala Thr Lys Val Gly Gly Asp
   85 90 95
   Met Ser Glu Arg Leu Asn Asp Gly Gly Leu Ser Ala Arg His Ile Val
   100 105 110
   Thr Ala Cys Glu Gln Ser Leu Arg Arg Leu Gly Val Asp His Ile Asp
   115 120 125
   Leu Tyr Gln Met His Arg Val Asp His Ala Ala Pro Trp Asp Glu Ile
   130 135 140
   Trp Gln Ala Met Asp Arg Leu Val Ala Ser Gly Lys Val Thr Tyr Val
   145 150 155 160
   Gly Ser Ser Asn Phe Ala Gly Trp Asn Val Ala Ala Ala Gln Asp Ala
   165 170 175
   Ala Arg Arg Arg Gln Ser Leu Gly Leu Val Ser Glu Gln Cys Leu Tyr
   180 185 190
   Asn Leu Ala Val Arg His Ala Glu Leu Glu Leu Leu Pro Ala Ala Gln
   195 200 205
   Ala Tyr Gly Leu Gly Val Phe Ala Trp Ser Pro Leu His Gly Gly Leu
   210 215 220
   Leu Ser Gly Val Leu Arg Lys Leu Ala Ala Gly Val Ala Val Lys Ser
   225 230 235 240
   Ala Gln Gly Arg Ala Gln Leu Leu Leu Pro Glu Leu Arg Ala Thr Ile
   245 250 255
   Glu Ala Tyr Glu Gly Phe Cys Gly Arg Ile Gly Ala Asp Pro Ala Glu
   260 265 270
   Val Gly Leu Ala Trp Val Leu Ser Arg Pro Gly Ile Ser Gly Ala Val
   275 280 285
   Ile Gly Pro Arg Thr Val Asp Gln Leu Asp Ser Ala Leu Arg Ser Leu
   290 295 300
   Asp Leu Val Leu Gly Glu Ala Glu Leu Ala Glu Leu Asp Ala Ile Phe
   305 310 315 320
   Pro Pro Leu Gly Lys Gly Gly Arg Ala Pro Asp Ala Trp Ile Ser
   325 330 335
<210> 76
   <211> 1233
   <212> DNA
   <213> Streptomyces ambofaciens
<220>
   <221> CDS
   <222> (1)..(1233)
<400> 76
   atg att gcc acc gcg tgc cgc gtc tgc ggc aac aag gag ctg ctc tcc      48
   Met Ile Ala Thr Ala Cys Arg Val Cys Gly Asn Lys Glu Leu Leu Ser
   1               5                   10                  15
   gta ctc gac ctg ggc gaa cag gcg ctc acc ggg gtg ttc ccg gcc gac      96
   Val Leu Asp Leu Gly Glu Gln Ala Leu Thr Gly Val Phe Pro Ala Asp
               20                  25                  30
   cgc gac gag gtc gtg ccg tcg gtc ccg ctg gaa ctc gtc gcg tgt tcc     144
   Arg Asp Glu Val Val Pro Ser Val Pro Leu Glu Leu Val Ala Cys Ser
           35                  40                  45
   ccg gcc gga tgc ggc ctc gtg caa ctg cgc cac acc ccc gac ccg gac     192
   Pro Ala Gly Cys Gly Leu Val Gln Leu Arg His Thr Pro Asp Pro Asp
       50                  55                  60
   ctc atg tac ggc gag ggc tac ggc tac cgc tcg ggc atc cgg ccc ttc     240
   Leu Met Tyr Gly Glu Gly Tyr Gly Tyr Arg Ser Gly Ile Arg Pro Phe
   65                  70                  75                  80
   atg gtc gac cac ctg cat ggc aag gtc gcc gcc gtc cgc cgg ctg gtg     288
   Met Val Asp His Leu His Gly Lys Val Ala Ala Val Arg Arg Leu Val
                   85                  90                  95
   gac ctc ggc ccg gac gac ctg gtc ctc gac atc ggc agc aac gac gcc     336
   Asp Leu Gly Pro Asp Asp Leu Val Leu Asp Ile Gly Ser Asn Asp Ala
               100                 105                 110
   acg ctc ctc aag ggc tac ccc gcc gac ggc ccg cga ctg gtc ggc atc     384
   Thr Leu Leu Lys Gly Tyr Pro Ala Asp Gly Pro Arg Leu Val Gly Ile
           115                 120                 125
   gac ccg acc ggc ggc aag ttc cgc gac ctg tac ccg ccg aac gcc gag     432
   Asp Pro Thr Gly Gly Lys Phe Arg Asp Leu Tyr Pro Pro Asn Ala Glu
       130                 135                 140
   ttg gtc gtg gac tac ttc acc cgc gcg acc ttc gag aac cgc ttc ggg     480
   Leu Val Val Asp Tyr Phe Thr Arg Ala Thr Phe Glu Asn Arg Phe Gly
   145                 150                 155                 160
   gcg cgg cgg gcg aag gcc gtc acc tcc atc gcg atg ttc tac gac ctg     528
   Ala Arg Arg Ala Lys Ala Val Thr Ser Ile Ala Met Phe Tyr Asp Leu
   165 170 175
   ccg gac ccg ctg cgc ttc atg agc gac gtc cgc gac gtc ctc gcc gag 576
   Pro Asp Pro Leu Arg Phe Met Ser Asp Val Arg Asp Val Leu Ala Glu
   180 185 190
   gac ggc gtc tgg atg atg gag cag agc tac ctg ccc gcc atg ctc gaa 624
   Asp Gly Val Trp Met Met Glu Gln Ser Tyr Leu Pro Ala Met Leu Glu
   195 200 205
   gcc gac gcg tac gac atc gtc tgc cac gag cac ctg gag tac tac gcg 672
   Ala Asp Ala Tyr Asp Ile Val Cys His Glu His Leu Glu Tyr Tyr Ala
   210 215 220
   ctg cgc cag atc gag tgg atg gcg gaa cgc gtc ggc ctc acc gtc atc 720
   Leu Arg Gln Ile Glu Trp Met Ala Glu Arg Val Gly Leu Thr Val Ile
   225 230 235 240
   cgg gcc gag ctc acc gag gtg tac ggc ggc agc ctg tgc gtc acg ctc 768
   Arg Ala Glu Leu Thr Glu Val Tyr Gly Gly Ser Leu Cys Val Thr Leu
   245 250 255
   gcc agg acc ggt tcc cgg cac ccg agg gac gag gcg ggc ctg gcc cgc 816
   Ala Arg Thr Gly Ser Arg His Pro Arg Asp Glu Ala Gly Leu Ala Arg
   260 265 270
   atc cgg gcc gcc gag gcc gcc gcg gga ctg gac ggc atg gca ccg ttc 864
   Ile Arg Ala Ala Glu Ala Ala Ala Gly Leu Asp Gly Met Ala Pro Phe
   275 280 285
   gag gcc ttc gcc cgc cgc gtc gcc gac cag cgg gac gcc ctg cgc ggc 912
   Glu Ala Phe Ala Arg Arg Val Ala Asp Gln Arg Asp Ala Leu Arg Gly
   290 295 300
   ttc ctg gac cgc tcc cgc caa gag ggc ctg ctg acc ctc ggg tac ggc 960
   Phe Leu Asp Arg Ser Arg Gln Glu Gly Leu Leu Thr Leu Gly Tyr Gly
   305 310 315 320
   gcc tcc acc aag ggc aac gtc atc ctc cag tac tgc ggg atc acc gaa 1008
   Ala Ser Thr Lys Gly Asn Val Ile Leu Gln Tyr Cys Gly Ile Thr Glu
   325 330 335
   cgg gac ctg ccg tgc atc ggc gag gtg agc gag gag aag gcg ggg cgt 1056
   Arg Asp Leu Pro Cys Ile Gly Glu Val Ser Glu Glu Lys Ala Gly Arg
   340 345 350
   ttc acc ccc ggg acg ggc atc ccc atc gtg tcc gag gag gac gcg aag 1104
   Phe Thr Pro Gly Thr Gly Ile Pro Ile Val Ser Glu Glu Asp Ala Lys
   355 360 365
   gcg cag aag ccg gac cag ttg ctg gtc ctg ccc tgg atc tac cgg gac 1152
   Ala Gln Lys Pro Asp Gln Leu Leu Val Leu Pro Trp Ile Tyr Arg Asp
   370 375 380
   ggg ttc gtc gaa cgg gag cgg gag ttc ctc gac ggc ggc ggc cga ctg 1200
   Gly Phe Val Glu Arg Glu Arg Glu Phe Leu Asp Gly Gly Gly Arg Leu
   385 390 395 400
   gtc ttc ccg ctg ccc gcg ctc gac gtc gtg tga 1233
   Val Phe Pro Leu Pro Ala Leu Asp Val Val
   405 410
<210> 77
   <211> 410
   <212> PRT
   <213> Streptomyces ambofaciens
<400> 77
   Met Ile Ala Thr Ala Cys Arg Val Cys Gly Asn Lys Glu Leu Leu Ser
   1               5                   10                  15
   Val Leu Asp Leu Gly Glu Gln Ala Leu Thr Gly Val Phe Pro Ala Asp
               20                  25                  30
   Arg Asp Glu Val Val Pro Ser Val Pro Leu Glu Leu Val Ala Cys Ser
           35                  40                  45
   Pro Ala Gly Cys Gly Leu Val Gln Leu Arg His Thr Pro Asp Pro Asp
       50                  55                  60
   Leu Met Tyr Gly Glu Gly Tyr Gly Tyr Arg Ser Gly Ile Arg Pro Phe
   65                  70                  75                  80
   Met Val Asp His Leu His Gly Lys Val Ala Ala Val Arg Arg Leu Val
                   85                  90                  95
   Asp Leu Gly Pro Asp Asp Leu Val Leu Asp Ile Gly Ser Asn Asp Ala
               100                 105                 110
   Thr Leu Leu Lys Gly Tyr Pro Ala Asp Gly Pro Arg Leu Val Gly Ile
           115                 120                 125
   Asp Pro Thr Gly Gly Lys Phe Arg Asp Leu Tyr Pro Pro Asn Ala Glu
       130                 135                 140
   Leu Val Val Asp Tyr Phe Thr Arg Ala Thr Phe Glu Asn Arg Phe Gly
   145                 150                 155                 160
   Ala Arg Arg Ala Lys Ala Val Thr Ser Ile Ala Met Phe Tyr Asp Leu
                   165                 170                 175
   Pro Asp Pro Leu Arg Phe Met Ser Asp Val Arg Asp Val Leu Ala Glu
               180                 185                 190
   Asp Gly Val Trp Met Met Glu Gln Ser Tyr Leu Pro Ala Met Leu Glu
           195                 200                 205
   Ala Asp Ala Tyr Asp Ile Val Cys His Glu His Leu Glu Tyr Tyr Ala
       210                 215                 220
   Leu Arg Gln Ile Glu Trp Met Ala Glu Arg Val Gly Leu Thr Val Ile
   225                 230                 235                 240
   Arg Ala Glu Leu Thr Glu Val Tyr Gly Gly Ser Leu Cys Val Thr Leu
                   245                 250                 255
   Ala Arg Thr Gly Ser Arg His Pro Arg Asp Glu Ala Gly Leu Ala Arg
               260                 265                 270
   Ile Arg Ala Ala Glu Ala Ala Ala Gly Leu Asp Gly Met Ala Pro Phe
   275 280 285
   Glu Ala Phe Ala Arg Arg Val Ala Asp Gln Arg Asp Ala Leu Arg Gly
   290 295 300
   Phe Leu Asp Arg Ser Arg Gln Glu Gly Leu Leu Thr Leu Gly Tyr Gly
   305 310 315 320
   Ala Ser Thr Lys Gly Asn Val Ile Leu Gln Tyr Cys Gly Ile Thr Glu
   325 330 335
   Arg Asp Leu Pro Cys Ile Gly Glu Val Ser Glu Glu Lys Ala Gly Arg
   340 345 350
   Phe Thr Pro Gly Thr Gly Ile Pro Ile Val Ser Glu Glu Asp Ala Lys
   355 360 365
   Ala Gln Lys Pro Asp Gln Leu Leu Val Leu Pro Trp Ile Tyr Arg Asp
   370 375 380
   Gly Phe Val Glu Arg Glu Arg Glu Phe Leu Asp Gly Gly Gly Arg Leu
   385 390 395 400
   Val Phe Pro Leu Pro Ala Leu Asp Val Val
   405 410
<210> 78
   <211> 1116
   <212> DNA
   <213> Streptomyces ambofaciens
<220>
   <221> CDS
   <222> (1)..(1116)
<400> 78
   gtg cac cgg gac aac gcc gcc gag ccg ttg gtc aaa tgc ctg gtc tgg 48
   Val His Arg Asp Asn Ala Ala Glu Pro Leu Val Lys Cys Leu Val Trp
   1 5 10 15
   gac ctg gac aac acc ctc tgg cag ggc acc ctc ctc gag gag gac gag 96
   Asp Leu Asp Asn Thr Leu Trp Gln Gly Thr Leu Leu Glu Glu Asp Glu
   20 25 30
   gtc cgg ctc gcc ccg gac gtc ctg cgg acc atc acc gag ctg gac gcg 144
   Val Arg Leu Ala Pro Asp Val Leu Arg Thr Ile Thr Glu Leu Asp Ala
   35 40 45
   cgc ggc atc ctg cag gcg gtg gcc agc aag aac gac cac gat cac gcc 192
   Arg Gly Ile Leu Gln Ala Val Ala Ser Lys Asn Asp His Asp His Ala
   50 55 60
   tgg gcg aag ctc gaa cag ctc ggc gtc gcc gag tac ttc gtg ctc ccg 240
   Trp Ala Lys Leu Glu Gln Leu Gly Val Ala Glu Tyr Phe Val Leu Pro
   65 70 75 80
   agg atc ggc tgg ggc ccg aag tcg aag tcg gtc cgc gag atc gcc gac 288
   Arg Ile Gly Trp Gly Pro Lys Ser Lys Ser Val Arg Glu Ile Ala Asp
   85 90 95
   cgg ctg aac ttc gcg ccg agc acc ctc gcc ttc atc gac gac cag ccc 336
   Arg Leu Asn Phe Ala Pro Ser Thr Leu Ala Phe Ile Asp Asp Gln Pro
   100 105 110
   ttc gaa cgg gcc gag gtc acc cac gaa ctg ccg gag gtc cgc acc tac 384
   Phe Glu Arg Ala Glu Val Thr His Glu Leu Pro Glu Val Arg Thr Tyr
   115 120 125
   gcc gcc gag cag gcg acc cgg ctg acc ggc ctc ccg gag ttc agc ccg 432
   Ala Ala Glu Gln Ala Thr Arg Leu Thr Gly Leu Pro Glu Phe Ser Pro
   130 135 140
   ggc acc gtc acc gtc gac tcg acc cgc cgc cgc tcc atg tac cag gcg 480
   Gly Thr Val Thr Val Asp Ser Thr Arg Arg Arg Ser Met Tyr Gln Ala
   145 150 155 160
   tcc ttc cgc cgg gac gcc gag cgg tcc gac ttc acc gga ccc gac gcg 528
   Ser Phe Arg Arg Asp Ala Glu Arg Ser Asp Phe Thr Gly Pro Asp Ala
   165 170 175
   gac ttc ctg cgc tcg ctg gac atc cgg atg cgg atc agc cgc gcc acg 576
   Asp Phe Leu Arg Ser Leu Asp Ile Arg Met Arg Ile Ser Arg Ala Thr
   180 185 190
   ccc ctg gag ctg tcg cgg gtc gag gaa ctg acc ctg cgc acc agc cag 624
   Pro Leu Glu Leu Ser Arg Val Glu Glu Leu Thr Leu Arg Thr Ser Gln
   195 200 205
   atg aac gcc acc gga gtg cac tac tcc gag gac gag ctg cgc gcc ctc 672
   Met Asn Ala Thr Gly Val His Tyr Ser Glu Asp Glu Leu Arg Ala Leu
   210 215 220
   atc gac gac ccc gac cac gag gtg ctg gtc acc acc gtc acc gat cgc 720
   Ile Asp Asp Pro Asp His Glu Val Leu Val Thr Thr Val Thr Asp Arg
   225 230 235 240
   ttc ggt ccc tac ggc gcg gtc ggc gtc gtg ctg ctg cgg cgc ggc ccc 768
   Phe Gly Pro Tyr Gly Ala Val Gly Val Val Leu Leu Arg Arg Gly Pro
   245 250 255
   gag gcc tgg cgg atc aag ctg ctg gcc acc tcg tgc cgg gtg gtg tcg 816
   Glu Ala Trp Arg Ile Lys Leu Leu Ala Thr Ser Cys Arg Val Val Ser
   260 265 270
   ctg ggg gcc ggc acg gtg atc ctg cgc tgg ctg acg gac cag gcg cac 864
   Leu Gly Ala Gly Thr Val Ile Leu Arg Trp Leu Thr Asp Gln Ala His
   275 280 285
   cgg gcc ggg gtg cac ctc gga gcc gac ttc cgg gcc acc gaa cgc aac 912
   Arg Ala Gly Val His Leu Gly Ala Asp Phe Arg Ala Thr Glu Arg Asn
   290 295 300
   cgg atg atg gaa gtg gcc tac cgc ttc gcc gga ttc acc gac gat ccc 960
   Arg Met Met Glu Val Ala Tyr Arg Phe Ala Gly Phe Thr Asp Asp Pro
   305 310 315 320
   tgc ccg tgc cag gac gcc tcc gcc gcc acc ggc gcg atc ggc cgc ctg 1008
   Cys Pro Cys Gln Asp Ala Ser Ala Ala Thr Gly Ala Ile Gly Arg Leu
   325 330 335
   cac ctg gtg ccg tcc ccc cgg ccc gcc ccc gac acc ctc cgt ctg gaa 1056
   His Leu Val Pro Ser Pro Arg Pro Ala Pro Asp Thr Leu Arg Leu Glu
   340 345 350
   gct ccc gac ctg gcc acg ggc cgg cgg cgc ccc ggc cag gat ccc gaa      1104
   Ala Pro Asp Leu Ala Thr Gly Arg Arg Arg Pro Gly Gln Asp Pro Glu
           355                 360                 365
   agg acc cca tga                                                      1116
   Arg Thr Pro
       370
<210> 79
   <211> 371
   <212> PRT
   <213> Streptomyces ambofaciens
<400> 79
   Val His Arg Asp Asn Ala Ala Glu Pro Leu Val Lys Cys Leu Val Trp
   1               5                   10                  15
   Asp Leu Asp Asn Thr Leu Trp Gln Gly Thr Leu Leu Glu Glu Asp Glu
               20                  25                  30
   Val Arg Leu Ala Pro Asp Val Leu Arg Thr Ile Thr Glu Leu Asp Ala
           35                  40                  45
   Arg Gly Ile Leu Gln Ala Val Ala Ser Lys Asn Asp His Asp His Ala
       50                  55                  60
   Trp Ala Lys Leu Glu Gln Leu Gly Val Ala Glu Tyr Phe Val Leu Pro
   65                  70                  75                  80
   Arg Ile Gly Trp Gly Pro Lys Ser Lys Ser Val Arg Glu Ile Ala Asp
                   85                  90                  95
   Arg Leu Asn Phe Ala Pro Ser Thr Leu Ala Phe Ile Asp Asp Gln Pro
               100                 105                 110
   Phe Glu Arg Ala Glu Val Thr His Glu Leu Pro Glu Val Arg Thr Tyr
           115                 120                 125
   Ala Ala Glu Gln Ala Thr Arg Leu Thr Gly Leu Pro Glu Phe Ser Pro
       130                 135                 140
   Gly Thr Val Thr Val Asp Ser Thr Arg Arg Arg Ser Met Tyr Gln Ala
   145                 150                 155                 160
   Ser Phe Arg Arg Asp Ala Glu Arg Ser Asp Phe Thr Gly Pro Asp Ala
                   165                 170                 175
   Asp Phe Leu Arg Ser Leu Asp Ile Arg Met Arg Ile Ser Arg Ala Thr
               180                 185                 190
   Pro Leu Glu Leu Ser Arg Val Glu Glu Leu Thr Leu Arg Thr Ser Gln
           195                 200                 205
   Met Asn Ala Thr Gly Val His Tyr Ser Glu Asp Glu Leu Arg Ala Leu
   210 215 220
   Ile Asp Asp Pro Asp His Glu Val Leu Val Thr Thr Val Thr Asp Arg
   225 230 235 240
   Phe Gly Pro Tyr Gly Ala Val Gly Val Val Leu Leu Arg Arg Gly Pro
   245 250 255
   Glu Ala Trp Arg Ile Lys Leu Leu Ala Thr Ser Cys Arg Val Val Ser
   260 265 270
   Leu Gly Ala Gly Thr Val Ile Leu Arg Trp Leu Thr Asp Gln Ala His
   275 280 285
   Arg Ala Gly Val His Leu Gly Ala Asp Phe Arg Ala Thr Glu Arg Asn
   290 295 300
   Arg Met Met Glu Val Ala Tyr Arg Phe Ala Gly Phe Thr Asp Asp Pro
   305 310 315 320
   Cys Pro Cys Gln Asp Ala Ser Ala Ala Thr Gly Ala Ile Gly Arg Leu
   325 330 335
   His Leu Val Pro Ser Pro Arg Pro Ala Pro Asp Thr Leu Arg Leu Glu
   340 345 350
   Ala Pro Asp Leu Ala Thr Gly Arg Arg Arg Pro Gly Gln Asp Pro Glu
   355 360 365
   Arg Thr Pro
   370
<210> 80
   <211> 1122
   <212> DNA
   <213> Streptomyces ambofaciens
<220>
   <221> CDS
   <222> (1)..(1122)
<400> 80
   gtg gct gac acc tcc cac gcc gac ggc acc gag gcc gag gag ctg ttc 48
   Val Ala Asp Thr Ser His Ala Asp Gly Thr Glu Ala Glu Glu Leu Phe
   1 5 10 15
   acc gcg ctg gtc ggc gac cgg gcg gcc gag tgg gac cgg acg ggc gag 96
   Thr Ala Leu Val Gly Asp Arg Ala Ala Glu Trp Asp Arg Thr Gly Glu
   20 25 30
   ctg ccc ctc ggc ctg ctg cac gac ctc ggc tcc cgg ggc ctg ctg tgc 144
   Leu Pro Leu Gly Leu Leu His Asp Leu Gly Ser Arg Gly Leu Leu Cys
   35 40 45
   gca cag gcg ccc gcg gtc cac ggc ggc ctc ggc tgg acc agc cgg cgc      192
   Ala Gln Ala Pro Ala Val His Gly Gly Leu Gly Trp Thr Ser Arg Arg
       50                  55                  60
   aac ggc gaa ctc acc gcg cac gtc ggc gcc ctc tgc agc tcg ttg cgg      240
   Asn Gly Glu Leu Thr Ala His Val Gly Ala Leu Cys Ser Ser Leu Arg
   65                  70                  75                  80
   agc gtc atg acc tcg cag ggc atg gcc gcc tgg acg ctg cgc cgg ctc      288
   Ser Val Met Thr Ser Gln Gly Met Ala Ala Trp Thr Leu Arg Arg Leu
                   85                  90                  95
   gcc gga gcg gac cag cag gcc tcc ctg gtg ccc cgg ttg acc ggc ggc      336
   Ala Gly Ala Asp Gln Gln Ala Ser Leu Val Pro Arg Leu Thr Gly Gly
               100                 105                 110
   gaa ctg gcc gcc gtg gcg ttc acc gag gcc ggc gcc ggc agc gac ctg      384
   Glu Leu Ala Ala Val Ala Phe Thr Glu Ala Gly Ala Gly Ser Asp Leu
           115                 120                 125
   tcc gcc ctg cgc acc cgc atc acc tcc gac ggc gac gaa gtc gtc gtc      432
   Ser Ala Leu Arg Thr Arg Ile Thr Ser Asp Gly Asp Glu Val Val Val
       130                 135                 140
   gac ggc gtc aag gtg tgg gcg acc aac gcc gcg tac gcg gac ctg ctg      480
   Asp Gly Val Lys Val Trp Ala Thr Asn Ala Ala Tyr Ala Asp Leu Leu
   145                 150                 155                 160
   gtg gtc ttc ggc cgc acg gag cag ggc gcg ggc gcc gtc gtg gtg ccc      528
   Val Val Phe Gly Arg Thr Glu Gln Gly Ala Gly Ala Val Val Val Pro
                   165                 170                 175
   gcc tcg gcc ccc ggc gta cgc gtc gag cgc atc acc gac gcg cac ggc      576
   Ala Ser Ala Pro Gly Val Arg Val Glu Arg Ile Thr Asp Ala His Gly
               180                 185                 190
   tgc cgc gcg gcc gga cac gcc gac atc cac ctg gac ggg gtc cgg ctg      624
   Cys Arg Ala Ala Gly His Ala Asp Ile His Leu Asp Gly Val Arg Leu
           195                 200                 205
   ccg gca gac gcc ctg ctc cag ggc cac gac cgc acc ccc gcc ctg ctg      672
   Pro Ala Asp Ala Leu Leu Gln Gly His Asp Arg Thr Pro Ala Leu Leu
       210                 215                 220
   gtc acc acc gcg ctg agc tac ggc cgg atg tcg gtg gcc tgg ggc tcc      720
   Val Thr Thr Ala Leu Ser Tyr Gly Arg Met Ser Val Ala Trp Gly Ser
   225                 230                 235                 240
   ctg ggc atc ctg cgc gcc tgc ctg gcc gcc gcc gca cgt cac gcc ggc      768
   Leu Gly Ile Leu Arg Ala Cys Leu Ala Ala Ala Ala Arg His Ala Gly
                   245                 250                 255
   gga cgg gag cag ttc ggc acg cgg ctc tcg gac cac cag ctc gtc gcc      816
   Gly Arg Glu Gln Phe Gly Thr Arg Leu Ser Asp His Gln Leu Val Ala
               260                 265                 270
   cgg cac ctc gcc gaa ctg ttc gtc gcc gaa cag cac gcc gcc cgg gcg      864
   Arg His Leu Ala Glu Leu Phe Val Ala Glu Gln His Ala Ala Arg Ala
           275                 280                 285
   tgc gag cac gcc agc gcc cag tgg gac gag ggc agc ccc gac atg gtg      912
   Cys Glu His Ala Ser Ala Gln Trp Asp Glu Gly Ser Pro Asp Met Val
       290                 295                 300
   gtc gcc gcc gtc ctg gcc aag cac gtc gcg gcc acc ggc gcc gca cgc      960
   Val Ala Ala Val Leu Ala Lys His Val Ala Ala Thr Gly Ala Ala Arg
   305                 310                 315                 320
   ggg gcc gaa cgg gcc gtg cag gtg ctg gcg tcg gcc ggg gcg cgg gag      1008
   Gly Ala Glu Arg Ala Val Gln Val Leu Ala Ser Ala Gly Ala Arg Glu
                   325                 330                 335
   gga cac gtc gtc gca cgg gcg cac cgc gac gcc aag ctc atg gag atc      1056
   Gly His Val Val Ala Arg Ala His Arg Asp Ala Lys Leu Met Glu Ile
               340                 345                 350
   atc gag ggc agc aac gag atc tgc gaa ctg gtc ctg gcc cgg cac gtg      1104
   Ile Glu Gly Ser Asn Glu Ile Cys Glu Leu Val Leu Ala Arg His Val
           355                 360                 365
   atg tcc gcg gcc ggg tga                                              1122
   Met Ser Ala Ala Gly
       370
<210> 81
   <211> 373
   <212> PRT
   <213> Streptomyces ambofaciens
<400> 81
   Val Ala Asp Thr Ser His Ala Asp Gly Thr Glu Ala Glu Glu Leu Phe
   1               5                   10                  15
   Thr Ala Leu Val Gly Asp Arg Ala Ala Glu Trp Asp Arg Thr Gly Glu
               20                  25                  30
   Leu Pro Leu Gly Leu Leu His Asp Leu Gly Ser Arg Gly Leu Leu Cys
           35                  40                  45
   Ala Gln Ala Pro Ala Val His Gly Gly Leu Gly Trp Thr Ser Arg Arg
       50                  55                  60
   Asn Gly Glu Leu Thr Ala His Val Gly Ala Leu Cys Ser Ser Leu Arg
   65                  70                  75                  80
   Ser Val Met Thr Ser Gln Gly Met Ala Ala Trp Thr Leu Arg Arg Leu
                   85                  90                  95
   Ala Gly Ala Asp Gln Gln Ala Ser Leu Val Pro Arg Leu Thr Gly Gly
               100                 105                 110
   Glu Leu Ala Ala Val Ala Phe Thr Glu Ala Gly Ala Gly Ser Asp Leu
           115                 120                 125
   Ser Ala Leu Arg Thr Arg Ile Thr Ser Asp Gly Asp Glu Val Val Val
       130                 135                 140
   Asp Gly Val Lys Val Trp Ala Thr Asn Ala Ala Tyr Ala Asp Leu Leu
   145                 150                 155                 160
   Val Val Phe Gly Arg Thr Glu Gln Gly Ala Gly Ala Val Val Val Pro
                   165                 170                 175
   Ala Ser Ala Pro Gly Val Arg Val Glu Arg Ile Thr Asp Ala His Gly
   180 185 190
   Cys Arg Ala Ala Gly His Ala Asp Ile His Leu Asp Gly Val Arg Leu
   195 200 205
   Pro Ala Asp Ala Leu Leu Gln Gly His Asp Arg Thr Pro Ala Leu Leu
   210 215 220
   Val Thr Thr Ala Leu Ser Tyr Gly Arg Met Ser Val Ala Trp Gly Ser
   225 230 235 240
   Leu Gly Ile Leu Arg Ala Cys Leu Ala Ala Ala Ala Arg His Ala Gly
   245 250 255
   Gly Arg Glu Gln Phe Gly Thr Arg Leu Ser Asp His Gln Leu Val Ala
   260 265 270
   Arg His Leu Ala Glu Leu Phe Val Ala Glu Gln His Ala Ala Arg Ala
   275 280 285
   Cys Glu His Ala Ser Ala Gln Trp Asp Glu Gly Ser Pro Asp Met Val
   290 295 300
   Val Ala Ala Val Leu Ala Lys His Val Ala Ala Thr Gly Ala Ala Arg
   305 310 315 320
   Gly Ala Glu Arg Ala Val Gln Val Leu Ala Ser Ala Gly Ala Arg Glu
   325 330 335
   Gly His Val Val Ala Arg Ala His Arg Asp Ala Lys Leu Met Glu Ile
   340 345 350
   Ile Glu Gly Ser Asn Glu Ile Cys Glu Leu Val Leu Ala Arg His Val
   355 360 365
   Met Ser Ala Ala Gly
   370
<210> 82
   <211> 312
   <212> DNA
   <213> Streptomyces ambofaciens
<220>
   <221> CDS
   <222> (1)..(312)
<400> 82
   atg acc acg acg tcc ggt cgg ccc gac ccc acc ggc acg acc ccc acc      48
   Met Thr Thr Thr Ser Gly Arg Pro Asp Pro Thr Gly Thr Thr Pro Thr
   1               5                   10                  15
   gcc gac gag gtc gcc ggg gaa ctg ctc ggg ttc ctc gag gac cgc acc      96
   Ala Asp Glu Val Ala Gly Glu Leu Leu Gly Phe Leu Glu Asp Arg Thr
               20                  25                  30
   aag acc acc tgg gag cgc gac cag gac ctg ttc gcc gtc ggc ggg atg      144
   Lys Thr Thr Trp Glu Arg Asp Gln Asp Leu Phe Ala Val Gly Gly Met
           35                  40                  45
   tcc tcg ctg ttc gcc atg cag ctc gtc gtg cac ctg gag aag acc tac      192
   Ser Ser Leu Phe Ala Met Gln Leu Val Val His Leu Glu Lys Thr Tyr
       50                  55                  60
   ggc atc gtc atc agc ggc gcc gac ctg atg ctc gac aac ttc cgc acc      240
   Gly Ile Val Ile Ser Gly Ala Asp Leu Met Leu Asp Asn Phe Arg Thr
   65                  70                  75                  80
   gtc gac gcg atg gtc cgg ctg gtc gga cgg ctg gcc gcg ccc ggc gag      288
   Val Asp Ala Met Val Arg Leu Val Gly Arg Leu Ala Ala Pro Gly Glu
                   85                  90                  95
   gcg acc ggg cac gca ggt ggc tga                                      312
   Ala Thr Gly His Ala Gly Gly
               100
<210> 83
   <211> 103
   <212> PRT
   <213> Streptomyces ambofaciens
<400> 83
   Met Thr Thr Thr Ser Gly Arg Pro Asp Pro Thr Gly Thr Thr Pro Thr
   1               5                   10                  15
   Ala Asp Glu Val Ala Gly Glu Leu Leu Gly Phe Leu Glu Asp Arg Thr
               20                  25                  30
   Lys Thr Thr Trp Glu Arg Asp Gln Asp Leu Phe Ala Val Gly Gly Met
           35                  40                  45
   Ser Ser Leu Phe Ala Met Gln Leu Val Val His Leu Glu Lys Thr Tyr
       50                  55                  60
   Gly Ile Val Ile Ser Gly Ala Asp Leu Met Leu Asp Asn Phe Arg Thr
   65                  70                  75                  80
   Val Asp Ala Met Val Arg Leu Val Gly Arg Leu Ala Ala Pro Gly Glu
                   85                  90                  95
   Ala Thr Gly His Ala Gly Gly
               100
<210> 84
   <211> 867
   <212> DNA
   <213> Streptomyces ambofaciens
<220>
   <221> CDS
   <222> (1)..(867)
<400> 84
   gtg ccc gat tcc aag gaa aat tct ccg ctc gtc gtg ctc ggc gcg ggc      48
   Val Pro Asp Ser Lys Glu Asn Ser Pro Leu Val Val Leu Gly Ala Gly
   1               5                   10                  15
   gtc atg ggc acg gcc atc gcg gca ctc gcc gtc gga cac ggg cac ccg      96
   Val Met Gly Thr Ala Ile Ala Ala Leu Ala Val Gly His Gly His Pro
               20                  25                  30
   gtc aca ctg gtc gac acc tcc gcc ggg gcg cgc gcg gcc gcc ccc gac     144
   Val Thr Leu Val Asp Thr Ser Ala Gly Ala Arg Ala Ala Ala Pro Asp
           35                  40                  45
   gcg gtc gcg ctg cat ctg cgg acg gcc cgg ctg atg ggc gcg ctg ccc     192
   Ala Val Ala Leu His Leu Arg Thr Ala Arg Leu Met Gly Ala Leu Pro
       50                  55                  60
   cac gac cgc ccg ccc ggg gag ctg acc gtc gag gag gcg ccg gcc gcc     240
   His Asp Arg Pro Pro Gly Glu Leu Thr Val Glu Glu Ala Pro Ala Ala
   65                  70                  75                  80
   gtc gcc acc gcg acc gcc gtg atc gag gcc gtc acc gag gac ccc gag     288
   Val Ala Thr Ala Thr Ala Val Ile Glu Ala Val Thr Glu Asp Pro Glu
                   85                  90                  95
   cgg aag gcc gag gtg ctg gcg gac ctg gcg tcc gtg gcg cgc ccg ggg     336
   Arg Lys Ala Glu Val Leu Ala Asp Leu Ala Ser Val Ala Arg Pro Gly
               100                 105                 110
   acg ctg ctc gtc agc aac acc tcg ggc gtc ccc atc gac gag ctg gcc     384
   Thr Leu Leu Val Ser Asn Thr Ser Gly Val Pro Ile Asp Glu Leu Ala
           115                 120                 125
   gac gcc gtc ccc cgc ccc gag gac ctc gtc ggc gtg cac ttc atg aac     432
   Asp Ala Val Pro Arg Pro Glu Asp Leu Val Gly Val His Phe Met Asn
       130                 135                 140
   ccc gcg tac gtg atc ccc acg gtc gag gtg gtc ctc gga ccg cgc agc     480
   Pro Ala Tyr Val Ile Pro Thr Val Glu Val Val Leu Gly Pro Arg Ser
   145                 150                 155                 160
   gga gag gcg gcc gcc cgg gcc acc cgg gac ctg ctg tcc ggc ctg ggc     528
   Gly Glu Ala Ala Ala Arg Ala Thr Arg Asp Leu Leu Ser Gly Leu Gly
                   165                 170                 175
   cgc cgg ggc atc gtc gtc ggc gac ggc gcc ggc ttc gtg acc agc cgc     576
   Arg Arg Gly Ile Val Val Gly Asp Gly Ala Gly Phe Val Thr Ser Arg
               180                 185                 190
   ctg ctg cac cgg atg ctg aac gac gcc atc gcg gtg gtg cac gag ggc     624
   Leu Leu His Arg Met Leu Asn Asp Ala Ile Ala Val Val His Glu Gly
           195                 200                 205
   cgg gcc acc ccg gag acc gtg gac gcg ctg atg cgc gac tgc atc ggc     672
   Arg Ala Thr Pro Glu Thr Val Asp Ala Leu Met Arg Asp Cys Ile Gly
       210                 215                 220
   cac cgc acc gga ccc ctg gcc acg gcc gac ctg atc gga ctg gac aac     720
   His Arg Thr Gly Pro Leu Ala Thr Ala Asp Leu Ile Gly Leu Asp Asn
   225                 230                 235                 240
   ctg gcc gac tcg ctg cgg gtg atg cac gaa cgg acc ggc gat ccg gcg     768
   Leu Ala Asp Ser Leu Arg Val Met His Glu Arg Thr Gly Asp Pro Ala
                   245                 250                 255
   ctc cgc ccg agc gag ctg ctg ctg gac aag gtc cgc cag ggc ctg ctc     816
   Leu Arg Pro Ser Glu Leu Leu Leu Asp Lys Val Arg Gln Gly Leu Leu
                   260                 265                 270
   ggc cgc aag agc ggc cgg gga ttc tac gac tac cag gag gcc acg cga     864
   Gly Arg Lys Ser Gly Arg Gly Phe Tyr Asp Tyr Gln Glu Ala Thr Arg
           275                 280                 285
   tga                                                                 867
<210> 85
   <211> 288
   <212> PRT
   <213> Streptomyces ambofaciens
<400> 85
   Val Pro Asp Ser Lys Glu Asn Ser Pro Leu Val Val Leu Gly Ala Gly
   1               5                   10                  15
   Val Met Gly Thr Ala Ile Ala Ala Leu Ala Val Gly His Gly His Pro
               20                  25                  30
   Val Thr Leu Val Asp Thr Ser Ala Gly Ala Arg Ala Ala Ala Pro Asp
           35                  40                  45
   Ala Val Ala Leu His Leu Arg Thr Ala Arg Leu Met Gly Ala Leu Pro
       50                  55                  60
   His Asp Arg Pro Pro Gly Glu Leu Thr Val Glu Glu Ala Pro Ala Ala
   65                  70                  75                  80
   Val Ala Thr Ala Thr Ala Val Ile Glu Ala Val Thr Glu Asp Pro Glu
                   85                  90                  95
   Arg Lys Ala Glu Val Leu Ala Asp Leu Ala Ser Val Ala Arg Pro Gly
               100                 105                 110
   Thr Leu Leu Val Ser Asn Thr Ser Gly Val Pro Ile Asp Glu Leu Ala
           115                 120                 125
   Asp Ala Val Pro Arg Pro Glu Asp Leu Val Gly Val His Phe Met Asn
       130                 135                 140
   Pro Ala Tyr Val Ile Pro Thr Val Glu Val Val Leu Gly Pro Arg Ser
   145                 150                 155                 160
   Gly Glu Ala Ala Ala Arg Ala Thr Arg Asp Leu Leu Ser Gly Leu Gly
                   165                 170                 175
   Arg Arg Gly Ile Val Val Gly Asp Gly Ala Gly Phe Val Thr Ser Arg
               180                 185                 190
   Leu Leu His Arg Met Leu Asn Asp Ala Ile Ala Val Val His Glu Gly
           195                 200                 205
   Arg Ala Thr Pro Glu Thr Val Asp Ala Leu Met Arg Asp Cys Ile Gly
       210                 215                 220
   His Arg Thr Gly Pro Leu Ala Thr Ala Asp Leu Ile Gly Leu Asp Asn
   225                 230                 235                 240
   Leu Ala Asp Ser Leu Arg Val Met His Glu Arg Thr Gly Asp Pro Ala
                   245                 250                 255
   Leu Arg Pro Ser Glu Leu Leu Leu Asp Lys Val Arg Gln Gly Leu Leu
               260                 265                 270
   Gly Arg Lys Ser Gly Arg Gly Phe Tyr Asp Tyr Gln Glu Ala Thr Arg
           275                 280                 285
<210> 86
   <211> 1040
   <212> DNA
   <213> Streptomyces fradiae
<400> 86
   ggcaggagcc acccgtgaca gggctgccgc ggcccgccgt ccgggtgccg ttccacgatc      60
   tgcgggacgt gcacgcggcc acgggggtgg agtcggagat cggcggcgcg ctgctgcgcg     120
   tcgccgcgcg cgggcgctat ctgctgggtg ccgaactcgc cgcgttcgag gagcggttcg     180
   ccgagtactg cggcaacgcc cactgtgtcg ccgtgggcag cgggctcgac gacgctcgtc     240
   tggcgctgtg ggcgctcggg gtgggcgagg gcgacgaggt gatcgtgccc tcgcacacgt     300
   tcatcgcgtc ctggctcgcg gtgtcggcga cgggtgccac cccggtgccg gtcgagcccg     360
   gtgatcccgg cgagccgggg cccggggcgt tcctgctcga cccggaccgg ctggaggccg     420
   cgctgacccc gcggaccagg gccgtgatgc ccgtgcatct ctacgggcac ccggtggatc     480
   tggacccggt cggggcgttc gcggagccgc acgggctggc cgtcgtggag gacgcggcgc     540
   aggccacggc ccgttaccgc gggaggcgga tcggcagcgg ccaccgcacg gcgttcagct     600
   tctacccggg gaagaacctg ggcgcgctcg gcgacggcgg tgccgtggtc acctcggacc     660
   cggaactcgc ggaccggctg cggctgttgc gcaactacgg cgcccgggag aagtaccggc     720
   acgaggagcg gggcaccaac tcccgcctgg acgagctgca ggcggccgtg ctgtcggtca     780
   agctgccgta cctggacgcc tggaacaccc gccgccggga gatcgccgcc cgctacggcg     840
   aggcgctggc cggtctgccg ggcgtcaccg tgccggaggg ccgcgtggcg gagccggtct     900
   ggcatcagta cgtgctgcgc agcccgtacc gcgaccggtt gcggcggcgg ctggccgagg     960
   cgggggtgga gaccctggtc cactatccgg tggccgtgca cgcgtcgggc gcgtacgcgg    1020
   gcgcggggcc gtgtcccgcc                                                1040
<210> 87
   <211> 388
   <212> PRT
   <213> Streptomyces fradiae
<400> 87
   Met Thr Gly Leu Pro Arg Pro Ala Val Arg Val Pro Phe His Asp Leu
   1               5                   10                  15
   Arg Asp Val His Ala Ala Thr Gly Val Glu Ser Glu Ile Gly Gly Ala
               20                  25                  30
   Leu Leu Arg Val Ala Ala Arg Gly Arg Tyr Leu Leu Gly Ala Glu Leu
           35                  40                  45
   Ala Ala Phe Glu Glu Arg Phe Ala Glu Tyr Cys Gly Asn Ala His Cys
       50                  55                  60
   Val Ala Val Gly Ser Gly Leu Asp Asp Ala Arg Leu Ala Leu Trp Ala
   65                  70                  75                  80
   Leu Gly Val Gly Glu Gly Asp Glu Val Ile Val Pro Ser His Thr Phe
                   85                  90                  95
   Ile Ala Ser Trp Leu Ala Val Ser Ala Thr Gly Ala Thr Pro Val Pro
               100                 105                 110
   Val Glu Pro Gly Asp Pro Gly Glu Pro Gly Pro Gly Ala Phe Leu Leu
           115                 120                 125
   Asp Pro Asp Arg Leu Glu Ala Ala Leu Thr Pro Arg Thr Arg Ala Val
       130                 135                 140
   Met Pro Val His Leu Tyr Gly His Pro Val Asp Leu Asp Pro Val Gly
   145                 150                 155                 160
   Ala Phe Ala Glu Pro His Gly Leu Ala Val Val Glu Asp Ala Ala Gln
                   165                 170                 175
   Ala Thr Ala Arg Tyr Arg Gly Arg Arg Ile Gly Ser Gly His Arg Thr
               180                 185                 190
   Ala Phe Ser Phe Tyr Pro Gly Lys Asn Leu Gly Ala Leu Gly Asp Gly
           195                 200                 205
   Gly Ala Val Val Thr Ser Asp Pro Glu Leu Ala Asp Arg Leu Arg Leu
       210                 215                 220
   Leu Arg Asn Tyr Gly Ala Arg Glu Lys Tyr Arg His Glu Glu Arg Gly
   225                 230                 235                 240
   Thr Asn Ser Arg Leu Asp Glu Leu Gln Ala Ala Val Leu Ser Val Lys
                   245                 250                 255
   Leu Pro Tyr Leu Asp Ala Trp Asn Thr Arg Arg Arg Glu Ile Ala Ala
               260                 265                 270
   Arg Tyr Gly Glu Ala Leu Ala Gly Leu Pro Gly Val Thr Val Pro Glu
   275 280 285
   Gly Arg Val Ala Glu Pro Val Trp His Gln Tyr Val Leu Arg Ser Pro
   290 295 300
   Tyr Arg Asp Arg Leu Arg Arg Arg Leu Ala Glu Ala Gly Val Glu Thr
   305 310 315 320
   Leu Val His Tyr Pro Val Ala Val His Ala Ser Gly Ala Tyr Ala Gly
   325 330 335
   Ala Gly Pro Cys Pro Ala Gly Gly Leu Pro Arg Ala Glu Arg Leu Ala
   340 345 350
   Gly Glu Val Leu Ser Leu Pro Ile Gly Pro His Leu Pro Asp Glu Ala
   355 360 365
   Val Glu Val Val Ile Ala Ala Val Gln Ser Ala Ala Leu Asp Ser Trp
   370 375 380
   Glu Glu Gly Pro
   385
<210> 88
   <211> 271
   <212> PRT
   <213> Streptomyces mycarofaciens
<400> 88
   Met Ser Pro Ile Ser Ala Ser Ala Pro Ala Ala Ser Arg Ser Thr Ala
   1 5 10 15
   Arg Arg Glu Leu Gly Gln Asn Phe Phe Arg Ser Ala Ala Ala Ala Cys
   20 25 30
   Arg Phe Ser Asp Gln Leu Asp Ala Phe Cys Ala Asp Leu Pro Gly Ser
   35 40 45
   Leu Ala Asp Val Leu Thr Val Glu Ile Gly Ala Gly Ser Gly Arg Val
   50 55 60
   Thr Lys Ala Leu Ala Ser Ala Gly Arg Ser Leu Leu Ala Val Glu Ile
   65 70 75 80
   Asp Ala Tyr Trp Ala Arg Arg Leu Thr Ala Glu Ser Leu Pro Asp Val
   85 90 95
   Thr Val Val Asn Glu Asp Phe Leu Asn Leu Gln Leu Pro Arg Gln Pro
   100 105 110
   Ile Arg Leu Ile Gly Asn Leu Pro Phe Val Ser Gly Thr Lys Ile Leu
           115                 120                 125
   Arg Arg Cys Leu Glu Leu Gly Pro Asn Arg Met Cys Gln Ala Val Phe
       130                 135                 140
   Leu Leu Gln Arg Glu Tyr Val Gly Lys Arg Thr Gly Ala Trp Gly Gly
   145                 150                 155                 160
   Asn Leu Phe Asn Ala Gln Trp Glu Pro Trp Tyr Thr Phe Glu Gly Gly
                   165                 170                 175
   Leu Ala Phe Ser Arg Asn Glu Phe Ser Pro Val Pro Arg Ala Asp Thr
               180                 185                 190
   Gln Thr Leu Val Val Met Pro Arg Arg Arg Pro Ser Val Pro Trp Arg
           195                 200                 205
   Glu Arg Thr Asp Tyr Gln Arg Phe Thr Gln Gln Ile Phe Asp Thr Gly
       210                 215                 220
   Gln Met Thr Ile Gly Glu Ala Ala Arg Lys Val Leu Arg Arg Gly His
   225                 230                 235                 240
   Ala Gln Phe Val Arg Ser Ala Gly Val Arg Pro Ala Asp Arg Val Lys
                   245                 250                 255
   Asp Leu Thr Val Arg Asp Trp Ala Ala Leu Phe Arg Ala Asn Pro
               260                 265                 270
<210> 89
   <211> 25
   <212> DNA
   <213> Artificial
<220>
   <223> Oligonucléotide SRMR1
<400> 89
   ctgccagtcc tctcccagca gtacg 25
<210> 90
   <211> 25
   <212> DNA
   <213> Artificial
<220>
   <223> Oligonucléotide SRMR2
<400> 90
   tgaagctgga cgtctcctac gtcgg 25
<210> 91
   <211> 2755
   <212> DNA
   <213> Artificial
<220>
   <223> Cassette excisable
<400> 91
   gatatctacc tcttcgtccc gaagcaactt gggtgggcgc tcgaccttgg gccagtgtgc     60
   ttctcacctg cggcgccagt ccatgggtgc tcgcggttgt ccgccgctgt gcgctggcgt    120
   tgtcacgcag ttagacactc accttcccac gggcggtgtc gtagggctcg gctaagttgc    180
   ccgcatgact gacagggatc cctcgagaag ctttatgctt gtaaaccgtt ttgtgaaaaa    240
   atttttaaaa taaaaaaggg gacctctagg gtccccaatt aattagtaat ataatctatt    300
   aaaggtcatt caaaaggtca tccaccggat caattcccct gctcgcgcag gctgggtgcc    360
   aagctctcgg gtaacatcaa ggcccgatcc ttggagccct tgccctcccg cacgatgatc    420
   gtgccgtgat cgaaatccag atccttgacc cgcagttgca aaccctcact gatccgtaat    480
   gtgagttagc tcactcatta ggcaccccag gctttacact ttatgcttcc ggctcgtatg    540
   ttgtgtggaa ttgtgagcgg ataacaattt cacacaggaa acagctatga ccatgattac    600
   gccaagctgc ggtggcgtac accgtcgcct cggtcggccc gtagagattg gcgatcccga    660
   ccgcagcacc accgagaacg tccccgacgt ggccgaccag cccgtcatcg tcaacgcctg    720
   accgcggtgc ggacaggccg tgtcgcgacc ggccgtgcgg aattaagccg gcccgtaccc    780
   tgtgaataga ggtccgctgt gacacaagaa tccctgttac ttctcgaccg tattgattcg    840
   gatgattcct acgcgagcct gcggaacgac caggaattct gggagccgct ggcccgccga    900
   gccctggagg agctcgggct gccggtgccg ccggtgctgc gggtgcccgg cgagagcacc    960
   aaccccgtac tggtcggcga gcccgacccg gtgatcaagc tgttcggcga gcactggtgc   1020
   ggtccggaga gcctcgcgtc ggagtcggag gcgtacgcgg tcctggcgga cgccccggtg   1080
   ccggtgcccc gcctcctcgg ccgcggcgag ctgcggcccg gcaccggagc ctggccgtgg   1140
   ccctacctgg tgatgagccg gatgaccggc accacctggc ggtccgcgat ggacggcacg   1200
   accgaccgga acgcgctgct cgccctggcc cgcgaactcg gccgggtgct cggccggctg   1260
   cacagggtgc cgctgaccgg gaacaccgtg ctcacccccc attccgaggt cttcccggaa   1320
   ctgctgcggg aacgccgcgc ggcgaccgtc gaggaccacc gcgggtgggg ctacctctcg   1380
   ccccggctgc tggaccgcct ggaggactgg ctgccggacg tggacacgct gctggccggc   1440
   cgcgaacccc ggttcgtcca cggcgacctg cacgggacca acatcttcgt ggacctggcc   1500
   gcgaccgagg tcaccgggat cgtcgacttc accgacgtct atgcgggaga ctcccgctac   1560
   agcctggtgc aactgcatct caacgccttc cggggcgacc gcgagatcct ggccgcgctg   1620
   ctcgacgggg cgcagtggaa gcggaccgag gacttcgccc gcgaactgct cgccttcacc   1680
   ttcctgcacg acttcgaggt gttcgaggag accccgctgg atctctccgg cttcaccgat   1740
   ccggaggaac tggcgcagtt cctctggggg ccgccggaca ccgcccccgg cgcctgacgc   1800
   cccgggccgc ccggcgccgc ccccggcccc cggcggccgc ccggagcccc gcccgcgctc   1860
   gggagccccg ggcccgcgcc gaagcccgct gctgcgagcc cggagcgggc cggccgacgg 1920
   cggtgcgggc ccggcggcgg acgctcagca gcggcgggcg tgaaaggccc tggcatcctc 1980
   gatcatctcc tccagggtgg tcggccgagc ttccatccca gctcggcaag gatcgatccg 2040
   cgcagatcag ttggaagaat ttgtccacta cgtgaaaggc gagatcacca aggtagtcgg 2100
   caaataatgt ctaacaattc gttcaagccg acgccgcttc gcggcgcggc ttaactcaag 2160
   cgttagatgc actaagcaca taattgctca cagccaaact atcaggtcaa gtctgctttt 2220
   attattttta agcgtgcata ataagcccta cacaaattgg gagatatatc atgaaaggct 2280
   ggctttttct tgttatcgca atagttggcg aagtaatcgc aacatccgca ttaaaatcta 2340
   gcgagggctt tactaagctg atccggtgga tgaccttttg aatgaccttt aatagattat 2400
   attactaatt aattggggac cctagaggtc ccctttttta ttttaaaaat tttttcacaa 2460
   aacggtttac aagcataaag cttgttaaca gatctcccgg ctcgtcggac actccggtac 2520
   ggccgtgact gaggaggtct accggaagca gatccggccc gtcatccaga ccggcgctgt 2580
   ggtcatggac ggcatcttca agcggggtcc ggcgcgatag tcacgcagat agacacgcac 2640
   agaaaacagg tgaggcagac cgtaacgtta cggtctgcct cacctggtgt ttctctgtcg 2700
   gggtggcggg atttgaaccc acgacctctt cgtcccgaac gaagcgcgcg atatc 2755
<210> 92
   <211> 2208
   <212> DNA
   <213> Artificial
<220>
   <223> Cassette excisable
<400> 92
   gatatcgcgc gcgcttcgtt cgggacgaag aggtcgtggg ttcaaatccc gccaccccga      60
   cagagaaaca ccaggtgagg cagaccgtaa cgttacggtc tgcctcacct gttttctgtg     120
   cgtgtctatc tgcgtgacta tcgcgccgga ccccgcttga agatgccgtc catgaccaca     180
   gcgccggtct ggatgacggg ccggatctgc ttccggtaga cctcctcagt cacggccgta     240
   ccggagtgtc cgacgagccg ggagatctgt taacaagctt tatgcttgta aaccgttttg     300
   tgaaaaaatt tttaaaataa aaaaggggac ctctagggtc cccaattaat tagtaatata     360
   atctattaaa ggtcattcaa aaggtcatcc accggatcaa ttcccctgct cgcgcaggct     420
   gggtgccaag ctctcgggta acatcaaggc ccgatccttg gagcccttgc cctcccgcac     480
   gatgatcgtg ccgtgatcga aatccagatc cttgacccgc agttgcaaac cctcactgat     540
   ccggctcacg gtaactgatg ccgtatttgc agtaccagcg tacggcccac agaatgatgt     600
   cacgctgaaa atgccggcct ttgaatgggt tcatgtgcag ctccatcagc aaaaggggat     660
   gataagttta tcaccaccga ctatttgcaa cagtgccgtt gatcgtgcta tgatcgactg     720
   atgtcatcag cggtggagtg caatgtcgtg caatacgaat ggcgaaaagc cgagctcatc     780
   ggtcagcttc tcaaccttgg ggttaccccc ggcggtgtgc tgctggtcca cagctccttc     840
   cgtagcgtcc ggcccctcga agatgggcca cttggactga tcgaggccct gcgtgctgcg 900
   ctgggtccgg gagggacgct cgtcatgccc tcgtggtcag gtctggacga cgagccgttc 960
   gatcctgcca cgtcgcccgt tacaccggac cttggagttg tctctgacac attctggcgc 1020
   ctgccaaatg taaagcgcag cgcccatcca tttgcctttg cggcagcggg gccacaggca 1080
   gagcagatca tctctgatcc attgcccctg ccacctcact cgcctgcaag cccggtcgcc 1140
   cgtgtccatg aactcgatgg gcaggtactt ctcctcggcg tgggacacga tgccaacacg 1200
   acgctgcatc ttgccgagtt gatggcaaag gttccctatg gggtgccgag acactgcacc 1260
   attcttcagg atggcaagtt ggtacgcgtc gattatctcg agaatgacca ctgctgtgag 1320
   cgctttgcct tggcggacag gtggctcaag gagaagagcc ttcagaagga aggtccagtc 1380
   ggtcatgcct ttgctcggtt gatccgctcc cgcgacattg tggcgacagc cctgggtcaa 1440
   ctgggccgag atccgttgat cttcctgcat ccgccagagg gcgggatgcg aagaatgcga 1500
   tgccgctcgc cagtcgattg gctgagctca tgagcggaga acgagatgac gttggagggg 1560
   caaggtcgcg ctgattgctg gggcaacacg tgaaaggcga gatcaccaag gtagtcggca 1620
   aataatgtct aacaattcgt tcaagccgac gccgcttcgc ggcgcggctt aactcaagcg 1680
   ttagatgcac taagcacata attgctcaca gccaaactat caggtcaagt ctgcttttat 1740
   tatttttaag cgtgcataat aagccctaca caaattggga gatatatcat gaaaggctgg 1800
   ctttttcttg ttatcgcaat agttggcgaa gtaatcgcaa catccgcatt aaaatctagc 1860
   gagggcttta ctaagctgat ccggtggatg accttttgaa tgacctttaa tagattatat 1920
   tactaattaa ttggggaccc tagaggtccc cttttttatt ttaaaaattt tttcacaaaa 1980
   cggtttacaa gcataaagct tctcgaggga tccctgtcag tcatgcgggc aacttagccg 2040
   agccctacga caccgcccgt gggaaggtga gtgtctaact gcgtgacaac gccagcgcac 2100
   agcggcggac aaccgcgagc acccatggac tggcgccgca ggtgagaagc acactggccc 2160
   aaggtcgagc gcccacccaa gttgcttcgg gacgaagagg cagatatc 2208
<210> 93
   <211> 60
   <212> DNA
   <213> Artificial
<220>
   <223> oligonucléotide ORF2A
<400> 93
   cccgcgcggc agcctctccg tgatcgagtc cggcgtgacc atcgcgcgcg cttcgttcgg 60
<210> 94
   <211> 60
   <212> DNA
   <213> Artificial
<220>
   <223> Oligonucléotide ORF2B
<400> 94
   gctccgtgcg tcatgcagga aggtgtcgta gtcgcggtag atctgcctct tcgtcccgaa 60
<210> 95
   <211> 35
   <212> DNA
   <213> Artificial
<220>
   <223> Cicatrice att3
<400> 95
   atcgcgcgcg cttcgttcgg gacgaagagg tagat 35
<210> 96
   <211> 59
   <212> DNA
   <213> Artificial
<220>
   <223> Oligonucléotide EDR8
<400> 96
   cgggatgatc gcttgtccgg cggccggatg cctagcctca tcgcgcgcgc ttcgttcgg 59
<210> 97
   <211> 60
   <212> DNA
   <213> Artificial
<220>
   <223> oligonucléotide EDR9
<400> 97
   cccgatccag aacgtctggt cggtgatcag gtcgctgttc atctgcctct tcgtcccgaa 60
<210> 98
   <211> 60
   <212> DNA
   <213> Artificial
<220>
   <223> oligonucléotide EDR3
<400> 98
   accggggcgg tcctcccctc cggggcgtca cggccgcgga atctgcctct tcgtcccgaa 60
<210> 99
   <211> 60
   <212> DNA
   <213> Artificial
<220>
   <223> oligonucléotide EDR4
<400> 99
   cacgcagcga gccgacgcac tgatggacga cacgatggcc atcgcgcgcg cttcgttcgg 60
<210> 100
   <211> 60
   <212> DNA
   <213> Artificial
<220>
   <223> oligonucléotide EDR5
<400> 100
   gggcgtgaag cgggcgagtg tggatgtcat gcgagtactc atcgcgcgcg cttcgttcgg 60
<210> 101
   <211> 60
   <212> DNA
   <213> Artificial
<220>
   <223> oligonucléotide EDR6
<400> 101
   cgggaaacgg cgtcgcactc ctcgggggcc gcgtcagccc atctgcctct tcgtcccgaa 60
<210> 102
   <211> 26
   <212> DNA
   <213> Artificial
<220>
   <223> oligonucléotide C9583
<400> 102
   ctgcaggtgc tccagcgcgt cgatct 26
<210> 103
   <211> 26
   <212> DNA
   <213> Artificial
<220>
   <223> oligonucléotide C9584
<400> 103
   ctgcagacgg aggcggacct gcggct 26
<210> 104
   <211> 33
   <212> DNA
   <213> Artificial
<220>
   <223> Cicatrice att1
<400> 104
   atcgcgcgct tcgttcggga cgaagaggta gat 33
<210> 105
   <211> 34
   <212> DNA
   <213> Artificial
<220>
   <223> cicatrice att2
<400> 105
   atcggcgcgc ttcgttcggg acgaagaggt agat 34
<210> 106
   <211> 10325
   <212> DNA
   <213> Streptomyces ambofaciens
<220>
   <221> misc_feature
   <222> (3620)..(4069)
   <223> n is a, c, g, or t
<400> 106
   ctgcagaggg cgccgacgtg cgcggtgagt tcgccgttgc gccggctggt ccagccgagg 60
   ccgccgtgga ccgcgggcgc ctgtgcgcac agcaggcccc gggagccgag gtcgtgcagc 120
   aggccgaggg gcagctcgcc cgtccggtcc cactcggccg cccggtcgcc gaccagcgcg 180
   gtgaacagct cctcggcctc ggtgccgtcg gcgtgggagg tgtcagccac ctgcgtgccc 240
   ggtcgcctcg ccgggcgcgg ccagccgtcc gaccagccgg accatcgcgt cgacggtgcg 300
   gaagttgtcg agcatcaggt cggcgccgct gatgacgatg ccgtaggtct tctccaggtg 360
   cacgacgagc tgcatggcga acagcgagga catcccgccg acggcgaaca ggtcctggtc 420
   gcgctcccag gtggtcttgg tgcggtcctc gaggaacccg agcagttccc cggcgacctc 480
   gtcggcggtg ggggtcgtgc cggtggggtc gggccgaccg gacgtcgtgg tcatcgcgtg 540
   gcctcctggt agtcgtagaa tccccggccg ctcttgcggc cgagcaggcc ctggcggacc 600
   ttgtccagca gcagctcgct cgggcggagc gccggatcgc cggtccgttc gtgcatcacc 660
   cgcagcgagt cggccaggtt gtccagtccg atcaggtcgg ccgtggccag gggtccggtg 720
   cggtggccga tgcagtcgcg catcagcgcg tccacggtct ccggggtggc ccggccctcg 780
   tgcaccaccg cgatggcgtc gttcagcatc cggtgcagca ggcggctggt cacgaagccg 840
   gcgccgtcgc cgacgacgat gccccggcgg cccaggccgg acagcaggtc ccgggtggcc 900
   cgggcggccg cctctccgct gcgcggtccg aggaccacct cgaccgtggg gatcacgtac 960
   gcggggttca tgaagtgcac gccgacgagg tcctcggggc gggggacggc gtcggccagc 1020
   tcgtcgatgg ggacgcccga ggtgttgctg acgagcagcg tccccgggcg cgccacggac 1080
   gccaggtccg ccagcacctc ggccttccgc tcggggtcct cggtgacggc ctcgatcacg 1140
   gcggtcgcgg tggcgacggc ggccggcgcc tcctcgacgg tcagctcccc gggcgggcgg 1200
   tcgtggggca gcgcgcccat cagccgggcc gtccgcagat gcagcgcgac cgcgtcgggg 1260
   gcggccgcgc gcgccccggc ggaggtgtcg accagtgtga ccgggtgccc gtgtccgacg 1320
   gcgagtgccg cgatggccgt gcccatgacg cccgcgccga gcacgacgag cggagaattt 1380
   tccttggaat cgggcacgga tcctccagaa tcccgggggc ggcggctgtg cgaaatgctg 1440
   tcccgaatcg cttccgcgct ccatcacccg ggcgtgactt tgtcacctag caagcggtgg 1500
   gaggccgggc ggaggctatg tcatctcggc cttgtccgct tacgagttcc gtccttaaag 1560
   tgcgcccggc gaacgcacgg cggaattgac tcgccgtccg tcaaggaccc gcggttcgaa 1620
   ggacctcgac gcatgaaggg tttcacatgg ctcatatcgc attcttcatc cttccggttg 1680
   ccgggcatgt gaatccgacc ctgggagtcg ccgaggaact ggtcgcgcgc ggccaccggg 1740
   tgacgttcgc gctgtccgag gacctcgccg agcgggcccg gctgatcggc gccgaggtgg 1800
   tcacctatcc ggtggacagg caacggttcc tggaccagat ggtgccgcgg caggacgcgg 1860
   acgagtacac ggacgaggac gagttcgtcc gggtcctgga gtggctgctg gacatgacgg 1920
   tgcagaccat ggaaccgctg gagaggcact tcgccgggga ccggcccgac gtcgtcgtca 1980
   acgatccgtc gtcgctgtgg acgggacggc tgctggcgga ccggtggggc atcccggtca 2040
   tccgcagcac tccgacctat gccgccaacg aacactggtc gctgcatccg ccggtcgact 2100
   cggccgagcc gccggacgac cccgagctgc acaagctgct cgcgcggatc gagcggctgc 2160
   tggaggagca gggcgtcgag cacgacctgg ccggcttcac cggggtcctg cacggcggtc 2220
   cggccctgct gtacatgccc cgctcgttcc agtacgcggg cgagaccttc gacgcacagc 2280
   accacttcgt cggcccctgc ccgccccgca ccgcgttcca cggcgagtgg aggccggggg 2340
   acgacgacgg ccggcccctg gtgctggtga gtctcggaac cctgtacaac gaccggccgg 2400
   acttcttccg cacctgcctg gaggcgttcg gcgacgagcc ctggaacgtg cttctggtgc 2460
   tgggcggcgg ggtgcccgcg gccgacctgg gcccgcttcc cggcaatgtc cgggtgaccg 2520
   acttcgtgtc gctgcgcgac gtcctgccgc acacggcggt ggtggtgaac cacggtggga 2580
   tgagcaccgc catggaggtg ttctcgcacg gtgtgccggt ggtggcgatc ccggtgatgc 2640
   cggagccccg ggccaccgcg cggcggatcg tcgaactggg cctgggcgac cagctgctga 2700
   actcggagct gacggccgag tccctgcgtg ccacggtacg gcgggtgctg gcggactccg 2760
   cgatcccggg gaacatgcgc gggttccggg agcagatcag ggcggccggc ggggcgcccg 2820
   cggcggccga cgcgatcgag ggactgctgc cccgggtggg ctgagacgtc cgcgcccgac 2880
   acgcgttcac cttccgaacg gcgggatcgc cccatgctca tcaccgaaac agcagtgccc 2940
   gacgtgttcc gcatcgatcc ggaaccgctg ccggaccacc ggggccggtt ctacgaagcg 3000
   gtgcgccgcg ggccgctgga ggccgccgtc gggcactcgg tcgaggtccg gcaggtccac 3060
   tgcaccgtct ccgggcgcaa cgtactgcgc ggcctgcacg ccaccaccct gccgccgggc 3120
   caggccaaga tcctcacctg tgtgcggggt gcggcgctga ctatggtggt cgacatgagg 3180
   gtcgggtcac cgggcttcgg acggtacgag gcggtgcggc aggatgcccc gtcgggcacc 3240
   gcgctctacc tgccggacgg catcggcctg ggctacgtgg ccctcgcgga cgacacatgc 3300
   atgaactacc tgtgcaccga ggagtacgtc ccgggcatgg tcatcgacgt ggacgccctg 3360
   gaccccgaac tcggcctgcc gtggggactg accggggatc ccgtccgctc cgcccgggac 3420
   gcggcggcgc cgtccctgcg ggcggccgcg gcggcgggaa ttctgcccac gtacgaggac 3480
   tgcttacggg tgcgcacgtc cgtgcccgcc cctcccgacc ggactcggct ctgacgcgac 3540
   ggacacgacc gccgcgcatc cgacgcgaat ccgacgcgaa tccgaactcg atttcccgaa 3600
   ccgtggacgg agcgcgtccn nnnnnnnnnn nnnnnnnnnn nnnnnnnnnn nnnnnnnnnn 3660
   nnnnnnnnnn nnnnnnnnnn nnnnnnnnnn nnnnnnnnnn nnnnnnnnnn nnnnnnnnnn 3720
   nnnnnnnnnn nnnnnnnnnn nnnnnnnnnn nnnnnnnnnn nnnnnnnnnn nnnnnnnnnn 3780
   nnnnnnnnnn nnnnnnnnnn nnnnnnnnnn nnnnnnnnnn nnnnnnnnnn nnnnnnnnnn 3840
   nnnnnnnnnn nnnnnnnnnn nnnnnnnnnn nnnnnnnnnn nnnnnnnnnn nnnnnnnnnn 3900
   nnnnnnnnnn nnnnnnnnnn nnnnnnnnnn nnnnnnnnnn nnnnnnnnnn nnnnnnnnnn 3960
   nnnnnnnnnn nnnnnnnnnn nnnnnnnnnn nnnnnnnnnn nnnnnnnnnn nnnnnnnnnn 4020
   nnnnnnnnnn nnnnnnnnnn nnnnnnnnnn nnnnnnnnnn nnnnnnnnnc cgccacaccc 4080
   cccagcagcg tcagcgtccg cctcgggacc acacccggca ggacgacggg gtgcggggcg 4140
   acgaggctga gggagccccg caactccacg gggcggctca accgcttgag ctgctccatc 4200
   aactgctggg ccgaggtggt ccccgtgtgg tggtccaccg ggccgcggcc gctcgccgag 4260
   gccagccgga ggttcccgcc cacccgcacc atgaagggca ggcccgcgag accgagccct 4320
   cgcaccagtc gtggcagcac ggccgcgcgt gcgtccatca ccacggggcg ggccaaggag 4380
   cggttggcct gagcggcttc ggccaccagg cggacgatgt tctcctcctc accgggtgcg 4440
   ccggggtgcc ggggtgtgcc ctccccgctc cggccgccgc ccagcgtcag gcgccagctg 4500
   accggggcgg ccgtcgtgcc cgaggccagc cacagcccgt gactctgctg gcagctcacc 4560
   acccggccca ggtcggagac gaagcgccgg ctcaccccga cggagcgcac ccccgccttg 4620
   gagaccacca tcggccagat cacccaggcg tcgggggtca gcctgtcgtc cacgtagcgg 4680
   gccagcgcgg cccgcacctc gccccagtcc caggtcgacc cggcgacgaa gtggtgcagg 4740
   ctctgttccg ccgcgccctc tccgacgaag gcggcgaggt tccgggcggt cttgcgtccc 4800
   tgtgccgtga gcagcccgcg tatgtactgc tcacctcttc tgcgctggtc cgcccggcgg 4860
   agcgaaccga gtaactccgc acatgcgtcg gagacgagcg agtcgaagtc gtgccgcgcg 4920
   gcgggaccca cggggggcgc cgcgccggcg ggtcgaagcg tgcgtaaggt cataggagtc 4980
   ctcgtggggg cctcgtcatc actgcgagtg gactgcgacc agcatcgcca attcacccgt 5040
   tgctccccag gtacaccggg cacactcgtt ccgcttcgct cccccggcgg cacggcccgc 5100
   gtgtggagca ctccccgctc tccggacggt gggggggaag cccaccgcac gcgcgccggt 5160
   agcggtgccg gggccccaca acccctcacc gacggggtcc gttcgaccac atgccgtgat 5220
   gatgccttcg ccgcacggcc tccttgaatg acggaccgtc agaaagacgt cactccgcgc 5280
   gcgcccgctc ccgccccgac cccgcccggc cgccgtcccg cccccgcgac cctgtgggcc 5340
   gacgccctct tgacggaccc ggaatcccgg gccgcgggac gggacggcga ggaactcgat 5400
   gccgcaaagg ttccgcgtcc tcaccggcac ctccgagatc cagcgcaacg gcatcgccaa 5460
   gctcctggct ttccaccact gagccgcacc ccctggcgag cagatccggg acaagacgcc 5520
   accttcgcga cagtgtttag gaaaagttaa gtaaagaatt ccgcgagcgg attgccaggg 5580
   agaacaaccc attgacgcgc accggtgcag cggccacatt gacggcacct gtcaagttca 5640
   cccccaggag cttggaatcc catgcaggca attcggcatc acgtcatgct cgtcatggcc 5700
   ttcgtgaccg tcgccacgac tttccttctc tggccgtcga cgcaatccgc gcaagcgttt 5760
   cccccgaccc cgaagcagac ggtactgaac cacctccgcg ccatttccgg gaatcacatc 5820
   gtctccggac agcacaacaa ggagcccgcc tccgccccgg gccagtacac ccagcaggtc 5880
   aaggacgtca ccgggcagta ccccggcctg tggggcggtg acctgatgtt cgccgcggcg 5940
   gacgtggccg gccgccagcg cgtcgtcgac caggccagga ccgagtgggc gaacggatcg 6000
   ctggtctcgc tcacctggca cgtctgcccg ccgaccggcg gcagcacctg tgcgttcgag 6060
   ggcggcgtca agtccacgct gacgaacgcg cagttctcgc aggtcctcac ggagggcagt 6120
   gccctgaaca gcgcatggaa gcggcgcctg gacgaggtcg tcccgtacct gcagcagctg 6180
   gagaacgcgg gcgtccccgt cctcttccgg ccgctgcacg agatgaacga atcctggaac 6240
   tggtggggaa accggcccgg agcgaacggc agcgcacgcc tctaccagat cacccgcgat 6300
   cacctcgccg ggacgaaagg gctggacaat ctgatctggg tctggaacgt ccaggacaat 6360
   ccggcgggaa actggaacag ctactatccg ggagatcagt acgtggacgt cgtttcgctg 6420
   gacgtctggt acaagagcca cccgagttcc gccgactacc agcagatgcg gagcatcgcg 6480
   ggaacaaaac ccatggccct cgcggagctg ggcaaaatgc cgaccgccgc gctgctggac 6540
   agccagacgc ggtggacatg gttcatgatg tggtccgagc atctgcgcgg gaacaattcc 6600
   aacgccgaaa tacagacggc gtatttccac ccccgtgtac tgaaccaggg ggaggtcgca 6660
   ctgccctgac gctcggcgct gcccggctct ctcacgcgcg ttctgacagg acgtcgcgga 6720
   gagtgcgggg caagcggccg gtgagctggg cgcagtcgtc gcggacccga tcccagcgct 6780
   gttcgcgtac cgaggcgaac agggaggaga aggcgtagag ccaccaggga tccaggccgg 6840
   tcgccgctgt ttcggcagag tacgtgtcgg ccgggatgtc cacgtaggcg accggtgtcc 6900
   gccacacctc cgccgccgtg gaggcgatgc ctgccaggtc cgtcgactcg ggtcccgtga 6960
   tgtcgtggtg acggccggtc ggcccgccca ccgcgagggc ggcgagggcg cgggccacgt 7020
   cgtcgcgcgc caccagggac acccggccat cggctgccgg cagcctcagc agcccggtcg 7080
   agcgcgcctg ggtgagccag cctaggaaga actcgatgta cagcgaggcc ctggcgaacg 7140
   agcacggcac gccggaggcg agcagcagat cctcggtgag ccggttgacg acggcgtagc 7200
   agaacgggga ggccgagtcg gcgtcgacgc tgctcagtgc cgcgacatgg ccgacgcgct 7260
   cggccaccac ggcggcgacg acgttgcggt ggtgcagcag cacccgtgcg tcgggcccgt 7320
   cgctggagac gaggaccaga gtgtccacgc ccttcagcgc cgcacgcaga gcgggcgggt 7380
   cggcgtagtc ggcgacggcg cactccaccc ccggcggtag tgcctcggca ggcagcttcc 7440
   gcctggtcat cgccacgacg tccacgtcgg cccggtccgc gagcagctgc accacccgcc 7500
   tgcccagact gcccgccgcc cctgtcaccg cgatacgcat cgtcgccccc gtcgccttgt 7560
   cgtcggtcgt accaccgtag gggccaaccc gcgaccaggg cttggaacga gccggcccgc 7620
   cagggcacag acgcgcgatc ggtccggttt tcccgtgctc ttttggaccg ggacgccgga 7680
   ccgcttcctt tctacggtgg agccgttccc gcccgagccc gcacgtcatc gacgtgcggg 7740
   gaagacagag gtgataccga tgctccgacg ccgtctcgga gggccgtcag gccccctcgt 7800
   cagtgccctg tgcctgggcg cgatgccctt cggcaccacc gtcgacgaga agacgtcctt 7860
   cgccatcctc gaccggttcg tcgaggccgg cggcagtctc gtcgacaccg ccgacaacta 7920
   cgcgttctgg gctcccggcg ggaccgggga cgagagcgag aacaccgtcg ggcgctggct 7980
   ggcgagccgc cgccgccgcg acgaggtggt gatctccacc aaggtgggtg cccgccccac 8040
   cgtccccggc agcggcctgg agaccgccga agggctgtcg gctcccgtca tacggaaggc 8100
   cgcggaggac agcctgcgac gcctgggcac cgatcgcatc gatctgtact ggacccacat 8160
   cgaggaccgg accgtccctc tggaggagac gctcggagct ctcgacgagc tggtcggcgg 8220
   cggcaaggtg gcggtgctgg gctgctccaa ccacgcgggc tggcgcatcg aacgggcccg 8280
   cgcgctcgcc cggaccaacg gctggacggc gtacacctgc gtccagcagc gctactccta 8340
   ccttcagccg cgcttcgacg tcggactgcc ggagagcgga cacgtccacg cgacctccga 8400
   actcctcgac cacgtacgca gcgagcccga cctgacgctg ctggcctact cgtccctgct 8460
   ctcgggcgcg tacacccggc cggacaagac cctgtccgcc gcctacgacc acccgggcac 8520
   cgggcagcgg ctgaccgtgc tgcgggaggt ggccgccgag ctcggtgcca ccgccaacca 8580
   ggtggtgctg tcctggttgc tcggaggtga tccgccggtg atcccgatcg tgggggtgag 8640
   ttccgtcgag cagctggacg aggtgctggc cgccgtcgag ctggagctgc cccgggagac 8700
   gagggcacgg ctggacctcg ccgggcgggg ctgacgggcc acacaccgcc cctcgcccgg 8760
   gacgggctga ccggccgcac accgtccctc agccgggacg ggctgacggc cacacacccg 8820
   cccctcagcc cggctgggct gggctgacgg ccacacaccc gctcctcagc ccggctgggc 8880
   tgggctgacg gccacacacc cgcccctcag cccggctggg ctgggctgac ggccacacgc 8940
   cgtccctcgc ccgggtcggg ctgaccagcc acacgccgtc cctcagtcgg cccgctcccc 9000
   ggcccgcccg gcgtcgacgt gccgccggtc gtggcgccgg gccgcgtcga tggcctgtgc 9060
   caggcgccgc acggctccgg gcacggcgtg gtccgccaga ttgccgtagc cgaggaccag 9120
   cgcggggccg gcggcggcca cggcccgcgc cccgccggag tcgccggccc gggccgtcgg 9180
   gtggtgctcg gcccgcgcca tccggtaggc gtcgagatcc gccagcctca cgtcacgccg 9240
   cgccgcctcc ctgacgacgg tgggcgccga gcagtccgtc agggacagca gcaggtggaa 9300
   gccggccgcc gcaccggaca cctggaaccc cggcagcgac gccgcgagtt ctcccatgag 9360
   gtgatcgcgg cgccgtttgt agcgcagccg tgccgcccgc aggtaccggt cgtaggaccc 9420
   gcgggccagg aaccgggcga acgcctcctg gtcgatgacg ggcggtggta cgcccccgac 9480
   gtgctccgcg cccagggcct cggtccagcg cggcggggtc accgcccagc cgatccgcag 9540
   ggccggcgag agcgtcttgc tgaccgaccc cagcagcgcg acgtgctccg gtgccatgcc 9600
   ctgcagggcg cccacggggt ggcggtcgta tcggaactcg gcgtcgtagt cgtcctccag 9660
   caccaggccg tcgacctcgc gggcccaggc gaccagctcg ccgcgacggg ccggggcgag 9720
   gaccacaccg gtcgggaact ggtgggcggg agcgacgatc accgaccgga cctgaggcgc 9780
   gcgccgcagc aggtcgaccc gcaggccgtc gccgtccacg ggaaccggga cgggcaccag 9840
   cccctccgcc ccgaccgtgg cgctcagccg cgaccagcca gggtcctcca gggccatgtg 9900
   ggtgtggccc tcggtccgca ggacccggca cagcctgcgc accgcgtcca gcgtgcccgc    9960
   gcagacgacg agctgccggg cttccagcga cgcgccgcgt ccccgcacga ggtaggccgc   10020
   gagccgctgc cggagccggg cgagaccggc cggatcgggg tagccgagct cgccccaggt   10080
   cagtgcgccg gtggcctccc gcaccgcgtc cgcccaccgt ccgcggggaa aggcccgcag   10140
   gtcgggtatg ccgggcagca tgtcgaactc cggctcccac cgcgtaccga cgtccgcctc   10200
   gggcgtcacc ggagcgggga cggcctgcgc cctgacccgg gtggccgagc cgctgcgcgc   10260
   ctccaggtac ccctccgcga cgagctgctc gtagggggga tcctctagag tcgacctgca   10320
   ggcct                                                               10325
<210> 107
   <211> 1218
   <212> DNA
   <213> Streptomyces ambofaciens
<220>
   <221> CDS
   <222> (1)..(1218)
<400> 107
   atg gct cat atc gca ttc ttc atc ctt ccg gtt gcc ggg cat gtg aat      48
   Met Ala His Ile Ala Phe Phe Ile Leu Pro Val Ala Gly His Val Asn
   1               5                   10                  15
   ccg acc ctg gga gtc gcc gag gaa ctg gtc gcg cgc ggc cac cgg gtg      96
   Pro Thr Leu Gly Val Ala Glu Glu Leu Val Ala Arg Gly His Arg Val
               20                  25                  30
   acg ttc gcg ctg tcc gag gac ctc gcc gag cgg gcc cgg ctg atc ggc     144
   Thr Phe Ala Leu Ser Glu Asp Leu Ala Glu Arg Ala Arg Leu Ile Gly
           35                  40                  45
   gcc gag gtg gtc acc tat ccg gtg gac agg caa cgg ttc ctg gac cag     192
   Ala Glu Val Val Thr Tyr Pro Val Asp Arg Gln Arg Phe Leu Asp Gln
       50                  55                  60
   atg gtg ccg cgg cag gac gcg gac gag tac acg gac gag gac gag ttc     240
   Met Val Pro Arg Gln Asp Ala Asp Glu Tyr Thr Asp Glu Asp Glu Phe
   65                  70                  75                  80
   gtc cgg gtc ctg gag tgg ctg ctg gac atg acg gtg cag acc atg gaa     288
   Val Arg Val Leu Glu Trp Leu Leu Asp Met Thr Val Gln Thr Met Glu
                   85                  90                  95
   ccg ctg gag agg cac ttc gcc ggg gac cgg ccc gac gtc gtc gtc aac     336
   Pro Leu Glu Arg His Phe Ala Gly Asp Arg Pro Asp Val Val Val Asn
               100                 105                 110
   gat ccg tcg tcg ctg tgg acg gga cgg ctg ctg gcg gac cgg tgg ggc     384
   Asp Pro Ser Ser Leu Trp Thr Gly Arg Leu Leu Ala Asp Arg Trp Gly
           115                 120                 125
   atc ccg gtc atc cgc agc act ccg acc tat gcc gcc aac gaa cac tgg     432
   Ile Pro Val Ile Arg Ser Thr Pro Thr Tyr Ala Ala Asn Glu His Trp
       130                 135                 140
   tcg ctg cat ccg ccg gtc gac tcg gcc gag ccg ccg gac gac ccc gag     480
   Ser Leu His Pro Pro Val Asp Ser Ala Glu Pro Pro Asp Asp Pro Glu
   145                 150                 155                 160
   ctg cac aag ctg ctc gcg cgg atc gag cgg ctg ctg gag gag cag ggc      528
   Leu His Lys Leu Leu Ala Arg Ile Glu Arg Leu Leu Glu Glu Gln Gly
                   165                 170                 175
   gtc gag cac gac ctg gcc ggc ttc acc ggg gtc ctg cac ggc ggt ccg      576
   Val Glu His Asp Leu Ala Gly Phe Thr Gly Val Leu His Gly Gly Pro
               180                 185                 190
   gcc ctg ctg tac atg ccc cgc tcg ttc cag tac gcg ggc gag acc ttc      624
   Ala Leu Leu Tyr Met Pro Arg Ser Phe Gln Tyr Ala Gly Glu Thr Phe
           195                 200                 205
   gac gca cag cac cac ttc gtc ggc ccc tgc ccg ccc cgc acc gcg ttc      672
   Asp Ala Gln His His Phe Val Gly Pro Cys Pro Pro Arg Thr Ala Phe
       210                 215                 220
   cac ggc gag tgg agg ccg ggg gac gac gac ggc cgg ccc ctg gtg ctg      720
   His Gly Glu Trp Arg Pro Gly Asp Asp Asp Gly Arg Pro Leu Val Leu
   225                 230                 235                 240
   gtg agt ctc gga acc ctg tac aac gac cgg ccg gac ttc ttc cgc acc      768
   Val Ser Leu Gly Thr Leu Tyr Asn Asp Arg Pro Asp Phe Phe Arg Thr
                   245                 250                 255
   tgc ctg gag gcg ttc ggc gac gag ccc tgg aac gtg ctt ctg gtg ctg      816
   Cys Leu Glu Ala Phe Gly Asp Glu Pro Trp Asn Val Leu Leu Val Leu
               260                 265                 270
   ggc ggc ggg gtg ccc gcg gcc gac ctg ggc ccg ctt ccc ggc aat gtc      864
   Gly Gly Gly Val Pro Ala Ala Asp Leu Gly Pro Leu Pro Gly Asn Val
           275                 280                 285
   cgg gtg acc gac ttc gtg tcg ctg cgc gac gtc ctg ccg cac acg gcg      912
   Arg Val Thr Asp Phe Val Ser Leu Arg Asp Val Leu Pro His Thr Ala
       290                 295                 300
   gtg gtg gtg aac cac ggt ggg atg agc acc gcc atg gag gtg ttc tcg      960
   Val Val Val Asn His Gly Gly Met Ser Thr Ala Met Glu Val Phe Ser
   305                 310                 315                 320
   cac ggt gtg ccg gtg gtg gcg atc ccg gtg atg ccg gag ccc cgg gcc     1008
   His Gly Val Pro Val Val Ala Ile Pro Val Met Pro Glu Pro Arg Ala
                   325                 330                 335
   acc gcg cgg cgg atc gtc gaa ctg ggc ctg ggc gac cag ctg ctg aac     1056
   Thr Ala Arg Arg Ile Val Glu Leu Gly Leu Gly Asp Gln Leu Leu Asn
               340                 345                 350
   tcg gag ctg acg gcc gag tcc ctg cgt gcc acg gta cgg cgg gtg ctg     1104
   Ser Glu Leu Thr Ala Glu Ser Leu Arg Ala Thr Val Arg Arg Val Leu
           355                 360                 365
   gcg gac tcc gcg atc ccg ggg aac atg cgc ggg ttc cgg gag cag atc     1152
   Ala Asp Ser Ala Ile Pro Gly Asn Met Arg Gly Phe Arg Glu Gln Ile
       370                 375                 380
   agg gcg gcc ggc ggg gcg ccc gcg gcg gcc gac gcg atc gag gga ctg     1200
   Arg Ala Ala Gly Gly Ala Pro Ala Ala Ala Asp Ala Ile Glu Gly Leu
   385                 390                 395                 400
   ctg ccc cgg gtg ggc tga                                             1218
   Leu Pro Arg Val Gly
                   405
<210> 108
   <211> 405
   <212> PRT
   <213> Streptomyces ambofaciens
<400> 108
   Met Ala His Ile Ala Phe Phe Ile Leu Pro Val Ala Gly His Val Asn
   1 5 10 15
   Pro Thr Leu Gly Val Ala Glu Glu Leu Val Ala Arg Gly His Arg Val
   20 25 30
   Thr Phe Ala Leu Ser Glu Asp Leu Ala Glu Arg Ala Arg Leu Ile Gly
   35 40 45
   Ala Glu Val Val Thr Tyr Pro Val Asp Arg Gln Arg Phe Leu Asp Gln
   50 55 60
   Met Val Pro Arg Gln Asp Ala Asp Glu Tyr Thr Asp Glu Asp Glu Phe
   65 70 75 80
   Val Arg Val Leu Glu Trp Leu Leu Asp Met Thr Val Gln Thr Met Glu
   85 90 95
   Pro Leu Glu Arg His Phe Ala Gly Asp Arg Pro Asp Val Val Val Asn
   100 105 110
   Asp Pro Ser Ser Leu Trp Thr Gly Arg Leu Leu Ala Asp Arg Trp Gly
   115 120 125
   Ile Pro Val Ile Arg Ser Thr Pro Thr Tyr Ala Ala Asn Glu His Trp
   130 135 140
   Ser Leu His Pro Pro Val Asp Ser Ala Glu Pro Pro Asp Asp Pro Glu
   145 150 155 160
   Leu His Lys Leu Leu Ala Arg Ile Glu Arg Leu Leu Glu Glu Gln Gly
   165 170 175
   Val Glu His Asp Leu Ala Gly Phe Thr Gly Val Leu His Gly Gly Pro
   180 185 190
   Ala Leu Leu Tyr Met Pro Arg Ser Phe Gln Tyr Ala Gly Glu Thr Phe
   195 200 205
   Asp Ala Gln His His Phe Val Gly Pro Cys Pro Pro Arg Thr Ala Phe
   210 215 220
   His Gly Glu Trp Arg Pro Gly Asp Asp Asp Gly Arg Pro Leu Val Leu
   225 230 235 240
   Val Ser Leu Gly Thr Leu Tyr Asn Asp Arg Pro Asp Phe Phe Arg Thr
   245 250 255
   Cys Leu Glu Ala Phe Gly Asp Glu Pro Trp Asn Val Leu Leu Val Leu
               260                 265                 270
   Gly Gly Gly Val Pro Ala Ala Asp Leu Gly Pro Leu Pro Gly Asn Val
           275                 280                 285
   Arg Val Thr Asp Phe Val Ser Leu Arg Asp Val Leu Pro His Thr Ala
       290                 295                 300
   Val Val Val Asn His Gly Gly Met Ser Thr Ala Met Glu Val Phe Ser
   305                 310                 315                 320
   His Gly Val Pro Val Val Ala Ile Pro Val Met Pro Glu Pro Arg Ala
                   325                 330                 335
   Thr Ala Arg Arg Ile Val Glu Leu Gly Leu Gly Asp Gln Leu Leu Asn
               340                 345                 350
   Ser Glu Leu Thr Ala Glu Ser Leu Arg Ala Thr Val Arg Arg Val Leu
           355                 360                 365
   Ala Asp Ser Ala Ile Pro Gly Asn Met Arg Gly Phe Arg Glu Gln Ile
       370                 375                 380
   Arg Ala Ala Gly Gly Ala Pro Ala Ala Ala Asp Ala Ile Glu Gly Leu
   385                 390                 395                 400
   Leu Pro Arg Val Gly
                   405
<210> 109
   <211> 621
   <212> DNA
   <213> Streptomyces ambofaciens
<220>
   <221> CDS
   <222> (1)..(621)
<400> 109
   atg ctc atc acc gaa aca gca gtg ccc gac gtg ttc cgc atc gat ccg      48
   Met Leu Ile Thr Glu Thr Ala Val Pro Asp Val Phe Arg Ile Asp Pro
   1               5                   10                  15
   gaa ccg ctg ccg gac cac cgg ggc cgg ttc tac gaa gcg gtg cgc cgc      96
   Glu Pro Leu Pro Asp His Arg Gly Arg Phe Tyr Glu Ala Val Arg Arg
               20                  25                  30
   ggg ccg ctg gag gcc gcc gtc ggg cac tcg gtc gag gtc cgg cag gtc     144
   Gly Pro Leu Glu Ala Ala Val Gly His Ser Val Glu Val Arg Gln Val
           35                  40                  45
   cac tgc acc gtc tcc ggg cgc aac gta ctg cgc ggc ctg cac gcc acc     192
   His Cys Thr Val Ser Gly Arg Asn Val Leu Arg Gly Leu His Ala Thr
       50                  55                  60
   acc ctg ccg ccg ggc cag gcc aag atc ctc acc tgt gtg cgg ggt gcg      240
   Thr Leu Pro Pro Gly Gln Ala Lys Ile Leu Thr Cys Val Arg Gly Ala
   65                  70                  75                  80
   gcg ctg act atg gtg gtc gac atg agg gtc ggg tca ccg ggc ttc gga      288
   Ala Leu Thr Met Val Val Asp Met Arg Val Gly Ser Pro Gly Phe Gly
                   85                  90                  95
   cgg tac gag gcg gtg cgg cag gat gcc ccg tcg ggc acc gcg ctc tac      336
   Arg Tyr Glu Ala Val Arg Gln Asp Ala Pro Ser Gly Thr Ala Leu Tyr
               100                 105                 110
   ctg ccg gac ggc atc ggc ctg ggc tac gtg gcc ctc gcg gac gac aca      384
   Leu Pro Asp Gly Ile Gly Leu Gly Tyr Val Ala Leu Ala Asp Asp Thr
           115                 120                 125
   tgc atg aac tac ctg tgc acc gag gag tac gtc ccg ggc atg gtc atc      432
   Cys Met Asn Tyr Leu Cys Thr Glu Glu Tyr Val Pro Gly Met Val Ile
       130                 135                 140
   gac gtg gac gcc ctg gac ccc gaa ctc ggc ctg ccg tgg gga ctg acc      480
   Asp Val Asp Ala Leu Asp Pro Glu Leu Gly Leu Pro Trp Gly Leu Thr
   145                 150                 155                 160
   ggg gat ccc gtc cgc tcc gcc cgg gac gcg gcg gcg ccg tcc ctg cgg      528
   Gly Asp Pro Val Arg Ser Ala Arg Asp Ala Ala Ala Pro Ser Leu Arg
                   165                 170                 175
   gcg gcc gcg gcg gcg gga att ctg ccc acg tac gag gac tgc tta cgg      576
   Ala Ala Ala Ala Ala Gly Ile Leu Pro Thr Tyr Glu Asp Cys Leu Arg
               180                 185                 190
   gtg cgc acg tcc gtg ccc gcc cct ccc gac cgg act cgg ctc tga          621
   Val Arg Thr Ser Val Pro Ala Pro Pro Asp Arg Thr Arg Leu
           195                 200                 205
<210> 110
   <211> 206
   <212> PRT
   <213> Streptomyces ambofaciens
<400> 110
   Met Leu Ile Thr Glu Thr Ala Val Pro Asp Val Phe Arg Ile Asp Pro
   1               5                   10                  15
   Glu Pro Leu Pro Asp His Arg Gly Arg Phe Tyr Glu Ala Val Arg Arg
               20                  25                  30
   Gly Pro Leu Glu Ala Ala Val Gly His Ser Val Glu Val Arg Gln Val
           35                  40                  45
   His Cys Thr Val Ser Gly Arg Asn Val Leu Arg Gly Leu His Ala Thr
       50                  55                  60
   Thr Leu Pro Pro Gly Gln Ala Lys Ile Leu Thr Cys Val Arg Gly Ala
   65                  70                  75                  80
   Ala Leu Thr Met Val Val Asp Met Arg Val Gly Ser Pro Gly Phe Gly
                   85                  90                  95
   Arg Tyr Glu Ala Val Arg Gln Asp Ala Pro Ser Gly Thr Ala Leu Tyr
               100                 105                 110
   Leu Pro Asp Gly Ile Gly Leu Gly Tyr Val Ala Leu Ala Asp Asp Thr
           115                 120                 125
   Cys Met Asn Tyr Leu Cys Thr Glu Glu Tyr Val Pro Gly Met Val Ile
       130                 135                 140
   Asp Val Asp Ala Leu Asp Pro Glu Leu Gly Leu Pro Trp Gly Leu Thr
   145                 150                 155                 160
   Gly Asp Pro Val Arg Ser Ala Arg Asp Ala Ala Ala Pro Ser Leu Arg
                   165                 170                 175
   Ala Ala Ala Ala Ala Gly Ile Leu Pro Thr Tyr Glu Asp Cys Leu Arg
               180                 185                 190
   Val Arg Thr Ser Val Pro Ala Pro Pro Asp Arg Thr Arg Leu
           195                 200                 205
<210> 111
   <211> 960
   <212> DNA
   <213> Streptomyces ambofaciens
<220>
   <221> CDS
   <222> (1)..(903)
<220>
   <221> misc_feature
   <222> (905)..(960)
   <223> n is a, c, g, or t
<400> 111
   atg acc tta cgc acg ctt cga ccc gcc ggc gcg gcg ccc ccc gtg ggt      48
   Met Thr Leu Arg Thr Leu Arg Pro Ala Gly Ala Ala Pro Pro Val Gly
   1               5                   10                  15
   ccc gcc gcg cgg cac gac ttc gac tcg ctc gtc tcc gac gca tgt gcg      96
   Pro Ala Ala Arg His Asp Phe Asp Ser Leu Val Ser Asp Ala Cys Ala
               20                  25                  30
   gag tta ctc ggt tcg ctc cgc cgg gcg gac cag cgc aga aga ggt gag     144
   Glu Leu Leu Gly Ser Leu Arg Arg Ala Asp Gln Arg Arg Arg Gly Glu
           35                  40                  45
   cag tac ata cgc ggg ctg ctc acg gca cag gga cgc aag acc gcc cgg     192
   Gln Tyr Ile Arg Gly Leu Leu Thr Ala Gln Gly Arg Lys Thr Ala Arg
       50                  55                  60
   aac ctc gcc gcc ttc gtc gga gag ggc gcg gcg gaa cag agc ctg cac     240
   Asn Leu Ala Ala Phe Val Gly Glu Gly Ala Ala Glu Gln Ser Leu His
   65                  70                  75                  80
   cac ttc gtc gcc ggg tcg acc tgg gac tgg ggc gag gtg cgg gcc gcg     288
   His Phe Val Ala Gly Ser Thr Trp Asp Trp Gly Glu Val Arg Ala Ala
                   85                  90                  95
   ctg gcc cgc tac gtg gac gac agg ctg acc ccc gac gcc tgg gtg atc      336
   Leu Ala Arg Tyr Val Asp Asp Arg Leu Thr Pro Asp Ala Trp Val Ile
               100                 105                 110
   tgg ccg atg gtg gtc tcc aag gcg ggg gtg cgc tcc gtc ggg gtg agc      384
   Trp Pro Met Val Val Ser Lys Ala Gly Val Arg Ser Val Gly Val Ser
           115                 120                 125
   cgg cgc ttc gtc tcc gac ctg ggc cgg gtg gtg agc tgc cag cag agt      432
   Arg Arg Phe Val Ser Asp Leu Gly Arg Val Val Ser Cys Gln Gln Ser
       130                 135                 140
   cac ggg ctg tgg ctg gcc tcg ggc acg acg gcc gcc ccg gtc agc tgg      480
   His Gly Leu Trp Leu Ala Ser Gly Thr Thr Ala Ala Pro Val Ser Trp
   145                 150                 155                 160
   cgc ctg acg ctg ggc ggc ggc cgg agc ggg gag ggc aca ccc cgg cac      528
   Arg Leu Thr Leu Gly Gly Gly Arg Ser Gly Glu Gly Thr Pro Arg His
                   165                 170                 175
   ccc ggc gca ccc ggt gag gag gag aac atc gtc cgc ctg gtg gcc gaa      576
   Pro Gly Ala Pro Gly Glu Glu Glu Asn Ile Val Arg Leu Val Ala Glu
               180                 185                 190
   gcc gct cag gcc aac cgc tcc ttg gcc cgc ccc gtg gtg atg gac gca      624
   Ala Ala Gln Ala Asn Arg Ser Leu Ala Arg Pro Val Val Met Asp Ala
           195                 200                 205
   cgc gcg gcc gtg ctg cca cga ctg gtg cga ggg ctc ggt ctc gcg ggc      672
   Arg Ala Ala Val Leu Pro Arg Leu Val Arg Gly Leu Gly Leu Ala Gly
       210                 215                 220
   ctg ccc ttc atg gtg cgg gtg ggc ggg aac ctc cgg ctg gcc tcg gcg      720
   Leu Pro Phe Met Val Arg Val Gly Gly Asn Leu Arg Leu Ala Ser Ala
   225                 230                 235                 240
   agc ggc cgc ggc ccg gtg gac cac cac acg ggg acc acc tcg gcc cag      768
   Ser Gly Arg Gly Pro Val Asp His His Thr Gly Thr Thr Ser Ala Gln
                   245                 250                 255
   cag ttg atg gag cag ctc aag cgg ttg agc cgc ccc gtg gag ttg cgg      816
   Gln Leu Met Glu Gln Leu Lys Arg Leu Ser Arg Pro Val Glu Leu Arg
               260                 265                 270
   ggc tcc ctc agc ctc gtc gcc ccg cac ccc gtc gtc ctg ccg ggt gtg      864
   Gly Ser Leu Ser Leu Val Ala Pro His Pro Val Val Leu Pro Gly Val
           275                 280                 285
   gtc ccg agg cgg acg ctg acg ctg ctg ggg ggt gtg gcg gnnnnnnnnn       913
   Val Pro Arg Arg Thr Leu Thr Leu Leu Gly Gly Val Ala
       290                 295                 300
   nnnnnnnnnn nnnnnnnnnn nnnnnnnnnn nnnnnnnnnn nnnnnnn                  960
<210> 112
   <211> 301
   <212> PRT
   <213> Streptomyces ambofaciens
<400> 112
   Met Thr Leu Arg Thr Leu Arg Pro Ala Gly Ala Ala Pro Pro Val Gly
   1               5                   10                  15
   Pro Ala Ala Arg His Asp Phe Asp Ser Leu Val Ser Asp Ala Cys Ala
               20                  25                  30
   Glu Leu Leu Gly Ser Leu Arg Arg Ala Asp Gln Arg Arg Arg Gly Glu
   35 40 45
   Gln Tyr Ile Arg Gly Leu Leu Thr Ala Gln Gly Arg Lys Thr Ala Arg
   50 55 60
   Asn Leu Ala Ala Phe Val Gly Glu Gly Ala Ala Glu Gln Ser Leu His
   65 70 75 80
   His Phe Val Ala Gly Ser Thr Trp Asp Trp Gly Glu Val Arg Ala Ala
   85 90 95
   Leu Ala Arg Tyr Val Asp Asp Arg Leu Thr Pro Asp Ala Trp Val Ile
   100 105 110
   Trp Pro Met Val Val Ser Lys Ala Gly Val Arg Ser Val Gly Val Ser
   115 120 125
   Arg Arg Phe Val Ser Asp Leu Gly Arg Val Val Ser Cys Gln Gln Ser
   130 135 140
   His Gly Leu Trp Leu Ala Ser Gly Thr Thr Ala Ala Pro Val Ser Trp
   145 150 155 160
   Arg Leu Thr Leu Gly Gly Gly Arg Ser Gly Glu Gly Thr Pro Arg His
   165 170 175
   Pro Gly Ala Pro Gly Glu Glu Glu Asn Ile Val Arg Leu Val Ala Glu
   180 185 190
   Ala Ala Gln Ala Asn Arg Ser Leu Ala Arg Pro Val Val Met Asp Ala
   195 200 205
   Arg Ala Ala Val Leu Pro Arg Leu Val Arg Gly Leu Gly Leu Ala Gly
   210 215 220
   Leu Pro Phe Met Val Arg Val Gly Gly Asn Leu Arg Leu Ala Ser Ala
   225 230 235 240
   Ser Gly Arg Gly Pro Val Asp His His Thr Gly Thr Thr Ser Ala Gln
   245 250 255
   Gln Leu Met Glu Gln Leu Lys Arg Leu Ser Arg Pro Val Glu Leu Arg
   260 265 270
   Gly Ser Leu Ser Leu Val Ala Pro His Pro Val Val Leu Pro Gly Val
   275 280 285
   Val Pro Arg Arg Thr Leu Thr Leu Leu Gly Gly Val Ala
   290 295 300
<210> 113
   <211> 1008
   <212> DNA
   <213> Streptomyces ambofaciens
<220>
   <221> CDS
   <222> (1)..(1008)
<400> 113
   atg cag gca att cgg cat cac gtc atg ctc gtc atg gcc ttc gtg acc      48
   Met Gln Ala Ile Arg His His Val Met Leu Val Met Ala Phe Val Thr
   1               5                   10                  15
   gtc gcc acg act ttc ctt ctc tgg ccg tcg acg caa tcc gcg caa gcg      96
   Val Ala Thr Thr Phe Leu Leu Trp Pro Ser Thr Gln Ser Ala Gln Ala
               20                  25                  30
   ttt ccc ccg acc ccg aag cag acg gta ctg aac cac ctc cgc gcc att     144
   Phe Pro Pro Thr Pro Lys Gln Thr Val Leu Asn His Leu Arg Ala Ile
           35                  40                  45
   tcc ggg aat cac atc gtc tcc gga cag cac aac aag gag ccc gcc tcc     192
   Ser Gly Asn His Ile Val Ser Gly Gln His Asn Lys Glu Pro Ala Ser
       50                  55                  60
   gcc ccg ggc cag tac acc cag cag gtc aag gac gtc acc ggg cag tac     240
   Ala Pro Gly Gln Tyr Thr Gln Gln Val Lys Asp Val Thr Gly Gln Tyr
   65                  70                  75                  80
   ccc ggc ctg tgg ggc ggt gac ctg atg ttc gcc gcg gcg gac gtg gcc     288
   Pro Gly Leu Trp Gly Gly Asp Leu Met Phe Ala Ala Ala Asp Val Ala
                   85                  90                  95
   ggc cgc cag cgc gtc gtc gac cag gcc agg acc gag tgg gcg aac gga     336
   Gly Arg Gln Arg Val Val Asp Gln Ala Arg Thr Glu Trp Ala Asn Gly
               100                 105                 110
   tcg ctg gtc tcg ctc acc tgg cac gtc tgc ccg ccg acc ggc ggc agc     384
   Ser Leu Val Ser Leu Thr Trp His Val Cys Pro Pro Thr Gly Gly Ser
           115                 120                 125
   acc tgt gcg ttc gag ggc ggc gtc aag tcc acg ctg acg aac gcg cag     432
   Thr Cys Ala Phe Glu Gly Gly Val Lys Ser Thr Leu Thr Asn Ala Gln
       130                 135                 140
   ttc tcg cag gtc ctc acg gag ggc agt gcc ctg aac agc gca tgg aag     480
   Phe Ser Gln Val Leu Thr Glu Gly Ser Ala Leu Asn Ser Ala Trp Lys
   145                 150                 155                 160
   cgg cgc ctg gac gag gtc gtc ccg tac ctg cag cag ctg gag aac gcg     528
   Arg Arg Leu Asp Glu Val Val Pro Tyr Leu Gln Gln Leu Glu Asn Ala
                   165                 170                 175
   ggc gtc ccc gtc ctc ttc cgg ccg ctg cac gag atg aac gaa tcc tgg     576
   Gly Val Pro Val Leu Phe Arg Pro Leu His Glu Met Asn Glu Ser Trp
               180                 185                 190
   aac tgg tgg gga aac cgg ccc gga gcg aac ggc agc gca cgc ctc tac     624
   Asn Trp Trp Gly Asn Arg Pro Gly Ala Asn Gly Ser Ala Arg Leu Tyr
           195                 200                 205
   cag atc acc cgc gat cac ctc gcc ggg acg aaa ggg ctg gac aat ctg     672
   Gln Ile Thr Arg Asp His Leu Ala Gly Thr Lys Gly Leu Asp Asn Leu
       210                 215                 220
   atc tgg gtc tgg aac gtc cag gac aat ccg gcg gga aac tgg aac agc      720
   Ile Trp Val Trp Asn Val Gln Asp Asn Pro Ala Gly Asn Trp Asn Ser
   225                 230                 235                 240
   tac tat ccg gga gat cag tac gtg gac gtc gtt tcg ctg gac gtc tgg      768
   Tyr Tyr Pro Gly Asp Gln Tyr Val Asp Val Val Ser Leu Asp Val Trp
                   245                 250                 255
   tac aag agc cac ccg agt tcc gcc gac tac cag cag atg cgg agc atc      816
   Tyr Lys Ser His Pro Ser Ser Ala Asp Tyr Gln Gln Met Arg Ser Ile
               260                 265                 270
   gcg gga aca aaa ccc atg gcc ctc gcg gag ctg ggc aaa atg ccg acc      864
   Ala Gly Thr Lys Pro Met Ala Leu Ala Glu Leu Gly Lys Met Pro Thr
           275                 280                 285
   gcc gcg ctg ctg gac agc cag acg cgg tgg aca tgg ttc atg atg tgg      912
   Ala Ala Leu Leu Asp Ser Gln Thr Arg Trp Thr Trp Phe Met Met Trp
       290                 295                 300
   tcc gag cat ctg cgc ggg aac aat tcc aac gcc gaa ata cag acg gcg      960
   Ser Glu His Leu Arg Gly Asn Asn Ser Asn Ala Glu Ile Gln Thr Ala
   305                 310                 315                 320
   tat ttc cac ccc cgt gta ctg aac cag ggg gag gtc gca ctg ccc tga     1008
   Tyr Phe His Pro Arg Val Leu Asn Gln Gly Glu Val Ala Leu Pro
                   325                 330                 335
<210> 114
   <211> 335
   <212> PRT
   <213> Streptomyces ambofaciens
<400> 114
   Met Gln Ala Ile Arg His His Val Met Leu Val Met Ala Phe Val Thr
   1               5                   10                  15
   Val Ala Thr Thr Phe Leu Leu Trp Pro Ser Thr Gln Ser Ala Gln Ala
               20                  25                  30
   Phe Pro Pro Thr Pro Lys Gln Thr Val Leu Asn His Leu Arg Ala Ile
           35                  40                  45
   Ser Gly Asn His Ile Val Ser Gly Gln His Asn Lys Glu Pro Ala Ser
       50                  55                  60
   Ala Pro Gly Gln Tyr Thr Gln Gln Val Lys Asp Val Thr Gly Gln Tyr
   65                  70                  75                  80
   Pro Gly Leu Trp Gly Gly Asp Leu Met Phe Ala Ala Ala Asp Val Ala
                   85                  90                  95
   Gly Arg Gln Arg Val Val Asp Gln Ala Arg Thr Glu Trp Ala Asn Gly
               100                 105                 110
   Ser Leu Val Ser Leu Thr Trp His Val Cys Pro Pro Thr Gly Gly Ser
           115                 120                 125
   Thr Cys Ala Phe Glu Gly Gly Val Lys Ser Thr Leu Thr Asn Ala Gln
   130 135 140
   Phe Ser Gln Val Leu Thr Glu Gly Ser Ala Leu Asn Ser Ala Trp Lys
   145 150 155 160
   Arg Arg Leu Asp Glu Val Val Pro Tyr Leu Gln Gln Leu Glu Asn Ala
   165 170 175
   Gly Val Pro Val Leu Phe Arg Pro Leu His Glu Met Asn Glu Ser Trp
   180 185 190
   Asn Trp Trp Gly Asn Arg Pro Gly Ala Asn Gly Ser Ala Arg Leu Tyr
   195 200 205
   Gln Ile Thr Arg Asp His Leu Ala Gly Thr Lys Gly Leu Asp Asn Leu
   210 215 220
   Ile Trp Val Trp Asn Val Gln Asp Asn Pro Ala Gly Asn Trp Asn Ser
   225 230 235 240
   Tyr Tyr Pro Gly Asp Gln Tyr Val Asp Val Val Ser Leu Asp Val Trp
   245 250 255
   Tyr Lys Ser His Pro Ser Ser Ala Asp Tyr Gln Gln Met Arg Ser Ile
   260 265 270
   Ala Gly Thr Lys Pro Met Ala Leu Ala Glu Leu Gly Lys Met Pro Thr
   275 280 285
   Ala Ala Leu Leu Asp Ser Gln Thr Arg Trp Thr Trp Phe Met Met Trp
   290 295 300
   Ser Glu His Leu Arg Gly Asn Asn Ser Asn Ala Glu Ile Gln Thr Ala
   305 310 315 320
   Tyr Phe His Pro Arg Val Leu Asn Gln Gly Glu Val Ala Leu Pro
   325 330 335
<210> 115
   <211> 1038
   <212> DNA
   <213> Streptomyces ambofaciens
<220>
   <221> CDS
   <222> (1)..(1038)
<220>
   <221> CDS2
   <222> (190)..(1038)
<400> 115
   atg acg tgc ggg ctc ggg cgg gaa cgg ctc cac cgt aga aag gaa gcg 48
   Met Thr Cys Gly Leu Gly Arg Glu Arg Leu His Arg Arg Lys Glu Ala
   1 5 10 15
   gtc cgg cgt ccc ggt cca aaa gag cac ggg aaa acc gga ccg atc gcg 96
   Val Arg Arg Pro Gly Pro Lys Glu His Gly Lys Thr Gly Pro Ile Ala
   20 25 30
   cgt ctg tgc cct ggc ggg ccg gct cgt tcc aag ccc tgg tcg cgg ttg 144
   Arg Leu Cys Pro Gly Gly Pro Ala Arg Ser Lys Pro Trp Ser Arg Leu
   35 40 45
   gcc ccc tac ggt ggt acg acc gac gac aag gcg acg ggg gcg acg atg 192
   Ala Pro Tyr Gly Gly Thr Thr Asp Asp Lys Ala Thr Gly Ala Thr Met
   50 55 60
   cgt atc gcg gtg aca ggg gcg gcg ggc agt ctg ggc agg cgg gtg gtg 240
   Arg Ile Ala Val Thr Gly Ala Ala Gly Ser Leu Gly Arg Arg Val Val
   65 70 75 80
   cag ctg ctc gcg gac cgg gcc gac gtg gac gtc gtg gcg atg acc agg 288
   Gln Leu Leu Ala Asp Arg Ala Asp Val Asp Val Val Ala Met Thr Arg
   85 90 95
   cgg aag ctg cct gcc gag gca cta ccg ccg ggg gtg gag tgc gcc gtc 336
   Arg Lys Leu Pro Ala Glu Ala Leu Pro Pro Gly Val Glu Cys Ala Val
   100 105 110
   gcc gac tac gcc gac ccg ccc gct ctg cgt gcg gcg ctg aag ggc gtg 384
   Ala Asp Tyr Ala Asp Pro Pro Ala Leu Arg Ala Ala Leu Lys Gly Val
   115 120 125
   gac act ctg gtc ctc gtc tcc agc gac ggg ccc gac gca cgg gtg ctg 432
   Asp Thr Leu Val Leu Val Ser Ser Asp Gly Pro Asp Ala Arg Val Leu
   130 135 140
   ctg cac cac cgc aac gtc gtc gcc gcc gtg gtg gcc gag cgc gtc ggc 480
   Leu His His Arg Asn Val Val Ala Ala Val Val Ala Glu Arg Val Gly
   145 150 155 160
   cat gtc gcg gca ctg agc agc gtc gac gcc gac tcg gcc tcc ccg ttc 528
   His Val Ala Ala Leu Ser Ser Val Asp Ala Asp Ser Ala Ser Pro Phe
   165 170 175
   tgc tac gcc gtc gtc aac cgg ctc acc gag gat ctg ctg ctc gcc tcc 576
   Cys Tyr Ala Val Val Asn Arg Leu Thr Glu Asp Leu Leu Leu Ala Ser
   180 185 190
   ggc gtg ccg tgc tcg ttc gcc agg gcc tcg ctg tac atc gag ttc ttc 624
   Gly Val Pro Cys Ser Phe Ala Arg Ala Ser Leu Tyr Ile Glu Phe Phe
   195 200 205
   cta ggc tgg ctc acc cag gcg cgc tcg acc ggg ctg ctg agg ctg ccg 672
   Leu Gly Trp Leu Thr Gln Ala Arg Ser Thr Gly Leu Leu Arg Leu Pro
   210 215 220
   gca gcc gat ggc cgg gtg tcc ctg gtg gcg cgc gac gac gtg gcc cgc 720
   Ala Ala Asp Gly Arg Val Ser Leu Val Ala Arg Asp Asp Val Ala Arg
   225 230 235 240
   gcc ctc gcc gcc ctc gcg gtg ggc ggg ccg acc ggc cgt cac cac gac 768
   Ala Leu Ala Ala Leu Ala Val Gly Gly Pro Thr Gly Arg His His Asp
   245 250 255
   atc acg gga ccc gag tcg acg gac ctg gca ggc atc gcc tcc acg gcg 816
   Ile Thr Gly Pro Glu Ser Thr Asp Leu Ala Gly Ile Ala Ser Thr Ala
   260 265 270
   gcg gag gtg tgg cgg aca ccg gtc gcc tac gtg gac atc ccg gcc gac        864
   Ala Glu Val Trp Arg Thr Pro Val Ala Tyr Val Asp Ile Pro Ala Asp
           275                 280                 285
   acg tac tct gcc gaa aca gcg gcg acc ggc ctg gat ccc tgg tgg ctc        912
   Thr Tyr Ser Ala Glu Thr Ala Ala Thr Gly Leu Asp Pro Trp Trp Leu
       290                 295                 300
   tac gcc ttc tcc tcc ctg ttc gcc tcg gta cgc gaa cag cgc tgg gat        960
   Tyr Ala Phe Ser Ser Leu Phe Ala Ser Val Arg Glu Gln Arg Trp Asp
   305                 310                 315                 320
   cgg gtc cgc gac gac tgc gcc cag ctc acc ggc cgc ttg ccc cgc act       1008
   Arg Val Arg Asp Asp Cys Ala Gln Leu Thr Gly Arg Leu Pro Arg Thr
                   325                 330                 335
   ctc cgc gac gtc ctg tca gaa cgc gcg tga                               1038
   Leu Arg Asp Val Leu Ser Glu Arg Ala
               340                 345
<210> 116
   <211> 345
   <212> PRT
   <213> Streptomyces ambofaciens
<400> 116
   Met Thr Cys Gly Leu Gly Arg Glu Arg Leu His Arg Arg Lys Glu Ala
   1               5                   10                  15
   Val Arg Arg Pro Gly Pro Lys Glu His Gly Lys Thr Gly Pro Ile Ala
               20                  25                  30
   Arg Leu Cys Pro Gly Gly Pro Ala Arg Ser Lys Pro Trp Ser Arg Leu
           35                  40                  45
   Ala Pro Tyr Gly Gly Thr Thr Asp Asp Lys Ala Thr Gly Ala Thr Met
       50                  55                  60
   Arg Ile Ala Val Thr Gly Ala Ala Gly Ser Leu Gly Arg Arg Val Val
   65                  70                  75                  80
   Gln Leu Leu Ala Asp Arg Ala Asp Val Asp Val Val Ala Met Thr Arg
                   85                  90                  95
   Arg Lys Leu Pro Ala Glu Ala Leu Pro Pro Gly Val Glu Cys Ala Val
               100                 105                 110
   Ala Asp Tyr Ala Asp Pro Pro Ala Leu Arg Ala Ala Leu Lys Gly Val
           115                 120                 125
   Asp Thr Leu Val Leu Val Ser Ser Asp Gly Pro Asp Ala Arg Val Leu
       130                 135                 140
   Leu His His Arg Asn Val Val Ala Ala Val Val Ala Glu Arg Val Gly
   145                 150                 155                 160
   His Val Ala Ala Leu Ser Ser Val Asp Ala Asp Ser Ala Ser Pro Phe
   165 170 175
   Cys Tyr Ala Val Val Asn Arg Leu Thr Glu Asp Leu Leu Leu Ala Ser
   180 185 190
   Gly Val Pro Cys Ser Phe Ala Arg Ala Ser Leu Tyr Ile Glu Phe Phe
   195 200 205
   Leu Gly Trp Leu Thr Gln Ala Arg Ser Thr Gly Leu Leu Arg Leu Pro
   210 215 220
   Ala Ala Asp Gly Arg Val Ser Leu Val Ala Arg Asp Asp Val Ala Arg
   225 230 235 240
   Ala Leu Ala Ala Leu Ala Val Gly Gly Pro Thr Gly Arg His His Asp
   245 250 255
   Ile Thr Gly Pro Glu Ser Thr Asp Leu Ala Gly Ile Ala Ser Thr Ala
   260 265 270
   Ala Glu Val Trp Arg Thr Pro Val Ala Tyr Val Asp Ile Pro Ala Asp
   275 280 285
   Thr Tyr Ser Ala Glu Thr Ala Ala Thr Gly Leu Asp Pro Trp Trp Leu
   290 295 300
   Tyr Ala Phe Ser Ser Leu Phe Ala Ser Val Arg Glu Gln Arg Trp Asp
   305 310 315 320
   Arg Val Arg Asp Asp Cys Ala Gln Leu Thr Gly Arg Leu Pro Arg Thr
   325 330 335
   Leu Arg Asp Val Leu Ser Glu Arg Ala
   340 345
<210> 117
   <211> 282
   <212> PRT
   <213> Streptomyces ambofaciens
<400> 117
   Met Arg Ile Ala Val Thr Gly Ala Ala Gly Ser Leu Gly Arg Arg Val
   1 5 10 15
   Val Gln Leu Leu Ala Asp Arg Ala Asp Val Asp Val Val Ala Met Thr
   20 25 30
   Arg Arg Lys Leu Pro Ala Glu Ala Leu Pro Pro Gly Val Glu Cys Ala
   35 40 45
   Val Ala Asp Tyr Ala Asp Pro Pro Ala Leu Arg Ala Ala Leu Lys Gly
   50 55 60
   Val Asp Thr Leu Val Leu Val Ser Ser Asp Gly Pro Asp Ala Arg Val
   65 70 75 80
   Leu Leu His His Arg Asn Val Val Ala Ala Val Val Ala Glu Arg Val
   85 90 95
   Gly His Val Ala Ala Leu Ser Ser Val Asp Ala Asp Ser Ala Ser Pro
   100 105 110
   Phe Cys Tyr Ala Val Val Asn Arg Leu Thr Glu Asp Leu Leu Leu Ala
   115 120 125
   Ser Gly Val Pro Cys Ser Phe Ala Arg Ala Ser Leu Tyr Ile Glu Phe
   130 135 140
   Phe Leu Gly Trp Leu Thr Gln Ala Arg Ser Thr Gly Leu Leu Arg Leu
   145 150 155 160
   Pro Ala Ala Asp Gly Arg Val Ser Leu Val Ala Arg Asp Asp Val Ala
   165 170 175
   Arg Ala Leu Ala Ala Leu Ala Val Gly Gly Pro Thr Gly Arg His His
   180 185 190
   Asp Ile Thr Gly Pro Glu Ser Thr Asp Leu Ala Gly Ile Ala Ser Thr
   195 200 205
   Ala Ala Glu Val Trp Arg Thr Pro Val Ala Tyr Val Asp Ile Pro Ala
   210 215 220
   Asp Thr Tyr Ser Ala Glu Thr Ala Ala Thr Gly Leu Asp Pro Trp Trp
   225 230 235 240
   Leu Tyr Ala Phe Ser Ser Leu Phe Ala Ser Val Arg Glu Gln Arg Trp
   245 250 255
   Asp Arg Val Arg Asp Asp Cys Ala Gln Leu Thr Gly Arg Leu Pro Arg
   260 265 270
   Thr Leu Arg Asp Val Leu Ser Glu Arg Ala
   275 280
<400> 118
<210> 119
   <211> 324
   <212> PRT
   <213> Streptomyces ambofaciens
<400> 119
   Met Leu Arg Arg Arg Leu Gly Gly Pro Ser Gly Pro Leu Val Ser Ala
   1               5                   10                  15
   Leu Cys Leu Gly Ala Met Pro Phe Gly Thr Thr Val Asp Glu Lys Thr
               20                  25                  30
   Ser Phe Ala Ile Leu Asp Arg Phe Val Glu Ala Gly Gly Ser Leu Val
           35                  40                  45
   Asp Thr Ala Asp Asn Tyr Ala Phe Trp Ala Pro Gly Gly Thr Gly Asp
       50                  55                  60
   Glu Ser Glu Asn Thr Val Gly Arg Trp Leu Ala Ser Arg Arg Arg Arg
   65                  70                  75                  80
   Asp Glu Val Val Ile Ser Thr Lys Val Gly Ala Arg Pro Thr Val Pro
                   85                  90                  95
   Gly Ser Gly Leu Glu Thr Ala Glu Gly Leu Ser Ala Pro Val Ile Arg
               100                 105                 110
   Lys Ala Ala Glu Asp Ser Leu Arg Arg Leu Gly Thr Asp Arg Ile Asp
           115                 120                 125
   Leu Tyr Trp Thr His Ile Glu Asp Arg Thr Val Pro Leu Glu Glu Thr
       130                 135                 140
   Leu Gly Ala Leu Asp Glu Leu Val Gly Gly Gly Lys Val Ala Val Leu
   145                 150                 155                 160
   Gly Cys Ser Asn His Ala Gly Trp Arg Ile Glu Arg Ala Arg Ala Leu
                   165                 170                 175
   Ala Arg Thr Asn Gly Trp Thr Ala Tyr Thr Cys Val Gln Gln Arg Tyr
   180 185 190
   Ser Tyr Leu Gln Pro Arg Phe Asp Val Gly Leu Pro Glu Ser Gly His
   195 200 205
   Val His Ala Thr Ser Glu Leu Leu Asp His Val Arg Ser Glu Pro Asp
   210 215 220
   Leu Thr Leu Leu Ala Tyr Ser Ser Leu Leu Ser Gly Ala Tyr Thr Arg
   225 230 235 240
   Pro Asp Lys Thr Leu Ser Ala Ala Tyr Asp His Pro Gly Thr Gly Gln
   245 250 255
   Arg Leu Thr Val Leu Arg Glu Val Ala Ala Glu Leu Gly Ala Thr Ala
   260 265 270
   Asn Gln Val Val Leu Ser Trp Leu Leu Gly Gly Asp Pro Pro Val Ile
   275 280 285
   Pro Ile Val Gly Val Ser Ser Val Glu Gln Leu Asp Glu Val Leu Ala
   290 295 300
   Ala Val Glu Leu Glu Leu Pro Arg Glu Thr Arg Ala Arg Leu Asp Leu
   305 310 315 320
   Ala Gly Arg Gly
<210> 120
   <211> 1227
   <212> DNA
   <213> Streptomyces ambofaciens
<220>
   <221> CDS
   <222> (1)..(1227)
<400> 120
   gtg acg ccc gag gcg gac gtc ggt acg cgg tgg gag ccg gag ttc gac 48
   Val Thr Pro Glu Ala Asp Val Gly Thr Arg Trp Glu Pro Glu Phe Asp
   1 5 10 15
   atg ctg ccc ggc ata ccc gac ctg cgg gcc ttt ccc cgc gga cgg tgg 96
   Met Leu Pro Gly Ile Pro Asp Leu Arg Ala Phe Pro Arg Gly Arg Trp
   20 25 30
   gcg gac gcg gtg cgg gag gcc acc ggc gca ctg acc tgg ggc gag ctc 144
   Ala Asp Ala Val Arg Glu Ala Thr Gly Ala Leu Thr Trp Gly Glu Leu
   35 40 45
   ggc tac ccc gat ccg gcc ggt ctc gcc cgg ctc cgg cag cgg ctc gcg 192
   Gly Tyr Pro Asp Pro Ala Gly Leu Ala Arg Leu Arg Gln Arg Leu Ala
   50 55 60
   gcc tac ctc gtg cgg gga cgc ggc gcg tcg ctg gaa gcc cgg cag ctc      240
   Ala Tyr Leu Val Arg Gly Arg Gly Ala Ser Leu Glu Ala Arg Gln Leu
   65                  70                  75                  80
   gtc gtc tgc gcg ggc acg ctg gac gcg gtg cgc agg ctg tgc cgg gtc      288
   Val Val Cys Ala Gly Thr Leu Asp Ala Val Arg Arg Leu Cys Arg Val
                   85                  90                  95
   ctg cgg acc gag ggc cac acc cac atg gcc ctg gag gac cct ggc tgg      336
   Leu Arg Thr Glu Gly His Thr His Met Ala Leu Glu Asp Pro Gly Trp
               100                 105                 110
   tcg cgg ctg agc gcc acg gtc ggg gcg gag ggg ctg gtg ccc gtc ccg      384
   Ser Arg Leu Ser Ala Thr Val Gly Ala Glu Gly Leu Val Pro Val Pro
           115                 120                 125
   gtt ccc gtg gac ggc gac ggc ctg cgg gtc gac ctg ctg cgg cgc gcg      432
   Val Pro Val Asp Gly Asp Gly Leu Arg Val Asp Leu Leu Arg Arg Ala
       130                 135                 140
   cct cag gtc cgg tcg gtg atc gtc gct ccc gcc cac cag ttc ccg acc      480
   Pro Gln Val Arg Ser Val Ile Val Ala Pro Ala His Gln Phe Pro Thr
   145                 150                 155                 160
   ggt gtg gtc ctc gcc ccg gcc cgt cgc ggc gag ctg gtc gcc tgg gcc      528
   Gly Val Val Leu Ala Pro Ala Arg Arg Gly Glu Leu Val Ala Trp Ala
                   165                 170                 175
   cgc gag gtc gac ggc ctg gtg ctg gag gac gac tac gac gcc gag ttc      576
   Arg Glu Val Asp Gly Leu Val Leu Glu Asp Asp Tyr Asp Ala Glu Phe
               180                 185                 190
   cga tac gac cgc cac ccc gtg ggc gcc ctg cag ggc atg gca ccg gag      624
   Arg Tyr Asp Arg His Pro Val Gly Ala Leu Gln Gly Met Ala Pro Glu
           195                 200                 205
   cac gtc gcg ctg ctg ggg tcg gtc agc aag acg ctc tcg ccg gcc ctg      672
   His Val Ala Leu Leu Gly Ser Val Ser Lys Thr Leu Ser Pro Ala Leu
       210                 215                 220
   cgg atc ggc tgg gcg gtg acc ccg ccg cgc tgg acc gag gcc ctg ggc      720
   Arg Ile Gly Trp Ala Val Thr Pro Pro Arg Trp Thr Glu Ala Leu Gly
   225                 230                 235                 240
   gcg gag cac gtc ggg ggc gta cca ccg ccc gtc atc gac cag gag gcg      768
   Ala Glu His Val Gly Gly Val Pro Pro Pro Val Ile Asp Gln Glu Ala
                   245                 250                 255
   ttc gcc cgg ttc ctg gcc cgc ggg tcc tac gac cgg tac ctg cgg gcg      816
   Phe Ala Arg Phe Leu Ala Arg Gly Ser Tyr Asp Arg Tyr Leu Arg Ala
               260                 265                 270
   gca cgg ctg cgc tac aaa cgg cgc cgc gat cac ctc atg gga gaa ctc      864
   Ala Arg Leu Arg Tyr Lys Arg Arg Arg Asp His Leu Met Gly Glu Leu
           275                 280                 285
   gcg gcg tcg ctg ccg ggg ttc cag gtg tcc ggt gcg gcg gcc ggc ttc      912
   Ala Ala Ser Leu Pro Gly Phe Gln Val Ser Gly Ala Ala Ala Gly Phe
       290                 295                 300
   cac ctg ctg ctg tcc ctg acg gac tgc tcg gcg ccc acc gtc gtc agg      960
   His Leu Leu Leu Ser Leu Thr Asp Cys Ser Ala Pro Thr Val Val Arg
   305                 310                 315                 320
   gag gcg gcg cgg cgt gac gtg agg ctg gcg gat ctc gac gcc tac cgg     1008
   Glu Ala Ala Arg Arg Asp Val Arg Leu Ala Asp Leu Asp Ala Tyr Arg
                   325                 330                 335
   atg gcg cgg gcc gag cac cac ccg acg gcc cgg gcc ggc gac tcc ggc     1056
   Met Ala Arg Ala Glu His His Pro Thr Ala Arg Ala Gly Asp Ser Gly
               340                 345                 350
   ggg gcg cgg gcc gtg gcc gcc gcc ggc ccc gcg ctg gtc ctc ggc tac     1104
   Gly Ala Arg Ala Val Ala Ala Ala Gly Pro Ala Leu Val Leu Gly Tyr
           355                 360                 365
   ggc aat ctg gcg gac cac gcc gtg ccc gga gcc gtg cgg cgc ctg gca     1152
   Gly Asn Leu Ala Asp His Ala Val Pro Gly Ala Val Arg Arg Leu Ala
       370                 375                 380
   cag gcc atc gac gcg gcc cgg cgc cac gac cgg cgg cac gtc gac gcc     1200
   Gln Ala Ile Asp Ala Ala Arg Arg His Asp Arg Arg His Val Asp Ala
   385                 390                 395                 400
   ggg cgg gcc ggg gag cgg gcc gac tga                                 1227
   Gly Arg Ala Gly Glu Arg Ala Asp
                   405
<210> 121
   <211> 408
   <212> PRT
   <213> Streptomyces ambofaciens
<400> 121
   Val Thr Pro Glu Ala Asp Val Gly Thr Arg Trp Glu Pro Glu Phe Asp
   1               5                   10                  15
   Met Leu Pro Gly Ile Pro Asp Leu Arg Ala Phe Pro Arg Gly Arg Trp
               20                  25                  30
   Ala Asp Ala Val Arg Glu Ala Thr Gly Ala Leu Thr Trp Gly Glu Leu
           35                  40                  45
   Gly Tyr Pro Asp Pro Ala Gly Leu Ala Arg Leu Arg Gln Arg Leu Ala
       50                  55                  60
   Ala Tyr Leu Val Arg Gly Arg Gly Ala Ser Leu Glu Ala Arg Gln Leu
   65                  70                  75                  80
   Val Val Cys Ala Gly Thr Leu Asp Ala Val Arg Arg Leu Cys Arg Val
                   85                  90                  95
   Leu Arg Thr Glu Gly His Thr His Met Ala Leu Glu Asp Pro Gly Trp
               100                 105                 110
   Ser Arg Leu Ser Ala Thr Val Gly Ala Glu Gly Leu Val Pro Val Pro
           115                 120                 125
   Val Pro Val Asp Gly Asp Gly Leu Arg Val Asp Leu Leu Arg Arg Ala
       130                 135                 140
   Pro Gln Val Arg Ser Val Ile Val Ala Pro Ala His Gln Phe Pro Thr
   145                 150                 155                 160
   Gly Val Val Leu Ala Pro Ala Arg Arg Gly Glu Leu Val Ala Trp Ala
                   165                 170                 175
   Arg Glu Val Asp Gly Leu Val Leu Glu Asp Asp Tyr Asp Ala Glu Phe
               180                 185                 190
   Arg Tyr Asp Arg His Pro Val Gly Ala Leu Gln Gly Met Ala Pro Glu
           195                 200                 205
   His Val Ala Leu Leu Gly Ser Val Ser Lys Thr Leu Ser Pro Ala Leu
       210                 215                 220
   Arg Ile Gly Trp Ala Val Thr Pro Pro Arg Trp Thr Glu Ala Leu Gly
   225                 230                 235                 240
   Ala Glu His Val Gly Gly Val Pro Pro Pro Val Ile Asp Gln Glu Ala
                   245                 250                 255
   Phe Ala Arg Phe Leu Ala Arg Gly Ser Tyr Asp Arg Tyr Leu Arg Ala
               260                 265                 270
   Ala Arg Leu Arg Tyr Lys Arg Arg Arg Asp His Leu Met Gly Glu Leu
           275                 280                 285
   Ala Ala Ser Leu Pro Gly Phe Gln Val Ser Gly Ala Ala Ala Gly Phe
       290                 295                 300
   His Leu Leu Leu Ser Leu Thr Asp Cys Ser Ala Pro Thr Val Val Arg
   305                 310                 315                 320
   Glu Ala Ala Arg Arg Asp Val Arg Leu Ala Asp Leu Asp Ala Tyr Arg
                   325                 330                 335
   Met Ala Arg Ala Glu His His Pro Thr Ala Arg Ala Gly Asp Ser Gly
               340                 345                 350
   Gly Ala Arg Ala Val Ala Ala Ala Gly Pro Ala Leu Val Leu Gly Tyr
           355                 360                 365
   Gly Asn Leu Ala Asp His Ala Val Pro Gly Ala Val Arg Arg Leu Ala
       370                 375                 380
   Gln Ala Ile Asp Ala Ala Arg Arg His Asp Arg Arg His Val Asp Ala
   385                 390                 395                 400
   Gly Arg Ala Gly Glu Arg Ala Asp
                   405
<210> 122
   <211> 46
   <212> DNA
   <213> Artificial
<220>
   <223> oligonucléotide EDR39
<400> 122
   cccaagcttg agaagggagc ggacattcat ggcccgcgcc gaacgc 46
<210> 123
   <211> 46
   <212> DNA
   <213> Artificial
<220>
   <223> oligonucléotide EDrt42
<400> 123
   cgggatccgg ctgaccatgg gagacgggcg catcgccgag ttcagc 46
<210> 124
   <211> 46
   <212> DNA
   <213> Artificial
<220>
   <223> Oligonucléotide EDR40
<400> 124
   cccaagcttg agaagggagc ggacattcaa tgctttggta aagcac 46
<210> 125
   <211> 38
   <212> DNA
   <213> Artificial
<220>
   <223> Oligonucléotide EDR41
<400> 125
   cccaagcttt caaggaacga cggggtggtc agtcaagt 38
<210> 126
   <211> 32
   <212> DNA
   <213> Artificial
<220>
   <223> oligonucléotide A
<400> 126
   ccagtagata tcccgccaac ccggagctgc ac 32
<210> 127
   <211> 32
   <212> DNA
   <213> Artificial
<220>
   <223> oligonucléotide B
<400> 127
   gaaaagatcc gtcatggggt cgtgcgctcc tt 32
<210> 128
   <211> 32
   <212> DNA
   <213> _{A}rtificial
<220>
   <223> oligonucléotide C
<400> 128
   cacgàcccca tgacggatct tttccgctgc at 32
<210> 129
   <211> 32
   <212> DNA
   <213> _{A}rtificial '
<220>
   <223> Oligonucléotide D
<400> 129
   gagccggata tcatcggtct tgccttgctc gt 32
<210> 130
   <211> 25
   <212> DNA
   <213> Artificial
<220>
   <223> Oligonucléotide ORF23c
<400> 130
   acgtgcgcgg tgagttcgcc gttgc 25
<210> 131
   <211> 25
   <212> DNA
   <213> Artificial
<220>
   <223> Oligonucléotide ORF25c
<400> 131
   ctgaacgacg ccatcgcggt ggtgc 25
<210> 132
   <211> 25
   <212> DNA
   <213> Artificial
<220>
   <223> Oligonucléotide ORF1*c
<400> 132
   gaccacctcg aaccgtccgg cgtca 25
<210> 133
   <211> 23
   <212> DNA
   <213> Artificial
<220>
   <223> Oligonucléotide ORF2*c
<400> 133
   ggcccggtcc agcgtgccga agc 23
<210> 134
   <211> 6174
   <212> DNA
   <213> Streptomyces ambofaciens
<220>
   <221> misc_feature
   <222> (3620)..(4069)
   <223> n is a, c, g, or t
<400> 134
   ctgcagaggg cgccgacgtg cgcggtgagt tcgccgttgc gccggctggt ccagccgagg 60
   ccgccgtgga ccgcgggcgc ctgtgcgcac agcaggcccc gggagccgag gtcgtgcagc 120
   aggccgaggg gcagctcgcc cgtccggtcc cactcggccg cccggtcgcc gaccagcgcg 180
   gtgaacagct cctcggcctc ggtgccgtcg gcgtgggagg tgtcagccac ctgcgtgccc 240
   ggtcgcctcg ccgggcgcgg ccagccgtcc gaccagccgg accatcgcgt cgacggtgcg 300
   gaagttgtcg agcatcaggt cggcgccgct gatgacgatg ccgtaggtct tctccaggtg 360
   cacgacgagc tgcatggcga acagcgagga catcccgccg acggcgaaca ggtcctggtc 420
   gcgctcccag gtggtcttgg tgcggtcctc gaggaacccg agcagttccc cggcgacctc 480
   gtcggcggtg ggggtcgtgc cggtggggtc gggccgaccg gacgtcgtgg tcatcgcgtg 540
   gcctcctggt agtcgtagaa tccccggccg ctcttgcggc cgagcaggcc ctggcggacc 600
   ttgtccagca gcagctcgct cgggcggagc gccggatcgc cggtccgttc gtgcatcacc 660
   cgcagcgagt cggccaggtt gtccagtccg atcaggtcgg ccgtggccag gggtccggtg 720
   cggtggccga tgcagtcgcg catcagcgcg tccacggtct ccggggtggc ccggccctcg 780
   tgcaccaccg cgatggcgtc gttcagcatc cggtgcagca ggcggctggt cacgaagccg 840
   gcgccgtcgc cgacgacgat gccccggcgg cccaggccgg acagcaggtc ccgggtggcc 900
   cgggcggccg cctctccgct gcgcggtccg aggaccacct cgaccgtggg gatcacgtac 960
   gcggggttca tgaagtgcac gccgacgagg tcctcggggc gggggacggc gtcggccagc 1020
   tcgtcgatgg ggacgcccga ggtgttgctg acgagcagcg tccccgggcg cgccacggac 1080
   gccaggtccg ccagcacctc ggccttccgc tcggggtcct cggtgacggc ctcgatcacg 1140
   gcggtcgcgg tggcgacggc ggccggcgcc tcctcgacgg tcagctcccc gggcgggcgg 1200
   tcgtggggca gcgcgcccat cagccgggcc gtccgcagat gcagcgcgac cgcgtcgggg 1260
   gcggccgcgc gcgccccggc ggaggtgtcg accagtgtga ccgggtgccc gtgtccgacg 1320
   gcgagtgccg cgatggccgt gcccatgacg cccgcgccga gcacgacgag cggagaattt 1380
   tccttggaat cgggcacgga tcctccagaa tcccgggggc ggcggctgtg cgaaatgctg 1440
   tcccgaatcg cttccgcgct ccatcacccg ggcgtgactt tgtcacctag caagcggtgg 1500
   gaggccgggc ggaggctatg tcatctcggc cttgtccgct tacgagttcc gtccttaaag 1560
   tgcgcccggc gaacgcacgg cggaattgac tcgccgtccg tcaaggaccc gcggttcgaa 1620
   ggacctcgac gcatgaaggg tttcacatgg ctcatatcgc attcttcatc cttccggttg 1680
   ccgggcatgt gaatccgacc ctgggagtcg ccgaggaact ggtcgcgcgc ggccaccggg 1740
   tgacgttcgc gctgtccgag gacctcgccg agcgggcccg gctgatcggc gccgaggtgg 1800
   tcacctatcc ggtggacagg caacggttcc tggaccagat ggtgccgcgg caggacgcgg 1860
   acgagtacac ggacgaggac gagttcgtcc gggtcctgga gtggctgctg gacatgacgg 1920
   tgcagaccat ggaaccgctg gagaggcact tcgccgggga ccggcccgac gtcgtcgtca 1980
   acgatccgtc gtcgctgtgg acgggacggc tgctggcgga ccggtggggc atcccggtca 2040
   tccgcagcac tccgacctat gccgccaacg aacactggtc gctgcatccg ccggtcgact 2100
   cggccgagcc gccggacgac cccgagctgc acaagctgct cgcgcggatc gagcggctgc 2160
   tggaggagca gggcgtcgag cacgacctgg ccggcttcac cggggtcctg cacggcggtc 2220
   cggccctgct gtacatgccc cgctcgttcc agtacgcggg cgagaccttc gacgcacagc 2280
   accacttcgt cggcccctgc ccgccccgca ccgcgttcca cggcgagtgg aggccggggg 2340
   acgacgacgg ccggcccctg gtgctggtga gtctcggaac cctgtacaac gaccggccgg 2400
   acttcttccg cacctgcctg gaggcgttcg gcgacgagcc ctggaacgtg cttctggtgc 2460
   tgggcggcgg ggtgcccgcg gccgacctgg gcccgcttcc cggcaatgtc cgggtgaccg 2520
   acttcgtgtc gctgcgcgac gtcctgccgc acacggcggt ggtggtgaac cacggtggga 2580
   tgagcaccgc catggaggtg ttctcgcacg gtgtgccggt ggtggcgatc ccggtgatgc 2640
   cggagccccg ggccaccgcg cggcggatcg tcgaactggg cctgggcgac cagctgctga 2700
   actcggagct gacggccgag tccctgcgtg ccacggtacg gcgggtgctg gcggactccg 2760
   cgatcccggg gaacatgcgc gggttccggg agcagatcag ggcggccggc ggggcgcccg 2820
   cggcggccga cgcgatcgag ggactgctgc cccgggtggg ctgagacgtc cgcgcccgac 2880
   acgcgttcac cttccgaacg gcgggatcgc cccatgctca tcaccgaaac agcagtgccc 2940
   gacgtgttcc gcatcgatcc ggaaccgctg ccggaccacc ggggccggtt ctacgaagcg 3000
   gtgcgccgcg ggccgctgga ggccgccgtc gggcactcgg tcgaggtccg gcaggtccac 3060
   tgcaccgtct ccgggcgcaa cgtactgcgc ggcctgcacg ccaccaccct gccgccgggc 3120
   caggccaaga tcctcacctg tgtgcggggt gcggcgctga ctatggtggt cgacatgagg 3180
   gtcgggtcac cgggcttcgg acggtacgag gcggtgcggc aggatgcccc gtcgggcacc 3240
   gcgctctacc tgccggacgg catcggcctg ggctacgtgg ccctcgcgga cgacacatgc 3300
   atgaactacc tgtgcaccga ggagtacgtc ccgggcatgg tcatcgacgt ggacgccctg 3360
   gaccccgaac tcggcctgcc gtggggactg accggggatc ccgtccgctc cgcccgggac 3420
   gcggcggcgc cgtccctgcg ggcggccgcg gcggcgggaa ttctgcccac gtacgaggac 3480
   tgcttacggg tgcgcacgtc cgtgcccgcc cctcccgacc ggactcggct ctgacgcgac 3540
   ggacacgacc gccgcgcatc cgacgcgaat ccgacgcgaa tccgaactcg atttcccgaa 3600
   ccgtggacgg agcgcgtccn nnnnnnnnnn nnnnnnnnnn nnnnnnnnnn nnnnnnnnnn 3660
   nnnnnnnnnn nnnnnnnnnn nnnnnnnnnn nnnnnnnnnn nnnnnnnnnn nnnnnnnnnn 3720
   nnnnnnnnnn nnnnnnnnnn nnnnnnnnnn nnnnnnnnnn nnnnnnnnnn nnnnnnnnnn 3780
   nnnnnnnnnn nnnnnnnnnn nnnnnnnnnn nnnnnnnnnn nnnnnnnnnn nnnnnnnnnn 3840
   nnnnnnnnnn nnnnnnnnnn nnnnnnnnnn nnnnnnnnnn nnnnnnnnnn nnnnnnnnnn 3900
   nnnnnnnnnn nnnnnnnnnn nnnnnnnnnn nnnnnnnnnn nnnnnnnnnn nnnnnnnnnn 3960
   nnnnnnnnnn nnnnnnnnnn nnnnnnnnnn nnnnnnnnnn nnnnnnnnnn nnnnnnnnnn 4020
   nnnnnnnnnn nnnnnnnnnn nnnnnnnnnn nnnnnnnnnn nnnnnnnnnc cgccacaccc 4080
   cccagcagcg tcagcgtccg cctcgggacc acacccggca ggacgacggg gtgcggggcg 4140
   acgaggctga gggagccccg caactccacg gggcggctca accgcttgag ctgctccatc 4200
   aactgctggg ccgaggtggt ccccgtgtgg tggtccaccg ggccgcggcc gctcgccgag 4260
   gccagccgga ggttcccgcc cacccgcacc atgaagggca ggcccgcgag accgagccct 4320
   cgcaccagtc gtggcagcac ggccgcgcgt gcgtccatca ccacggggcg ggccaaggag 4380
   cggttggcct gagcggcttc ggccaccagg cggacgatgt tctcctcctc accgggtgcg 4440
   ccggggtgcc ggggtgtgcc ctccccgctc cggccgccgc ccagcgtcag gcgccagctg 4500
   accggggcgg ccgtcgtgcc cgaggccagc cacagcccgt gactctgctg gcagctcacc 4560
   acccggccca ggtcggagac gaagcgccgg ctcaccccga cggagcgcac ccccgccttg 4620
   gagaccacca tcggccagat cacccaggcg tcgggggtca gcctgtcgtc cacgtagcgg 4680
   gccagcgcgg cccgcacctc gccccagtcc caggtcgacc cggcgacgaa gtggtgcagg 4740
   ctctgttccg ccgcgccctc tccgacgaag gcggcgaggt tccgggcggt cttgcgtccc 4800
   tgtgccgtga gcagcccgcg tatgtactgc tcacctcttc tgcgctggtc cgcccggcgg 4860
   agcgaaccga gtaactccgc acatgcgtcg gagacgagcg agtcgaagtc gtgccgcgcg 4920
   gcgggaccca cggggggcgc cgcgccggcg ggtcgaagcg tgcgtaaggt cataggagtc 4980
   ctcgtggggg cctcgtcatc actgcgagtg gactgcgacc agcatcgcca attcacccgt 5040
   tgctccccag gtacaccggg cacactcgtt ccgcttcgct cccccggcgg cacggcccgc 5100
   gtgtggagca ctccccgctc tccggacggt gggggggaag cccaccgcac gcgcgccggt 5160
   agcggtgccg gggccccaca acccctcacc gacggggtcc gttcgaccac atgccgtgat 5220
   gatgccttcg ccgcacggcc tccttgaatg acggaccgtc agaaagacgt cactccgcgc 5280
   gcgcccgctc ccgccccgac cccgcccggc cgccgtcccg cccccgcgac cctgtgggcc 5340
   gacgccctct tgacggaccc ggaatcccgg gccgcgggac gggacggcga ggaactcgat 5400
   gccgcaaagg ttccgcgtcc tcaccggcac ctccgagatc cagcgcaacg gcatcgccaa 5460
   gctcctggct ttccaccact gagccgcacc ccctggcgag cagatccggg acaagacgcc 5520
   accttcgcga cagtgtttag gaaaagttaa gtaaagaatt ccgcgagcgg attgccaggg 5580
   agaacaaccc attgacgcgc accggtgcag cggccacatt gacggcacct gtcaagttca 5640
   cccccaggag cttggaatcc catgcaggca attcggcatc acgtcatgct cgtcatggcc 5700
   ttcgtgaccg tcgccacgac tttccttctc tggccgtcga cgcaatccgc gcaagcgttt 5760
   cccccgaccc cgaagcagac ggtactgaac cacctccgcg ccatttccgg gaatcacatc 5820
   gtctccggac agcacaacaa ggagcccgcc tccgccccgg gccagtacac ccagcaggtc 5880
   aaggacgtca ccgggcagta ccccggcctg tggggcggtg acctgatgtt cgccgcggcg 5940
   gacgtggccg gccgccagcg cgtcgtcgac caggccagga ccgagtgggc gaacggatcg 6000
   ctggtctcgc tcacctggca cgtctgcccg ccgaccggcg gcagcacctg tgcgttcgag 6060
   ggcggcgtca agtccacgct gacgaacgcg cagttctcgc aggtcctcac ggagggcagt 6120
   gccctgaaca gcgcatggaa gcggcgcctg gacgaggtcg tcccgtacct gcag 6174
<210> 135
   <211> 4770
   <212> DNA
   <213> Streptomyces ambofaciens
<400> 135
   gaattccgcg agcggattgc cagggagaac aacccattga cgcgcaccgg tgcagcggcc 60
   acattgacgg cacctgtcaa gttcaccccc aggagcttgg aatcccatgc aggcaattcg 120
   gcatcacgtc atgctcgtca tggccttcgt gaccgtcgcc acgactttcc ttctctggcc 180
   gtcgacgcaa tccgcgcaag cgtttccccc gaccccgaag cagacggtac tgaaccacct 240
   ccgcgccatt tccgggaatc acatcgtctc cggacagcac aacaaggagc ccgcctccgc 300
   cccgggccag tacacccagc aggtcaagga cgtcaccggg cagtaccccg gcctgtgggg 360
   cggtgacctg atgttcgccg cggcggacgt ggccggccgc cagcgcgtcg tcgaccaggc 420
   caggaccgag tgggcgaacg gatcgctggt ctcgctcacc tggcacgtct gcccgccgac 480
   cggcggcagc acctgtgcgt tcgagggcgg cgtcaagtcc acgctgacga acgcgcagtt 540
   ctcgcaggtc ctcacggagg gcagtgccct gaacagcgca tggaagcggc gcctggacga 600
   ggtcgtcccg tacctgcagc agctggagaa cgcgggcgtc cccgtcctct tccggccgct 660
   gcacgagatg aacgaatcct ggaactggtg gggaaaccgg cccggagcga acggcagcgc 720
   acgcctctac cagatcaccc gcgatcacct cgccgggacg aaagggctgg acaatctgat 780
   ctgggtctgg aacgtccagg acaatccggc gggaaactgg aacagctact atccgggaga 840
   tcagtacgtg gacgtcgttt cgctggacgt ctggtacaag agccacccga gttccgccga 900
   ctaccagcag atgcggagca tcgcgggaac aaaacccatg gccctcgcgg agctgggcaa 960
   aatgccgacc gccgcgctgc tggacagcca gacgcggtgg acatggttca tgatgtggtc 1020
   cgagcatctg cgcgggaaca attccaacgc cgaaatacag acggcgtatt tccacccccg 1080
   tgtactgaac cagggggagg tcgcactgcc ctgacgctcg gcgctgcccg gctctctcac 1140
   gcgcgttctg acaggacgtc gcggagagtg cggggcaagc ggccggtgag ctgggcgcag 1200
   tcgtcgcgga cccgatccca gcgctgttcg cgtaccgagg cgaacaggga ggagaaggcg 1260
   tagagccacc agggatccag gccggtcgcc gctgtttcgg cagagtacgt gtcggccggg 1320
   atgtccacgt aggcgaccgg tgtccgccac acctccgccg ccgtggaggc gatgcctgcc 1380
   aggtccgtcg actcgggtcc cgtgatgtcg tggtgacggc cggtcggccc gcccaccgcg 1440
   agggcggcga gggcgcgggc cacgtcgtcg cgcgccacca gggacacccg gccatcggct 1500
   gccggcagcc tcagcagccc ggtcgagcgc gcctgggtga gccagcctag gaagaactcg 1560
   atgtacagcg aggccctggc gaacgagcac ggcacgccgg aggcgagcag cagatcctcg 1620
   gtgagccggt tgacgacggc gtagcagaac ggggaggccg agtcggcgtc gacgctgctc 1680
   agtgccgcga catggccgac gcgctcggcc accacggcgg cgacgacgtt gcggtggtgc 1740
   agcagcaccc gtgcgtcggg cccgtcgctg gagacgagga ccagagtgtc cacgcccttc 1800
   agcgccgcac gcagagcggg cgggtcggcg tagtcggcga cggcgcactc cacccccggc 1860
   ggtagtgcct cggcaggcag cttccgcctg gtcatcgcca cgacgtccac gtcggcccgg 1920
   tccgcgagca gctgcaccac ccgcctgccc agactgcccg ccgcccctgt caccgcgata 1980
   cgcatcgtcg cccccgtcgc cttgtcgtcg gtcgtaccac cgtagggggc caaccgcgac 2040
   cagggcttgg aacgagccgg cccgccaggg cacagacgcg cgatcggtcc ggttttcccg 2100
   tgctcttttg gaccgggacg ccggaccgct tcctttctac ggtggagccg ttcccgcccg 2160
   agcccgcacg tcatcgacgt gcggggaaga cagaggtgat accgatgctc cgacgccgtc 2220
   tcggagggcc gtcaggcccc ctcgtcagtg ccctgtgcct gggcgcgatg cccttcggca 2280
   ccaccgtcga cgagaagacg tccttcgcca tcctcgaccg gttcgtcgag gccggcggca 2340
   gtctcgtcga caccgccgac aactacgcgt tctgggctcc cggcgggacc ggggacgaga 2400
   gcgagaacac cgtcgggcgc tggctggcga gccgccgccg ccgcgacgag gtggtgatct 2460
   ccaccaaggt gggtgcccgc cccaccgtcc ccggcagcgg cctggagacc gccgaagggc 2520
   tgtcggctcc cgtcatacgg aaggccgcgg aggacagcct gcgacgcctg ggcaccgatc 2580
   gcatcgatct gtactggacc cacatcgagg accggaccgt ccctctggag gagacgctcg 2640
   gagctctcga cgagctggtc ggcggcggca aggtggcggt gctgggctgc tccaaccacg 2700
   cgggctggcg catcgaacgg gcccgcgcgc tcgcccggac caacggctgg acggcgtaca 2760
   cctgcgtcca gcagcgctac tcctaccttc agccgcgctt cgacgtcgga ctgccggaga 2820
   gcggacacgt ccacgcgacc tccgaactcc tcgaccacgt acgcagcgag cccgacctga 2880
   cgctgctggc ctactcgtcc ctgctctcgg gcgcgtacac ccggccggac aagaccctgt 2940
   ccgccgccta cgaccacccg ggcaccgggc agcggctgac cgtgctgcgg gaggtggccg 3000
   ccgagctcgg tgccaccgcc aaccaggtgg tgctgtcctg gttgctcgga ggtgatccgc 3060
   cggtgatccc gatcgtgggg gtgagttccg tcgagcagct ggacgaggtg ctggccgccg 3120
   tcgagctgga gctgccccgg gagacgaggg cacggctgga cctcgccggg cggggctgac 3180
   gggccacaca ccgcccctcg cccgggacgg gctgaccggc cgcacaccgt ccctcagccg 3240
   ggacgggctg acggccacac acccgcccct cagcccggct gggctgggct gacggccaca 3300
   cacccgctcc tcagcccggc tgggctgggc tgacggccac acacccgccc ctcagcccgg 3360
   ctgggctggg ctgacggcca cacgccgtcc ctcgcccggg tcgggctgac cagccacacg 3420
   ccgtccctca gtcggcccgc tccccggccc gcccggcgtc gacgtgccgc cggtcgtggc 3480
   gccgggccgc gtcgatggcc tgtgccaggc gccgcacggc tccgggcacg gcgtggtccg 3540
   ccagattgcc gtagccgagg accagcgcgg ggccggcggc ggccacggcc cgcgccccgc 3600
   cggagtcgcc ggcccgggcc gtcgggtggt gctcggcccg cgccatccgg taggcgtcga 3660
   gatccgccag cctcacgtca cgccgcgccg cctccctgac gacggtgggc gccgagcagt 3720
   ccgtcaggga cagcagcagg tggaagccgg ccgccgcacc ggacacctgg aaccccggca 3780
   gcgacgccgc gagttctccc atgaggtgat cgcggcgccg tttgtagcgc agccgtgccg 3840
   cccgcaggta ccggtcgtag gacccgcggg ccaggaaccg ggcgaacgcc tcctggtcga 3900
   tgacgggcgg tggtacgccc ccgacgtgct ccgcgcccag ggcctcggtc cagcgcggcg 3960
   gggtcaccgc ccagccgatc cgcagggccg gcgagagcgt cttgctgacc gaccccagca 4020
   gcgcgacgtg ctccggtgcc atgccctgca gggcgcccac ggggtggcgg tcgtatcgga 4080
   actcggcgtc gtagtcgtcc tccagcacca ggccgtcgac ctcgcgggcc caggcgacca 4140
   gctcgccgcg acgggccggg gcgaggacca caccggtcgg gaactggtgg gcgggagcga 4200
   cgatcaccga ccggacctga ggcgcgcgcc gcagcaggtc gacccgcagg ccgtcgccgt 4260
   ccacgggaac cgggacgggc accagcccct ccgccccgac cgtggcgctc agccgcgacc 4320
   agccagggtc ctccagggcc atgtgggtgt ggccctcggt ccgcaggacc cggcacagcc 4380
   tgcgcaccgc gtccagcgtg cccgcgcaga cgacgagctg ccgggcttcc agcgacgcgc 4440
   cgcgtccccg cacgaggtag gccgcgagcc gctgccggag ccgggcgaga ccggccggat 4500
   cggggtagcc gagctcgccc caggtcagtg cgccggtggc ctcccgcacc gcgtccgccc 4560
   accgtccgcg gggaaaggcc cgcaggtcgg gtatgccggg cagcatgtcg aactccggct 4620
   cccaccgcgt accgacgtcc gcctcgggcg tcaccggagc ggggacggcc tgcgccctga 4680
   cccgggtggc cgagccgctg cgcgcctcca ggtacccctc cgcgacgagc tgctcgtagg 4740
   ggggatcctc tagagtcgac ctgcaggcct 4770
<210> 136
   <211> 28
   <212> DNA
   <213> Artificial
<220>
   <223> oligonucléotide EDR31
<400> 136
   cccaagcttc tgcgcccgcg ggcgtgaa 28
<210> 137
   <211> 28
   <212> DNA
   <213> Artificial
<220>
   <223> oligonucléotide EDR37
<400> 137
   gctctagaac cgtgtagccg cgccccgg 28
<210> 138
   <211> 30
   <212> DNA
   <213> Artificial
<220>
   <223> Oligonucléotide KF30
<400> 138
   aagcttgtgt gcccggtgta cctggggagc 30
<210> 139
   <211> 30
   <212> DNA
   <213> Artificial
<220>
   <223> Oligonucléotide KF31
<400> 139
   ggatcccgcg acggacacga ccgccgcgca 30
<210> 140
   <211> 12134
   <212> DNA
   <213> Streptomyces ambofaciens
<400> 140
   ctgcagaggg cgccgacgtg cgcggtgagt tcgccgttgc gccggctggt ccagccgagg     60
   ccgccgtgga ccgcgggcgc ctgtgcgcac agcaggcccc gggagccgag gtcgtgcagc    120
   aggccgaggg gcagctcgcc cgtccggtcc cactcggccg cccggtcgcc gaccagcgcg    180
   gtgaacagct cctcggcctc ggtgccgtcg gcgtgggagg tgtcagccac ctgcgtgccc    240
   ggtcgcctcg ccgggcgcgg ccagccgtcc gaccagccgg accatcgcgt cgacggtgcg    300
   gaagttgtcg agcatcaggt cggcgccgct gatgacgatg ccgtaggtct tctccaggtg    360
   cacgacgagc tgcatggcga acagcgagga catcccgccg acggcgaaca ggtcctggtc    420
   gcgctcccag gtggtcttgg tgcggtcctc gaggaacccg agcagttccc cggcgacctc    480
   gtcggcggtg ggggtcgtgc cggtggggtc gggccgaccg gacgtcgtgg tcatcgcgtg    540
   gcctcctggt agtcgtagaa tccccggccg ctcttgcggc cgagcaggcc ctggcggacc    600
   ttgtccagca gcagctcgct cgggcggagc gccggatcgc cggtccgttc gtgcatcacc    660
   cgcagcgagt cggccaggtt gtccagtccg atcaggtcgg ccgtggccag gggtccggtg    720
   cggtggccga tgcagtcgcg catcagcgcg tccacggtct ccggggtggc ccggccctcg    780
   tgcaccaccg cgatggcgtc gttcagcatc cggtgcagca ggcggctggt cacgaagccg    840
   gcgccgtcgc cgacgacgat gccccggcgg cccaggccgg acagcaggtc ccgggtggcc    900
   cgggcggccg cctctccgct gcgcggtccg aggaccacct cgaccgtggg gatcacgtac    960
   gcggggttca tgaagtgcac gccgacgagg tcctcggggc gggggacggc gtcggccagc 1020
   tcgtcgatgg ggacgcccga ggtgttgctg acgagcagcg tccccgggcg cgccacggac 1080
   gccaggtccg ccagcacctc ggccttccgc tcggggtcct cggtgacggc ctcgatcacg 1140
   gcggtcgcgg tggcgacggc ggccggcgcc tcctcgacgg tcagctcccc gggcgggcgg 1200
   tcgtggggca gcgcgcccat cagccgggcc gtccgcagat gcagcgcgac cgcgtcgggg 1260
   gcggccgcgc gcgccccggc ggaggtgtcg accagtgtga ccgggtgccc gtgtccgacg 1320
   gcgagtgccg cgatggccgt gcccatgacg cccgcgccga gcacgacgag cggagaattt 1380
   tccttggaat cgggcacgga tcctccagaa tcccgggggc ggcggctgtg cgaaatgctg 1440
   tcccgaatcg cttccgcgct ccatcacccg ggcgtgactt tgtcacctag caagcggtgg 1500
   gaggccgggc ggaggctatg tcatctcggc cttgtccgct tacgagttcc gtccttaaag 1560
   tgcgcccggc gaacgcacgg cggaattgac tcgccgtccg tcaaggaccc gcggttcgaa 1620
   ggacctcgac gcatgaaggg tttcacatgg ctcatatcgc attcttcatc cttccggttg 1680
   ccgggcatgt gaatccgacc ctgggagtcg ccgaggaact ggtcgcgcgc ggccaccggg 1740
   tgacgttcgc gctgtccgag gacctcgccg agcgggcccg gctgatcggc gccgaggtgg 1800
   tcacctatcc ggtggacagg caacggttcc tggaccagat ggtgccgcgg caggacgcgg 1860
   acgagtacac ggacgaggac gagttcgtcc gggtcctgga gtggctgctg gacatgacgg 1920
   tgcagaccat ggaaccgctg gagaggcact tcgccgggga ccggcccgac gtcgtcgtca 1980
   acgatccgtc gtcgctgtgg acgggacggc tgctggcgga ccggtggggc atcccggtca 2040
   tccgcagcac tccgacctat gccgccaacg aacactggtc gctgcatccg ccggtcgact 2100
   cggccgagcc gccggacgac cccgagctgc acaagctgct cgcgcggatc gagcggctgc 2160
   tggaggagca gggcgtcgag cacgacctgg ccggcttcac cggggtcctg cacggcggtc 2220
   cggccctgct gtacatgccc cgctcgttcc agtacgcggg cgagaccttc gacgcacagc 2280
   accacttcgt cggcccctgc ccgccccgca ccgcgttcca cggcgagtgg aggccggggg 2340
   acgacgacgg ccggcccctg gtgctggtga gtctcggaac cctgtacaac gaccggccgg 2400
   acttcttccg cacctgcctg gaggcgttcg gcgacgagcc ctggaacgtg cttctggtgc 2460
   tgggcggcgg ggtgcccgcg gccgacctgg gcccgcttcc cggcaatgtc cgggtgaccg 2520
   acttcgtgtc gctgcgcgac gtcctgccgc acacggcggt ggtggtgaac cacggtggga 2580
   tgagcaccgc catggaggtg ttctcgcacg gtgtgccggt ggtggcgatc ccggtgatgc 2640
   cggagccccg ggccaccgcg cggcggatcg tcgaactggg cctgggcgac cagctgctga 2700
   actcggagct gacggccgag tccctgcgtg ccacggtacg gcgggtgctg gcggactccg 2760
   cgatcccggg gaacatgcgc gggttccggg agcagatcag ggcggccggc ggggcgcccg 2820
   cggcggccga cgcgatcgag ggactgctgc cccgggtggg ctgagacgtc cgcgcccgac 2880
   acgcgttcac cttccgaacg gcgggatcgc cccatgctca tcaccgaaac agcagtgccc 2940
   gacgtgttcc gcatcgatcc ggaaccgctg ccggaccacc ggggccggtt ctacgaagcg 3000
   gtgcgccgcg ggccgctgga ggccgccgtc gggcactcgg tcgaggtccg gcaggtccac 3060
   tgcaccgtct ccgggcgcaa cgtactgcgc ggcctgcacg ccaccaccct gccgccgggc 3120
   caggccaaga tcctcacctg tgtgcggggt gcggcgctga ctatggtggt cgacatgagg 3180
   gtcgggtcac cgggcttcgg acggtacgag gcggtgcggc aggatgcccc gtcgggcacc 3240
   gcgctctacc tgccggacgg catcggcctg ggctacgtgg ccctcgcgga cgacacatgc 3300
   atgaactacc tgtgcaccga ggagtacgtc ccgggcatgg tcatcgacgt ggacgccctg 3360
   gaccccgaac tcggcctgcc gtggggactg accggggatc ccgtccgctc cgcccgggac 3420
   gcggcggcgc cgtccctgcg ggcggccgcg gcggcgggaa ttctgcccac gtacgaggac 3480
   tgcttacggg tgcgcacgtc cgtgcccgcc cctcccgacc ggactcggct ctgacgcgac 3540
   ggacacgacc gccgcgcatc cgacgcgaat ccgacgcgaa tccgaactcg atttcccgaa 3600
   ccgtggacgg agcgcgccga ggagcggaaa ggtctgccgc agaaacaggc tgcggccctt 3660
   tctttccgct ttttccggta ccgggacggc cgttccgatg aaagcgaacg tccgcatgcg 3720
   acggccgtcc ctcccgtacg ctcctccccc gtcgcggccg gccgggcggg cgggccggcc 3780
   gaccgcgacg gggcggggcg gggtcacacc gccgtcgccc caccgaggtc gccgcgcgcg 3840
   gagggctgcg ccagccgcag cgtgtgggcg atggacgcca gggtcacgtg ccggtgccag 3900
   ccctggaacg accgtccttc gaagtcgcgc atgcccacgc ccacgctcac ccggtcgaag 3960
   tcggcgtcga cctgttcggt gagcatcgcc agccgcagca gggcgccgcg gtcccaggac 4020
   gtgaggtcgg tcagccacag gtcggcgggg cgccggcggt tgccccgcca cacccccagc 4080
   agcgtcagcg tccgcctcgg gaccacaccc ggcaggacga cggggtgcgg ggcgacgagg 4140
   ctgagggagc cccgcaactc cacggggcgg ctcaaccgct tgagctgctc catcaactgc 4200
   tgggccgagg tggtccccgt gtggtggtcc accgggccgc ggccgctcgc cgaggccagc 4260
   cggaggttcc cgcccacccg caccatgaag ggcaggcccg cgagaccgag ccctcgcacc 4320
   agtcgtggca gcacggccgc gcgtgcgtcc atcaccacgg ggcgggccaa ggagcggttg 4380
   gcctgagcgg cttcggccac caggcggacg atgttctcct cctcaccggg tgcgccgggg 4440
   tgccggggtg tgccctcccc gctccggccg ccgcccagcg tcaggcgcca gctgaccggg 4500
   gcggccgtcg tgcccgaggc cagccacagc ccgtgactct gctggcagct caccacccgg 4560
   cccaggtcgg agacgaagcg ccggctcacc ccgacggagc gcacccccgc cttggagacc 4620
   accatcggcc agatcaccca ggcgtcgggg gtcagcctgt cgtccacgta gcgggccagc 4680
   gcggcccgca cctcgcccca gtcccaggtc gacccggcga cgaagtggtg caggctctgt 4740
   tccgccgcgc cctctccgac gaaggcggcg aggttccggg cggtcttgcg tccctgtgcc 4800
   gtgagcagcc cgcgtatgta ctgctcacct cttctgcgct ggtccgcccg gcggagcgaa 4860
   ccgagtaact ccgcacatgc gtcggagacg agcgagtcga agtcgtgccg cgcggcggga 4920
   cccacggggg gcgccgcgcc ggcgggtcga agcgtgcgta aggtcatagg agtcctcgtg 4980
   ggggcctcgt catcactgcg agtggactgc gaccagcatc gccaattcac ccgttgctcc 5040
   ccaggtacac cgggcacact cgttccgctt cgctcccccg gcggcacggc ccgcgtgtgg 5100
   agcactcccc gctctccgga cggtgggggg gaagcccacc gcacgcgcgc cggtagcggt 5160
   gccggggccc cacaacccct caccgacggg gtccgttcga ccacatgccg tgatgatgcc 5220
   ttcgccgcac ggcctccttg aatgacggac cgtcagaaag acgtcactcc gcgcgcgccc 5280
   gctcccgccc cgaccccgcc cggccgccgt cccgcccccg cgaccctgtg ggccgacgcc 5340
   ctcttgacgg acccggaatc ccgggccgcg ggacgggacg gcgaggaact cgatgccgca 5400
   aaggttccgc gtcctcaccg gcacctccga gatccagcgc aacggcatcg ccaagctcct 5460
   ggctttccac cactgagccg caccccctgg cgagcagatc cgggacaaga cgccaccttc 5520
   gcgacagtgt ttaggaaaag ttaagtaaag aattccgcga gcggattgcc agggagaaca 5580
   acccattgac gcgcaccggt gcagcggcca cattgacggc acctgtcaag ttcaccccca 5640
   ggagcttgga atcccatgca ggcaattcgg catcacgtca tgctcgtcat ggccttcgtg 5700
   accgtcgcca cgactttcct tctctggccg tcgacgcaat ccgcgcaagc gtttcccccg 5760
   accccgaagc agacggtact gaaccacctc cgcgccattt ccgggaatca catcgtctcc 5820
   ggacagcaca acaaggagcc cgcctccgcc ccgggccagt acacccagca ggtcaaggac 5880
   gtcaccgggc agtaccccgg cctgtggggc ggtgacctga tgttcgccgc ggcggacgtg 5940
   gccggccgcc agcgcgtcgt cgaccaggcc aggaccgagt gggcgaacgg atcgctggtc 6000
   tcgctcacct ggcacgtctg cccgccgacc ggcggcagca cctgtgcgtt cgagggcggc 6060
   gtcaagtcca cgctgacgaa cgcgcagttc tcgcaggtcc tcacggaggg cagtgccctg 6120
   aacagcgcat ggaagcggcg cctggacgag gtcgtcccgt acctgcagca gctggagaac 6180
   gcgggcgtcc ccgtcctctt ccggccgctg cacgagatga acgaatcctg gaactggtgg 6240
   ggaaaccggc ccggagcgaa cggcagcgca cgcctctacc agatcacccg cgatcacctc 6300
   gccgggacga aagggctgga caatctgatc tgggtctgga acgtccagga caatccggcg 6360
   ggaaactgga acagctacta tccgggagat cagtacgtgg acgtcgtttc gctggacgtc 6420
   tggtacaaga gccacccgag ttccgccgac taccagcaga tgcggagcat cgcgggaaca 6480
   aaacccatgg ccctcgcgga gctgggcaaa atgccgaccg ccgcgctgct ggacagccag 6540
   acgcggtgga catggttcat gatgtggtcc gagcatctgc gcgggaacaa ttccaacgcc 6600
   gaaatacaga cggcgtattt ccacccccgt gtactgaacc agggggaggt cgcactgccc 6660
   tgacgctcgg cgctgcccgg ctctctcacg cgcgttctga caggacgtcg cggagagtgc 6720
   ggggcaagcg gccggtgagc tgggcgcagt cgtcgcggac ccgatcccag cgctgttcgc 6780
   gtaccgaggc gaacagggag gagaaggcgt agagccacca gggatccagg ccggtcgccg 6840
   ctgtttcggc agagtacgtg tcggccggga tgtccacgta ggcgaccggt gtccgccaca 6900
   cctccgccgc cgtggaggcg atgcctgcca ggtccgtcga ctcgggtccc gtgatgtcgt 6960
   ggtgacggcc ggtcggcccg cccaccgcga gggcggcgag ggcgcgggcc acgtcgtcgc 7020
   gcgccaccag ggacacccgg ccatcggctg ccggcagcct cagcagcccg gtcgagcgcg 7080
   cctgggtgag ccagcctagg aagaactcga tgtacagcga ggccctggcg aacgagcacg 7140
   gcacgccgga ggcgagcagc agatcctcgg tgagccggtt gacgacggcg tagcagaacg 7200
   gggaggccga gtcggcgtcg acgctgctca gtgccgcgac atggccgacg cgctcggcca 7260
   ccacggcggc gacgacgttg cggtggtgca gcagcacccg tgcgtcgggc ccgtcgctgg 7320
   agacgaggac cagagtgtcc acgcccttca gcgccgcacg cagagcgggc gggtcggcgt 7380
   agtcggcgac ggcgcactcc acccccggcg gtagtgcctc ggcaggcagc ttccgcctgg 7440
   tcatcgccac gacgtccacg tcggcccggt ccgcgagcag ctgcaccacc cgcctgccca 7500
   gactgcccgc cgcccctgtc accgcgatac gcatcgtcgc ccccgtcgcc ttgtcgtcgg 7560
   tcgtaccacc gtagggggcc aaccgcgacc agggcttgga acgagccggc ccgccagggc 7620
   acagacgcgc gatcggtccg gttttcccgt gctcttttgg accgggacgc cggaccgctt 7680
   cctttctacg gtggagccgt tcccgcccga gcccgcacgt catcgacgtg cggggaagac 7740
   agaggtgata ccgatgctcc gacgccgtct cggagggccg tcaggccccc tcgtcagtgc 7800
   cctgtgcctg ggcgcgatgc ccttcggcac caccgtcgac gagaagacgt ccttcgccat 7860
   cctcgaccgg ttcgtcgagg ccggcggcag tctcgtcgac accgccgaca actacgcgtt 7920
   ctgggctccc ggcgggaccg gggacgagag cgagaacacc gtcgggcgct ggctggcgag 7980
   ccgccgccgc cgcgacgagg tggtgatctc caccaaggtg ggtgcccgcc ccaccgtccc 8040
   cggcagcggc ctggagaccg ccgaagggct gtcggctccc gtcatacgga aggccgcgga 8100
   ggacagcctg cgacgcctgg gcaccgatcg catcgatctg tactggaccc acatcgagga 8160
   ccggaccgtc cctctggagg agacgctcgg agctctcgac gagctggtcg gcggcggcaa 8220
   ggtggcggtg ctgggctgct ccaaccacgc gggctggcgc atcgaacggg cccgcgcgct 8280
   cgcccggacc aacggctgga cggcgtacac ctgcgtccag cagcgctact cctaccttca 8340
   gccgcgcttc gacgtcggac tgccggagag cggacacgtc cacgcgacct ccgaactcct 8400
   cgaccacgta cgcagcgagc ccgacctgac gctgctggcc tactcgtccc tgctctcggg 8460
   cgcgtacacc cggccggaca agaccctgtc cgccgcctac gaccacccgg gcaccgggca 8520
   gcggctgacc gtgctgcggg aggtggccgc cgagctcggt gccaccgcca accaggtggt 8580
   gctgtcctgg ttgctcggag gtgatccgcc ggtgatcccg atcgtggggg tgagttccgt 8640
   cgagcagctg gacgaggtgc tggccgccgt cgagctggag ctgccccggg agacgagggc 8700
   acggctggac ctcgccgggc ggggctgacg ggccacacac cgcccctcgc ccgggacggg 8760
   ctgaccggcc gcacaccgtc cctcagccgg gacgggctga cggccacaca cccgcccctc 8820
   agcccggctg ggctgggctg acggccacac acccgctcct cagcccggct gggctgggct 8880
   gacggccaca cacccgcccc tcagcccggc tgggctgggc tgacggccac acgccgtccc 8940
   tcgcccgggt cgggctgacc agccacacgc cgtccctcag tcggcccgct ccccggcccg 9000
   cccggcgtcg acgtgccgcc ggtcgtggcg ccgggccgcg tcgatggcct gtgccaggcg 9060
   ccgcacggct ccgggcacgg cgtggtccgc cagattgccg tagccgagga ccagcgcggg 9120
   gccggcggcg gccacggccc gcgccccgcc ggagtcgccg gcccgggccg tcgggtggtg 9180
   ctcggcccgc gccatccggt aggcgtcgag atccgccagc ctcacgtcac gccgcgccgc 9240
   ctccctgacg acggtgggcg ccgagcagtc cgtcagggac agcagcaggt ggaagccggc 9300
   cgccgcaccg gacacctgga accccggcag cgacgccgcg agttctccca tgaggtgatc 9360
   gcggcgccgt ttgtagcgca gccgtgccgc ccgcaggtac cggtcgtagg acccgcgggc 9420
   caggaaccgg gcgaacgcct cctggtcgat gacgggcggt ggtacgcccc cgacgtgctc 9480
   cgcgcccagg gcctcggtcc agcgcggcgg ggtcaccgcc cagccgatcc gcagggccgg 9540
   cgagagcgtc ttgctgaccg accccagcag cgcgacgtgc tccggtgcca tgccctgcag 9600
   ggcgcccacg gggtggcggt cgtatcggaa ctcggcgtcg tagtcgtcct ccagcaccag 9660
   gccgtcgacc tcgcgggccc aggcgaccag ctcgccgcga cgggccgggg cgaggaccac 9720
   accggtcggg aactggtggg cgggagcgac gatcaccgac cggacctgag gcgcgcgccg 9780
   cagcaggtcg acccgcaggc cgtcgccgtc cacgggaacc gggacgggca ccagcccctc 9840
   cgccccgacc gtggcgctca gccgcgacca gccagggtcc tccagggcca tgtgggtgtg 9900
   gccctcggtc cgcaggaccc ggcacagcct gcgcaccgcg tccagcgtgc ccgcgcagac 9960
   gacgagctgc cgggcttcca gcgacgcgcc gcgtccccgc acgaggtagg ccgcgagccg 10020
   ctgccggagc cgggcgagac cggccggatc ggggtagccg agctcgcccc aggtcagtgc 10080
   gccggtggcc tcccgcaccg cgtccgccca ccgtccgcgg ggaaaggccc gcaggtcggg 10140
   tatgccgggc agcatgtcga actccggctc ccaccgcgta ccgacgtccg cctcgggcgt 10200
   caccggagcg gggacggcct gcgccctgac ccgggtggcc gagccgctgc gcgcctccag 10260
   gtacccctcc gcgacgagct gcgcgtaggc ctccgtcacc acccaccgcg agcagcccag 10320
   gtccgccgcc agcgccctgc tcggcggcag cgcgctgccg gaggcgatcc gcccaccggt 10380
   caccgccgcc cgcagggcac ggctcagccg gacgtgcagc gggccgtccg ccggcccgcc 10440
   cagttcgaga aaggttcccc atgccgggtc ggtccggtcg tcgcccacgg cagccgatgc 10500
   tacccaccgc cccgcgacgg ccgggcggcg gcgtccacgg gcccggtgca cactggatgg 10560
   cgtggcggag acggaccggc atctcgacca gcggctggag caggccatca tggacctgct 10620
   ggagcgacgg gcggccactg cctcgatctg tccgtccgac gccgcgcgtc gggtccacga 10680
   gggtgacgac gagggctggc gagccctgct ggaacccgcg cgccgagcgg cctggcgtct 10740
   ggtggggtcc ggcgaggtcg aggtgaccca ggccggccgg cccgtcacgc aggccgaggc 10800
   ccgcgggccc gtccgcattc gtcgggcggg ttcctgacgg ctcgatccga aggcggaact 10860
   cccgtgcccc ggcggcacgg gagctcgacg ccgggaggtc agcgccgcag ggtgagcaca 10920
   cccggccgcc acggcagctg gtcgtagggg ccgcccgcgg tgggtgactt gccctggtag 10980
   aggaaccgca ggttgcaggg gtcgacggtc atggtctggt cggggttgtc gcggaccagg 11040
   tcaccgtggc tgatgtcgtt ggtccaggtg gccccgctgt tggccttgcc cgcgaagggg 11100
   ttgctctcgc tcgcggcctg cggggtccac gagccgctca ggctggaggc cgtgaaggaa 11160
   cggaagtagc gtccgttcgc gcccatcgcc tcgacgatca tcaggtactg gttctggccc   11220
   tgaaccttgt agacctgcac gccctcgaac aggttggcct tcgtgtcgct catgatcgtc   11280
   gtgtacgacg agccgaagct gcccgggaag ttcccgatcg gcatgctcgc ccggtagatc   11340
   ttgccgttgt caccggcgaa gaacaggtac atgttctgtc cgtcggcgat cagggtctgg   11400
   tcgatcgggc cggtgtcgga tcctgagatg ctcccggtga acaacggctg cggagccgac   11460
   cagccgttgg ggttggccgg gtcgctggac gtgcggtaga cgaagggcca cgcgccccac   11520
   tggtacgcca gcacccagac gttcttgggc gcgaagtaga acagggtggg cgccaccgcc   11580
   gcctgactca tcccggtctg gccggccgac gccatgtccg accagttcgt gaaggggctg   11640
   aacatcatcg agccgtagga cgaccccgac acgttcgacg cgtagaccag gtgcttgccg   11700
   ttgtgggtca cggtggtgaa gtccttgacc gccgcccacc cgttcgccgg ctgcgccagc   11760
   acacccgtcg acgaccaccg gtacgccgac ggaagggcac acgccccgtc cgtcgggggc   11820
   gtcccggtca ggccggacca cttctgcccg ctgccaccga cgcacgtccc gatctgcacc   11880
   gccgtgccgt tggccgtacc ggcgcccgcg gcctccaggc acagcccgga ctccacgccg   11940
   acgaccgtgc cgtcggagtt cacccgccac tgctggttcg caccgccgga acagctccag   12000
   atctgcaccc gcgtcccggg tgtggtggcg tgacccggaa cgtccaggca cttgttgccg   12060
   tacacggtca gccgacggtc gtccgtcaac gtccactgct gattgctccc gccccagcag   12120
   tcgtatatct gcag                                                     12134
<210> 141
   <211> 1164
   <212> DNA
   <213> Streptomyces ambofaciens
<220>
   <221> CDS
   <222> (1)..(1164)
<400> 141
   atg acc tta cgc acg ctt cga ccc gcc ggc gcg gcg ccc ccc gtg ggt      48
   Met Thr Leu Arg Thr Leu Arg Pro Ala Gly Ala Ala Pro Pro Val Gly
   1               5                   10                  15
   ccc gcc gcg cgg cac gac ttc gac tcg ctc gtc tcc gac gca tgt gcg      96
   Pro Ala Ala Arg His Asp Phe Asp Ser Leu Val Ser Asp Ala Cys Ala
               20                  25                  30
   gag tta ctc ggt tcg ctc cgc cgg gcg gac cag cgc aga aga ggt gag     144
   Glu Leu Leu Gly Ser Leu Arg Arg Ala Asp Gln Arg Arg Arg Gly Glu
           35                  40                  45
   cag tac ata cgc ggg ctg ctc acg gca cag gga cgc aag acc gcc cgg     192
   Gln Tyr Ile Arg Gly Leu Leu Thr Ala Gln Gly Arg Lys Thr Ala Arg
       50                  55                  60
   aac ctc gcc gcc ttc gtc gga gag ggc gcg gcg gaa cag agc ctg cac     240
   Asn Leu Ala Ala Phe Val Gly Glu Gly Ala Ala Glu Gln Ser Leu His
   65                  70                  75                  80
   cac ttc gtc gcc ggg tcg acc tgg gac tgg ggc gag gtg cgg gcc gcg     288
   His Phe Val Ala Gly Ser Thr Trp Asp Trp Gly Glu Val Arg Ala Ala
                   85                  90                  95
   ctg gcc cgc tac gtg gac gac agg ctg acc ccc gac gcc tgg gtg atc     336
   Leu Ala Arg Tyr Val Asp Asp Arg Leu Thr Pro Asp Ala Trp Val Ile
               100                 105                 110
   tgg ccg atg gtg gtc tcc aag gcg ggg gtg cgc tcc gtc ggg gtg agc     384
   Trp Pro Met Val Val Ser Lys Ala Gly Val Arg Ser Val Gly Val Ser
           115                 120                 125
   cgg cgc ttc gtc tcc gac ctg ggc cgg gtg gtg agc tgc cag cag agt     432
   Arg Arg Phe Val Ser Asp Leu Gly Arg Val Val Ser Cys Gln Gln Ser
       130                 135                 140
   cac ggg ctg tgg ctg gcc tcg ggc acg acg gcc gcc ccg gtc agc tgg     480
   His Gly Leu Trp Leu Ala Ser Gly Thr Thr Ala Ala Pro Val Ser Trp
   145                 150                 155                 160
   cgc ctg acg ctg ggc ggc ggc cgg agc ggg gag ggc aca ccc cgg cac     528
   Arg Leu Thr Leu Gly Gly Gly Arg Ser Gly Glu Gly Thr Pro Arg His
                   165                 170                 175
   ccc ggc gca ccc ggt gag gag gag aac atc gtc cgc ctg gtg gcc gaa     576
   Pro Gly Ala Pro Gly Glu Glu Glu Asn Ile Val Arg Leu Val Ala Glu
               180                 185                 190
   gcc gct cag gcc aac cgc tcc ttg gcc cgc ccc gtg gtg atg gac gca     624
   Ala Ala Gln Ala Asn Arg Ser Leu Ala Arg Pro Val Val Met Asp Ala
           195                 200                 205
   cgc gcg gcc gtg ctg cca cga ctg gtg cga ggg ctc ggt ctc gcg ggc     672
   Arg Ala Ala Val Leu Pro Arg Leu Val Arg Gly Leu Gly Leu Ala Gly
       210                 215                 220
   ctg ccc ttc atg gtg cgg gtg ggc ggg aac ctc cgg ctg gcc tcg gcg     720
   Leu Pro Phe Met Val Arg Val Gly Gly Asn Leu Arg Leu Ala Ser Ala
   225                 230                 235                 240
   agc ggc cgc ggc ccg gtg gac cac cac acg ggg acc acc tcg gcc cag     768
   Ser Gly Arg Gly Pro Val Asp His His Thr Gly Thr Thr Ser Ala Gln
                   245                 250                 255
   cag ttg atg gag cag ctc aag cgg ttg agc cgc ccc gtg gag ttg cgg     816
   Gln Leu Met Glu Gln Leu Lys Arg Leu Ser Arg Pro Val Glu Leu Arg
               260                 265                 270
   ggc tcc ctc agc ctc gtc gcc ccg cac ccc gtc gtc ctg ccg ggt gtg     864
   Gly Ser Leu Ser Leu Val Ala Pro His Pro Val Val Leu Pro Gly Val
           275                 280                 285
   gtc ccg agg cgg acg ctg acg ctg ctg ggg gtg tgg cgg ggc aac cgc     912
   Val Pro Arg Arg Thr Leu Thr Leu Leu Gly Val Trp Arg Gly Asn Arg
       290                 295                 300
   cgg cgc ccc gcc gac ctg tgg ctg acc gac ctc acg tcc tgg gac cgc     960
   Arg Arg Pro Ala Asp Leu Trp Leu Thr Asp Leu Thr Ser Trp Asp Arg
   305                 310                 315                 320
   ggc gcc ctg ctg cgg ctg gcg atg ctc acc gaa cag gtc gac gcc gac    1008
   Gly Ala Leu Leu Arg Leu Ala Met Leu Thr Glu Gln Val Asp Ala Asp
                   325                 330                 335
   ttc gac cgg gtg agc gtg ggc gtg ggc atg cgc gac ttc gaa gga cgg    1056
   Phe Asp Arg Val Ser Val Gly Val Gly Met Arg Asp Phe Glu Gly Arg
               340                 345                 350
   tcg ttc cag ggc tgg cac cgg cac gtg acc ctg gcg tcc atc gcc cac    1104
   Ser Phe Gln Gly Trp His Arg His Val Thr Leu Ala Ser Ile Ala His
           355                 360                 365
   acg ctg cgg ctg gcg cag ccc tcc gcg cgc ggc gac ctc ggt ggg gcg      1152
   Thr Leu Arg Leu Ala Gln Pro Ser Ala Arg Gly Asp Leu Gly Gly Ala
       370                 375                 380
   acg gcg gtg tga                                                      1164
   Thr Ala Val
   385
<210> 142
   <211> 387
   <212> PRT
   <213> Streptomyces ambofaciens
<400> 142
   Met Thr Leu Arg Thr Leu Arg Pro Ala Gly Ala Ala Pro Pro Val Gly
   1               5                   10                  15
   Pro Ala Ala Arg His Asp Phe Asp Ser Leu Val Ser Asp Ala Cys Ala
               20                  25                  30
   Glu Leu Leu Gly Ser Leu Arg Arg Ala Asp Gln Arg Arg Arg Gly Glu
           35                  40                  45
   Gln Tyr Ile Arg Gly Leu Leu Thr Ala Gln Gly Arg Lys Thr Ala Arg
       50                  55                  60
   Asn Leu Ala Ala Phe Val Gly Glu Gly Ala Ala Glu Gln Ser Leu His
   65                  70                  75                  80
   His Phe Val Ala Gly Ser Thr Trp Asp Trp Gly Glu Val Arg Ala Ala
                   85                  90                  95
   Leu Ala Arg Tyr Val Asp Asp Arg Leu Thr Pro Asp Ala Trp Val Ile
               100                 105                 110
   Trp Pro Met Val Val Ser Lys Ala Gly Val Arg Ser Val Gly Val Ser
           115                 120                 125
   Arg Arg Phe Val Ser Asp Leu Gly Arg Val Val Ser Cys Gln Gln Ser
       130                 135                 140
   His Gly Leu Trp Leu Ala Ser Gly Thr Thr Ala Ala Pro Val Ser Trp
   145                 150                 155                 160
   Arg Leu Thr Leu Gly Gly Gly Arg Ser Gly Glu Gly Thr Pro Arg His
                   165                 170                 175
   Pro Gly Ala Pro Gly Glu Glu Glu Asn Ile Val Arg Leu Val Ala Glu
               180                 185                 190
   Ala Ala Gln Ala Asn Arg Ser Leu Ala Arg Pro Val Val Met Asp Ala
   195 200 205
   Arg Ala Ala Val Leu Pro Arg Leu Val Arg Gly Leu Gly Leu Ala Gly
   210 215 220
   Leu Pro Phe Met Val Arg Val Gly Gly Asn Leu Arg Leu Ala Ser Ala
   225 230 235 240
   Ser Gly Arg Gly Pro Val Asp His His Thr Gly Thr Thr Ser Ala Gln
   245 250 255
   Gln Leu Met Glu Gln Leu Lys Arg Leu Ser Arg Pro Val Glu Leu Arg
   260 265 270
   Gly Ser Leu Ser Leu Val Ala Pro His Pro Val Val Leu Pro Gly Val
   275 280 285
   Val Pro Arg Arg Thr Leu Thr Leu Leu Gly Val Trp Arg Gly Asn Arg
   290 295 300
   Arg Arg Pro Ala Asp Leu Trp Leu Thr Asp Leu Thr Ser Trp Asp Arg
   305 310 315 320
   Gly Ala Leu Leu Arg Leu Ala Met Leu Thr Glu Gln Val Asp Ala Asp
   325 330 335
   Phe Asp Arg Val Ser Val Gly Val Gly Met Arg Asp Phe Glu Gly Arg
   340 345 350
   Ser Phe Gln Gly Trp His Arg His Val Thr Leu Ala Ser Ile Ala His
   355 360 365
   Thr Leu Arg Leu Ala Gln Pro Ser Ala Arg Gly Asp Leu Gly Gly Ala
   370 375 380
   Thr Ala Val
   385
<210> 143
   <211> 849
   <212> DNA
   <213> Streptomyces ambofaciens
<220>
   <221> CDS
   <222> (1)..(849)
<400> 143
   atg cgt atc gcg gtg aca ggg gcg gcg ggc agt ctg ggc agg cgg gtg      48
   Met Arg Ile Ala Val Thr Gly Ala Ala Gly Ser Leu Gly Arg Arg Val
   1               5                   10                  15
   gtg cag ctg ctc gcg gac cgg gcc gac gtg gac gtc gtg gcg atg acc      96
   Val Gln Leu Leu Ala Asp Arg Ala Asp Val Asp Val Val Ala Met Thr
                   20                       25                       30
   agg cgg aag ctg cct gcc gag gca cta ccg ccg ggg gtg gag tgc gcc      144
   Arg Arg Lys Leu Pro Ala Glu Ala Leu Pro Pro Gly Val Glu Cys Ala
           35                  40                  45
   gtc gcc gac tac gcc gac ccg ccc gct ctg cgt gcg gcg ctg aag ggc      192
   Val Ala Asp Tyr Ala Asp Pro Pro Ala Leu Arg Ala Ala Leu Lys Gly
       50                  55                  60
   gtg gac act ctg gtc ctc gtc tcc agc gac ggg ccc gac gca cgg gtg      240
   Val Asp Thr Leu Val Leu Val Ser Ser Asp Gly Pro Asp Ala Arg Val
   65                  70                  75                  80
   ctg ctg cac cac cgc aac gtc gtc gcc gcc gtg gtg gcc gag cgc gtc      288
   Leu Leu His His Arg Asn Val Val Ala Ala Val Val Ala Glu Arg Val
                   85                  90                  95
   ggc cat gtc gcg gca ctg agc agc gtc gac gcc gac tcg gcc tcc ccg      336
   Gly His Val Ala Ala Leu Ser Ser Val Asp Ala Asp Ser Ala Ser Pro
               100                 105                 110
   ttc tgc tac gcc gtc gtc aac cgg ctc acc gag gat ctg ctg ctc gcc      384
   Phe Cys Tyr Ala Val Val Asn Arg Leu Thr Glu Asp Leu Leu Leu Ala
           115                 120                 125
   tcc ggc gtg ccg tgc tcg ttc gcc agg gcc tcg ctg tac atc gag ttc      432
   Ser Gly Val Pro Cys Ser Phe Ala Arg Ala Ser Leu Tyr Ile Glu Phe
       130                 135                 140
   ttc cta ggc tgg ctc acc cag gcg cgc tcg acc ggg ctg ctg agg ctg      480
   Phe Leu Gly Trp Leu Thr Gln Ala Arg Ser Thr Gly Leu Leu Arg Leu
   145                 150                 155                 160
   ccg gca gcc gat ggc cgg gtg tcc ctg gtg gcg cgc gac gac gtg gcc      528
   Pro Ala Ala Asp Gly Arg Val Ser Leu Val Ala Arg Asp Asp Val Ala
                   165                 170                 175
   cgc gcc ctc gcc gcc ctc gcg gtg ggc ggg ccg acc ggc cgt cac cac      576
   Arg Ala Leu Ala Ala Leu Ala Val Gly Gly Pro Thr Gly Arg His His
               180                 185                 190
   gac atc acg gga ccc gag tcg acg gac ctg gca ggc atc gcc tcc acg      624
   Asp Ile Thr Gly Pro Glu Ser Thr Asp Leu Ala Gly Ile Ala Ser Thr
           195                 200                 205
   gcg gcg gag gtg tgg cgg aca ccg gtc gcc tac gtg gac atc ccg gcc      672
   Ala Ala Glu Val Trp Arg Thr Pro Val Ala Tyr Val Asp Ile Pro Ala
       210                 215                 220
   gac acg tac tct gcc gaa aca gcg gcg acc ggc ctg gat ccc tgg tgg      720
   Asp Thr Tyr Ser Ala Glu Thr Ala Ala Thr Gly Leu Asp Pro Trp Trp
   225                 230                 235                 240
   ctc tac gcc ttc tcc tcc ctg ttc gcc tcg gta cgc gaa cag cgc tgg      768
   Leu Tyr Ala Phe Ser Ser Leu Phe Ala Ser Val Arg Glu Gln Arg Trp
                   245                 250                 255
   gat cgg gtc cgc gac gac tgc gcc cag ctc acc ggc cgc ttg ccc cgc      816
   Asp Arg Val Arg Asp Asp Cys Ala Gln Leu Thr Gly Arg Leu Pro Arg
               260                 265                 270
   act ctc cgc gac gtc ctg tca gaa cgc gcg tga                          849
   Thr Leu Arg Asp Val Leu Ser Glu Arg Ala
           275                 280
<210> 144
   <211> 282
   <212> PRT
   <213> Streptomyces ambofacjens
<400> 144
   Met Arg Ile Ala Val Thr Gly Ala Ala Gly Ser Leu Gly Arg Arg Val
   1               5                   10                  15
   Val Gln Leu Leu Ala Asp Arg Ala Asp Val Asp Val Val Ala Met Thr
               20                  25                  30
   Arg Arg Lys Leu Pro Ala Glu Ala Leu Pro Pro Gly Val Glu Cys Ala
           35                  40                  45
   Val Ala Asp Tyr Ala Asp Pro Pro Ala Leu Arg Ala Ala Leu Lys Gly
       50                  55                  60
   Val Asp Thr Leu Val Leu Val Ser Ser Asp Gly Pro Asp Ala Arg Val
   65                  70                  75                  80
   Leu Leu His His Arg Asn Val Val Ala Ala Val Val Ala Glu Arg Val
                   85                  90                  95
   Gly His Val Ala Ala Leu Ser Ser Val Asp Ala Asp Ser Ala Ser Pro
               100                 105                 110
   Phe Cys Tyr Ala Val Val Asn Arg Leu Thr Glu Asp Leu Leu Leu Ala
           115                 120                 125
   Ser Gly Val Pro Cys Ser Phe Ala Arg Ala Ser Leu Tyr Ile Glu Phe
       130                 135                 140
   Phe Leu Gly Trp Leu Thr Gln Ala Arg Ser Thr Gly Leu Leu Arg Leu
   145                 150                 155                 160
   Pro Ala Ala Asp Gly Arg Val Ser Leu Val Ala Arg Asp Asp Val Ala
                   165                 170                 175
   Arg Ala Leu Ala Ala Leu Ala Val Gly Gly Pro Thr Gly Arg His His
               180                 185                 190
   Asp Ile Thr Gly Pro Glu Ser Thr Asp Leu Ala Gly Ile Ala Ser Thr
           195                 200                 205
   Ala Ala Glu Val Trp Arg Thr Pro Val Ala Tyr Val Asp Ile Pro Ala
       210                 215                 220
   Asp Thr Tyr Ser Ala Glu Thr Ala Ala Thr Gly Leu Asp Pro Trp Trp
   225                 230                 235                 240
   Leu Tyr Ala Phe Ser Ser Leu Phe Ala Ser Val Arg Glu Gln Arg Trp
                   245                 250                 255
   Asp Arg Val Arg Asp Asp Cys Ala Gln Leu Thr Gly Arg Leu Pro Arg
               260                 265                 270
   Thr Leu Arg Asp Val Leu Ser Glu Arg Ala
           275                 280
<210> 145
   <211> 1512
   <212> DNA
   <213> Streptomyces ambofaciens
<220>
   <221> CDS
   <222> (1)..(1512)
<400> 145
   gtg ggc gac gac cgg acc gac ccg gca tgg gga acc ttt ctc gaa ctg      48
   Val Gly Asp Asp Arg Thr Asp Pro Ala Trp Gly Thr Phe Leu Glu Leu
   1               5                   10                  15
   ggc ggg ccg gcg gac ggc ccg ctg cac gtc cgg ctg agc cgt gcc ctg      96
   Gly Gly Pro Ala Asp Gly Pro Leu His Val Arg Leu Ser Arg Ala Leu
               20                  25                  30
   cgg gcg gcg gtg acc ggt ggg cgg atc gcc tcc ggc agc gcg ctg ccg     144
   Arg Ala Ala Val Thr Gly Gly Arg Ile Ala Ser Gly Ser Ala Leu Pro
           35                  40                  45
   ccg agc agg gcg ctg gcg gcg gac ctg ggc tgc tcg cgg tgg gtg gtg     192
   Pro Ser Arg Ala Leu Ala Ala Asp Leu Gly Cys Ser Arg Trp Val Val
       50                  55                  60
   acg gag gcc tac gcg cag ctc gtc gcg gag ggg tac ctg gag gcg cgc     240
   Thr Glu Ala Tyr Ala Gln Leu Val Ala Glu Gly Tyr Leu Glu Ala Arg
   65                  70                  75                  80
   agc ggc tcg gcc acc cgg gtc agg gcg cag gcc gtc ccc gct ccg gtg     288
   Ser Gly Ser Ala Thr Arg Val Arg Ala Gln Ala Val Pro Ala Pro Val
                   85                  90                  95
   acg ccc gag gcg gac gtc ggt acg cgg tgg gag ccg gag ttc gac atg     336
   Thr Pro Glu Ala Asp Val Gly Thr Arg Trp Glu Pro Glu Phe Asp Met
               100                 105                 110
   ctg ccc ggc ata ccc gac ctg cgg gcc ttt ccc cgc gga cgg tgg gcg     384
   Leu Pro Gly Ile Pro Asp Leu Arg Ala Phe Pro Arg Gly Arg Trp Ala
           115                 120                 125
   gac gcg gtg cgg gag gcc acc ggc gca ctg acc tgg ggc gag ctc ggc     432
   Asp Ala Val Arg Glu Ala Thr Gly Ala Leu Thr Trp Gly Glu Leu Gly
       130                 135                 140
   tac ccc gat ccg gcc ggt ctc gcc cgg ctc cgg cag cgg ctc gcg gcc     480
   Tyr Pro Asp Pro Ala Gly Leu Ala Arg Leu Arg Gln Arg Leu Ala Ala
   145                 150                 155                 160
   tac ctc gtg cgg gga cgc ggc gcg tcg ctg gaa gcc cgg cag ctc gtc     528
   Tyr Leu Val Arg Gly Arg Gly Ala Ser Leu Glu Ala Arg Gln Leu Val
                   165                 170                 175
   gtc tgc gcg ggc acg ctg gac gcg gtg cgc agg ctg tgc cgg gtc ctg     576
   Val Cys Ala Gly Thr Leu Asp Ala Val Arg Arg Leu Cys Arg Val Leu
               180                 185                 190
   cgg acc gag ggc cac acc cac atg gcc ctg gag gac cct ggc tgg tcg      624
   Arg Thr Glu Gly His Thr His Met Ala Leu Glu Asp Pro Gly Trp Ser
           195                 200                 205
   cgg ctg agc gcc acg gtc ggg gcg gag ggg ctg gtg ccc gtc ccg gtt      672
   Arg Leu Ser Ala Thr Val Gly Ala Glu Gly Leu Val Pro Val Pro Val
       210                 215                 220
   ccc gtg gac ggc gac ggc ctg cgg gtc gac ctg ctg cgg cgc gcg cct      720
   Pro Val Asp Gly Asp Gly Leu Arg Val Asp Leu Leu Arg Arg Ala Pro
   225                 230                 235                 240
   cag gtc cgg tcg gtg atc gtc gct ccc gcc cac cag ttc ccg acc ggt      768
   Gln Val Arg Ser Val Ile Val Ala Pro Ala His Gln Phe Pro Thr Gly
                   245                 250                 255
   gtg gtc ctc gcc ccg gcc cgt cgc ggc gag ctg gtc gcc tgg gcc cgc      816
   Val Val Leu Ala Pro Ala Arg Arg Gly Glu Leu Val Ala Trp Ala Arg
               260                 265                 270
   gag gtc gac ggc ctg gtg ctg gag gac gac tac gac gcc gag ttc cga      864
   Glu Val Asp Gly Leu Val Leu Glu Asp Asp Tyr Asp Ala Glu Phe Arg
           275                 280                 285
   tac gac cgc cac ccc gtg ggc gcc ctg cag ggc atg gca ccg gag cac      912
   Tyr Asp Arg His Pro Val Gly Ala Leu Gln Gly Met Ala Pro Glu His
       290                 295                 300
   gtc gcg ctg ctg ggg tcg gtc agc aag acg ctc tcg ccg gcc ctg cgg      960
   Val Ala Leu Leu Gly Ser Val Ser Lys Thr Leu Ser Pro Ala Leu Arg
   305                 310                 315                 320
   atc ggc tgg gcg gtg acc ccg ccg cgc tgg acc gag gcc ctg ggc gcg     1008
   Ile Gly Trp Ala Val Thr Pro Pro Arg Trp Thr Glu Ala Leu Gly Ala
                   325                 330                 335
   gag cac gtc ggg ggc gta cca ccg ccc gtc atc gac cag gag gcg ttc     1056
   Glu His Val Gly Gly Val Pro Pro Pro Val Ile Asp Gln Glu Ala Phe
               340                 345                 350
   gcc cgg ttc ctg gcc cgc ggg tcc tac gac cgg tac ctg cgg gcg gca     1104
   Ala Arg Phe Leu Ala Arg Gly Ser Tyr Asp Arg Tyr Leu Arg Ala Ala
           355                 360                 365
   cgg ctg cgc tac aaa cgg cgc cgc gat cac ctc atg gga gaa ctc gcg     1152
   Arg Leu Arg Tyr Lys Arg Arg Arg Asp His Leu Met Gly Glu Leu Ala
       370                 375                 380
   gcg tcg ctg ccg ggg ttc cag gtg tcc ggt gcg gcg gcc ggc ttc cac     1200
   Ala Ser Leu Pro Gly Phe Gln Val Ser Gly Ala Ala Ala Gly Phe His
   385                 390                 395                 400
   ctg ctg ctg tcc ctg acg gac tgc tcg gcg ccc acc gtc gtc agg gag     1248
   Leu Leu Leu Ser Leu Thr Asp Cys Ser Ala Pro Thr Val Val Arg Glu
                   405                 410                 415
   gcg gcg cgg cgt gac gtg agg ctg gcg gat ctc gac gcc tac cgg atg     1296
   Ala Ala Arg Arg Asp Val Arg Leu Ala Asp Leu Asp Ala Tyr Arg Met
               420                 425                 430
   gcg cgg gcc gag cac cac ccg acg gcc cgg gcc ggc gac tcc ggc ggg     1344
   Ala Arg Ala Glu His His Pro Thr Ala Arg Ala Gly Asp Ser Gly Gly
           435                 440                 445
   gcg cgg gcc gtg gcc gcc gcc ggc ccc gcg ctg gtc ctc ggc tac ggc     1392
   Ala Arg Ala Val Ala Ala Ala Gly Pro Ala Leu Val Leu Gly Tyr Gly
       450                 455                 460
   aat ctg gcg gac cac gcc gtg ccc gga gcc gtg cgg cgc ctg gca cag      1440
   Asn Leu Ala Asp His Ala Val Pro Gly Ala Val Arg Arg Leu Ala Gln
   465                 470                 475                 480
   gcc atc gac gcg gcc cgg cgc cac gac cgg cgg cac gtc gac gcc ggg      1488
   Ala Ile Asp Ala Ala Arg Arg His Asp Arg Arg His Val Asp Ala Gly
                   485                 490                 495
   cgg gcc ggg gag cgg gcc gac tga                                      1512
   Arg Ala Gly Glu Arg Ala Asp
               500
<210> 146
   <211> 503
   <212> PRT
   <213> Streptomyces ambofaciens
<400> 146
   Val Gly Asp Asp Arg Thr Asp Pro Ala Trp Gly Thr Phe Leu Glu Leu
   1               5                   10                  15
   Gly Gly Pro Ala Asp Gly Pro Leu His Val Arg Leu Ser Arg Ala Leu
               20                  25                  30
   Arg Ala Ala Val Thr Gly Gly Arg Ile Ala Ser Gly Ser Ala Leu Pro
           35                  40                  45
   Pro Ser Arg Ala Leu Ala Ala Asp Leu Gly Cys Ser Arg Trp Val Val
       50                  55                  60
   Thr Glu Ala Tyr Ala Gln Leu Val Ala Glu Gly Tyr Leu Glu Ala Arg
   65                  70                  75                  80
   Ser Gly Ser Ala Thr Arg Val Arg Ala Gln Ala Val Pro Ala Pro Val
                   85                  90                  95
   Thr Pro Glu Ala Asp Val Gly Thr Arg Trp Glu Pro Glu Phe Asp Met
               100                 105                 110
   Leu Pro Gly Ile Pro Asp Leu Arg Ala Phe Pro Arg Gly Arg Trp Ala
           115                 120                 125
   Asp Ala Val Arg Glu Ala Thr Gly Ala Leu Thr Trp Gly Glu Leu Gly
       130                 135                 140
   Tyr Pro Asp Pro Ala Gly Leu Ala Arg Leu Arg Gln Arg Leu Ala Ala
   145                 150                 155                 160
   Tyr Leu Val Arg Gly Arg Gly Ala Ser Leu Glu Ala Arg Gln Leu Val
                   165                 170                 175
   Val Cys Ala Gly Thr Leu Asp Ala Val Arg Arg Leu Cys Arg Val Leu
               180                 185                 190
   Arg Thr Glu Gly His Thr His Met Ala Leu Glu Asp Pro Gly Trp Ser
   195 200 205
   Arg Leu Ser Ala Thr Val Gly Ala Glu Gly Leu Val Pro Val Pro Val
   210 215 220
   Pro Val Asp Gly Asp Gly Leu Arg Val Asp Leu Leu Arg Arg Ala Pro
   225 230 235 240
   Gln Val Arg Ser Val Ile Val Ala Pro Ala His Gln Phe Pro Thr Gly
   245 250 255
   Val Val Leu Ala Pro Ala Arg Arg Gly Glu Leu Val Ala Trp Ala Arg
   260 265 270
   Glu Val Asp Gly Leu Val Leu Glu Asp Asp Tyr Asp Ala Glu Phe Arg
   275 280 285
   Tyr Asp Arg His Pro Val Gly Ala Leu Gln Gly Met Ala Pro Glu His
   290 295 300
   Val Ala Leu Leu Gly Ser Val Ser Lys Thr Leu Ser Pro Ala Leu Arg
   305 310 315 320
   Ile Gly Trp Ala Val Thr Pro Pro Arg Trp Thr Glu Ala Leu Gly Ala
   325 330 335
   Glu His Val Gly Gly Val Pro Pro Pro Val Ile Asp Gln Glu Ala Phe
   340 345 350
   Ala Arg Phe Leu Ala Arg Gly Ser Tyr Asp Arg Tyr Leu Arg Ala Ala
   355 360 365
   Arg Leu Arg Tyr Lys Arg Arg Arg Asp His Leu Met Gly Glu Leu Ala
   370 375 380
   Ala Ser Leu Pro Gly Phe Gln Val Ser Gly Ala Ala Ala Gly Phe His
   385 390 395 400
   Leu Leu Leu Ser Leu Thr Asp Cys Ser Ala Pro Thr Val Val Arg Glu
   405 410 415
   Ala Ala Arg Arg Asp Val Arg Leu Ala Asp Leu Asp Ala Tyr Arg Met
   420 425 430
   Ala Arg Ala Glu His His Pro Thr Ala Arg Ala Gly Asp Ser Gly Gly
   435 440 445
   Ala Arg Ala Val Ala Ala Ala Gly Pro Ala Leu Val Leu Gly Tyr Gly
   450 455 460
   Asn Leu Ala Asp His Ala Val Pro Gly Ala Val Arg Arg Leu Ala Gln
   465 470 475 480
   Ala Ile Asp Ala Ala Arg Arg His Asp Arg Arg His Val Asp Ala Gly
   485 490 495
   Arg Ala Gly Glu Arg Ala Asp
   500
<210> 147
   <211> 276
   <212> DNA
   <213> Streptomyces ambofaciens
<220>
   <221> CDS
   <222> (1)..(276)
<400> 147
   gtg gcg gag acg gac cgg cat ctc gac cag cgg ctg gag cag gcc atc 48
   Val Ala Glu Thr Asp Arg His Leu Asp Gln Arg Leu Glu Gln Ala Ile
   1 5 10 15
   atg gac ctg ctg gag cga cgg gcg gcc act gcc tcg atc tgt ccg tcc 96
   Met Asp Leu Leu Glu Arg Arg Ala Ala Thr Ala Ser Ile Cys Pro Ser
   20 25 30
   gac gcc gcg cgt cgg gtc cac gag ggt gac gac gag ggc tgg cga gcc 144
   Asp Ala Ala Arg Arg Val His Glu Gly Asp Asp Glu Gly Trp Arg Ala
   35 40 45
   ctg ctg gaa ccc gcg cgc cga gcg gcc tgg cgt ctg gtg ggg tcc ggc 192
   Leu Leu Glu Pro Ala Arg Arg Ala Ala Trp Arg Leu Val Gly Ser Gly
   50 55 60
   gag gtc gag gtg acc cag gcc ggc cgg ccc gtc acg cag gcc gag gcc 240
   Glu Val Glu Val Thr Gln Ala Gly Arg Pro Val Thr Gln Ala Glu Ala
   65 70 75 80
   cgc ggg ccc gtc cgc att cgt cgg gcg ggt tcc tga 276
   Arg Gly Pro Val Arg Ile Arg Arg Ala Gly Ser
   85 90
<210> 148
   <211> 91
   <212> PRT
   <213> Streptomyces ambofaciens
<400> 148
   Val Ala Glu Thr Asp Arg His Leu Asp Gln Arg Leu Glu Gln Ala Ile
   1 5 10 15
   Met Asp Leu Leu Glu Arg Arg Ala Ala Thr Ala Ser Ile Cys Pro Ser
   20 25 30
   Asp Ala Ala Arg Arg Val His Glu Gly Asp Asp Glu Gly Trp Arg Ala
   35 40 45
   Leu Leu Glu Pro Ala Arg Arg Ala Ala Trp Arg Leu Val Gly Ser Gly
   50 55 60
   Glu Val Glu Val Thr Gln Ala Gly Arg Pro Val Thr Gln Ala Glu Ala
   65 70 75 80
   Arg Gly Pro Val Arg Ile Arg Arg Ala Gly Ser
   85 90
<210> 149
   <211> 1236
   <212> DNA
   <213> Streptomyces ambofaciens
<220>
   <221> CDS
   <222> (1)..(1236)
<400> 149
   ctg cag ata tac gac tgc tgg ggc ggg agc aat cag cag tgg acg ttg      48
   Leu Gln Ile Tyr Asp Cys Trp Gly Gly Ser Asn Gln Gln Trp Thr Leu
   1               5                   10                  15
   acg gac gac cgt cgg ctg acc gtg tac ggc aac aag tgc ctg gac gtt      96
   Thr Asp Asp Arg Arg Leu Thr Val Tyr Gly Asn Lys Cys Leu Asp Val
               20                  25                  30
   ccg ggt cac gcc acc aca ccc ggg acg cgg gtg cag atc tgg agc tgt      144
   Pro Gly His Ala Thr Thr Pro Gly Thr Arg Val Gln Ile Trp Ser Cys
           35                  40                  45
   tcc ggc ggt gcg aac cag cag tgg cgg gtg aac tcc gac ggc acg gtc      192
   Ser Gly Gly Ala Asn Gln Gln Trp Arg Val Asn Ser Asp Gly Thr Val
       50                  55                  60
   gtc ggc gtg gag tcc ggg ctg tgc ctg gag gcc gcg ggc gcc ggt acg      240
   Val Gly Val Glu Ser Gly Leu Cys Leu Glu Ala Ala Gly Ala Gly Thr
   65                  70                  75                  80
   gcc aac ggc acg gcg gtg cag atc ggg acg tgc gtc ggt ggc agc ggg      288
   Ala Asn Gly Thr Ala Val Gln Ile Gly Thr Cys Val Gly Gly Ser Gly
                   85                  90                  95
   cag aag tgg tcc ggc ctg acc ggg acg ccc ccg acg gac ggg gcg tgt      336
   Gln Lys Trp Ser Gly Leu Thr Gly Thr Pro Pro Thr Asp Gly Ala Cys
               100                 105                 110
   gcc ctt ccg tcg gcg tac cgg tgg tcg tcg acg ggt gtg ctg gcg cag      384
   Ala Leu Pro Ser Ala Tyr Arg Trp Ser Ser Thr Gly Val Leu Ala Gln
           115                 120                 125
   ccg gcg aac ggg tgg gcg gcg gtc aag gac ttc acc acc gtg acc cac      432
   Pro Ala Asn Gly Trp Ala Ala Val Lys Asp Phe Thr Thr Val Thr His
       130                 135                 140
   aac ggc aag cac ctg gtc tac gcg tcg aac gtg tcg ggg tcg tcc tac      480
   Asn Gly Lys His Leu Val Tyr Ala Ser Asn Val Ser Gly Ser Ser Tyr
   145                 150                 155                 160
   ggc tcg atg atg ttc agc ccc ttc acg aac tgg tcg gac atg gcg tcg      528
   Gly Ser Met Met Phe Ser Pro Phe Thr Asn Trp Ser Asp Met Ala Ser
                   165                 170                 175
   gcc ggc cag acc ggg atg agt cag gcg gcg gtg gcg ccc acc ctg ttc      576
   Ala Gly Gln Thr Gly Met Ser Gln Ala Ala Val Ala Pro Thr Leu Phe
               180                 185                 190
   tac ttc gcg ccc aag aac gtc tgg gtg ctg gcg tac cag tgg ggc gcg      624
   Tyr Phe Ala Pro Lys Asn Val Trp Val Leu Ala Tyr Gln Trp Gly Ala
           195                 200                 205
   tgg ccc ttc gtc tac cgc acg tcc agc gac ccg gcc aac ccc aac ggc      672
   Trp Pro Phe Val Tyr Arg Thr Ser Ser Asp Pro Ala Asn Pro Asn Gly
       210                 215                 220
   tgg tcg gct ccg cag ccg ttg ttc acc ggg agc atc tca gga tcc gac      720
   Trp Ser Ala Pro Gln Pro Leu Phe Thr Gly Ser Ile Ser Gly Ser Asp
   225                 230                 235                 240
   acc ggc ccg atc gac cag acc ctg atc gcc gac gga cag aac atg tac      768
   Thr Gly Pro Ile Asp Gln Thr Leu Ile Ala Asp Gly Gln Asn Met Tyr
                   245                 250                 255
   ctg ttc ttc gcc ggt gac aac ggc aag atc tac cgg gcg agc atg ccg      816
   Leu Phe Phe Ala Gly Asp Asn Gly Lys Ile Tyr Arg Ala Ser Met Pro
               260                 265                 270
   atc ggg aac ttc ccg ggc agc ttc ggc tcg tcg tac acg acg atc atg      864
   Ile Gly Asn Phe Pro Gly Ser Phe Gly Ser Ser Tyr Thr Thr Ile Met
           275                 280                 285
   agc gac acg aag gcc aac ctg ttc gag ggc gtg cag gtc tac aag gtt      912
   Ser Asp Thr Lys Ala Asn Leu Phe Glu Gly Val Gln Val Tyr Lys Val
       290                 295                 300
   cag ggc cag aac cag tac ctg atg atc gtc gag gcg atg ggc gcg aac      960
   Gln Gly Gln Asn Gln Tyr Leu Met Ile Val Glu Ala Met Gly Ala Asn
   305                 310                 315                 320
   gga cgc tac ttc cgt tcc ttc acg gcc tcc agc ctg agc ggc tcg tgg     1008
   Gly Arg Tyr Phe Arg Ser Phe Thr Ala Ser Ser Leu Ser Gly Ser Trp
                   325                 330                 335
   acc ccg cag gcc gcg agc gag agc aac ccc ttc gcg ggc aag gcc aac     1056
   Thr Pro Gln Ala Ala Ser Glu Ser Asn Pro Phe Ala Gly Lys Ala Asn
               340                 345                 350
   agc ggg gcc acc tgg acc aac gac atc agc cac ggt gac ctg gtc cgc     1104
   Ser Gly Ala Thr Trp Thr Asn Asp Ile Ser His Gly Asp Leu Val Arg
           355                 360                 365
   gac aac ccc gac cag acc atg acc gtc gac ccc tgc aac ctg cgg ttc     1152
   Asp Asn Pro Asp Gln Thr Met Thr Val Asp Pro Cys Asn Leu Arg Phe
       370                 375                 380
   ctc tac cag ggc aag tca ccc acc gcg ggc ggc ccc tac gac cag ctg     1200
   Leu Tyr Gln Gly Lys Ser Pro Thr Ala Gly Gly Pro Tyr Asp Gln Leu
   385                 390                 395                 400
   ccg tgg cgg ccg ggt gtg ctc acc ctg cgg cgc tga                     1236
   Pro Trp Arg Pro Gly Val Leu Thr Leu Arg Arg
                   405                 410
<210> 150
   <211> 411
   <212> PRT
   <213> Streptomyces ambofaciens
<400> 150
   Leu Gln Ile Tyr Asp Cys Trp Gly Gly Ser Asn Gln Gln Trp Thr Leu
   1               5                   10                  15
   Thr Asp Asp Arg Arg Leu Thr Val Tyr Gly Asn Lys Cys Leu Asp Val
               20                  25                  30
   Pro Gly His Ala Thr Thr Pro Gly Thr Arg Val Gln Ile Trp Ser Cys
           35                  40                  45
   Ser Gly Gly Ala Asn Gln Gln Trp Arg Val Asn Ser Asp Gly Thr Val
       50                  55                  60
   Val Gly Val Glu Ser Gly Leu Cys Leu Glu Ala Ala Gly Ala Gly Thr
   65                  70                  75                  80
   Ala Asn Gly Thr Ala Val Gln Ile Gly Thr Cys Val Gly Gly Ser Gly
                   85                  90                  95
   Gln Lys Trp Ser Gly Leu Thr Gly Thr Pro Pro Thr Asp Gly Ala Cys
               100                 105                 110
   Ala Leu Pro Ser Ala Tyr Arg Trp Ser Ser Thr Gly Val Leu Ala Gln
           115                 120                 125
   Pro Ala Asn Gly Trp Ala Ala Val Lys Asp Phe Thr Thr Val Thr His
       130                 135                 140
   Asn Gly Lys His Leu Val Tyr Ala Ser Asn Val Ser Gly Ser Ser Tyr
   145                 150                 155                 160
   Gly Ser Met Met Phe Ser Pro Phe Thr Asn Trp Ser Asp Met Ala Ser
                   165                 170                 175
   Ala Gly Gln Thr Gly Met Ser Gln Ala Ala Val Ala Pro Thr Leu Phe
               180                 185                 190
   Tyr Phe Ala Pro Lys Asn Val Trp Val Leu Ala Tyr Gln Trp Gly Ala
           195                 200                 205
   Trp Pro Phe Val Tyr Arg Thr Ser Ser Asp Pro Ala Asn Pro Asn Gly
       210                 215                 220
   Trp Ser Ala Pro Gln Pro Leu Phe Thr Gly Ser Ile Ser Gly Ser Asp
   225                 230                 235                 240
   Thr Gly Pro Ile Asp Gln Thr Leu Ile Ala Asp Gly Gln Asn Met Tyr
                   245                 250                 255
   Leu Phe Phe Ala Gly Asp Asn Gly Lys Ile Tyr Arg Ala Ser Met Pro
               260                 265                 270
   Ile Gly Asn Phe Pro Gly Ser Phe Gly Ser Ser Tyr Thr Thr Ile Met
                   275                 280                 285
   Ser Asp Thr Lys Ala Asn Leu Phe Glu Gly Val Gln Val Tyr Lys Val
       290                 295                 300
   Gln Gly Gln Asn Gln Tyr Leu Met Ile Val Glu Ala Met Gly Ala Asn
   305                 310                 315                 320
   Gly Arg Tyr Phe Arg Ser Phe Thr Ala Ser Ser Leu Ser Gly Ser Trp
                   325                 330                 335
   Thr Pro Gln Ala Ala Ser Glu Ser Asn Pro Phe Ala Gly Lys Ala Asn
               340                 345                 350
   Ser Gly Ala Thr Trp Thr Asn Asp Ile Ser His Gly Asp Leu Val Arg
           355                 360                 365
   Asp Asn Pro Asp Gln Thr Met Thr Val Asp Pro Cys Asn Leu Arg Phe
       370                 375                 380
   Leu Tyr Gln Gly Lys Ser Pro Thr Ala Gly Gly Pro Tyr Asp Gln Leu
   385                 390                 395                 400
   Pro Trp Arg Pro Gly Val Leu Thr Leu Arg Arg
                   405                 410
<210> 151
   <211> 22
   <212> DNA
   <213> Artificial
<220>
   <223> Oligonucléot-ide KF36
<400> 151
   ttgccgtagc cgaggaccag cg 22
<210> 152
   <211> 22
   <212> DNA
   <213> Artificial
<220>
   <223> Oligonucléotide KF37
<400> 152
   cacatggccc tggaggaccc tg 22
<210> 153
   <211> 30
   <212> DNA
   <213> Artificial
<220>
   <223> oligonucléotide KF42
<400> 153
   aagcttgtac ggcccacaga atgatgtcac 30
<210> 154
   <211> 30
   <212> DNA
   <213> Artificial
<220>
   <223> Oligonucléotide KF43
<400> 154
   aagcttcgac taccttggtg atctcgcctt 30
<210> 155
   <211> 65
   <212> DNA
   <213> Artificial
<220>
   <223> oligonucléotide KF32
<400> 155
   caaccgcttg agctgctcca tcaactgctg ggccgaggta tcgcgcgcgc ttcgttcggg 60
acgaa 65
<210> 156
   <211> 65
   <212> DNA
   <213> Artificial
<220>
   <223> Oligonucléotide KF33
<400> 156
   tgggtcccgc cgcgcggcac gacttcgact cgctcgtcta tctgcctctt cgtcccgaag 60
caact 65
<210> 157
   <211> 65
   <212> DNA
   <213> Artificial
<220>
   <223> Oligonucléotide EDR71
<400> 157
   cgtcatcgac gtgcggggaa gacagaggtg ataccgatga tcgcgcgcgc ttcgttcggg 60
acgaa 65
<210> 158
   <211> 65
   <212> DNA
   <213> Artificial
<220>
   <223> Oligonucléotide EDR72
<400> 158
   gccagcacct cgtccagctg ctcgacggaa ctcaccccca tctgcctctt cgtcccgaag 60
caact 65
<210> 159
   <211> 66
   <212> DNA
   <213> Artificial
<220>
   <223> oligonucléotide KF52
<400> 159
   gatccgccag cctcacgtca cgccgcgccg cctccctgac atcgcgcgcg cttcgttcgg 60
gacgaa 66
<210> 160
   <211> 66
   <212> DNA
   <213> Artificial
<220>
   <223> Oligonucléotide KF53
<400> 160
   gaggcggacg tcggtacgcg gtgggagccg gagttcgaca atctgcctct tcgtcccgaa 60
gcaact 66
<210> 161
   <211> 2540
   <212> DNA
   <213> Streptomyces ambofaciens.
<400> 161
   ctgcagggcg cccacggggt ggcggtcgta tcggaactcg gcgtcgtagt cgtcctccag 60
   caccaggccg tcgacctcgc gggcccaggc gaccagctcg ccgcgacggg ccggggcgag 120
   gaccacaccg gtcgggaact ggtgggcggg agcgacgatc accgaccgga cctgaggcgc 180
   gcgccgcagc aggtcgaccc gcaggccgtc gccgtccacg ggaaccggga cgggcaccag 240
   cccctccgcc ccgaccgtgg cgctcagccg cgaccagcca gggtcctcca gggccatgtg 300
   ggtgtggccc tcggtccgca ggacccggca cagcctgcgc accgcgtcca gcgtgcccgc 360
   gcagacgacg agctgccggg cttccagcga cgcgccgcgt ccccgcacga ggtaggccgc 420
   gagccgctgc cggagccggg cgagaccggc cggatcgggg tagccgagct cgccccaggt 480
   cagtgcgccg gtggcctccc gcaccgcgtc cgcccaccgt ccgcggggaa aggcccgcag 540
   gtcgggtatg ccgggcagca tgtcgaactc cggctcccac cgcgtaccga cgtccgcctc 600
   gggcgtcacc ggagcgggga cggcctgcgc cctgacccgg gtggccgagc cgctgcgcgc 660
   ctccaggtac ccctccgcga cgagctgcgc gtaggcctcc gtcaccaccc accgcgagca 720
   gcccaggtcc gccgccagcg ccctgctcgg cggcagcgcg ctgccggagg cgatccgccc 780
   accggtcacc gccgcccgca gggcacggct cagccggacg tgcagcgggc cgtccgccgg 840
   cccgcccagt tcgagaaagg ttccccatgc cgggtcggtc cggtcgtcgc ccacggcagc 900
   cgatgctacc caccgccccg cgacggccgg gcggcggcgt ccacgggccc ggtgcacact 960
   ggatggcgtg gcggagacgg accggcatct cgaccagcgg ctggagcagg ccatcatgga   1020
   cctgctggag cgacgggcgg ccactgcctc gatctgtccg tccgacgccg cgcgtcgggt   1080
   ccacgagggt gacgacgagg gctggcgagc cctgctggaa cccgcgcgcc gagcggcctg   1140
   gcgtctggtg gggtccggcg aggtcgaggt gacccaggcc ggccggcccg tcacgcaggc   1200
   cgaggcccgc gggcccgtcc gcattcgtcg ggcgggttcc tgacggctcg atccgaaggc   1260
   ggaactcccg tgccccggcg gcacgggagc tcgacgccgg gaggtcagcg ccgcagggtg   1320
   agcacacccg gccgccacgg cagctggtcg taggggccgc ccgcggtggg tgacttgccc   1380
   tggtagagga accgcaggtt gcaggggtcg acggtcatgg tctggtcggg gttgtcgcgg   1440
   accaggtcac cgtggctgat gtcgttggtc caggtggccc cgctgttggc cttgcccgcg   1500
   aaggggttgc tctcgctcgc ggcctgcggg gtccacgagc cgctcaggct ggaggccgtg   1560
   aaggaacgga agtagcgtcc gttcgcgccc atcgcctcga cgatcatcag gtactggttc   1620
   tggccctgaa ccttgtagac ctgcacgccc tcgaacaggt tggccttcgt gtcgctcatg   1680
   atcgtcgtgt acgacgagcc gaagctgccc gggaagttcc cgatcggcat gctcgcccgg   1740
   tagatcttgc cgttgtcacc ggcgaagaac aggtacatgt tctgtccgtc ggcgatcagg   1800
   gtctggtcga tcgggccggt gtcggatcct gagatgctcc cggtgaacaa cggctgcgga   1860
   gccgaccagc cgttggggtt ggccgggtcg ctggacgtgc ggtagacgaa gggccacgcg   1920
   ccccactggt acgccagcac ccagacgttc ttgggcgcga agtagaacag ggtgggcgcc   1980
   accgccgcct gactcatccc ggtctggccg gccgacgcca tgtccgacca gttcgtgaag   2040
   gggctgaaca tcatcgagcc gtaggacgac cccgacacgt tcgacgcgta gaccaggtgc   2100
   ttgccgttgt gggtcacggt ggtgaagtcc ttgaccgccg cccacccgtt cgccggctgc   2160
   gccagcacac ccgtcgacga ccaccggtac gccgacggaa gggcacacgc cccgtccgtc   2220
   gggggcgtcc cggtcaggcc ggaccacttc tgcccgctgc caccgacgca cgtcccgatc   2280
   tgcaccgccg tgccgttggc cgtaccggcg cccgcggcct ccaggcacag cccggactcc   2340
   acgccgacga ccgtgccgtc ggagttcacc cgccactgct ggttcgcacc gccggaacag   2400
   ctccagatct gcacccgcgt cccgggtgtg gtggcgtgac ccggaacgtc caggcacttg   2460
   ttgccgtaca cggtcagccg acggtcgtcc gtcaacgtcc actgctgatt gctcccgccc   2520
   cagcagtcgt atatctgcag                                               2540